# EUROPEAN PATENT SPECIFICATION

(11) **EP 3 960 744 B1**
(45) Date of publication and mention of the grant of the patent: **21.08.2024**
(21) Application number: 20795315.9
(22) Date of filing: 14.04.2020
(51) Int. Cl.: C09K 11/06, C07F 5/02, H10K 85/30, H10K 85/60, H10K 59/00, C08G 61/12, C09D 11/037, C09D 11/10, C09D 165/00, C09D 11/32

(54) **CYCLOALKANE-FUSED POLYCYCLIC AROMATIC COMPOUND**
CYCLOALKANKONDENSIERTE POLYCYCLISCHE AROMATISCHE VERBINDUNG
COMPOSÉ AROMATIQUE POLYCYCLIQUE CONDENSÉ PAR UN CYCLOALCANE

(30) Priority: 22.04.2019 JP 2019080882; 04.10.2019 JP 2019183417; 21.11.2019 JP 2019210200; 18.12.2019 JP 2019228391
(43) Date of publication of application: 02.03.2022
(73) Proprietor: Kwansei Gakuin Educational Foundation, Nishinomiya-shi, Hyogo 662-8501 (JP); SK Materials JNC Co., Ltd., Gyeonggi-do, 18469 (KR)
(72) Inventor: HATAKEYAMA, Takuji, Sanda-shi, Hyogo 669-1337 (JP); SHIREN, Kazushi, Ichihara-shi, Chiba 290-8551 (JP); KAGEYAMA, Akiko, Ichihara-shi, Chiba 290-8551 (JP); TANAKA, Hiroyuki, Ichihara-shi, Chiba 290-8551 (JP); MIZUTANI, Akihide, Ichihara-shi, Chiba 290-8551 (JP); SASADA, Yasuyuki, Ichihara-shi, Chiba 290-8551 (JP)
(74) Representative: Hoffmann Eitle
(86) International application number: PCT/JP2020/016371
(87) International publication number: WO 2020/218079

(56) References cited:
- WO-A1-2015/102118
- WO-A1-2016/143624
- WO-A1-2016/152418
- WO-A1-2017/138526
- WO-A1-2017/138526
- WO-A1-2020/017931
- CN-A- 108 358 905
- CN-A- 108 358 905

## Description

### BACKGROUND

### Technical Field

The present invention relates to a cycloalkane-fused polycyclic aromatic compound and a multimer thereof (hereinafter, they are also collectively referred to as "polycyclic aromatic compound"), a material for an organic device comprising said polycyclic aromatic compound and an organic electroluminescent element using the polycyclic aromatic compound, as well as a display apparatus and a lighting apparatus. Incidentally, here, the term "organic electroluminescent element" may be referred to as "organic EL element" or simply "element".

### Related Art

Conventionally, a display apparatus employing a luminescent element that is electroluminescent can be subjected to reduction in power consumption and reduction in thickness, and therefore various studies have been conducted thereon. Moreover, an organic electroluminescent element formed of an organic material has been studied actively because reduction in weight and expansion in size are easily achieved. Particularly, active research has been hitherto conducted on development of an organic material having luminescence characteristics for blue light, which is one of the three primary colors of light, and development of an organic material having charge transport capability for holes, electrons, and the like (having a potential for serving as a semiconductor or a superconductor), irrespective of whether the organic material is a polymer compound or a low molecular weight compound.

An organic EL element has a structure having a pair of electrodes composed of a positive electrode and a negative electrode, and a single layer or a plurality of layers which are disposed between the pair of electrodes and contain an organic compound. The layer containing an organic compound includes a light emitting layer, a charge transport/injection layer for transporting or injecting charges such as holes or electrons, and the like, and various organic materials suitable for these layers have been developed.

As a material for the light emitting layer, for example, a benzofluorene-based compound or the like has been developed (WO 2004/061047 A). Furthermore, as a hole transport material, for example, a triphenylamine-based compound or the like has been developed (JP 2001-172232 A). Furthermore, as an electron transport material, for example, an anthracene-based compound or the like has been developed (JP 2005-170911 A).

Furthermore, in recent years, a material obtained by improving a triphenylamine derivative has also been reported as a material used in an organic EL element or an organic thin film solar cell (WO 2012/118164 A). This material is characterized in that flatness thereof has been increased by linking aromatic rings constituting triphenylamine with reference to N,N'-diphenyl-N,N'-bis(3-methylphenyl)-1,1'-biphenyl-4,4'-diamine (TPD) which has been already put to practical use. In this literature, for example, evaluation of the charge transporting characteristics of a NO-linked system compound (compound 1 of page 63) has been made. However, there is no description on a method for manufacturing materials other than the NO-linked system compound. Furthermore, when elements to be linked are different, the overall electron state of the compound is different. Therefore, the characteristics obtainable from materials other than the NO-linked system compound are not known. Examples of such a compound are also found elsewhere (WO 2011/107186 A). For example, since a compound having a conjugated structure involving high energy of triplet exciton (T1) can emit phosphorescent light having a shorter wavelength, the compound is useful as a material for a blue light emitting layer. Furthermore, there is also a demand for a novel compound having a conjugated structure with high T1 as each of an electron transport material and a hole transport material with a light emitting layer interposed therebetween. CN 108358905 A relates to the technical field of organic electroluminescent materials and particularly relates to a compound, a light-emitting material, a device and a display device. It is said that the compound can be used as a thermally activated delayed fluorescent (TADF) material. When the compound is used as a light-emitting material or a main body material of a light-emitting layer of an organic light-emitting device, the compound can allegedly achieve high light-emitting efficiency. WO 2017/138526 A pertains to a delayed fluorescent organic EL element having optimal light emission characteristics, by a polycyclic aromatic compound as represented by the general formula (1) of this document or a multimer of a polycyclic aromatic compound having a plurality of structures represented by general formula (1).

A host material for an organic EL element is generally a molecule in which a plurality of existing aromatic rings of benzene, carbazole, and the like is linked to one another via a single bond, a phosphorus atom, or a silicon atom. This is because a large HOMO-LUMO gap required for a host material (band gap Eg in a thin film) is secured by linking many aromatic rings each having a relatively small conjugated system. Furthermore, a host material for an organic EL element, using a phosphorescent material or a thermally activated delayed fluorescent material needs high triplet excitation energy (E_{T}). However, the triplet excitation energy (E_{T}) can be increased by localizing SOMO1 and SOMO2 in a triplet excited state (T1) by linking a donor-like or acceptor-like aromatic ring or substituent to a molecule, and thereby reducing an exchange interaction between the two orbitals. However, an aromatic ring having a small conjugated system does not have sufficient redox stability, and an element using a molecule obtained by linking existing aromatic rings as a host material does not have a sufficient lifetime. Meanwhile, a polycyclic aromatic compound having an extended n-conjugated system generally has excellent redox stability. However, since the HOMO-LUMO gap (band gap Eg in a thin film) or triplet excitation energy (E_{T}) is low, the polycyclic aromatic compound has been considered to be unsuitable as a host material.

### Prior art references

WO 2004/061047 A
JP 2001-172232 A
JP 2005-170911 A
WO 2012/118164 A
WO 2011/107186 A
WO 2015/102118 A
CN 108358905 A
WO 2017/138526 A

### SUMMARY

As described above, various materials have been developed as a material used for an organic EL element. However, in order to increase a selection range of the material for the organic EL element, it is desired to develop a material formed from a compound different from conventional compounds. Particularly, characteristics of an organic EL element obtained from a material other than NO-linked system compounds reported in WO 2004/061047 A, JP 2001-172232 A, JP 2005-170911 A, and WO 2012/118164 A, and a method for manufacturing the organic EL element are not known.

Furthermore, WO 2015/102118 A has reported a boron-containing polycyclic aromatic compound and an organic EL element using the polycyclic aromatic compound. However, in order to further improve characteristics of the element, a material for a light emitting layer capable of improving luminous efficiency and element lifetime, particularly a dopant material is required.

As a result of intensive studies to solve the above problems, the present inventors have found that an excellent organic EL element is obtained by disposing a layer containing a polycyclic aromatic compound fused with cycloalkane between a pair of electrodes to constitute, for example, an organic EL element, and have completed the present invention. That is, the present invention provides such a cycloalkane-fused polycyclic aromatic compound as follows or a multimer thereof, and further provides a material for an organic device containing such a cycloalkane-fused polycyclic aromatic compound as follows or a multimer thereof, such as an organic EL element.

Here, a chemical structure or a substituent may be represented by the number of carbon atoms. However, in a case where a chemical structure is substituted by a substituent or in a case where a substituent is further substituted by a substituent, the number of carbon atoms means the number of carbon atoms of each chemical structure or each substituent, and does not mean the total number of carbon atoms of a chemical structure and a substituent or the total number of carbon atoms of a substituent and a substituent. For example, "substituent B having the number of carbon atoms of Y substituted by substituent A having the number of carbon atoms X" means that "substituent B having the number of carbon atoms of Y" is substituted by "substituent A having the number of carbon atoms of X", and the number of carbon atoms of Y is not the total number of carbon atoms of the substituent A and the substituent B. For example, "substituent B having the number of carbon atoms of Y substituted by substituent A" means that substituent B having the number of carbon atoms of Y is substituted by "substituent A (in which the number of carbon atoms is not limited)", and the number of carbon atoms of Y is not the total carbon number of the substituent A and the substituent B.

[1] A polycyclic aromatic compound represented by the following general formula (1) or a multimer of a polycyclic aromatic compound having a plurality of structures each represented by the following general formula (1) : 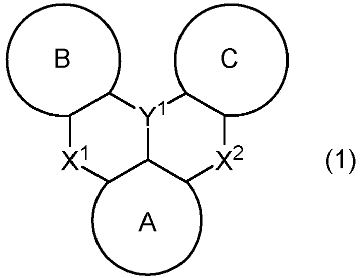 wherein in the above formula (1),
   ring A, ring B, and ring C each independently represent an aryl ring or a heteroaryl ring, while at least one hydrogen atom in these rings may be substituted,
   Y¹ represents B, P, P=O, P=S, Al, Ga, As, Si-R, or Ge-R, while R in the Si-R and Ge-R represents an aryl, an alkyl or a cycloalkyl,
   X¹ and X² each independently represent >0, >N-R, >C(-R)₂, >S, or >Se, while R of the >N-R represents an optionally substituted aryl, an optionally substituted heteroaryl, an optionally substituted alkyl, or an optionally substituted cycloalkyl, R of the >C(-R)₂ represents a hydrogen atom, an optionally substituted aryl, an optionally substituted alkyl, or an optionally substituted cycloalkyl, and at least one of R in the >N-R and R in the >C(-R)₂ may be bonded to at least one of the ring A, ring B, and ring C via a linking group or a single bond,
   at least one hydrogen atom in the compound or structure represented by formula (1) may be substituted by a deuterium atom, cyano, or a halogen atom, and
   at least one of the ring A, ring B, ring C, aryl and heteroaryl in the compound or structure represented by the formula (1) is fused with at least one cycloalkane, at least one hydrogen atom in the cycloalkane is substituted at an α-position carbon atom of the cycloalkane, at least one -CH₂- in the cycloalkane may be substituted with -O-.
[2] The polycyclic aromatic compound or the multimer thereof according to the above [1], wherein
   in the above formula (1),
   the ring A, ring B, and ring C each independently represent an aryl ring or a heteroaryl ring, while at least one hydrogen atom in these rings may be substituted by a substituted or unsubstituted aryl, a substituted or unsubstituted heteroaryl, a substituted or unsubstituted diarylamino, a substituted or unsubstituted diheteroarylamino, a substituted or unsubstituted arylheteroarylamino, a substituted or unsubstituted diarylboryl (the two aryls may be bonded via a single bond or a linking group), a substituted or unsubstituted alkyl, a substituted or unsubstituted cycloalkyl, a substituted or unsubstituted alkoxy, a substituted or unsubstituted aryloxy, or a substituted silyl, and each of these rings has a 5-membered or 6-membered ring sharing a bond with a fused bicyclic structure at a center of the above formula constituted by Y¹, X¹, and X²,
   Y¹ represents B, P, P=O, P=S, Al, Ga, As, Si-R, or Ge-R, while R in the Si-R and Ge-R represents an aryl, an alkyl or a cycloalkyl,
   X¹ and X² each independently represent >O, >N-R, >C(-R)₂, >S, or >Se, while R in the >N-R represents an aryl optionally substituted by an alkyl or a cycloalkyl, a heteroaryl optionally substituted by an alkyl or a cycloalkyl, an alkyl, or a cycloalkyl, R in the >C(-R)₂, represents a hydrogen atom, an aryl optionally substituted by an alkyl or a cycloalkyl, an alkyl, or a cycloalkyl, at least one of R in the >N-R and R in the >C(-R)₂ may be bonded to at least one of the ring A, ring B, and ring C via -O-, -S-, -C(-R)₂-, or a single bond, and R in the -C(-R)₂- represents a hydrogen atom or an alkyl or a cycloalkyl,
   at least one hydrogen atom in the compound or structure represented by formula (1) may be substituted by a deuterium atom, cyano, or a halogen atom,
   in a case of a multimer, the multimer is a dimer or a trimer having two or three structures each represented by general formula (1), and
   at least one of the ring A, ring B, ring C, aryl and heteroaryl in the compound or structure represented by the formula (1) is fused with at least one cycloalkane, at least one hydrogen atom in the cycloalkane is substituted at an α-position carbon atom of the cycloalkane, at least one -CH₂- in the cycloalkane may be substituted with -O-.
[3] The polycyclic aromatic compound according to the above [1], represented by the following general formula (2): 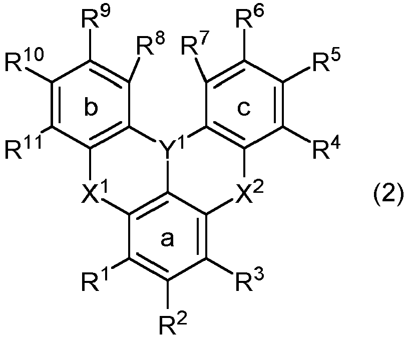
   wherein in the above formula (2),
   any "-C (-R) =" (where R is R¹ to R¹¹ in the formula (2)) in ring a, ring b, and ring c may be replaced with "-N=", any "-C(-R)=C(-R)-" (where R is R¹ to R¹¹ in the formula (2)) may be replaced with "-N(-R)-", "-O-", or "-S-", R of the "-N(-R)-" represents an aryl, an alkyl, or a cycloalkyl,
   R¹ to R¹¹ each independently represent a hydrogen atom, an aryl, a heteroaryl, a diarylamino, a diheteroarylamino, an arylheteroarylamino, a diarylboryl (the two aryls may be bonded via a single bond or a linking group), an alkyl, a cycloalkyl, an alkoxy, an aryloxy, a triarylsilyl, a trialkylsilyl, a tricycloalkylsilyl, a dialkylcycloalkylsilyl, or an alkyldicycloalkylsilyl, while at least one hydrogen atom in these may be substituted by an aryl, a heteroaryl, an alkyl, or a cycloalkyl, adjacent groups among R¹ to R¹¹ may be bonded to each other to form an aryl ring or a heteroaryl ring together with ring a, ring b, or ring c, at least one hydrogen atom in the ring thus formed may be substituted by an aryl, a heteroaryl, a diarylamino, a diheteroarylamino, an arylheteroarylamino, a diarylboryl (the two aryls may be bonded via a single bond or a linking group), an alkyl, a cycloalkyl, an alkoxy, an aryloxy, a triarylsilyl, a trialkylsilyl, a tricycloalkylsilyl, a dialkylcycloalkylsilyl, or an alkyldicycloalkylsilyl, while at least one hydrogen atom in these may be substituted by an aryl, a heteroaryl, an alkyl, or a cycloalkyl,
   Y¹ represents B, P, P=O, P=S, Al, Ga, As, Si-R, or Ge-R, while R in the Si-R and Ge-R represents an aryl having 6 to 12 carbon atoms, an alkyl having 1 to 6 carbon atoms or a cycloalkyl having 3 to 14 carbon atoms,
   X¹ and X² each independently represent >O, >N-R, >C(-R)₂, >S, or >Se, while R in the >N-R represents an aryl having 6 to 12 carbon atoms, a heteroaryl having 2 to 15 carbon atoms, an alkyl having 1 to 6 carbon atoms, or a cycloalkyl having 3 to 14 carbon atoms, R in the >C(-R)₂ represents a hydrogen atom, an aryl having 6 to 12 carbon atoms, an alkyl having 1 to 6 carbon atoms, or a cycloalkyl having 3 to 14 carbon atoms, at least one of R in the >N-R and R in the >C(-R)₂ may be bonded to at least one of the ring a, ring b, and ring c via -O-, -S-, -C(-R)₂-, or a single bond, and R in the - C(-R)₂- represents an alkyl having 1 to 6 carbon atoms or a cycloalkyl having 3 to 14 carbon atoms,
   at least one hydrogen atom in the compound represented by formula (2) may be substituted by a deuterium atom, cyano or a halogen atom, and
   at least one of the ring a, ring b, ring c, formed ring, aryl and heteroaryl in the compound represented by the formula (2) is fused with at least one cycloalkane having 3 to 24 carbon atoms, at least one hydrogen atom in the cycloalkane is substituted at an α-position carbon atom of the cycloalkane by an aryl having 6 to 30 carbon atoms, a heteroaryl having 2 to 30 carbon atoms, an alkyl having 1 to 24 carbon atoms, or a cycloalkyl having 3 to 24 carbon atoms, at least one -CH₂- in the cycloalkane may be substituted with -O-.
[4] The polycyclic aromatic compound according to the above [3], wherein
   in the above formula (2),
   any "-C (-R) =" (where R is R¹ to R¹¹ in the formula (2)) in ring a, ring b, and ring c may be replaced with "-N=", any "-C(-R)=C(-R)-" (where R is R¹ to R¹¹ in the formula (2)) may be replaced with "-N(-R)-", "-O-", or "-S-", R of the "-N(-R)-" represents an aryl having 6 to 12 carbon atoms, an alkyl having 1 to 6 carbon atoms, or a cycloalkyl having 3 to 14 carbon atoms,
   R¹ to R¹¹ each independently represent a hydrogen atom, an aryl having 6 to 30 carbon atoms, a heteroaryl having 2 to 30 carbon atoms, a diarylamino (the aryl is an aryl having 6 to 12 carbon atoms), a diarylboryl (the aryl is an aryl having 6 to 12 carbon atoms, the two aryls may be bonded via a single bond or a linking group), an alkyl having 1 to 24 carbon atoms, a cycloalkyl having 3 to 24 carbon atoms, a triarylsilyl (the aryl is an aryl having 6 to 12 carbon atoms), or a trialkylsilyl (the alkyl is an alkyl having 1 to 6 carbon atoms), adjacent groups among R¹ to R¹¹ may be bonded to each other to form an aryl ring having 9 to 16 carbon atoms or a heteroaryl ring having 6 to 15 carbon atoms together with ring a, ring b, or ring c, at least one hydrogen atom in the ring thus formed may be substituted by an aryl having 6 to 10 carbon atoms, an alkyl having 1 to 12 carbon atoms, a cycloalkyl having 3 to 16 carbon atoms, a triarylsilyl (the aryl is an aryl having 6 to 12 carbon atoms), or a trialkylsilyl (the alkyl is an alkyl having 1 to 5 carbon atoms),
   Y¹ represents B, P, P=O, P=S, or Si-R, while R in the Si-R represents an aryl having 6 to 10 carbon atoms, an alkyl having 1 to 5 carbon atoms or a cycloalkyl having 5 to 10 carbon atoms,
   X¹ and X² each independently represent >O, >N-R, >C(-R)₂, or >S, while R in the >N-R represents an aryl having 6 to 10 carbon atoms, an alkyl having 1 to 5 carbon atoms, or a cycloalkyl having 5 to 10 carbon atoms, R in the >C(-R)₂ represents a hydrogen atom, an aryl having 6 to 10 carbon atoms, an alkyl having 1 to 5 carbon atoms, or a cycloalkyl having 5 to 10 carbon atoms,
   at least one hydrogen atom in the compound represented by formula (2) may be substituted by a deuterium atom, cyano or a halogen atom, and
   at least one of the ring a, ring b, ring c, formed ring, aryl and heteroaryl in the compound represented by the formula (2) is fused with at least one cycloalkane having 3 to 20 carbon atoms, at least one hydrogen atom in the cycloalkane is substituted at an α-position carbon atom of the cycloalkane by an aryl having 6 to 16 carbon atoms, a heteroaryl having 2 to 22 carbon atoms, an alkyl having 1 to 12 carbon atoms, or a cycloalkyl having 3 to 16 carbon atoms
      or wherein
   in the above formula (2),
   any "-C (-R) =" (where R is R¹ to R¹¹ in the formula (2)) in ring a, ring b, and ring c may be replaced with "-N=", any "-C(-R)=C(-R)-" (where R is R¹ to R¹¹ in the formula (2)) may be replaced with "-N(-R)-", "-O-", or "-S-", R of the "-N(-R)-" represents an aryl having 6 to 10 carbon atoms, an alkyl having 1 to 5 carbon atoms, or a cycloalkyl having 5 to 10 carbon atoms,
   R¹ to R¹¹ each independently represent a hydrogen atom, an aryl having 6 to 16 carbon atoms, a heteroaryl having 2 to 20 carbon atoms, a diarylamino (the aryl is an aryl having 6 to 10 carbon atoms), an alkyl having 1 to 12 carbon atoms, or a cycloalkyl having 3 to 16 carbon atoms,
   Y¹ represents B, P, P=O, or P=S,
   X¹ and X² each independently represent >O, >N-R, or >C(-R)₂, while R in the >N-R represents an aryl having 6 to 10 carbon atoms, an alkyl having 1 to 5 carbon atoms, or a cycloalkyl having 5 to 10 carbon atoms, R in the >C(-R)₂ represents a hydrogen atom, an aryl having 6 to 10 carbon atoms, an alkyl having 1 to 5 carbon atoms, or a cycloalkyl having 5 to 10 carbon atoms,
   at least one hydrogen atom in the compound represented by formula (2) may be substituted by a deuterium atom, cyano or a halogen atom, and
   at least one of the ring a, ring b, ring c, and aryl having 6 to 10 carbon atoms as R of the >N-R in the compound represented by the formula (2) is fused with at least one cycloalkane having 3 to 16 carbon atoms, at least one hydrogen atom in the cycloalkane is substituted at an α-position carbon atom of the cycloalkane by an alkyl having 1 to 6 carbon atoms or a cycloalkyl having 3 to 14 carbon atoms
      or wherein
   in the above formula (2),
   R¹ to R¹¹ each independently represent a hydrogen atom, an aryl having 6 to 16 carbon atoms, a diarylamino (the aryl is an aryl having 6 to 10 carbon atoms), an alkyl having 1 to 12 carbon atoms, or a cycloalkyl having 3 to 16 carbon atoms,
   Y¹ represents B,
   X¹ and X² both represent >N-R, or X¹ represents >N-R and X² represents >O, while R in the >N-R represents an aryl having 6 to 10 carbon atoms, an alkyl having 1 to 5 carbon atoms, or a cycloalkyl having 5 to 10 carbon atoms,
   at least one hydrogen atom in the compound represented by formula (2) may be substituted by a deuterium atom or a halogen atom, and
   at least one of the ring a, ring b, ring c, and aryl having 6 to 10 carbon atoms as R of the >N-R in the compound represented by the formula (2) is fused with at least one cycloalkane having 3 to 14 carbon atoms, at least one hydrogen atom in the cycloalkane is substituted at an α-position carbon atom of the cycloalkane by an alkyl having 1 to 5.)
[5] The polycyclic aromatic compound or the multimer thereof according to any one of the above [1] to [4], which is substituted by a diarylamino group fused with one or more cycloalkanes, a carbazolyl group fused with one or more cycloalkanes, or a benzocarbazolyl group fused with one or more cycloalkanes.
[6] The polycyclic aromatic compound according to any one of the above [3] or [4], wherein R² is a diarylamino group fused with one or more cycloalkanes or a carbazolyl group fused with one or more cycloalkanes.
[7] The polycyclic aromatic compound according to the above [5] or [6], wherein the cycloalkane is a cycloalkane having 3 to 20 carbon atoms.
[8] The polycyclic aromatic compound or the multimer thereof according to any one of the above [1] to [6], wherein the halogen is fluorine.
[9] A polycyclic aromatic compound according to the above [1], which is represented by any one of the following structural formulas. 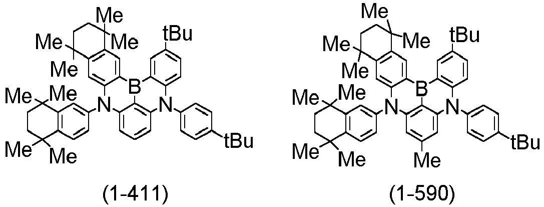 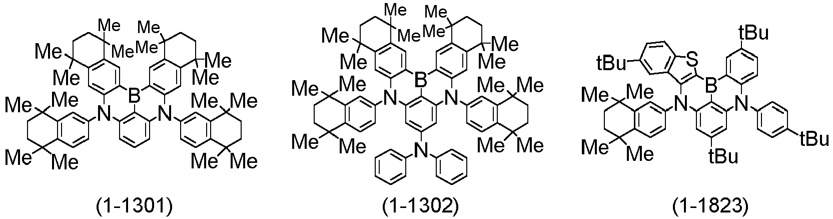 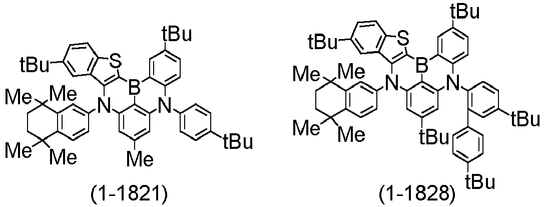 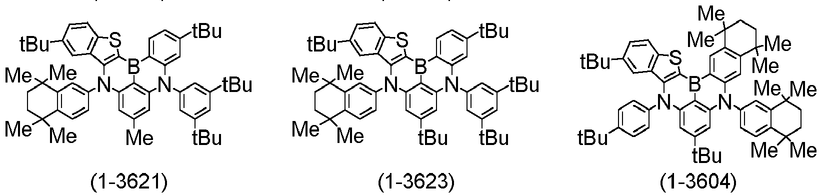 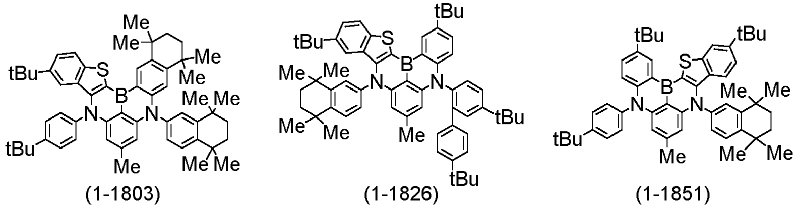 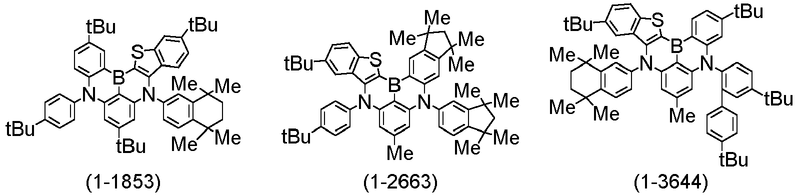 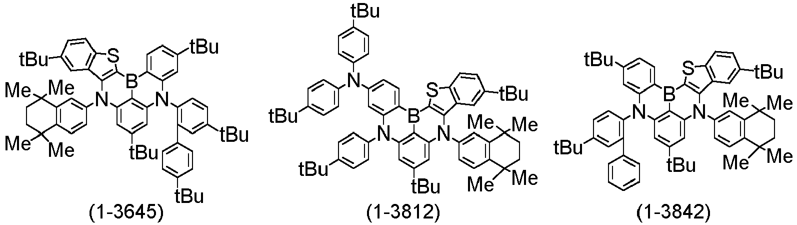 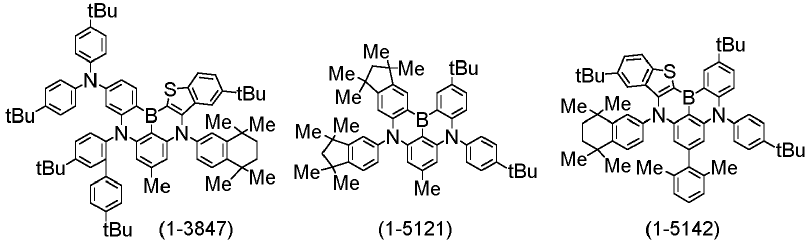 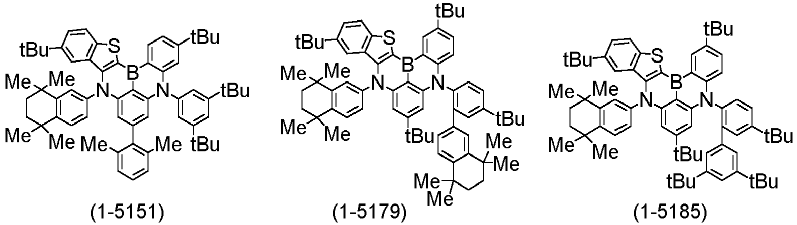 wherein in each of the above structural formulas, "Me" indicates a methyl group and "tBu" indicates a t-butyl group.
[10] A material for an organic device, the material comprising the polycyclic aromatic compound or the multimer thereof according to any one of the above [1] to [9].
[11] The material for an organic device according to 1 [10], which is a material for an organic electroluminescent element, a material for an organic field effect transistor, or a material for an organic thin film solar cell,
   preferably wherein the material for an organic electroluminescent element is a material for a light emitting layer.
[12] An organic electroluminescent element comprising: a pair of electrodes composed of a positive electrode and a negative electrode; and an organic layer disposed between the pair of electrodes and comprising the polycyclic aromatic compound or the multimer thereof according to any one of the above [1] to [9].
[13] The organic electroluminescent element according to the above [12], wherein the organic layer is a light emitting layer, preferably wherein the light emitting layer comprises a host and the polycyclic aromatic compound or the multimer thereof, the reactive compound, the polymer compound, the crosslinked polymer, the pendant type polymer compound, or the pendant type crosslinked polymer as a dopant,
   preferably wherein the host is an anthracene-based compound, a fluorene-based compound, or a dibenzochrysene-based compound.
[14] The organic electroluminescent element according to [12] or [13], further comprising at least one of an electron transport layer and an electron injection layer disposed between the negative electrode and the light emitting layer, wherein at least one of the electron transport layer and the electron injection layer contains at least one selected from the group consisting of a borane derivative, a pyridine derivative, a fluoranthene derivative, a BO-based derivative, an anthracene derivative, a benzofluorene derivative, a phosphine oxide derivative, a pyrimidine derivative, a carbazole derivative, a triazine derivative, a benzimidazole derivative, a phenanthroline derivative, and a quinolinol-based metal complex,
   preferably wherein at least one layer of the electron transport layer and electron injection layer further include at least one selected from the group consisting of an alkali metal, an alkaline earth metal, a rare earth metal, an oxide of an alkali metal, a halide of an alkali metal, an oxide of an alkaline earth metal, a halide of an alkaline earth metal, an oxide of a rare earth metal, a halide of a rare earth metal, an organic complex of an alkali metal, an organic complex of an alkaline earth metal, and an organic complex of a rare earth metal,
   further preferably wherein at least one layer of a hole injection layer, a hole transport layer, a light emitting layer, an electron transport layer, and an electron injection layer comprises a polymer compound obtained by polymerizing a low molecular weight compound that can form each of the layers as a monomer or a crosslinked polymer obtained by further crosslinking the polymer compound, or a pendant type polymer compound obtained by a reaction between the low molecular weight compound that can form each of the layers and a main chain type polymer or a pendant type crosslinked polymer obtained by further crosslinking the pendant type polymer compound.
[15] A display apparatus or a lighting apparatus comprising the organic electroluminescent element according to any one of the above [12] to [14].

According to a preferable embodiment of the present invention, a novel cycloalkane-fused polycyclic aromatic compound that can be used as a material for an organic device such as a material for an organic EL element can be provided, and an excellent organic device such as an organic EL element can be provided by using this cycloalkane-fused polycyclic aromatic compound.

Specifically, the present inventors have found that a polycyclic aromatic compound (basic skeleton portion) in which aromatic rings are linked to each other via a hetero element such as boron, phosphorus, oxygen, nitrogen, or sulfur has a large HOMO-LUMO gap (band gap Eg in a thin film) and high triplet excitation energy (E_{T}). It is considered that this is because a decrease in the HOMO-LUMO gap that comes along with extension of a conjugated system is suppressed due to low aromaticity of a 6-membered ring containing a hetero element, and SOMO1 and SOMO2 in a triplet excited state (T1) are localized by electronic perturbation of the hetero element. Furthermore, the polycyclic aromatic compound (basic skeleton portion) containing a hetero element according to an aspect of the present invention reduces an exchange interaction between the two orbitals due to the localization of SOMO1 and SOMO2 in the triplet excited state (T1), and therefore an energy difference between the triplet excited state (T1) and a singlet excited state (S1) is small. The polycyclic aromatic compound exhibits thermally activated delayed fluorescence, and therefore is also useful as a fluorescent material for an organic EL element (including an element using thermally activated delayed fluorescence). Furthermore, a material having high triplet excitation energy (E_{T}) is also useful as an electron transport layer or a hole transport layer of a phosphorescence organic EL element or an organic EL element using thermally activated delayed fluorescence. Moreover, the polycyclic aromatic compound (basic skeleton portion) can arbitrarily shift energy of HOMO and LUMO by introducing a substituent, and therefore ionization potential or electron affinity can be optimized in accordance with a peripheral material.

In addition to the characteristics of the basic skeleton portion, the compound according to an aspect of the present invention can be expected to improve element life and luminance efficiency by suppressing concentration quenching phenomenon by fusing cycloalkane. The element life and luminance efficiency are important characteristics of organic devices, and the use of the compound of the present invention can be expected to have an effect on improving the characteristics of the device. Furthermore, by using the compound according to an aspect of the present invention, it can be expected to lower a melting point or a sublimation temperature. This means that thermal decomposition of a material and the like can be avoided because purification can be performed at a relatively low temperature in sublimation purification which is almost indispensable as a purification method for a material for an organic device such as an organic EL element, requiring high purity. Furthermore, this also applies to a vacuum vapor deposition process which is a powerful means for manufacturing an organic device such as an organic EL element. Since the process can be performed at a relatively low temperature, thermal decomposition of a material can be avoided. As a result, a high performance compound for an organic device can be obtained. Furthermore, since many of polycyclic aromatic compound multimers have a high sublimation temperature due to high molecular weight, high planarity, and the like, it is more effective to lower the sublimation temperature by fused with a cycloalkane. Furthermore, since solubility in an organic solvent is improved by fused a cycloalkane, application to manufacture of an element using a coating process is also possible. However, the present invention is not particularly limited to these principles.

### BRIEF DESCRIPTION OF DRAWINGS

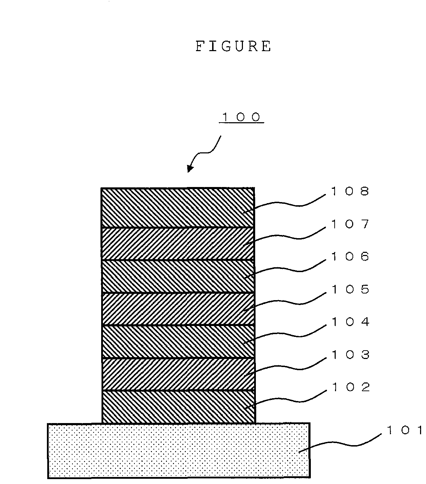

FIG. 1 is a schematic cross-sectional view illustrating an organic EL element according to the present embodiment.

### DETAILED DESCRIPTION

### 1. Cycloalkane-fused polycyclic aromatic compound and multimer thereof

The invention of the present application is a polycyclic aromatic compound represented by the following general formula (1) or a multimer of a polycyclic aromatic compound having a plurality of structures each represented by the following general formula (1), and is preferably a polycyclic aromatic compound represented by the following general formula (2) or a multimer of a polycyclic aromatic compound having a plurality of structures each represented by the following general formula (2). At least one of ring A, ring B, ring C, aryl and heteroaryl in these compounds or structures is fused with a cycloalkane (s) .

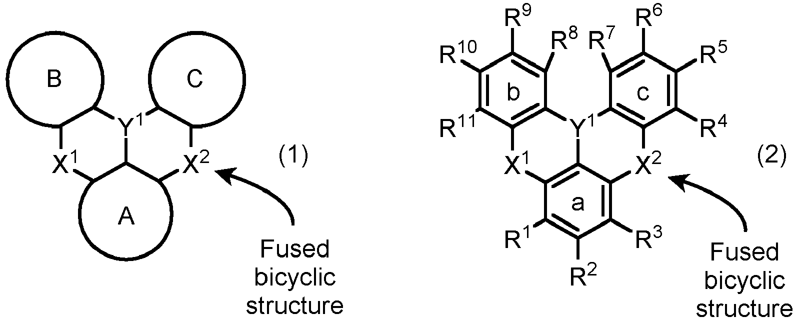

The ring A, ring B, and ring C in general formula (1) each independently represent an aryl ring or a heteroaryl ring, and at least one hydrogen atom in these rings may be substituted by a substituent. This substituent is preferably a substituted or unsubstituted aryl, a substituted or unsubstituted heteroaryl, a substituted or unsubstituted diarylamino, a substituted or unsubstituted diheteroarylamino, a substituted or unsubstituted arylheteroarylamino (an amino group having an aryl and a heteroaryl), a substituted or unsubstituted diarylboryl (the two aryls may be bonded via a single bond or a linking group), a substituted or unsubstituted alkyl, a substituted or unsubstituted cycloalkyl, a substituted or unsubstituted alkoxy, a substituted or unsubstituted aryloxy, or a substituted silyl. In a case where these groups have substituents, examples of the substituents include an aryl, a heteroaryl, an alkyl, and a cycloalkyl. Furthermore, the aryl ring or heteroaryl ring preferably has a 5-membered ring or 6-membered ring sharing a bond with a fused bicyclic structure at the center of general formula (1) constituted by Y¹, X¹, and X².

Here, the "fused bicyclic structure" means a structure in which two saturated hydrocarbon rings including Y¹, X¹, and X² and indicated at the center of general formula (1) are fused. Furthermore, the "6-membered ring sharing a bond with the fused bicyclic structure" means, for example, ring a (benzene ring (6-membered ring)) fused to the fused bicyclic structure as represented by the above general formula (2). Furthermore, the phrase "aryl ring or heteroaryl ring (which is ring A) has this 6-membered ring" means that the ring A is formed only from this 6-membered ring, or other rings and the like are further fused to this 6-membered ring so as to include this 6-membered ring to form the ring A. In other words, the "aryl ring or heteroaryl ring (which is ring A) having a 6-membered ring" as used herein means that the 6-membered ring constituting the entirety or a part of the ring A is fused to the fused bicyclic structure. Similar description applies to the "ring B (ring b)", "ring C (ring c)", and the "5-membered ring".

The ring A (or ring B or ring C) in general formula (1) corresponds to ring a and its substituents R¹ to R³ in general formula (2) (or ring b and its substituents R⁸ to R¹¹, or ring c and its substituents R⁴ to R⁷). That is, general formula (2) corresponds to a structure in which "rings A to C having 6-membered rings" have been selected as the rings A to C of general formula (1). For this meaning, the rings of general formula (2) are represented by small letters a to c.

In general formula (2), adjacent groups among the substituents R¹ to R¹¹ of the ring a, ring b, and ring c may be bonded to each other to form an aryl ring or a heteroaryl ring together with the ring a, ring b, or ring c, and at least one hydrogen atom in the ring thus formed may be substituted by an aryl, a heteroaryl, a diarylamino, a diheteroarylamino, an arylheteroarylamino, a diarylboryl (the two aryls may be bonded via a single bond or a linking group), an alkyl, a cycloalkyl, an alkoxy, an aryloxy, a triarylsilyl, a trialkylsilyl, a tricycloalkylsilyl, a dialkylcycloalkylsilyl, or an alkyldicycloalkylsilyl, while at least one hydrogen atom in these may be substituted by an aryl, a heteroaryl, an alkyl, or a cycloalkyl. Therefore, in a polycyclic aromatic compound represented by general formula (2), a ring structure constituting the compound changes as represented by the following formulas (2-1) and (2-2) according to a mutual bonding form of substituents in the ring a, ring b, and ring c. Ring A', ring B', and ring C' in the formulas correspond to the ring A, ring B, and ring C in general formula (1), respectively.

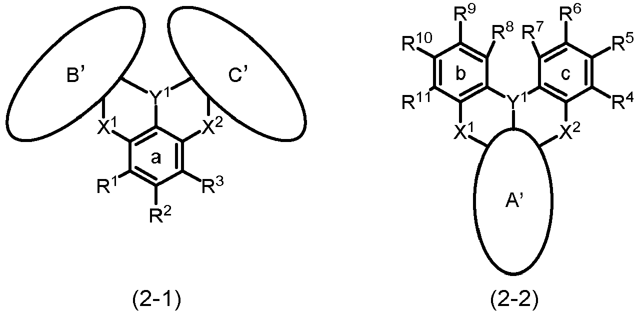

The ring A', ring B' and, ring C' in the above formulas (2-1) and (2-2) each represent, to be described in connection with general formula (2), an aryl ring or a heteroaryl ring formed by bonding adjacent groups among the substituents R¹ to R¹¹ together with the ring a, ring b, and ring c, respectively (may also be referred to as a fused ring obtained by fusing another ring structure to the ring a, ring b, or ring c). Incidentally, although not indicated in the formula, there is also a compound in which all of the ring a, ring b, and ring c have been changed to the ring A', ring B' and ring C'. Furthermore, as apparent from the above formulas (2-1) and (2-2), for example, R⁸ of the ring b and R⁷ of the ring c, R¹¹ of the ring b and R¹ of the ring a, R⁴ of the ring c and R³ of the ring a, and the like do not correspond to "adjacent groups", and these groups are not bonded to each other. That is, the term "adjacent groups" means adjacent groups on the same ring.

The compound represented by formula (2-1) or (2-2) is, for example, a compound having ring A' (or ring B' or ring C') that is formed by fusing a benzene ring, an indole ring, a pyrrole ring, a benzofuran ring, or a benzothiophene ring to a benzene ring which is ring a (or ring b or ring c), and the fused ring A' (or fused ring B' or fused ring C') that has been formed is a naphthalene ring, a carbazole ring, an indole ring, a dibenzofuran ring, or a dibenzothiophene ring.

Y¹ in general formula (1) represents B, P, P=O, P=S, Al, Ga, As, Si-R, or Ge-R, and R of the Si-R and Ge-R represents an aryl, an alkyl or a cycloalkyl. In a case Y¹ represents P=O, P=S, Si-R, or Ge-R, an atom bonded to the ring A, ring B, or ring C is P, Si, or Ge. Y¹ is preferably B, P, P=O, P=S, or Si-R, and B is particularly preferable. This description also applies to Y¹ in general formula (2).

X¹ and X² in general formula (1) each independently represent >O, >N-R, >C(-R)₂, >S, or >Se, R in the >N-R represents an optionally substituted aryl, an optionally substituted heteroaryl, an optionally substituted alkyl, or an optionally substituted cycloalkyl, R in the >C(-R)₂ represents a hydrogen atom, an optionally substituted aryl, an optionally substituted alkyl, or an optionally substituted cycloalkyl, and at least one of R in the N-R and R in the >C(-R)₂ may be bonded to at least one of the ring A, ring B and ring C via a linking group or a single bond. The linking group is preferably -O-, -S-, or -C(-R)₂-. Note that R of the "-C(-R)₂-" represents a hydrogen atom, an alkyl or a cycloalkyl. This description also applies to X¹ and X² in general formula (2).

Here, the provision that "at least one of R in the >N-R and R in the >C(-R)₂ is bonded to at least one of the ring A, ring B, and ring C via a linking group or a single bond" for general formula (1) corresponds to the provision that "at least one of R in the >N-R and R in the >C(-R)₂ is bonded to at least one of the ring a, ring b, and ring c via -O-, - S-, -C(-R)₂- or a single bond" for general formula (2).

This provision can be expressed by a compound having a ring structure represented by the following formula (2-3-1), in which X¹ and X² are incorporated into the fused rings B' and C', respectively. That is, for example, the compound is a compound having ring B' (or ring C') formed by fusing another ring to a benzene ring which is ring b (or ring c) in general formula (2) so as to incorporate X¹ (or X²). The formed fused ring B' (or fused ring C') is, for example, a phenoxazine ring, a phenothiazine ring, or an acridine ring.

Furthermore, the above provision can be expressed by a compound having a ring structure in which at least one of X¹ and X² are/is incorporated into the fused ring A', represented by the following formula (2-3-2) or (2-3-3). That is, for example, the compound is a compound having ring A' formed by fusing another ring to a benzene ring which is ring a in general formula (2) so as to incorporate X¹ (or X², or X¹ and X²). The formed fused ring A' is, for example, a phenoxazine ring, a phenothiazine ring, or an acridine ring.

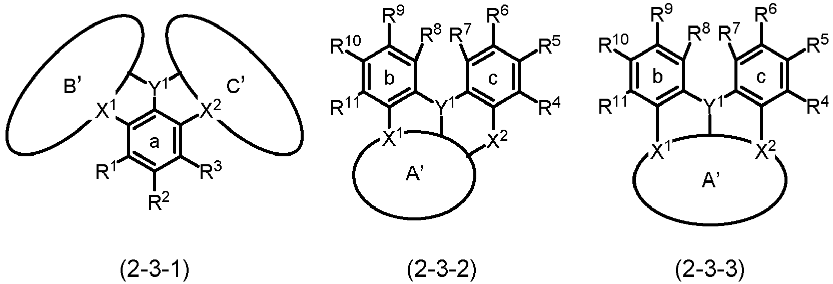

In formula (2), any "-C(-R)=" (in which R represents any one of R¹ to R¹¹ in formula (2)) in ring a, ring b, and ring c may be replaced with "-N=".

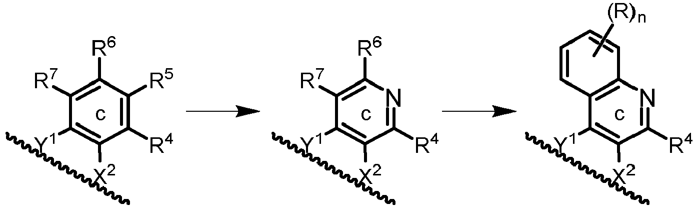

As described above, for example, "-C(-R⁵)=" in ring c may be replaced with "-N=". In this manner, ring c expressed as a benzene ring in formula (2) may be changed to a pyridine ring, a pyrimidine ring, a pyridazine ring, a pyrazine ring, or another nitrogen-containing heteroaryl ring. In a case where there are adjacent groups on ring c (R⁶ and R⁷ in the above formula), as described above, these groups may be bonded to each other to form a heteroaryl ring (a quinoline ring in the above formula) together with ring c, and the formed ring may be further substituted (indicated by nR's) .

In addition, there are the following modifications.

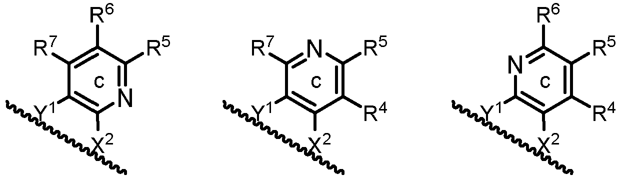

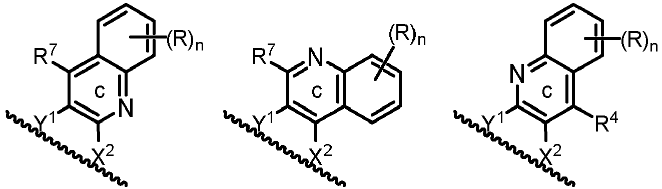

The same applies to a case where another portion is replaced with "-N=" or a case where ring a or ring b is changed.

In formula (2), any "-C(-R)=-C(-R)-" (in which R represents any one of R¹ to R¹¹ in formula (2)) in ring a, ring b, and ring c may be replaced with "-N(-R-)", "-O-", or "-S-", and R in the "-N(-R-)" represents an aryl, an alkyl, or a cycloalkyl. Note that details of the substituents listed here will be collectively described later.

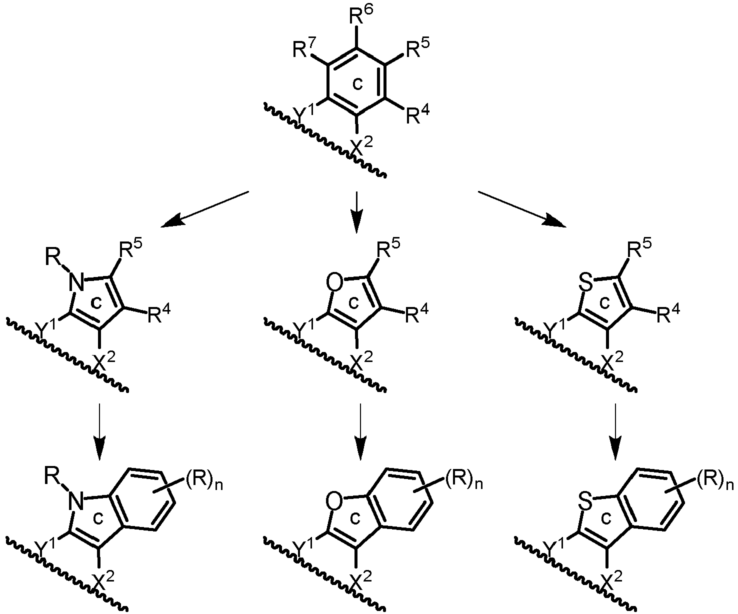

As described above, for example, "-C(-R⁷)=C(-R⁶)-" in ring c may be replaced with "-N(-R)-", "-O-", or "-S-". In this manner, ring c expressed as a benzene ring in formula (2) may be changed to an R-substituted pyrrole ring, an R-substituted furan ring, an R-substituted thiophene ring, or another nitrogen/oxygen/sulfur-containing heteroaryl ring. In a case where there are adjacent groups on ring c (R⁴ and R⁵ in the above formula), as described above, these groups may be bonded to each other to form a heteroaryl ring (an R-substituted indole ring, an R-substituted benzofuran ring, or an R-substituted benzothiophene ring in the above formula) together with ring c, and the formed ring may be further substituted (indicated by nR's) .

In addition, there are the following modifications.

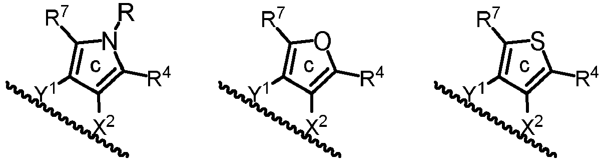

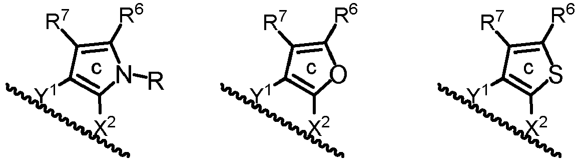

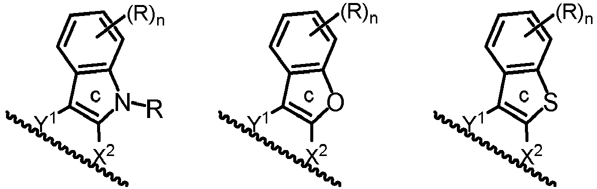

The same applies to a case where another portion is replaced with "-N(-R)-", "-O-", or "-S-" or a case where ring a or ring b is changed.

Note that in the description of the above formulas (2-1), (2-2), (2-3-1), (2-3-2), and (2-3-3), ring a, ring b, and ring c have been described as benzene rings. However, the same applies to a case where each of ring a to ring c is changed to a nitrogen-containing heteroaryl ring (six-membered ring or five-membered ring) or an oxygen/sulfur-containing heteroaryl ring (five-membered ring).

The "aryl ring" as the ring A, ring B, or ring C of general formula (1) is, for example, an aryl ring having 6 to 30 carbon atoms, and the aryl ring is preferably an aryl ring having 6 to 16 carbon atoms, more preferably an aryl ring having 6 to 12 carbon atoms, and particularly preferably an aryl ring having 6 to 10 carbon atoms. Incidentally, this "aryl ring" corresponds to the "aryl ring formed by bonding adjacent groups among R¹ to R¹¹ together with the ring a, ring b, or ring c" defined by general formula (2). Furthermore, ring a (or ring b or ring c) is already constituted by a benzene ring having 6 carbon atoms, and therefore the carbon number of 9 in total of a fused ring obtained by fusing a 5-membered ring to this benzene ring is a lower limit of the carbon number.

Specific examples of the "aryl ring" include a benzene ring which is a monocyclic system; a biphenyl ring which is a bicyclic system; a naphthalene ring which is a fused bicyclic system; a terphenyl ring (m-terphenyl, o-terphenyl, or p-terphenyl) which is a tricyclic system; an acenaphthylene ring, a fluorene ring, a phenalene ring, a phenanthrene ring, and an anthracene ring which are fused tricyclic systems; a triphenylene ring, a pyrene ring, and a naphthacene ring which are fused tetracyclic systems; and a perylene ring and a pentacene ring which are fused pentacyclic systems.

The "heteroaryl ring" as the ring A, ring B, or ring C of general formula (1) is, for example, a heteroaryl ring having 2 to 30 carbon atoms, and the heteroaryl ring is preferably a heteroaryl ring having 2 to 25 carbon atoms, more preferably a heteroaryl ring having 2 to 20 carbon atoms, still more preferably a heteroaryl ring having 2 to 15 carbon atoms, and particularly preferably a heteroaryl ring having 2 to 10 carbon atoms. Furthermore, examples of the "heteroaryl ring" include a heterocyclic ring containing 1 to 5 heteroatoms selected from an oxygen atom, a sulfur atom, and a nitrogen atom in addition to a carbon atom as a ring-constituting atom. Incidentally, this "heteroaryl ring" corresponds to the "heteroaryl ring formed by bonding adjacent groups among the R¹ to R¹¹ together with the ring a, ring b, or ring c" defined by general formula (2). Furthermore, the ring a (or ring b or ring c) is already constituted by a benzene ring having 6 carbon atoms, and therefore the carbon number of 6 in total of a fused ring obtained by fusing a 5-membered ring to this benzene ring is a lower limit of the carbon number.

Specific examples of the "heteroaryl ring" include a pyrrole ring, an oxazole ring, an isoxazole ring, a thiazole ring, an isothiazole ring, an imidazole ring, an oxadiazole ring, a thiadiazole ring, a triazole ring, a tetrazole ring, a pyrazole ring, a pyridine ring, a pyrimidine ring, a pyridazine ring, a pyrazine ring, a triazine ring, an indole ring, an isoindole ring, a 1H-indazole ring, a benzimidazole ring, a benzoxazole ring, a benzothiazole ring, a 1H-benzotriazole ring, a quinoline ring, an isoquinoline ring, a cinnoline ring, a quinazoline ring, a quinoxaline ring, a phenanthroline ring, a phthalazine ring, a naphthyridine ring, a purine ring, a pteridine ring, a carbazole ring, an acridine ring, a phenoxathiin ring, a phenoxazine ring, a phenothiazine ring, a phenazine ring, a phenazasiline ring, an indolizine ring, a furan ring, a benzofuran ring, an isobenzofuran ring, a dibenzofuran ring, a naphtobenzofuran ring, a thiophene ring, a benzothiophene ring, a isobenzothiophene ring, a dibenzothiophene ring, a naphtobenzothiophene ring, a benzophosphole ring, a dibenzophosphole ring, a benzophosphole oxide ring, a dibenzophosphole oxide ring, a furazane ring, a thianthrene ring, an indolocarbazole ring, a benzoindolocarbazole ring, and a benzobenzoindolocarbazole ring.

At least one hydrogen atom in the above "aryl ring" or "heteroaryl ring" may be substituted by a substituted or unsubstituted "aryl", a substituted or unsubstituted "heteroaryl", a substituted or unsubstituted "diarylamino", a substituted or unsubstituted "diheteroarylamino", a substituted or unsubstituted "arylheteroarylamino", a substituted or unsubstituted diarylboryl (the two aryls may be bonded via a single bond or a linking group), a substituted or unsubstituted "alkyl", a substituted or unsubstituted "cycloalkyl", a substituted or unsubstituted "alkoxy", a substituted or unsubstituted "aryloxy", or a substituted "silyl", which is a primary substituent. Examples of the "aryl", the "heteroaryl" and the aryl of the "diarylamino", the heteroaryl of the "diheteroarylamino", the aryl and the heteroaryl of the "arylheteroarylamino", the aryl of the "diarylboryl", and the aryl of the "aryloxy" as the primary substituents include a monovalent group of the "aryl ring" or "heteroaryl ring" described above.

Furthermore, the "alkyl" as the primary substituent may be either linear or branched, and examples thereof include a linear alkyl having 1 to 24 carbon atoms and a branched alkyl having 3 to 24 carbon atoms. An alkyl having 1 to 18 carbon atoms (branched alkyl having 3 to 18 carbon atoms) is preferable, an alkyl having 1 to 12 carbon atoms (branched alkyl having 3 to 12 carbon atoms) is more preferable, an alkyl having 1 to 6 carbon atoms (branched alkyl having 3 to 6 carbon atoms) is still more preferable, and an alkyl having 1 to 5 carbon atoms (branched alkyl having 3 to 5 carbon atoms) and an alkyl having 1 to 4 carbon atoms (branched alkyl having 3 to 4 carbon atoms) is particularly preferable.

Specific examples of the alkyl include methyl, ethyl, n-propyl, isopropyl, n-butyl, isobutyl, s-butyl, t-butyl, n-pentyl, isopentyl, neopentyl, t-pentyl (t-amyl), n-hexyl, 1-methylpentyl, 3,3-dimethylbutyl, 2-ethylbutyl, n-heptyl, 1-methylhexyl, n-octyl, t-octyl (1,1,3,3-tetramethylbutyl), 1-methylheptyl, 2-ethylhexyl, 2-propylpentyl, n-nonyl, 2,2-dimethylheptyl, 2,6-dimethyl-4-heptyl, 3,5,5-trimethylhexyl, n-decyl, n-undecyl, 1-methyldecyl, n-dodecyl, n-tridecyl, 1-hexylheptyl, n-tetradecyl, n-pentadecyl, n-hexadecyl, n-heptadecyl, n-octadecyl, and n-eicosyl.

Also, for example, 1-ethyl-1-methylpropyl, 1,1-diethylpropyl, 1,1-dimethylbutyl, 1-ethyl-1-methylbutyl, 1,1,4-trimethylpentyl, 1,1,2-trimethylpropyl, 1,1-dimethyloctyl, 1,1-dimethylpentyl, 1,1-dimethylheptyl, 1,1,5-trimethylhexyl, 1-ethyl-1-methylhexyl, 1-ethyl-1,3-dimethylbutyl, 1,1,2,2-tetramethylpropyl, 1-butyl-1-methylpentyl, 1,1-diethylbutyl, 1-ethyl-1-methylpentyl, 1,1,3-trimethylbutyl, 1-propyl-1-methylpentyl, 1,1,2-trimethylpropyl, 1-ethyl-1,2,2-trimethylpropyl, 1-propyl-1-methylbutyl, 1,1-dimethylhexyl and the like can also be mentioned.

Examples of the "cycloalkyl" as the primary substituent include a cycloalkyl having 3 to 24 carbon atoms, a cycloalkyl having 3 to 20 carbon atoms, a cycloalkyl having 3 to 16 carbon atoms, a cycloalkyl having 3 to 14 carbon atoms, a cycloalkyl having 3 to 12 carbon atoms, a cycloalkyl having 5 to 10 carbon atoms, a cycloalkyl having 5 to 8 carbon atoms, a cycloalkyl having 5 or 6 carbon atoms, and a cycloalkyl having 5 carbon atoms.

Specific examples of the cycloalkyl include cyclopropyl, cyclobutyl, cyclopentyl, cyclohexyl, cycloheptyl, cyclooctyl, cyclononyl, cyclodecyl, and these substitutes with alkyl having 1 to 4 carbon atoms or 1 to 5 carbon atoms (particularly methyl group), norbornenyl, bicyclo[1.0.1]butyl, bicyclo[1.1.1]pentyl, bicyclo[2.0.1]pentyl, bicyclo[1.2.1]hexyl, bicyclo[3.0.1]hexyl, bicyclo[2.1.2]heptyl, bicyclo[2.2.2]octyl, adamantyl, diamantyl, decahydronaphthalenyl, and decahydroazulenyl.

Furthermore, examples of the "alkoxy" as the primary substituent include a linear alkoxy having 1 to 24 carbon atoms and a branched alkoxy having 3 to 24 carbon atoms. An alkoxy having 1 to 18 carbon atoms (branched alkoxy having 3 to 18 carbon atoms) is preferable, an alkoxy having 1 to 12 carbon atoms (branched alkoxy having 3 to 12 carbon atoms) is more preferable, an alkoxy having 1 to 6 carbon atoms (branched alkoxy having 3 to 6 carbon atoms) is still more preferable, and an alkoxy having 1 to 5 carbon atoms (branched alkoxy having 3 or 5 carbon atoms) and an alkoxy having 1 to 4 carbon atoms (branched alkoxy having 3 or 4 carbon atoms) is particularly preferable.

Specific examples of the alkoxy include methoxy, ethoxy, propoxy, isopropoxy, n-butoxy, isobutoxy, s-butoxy, t-butoxy, t-amyloxy, n-pentyloxy, isopentyloxy, neopentyloxy, t-pentyloxy, n-hexyloxy, 1-methylpentyloxy, 3,3-dimethylbutoxy, 2-ethylbutoxy, n-heptyloxy, 1-methylhexyloxy, n-octyloxy, t-octyloxy, 1-methylheptyloxy, 2-ethylhexyloxy, 2-propylpentyloxy, n-nonyloxy, 2,2-dimethylheptyloxy, 2,6-dimethyl-4-heptyloxy, 3,5,5-trimethylhexyloxy, n-decyloxy, n-undecyloxy, 1-methyldecyloxy, n-dodecyloxy, n-tridecyloxy, 1-hexylheptyloxy, n-tetradecyloxy, n-pentadecyloxy, n-hexadecyloxy, n-heptadecyloxy, n-octadecyloxy, and n-eicosyloxy.

Furthermore, examples of the "substituted silyl" as the primary substituent include triarylsilyl, trialkylsilyl, tricycloalkylsilyl, dialkylcycloalkylsilyl, or alkyldicycloalkylsilyl, which is a silyl substituted with at least one of aryl, alkyl and cycloalkyl.

Examples of the "triarylsilyl" include silyl groups in which three hydrogen atoms in the silyl group are each independently substituted with an aryl, and this aryl refers to the group explained as "aryl" in the primary substituent described above. Specific "triarylsilyl" is, for example, triphenylsilyl, diphenylmononaphthylsilyl, monophenyldinaphthylsilyl, trinaphthylsilyl or the like.

Examples of the "trialkylsilyl" include silyl groups in which three hydrogen atoms in the silyl group are each independently substituted with an alkyl, and this alkyl refers to the group explained as "alkyl" in the primary substituent described above. Preferred alkyl for substitution is an alkyl having 1 to 5 carbon atoms or 1 to 4 carbon atoms, specifically methyl, ethyl, propyl, i-propyl, butyl, sec-butyl, t-butyl, cyclobutyl or the like.

Specific trialkylsilyls include trimethylsilyl, triethylsilyl, tripropylsilyl, tri i-propylsilyl, tributylsilyl, tri secbutylsilyl, tri t-butylsilyl, ethyldimethylsilyl, propyldimethylsilyl, i-propyldimethylsilyl, butyldimethylsilyl, secbutyldimethylsilyl, t-butyldimethylsilyl, methyldiethylsilyl, propyldiethylsilyl, i-propyldiethylsilyl, butyldiethylsilyl, secbutyldiethylsilyl, t-butyldiethylsilyl, methyldipropylsilyl, ethyldipropylsilyl, butyldipropylsilyl, sec-butyldipropylsilyl, t-butyldipropylsilyl, methyl di i-propylsilyl, ethyl di i-propylsilyl, butyl di i-propylsilyl, sec-butyl di i-propylsilyl, t-butyl di i-propylsilyl.

Examples of the "tricycloalkylsilyl" include silyl groups in which three hydrogen atoms in the silyl group are each independently substituted with cycloalkyl, and this cycloalkyl refers to the group explained as "cycloalkyl" in the primary substituent described above. Preferred cycloalkyl for substitution is a cycloalkyl having 5 to 10 carbon atoms, specifically cyclopentyl, cyclohexyl, cycloheptyl, cyclooctyl, cyclononyl, cyclodecyl, bicyclo[1.1.1]pentyl, bicyclo[2.0.1]pentyl, bicyclo[1.2.1]hexyl, Bicyclo[3.0.1]hexyl, bicyclo[2.1.2]heptyl, bicyclo[2.2.2]octyl, adamanthyl, decahydronaphthalenyl, and decahydroazulenyl.

Specific examples of tricycloalkylsilyl include tricyclopentylsilyl, tricyclohexylsilyl, and the like.

Specific examples of the dialkylcycloalkylsilyl in which two alkyls and one cycloalkyl are substituted and the alkyldicycloalkylsilyl in which one alkyl and two cycloalkyls are substituted include silyls substituted with groups selected from the specific alkyls and cycloalkyls described above.

As the "aryl" in the "diarylboryl" as a primary substituent, the above description on the aryl can be cited. The two aryls may be bonded to each other via a single bond or a linking group (for example, >C(-R)₂, >O, >S, or >N-R). Here, R in each of the moiety >C(-R)₂ and the moiety >N-R is a hydrogen atom (only in case >C(-R)₂), an aryl, a heteroaryl, a diarylamino, an alkyl, a cycloalkyl, an alkoxy, or an aryloxy (these are primary substituents). The primary substituent may be further substituted by an aryl, a heteroaryl, an alkyl, or a cycloalkyl (these are secondary substituents). As specific examples of these groups, the above description on the aryl, the heteroaryl, the diarylamino, the alkyl, the cycloalkyl, the alkoxy, or the aryloxy as a primary substituent can be cited.

At least one hydrogen atom in the substituted or unsubstituted "aryl", the substituted or unsubstituted "heteroaryl", the substituted or unsubstituted "diarylamino", the substituted or unsubstituted "diheteroarylamino", the substituted or unsubstituted "arylheteroarylamino", the substituted or unsubstituted "diarylboryl (the two aryls may be bonded to each other via a single bond or a linking group)", the substituted or unsubstituted "alkyl", the substituted or unsubstituted "cycloalkyl", the substituted or unsubstituted "alkoxy", the substituted or unsubstituted "aryloxy", or the substituted "silyl" as a primary substituent may be substituted by a secondary substituent as described to be "substituted or unsubstituted". Examples of this secondary substituent include an aryl, a heteroaryl, an alkyl, and a cycloalkyl. As specific examples thereof, the above description on the monovalent group of the "aryl ring" or "heteroaryl ring" and the "alkyl" or the "cycloalkyl" as a primary substituent can be referred to. The aryl or the heteroaryl as a secondary substituent includes a structure in which at least one hydrogen atom in the aryl or the heteroaryl is substituted by an aryl such as phenyl (specific examples are the groups described above), an alkyl such as methyl (specific examples are the groups described above), or a cycloalkyl such as cyclohexyl (specific examples are the groups described above). For example, when the secondary substituent is a carbazolyl group, a carbazolyl group in which at least one hydrogen atom at the 9-position is substituted by an aryl such as phenyl, an alkyl such as methyl, or a cycloalkyl such as cyclohexyl is also included in the heteroaryl as the secondary substituent.

Examples of the aryl, the heteroaryl, the aryl of the diarylamino, the heteroaryl of the diheteroarylamino, the aryl and the heteroaryl of the arylheteroarylamino, the aryl of the diarylboryl, or the aryl of the aryloxy in R¹ to R¹¹ in general formula (2) include the monovalent groups of the "aryl ring" or the "heteroaryl ring" described in general formula (1). As the alkyl, the cycloalkyl, or the alkoxy in R¹ to R¹¹, the description on the "alkyl", the "cycloalkyl", or the "alkoxy" as a primary substituent in the above description of general formula (1) can be referred to. As the triarylsilyl, the trialkylsilyl, the tricycloalkylsilyl, the dialkylcycloalkylsilyl, or the alkyldicycloalkylsilyl in R¹ to R¹¹, the description on the "substituted silyl" as a primary substituent in the above description of general formula (1) can be referred to. The same also applies to an aryl, a heteroaryl, an alkyl, or a cycloalkyl as a substituent on these groups. The same also applies to a heteroaryl, a diarylamino, a diheteroarylamino, an arylheteroarylamino, a diarylboryl (the two aryls may be bonded to each other via a single bond or a linking group), an alkyl, a cycloalkyl, an alkoxy, an aryloxy, a triarylsilyl, a trialkylsilyl, a tricycloalkylsilyl, a dialkylcycloalkylsilyl, or a alkyldicycloalkylsilyl as a substituent on these groups in a case of forming an aryl ring or a heteroaryl ring by bonding adjacent groups among R¹ to R¹¹ together with ring a, ring b, or ring c, and an aryl, a heteroaryl, an alkyl, or a cycloalkyl as a further substituent.

Specifically, the group can adjust an emission wavelength by steric hindrance, an electron donating property, and an electron withdrawing property of the structure of a primary substituent, and is preferably a group represented by any one of the following structural formulas, more preferably methyl, t-butyl, t-amyl, t-octyl, phenyl, o-tolyl, p-tolyl, 2,4-xylyl, 2,5-xylyl, 2,6-xylyl, 2,4,6-mesityl, diphenylamino, di-p-tolylamino, bis(p-(t-butyl) phenyl) amino, carbazolyl, 3,6-dimethylcarbazolyl, 3,6-di-t-butylcarbazolyl, or phenoxy, and more preferably methyl, t-butyl, t-amyl, t-octyl, phenyl, o-tolyl, 2,6-xylyl, 2,4,6-mesityl, diphenylamino, di-p-tolylamino, bis(p-(t-butyl) phenyl) amino, carbazolyl, 3,6-dimethylcarbazolyl, or 3,6-di-t-butylcarbazolyl. Larger steric hindrance is more preferable for selective synthesis from a viewpoint of easiness of synthesis. Specifically, t-butyl, t-amyl, t-octyl, o-tolyl, p-tolyl, 2,4-xylyl, 2,5-xylyl, 2,6-xylyl, 2,4,6-mesityl, di-p-tolylamino, bis(p-(t-butyl) phenyl) amino, 3,6-dimethylcarbazolyl, and 3,6-di-t-butylcarbazolyl are preferable.

In the following structural formulae, "Me" represents methyl, "tBu" represents t-butyl, "tAm" represents t-amyl, "t-octyl" represents t-octyl, and the symbol "*" indicates a bonding position.

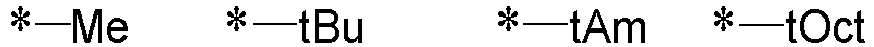

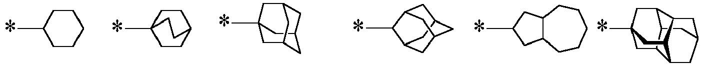

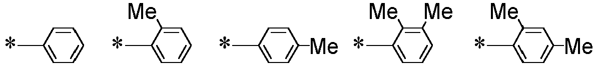

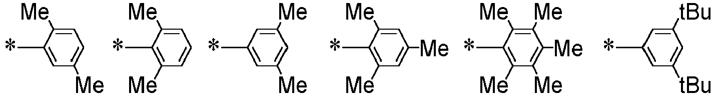

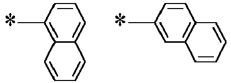

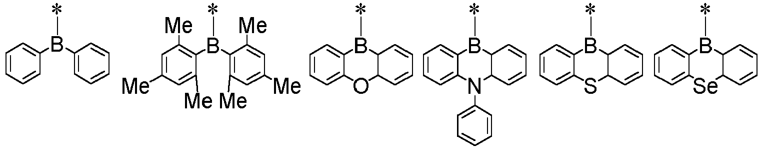

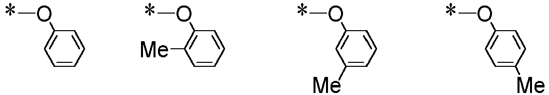

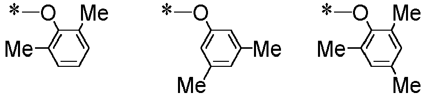

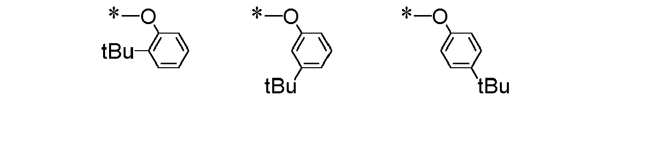

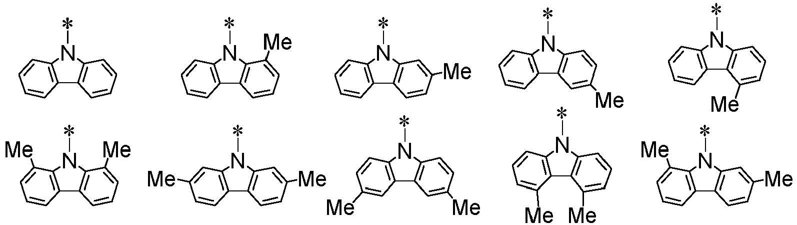

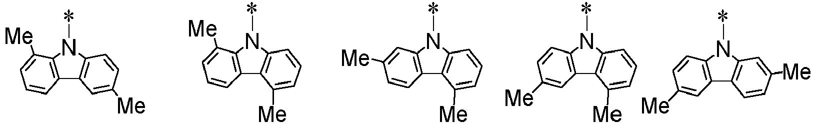

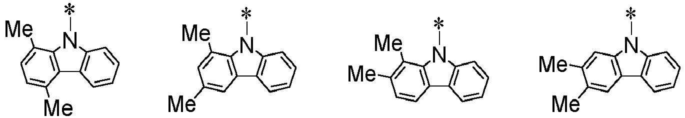

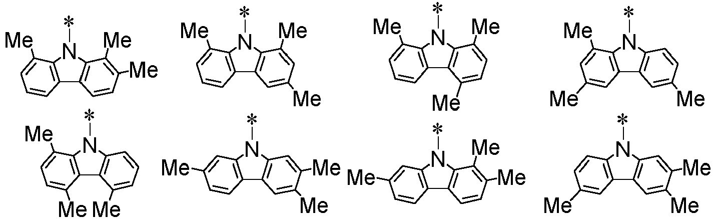

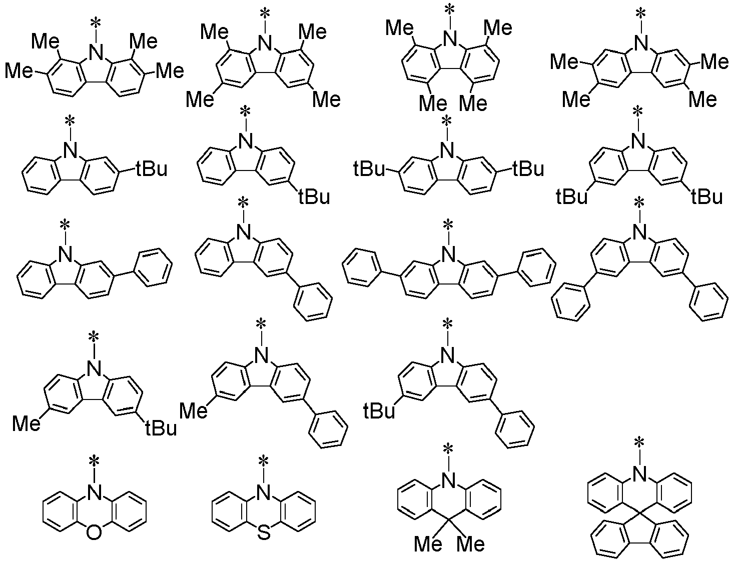

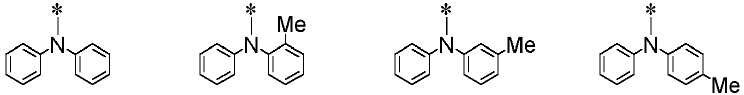

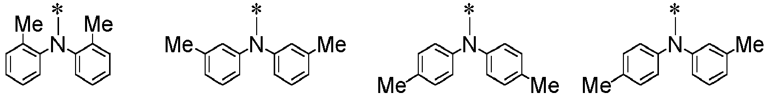

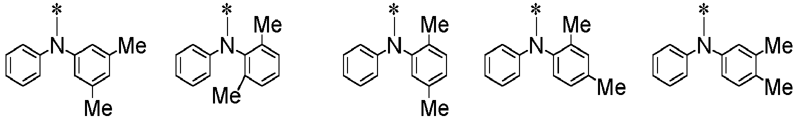

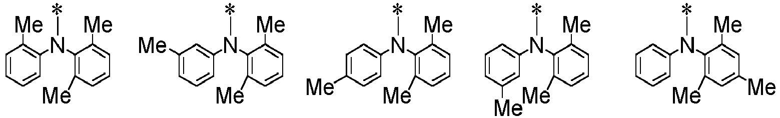

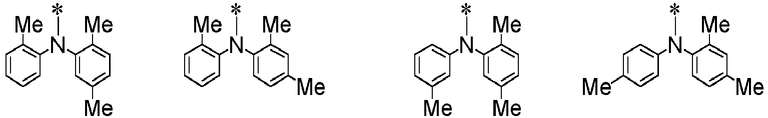

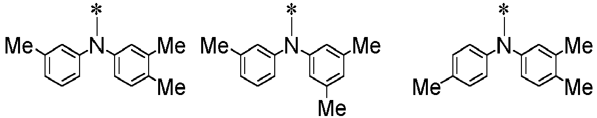

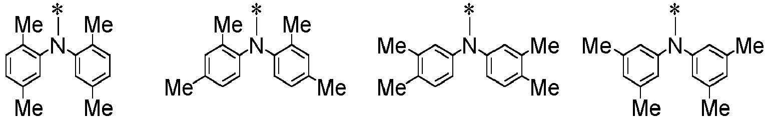

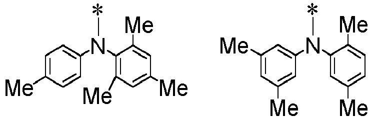

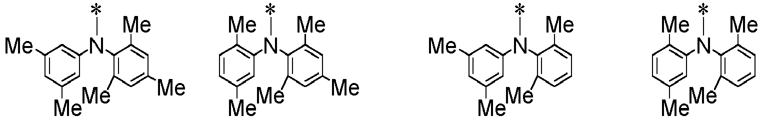

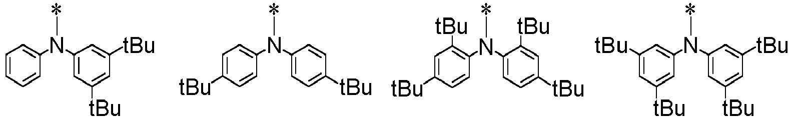

In the general formula (2), R of "-N(-R)-" to which any "-C(-R)=C(-R)-" (where R is R¹ to R¹¹ in the formula (2)) can be replaced is an aryl, an alkyl, or a cycloalkyl, examples of the aryl, alkyl or cycloalkyl include the groups described above. In particular, an aryl having 6 to 10 carbon atoms (for example, phenyl and naphthyl), alkyls having 1 to 5 carbon atoms or having 1 to 4 carbon atoms (for example, methyl and ethyl), or a cycloalkyl having 5 to 10 carbon atoms (preferably cyclohexyl or adamanthyl) is preferred.

R in the Si-R and Ge-R in Y¹ of general formula (1) represents an aryl, an alkyl or a cycloalkyl, and examples of this aryl, alkyl or a cycloalkyl include those described above. Particularly, an aryl having 6 to 10 carbon atoms (for example, phenyl or naphthyl) and an alkyl having 1 to 5 carbon atoms or having 1 to 4 carbon atoms (for example, methyl or ethyl) or a cycloalkyl having 5 to 10 carbon atoms (preferably, cyclohexyl or adamanthyl) are preferable. This description also applies to Y¹ in general formula (2).

R in the >N-R in X¹ and X² of general formula (1) represents an aryl, a heteroaryl, an alkyl, or a cycloalkyl which may be substituted by the secondary substituent described above, and at least one hydrogen atom in the aryl, heteroaryl, alkyl, or cycloalkyl may be substituted by, for example, an alkyl or a cycloalkyl. Examples of this aryl, heteroaryl, alkyl, or cycloalkyl include those described above. Particularly, an aryl having 6 to 10 carbon atoms (for example, phenyl or naphthyl), a heteroaryl having 2 to 15 carbon atoms (for example, carbazolyl, dibenzofuranyl, dibenzothiophenyl), an alkyl having 1 to 5 carbon atoms or having 1 to 4 carbon atoms (for example, methyl or ethyl), or a cycloalkyl having 5 to 10 carbon atoms (preferably, cyclohexyl or adamanthyl) are preferable. Phenyl, naphthyl, carbazolyl, dibenzofuranyl or dibenzothiophenyl are more preferable, dibenzofuranyl or dibenzothiophenyl is further preferable. Further, it is particularly preferable that the dibenzofuranyl or dibenzothiophenyl is substituted with a second substituent. Specific examples of the second substituent include t-butyl, phenyl, d5-phenyl, and the like. These substituents are preferably substituted at the para position of the oxygen atom or the sulfur atom. This description also applies to X¹ and X² in general formula (2).

R in the >C(-R)₂ in X¹ and X² of general formula (1) represents a hydrogen atom, an aryl, an alkyl, or a cycloalkyl which may be substituted by the secondary substituent described above, and at least one hydrogen atom in the aryl may be substituted by, for example, an alkyl. Examples of this aryl, alkyl, or cycloalkyl include those described above. Particularly, an aryl having 6 to 10 carbon atoms (for example, phenyl or naphthyl), an alkyl having 1 to 5 carbon atoms or having 1 to 4 carbon atoms (for example, methyl or ethyl), or a cycloalkyl having 5 to 10 carbon atoms (preferably, cyclohexyl or adamanthyl) are preferable. This description also applies to X¹ and X² in general formula (2).

R of the "-C(-R)₂-" as a linking group for general formula (1) represents a hydrogen atom, an alkyl or a cycloalkyl, and examples of this alkyl or cycloalkyl include those described above. Particularly, an alkyl having 1 to 5 carbon atoms or having 1 to 4 carbon atoms (for example, methyl or ethyl) or a cycloalkyl having 5 to 10 carbon atoms (preferably, cyclohexyl or adamanthyl) is preferable. This description also applies to "-C(-R)₂-" as a linking group for general formula (2).

Furthermore, the invention of the present application is a multimer of a polycyclic aromatic compound having a plurality of unit structures each represented by general formula (1), and preferably a multimer of a polycyclic aromatic compound having a plurality of unit structures each represented by general formula (2). The multimer is preferably a dimer to a hexamer, more preferably a dimer to a trimer, and a particularly preferably a dimer. The multimer only needs to have the plurality of unit structures described above in one compound. For example, the multimer may have a form (a linking type multimer) in which the plurality of unit structures is bonded to one another with a linking group such as a single bond, an alkylene group having 1 to 3 carbon atoms, a phenylene group, or a naphthylene group. In addition, the multimer may have a form (a ring sharing type multimer) in which a plurality of unit structures is bonded such that any ring contained in the unit structure (ring A, ring B, or ring C, or ring a, ring b, or ring c) is shared by the plurality of unit structures, or may have a form (a ring fusing type multimer) in which the unit structures are bonded to one another such that any rings contained in the unit structures (ring A, ring B, or ring C, or ring a, ring b, or ring c) are fused to each other. The ring sharing type multimer and ring fusing type multimer are preferred, the ring sharing type multimer is more preferred.

Examples of such a multimer include multimer compounds represented by the following formulas (2-4), (2-4-1), (2-4-2), (2-5-1) to (2-5-4), and (2-6). To be described in connection with general formula (2), the multimer compound represented by the following formula (2-4) includes a plurality of unit structures each represented by general formula (2) in one compound so as to share a benzene ring as ring a (a ring sharing type multimer). Furthermore, to be described in connection with general formula (2), the multimer compound represented by the following formula (2-4-1) includes two unit structures each represented by general formula (2) in one compound so as to share a benzene ring as ring a (a ring sharing type multimer).
Furthermore, to be described in connection with general formula (2), the multimer compound represented by the following formula (2-4-2) includes three unit structures each represented by general formula (2) in one compound so as to share a benzene ring as ring a (a ring sharing type multimer).
Furthermore, to be described in connection with general formula (2), each of the multimer compounds represented by any one of the following formulas (2-5-1) to (2-5-4) includes a plurality of unit structures each represented by general formula (2) in one compound so as to share a benzene ring as ring b (or ring c) (a ring sharing type multimer). Furthermore, to be described in connection with general formula (2), for example, the multimer compound represented by the following formula (2-6) includes a plurality of unit structures each represented by general formula (2) in one compound such that a benzene ring as ring b (or ring a or ring c) of a certain unit structure and a benzene ring as ring b (or ring a or ring c) of a certain unit structure are fused (a ring fusing type multimer).

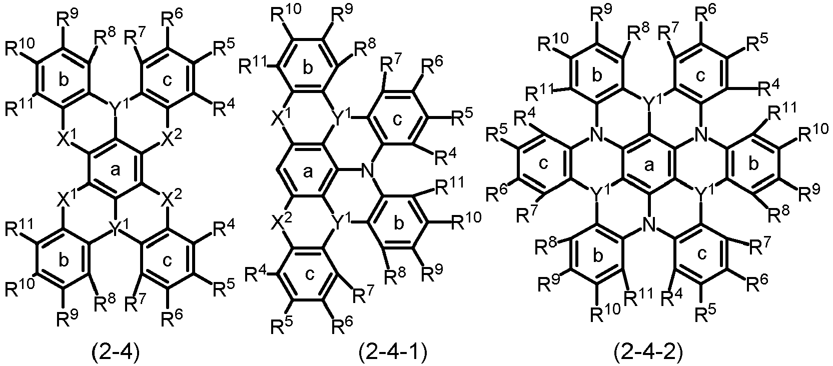

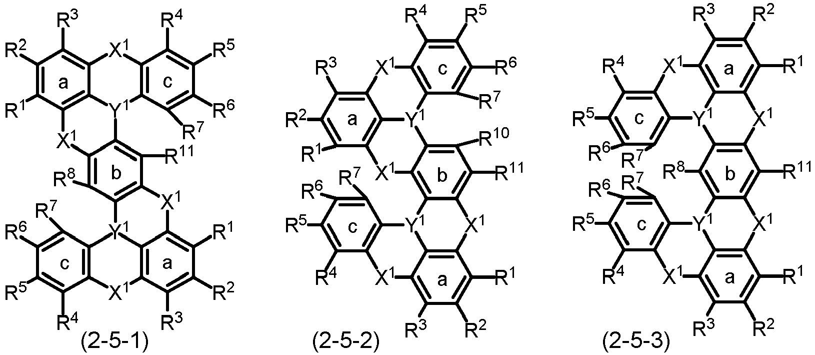

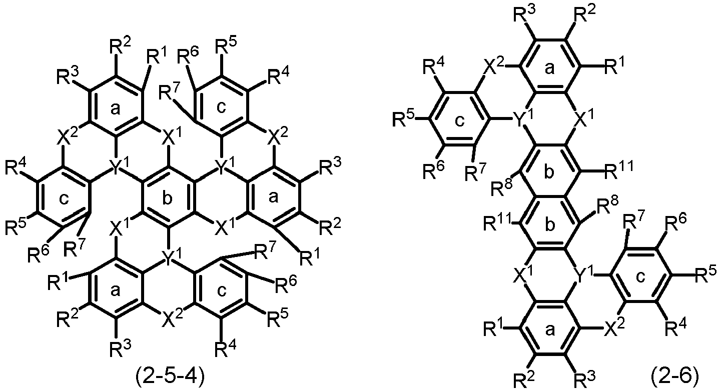

The multimer compound may be a multimer in which a multimer form represented by formula (2-4), (2-4-1), or (2-4-2) and a multimer form represented by any one of formula (2-5-1) to (2-5-4) or (2-6) are combined, may be a multimer in which a multimer form represented by any one of formulas (2-5-1) to (2-5-4) and a multimer form represented by formula (2-6) are combined, or may be a multimer in which a multimer form represented by formula (2-4), (2-4-1), or (2-4-2), a multimer form represented by any one of formulas (2-5-1) to (2-5-4), and a multimer form represented by formula (2-6) are combined.

Furthermore, all or a part of the hydrogen atoms in the chemical structures of the polycyclic aromatic compound represented by general formula (1) or (2) and a multimer thereof may be deuterium atoms, cyanos, or halogen atoms. For example, in formula (1), a hydrogen atom in the ring A, ring B, ring C (ring A to ring C are aryl rings or heteroaryl rings), substituents on the ring A to ring C, R (= an alkyl, a cycloalkyl or an aryl) when Y¹ represents Si-R or Ge-R, and R (= an alkyl, a cycloalkyl, or an aryl) when X¹ and X² each represent >N-R or >C(-R)₂ can be substituted by a deuterium atom, cyano, or a halogen atom. Among these, a form in which all or a part of the hydrogen atoms in the aryl or heteroaryl have been substituted by deuterium atoms, cyanos, or halogen atoms may be mentioned.
The halogen is fluorine, chlorine, bromine, or iodine, preferably fluorine, chlorine, or bromine, and more preferably fluorine or chlorine.

Furthermore, the polycyclic aromatic compound and the multimer according to an aspect of the present invention can be used as a material for an organic device. Examples of the organic device include an organic electroluminescent element, an organic field effect transistor, and an organic thin film solar cell. Particularly, in the organic electroluminescent element, a compound in which Y¹ represents B, and X¹ and X² each represent >N-R, a compound in which Y¹ represents B, X¹ represents >O, and X² represents >N-R, and a compound in which Y¹ represents B, and X¹ and X² each represent >O are preferably used as a dopant material for a light emitting layer. A compound in which Y¹ represents B, X¹ represents >O, and X² represents >N-R, and a compound in which Y¹ represents B, and X¹ and X² each represent >O are preferably used as a host material for a light emitting layer. A compound in which Y¹ represents B, and X¹ and X² each represent >O, and a compound in which Y¹ represents P=O, and X¹ and X² each represent >O are preferably used as an electron transport material.

At least one of aromatic rings and aromatic heterocyclic rings in chemical structures of a polycyclic aromatic compound represented by general formula (1) or (2) and a multimer thereof is fused with at least one cycloalkane.

For example, at least one of aryl rings and heteroaryl rings which are ring A, ring B, ring C, ring a, ring b, and ring c, an aryl group (an aryl group portion in an aryl, a diarylamino, an arylheteroarylamino, a diarylboryl, or an aryloxy) and a heteroaryl group (a heteroaryl portion in a heteroaryl, a diheteroarylamino, or an arylheteroarylamino) as first and second substituents on rings A to C, an aryl group (same as above) and a heteroaryl group (same as above) as first and second substituents on rings a to c, an aryl group (same as above) as R of Si-R and Ge-R which are Y¹, and an aryl group (same as above) and a heteroaryl group (same as above) as R of >N-R and >C(-R)₂ which are X¹ and X² is fused with at least one cycloalkane.

Preferably, at least one of aryl rings and heteroaryl rings which are ring A, ring B, ring C, ring a, ring b, and ring c, an aryl group (an aryl group portion in an aryl, a diarylamino, a diarylboryl, or an aryloxy) and a heteroaryl group (a heteroaryl portion in a heteroaryl or a diheteroarylamino) as first substituents on rings A to C, an aryl group (same as above) and a heteroaryl group (same as above) as first substituents on rings a to c, and an aryl group (same as above) and a heteroaryl group (same as above) as R of >N-R and >C(-R)₂ which are X¹ and X² is fused with at least one cycloalkane.

More preferably, at least one of aryl rings which are ring A, ring B, ring C, ring a, ring b, and ring c, an aryl group (an aryl group portion in an aryl or a diarylamino) and a heteroaryl group (a heteroaryl portion in a heteroaryl) as first substituents on rings A to C, an aryl group (same as above) and a heteroaryl group (same as above) as first substituents on rings a to c, and an aryl group (same as above) as R of >N-R and >C(-R)₂ which are X¹ and X² is fused with at least one cycloalkane.

Still more preferably, at least one of aryl rings which are ring A, ring B, ring C, ring a, ring b, and ring c, an aryl group (an aryl group portion in an aryl or a diarylamino) as first substituents on rings A to C, an aryl group (same as above) as first substituents on rings a to c, and an aryl group (same as above) as R of >N-R and >C(-R)₂ which are X¹ and X² is fused with at least one cycloalkane.

Examples of the "cycloalkane" include a cycloalkane having 3 to 24 carbon atoms, a cycloalkane having 3 to 20 carbon atoms, a cycloalkane having 3 to 16 carbon atoms, a cycloalkane having 3 to 14 carbon atoms, a cycloalkane having 5 to 10 carbon atoms, a cycloalkane having 5 to 8 carbon atoms, a cycloalkane having 5 or 6 carbon atoms, a cycloalkane having 5 carbon atoms.

Specific examples of the cycloalkane include cyclopropane, cyclobutane, cyclopentane, cyclohexane, cycloheptane, cyclooctane, cyclononane, cyclodecane, norbornene, bicyclo[1.0.1]butane, bicyclo[1.1.1]pentane, bicyclo[2.0.1]pentane, bicyclo[1.2.1]hexane, bicyclo[3.0.1]hexane, bicyclo[2.1.2]heptane, bicyclo[2.2.2]octane, adamantane, diamantane, decahydronaphthalene, and decahydroazulene, and C₁₋₅ or C₁₋₄ alkyl (particularly methyl)-substitutes thereof, halogen (particularly fluorine)-substitutes thereof, and deuterium-substitutes thereof.

At least one hydrogen atom at an α-position carbon atom of a cycloalkane (a carbon atom adjacent to a carbon atom at a fused portion in a cycloalkane fused with an aromatic ring or an aromatic heterocyclic ring) as indicated by the following structural formula is substituted. A structure is more preferable in which two hydrogen atoms at an α-position carbon atom are substituted. A structure is still more preferable in which four hydrogen atoms in total at two α-position carbon atoms are substituted. Examples of the substituent include a C₁₋₅ or C₁₋₄ alkyl (particularly methyl)-substitute, a halogen (particularly fluorine)-substitute, and a deuterium-substitute.

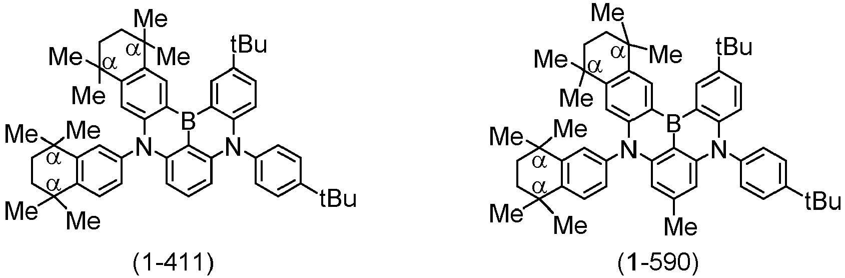

The number of cycloalkanes fused with one aromatic ring or one aromatic heterocyclic ring is preferably one to three, more preferably one or two, and still more preferably one. Examples in which one or more cycloalkanes are fused with one benzene ring (phenyl group) are illustrated below. The symbol * in each structural formula means a benzene ring included in a skeleton structure of a compound in a case of a benzene ring, and means a bond used for substitution of a Skelton structure of a compound in a case of a phenyl group. As illustrated by formulas (Cy-1-4) and (Cy-2-4), fused cycloalkanes may be fused with each other. The same applies to a case where a ring (group) to be fused is an aromatic ring or an aromatic heterocyclic ring other than a benzene ring (phenyl group), and a case where a cycloalkane to be fused is a cycloalkane other than cyclopentane or cyclohexane.

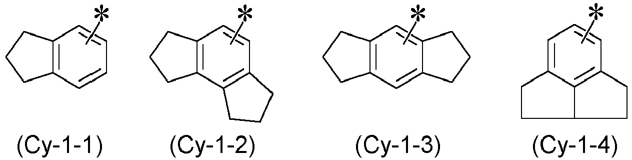

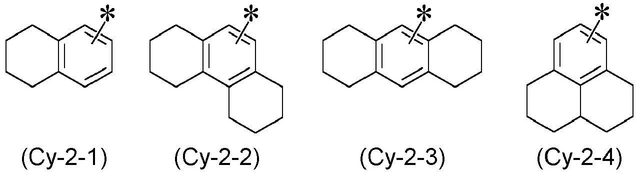

At least one -CH₂- in a cycloalkane may be substituted by -O-. However, when a plurality of -CH₂-s is substituted by -O-s, adjacent - CH₂-s are not substituted by -O-s. Examples in which one or more -CH₂-s are substituted by -O-s in a cycloalkane fused with one benzene ring (phenyl group) are illustrated below. The symbol * in each structural formula means a benzene ring included in a skeleton structure of a compound in a case of a benzene ring, and means a bond used for substitution of a Skelton structure of a compound in a case of a phenyl group. The same applies to a case where a ring (group) to be fused is an aromatic ring or an aromatic heterocyclic ring other than a benzene ring (phenyl group), and a case where a cycloalkane to be fused is a cycloalkane other than cyclopentane or cyclohexane.

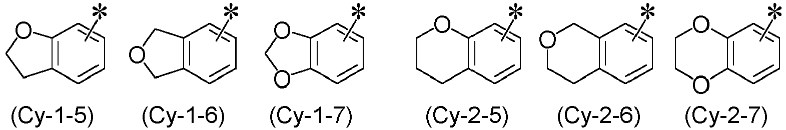

At least one hydrogen atom in a cycloalkane may be substituted provided that at least one hydrogen atom in a cycloalkane fused with at least one of the ring A, ring B, ring C, aryl and heteroaryl in the compound or structure represented by the formula (1) is substituted at an α-position carbon atom of the cycloalkane. Examples of the substituent include an aryl, a heteroaryl, a diarylamino, a diheteroarylamino, an arylheteroarylamino, a diarylboryl (the two aryls may be bonded to each other via a single bond or a linking group), an alkyl, a cycloalkyl, an alkoxy, an aryloxy, a substituted silyl, a deuterium atom, a cyano, and a halogen atom. For details of these substituents, the above description on the first substituent can be cited. Among these substituents, an alkyl (for example, an alkyl having 1 to 6 carbon atoms), a cycloalkyl (for example, a cycloalkyl having 3 to 14 carbon atoms), a halogen atom (for example, a fluorine atom), a deuterium atom, and the like are preferable. When a cycloalkyl is used as a substituent, a spiro structure may be formed as a substitution form. Examples in which a spiro structure is formed in a cycloalkane fused with one benzene ring (phenyl group) are illustrated below. The symbol * in each structural formula means a benzene ring included in a skeleton structure of a compound in a case of a benzene ring, and means a bond used for substitution of a skeleton structure of a compound in a case of a phenyl group.

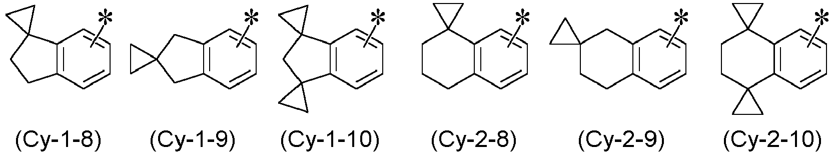

Examples of another form of cycloalkane fusing include a form in which a polycyclic aromatic compound represented by general formula (1) or (2) or a multimer thereof is substituted by a cycloalkane-fused diarylamino group (an aryl group portion thereof is fused), a cycloalkane-fused carbazolyl group (a benzene ring portion thereof is fused), or a cycloalkane-fused benzocarbazolyl group (a benzene ring portion thereof is fused). Examples of the "diarylamino group" include the groups described as the above "first substituent".

More specific examples thereof include a form in which R² in a polycyclic aromatic compound represented by general formula (2) or a multimer thereof is a cycloalkane-fused diarylamino group (an aryl group portion thereof is fused) or a cycloalkane-fused carbazolyl group (a benzene ring portion thereof is fused).

Examples thereof include a polycyclic aromatic compound represented by the following general formula (2-A) and a multimer of a polycyclic aromatic compound having a plurality of structures each represented by the following general formula (2-A). In the following structural formula, Cy represents a cycloalkane, n's each independently represent an integer of 1 to 3 (preferably 1), and "=(Cy)n" means that n cycloalkanes are fused with a structure to be fused at any positions (in the following structural formula, n cycloalkanes are fused with a benzene ring (phenyl group)), and the definitions of the reference numerals in the structural formula are the same as those in general formula (2).

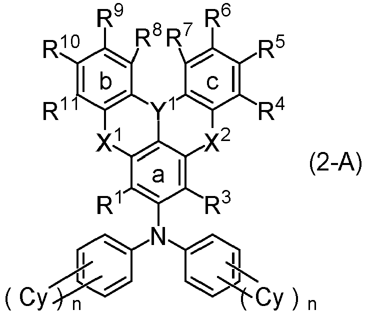

Specific examples thereof include compounds represented by the following formulas "1-Cy-(1)" to "1-Cy-(4401)". In the following formulas, Cy represents a cycloalkane, n's each independently represent 0 to a maximum fusible number (all n's do not represent 0), preferably 0 to 2 (all n's do not represent 0), and more preferably 1, and "=(Cy)n" means that n cycloalkanes are fused with a structure to be fused at any positions (for example, in the following formula "1-Cy-(1)", n cycloalkanes are fused with each benzene ring at any positions). Note that, in the following structural formulas, "OPh" represents a phenoxy group, "Me" represents a methyl group, and each compound is substituted at an α-position carbon atom of a cycloalkane as specified in the appended claims and may be substituted by the above first and second substituents.

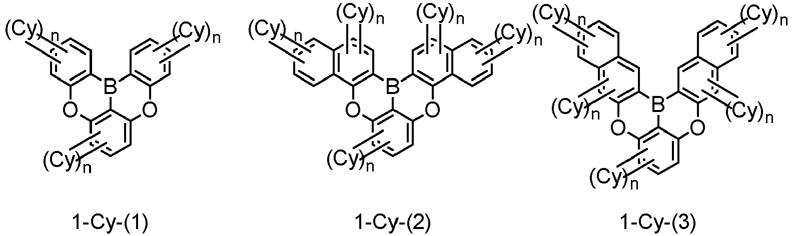

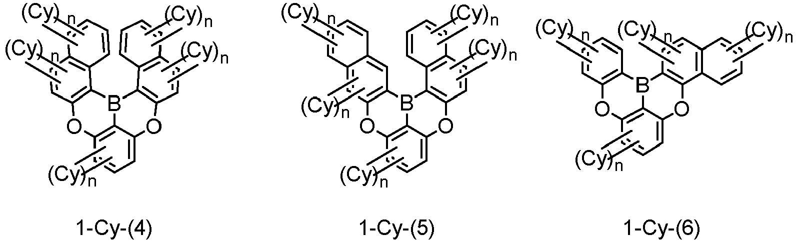

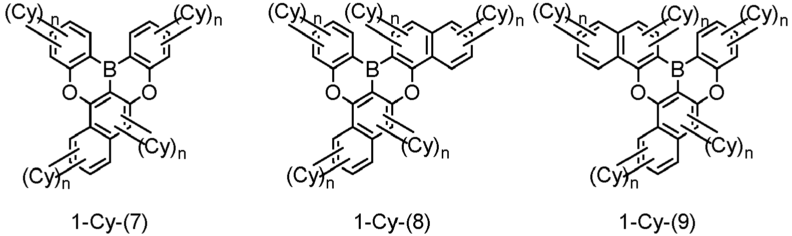

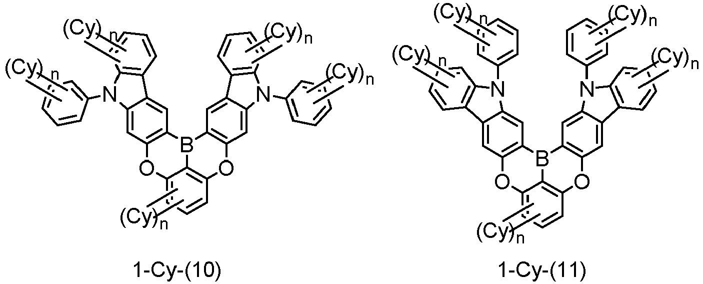

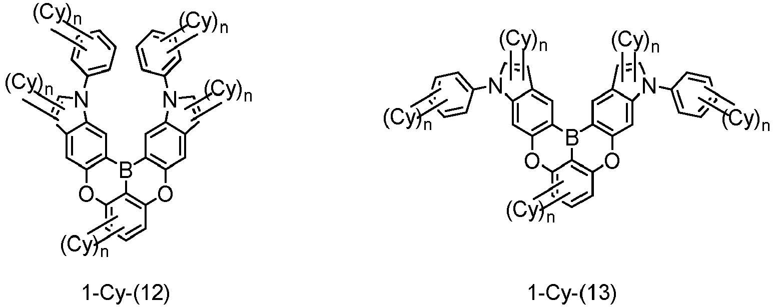

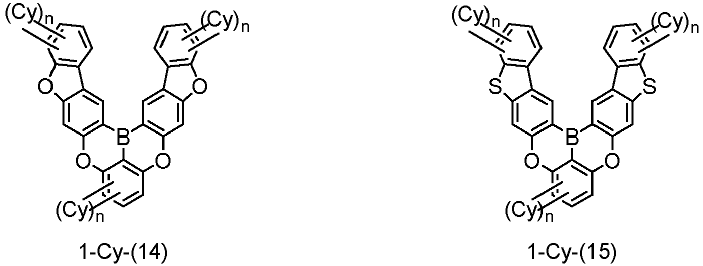

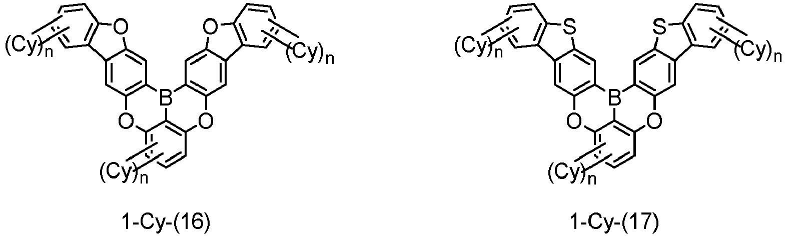

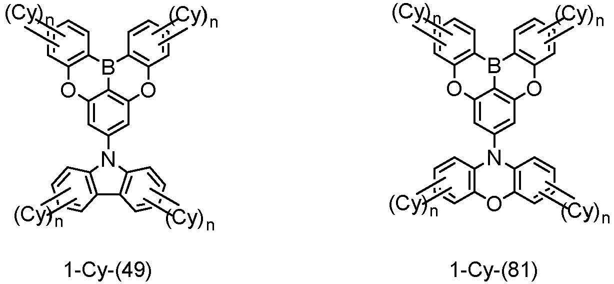

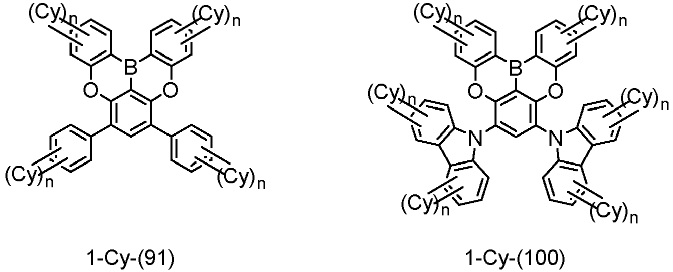

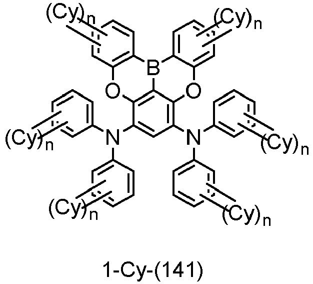

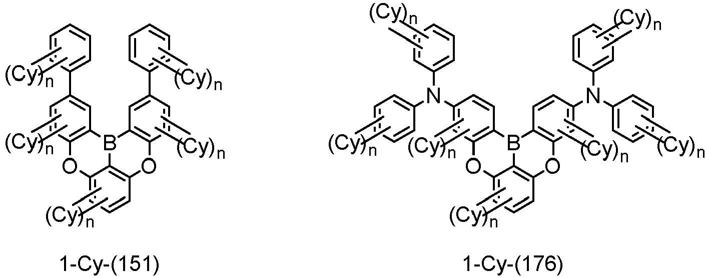

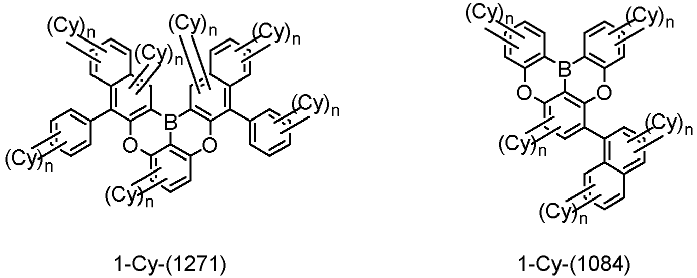

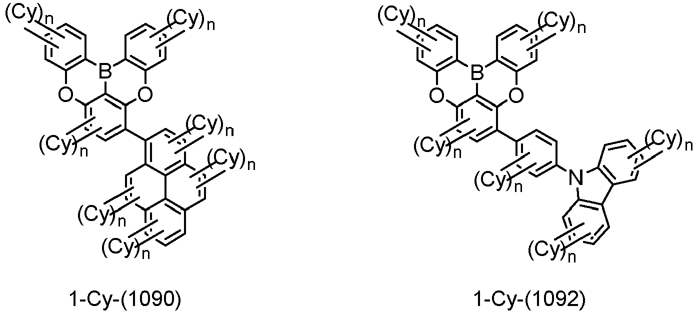

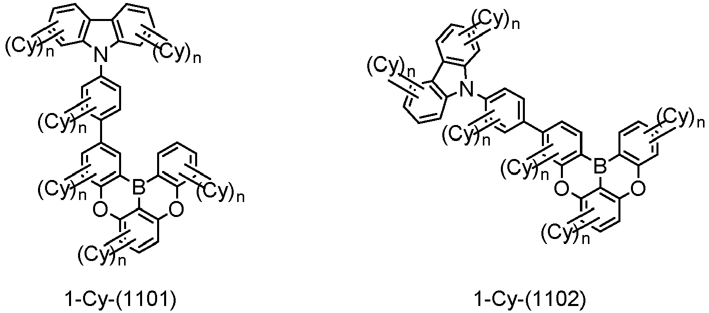

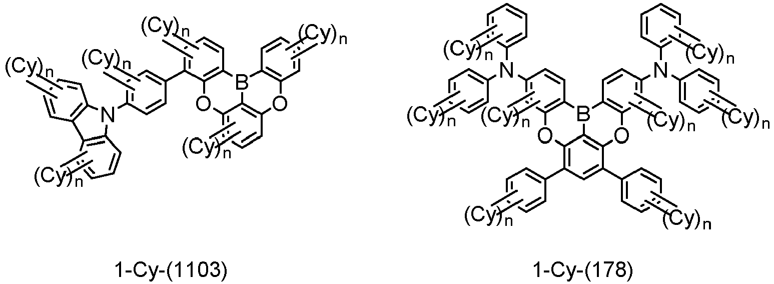

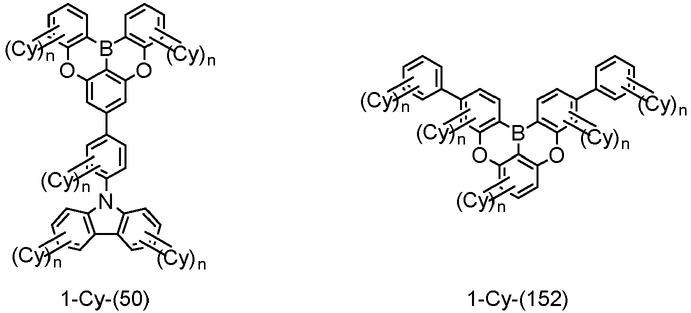

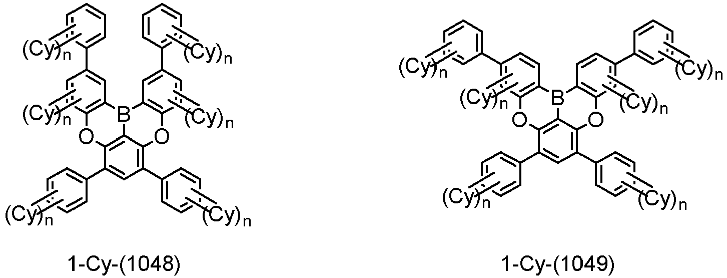

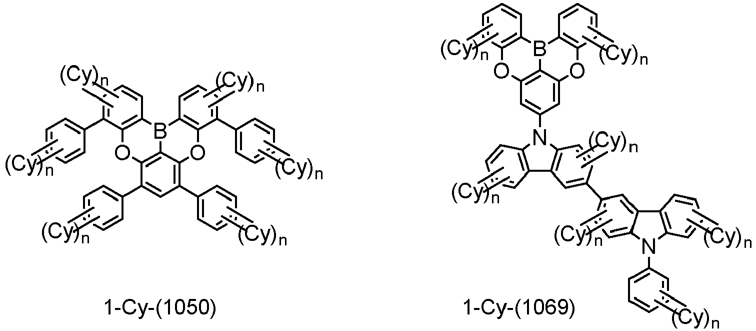

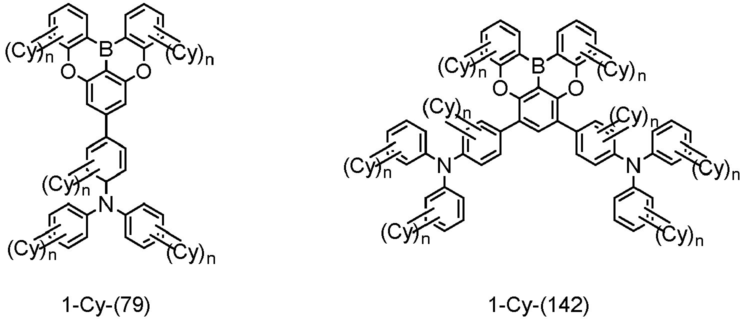

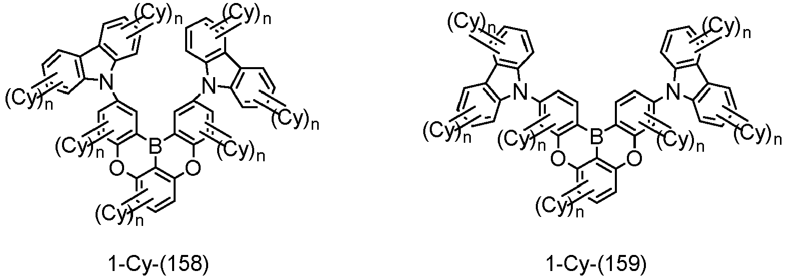

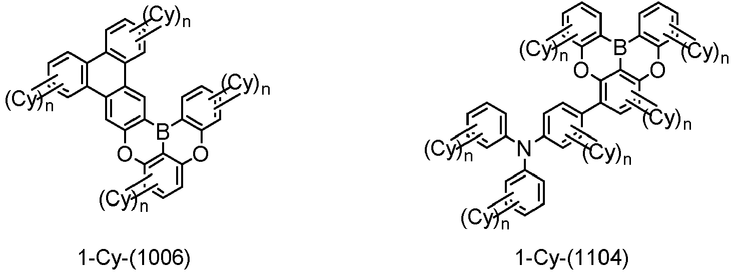

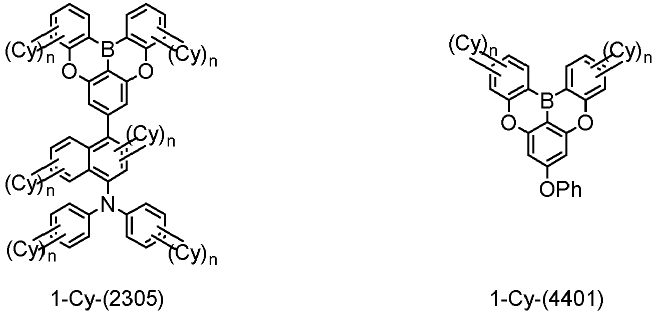

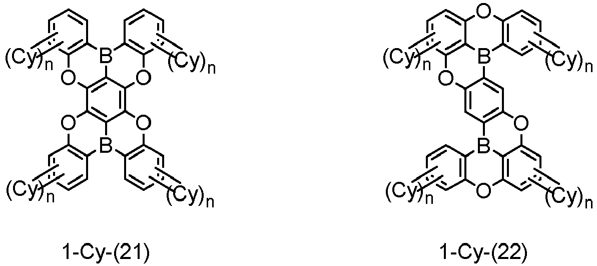

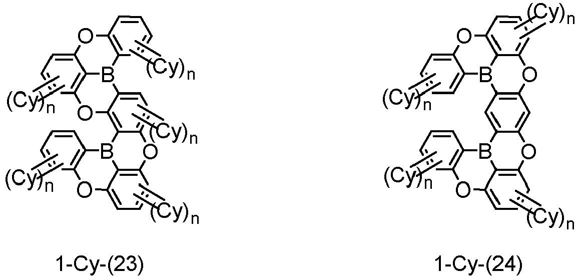

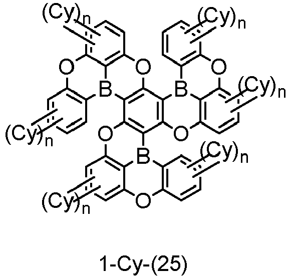

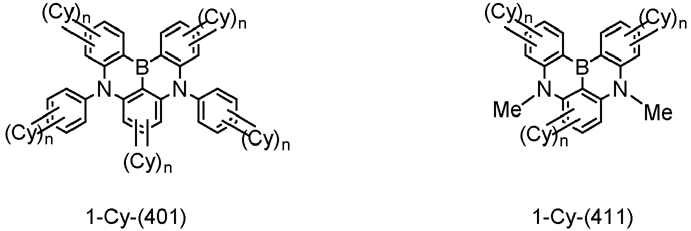

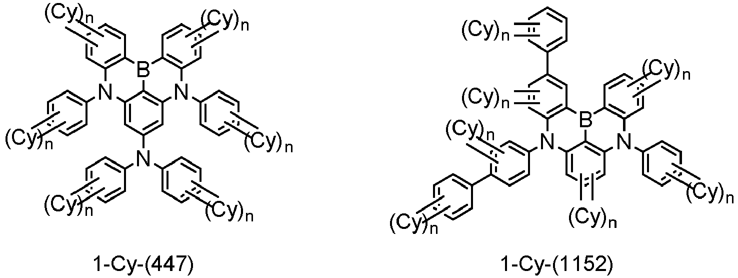

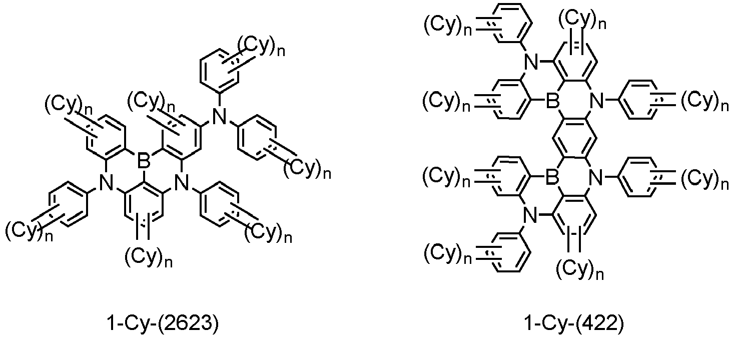

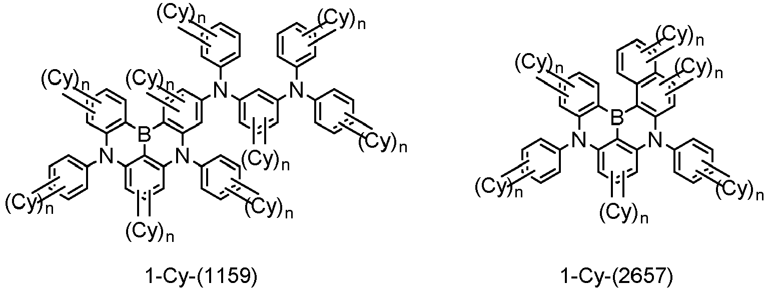

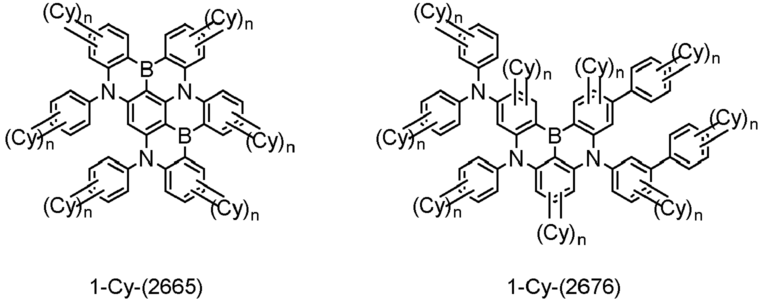

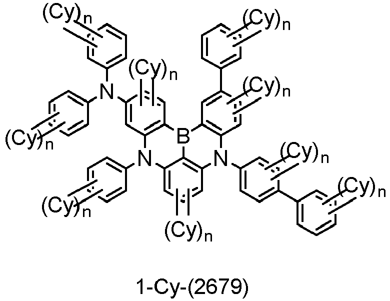

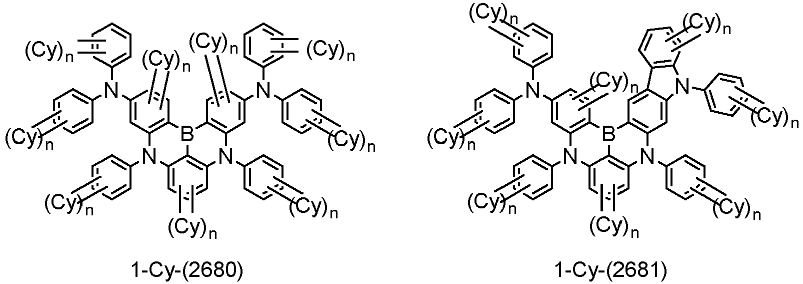

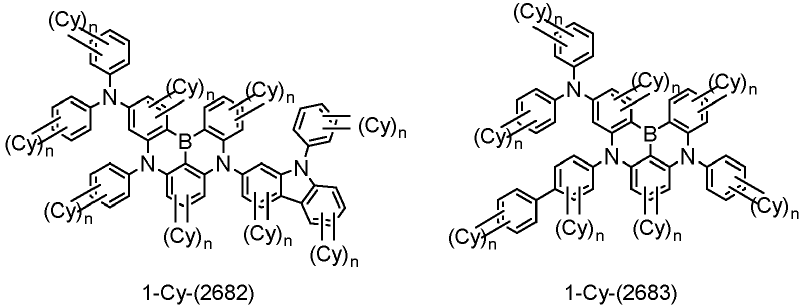

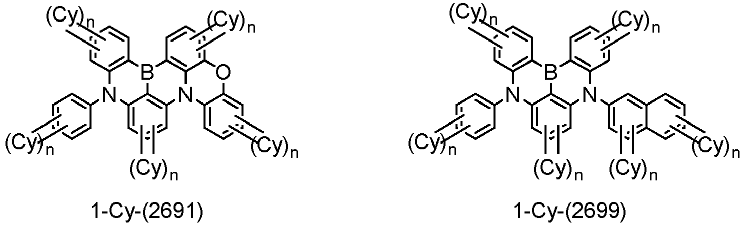

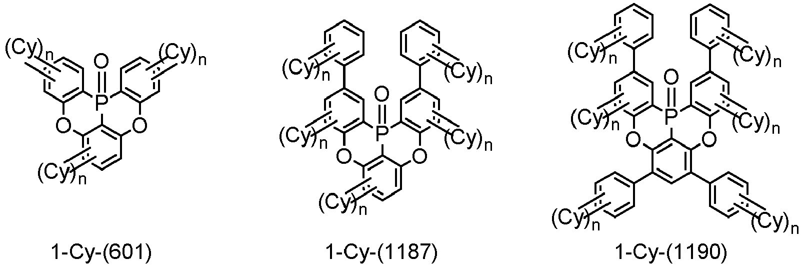

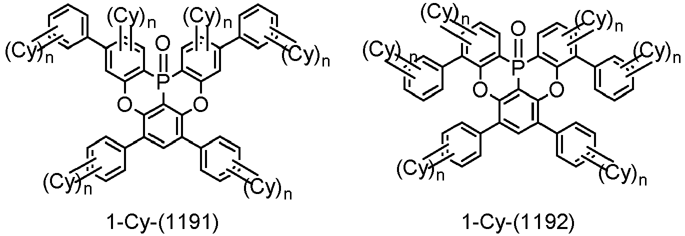

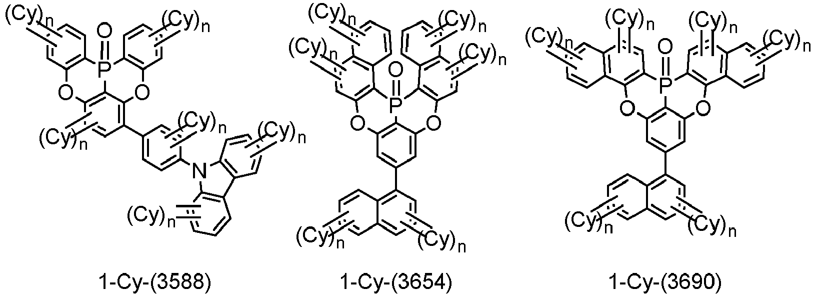

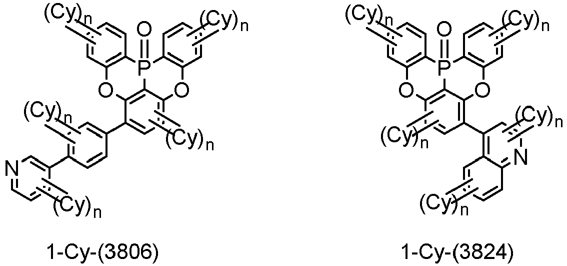

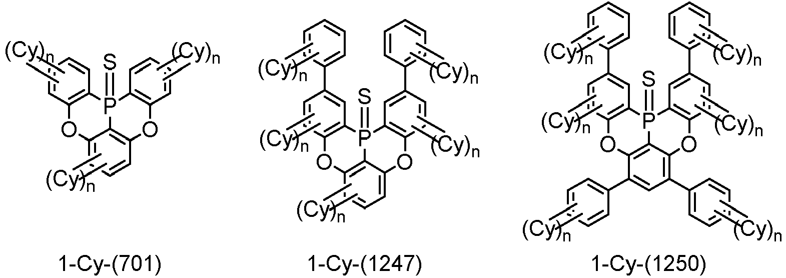

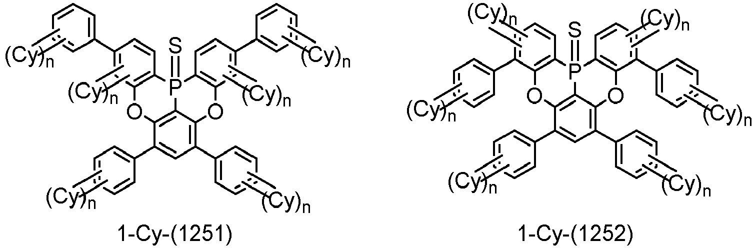

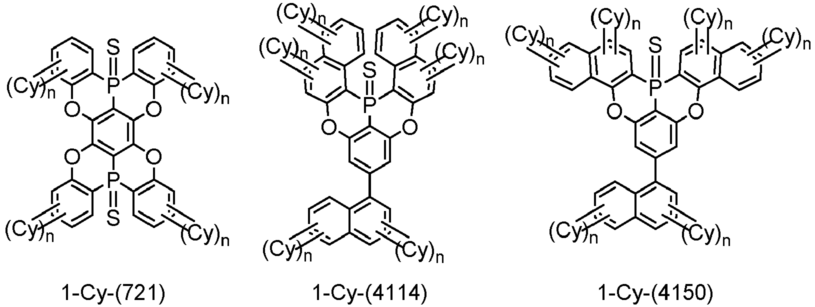

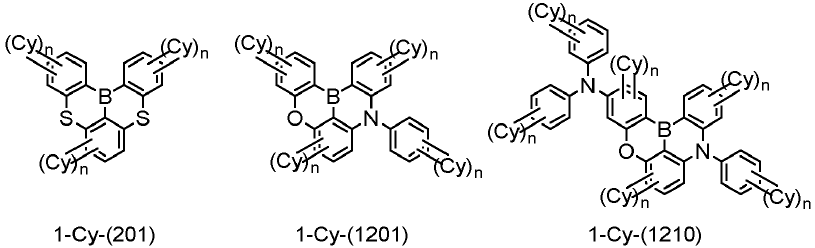

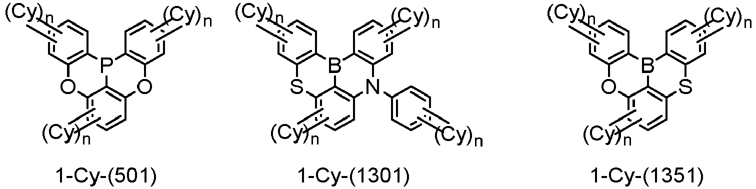

More specific examples of the cycloalkane-fused polycyclic aromatic compound of the present invention are compounds represented by the following structural formulas. In the following structural formulas, "D" represents a deuterium atom, "Me" represents a methyl group, "Et" represents an ethyl group, "iPr" represents an isopropyl group, "tBu" represents a t-butyl group, and "tAm" represents t-amil group, "Ph" represents a phenyl group, "F" represents a fluorine atom, "CN" represents a cyano group, "TMS" represents a trimethylsilyl group, and "TPhS" represents a triphenylsilyl group. In the formulas (1-1001) to (1-1265), the notation of the methyl group (Me) in the structural formula is omitted.

In this paragraph, compound (1-5) is an inventive compound. The remaining compounds are comparative compounds.

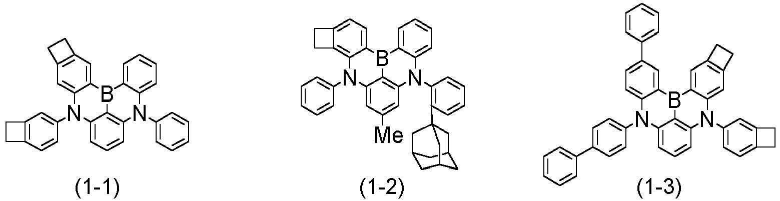

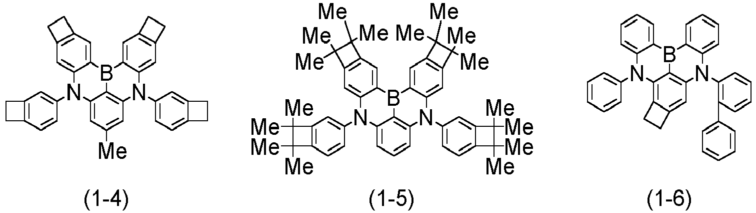

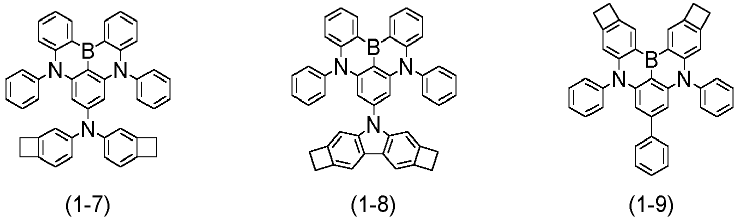

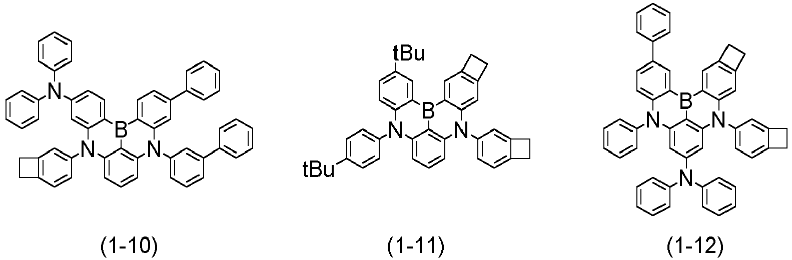

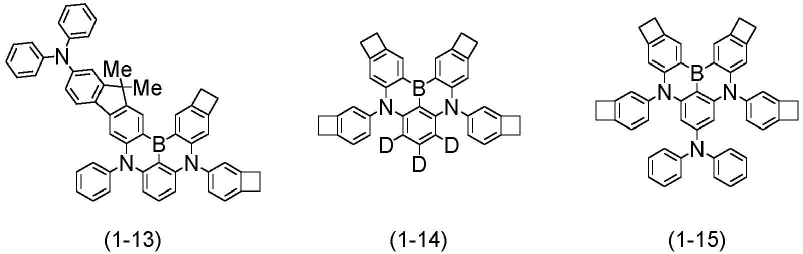

In this paragraph, compound (1-29) is an inventive compound. The remaining compounds are comparative compounds.

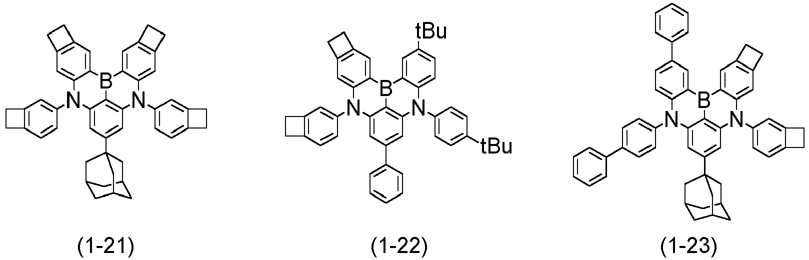

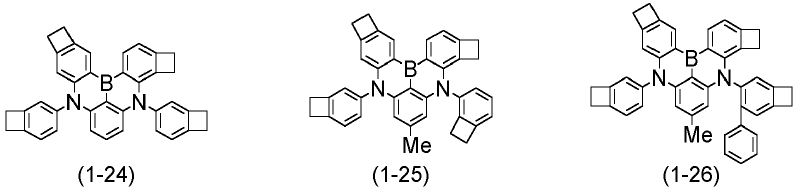

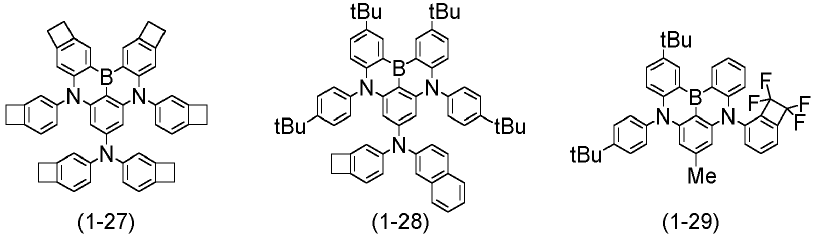

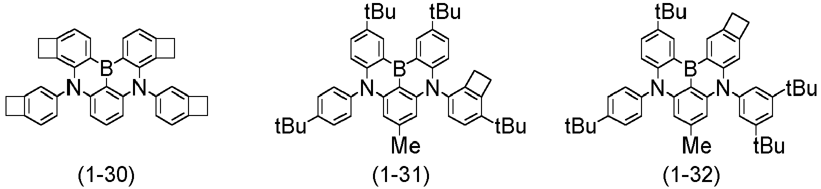

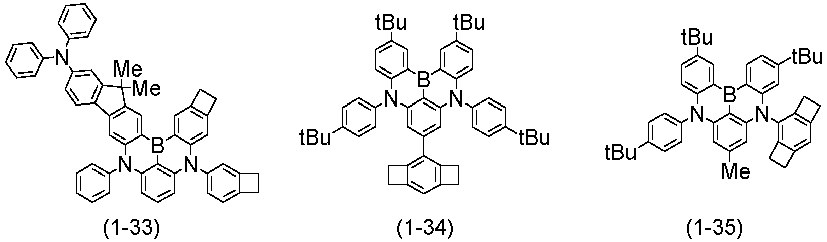

In this paragraph, compound (1-54) is an inventive compound. The remaining compounds are comparative compounds.

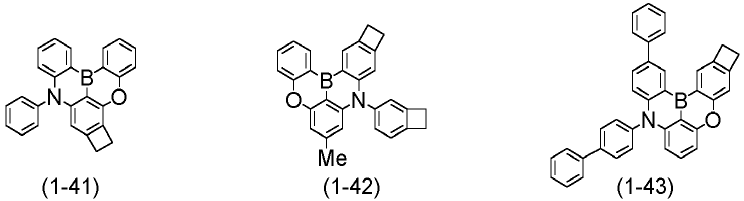

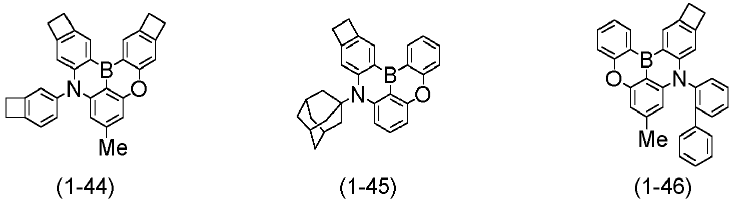

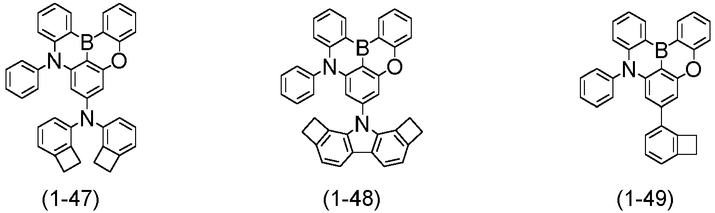

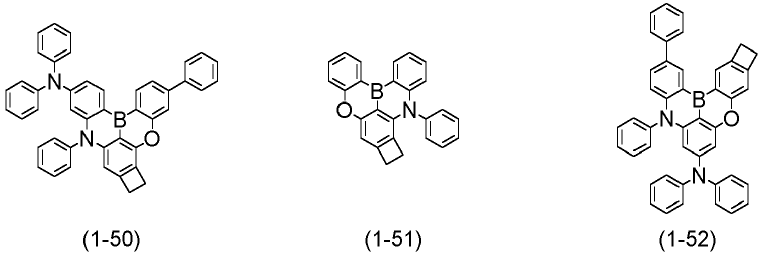

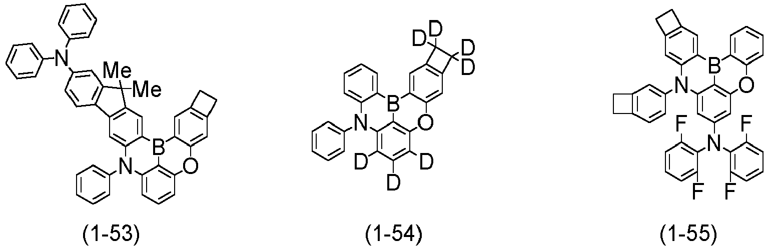

In this paragraph, compound (1-65) is an inventive compound. The remaining compounds are comparative compounds.

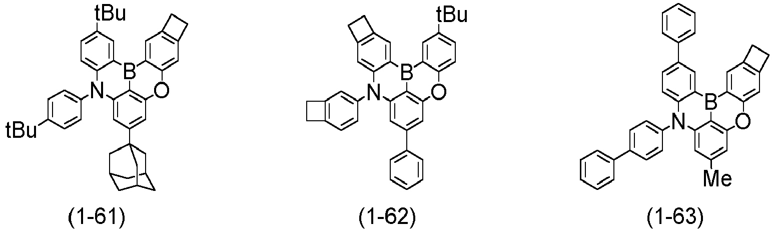

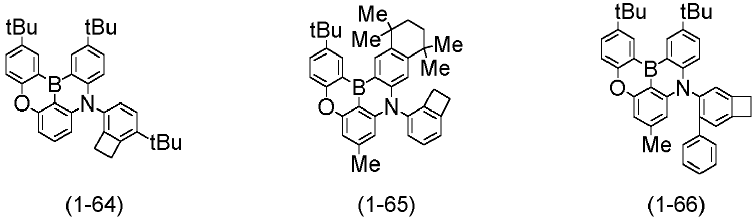

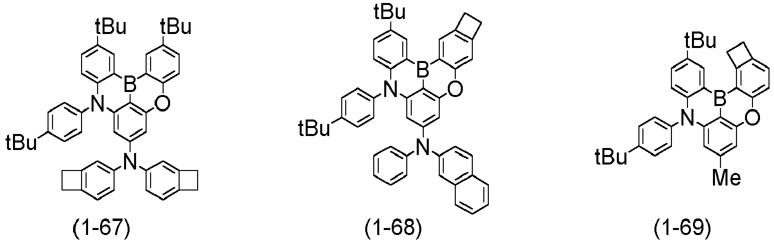

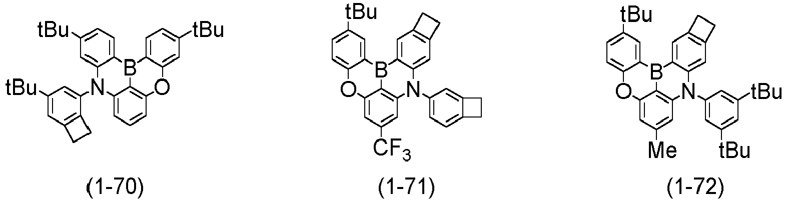

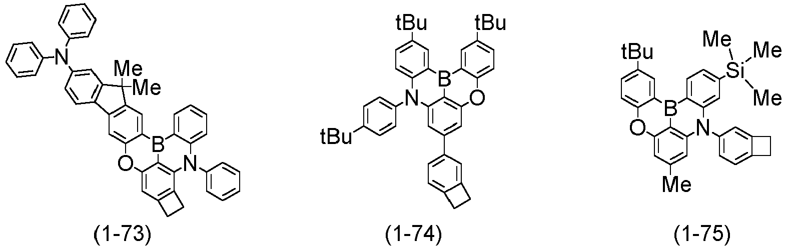

In this paragraph, all compounds are comparative compounds.

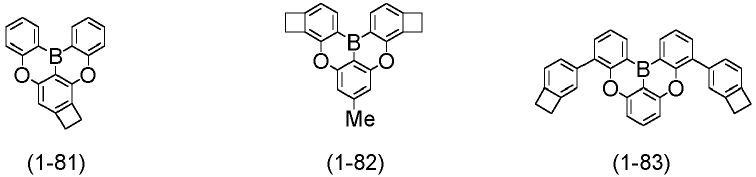

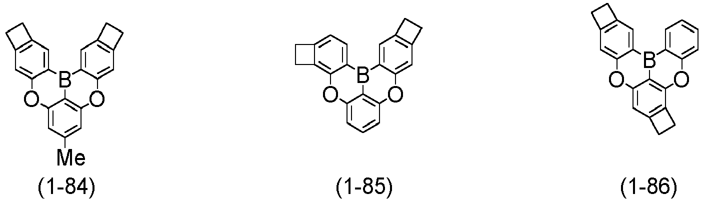

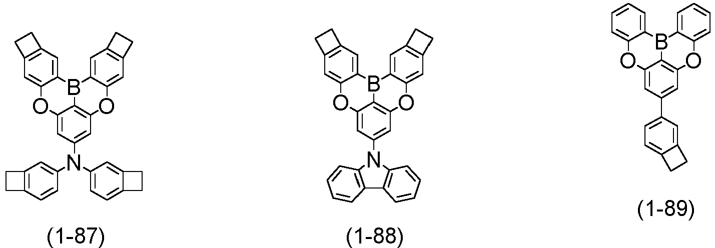

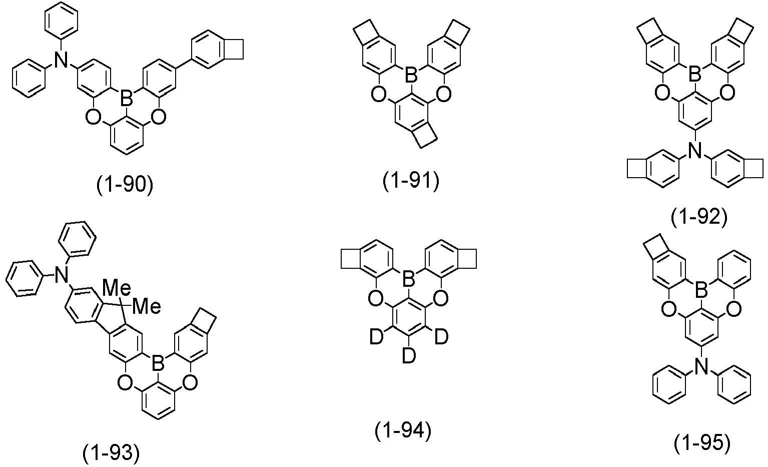

In this paragraph, all compounds are comparative compounds.

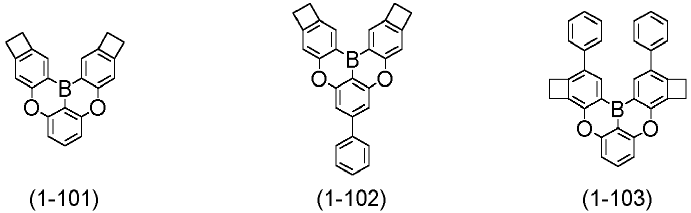

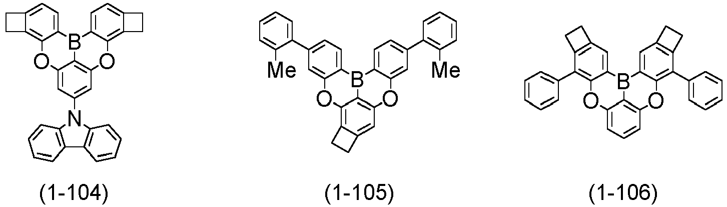

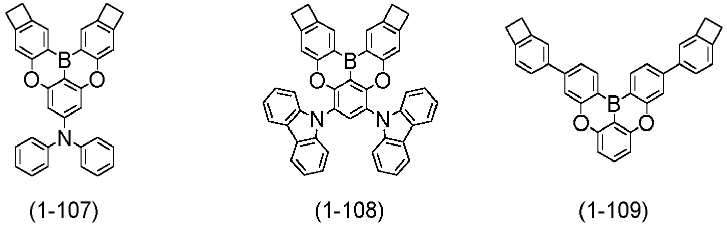

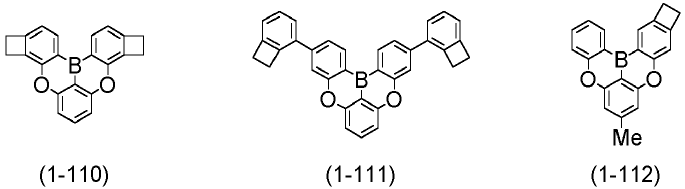

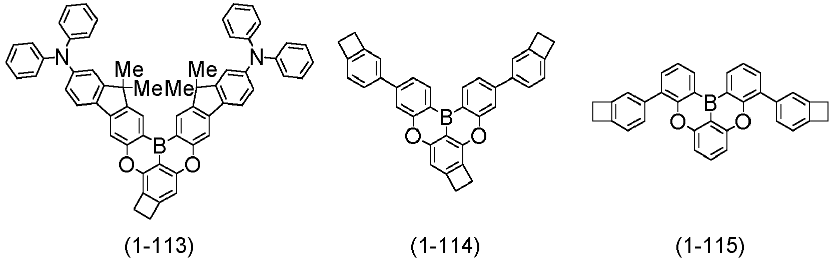

In this paragraph, all compounds are comparative compounds.

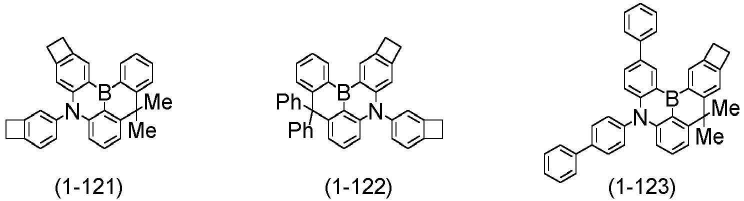

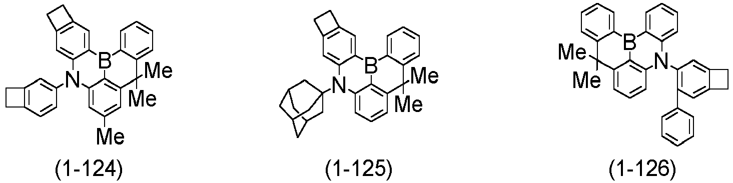

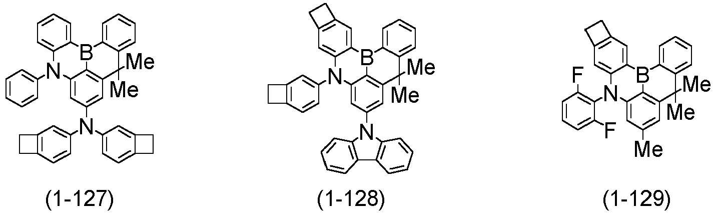

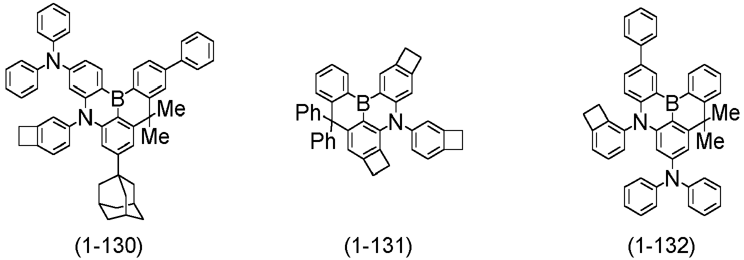

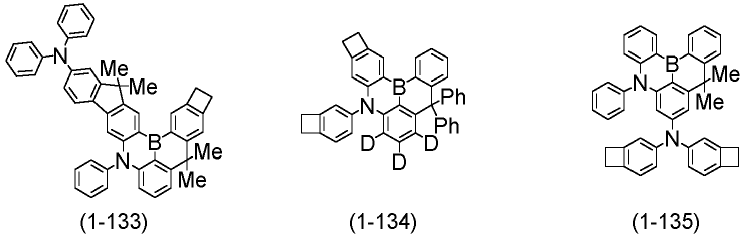

In this paragraph, all compounds are comparative compounds.

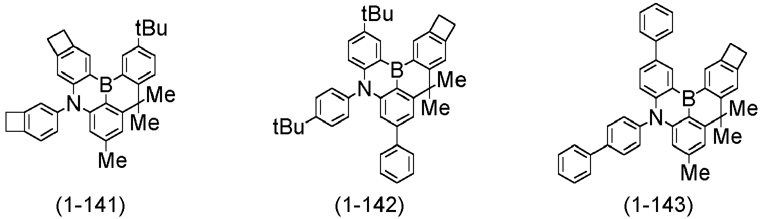

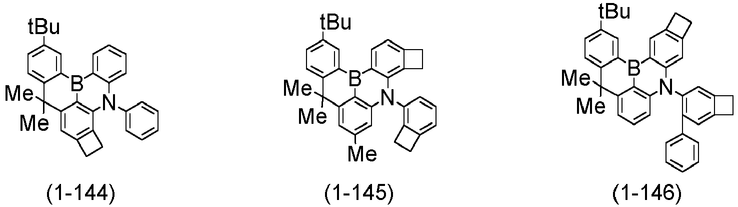

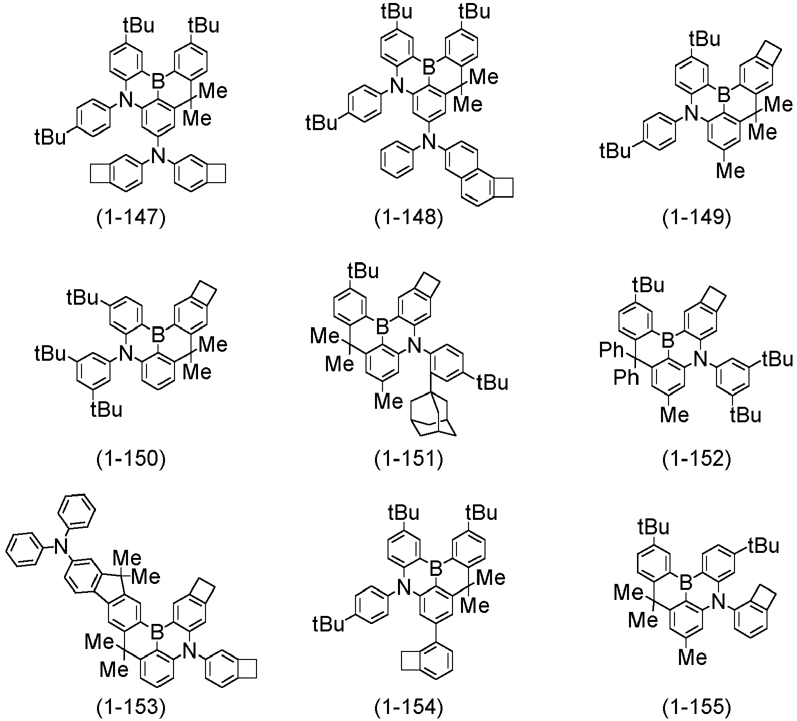

In this paragraph, all compounds except compound (1-205) are inventive compounds.

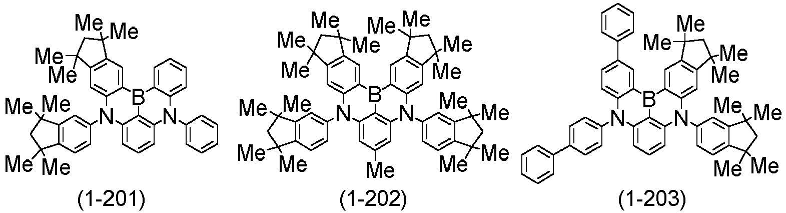

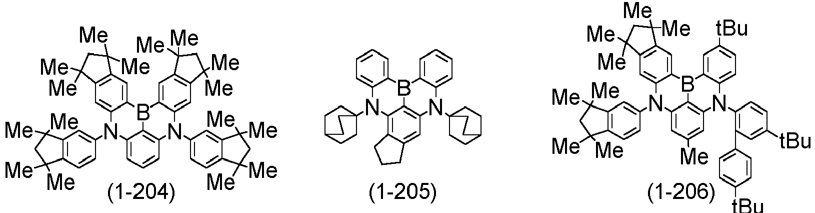

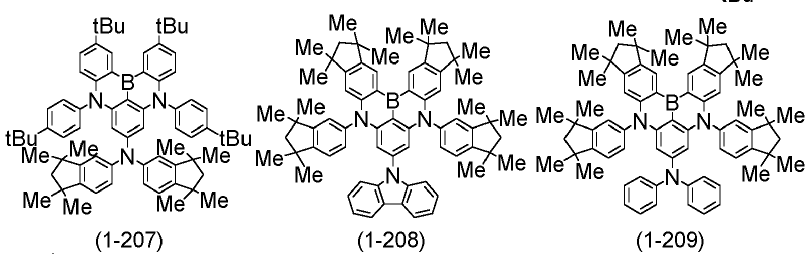

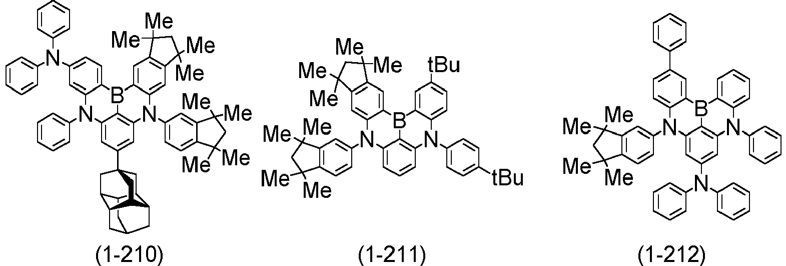

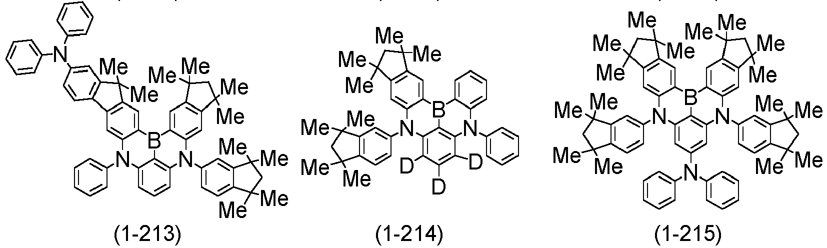

In this paragraph, all compounds except compound (1-234) are inventive compounds.

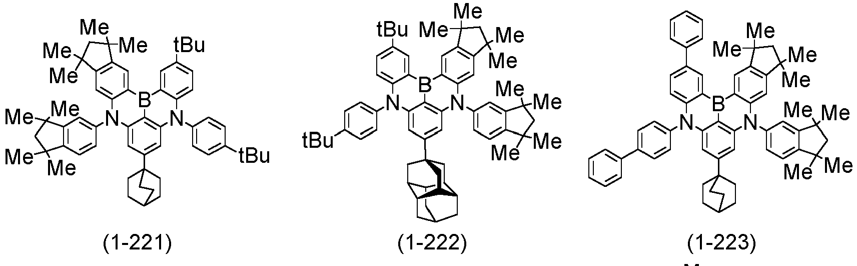

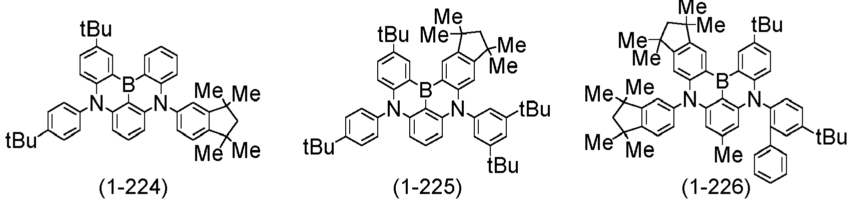

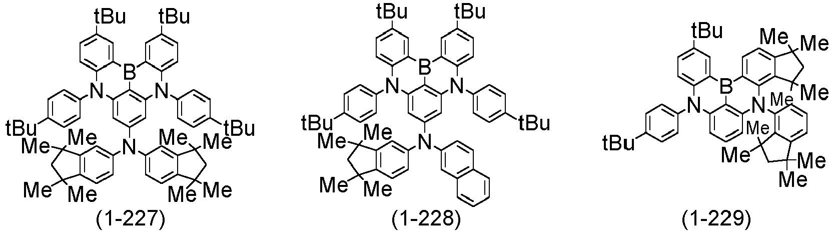

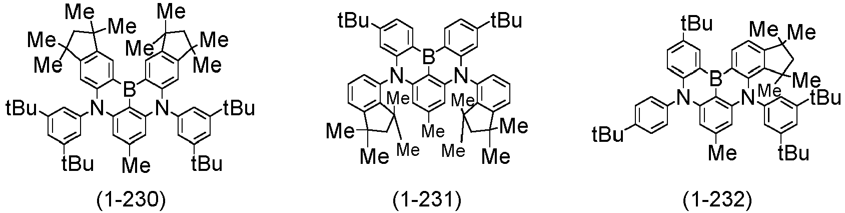

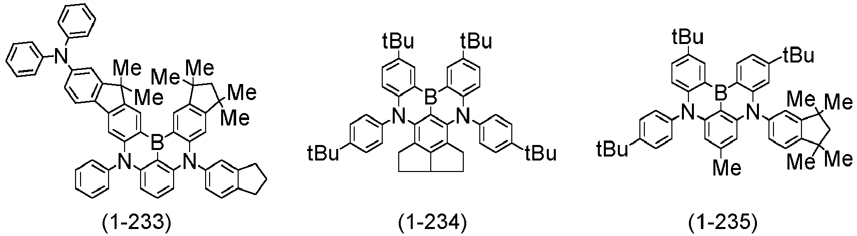

In this paragraph, all compounds are inventive compounds.

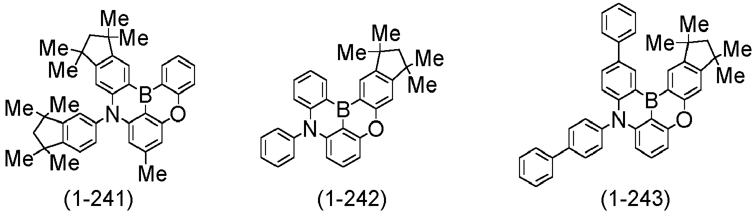

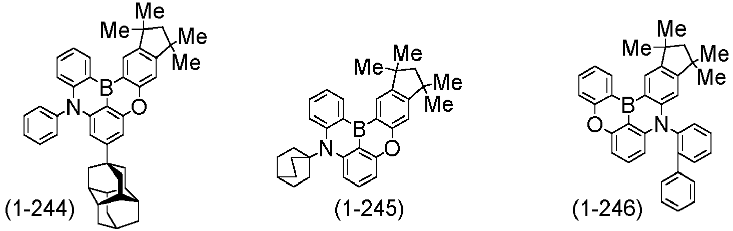

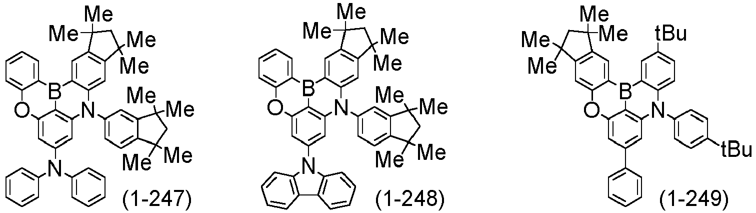

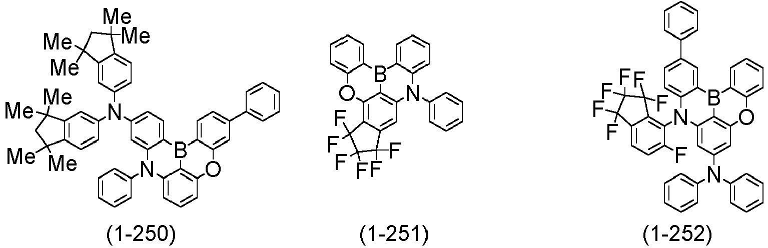

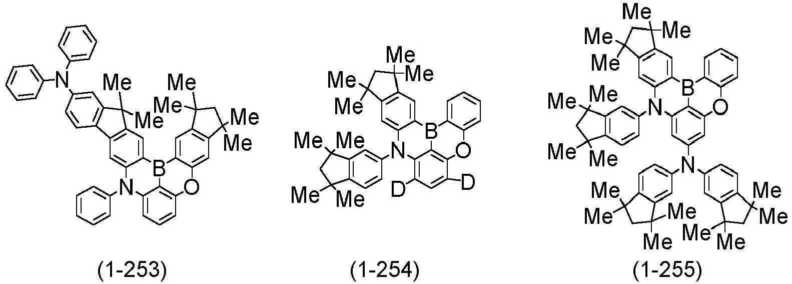

In this paragraph, all compounds except compounds (1-268) and (1-274) are inventive compounds.

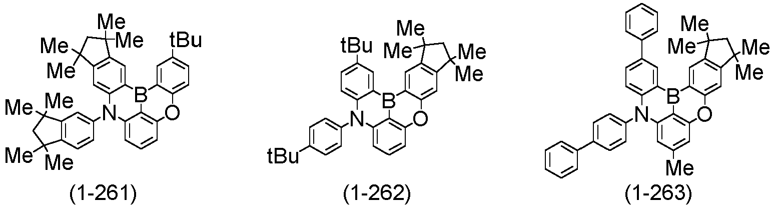

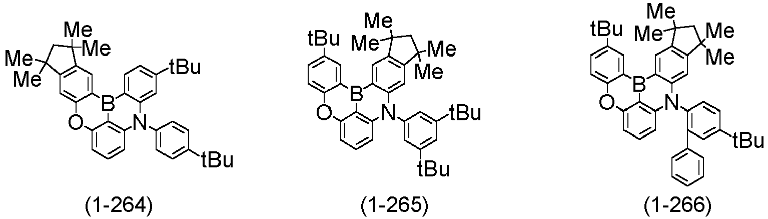

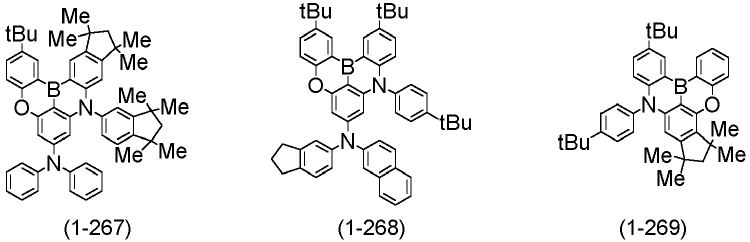

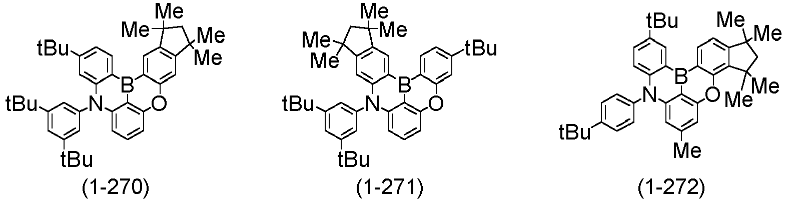

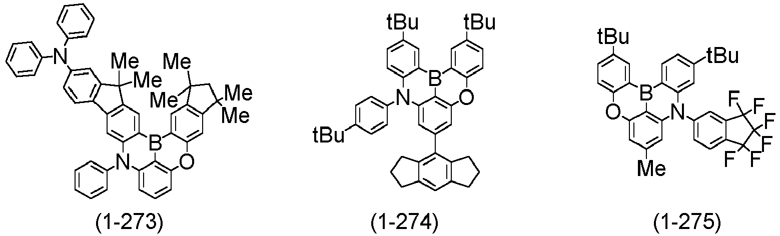

In this paragraph, all compounds except compound (1-286) are inventive compounds.

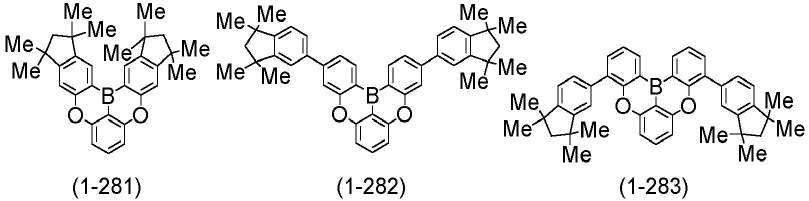

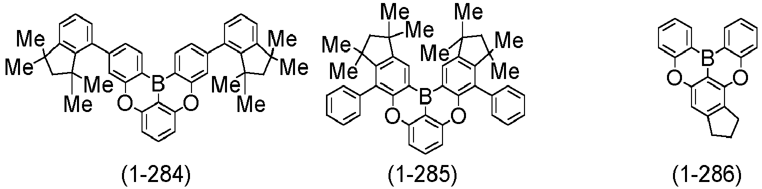

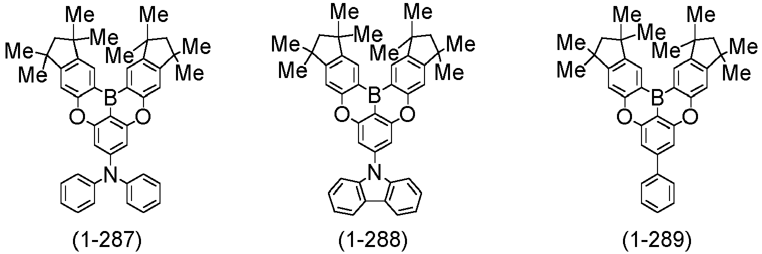

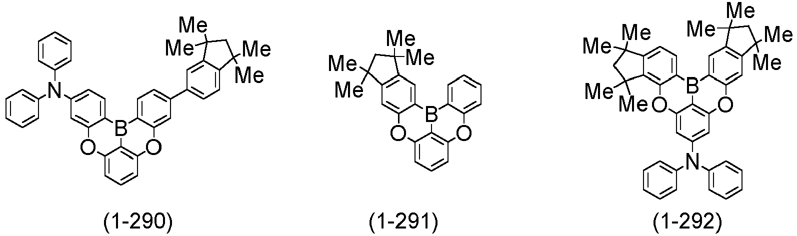

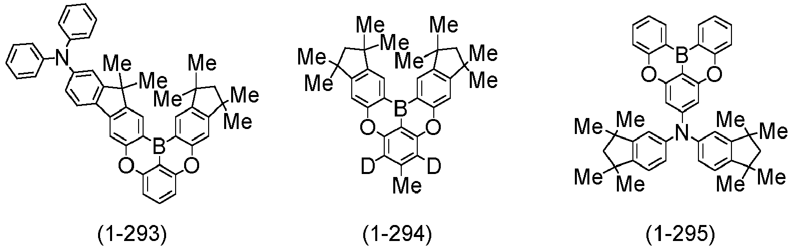

In this paragraph, all compounds except compounds (1-310) and (1-311) are inventive compounds.

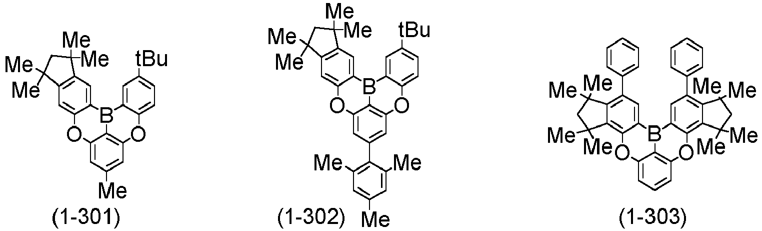

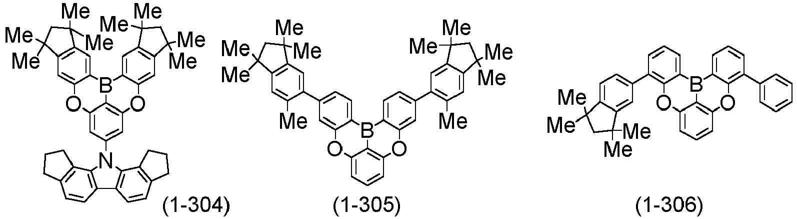

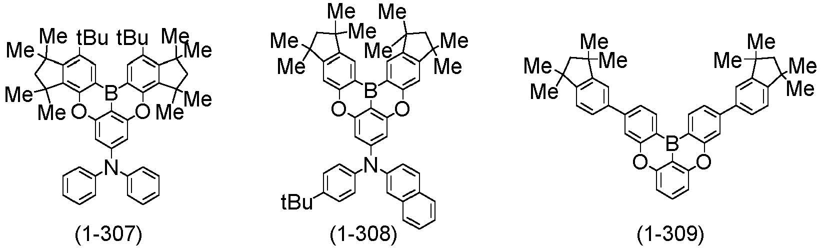

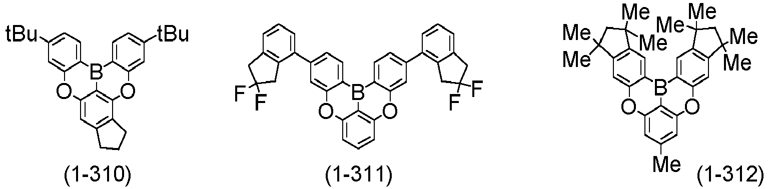

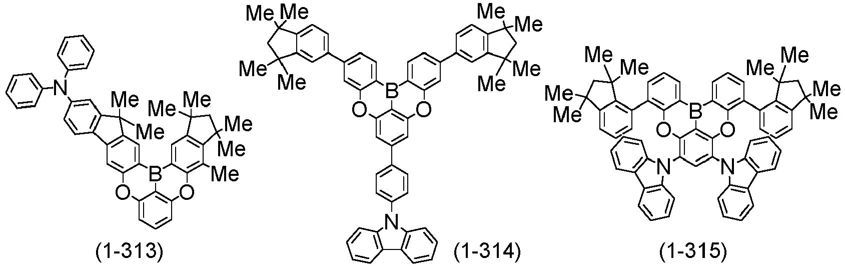

In this paragraph, all compounds are inventive compounds.

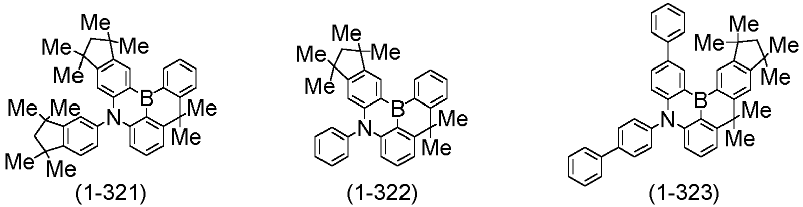

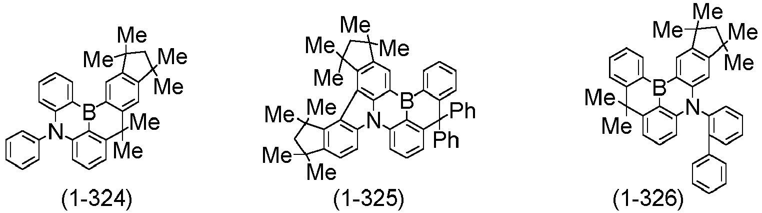

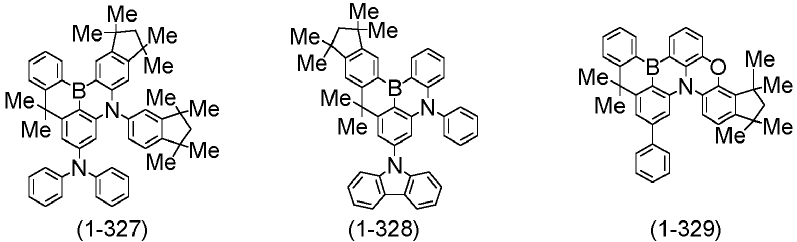

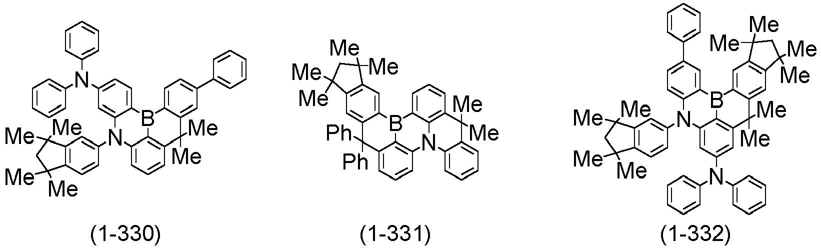

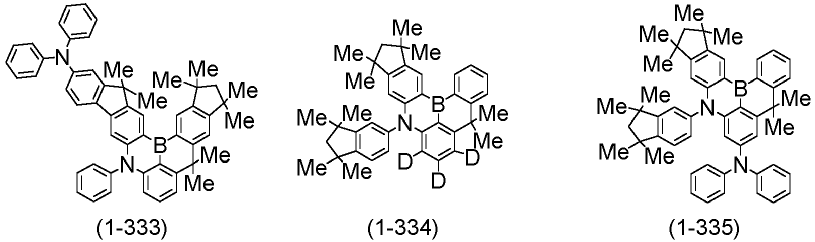

In this paragraph, all compounds are inventive compounds.

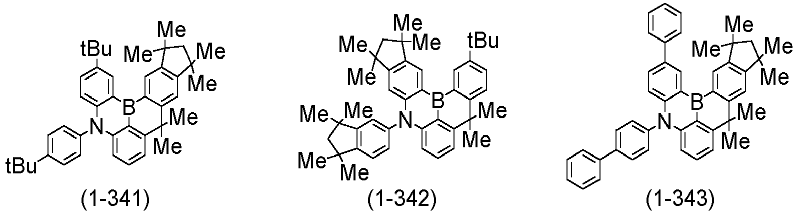

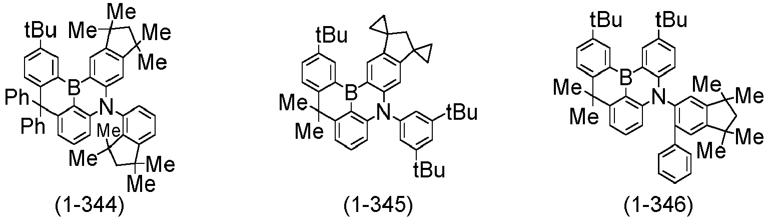

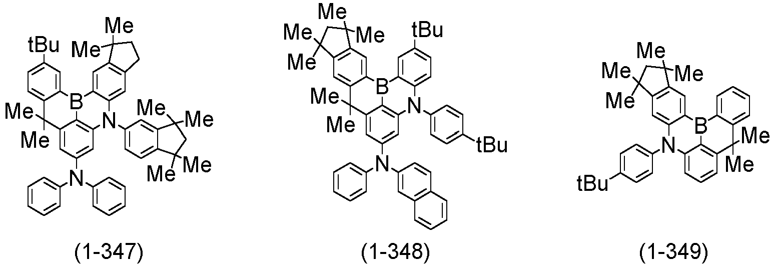

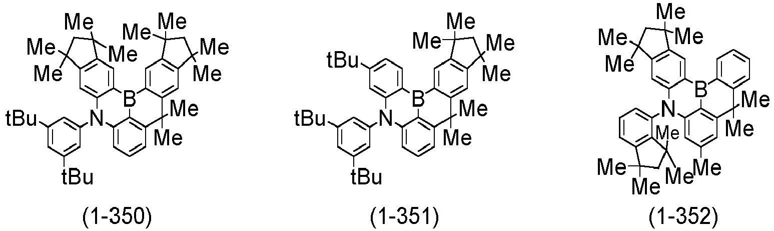

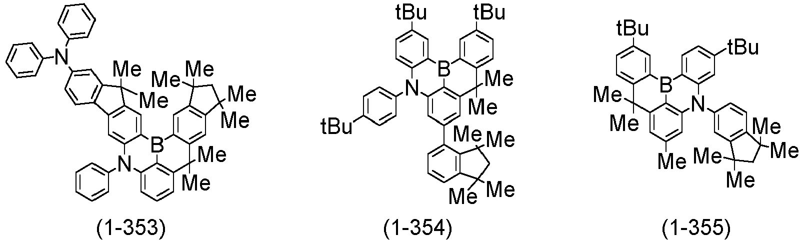

In this paragraph, all compounds except compound (1-405) are inventive compounds.

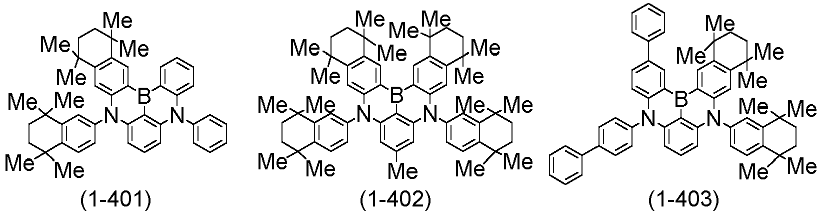

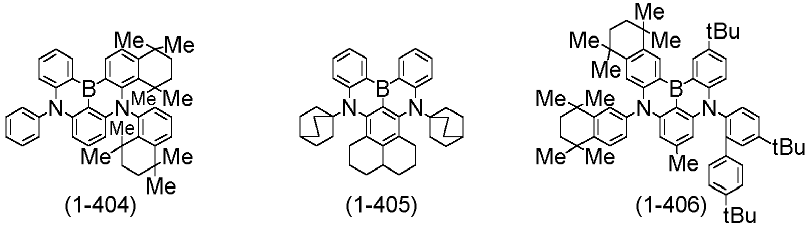

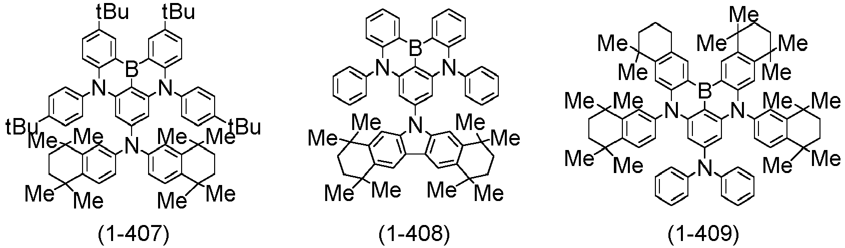

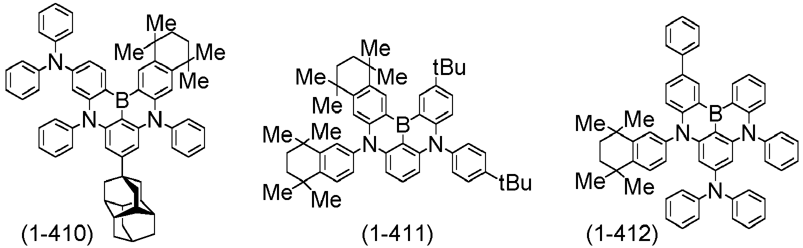

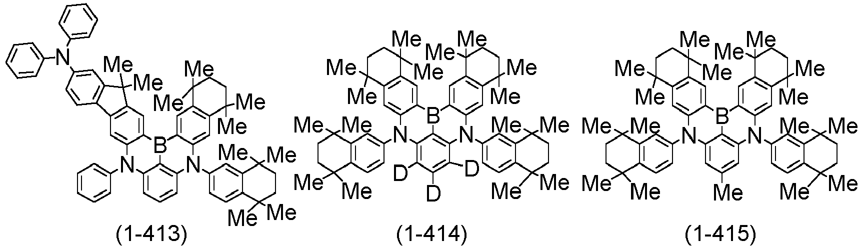

In this paragraph, all compounds except compound (1-433) are inventive compounds.

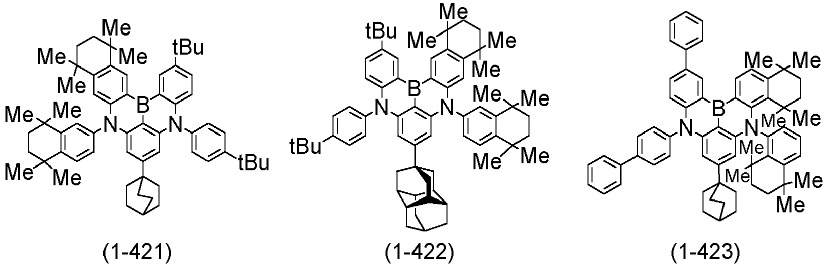

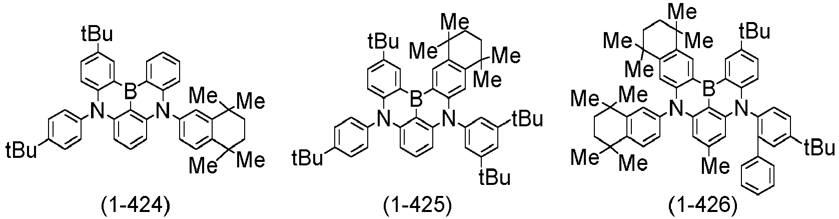

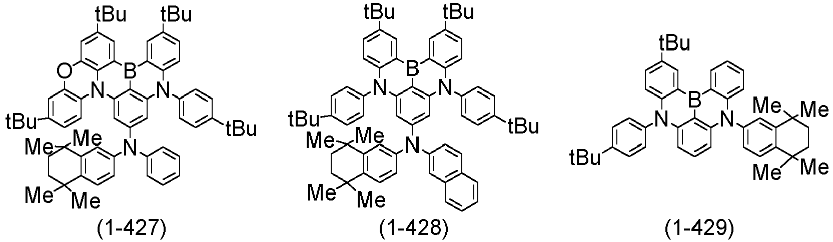

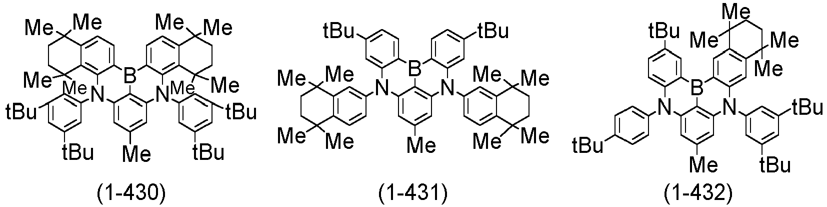

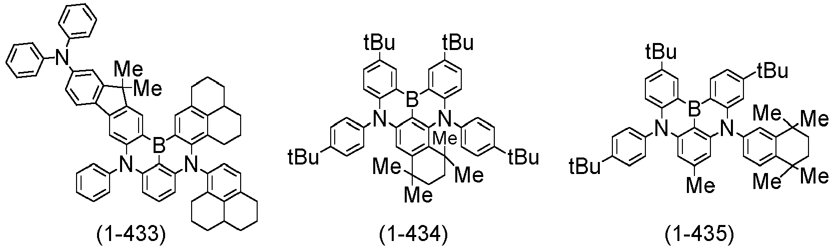

In this paragraph, all compounds are inventive compounds.

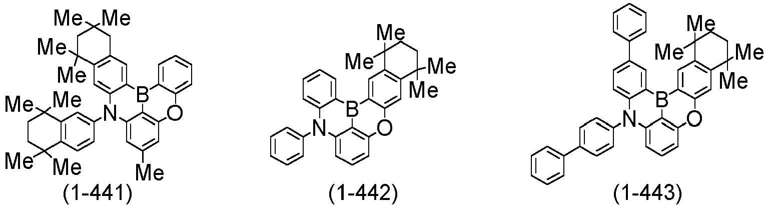

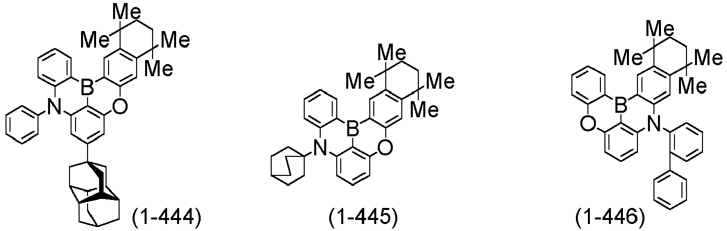

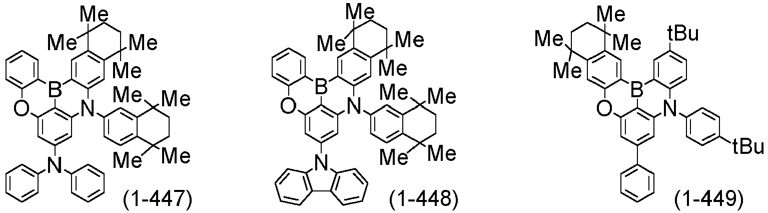

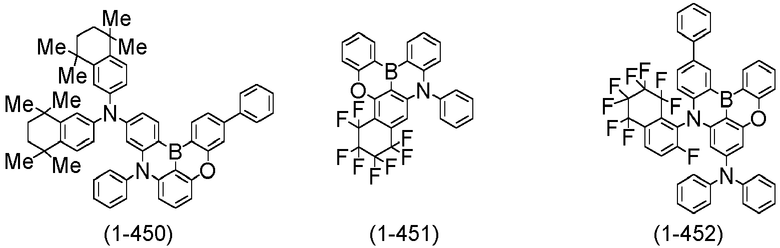

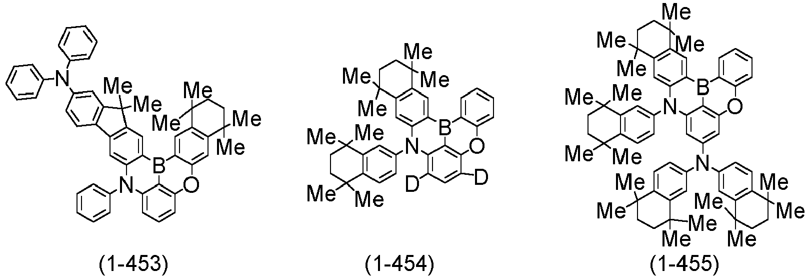

In this paragraph, all compounds except compound (1-474) are inventive compounds.

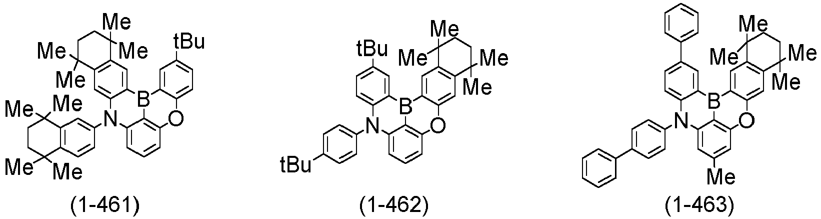

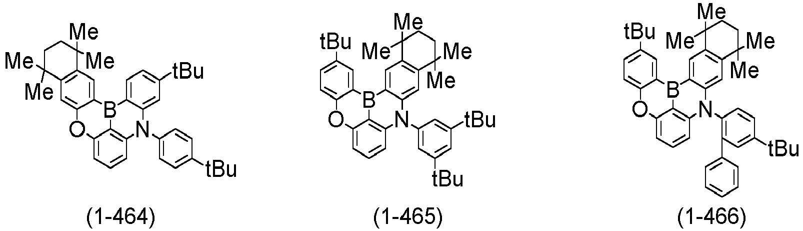

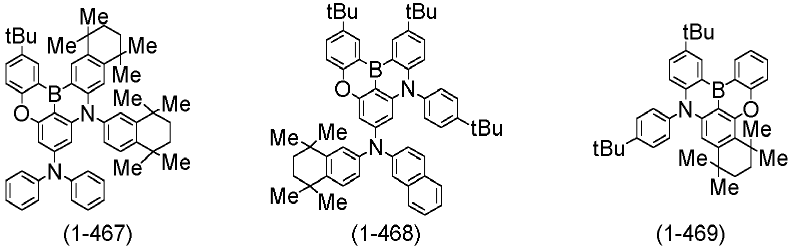

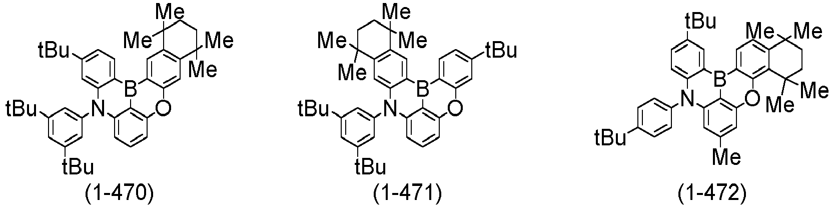

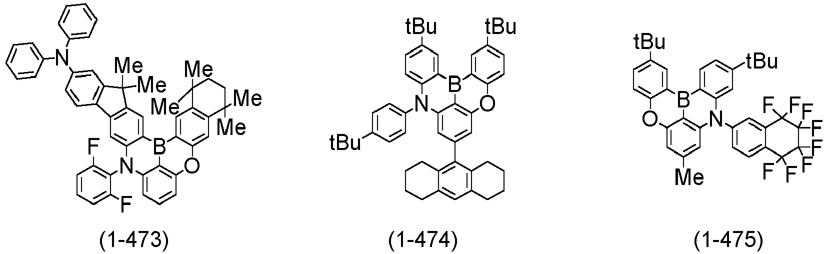

In this paragraph, all compounds except compound (1-486) are inventive compounds.

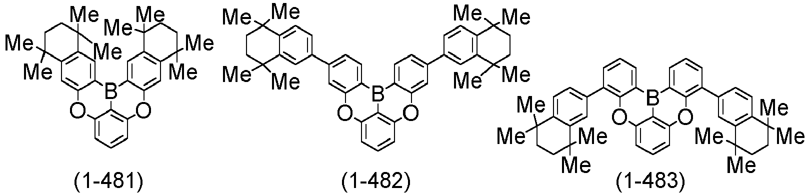

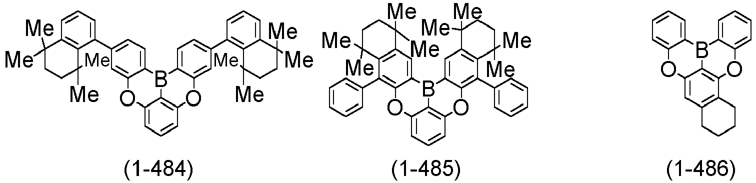

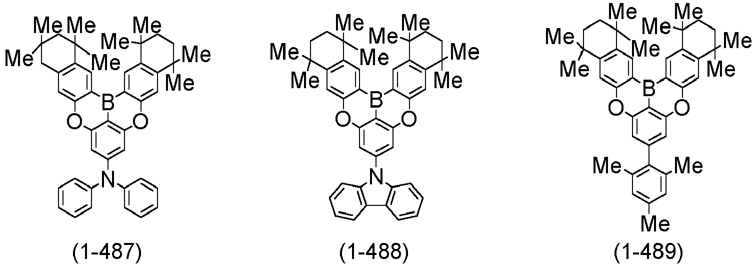

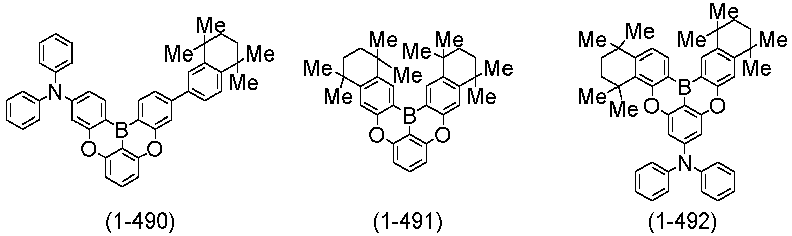

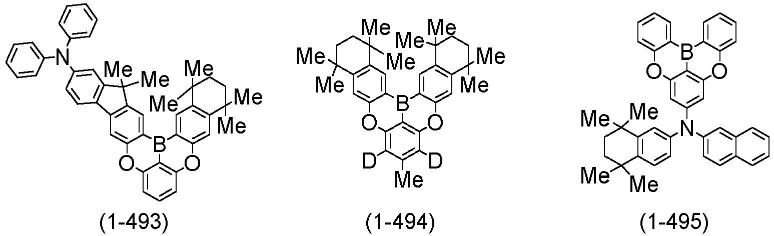

In this paragraph, all compounds are inventive compounds.

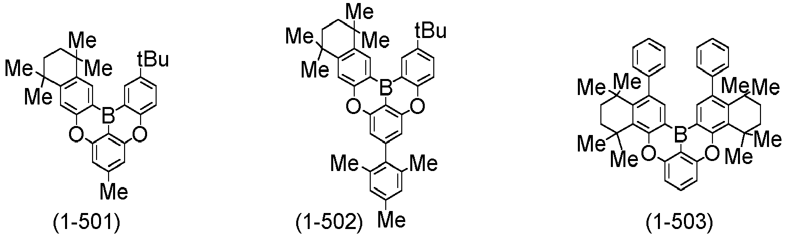

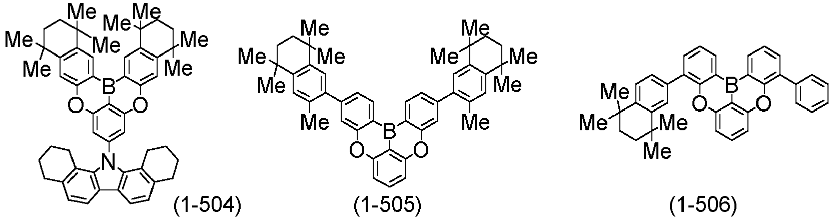

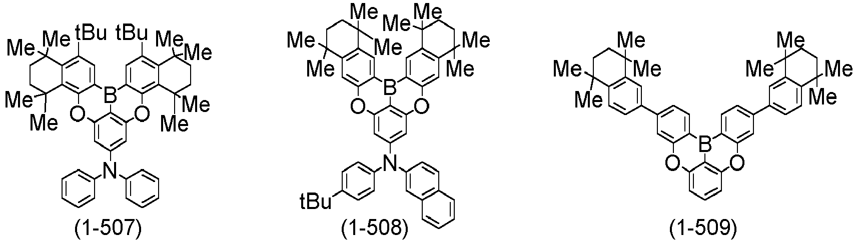

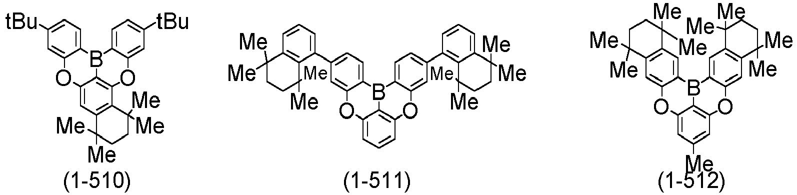

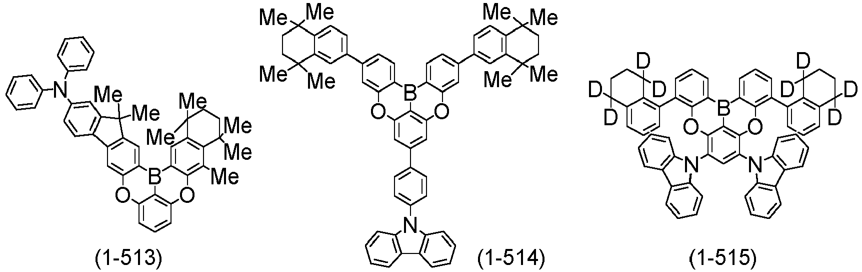

In this paragraph, all compounds are inventive compounds.

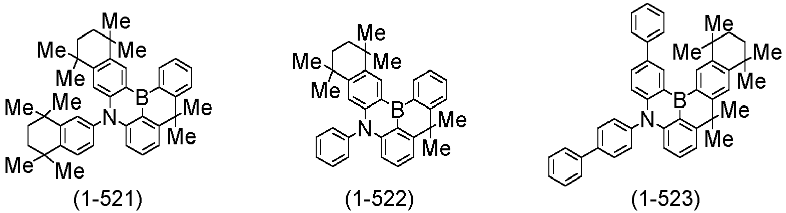

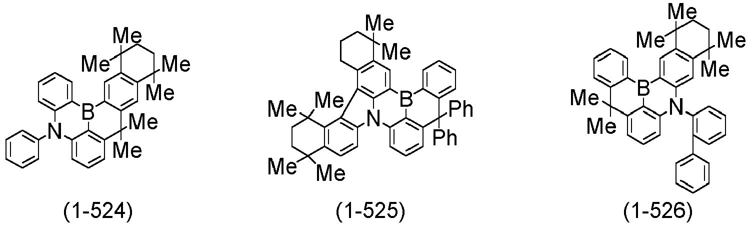

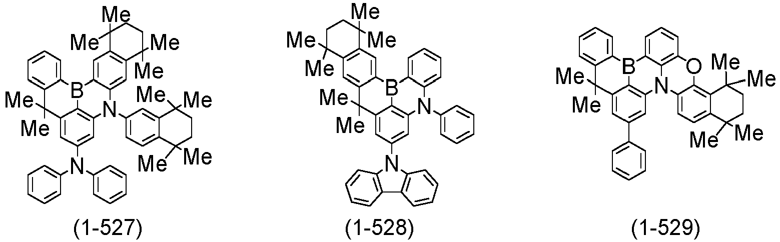

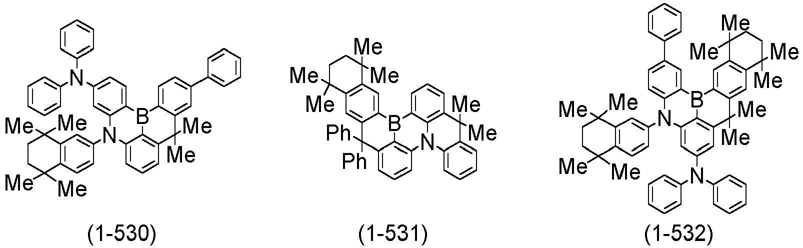

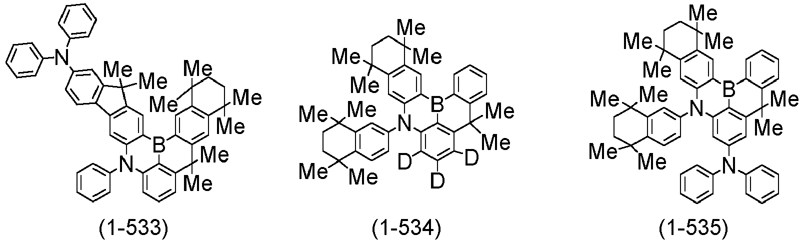

In this paragraph, all compounds except compound (1-554) are inventive compounds.

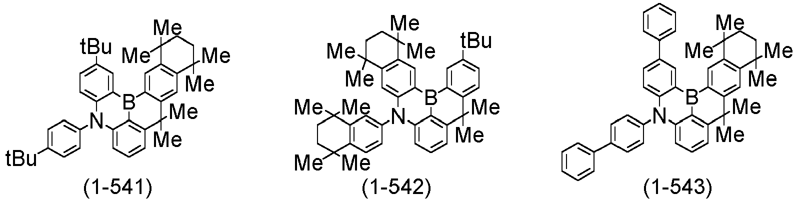

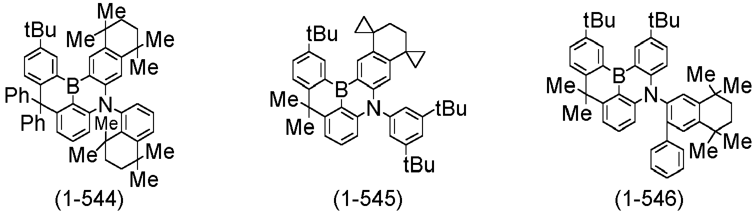

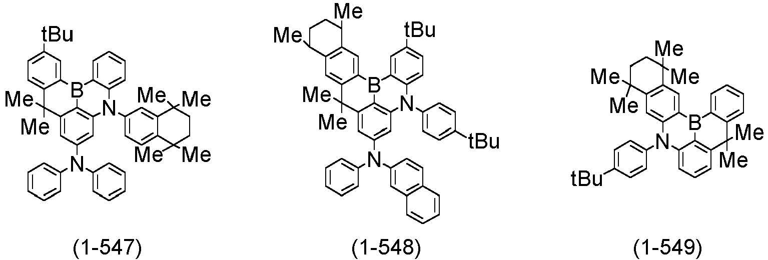

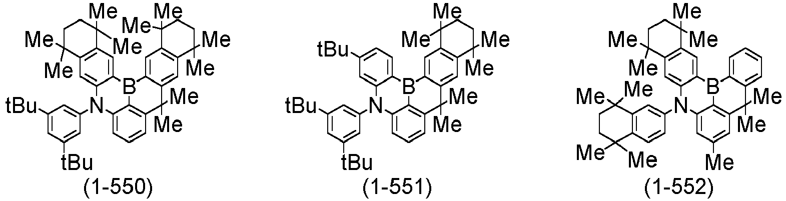

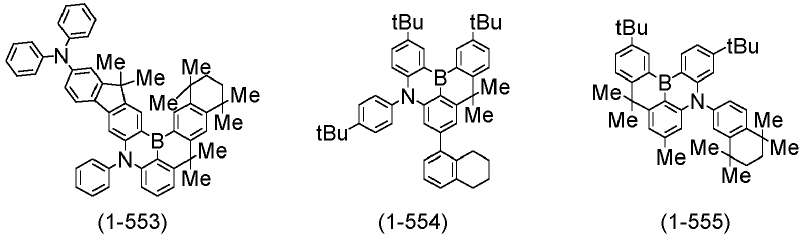

In this paragraph, all compounds except compounds (1-565) and (1-568) are inventive compounds.

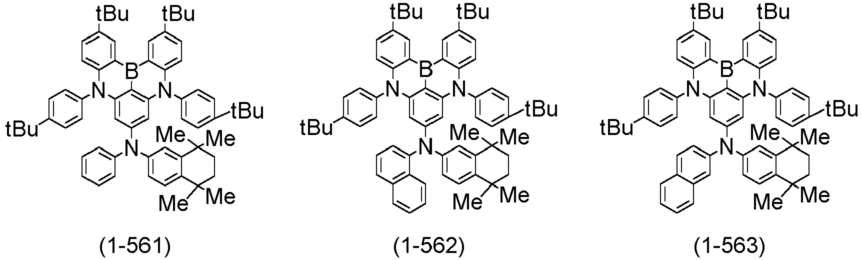

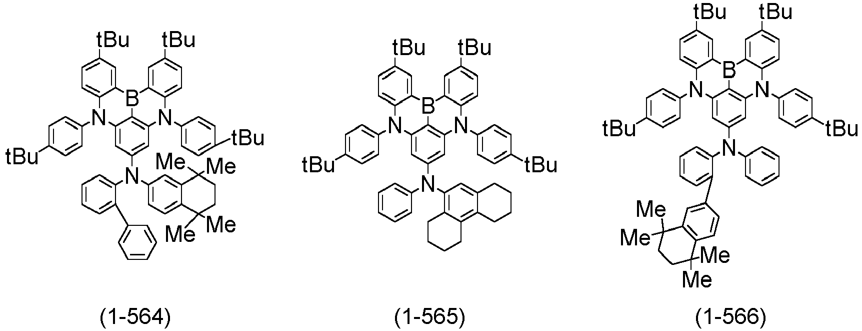

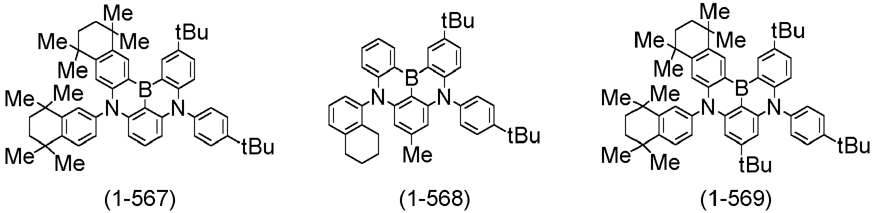

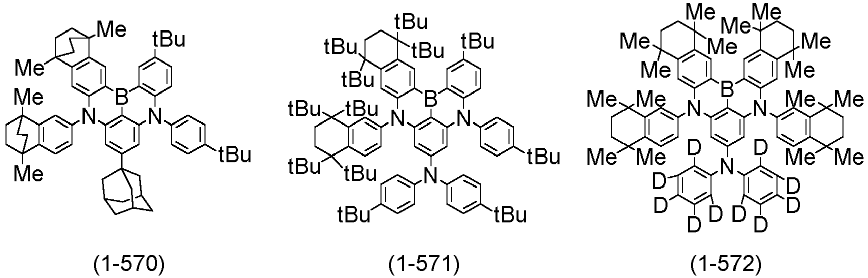

In this paragraph, all compounds except compounds (1-582), (1-583), (1-584) are inventive compounds.

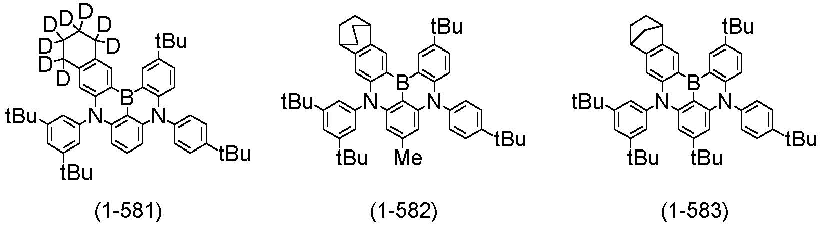

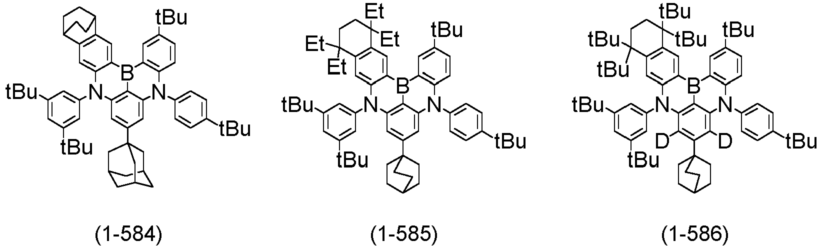

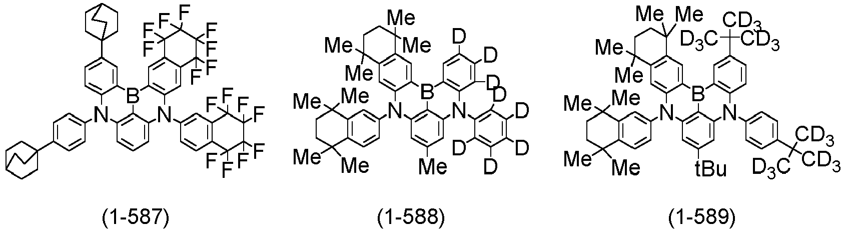

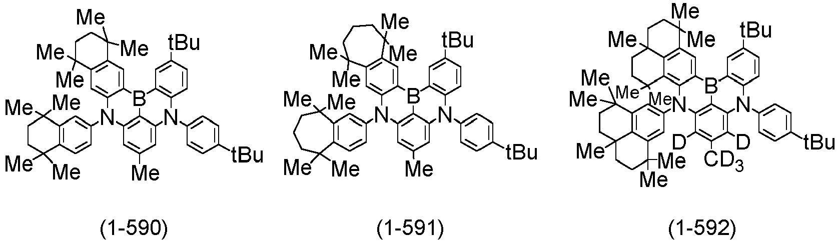

In this paragraph, all compounds except compound (1-613) are inventive compounds.

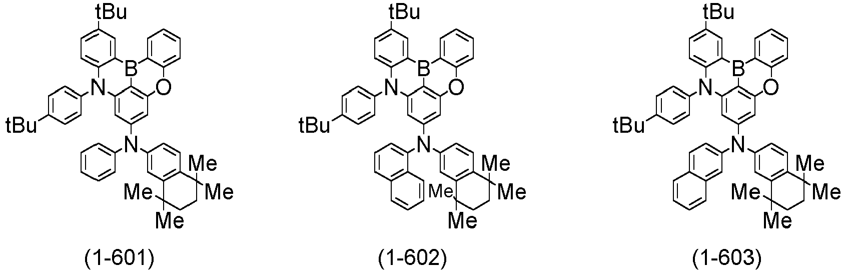

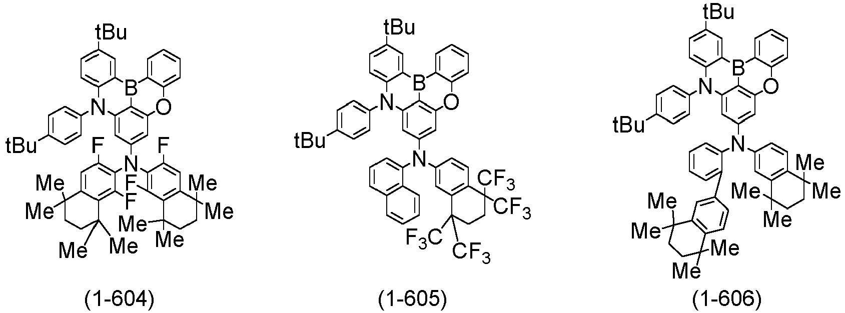

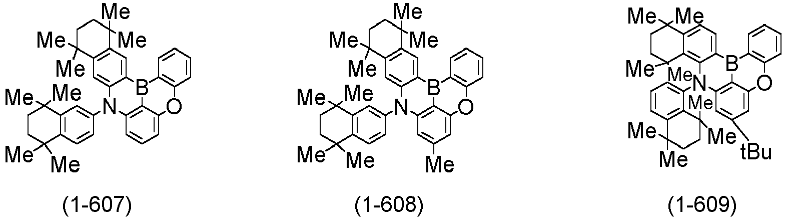

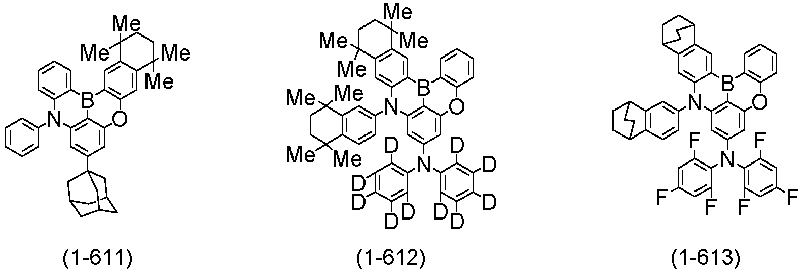

In this paragraph, all compounds except compound (1-631) are inventive compounds.

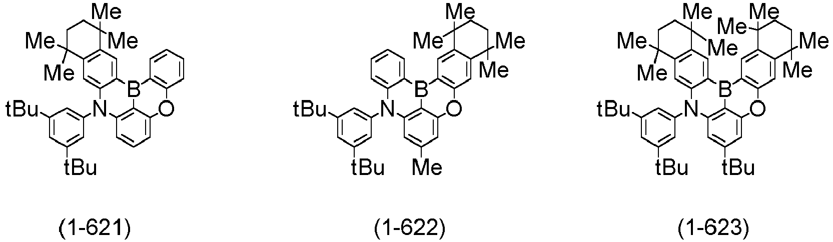

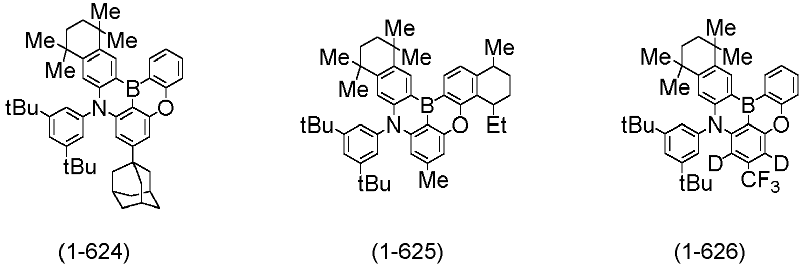

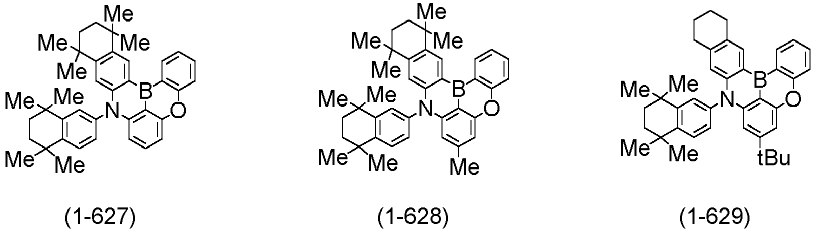

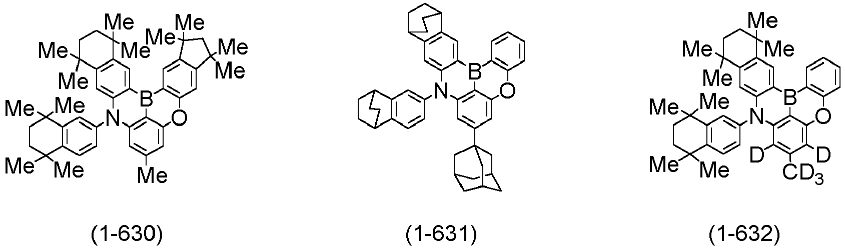

In this paragraph, all compounds are inventive compounds.

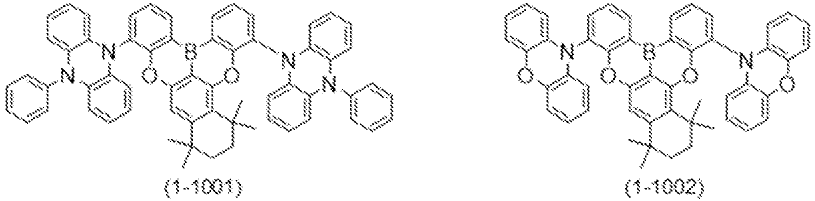

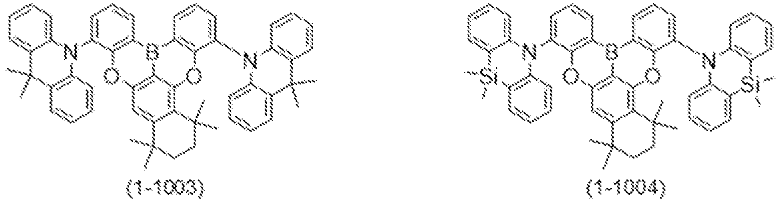

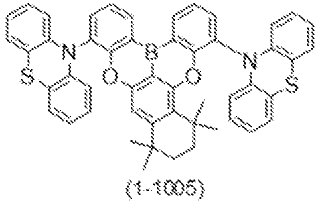

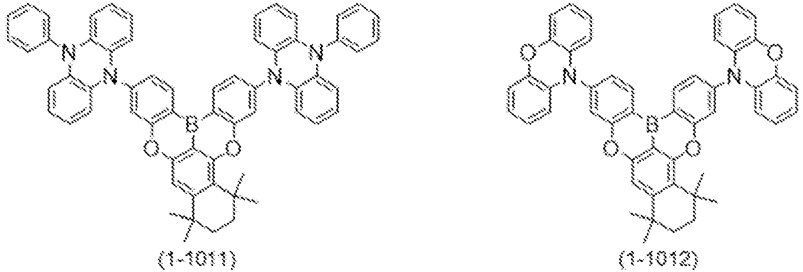

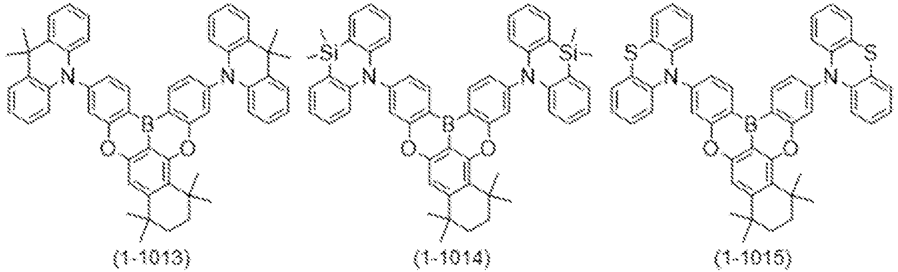

In this paragraph, all compounds are inventive compounds.

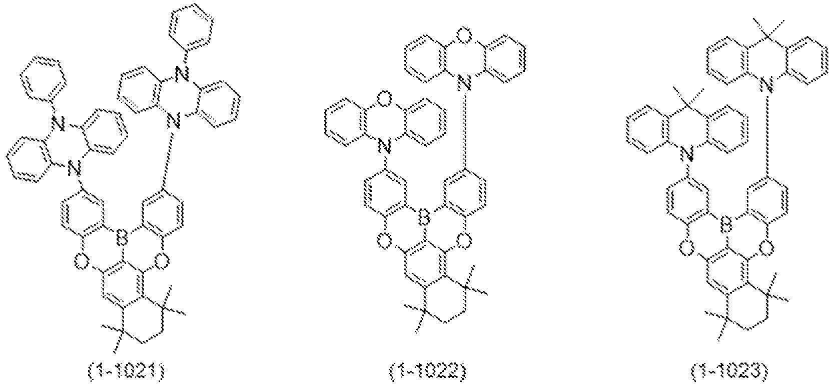

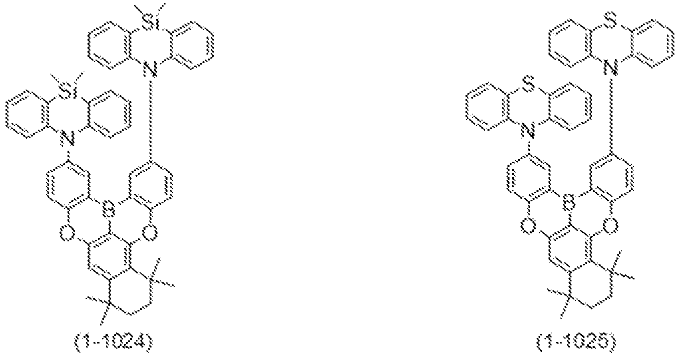

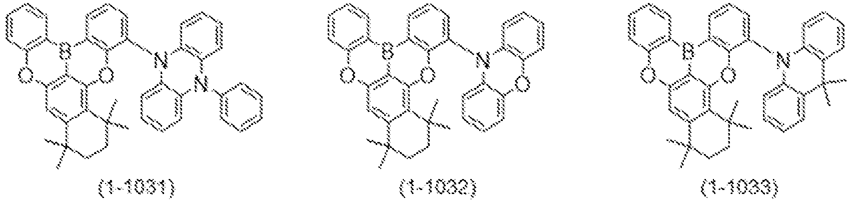

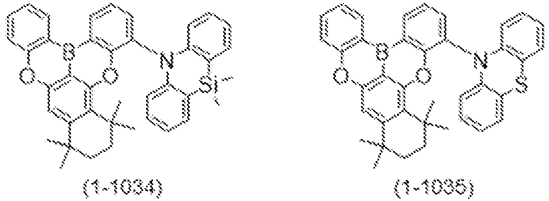

In this paragraph, all compounds are inventive compounds.

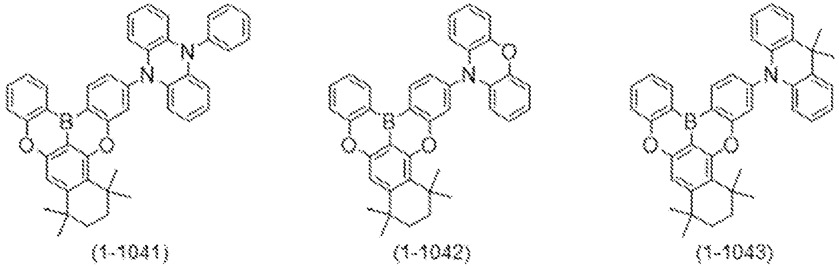

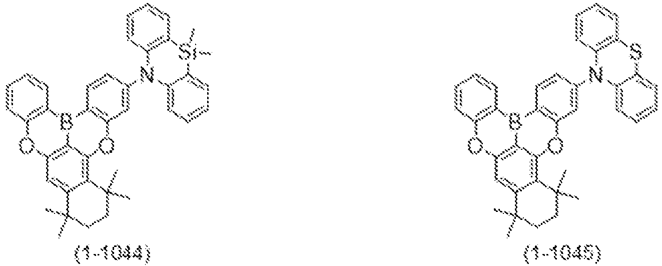

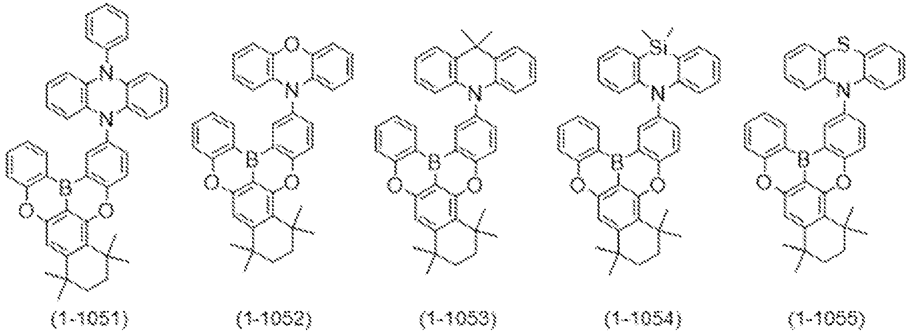

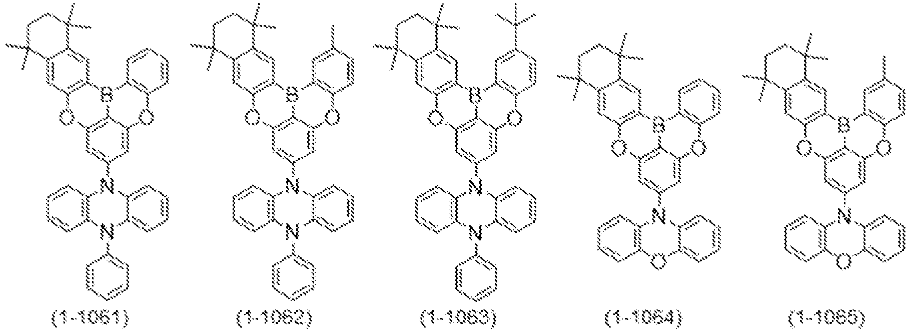

In this paragraph, all compounds are inventive compounds.

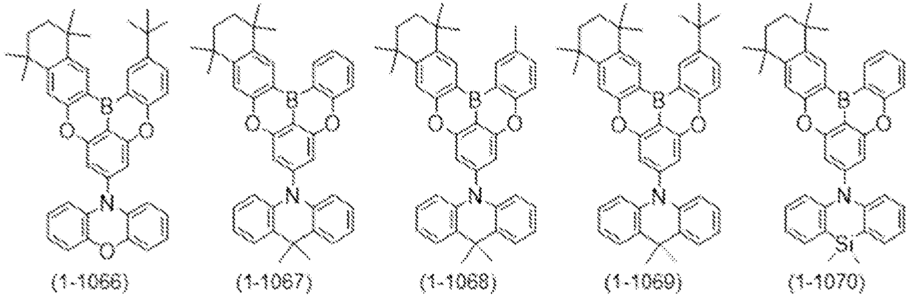

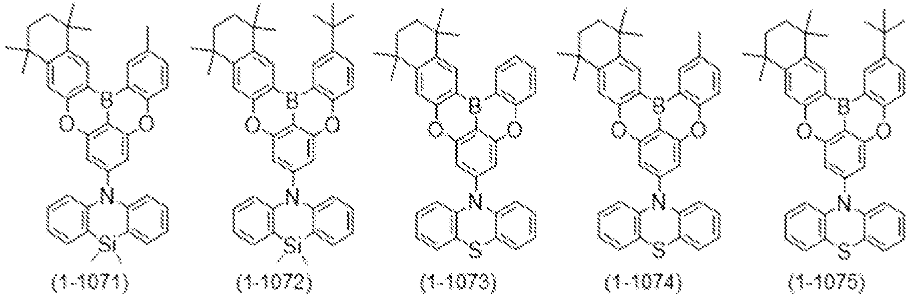

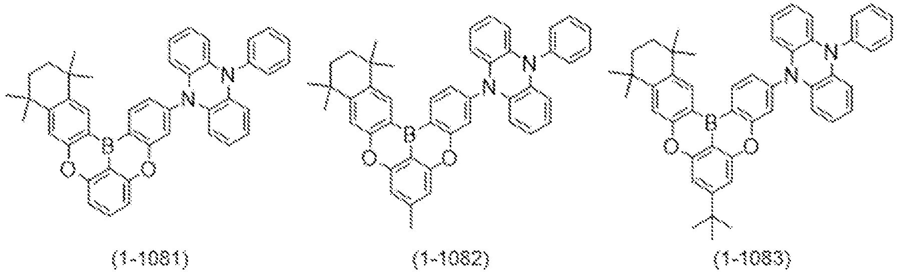

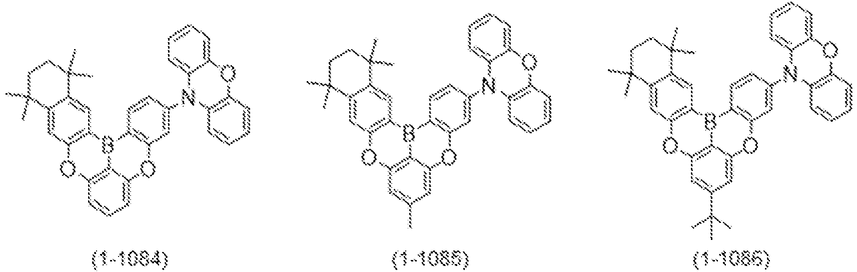

In this paragraph, all compounds are inventive compounds.

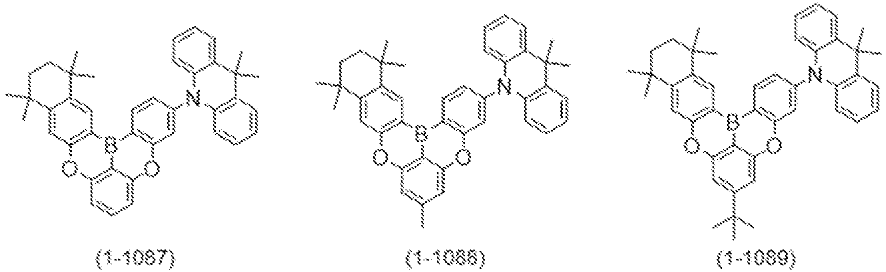

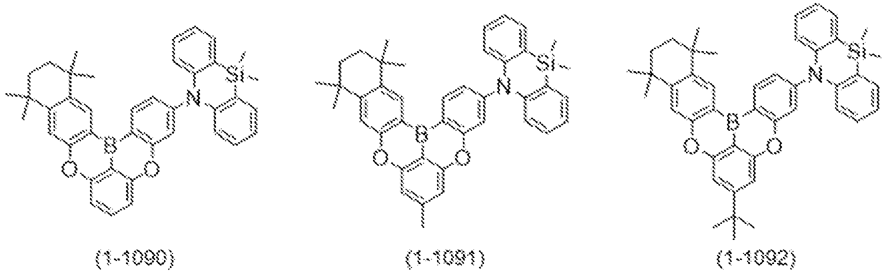

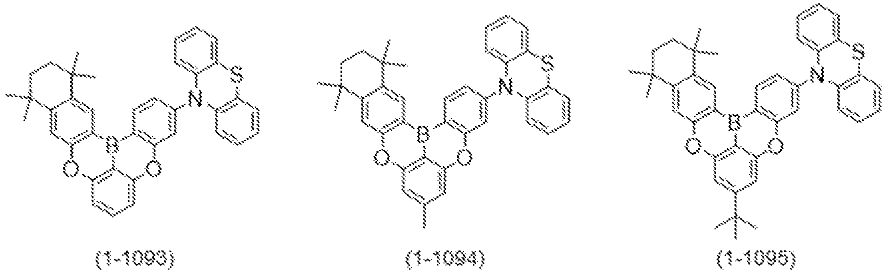

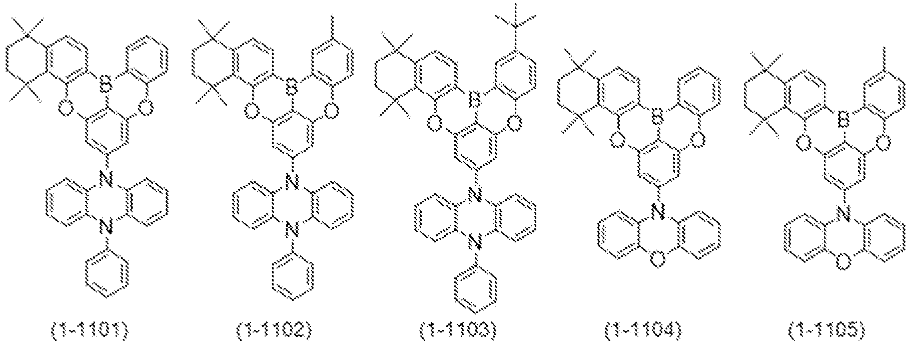

In this paragraph, all compounds are inventive compounds.

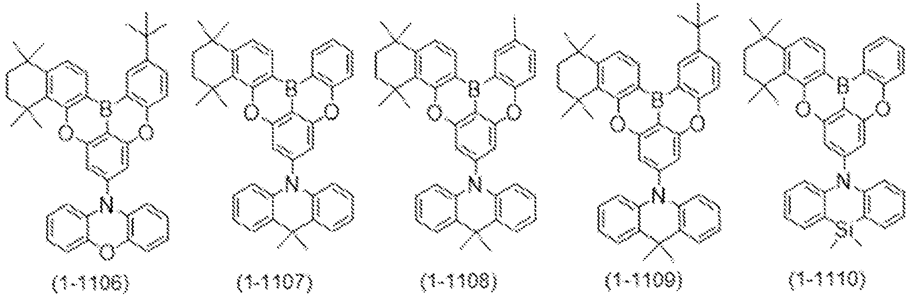

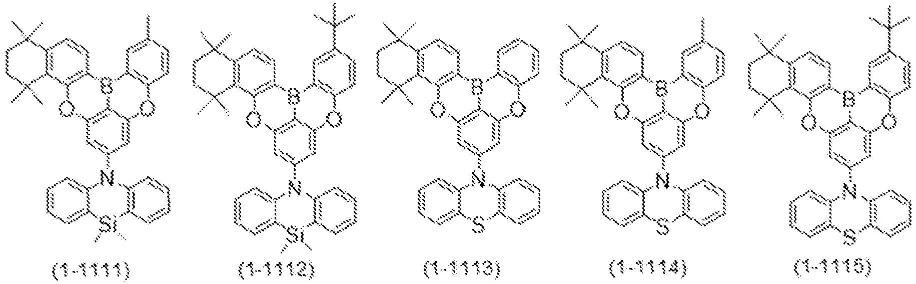

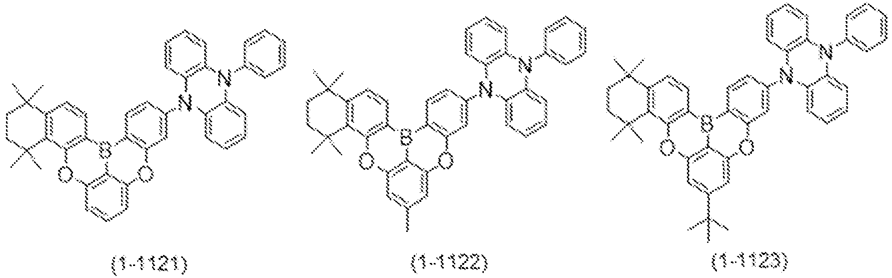

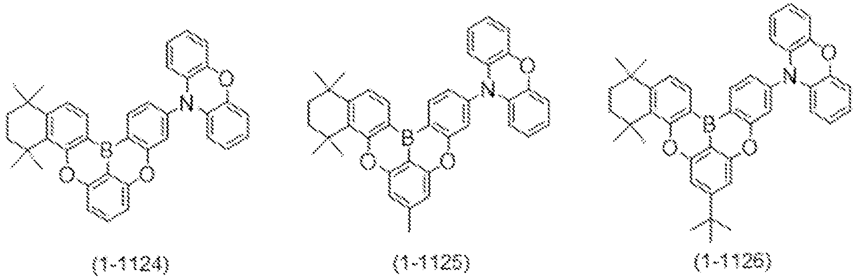

In this paragraph, all compounds except compounds (1-1141) and (1-1142) are inventive compounds.

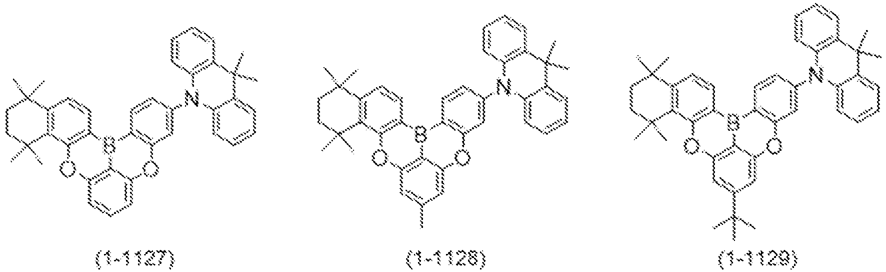

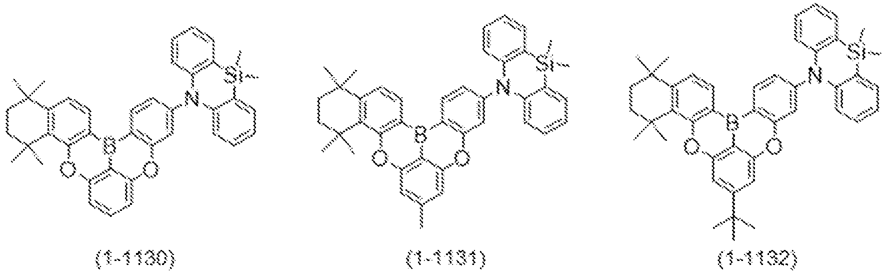

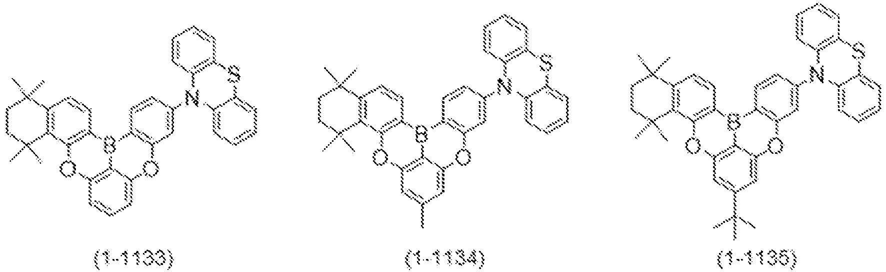

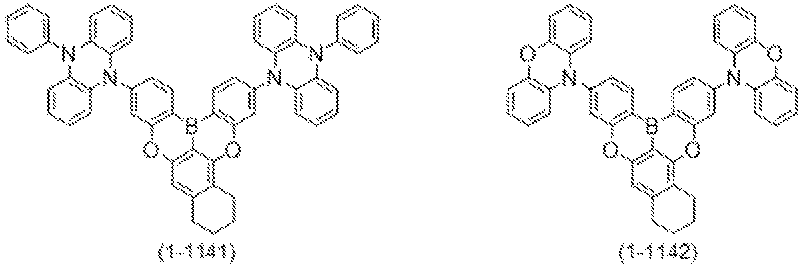

In this paragraph, all compounds except compounds (1-1143), (1-1144) and (1-1145) are inventive compounds.

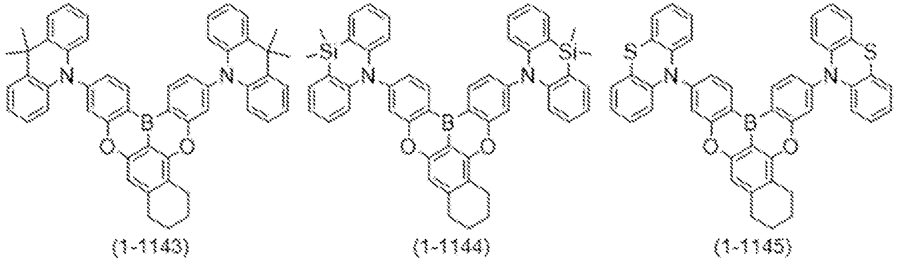

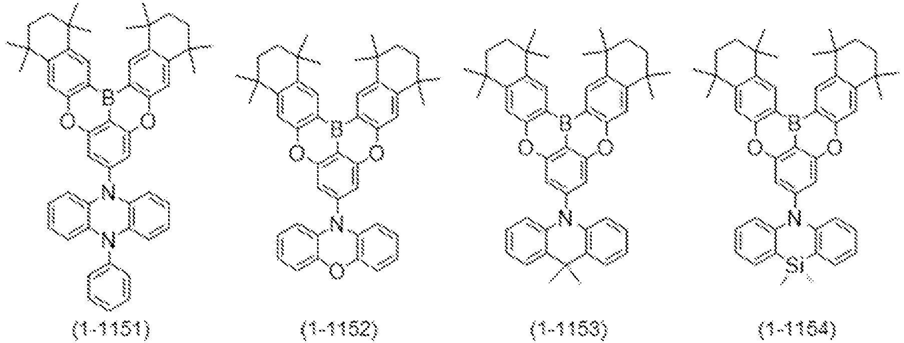

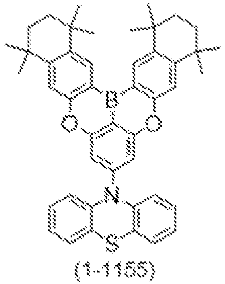

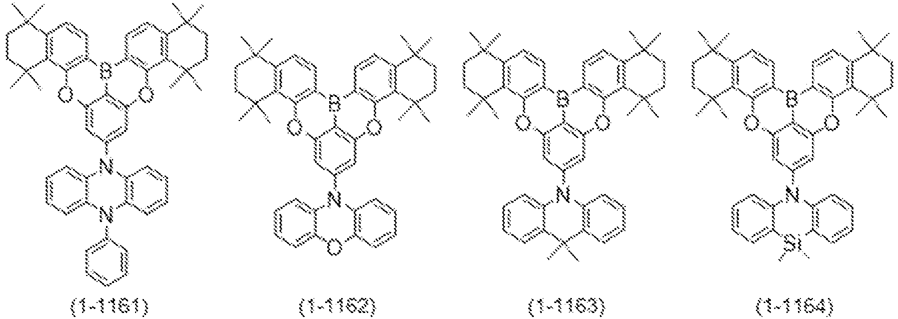

In this paragraph, all compounds are inventive compounds.

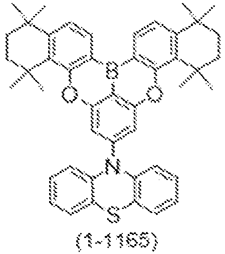

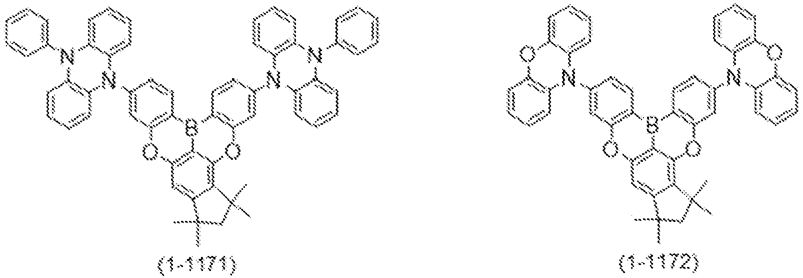

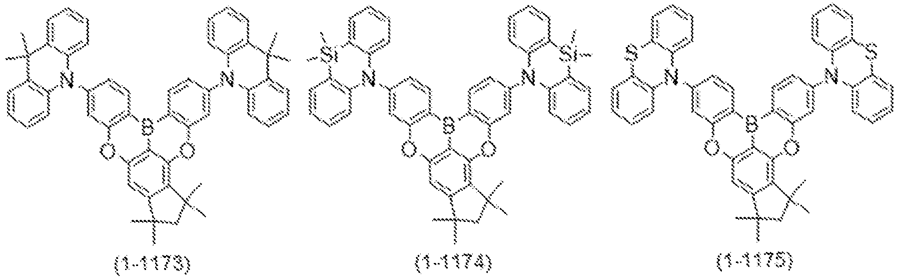

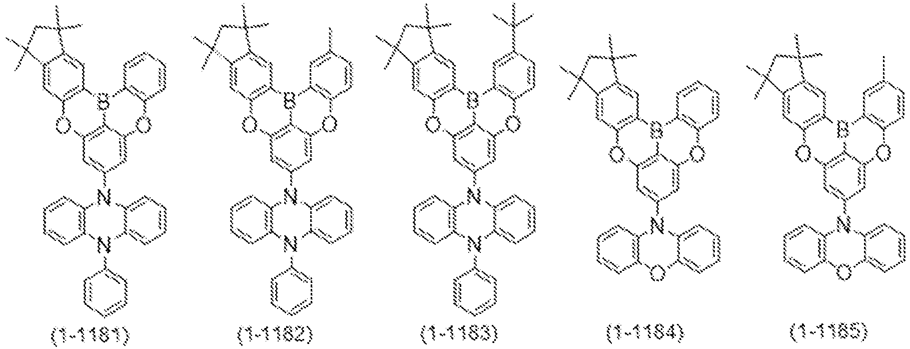

In this paragraph, all compounds are inventive compounds.

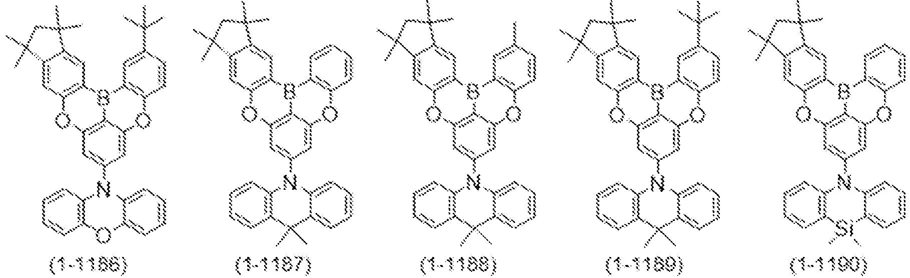

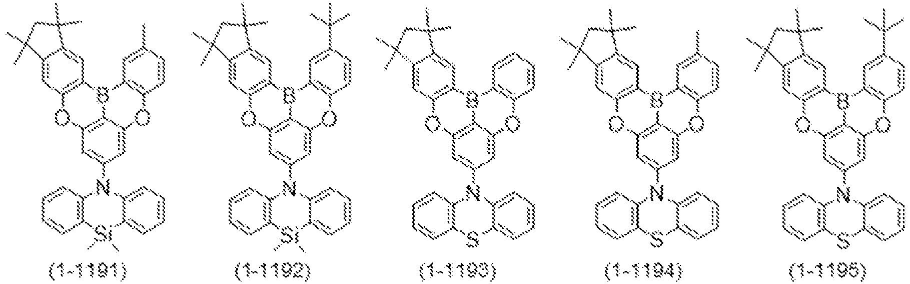

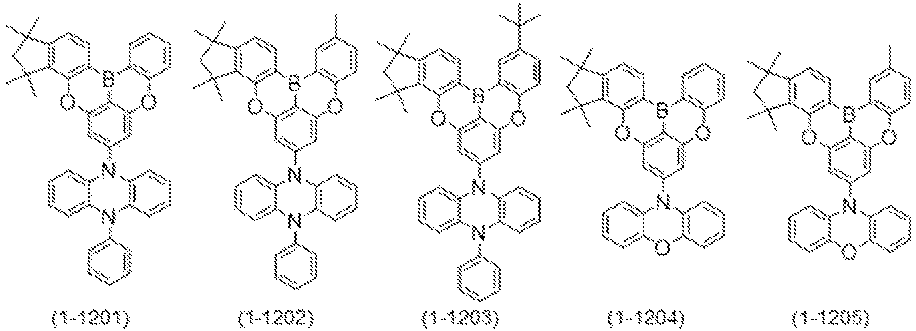

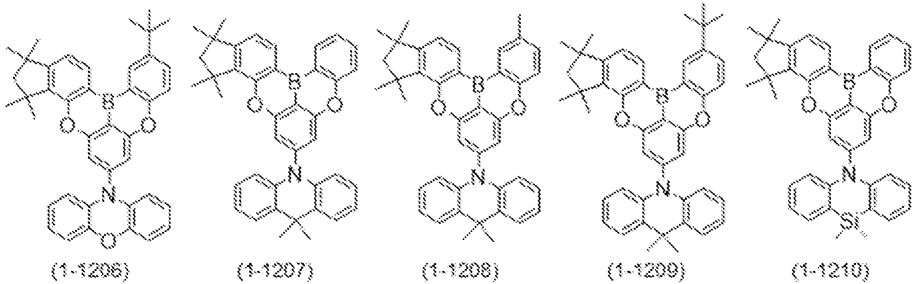

In this paragraph, all compounds are inventive compounds.

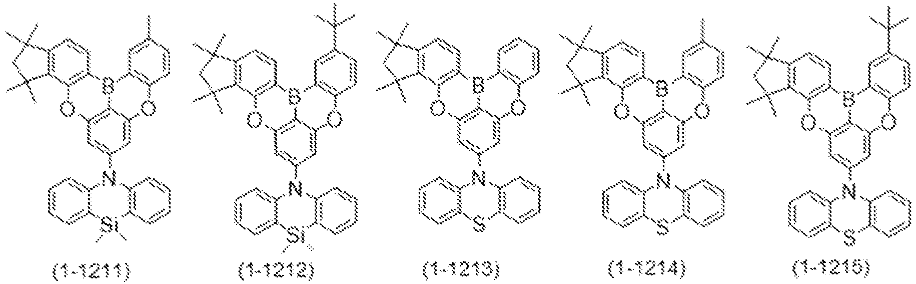

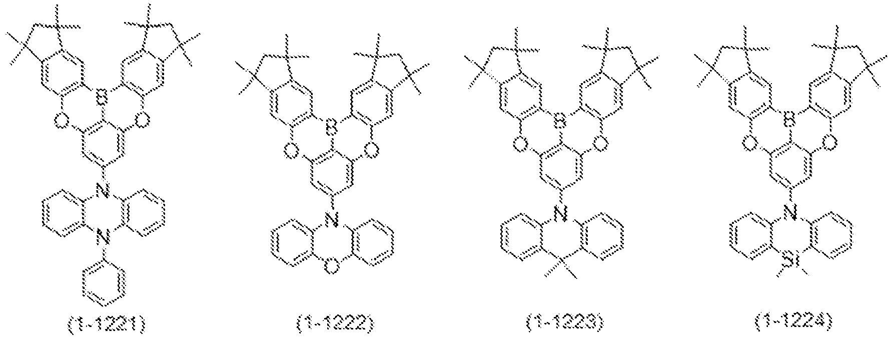

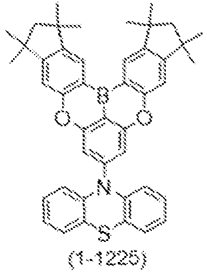

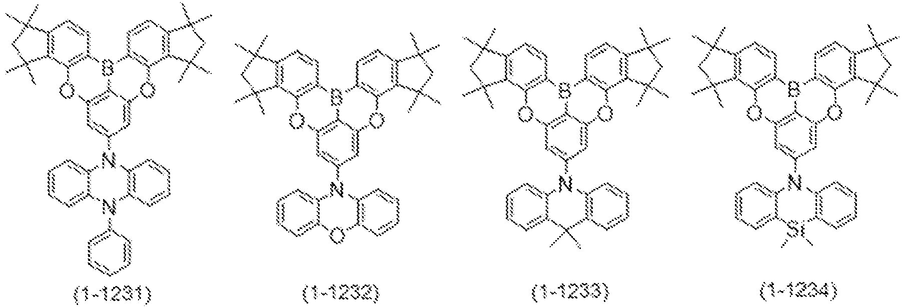

In this paragraph, compound (1-1235) is an inventive compound. The remaining compounds are comparative compounds.

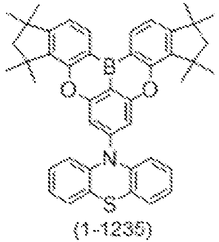

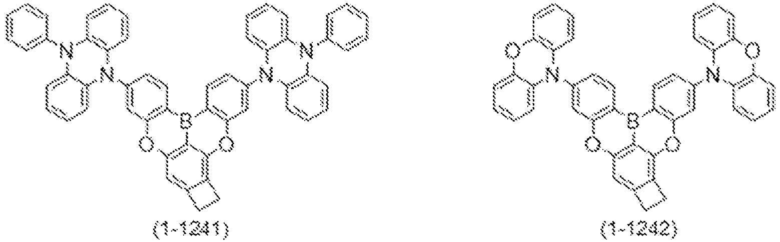

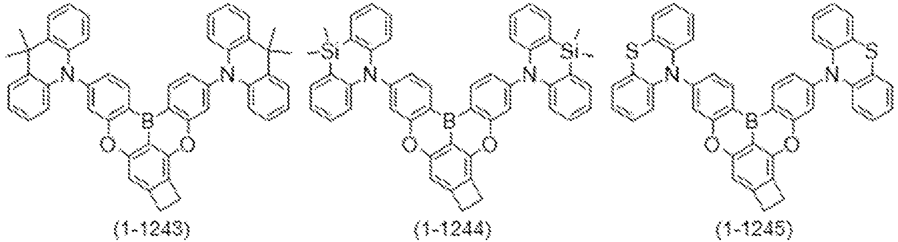

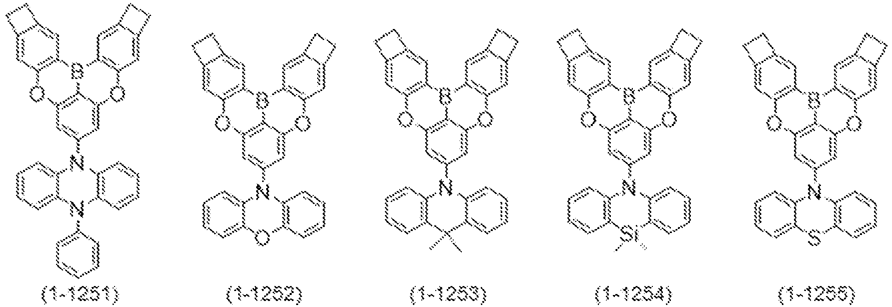

In this paragraph, the compounds are comparative compounds.

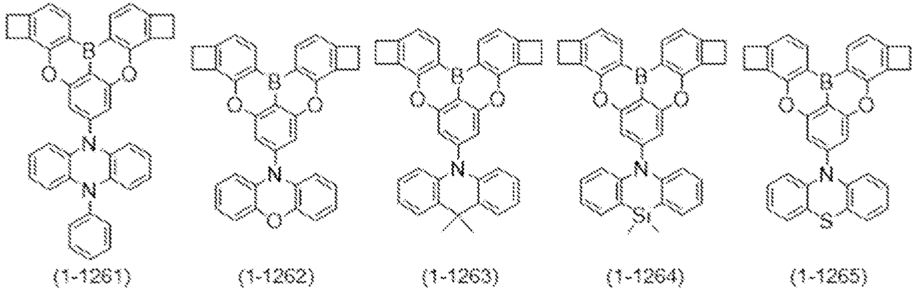

In this paragraph, all compounds are inventive compounds.

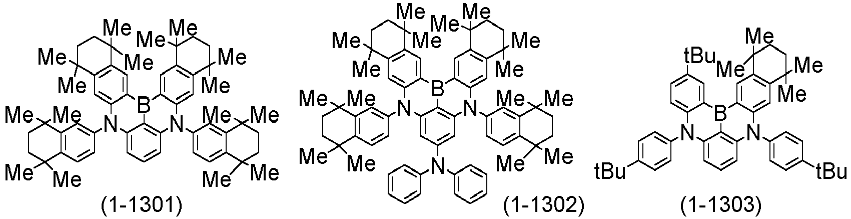

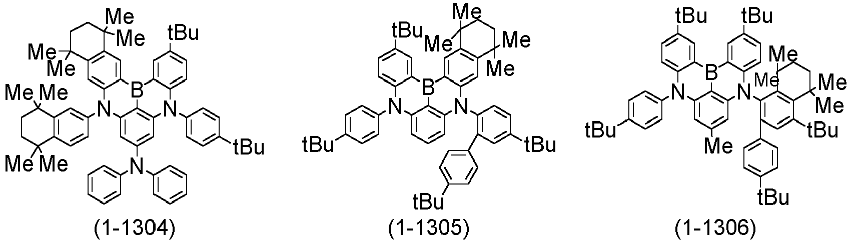

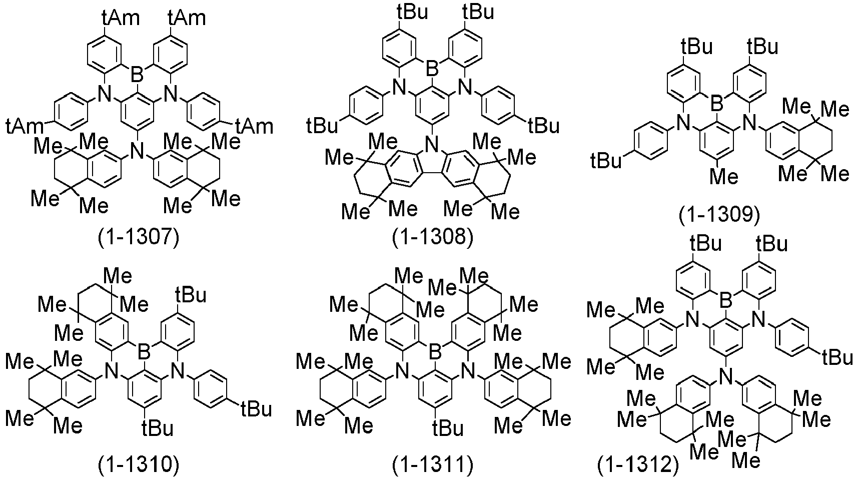

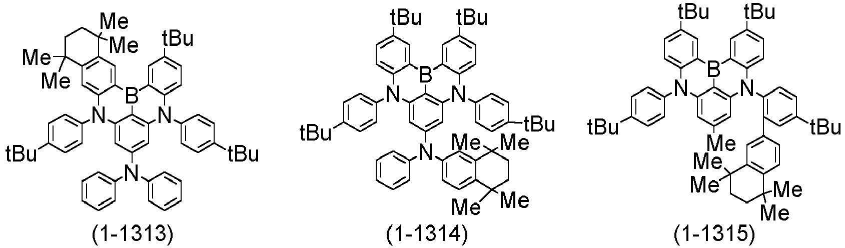

In this paragraph, all compounds except compound (1-1333) are inventive compounds.

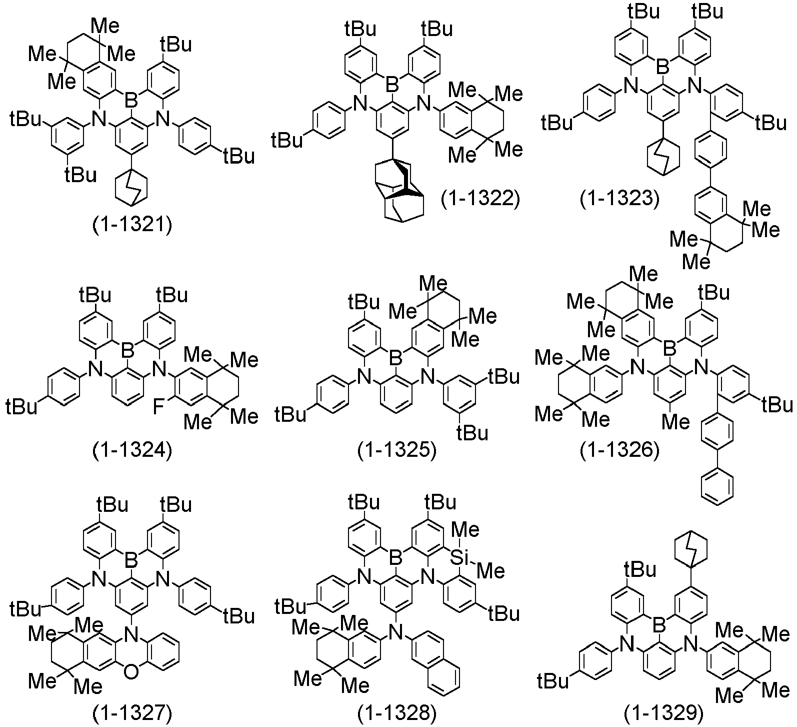

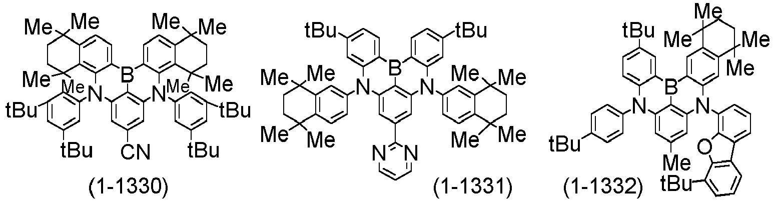

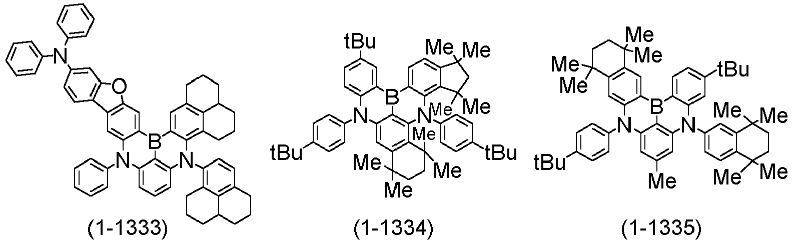

In this paragraph, compound (1-1405) is an inventive compound. The remaining compounds are comparative compounds.

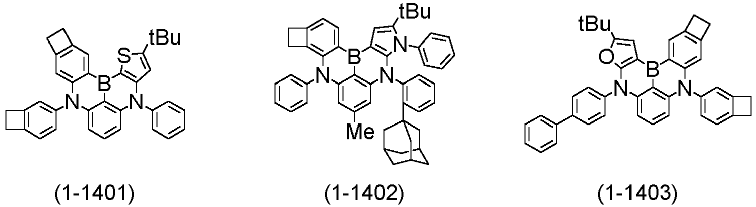

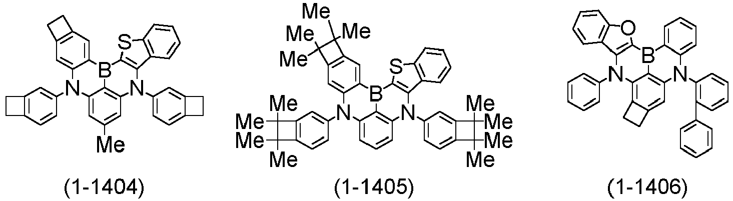

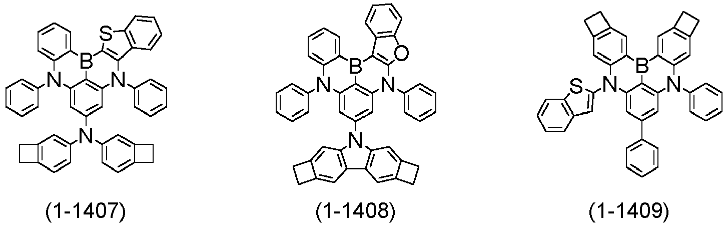

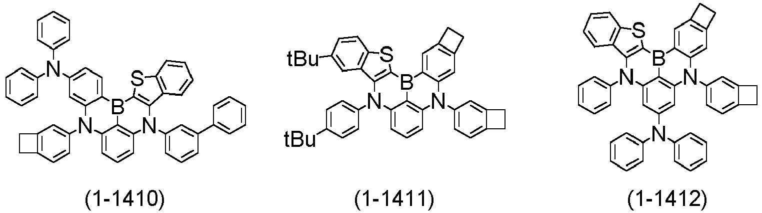

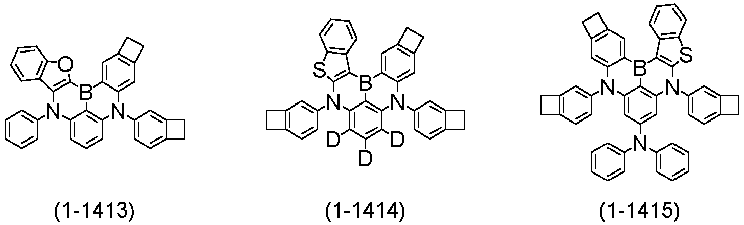

In this paragraph, all compounds are comparative compounds.

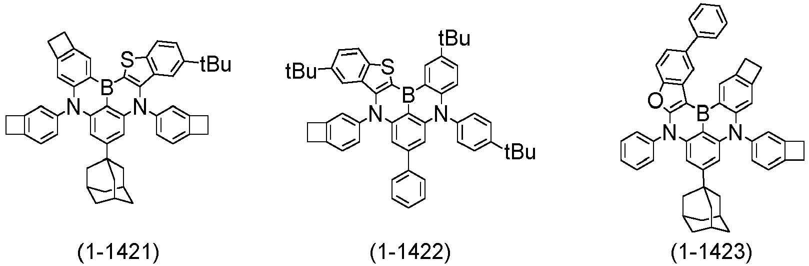

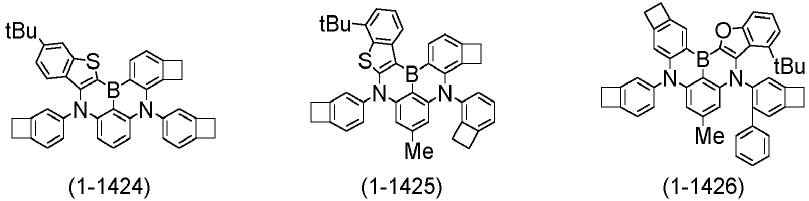

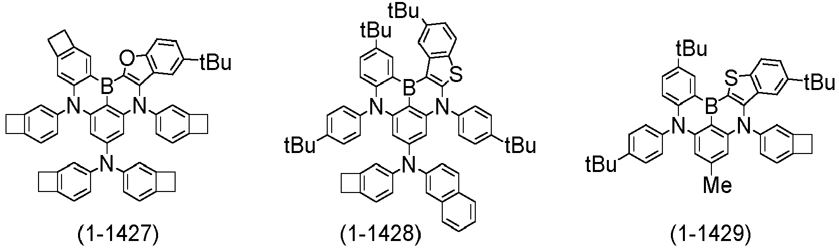

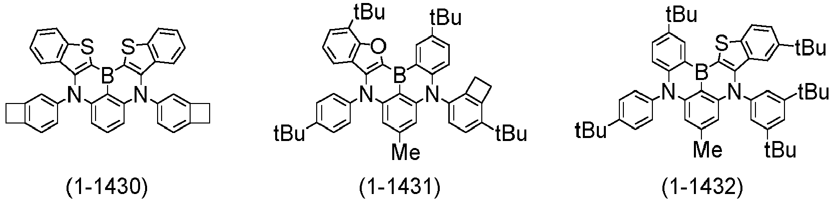

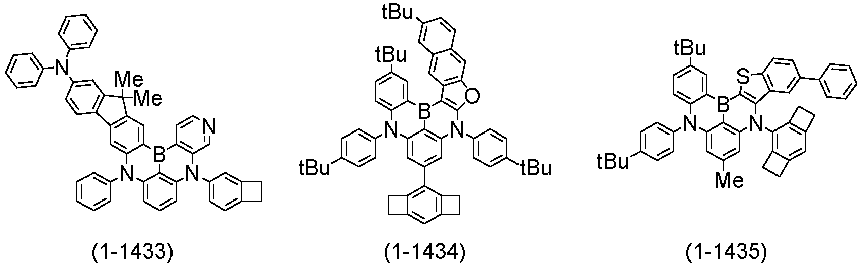

In this paragraph, compound (1-1454) is an inventive compound. The remaining compounds are comparative compounds.

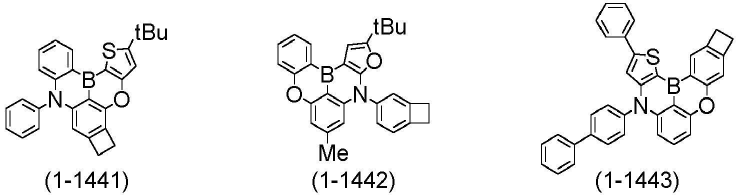

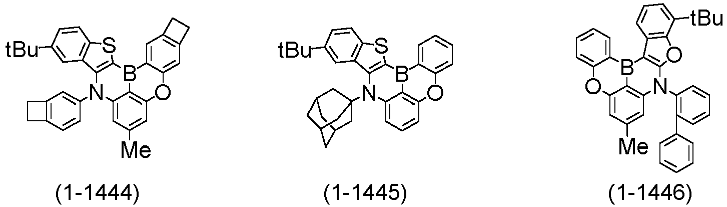

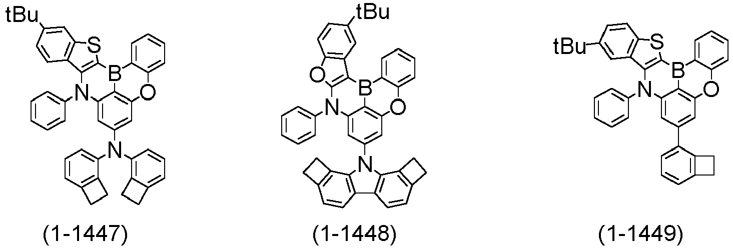

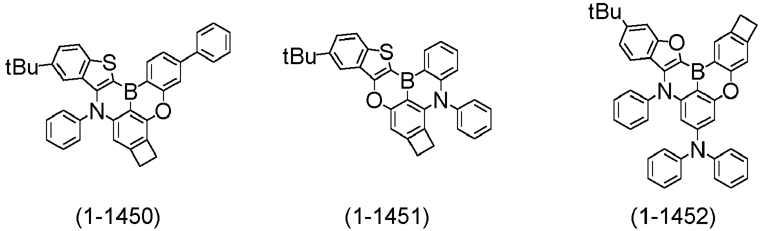

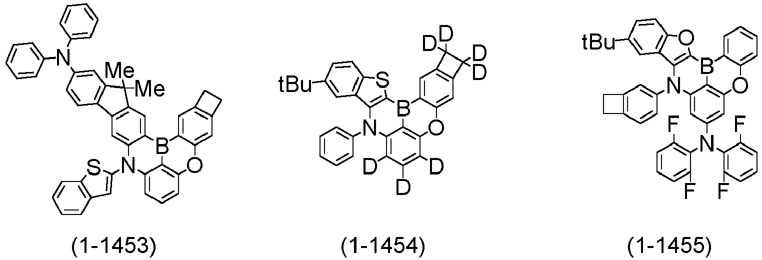

In this paragraph, compound (1-1465) is an inventive compound. The remaining compounds are comparative compounds.

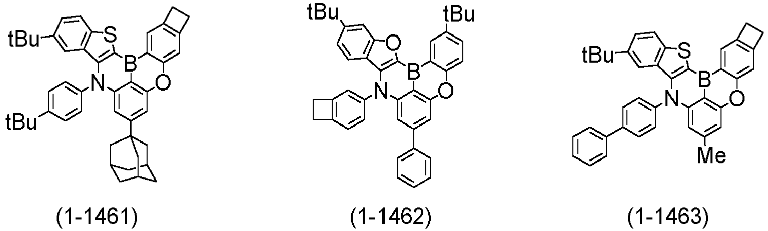

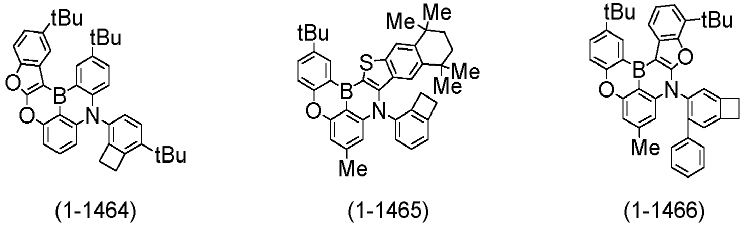

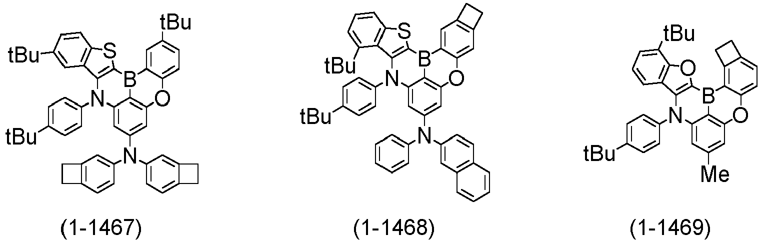

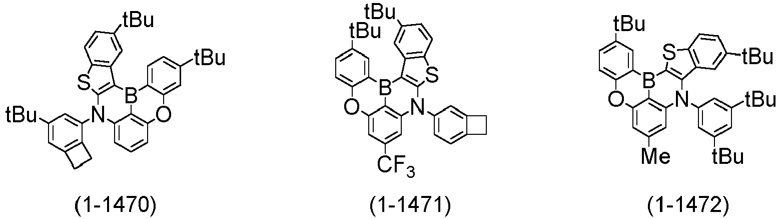

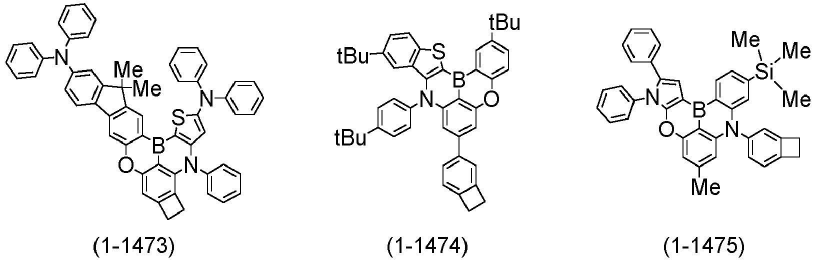

In this paragraph all compounds are comparative compounds.

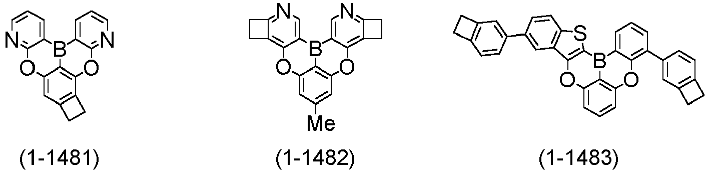

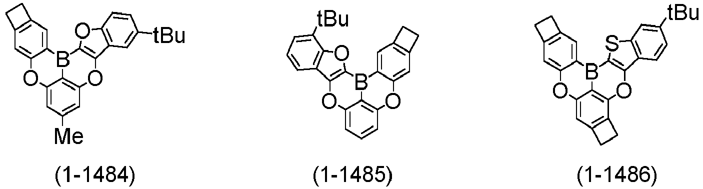

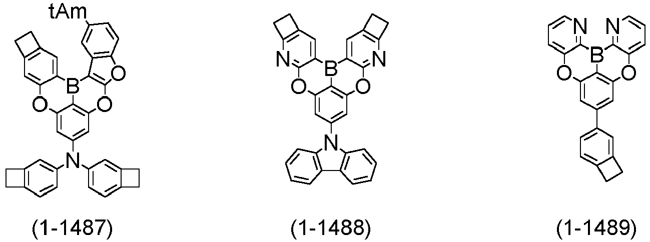

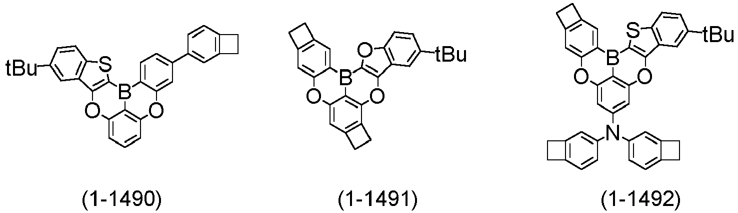

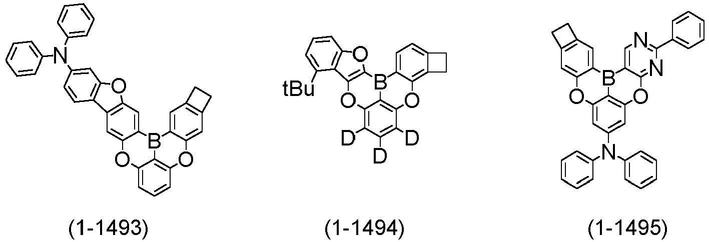

In this paragraph all compounds are comparative compounds.

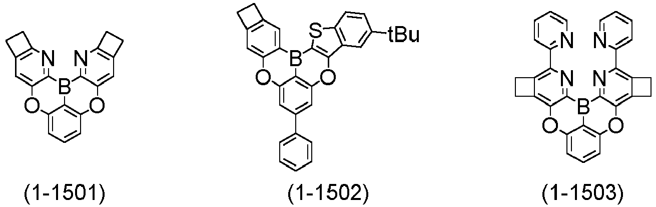

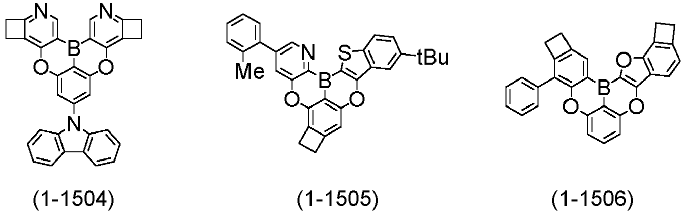

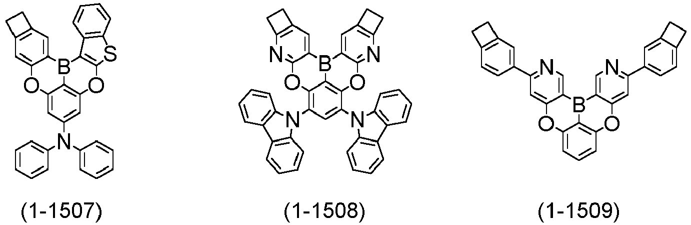

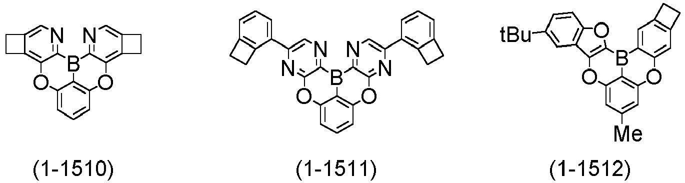

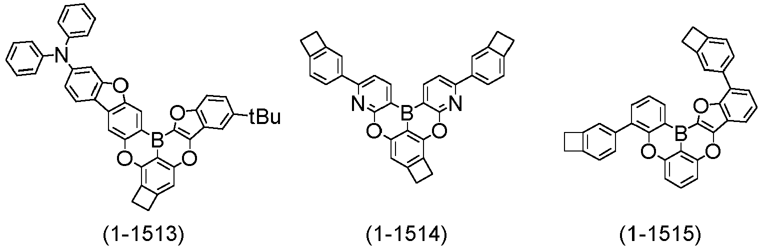

In this paragraph all compounds are comparative compounds.

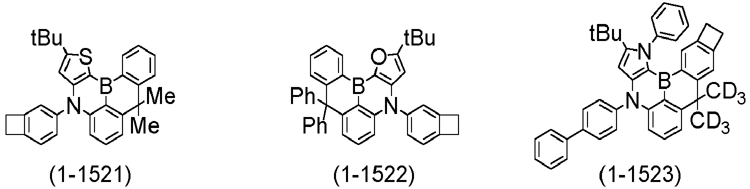

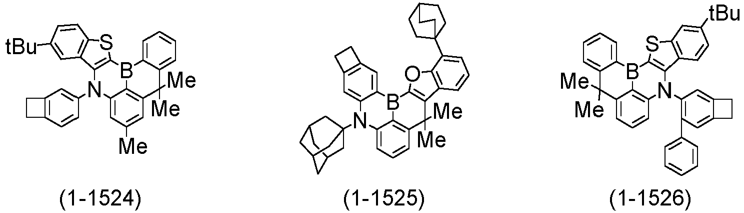

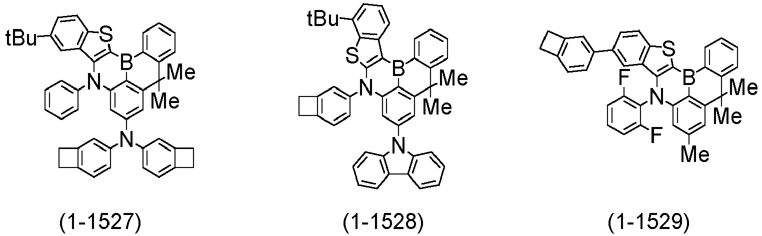

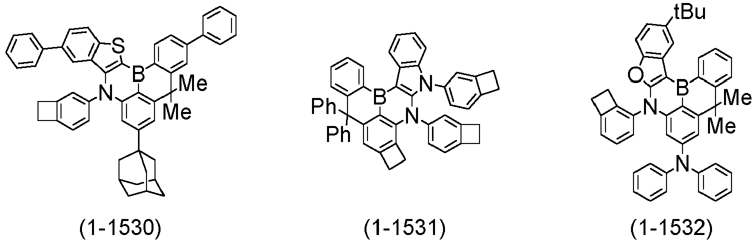

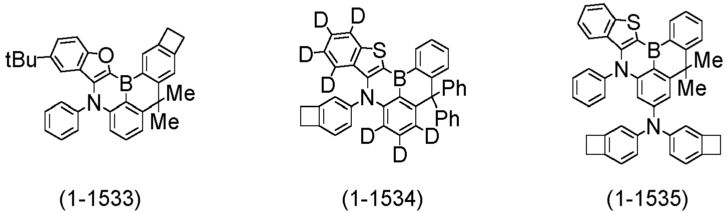

In this paragraph all compounds are comparative compounds.

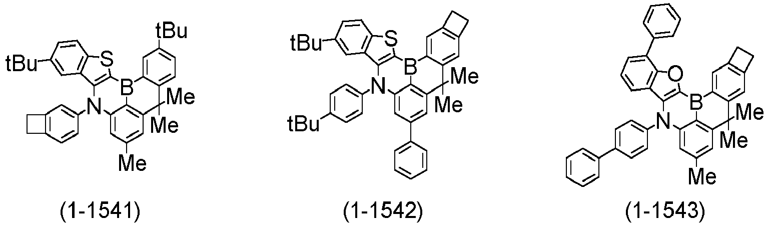

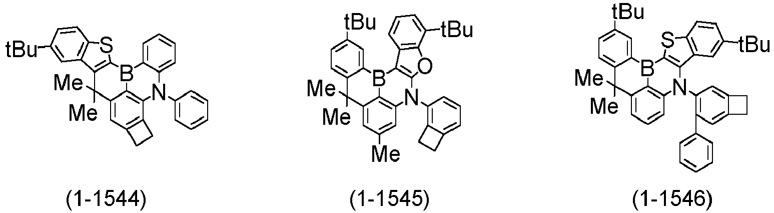

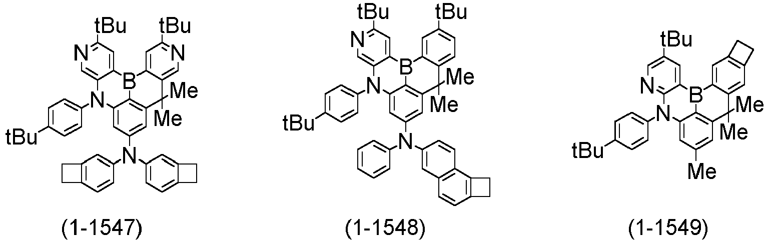

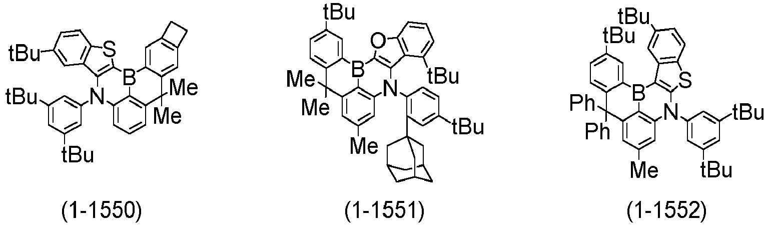

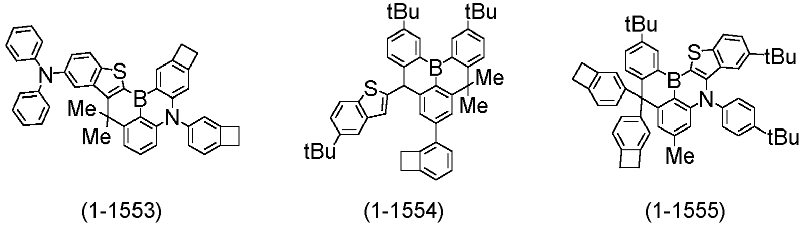

In this paragraph all compounds except compound (1-1605) are inventive compounds.

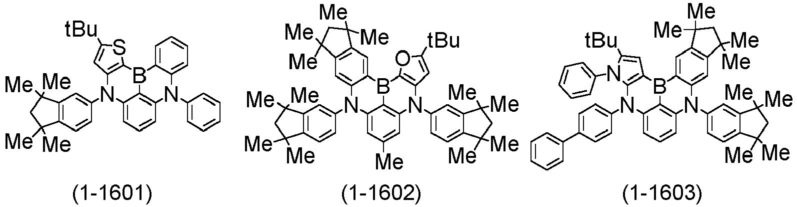

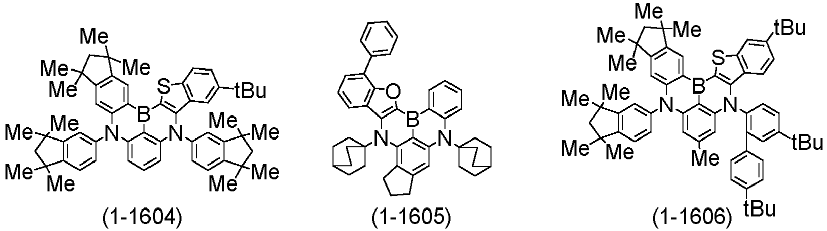

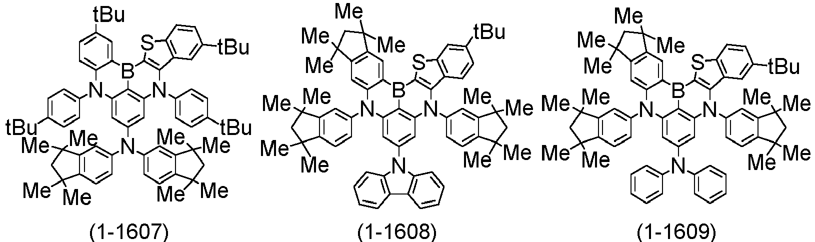

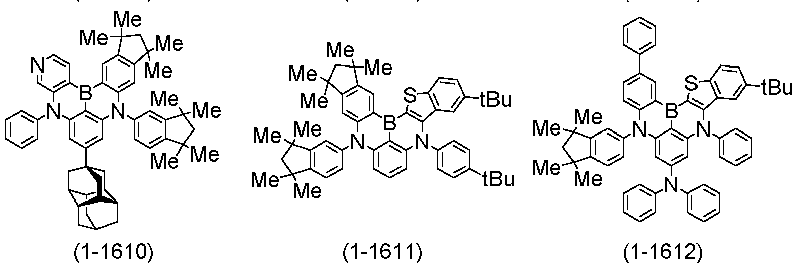

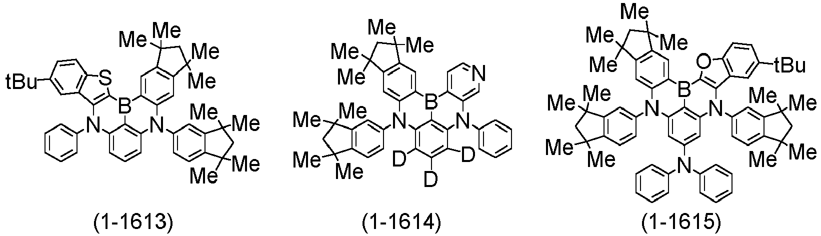

In this paragraph all compounds are inventive compounds.

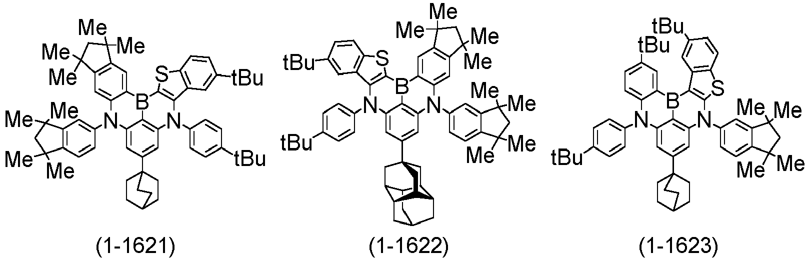

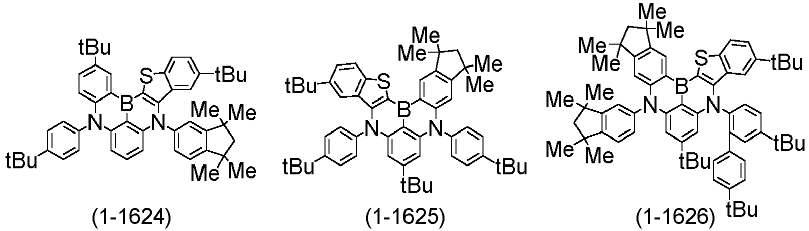

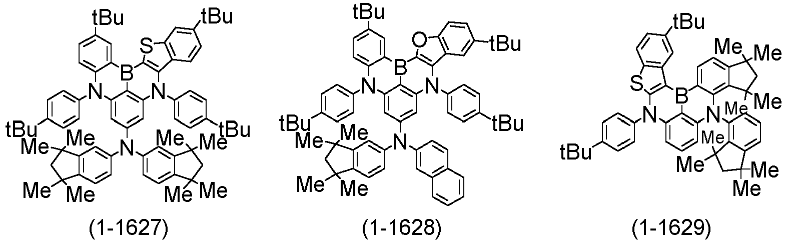

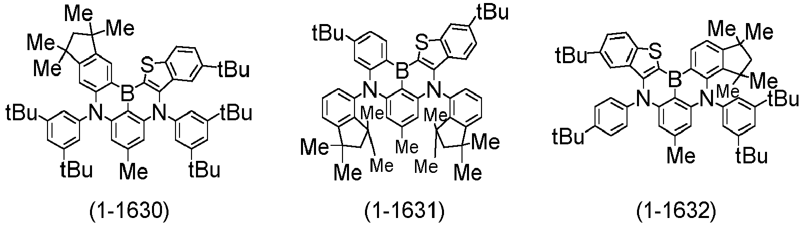

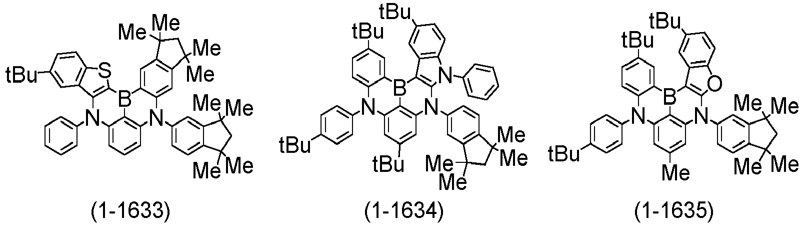

In this paragraph all compounds are inventive compounds.

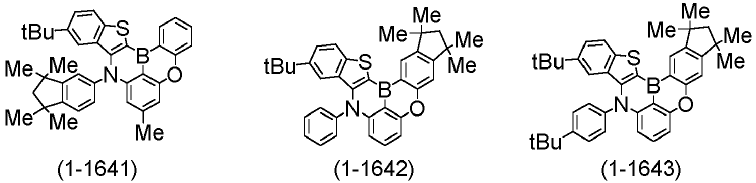

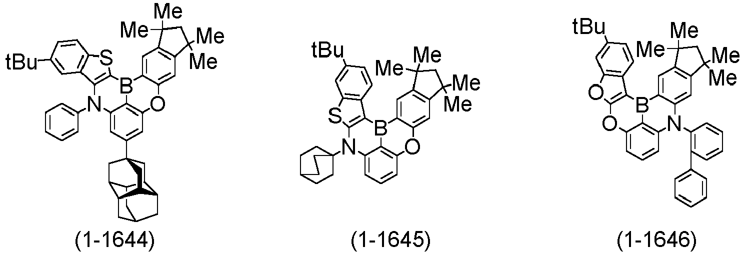

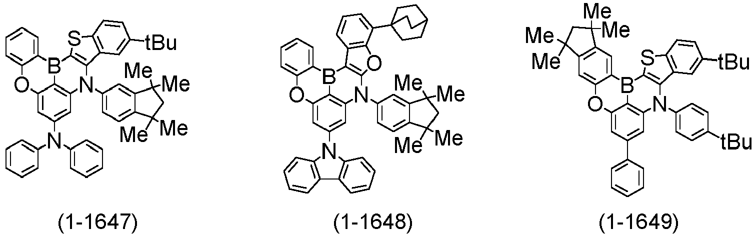

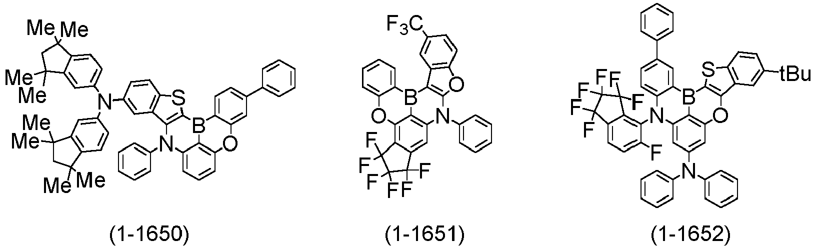

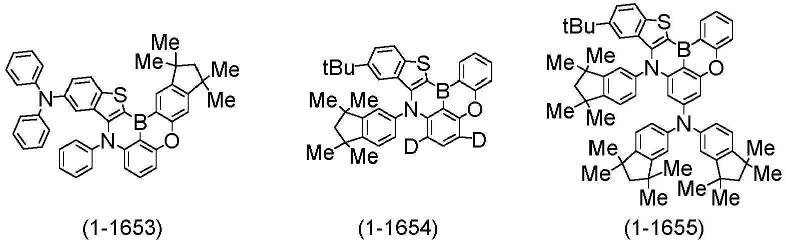

In this paragraph all compounds except compounds (1-1668) and (1-1674) are inventive compounds.

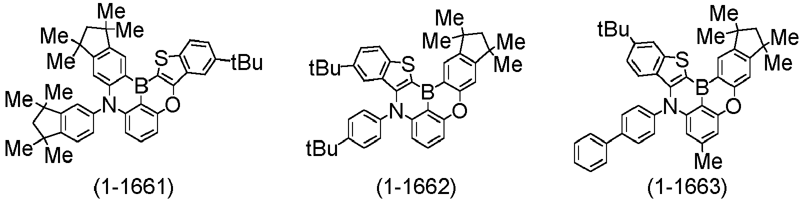

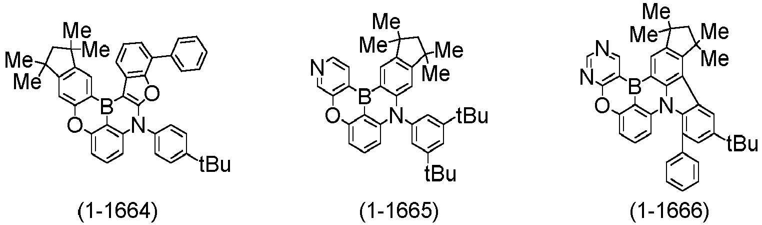

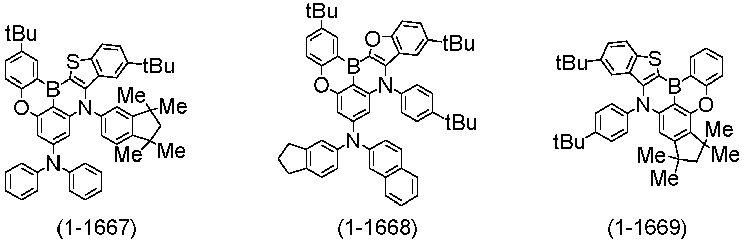

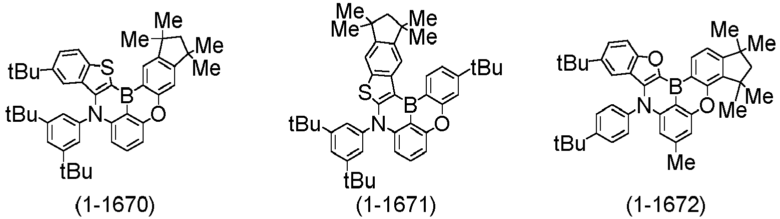

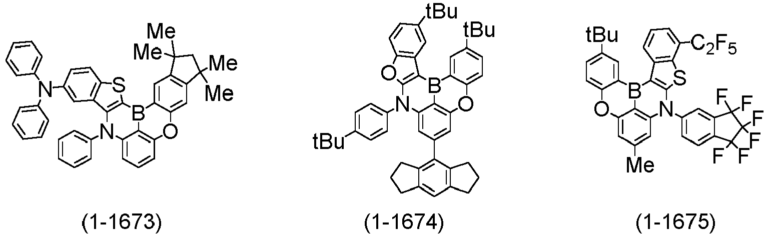

In this paragraph all compounds except compound (1-1686) are inventive compounds.

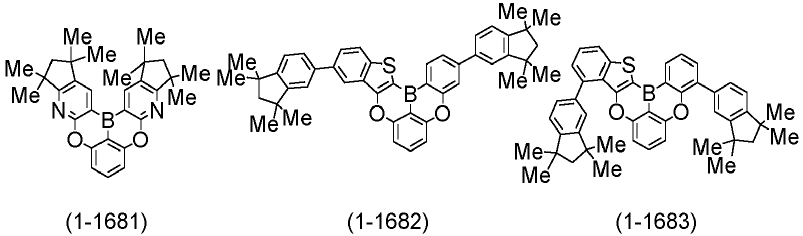

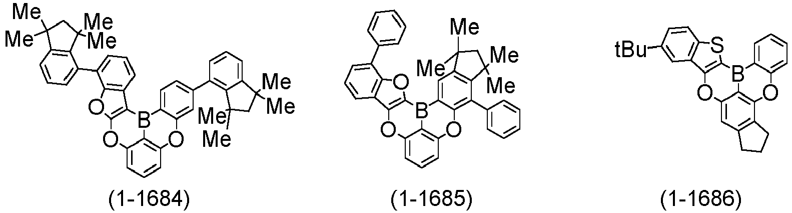

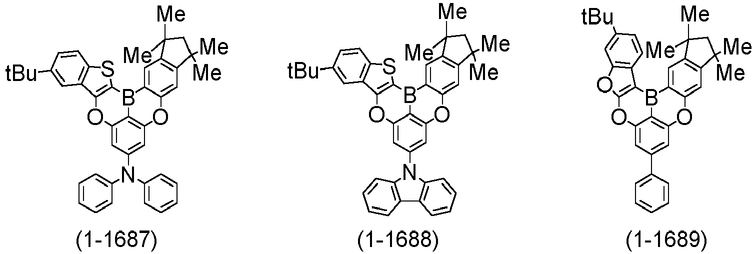

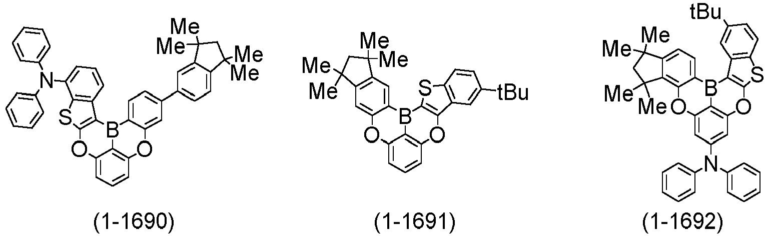

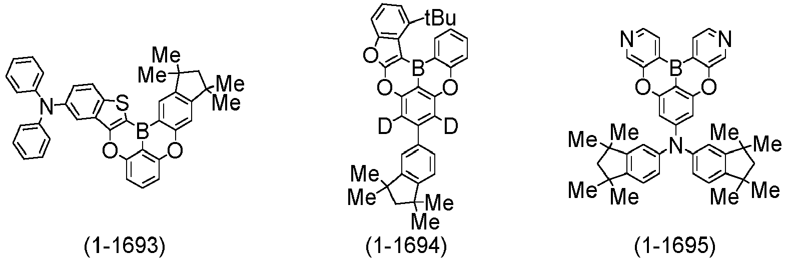

In this paragraph all compounds except compounds (1-1710) and (1-1711) are inventive compounds.

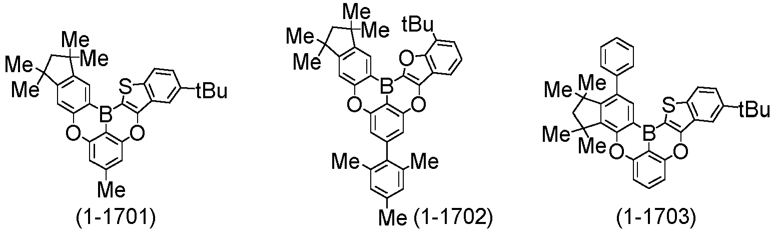

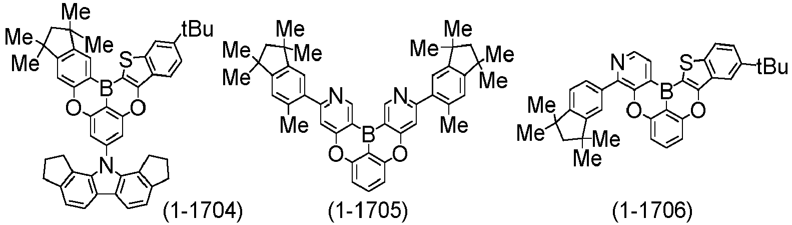

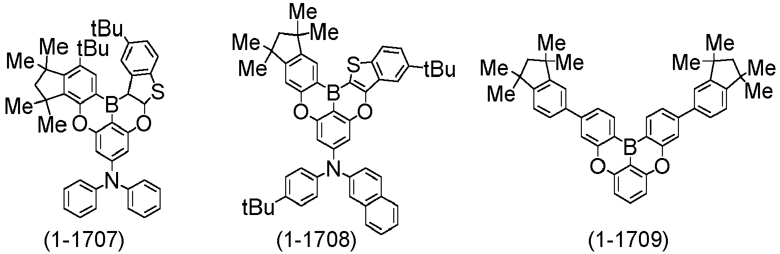

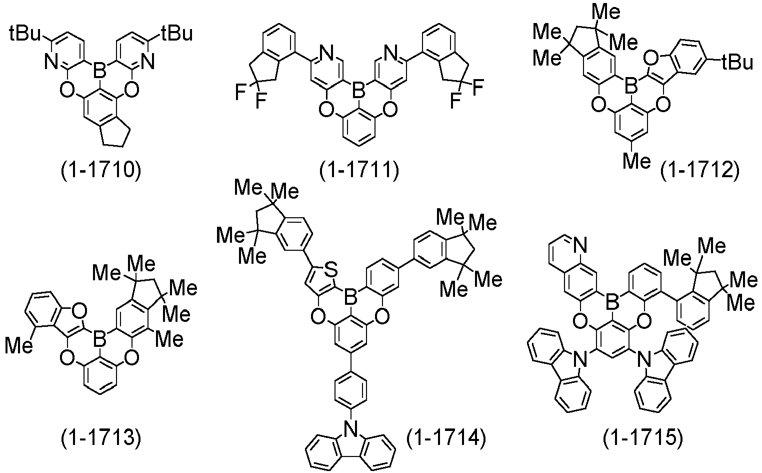

In this paragraph all compounds are inventive compounds.

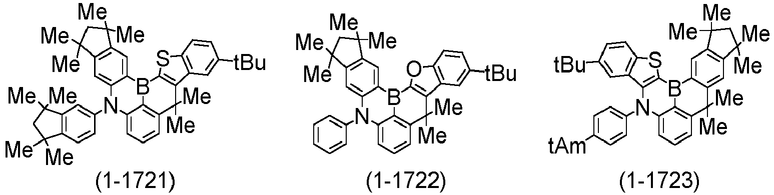

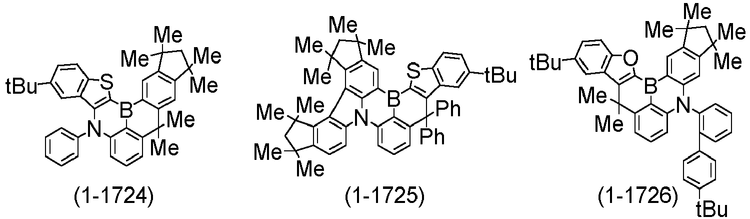

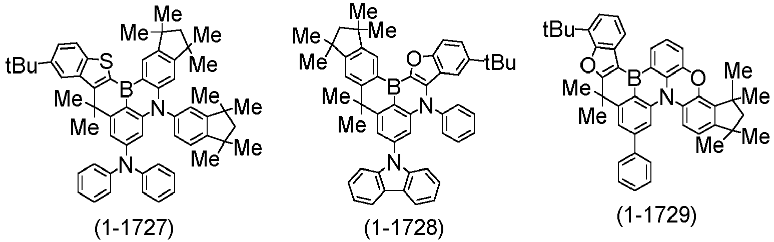

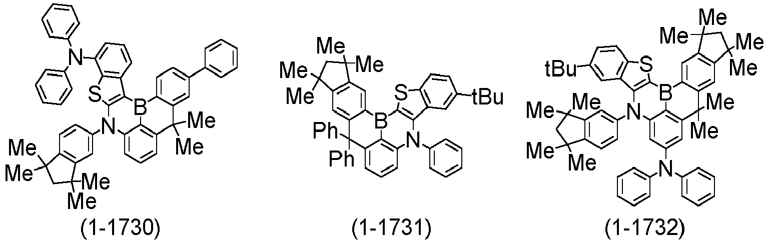

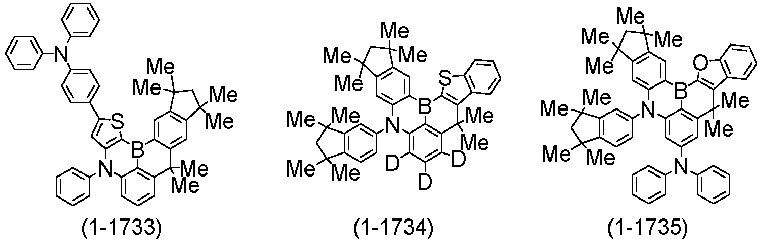

In this paragraph all compounds are inventive compounds.

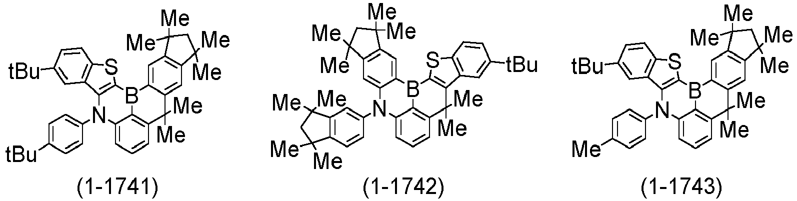

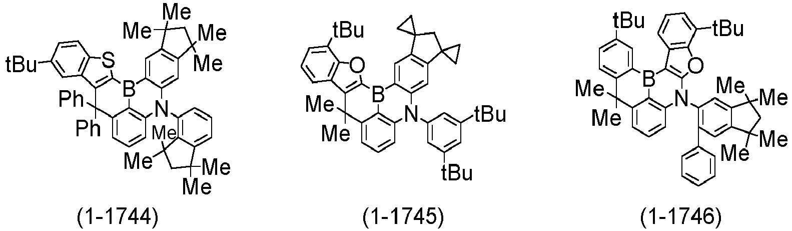

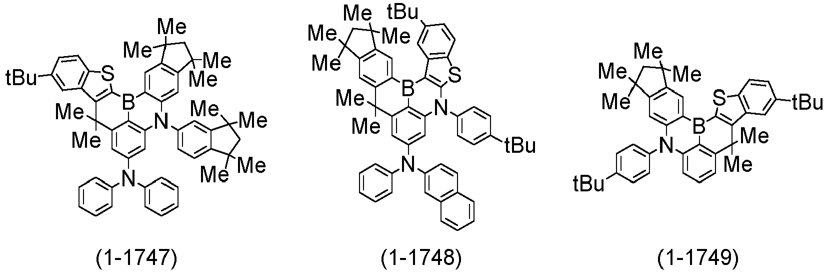

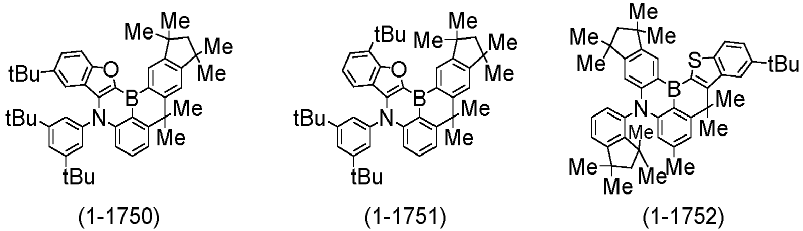

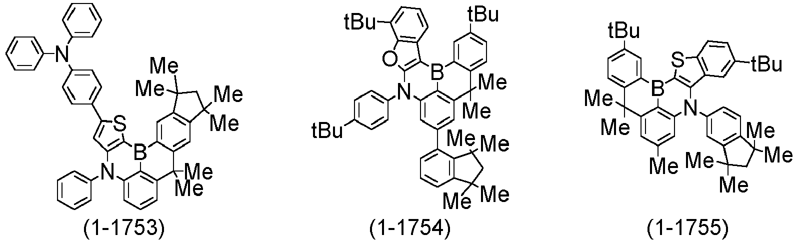

In this paragraph all compounds are inventive compounds.

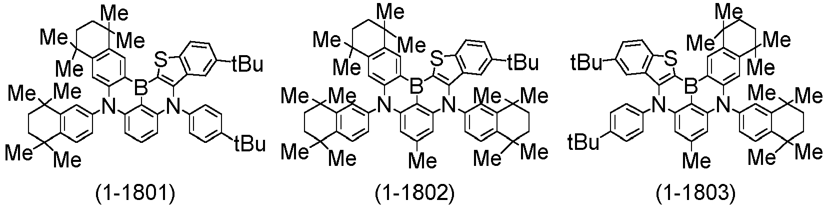

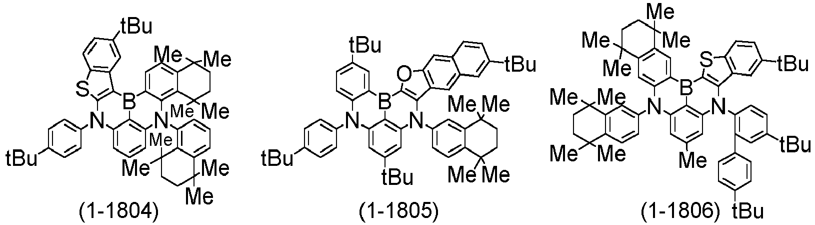

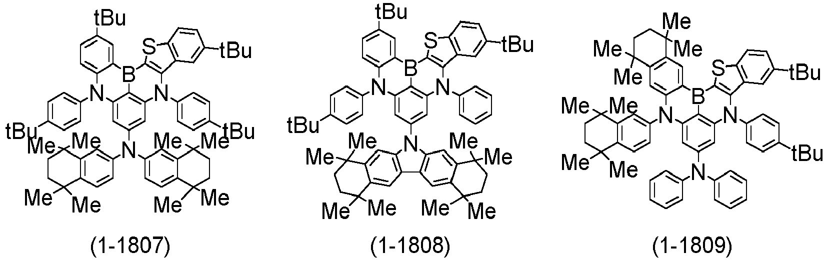

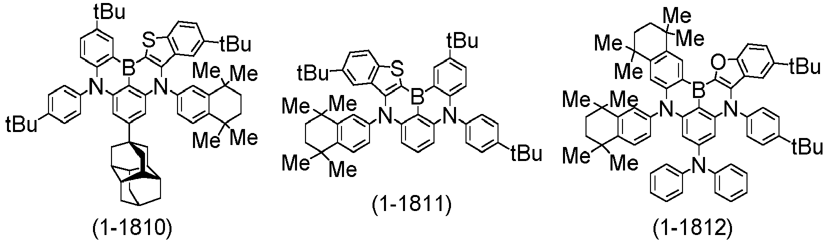

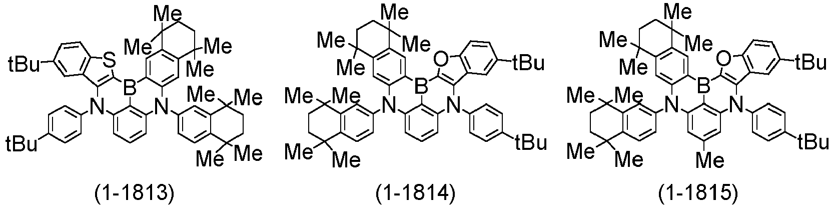

In this paragraph all compounds are inventive compounds.

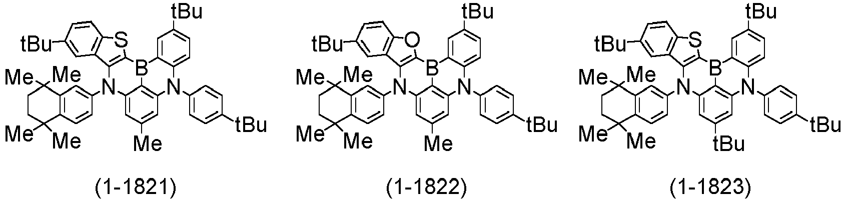

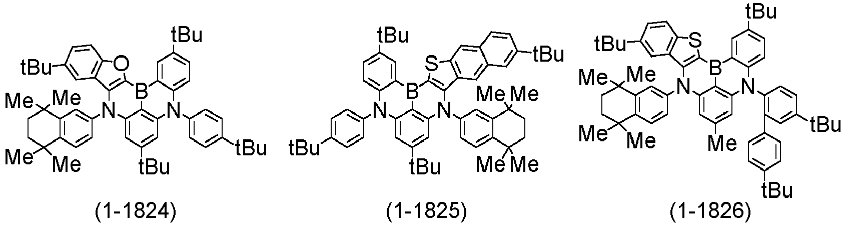

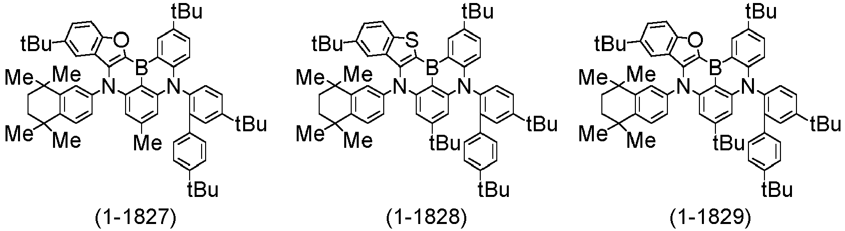

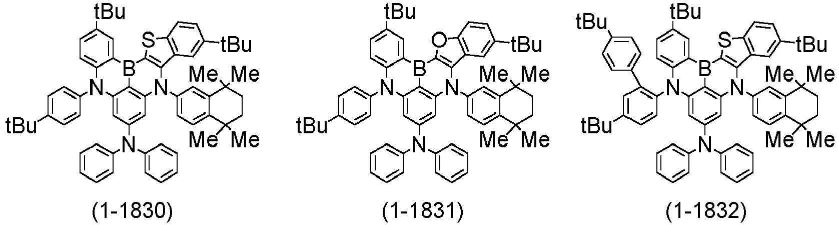

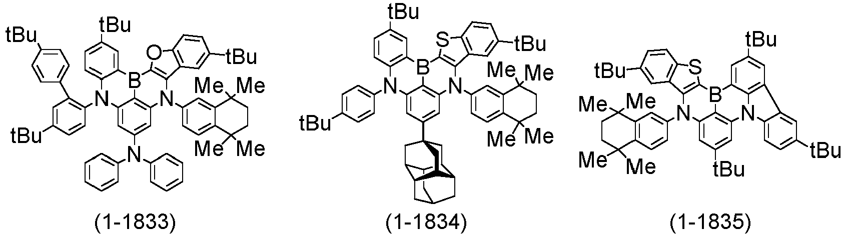

In this paragraph all compounds are inventive compounds.

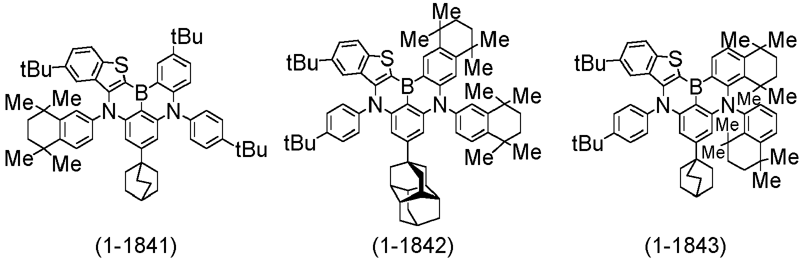

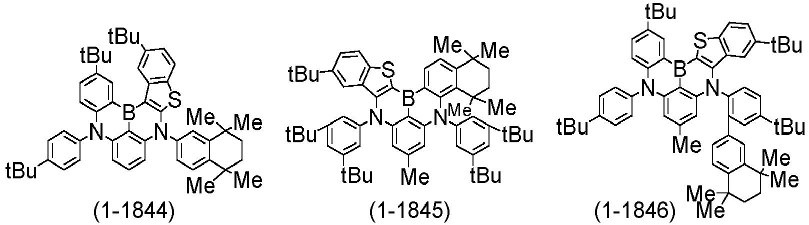

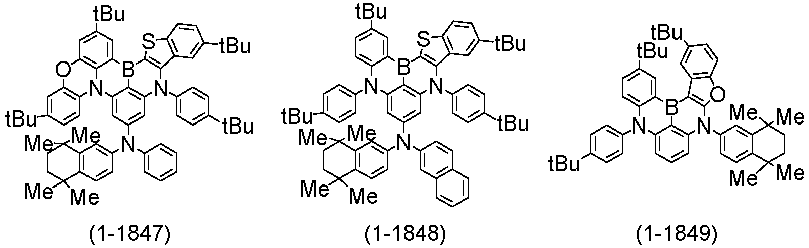

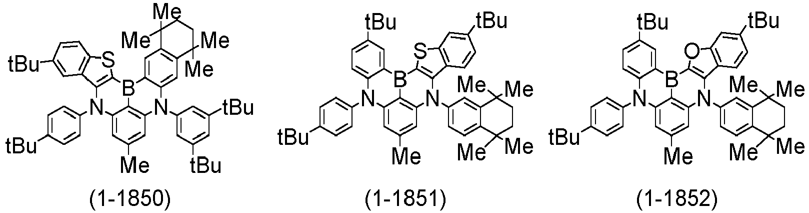

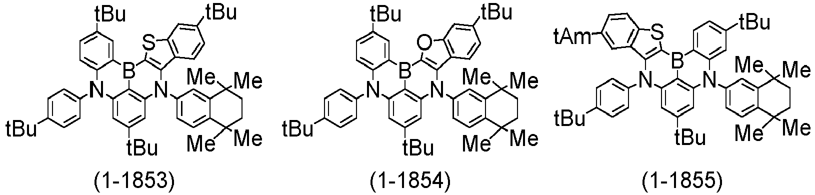

In this paragraph all compounds are inventive compounds.

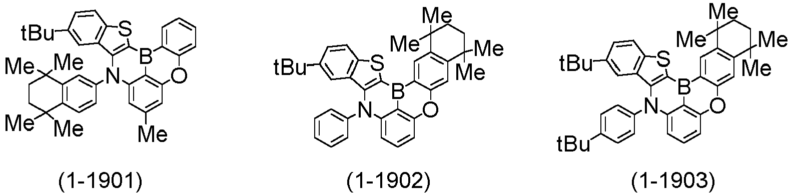

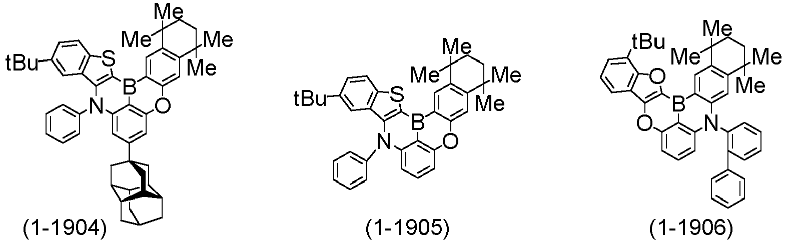

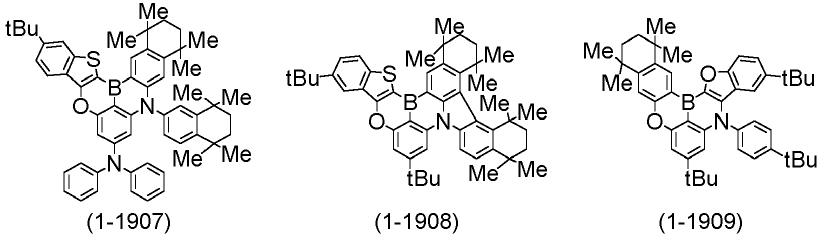

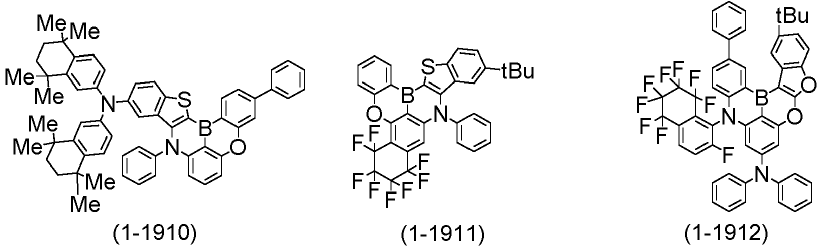

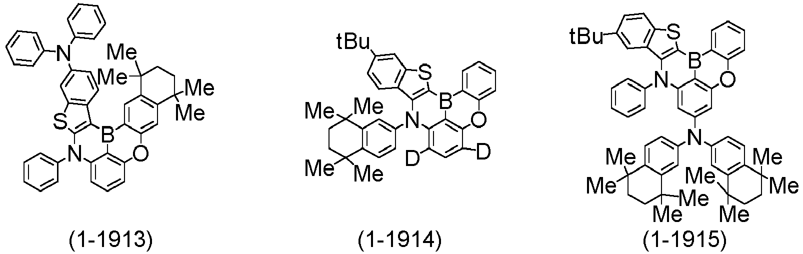

In this paragraph all compounds except compound (1-1934) are inventive compounds.

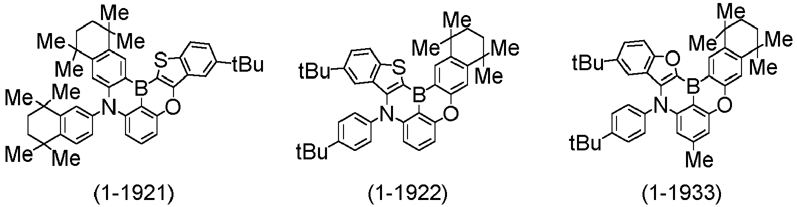

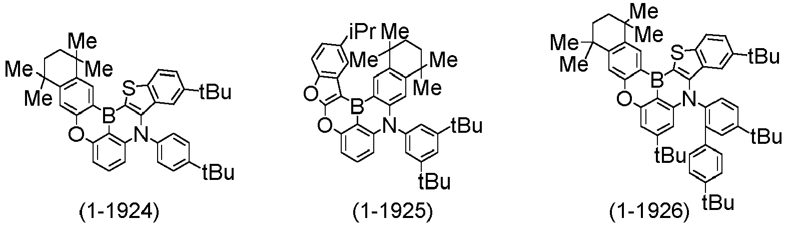

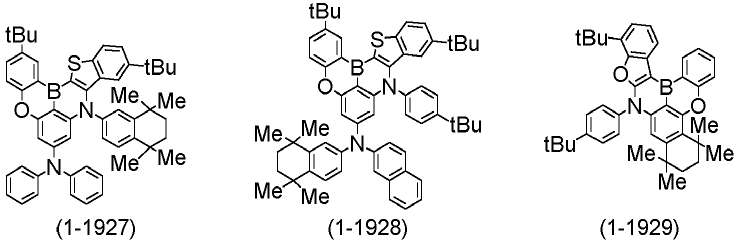

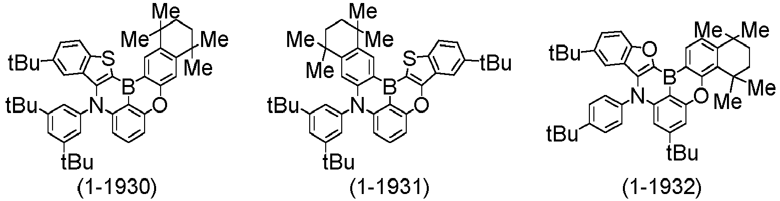

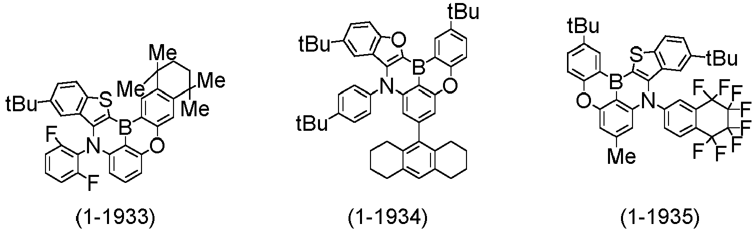

In this paragraph all compounds except compound (1-1946) are inventive compounds.

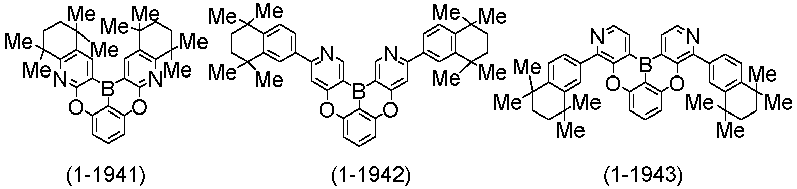

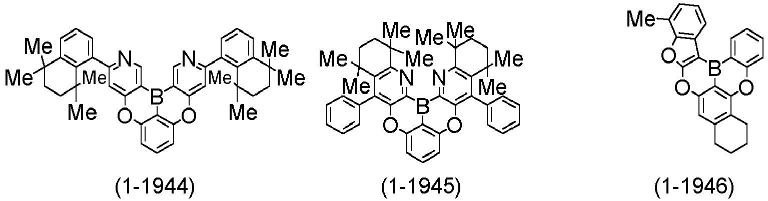

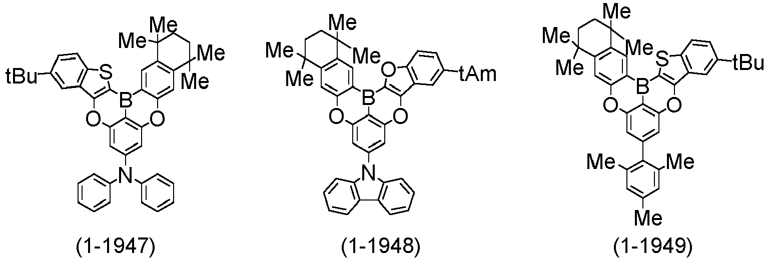

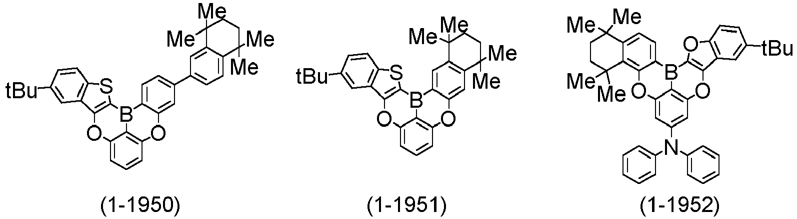

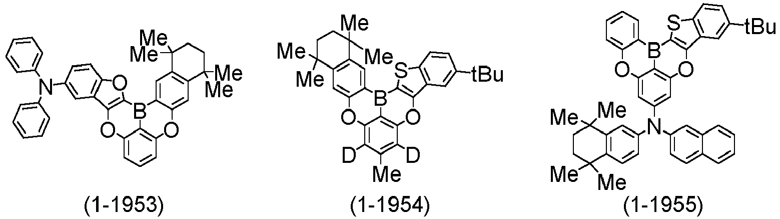

In this paragraph all compounds are inventive compounds.

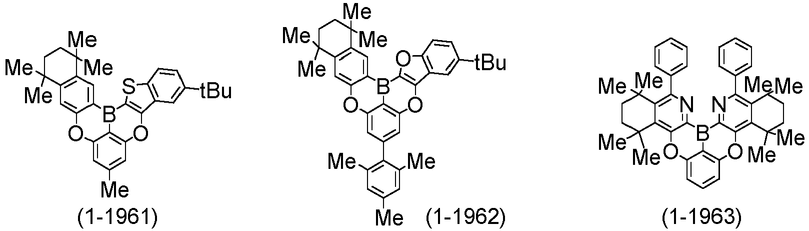

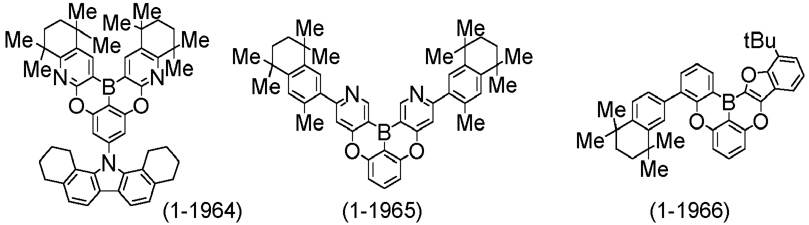

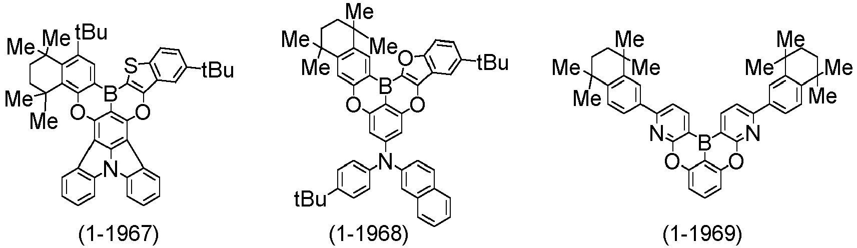

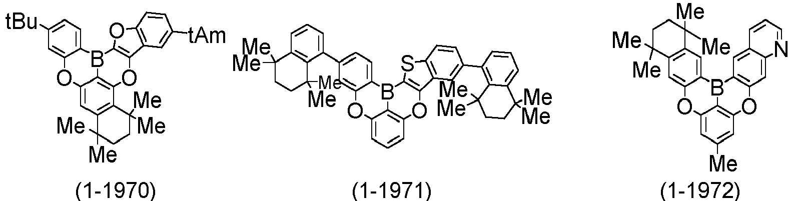

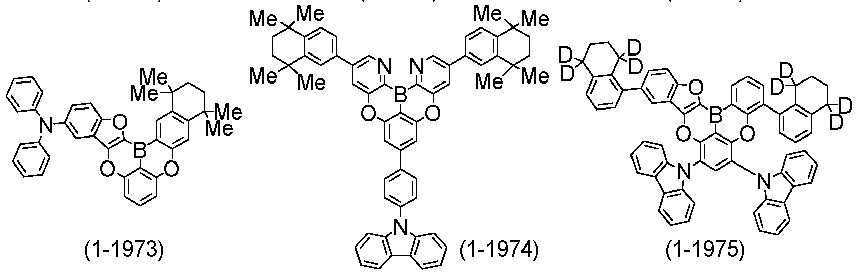

In this paragraph all compounds are inventive compounds.

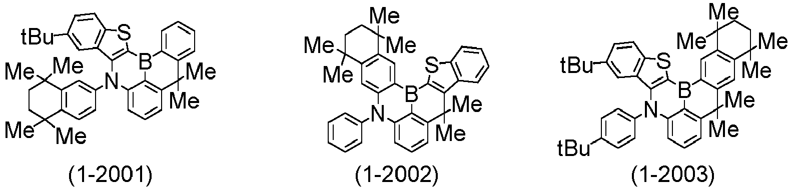

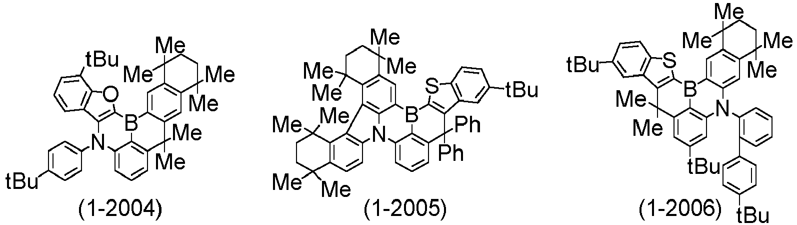

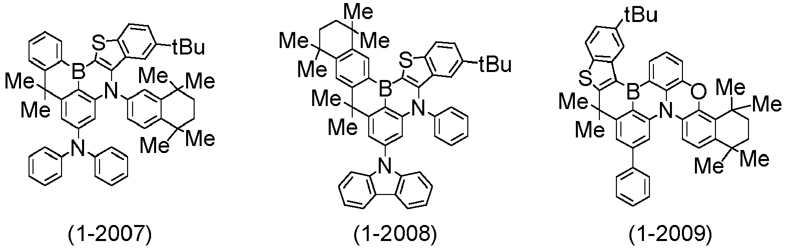

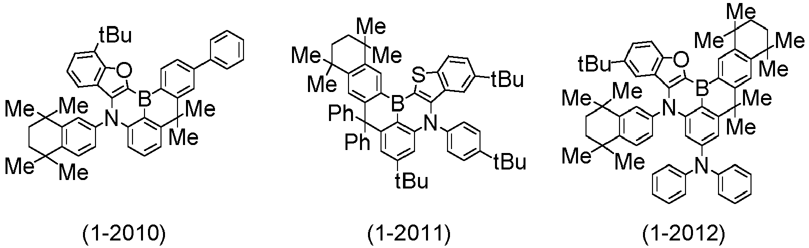

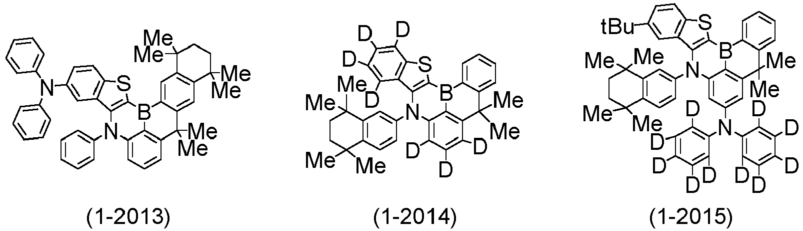

In this paragraph all compounds except compounds (1-2038) and (1-2044) are inventive compounds.

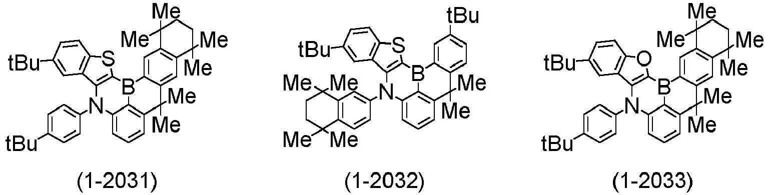

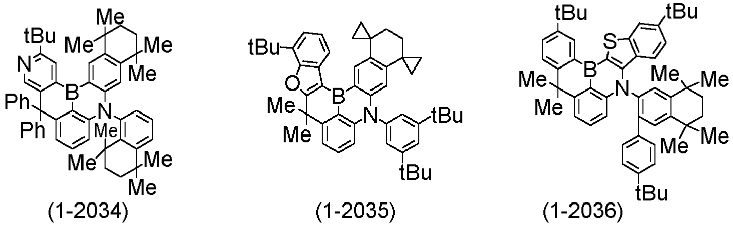

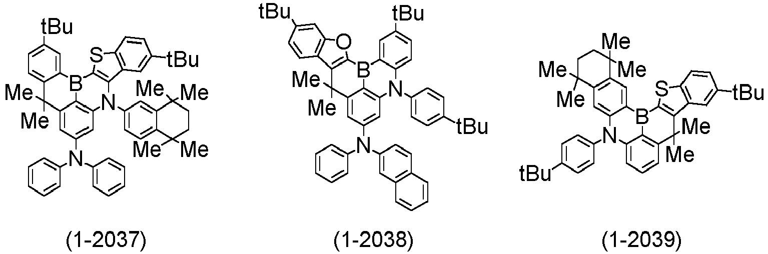

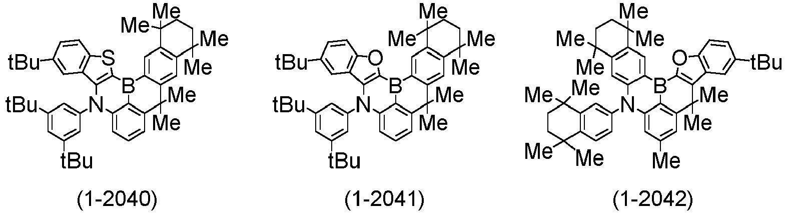

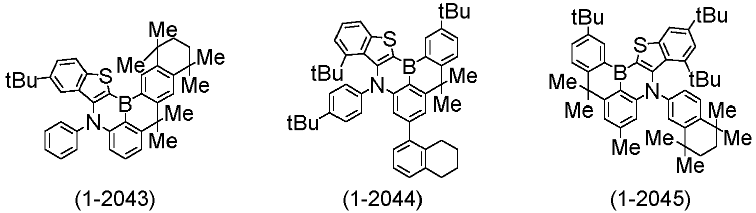

In this paragraph all compounds except compounds (1-2105) and (1-2108) are inventive compounds.

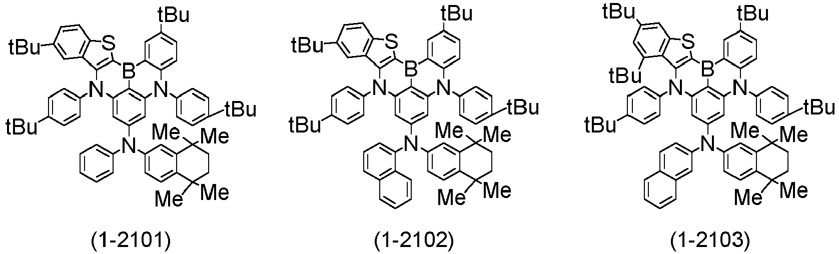

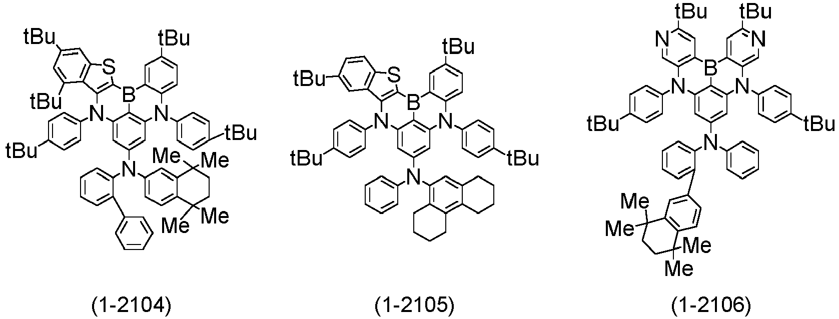

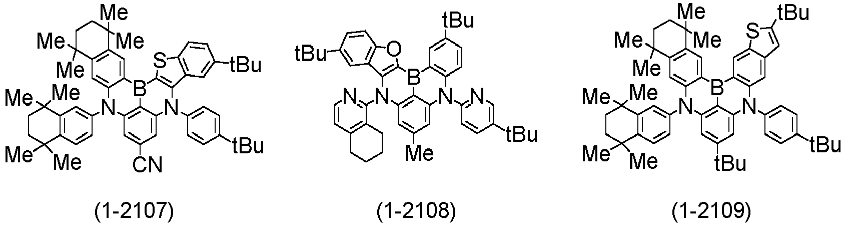

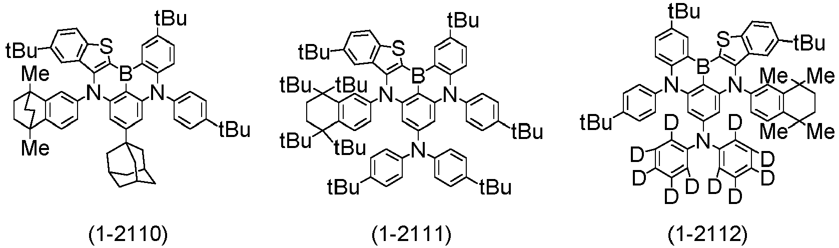

In this paragraph all compounds except compounds (1-2152), (1-2153), (1-2154) are inventive compounds.

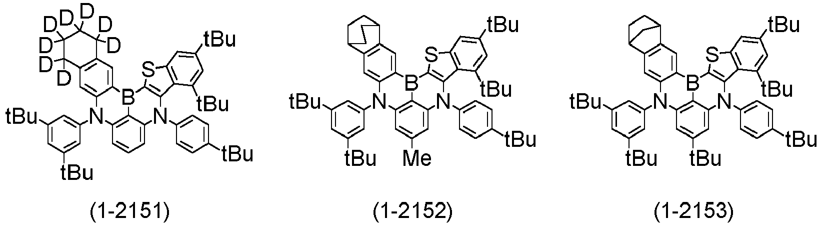

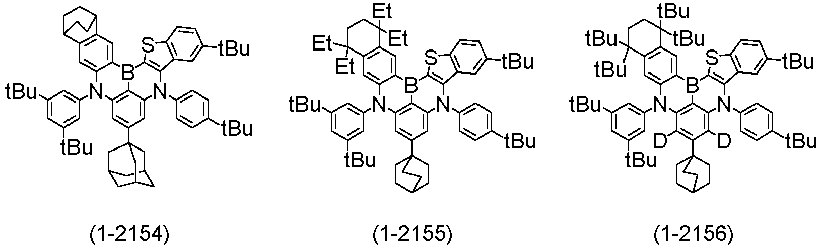

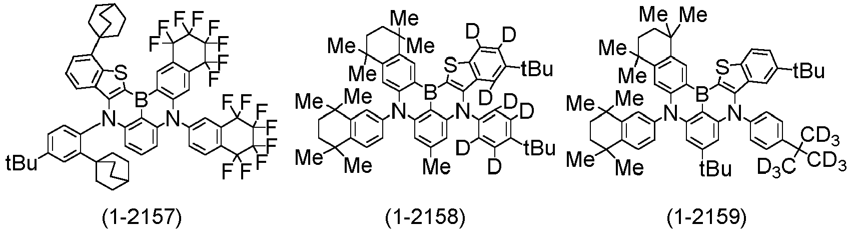

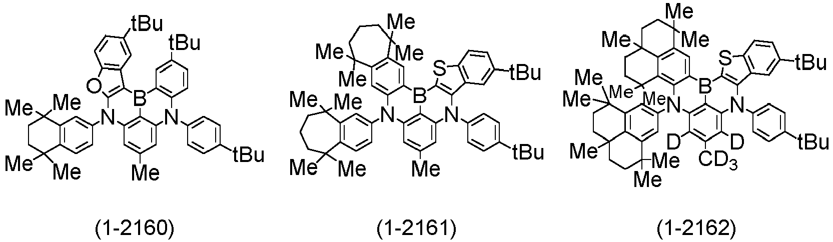

In this paragraph all compounds except compound (1-2212) are inventive compounds.

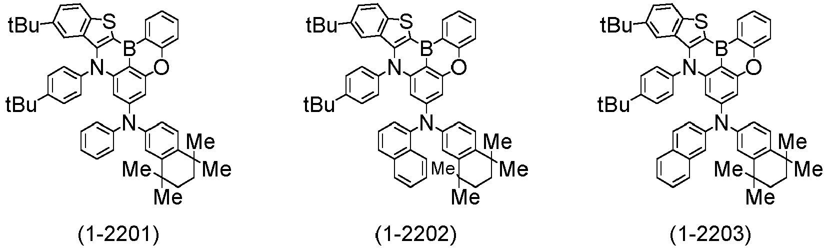

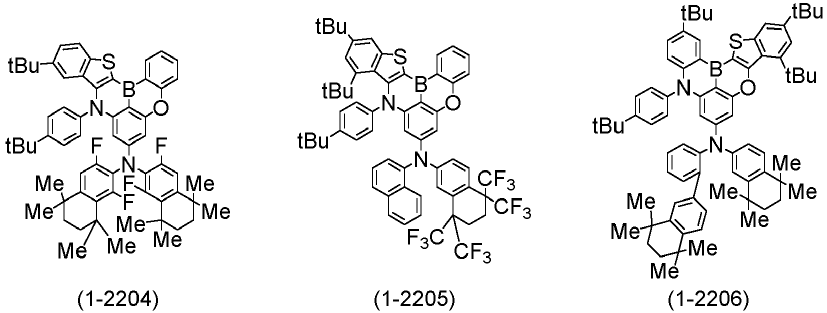

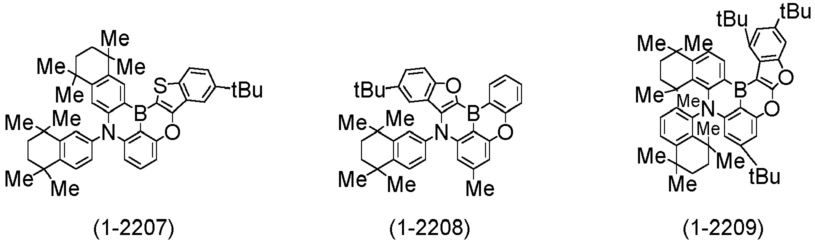

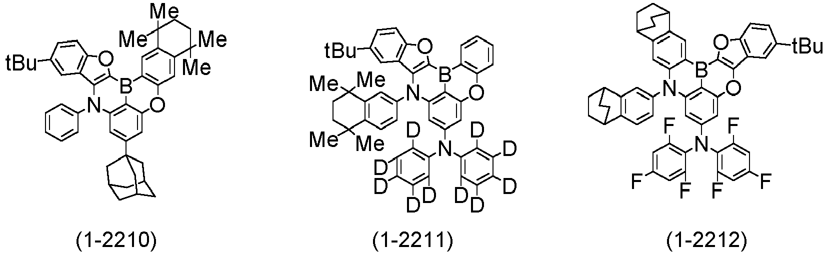

In this paragraph all compounds except compound (1-2241) are inventive compounds.

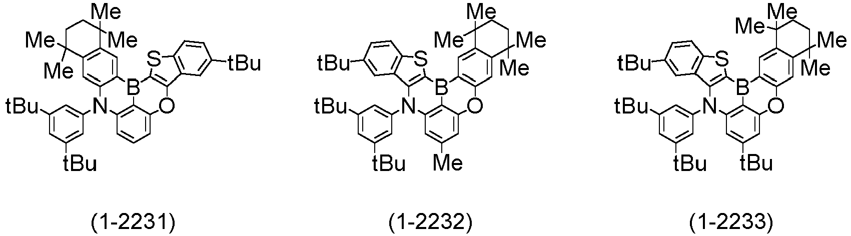

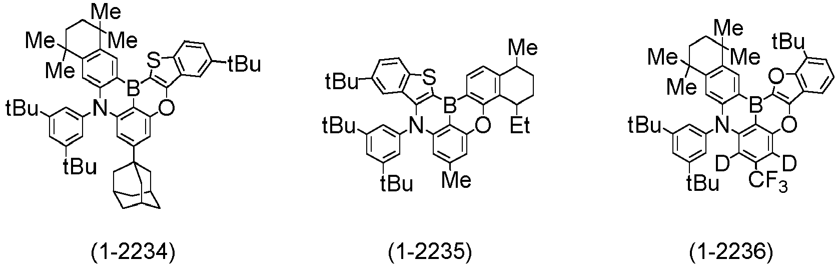

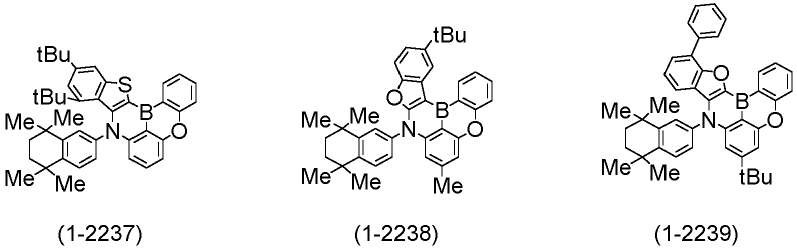

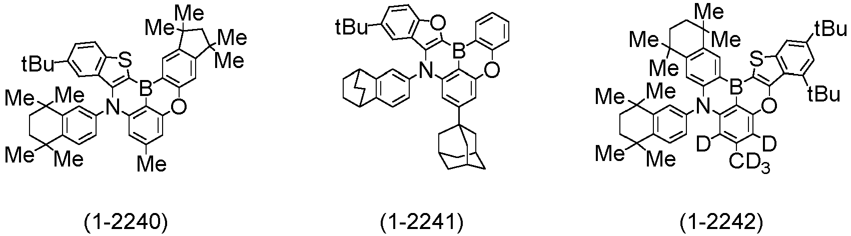

In this paragraph all compounds except compounds (1-2301), (1-2303), (1-2305) to (1-2308), (1-2311), (1-2312) are inventive compounds.

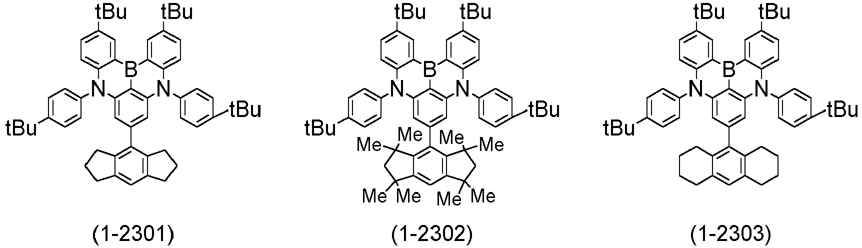

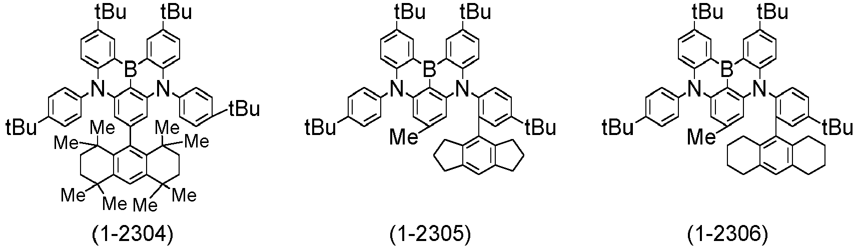

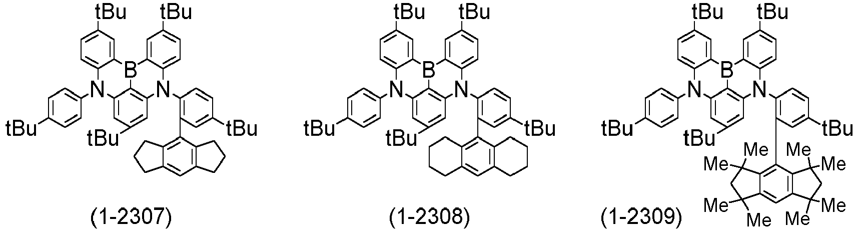

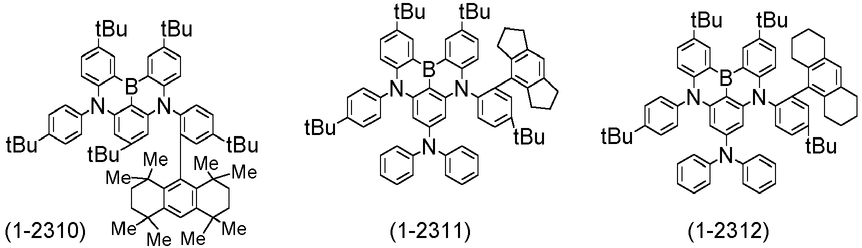

In this paragraph all compounds except compounds (1-2322), (1-2324) are inventive compounds.

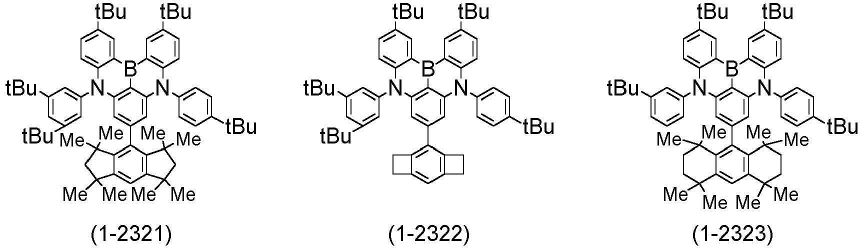

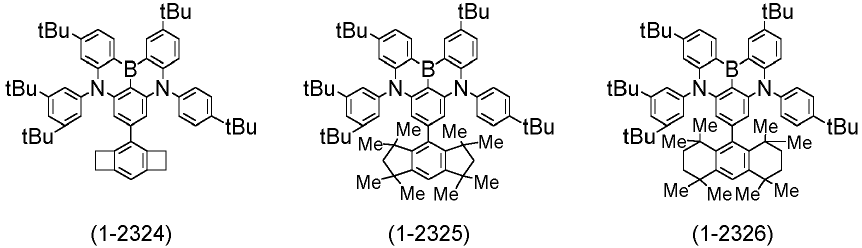

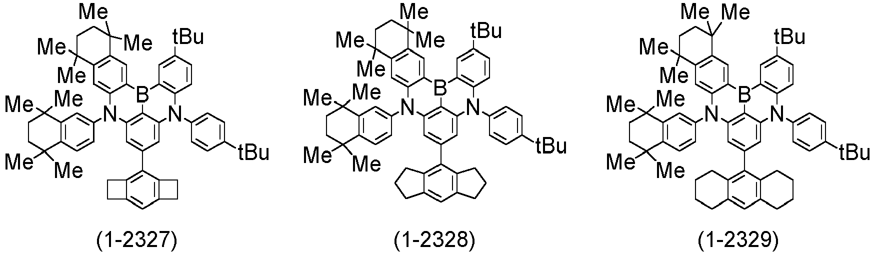

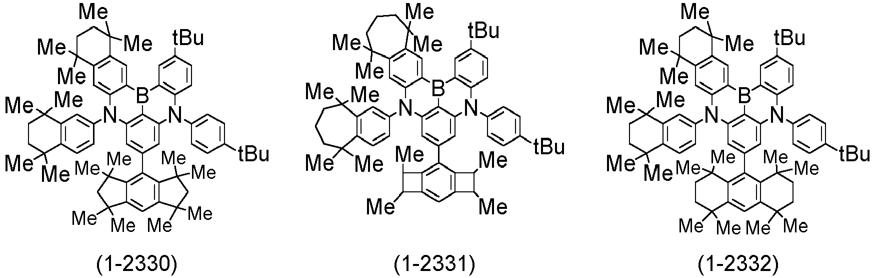

In this paragraph all compounds except compounds (1-2401), (1-2403), (1-2405) to (1-2408), (1-2411), (1-2412) are inventive compounds.

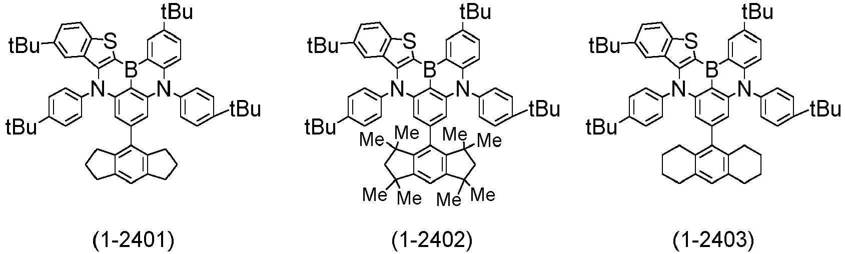

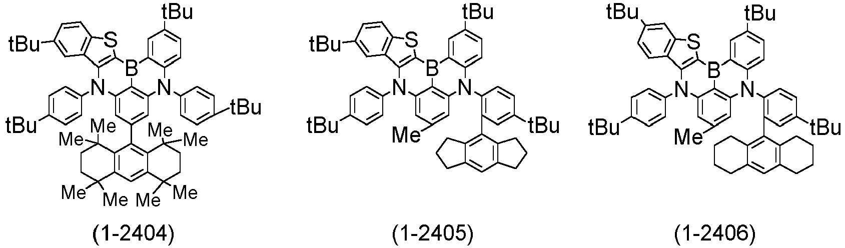

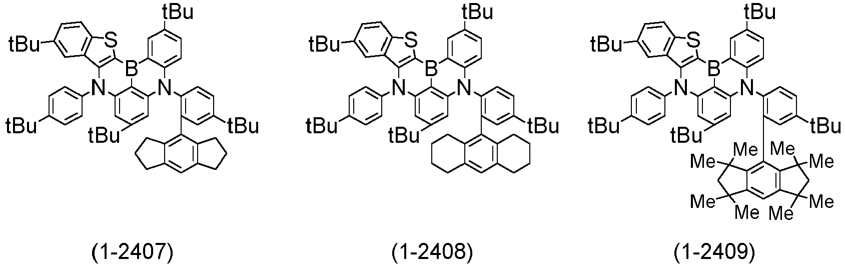

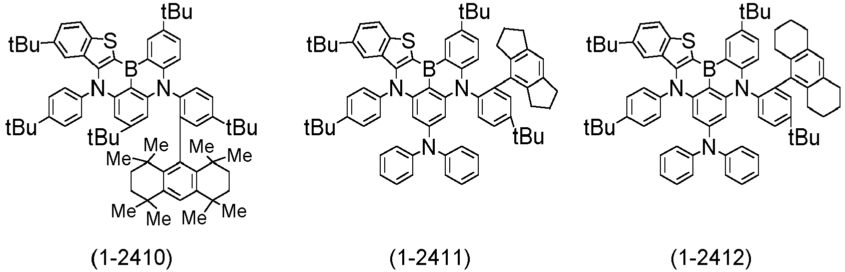

In this paragraph all compounds except compound (1-2422), (1-2424) are inventive compounds.

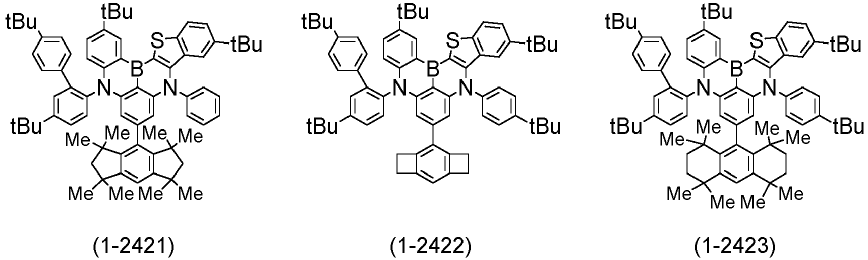

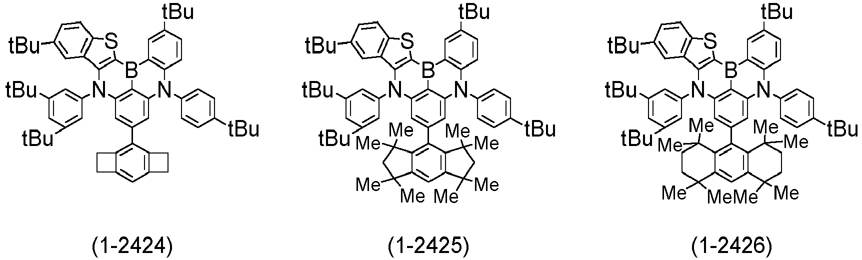

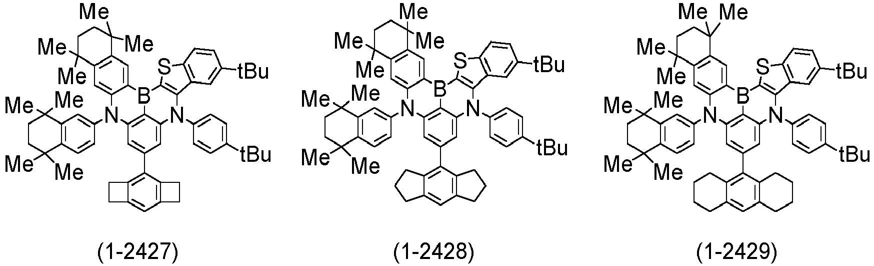

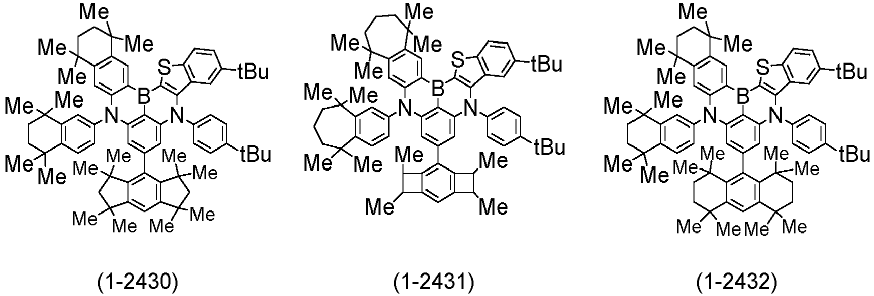

In this paragraph all compounds are inventive compounds.

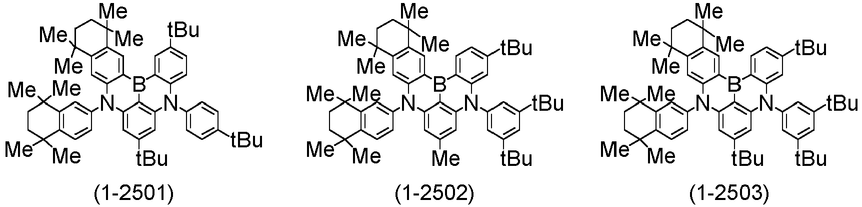

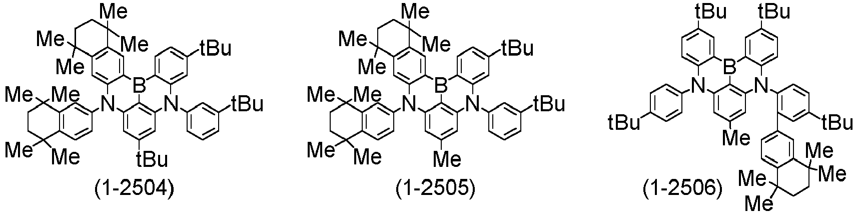

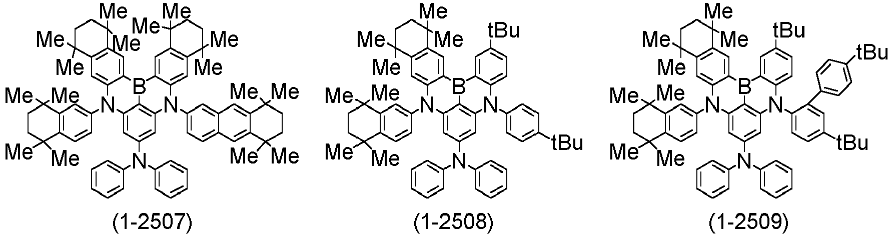

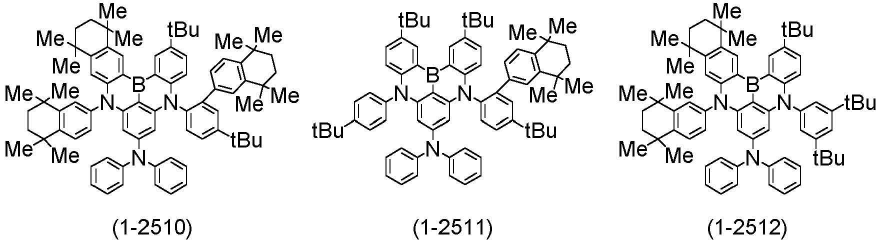

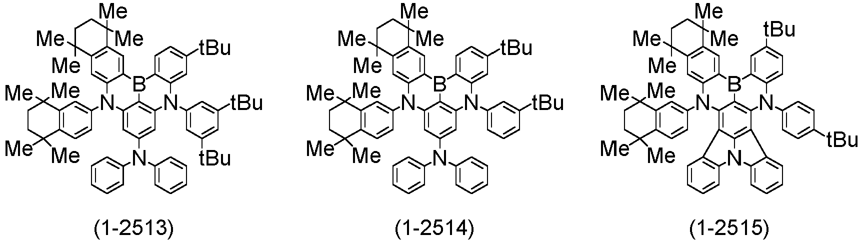

In this paragraph all compounds are inventive compounds.

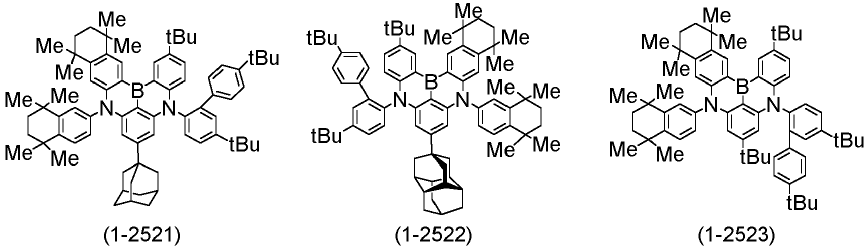

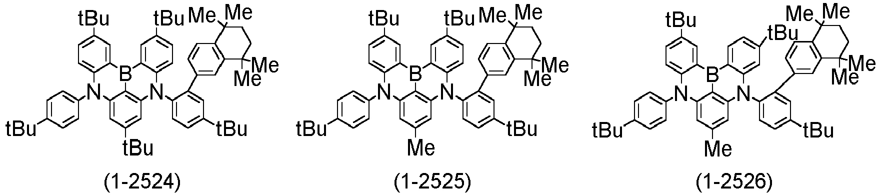

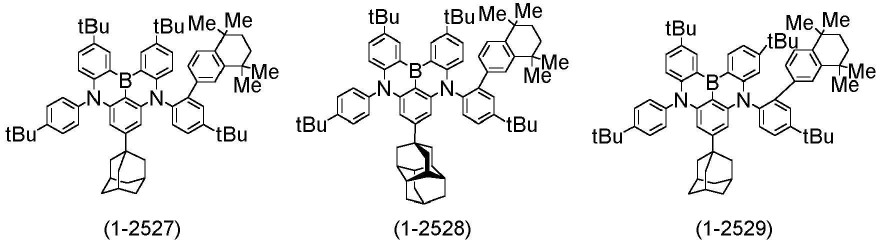

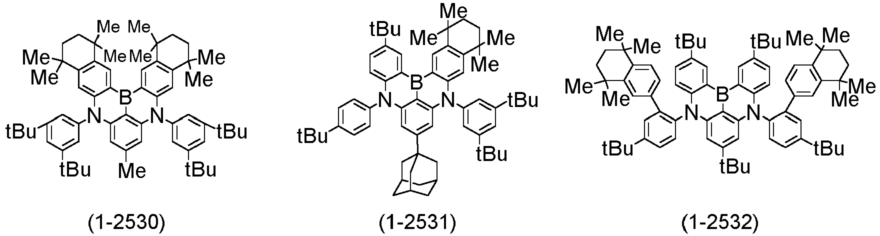

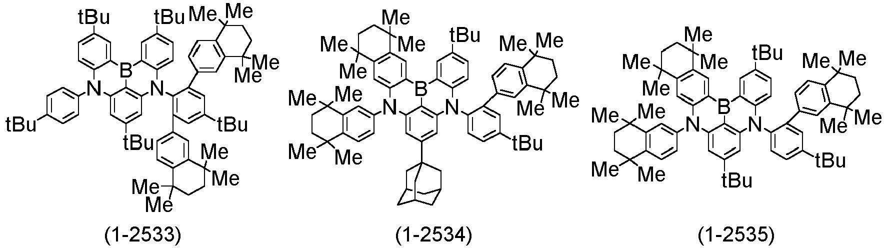

In this paragraph all compounds are inventive compounds.

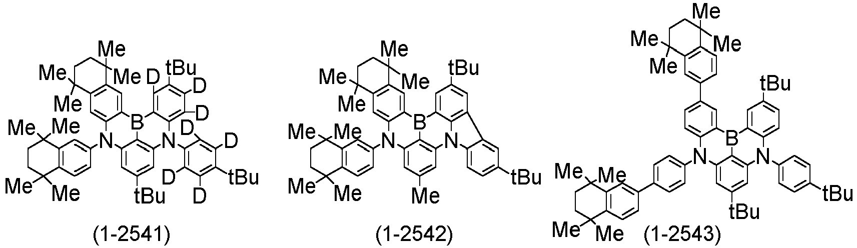

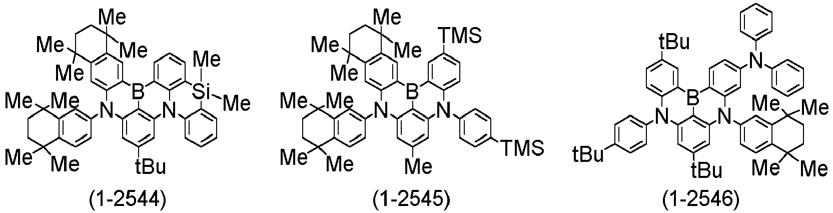

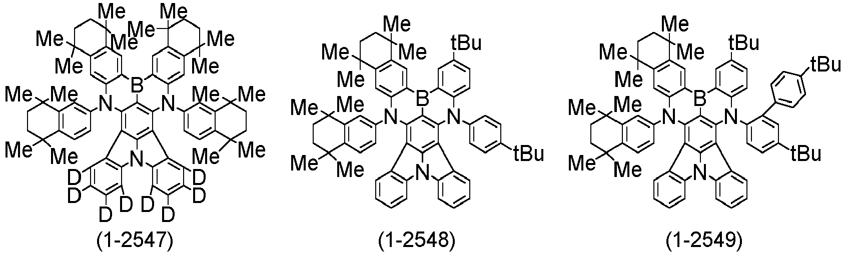

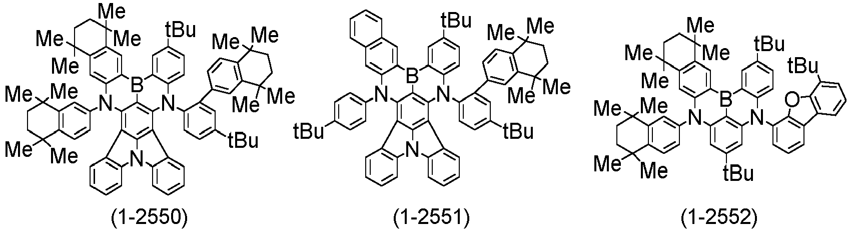

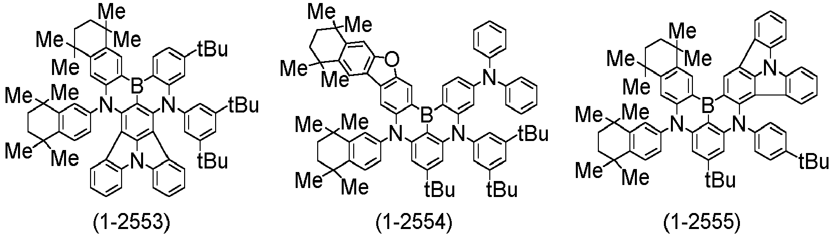

In this paragraph all compounds are inventive compounds.

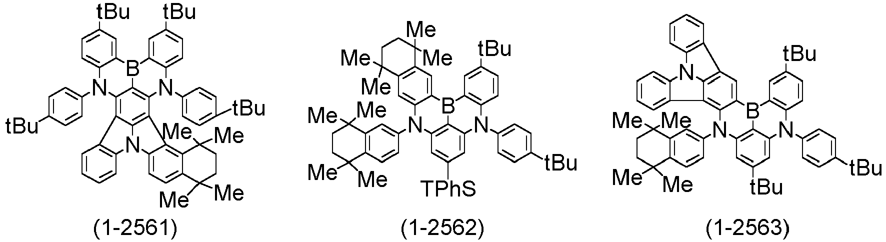

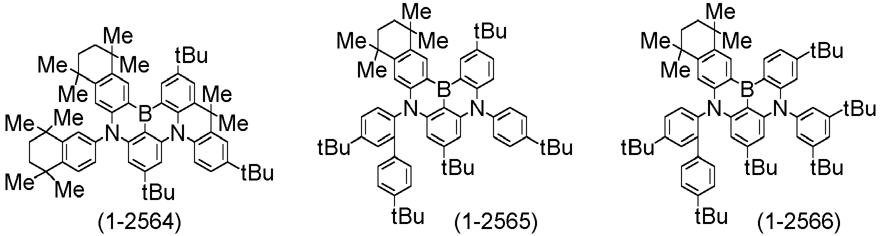

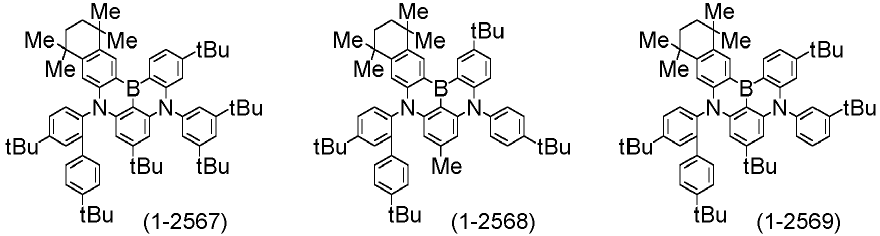

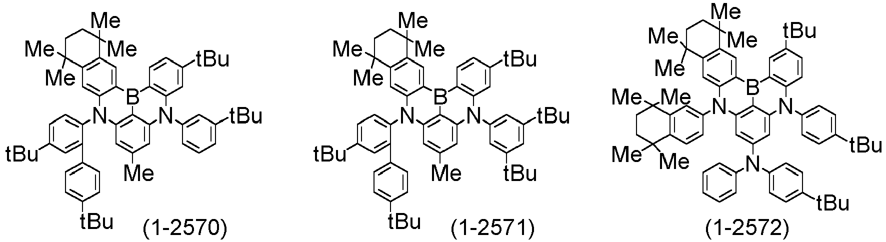

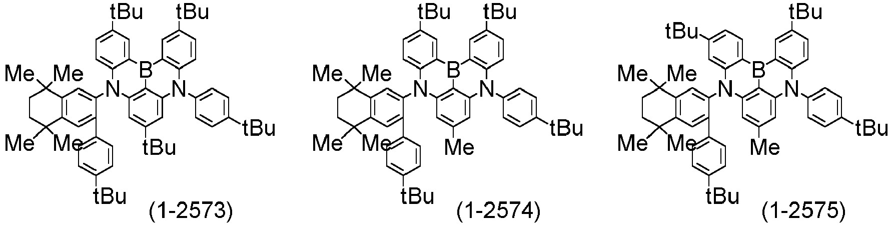

In this paragraph all compounds are inventive compounds.

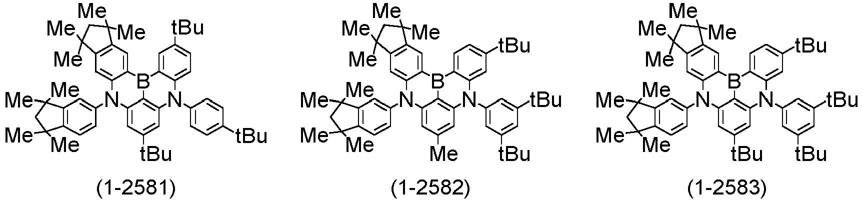

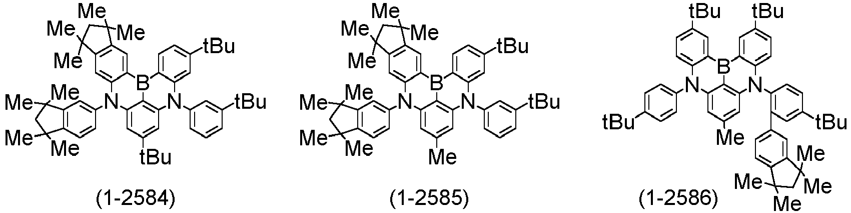

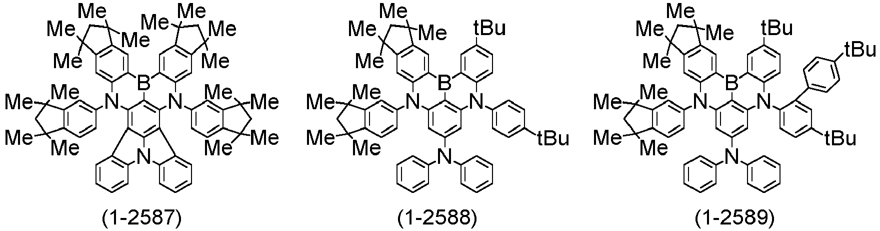

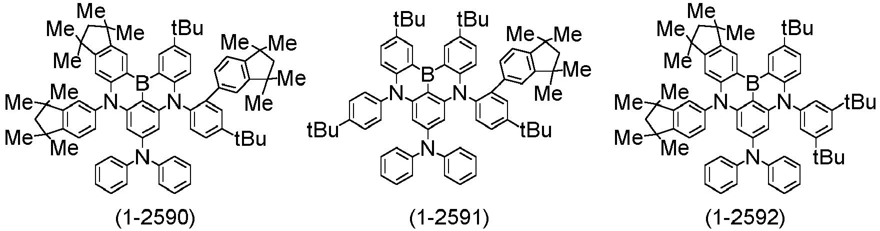

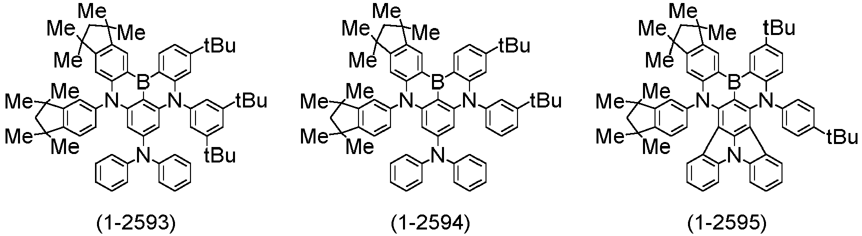

In this paragraph all compounds are inventive compounds.

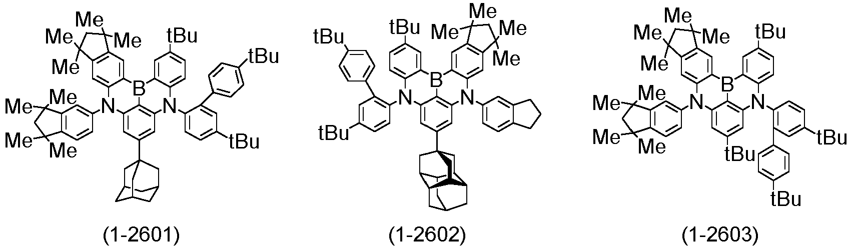

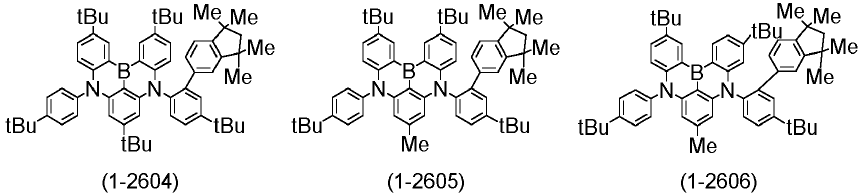

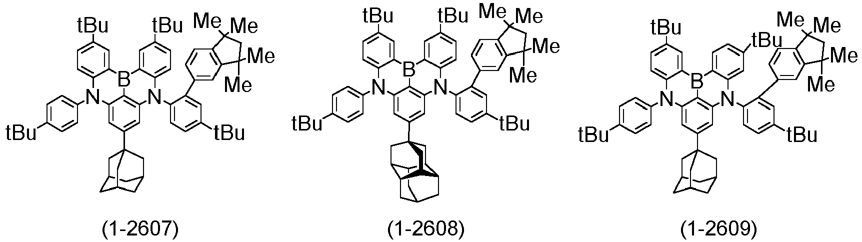

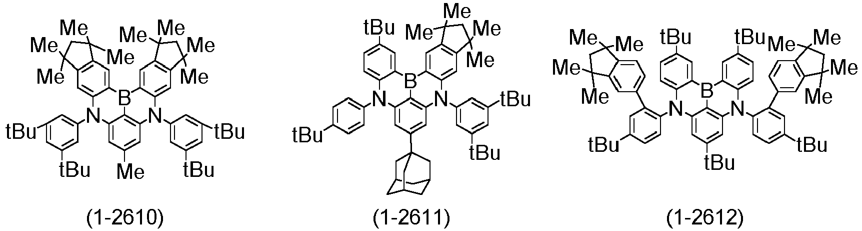

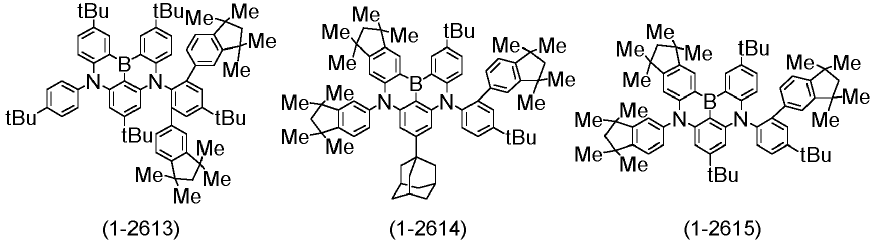

In this paragraph all compounds are inventive compounds.

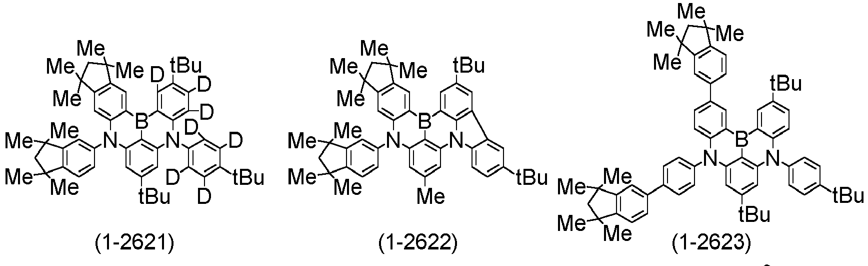

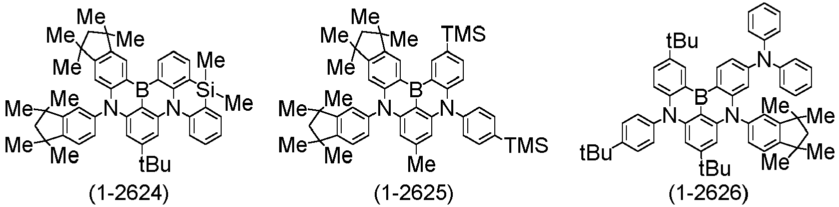

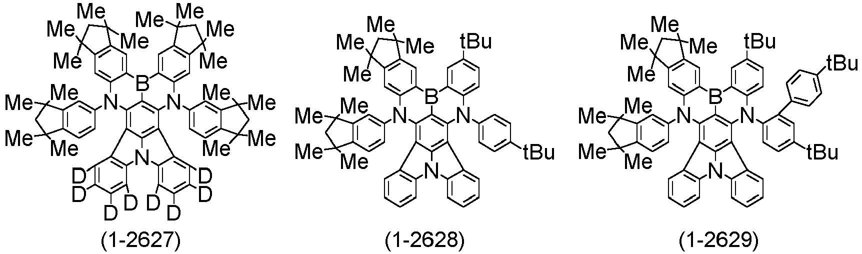

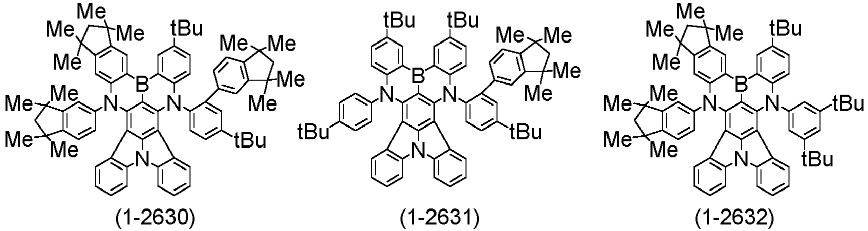

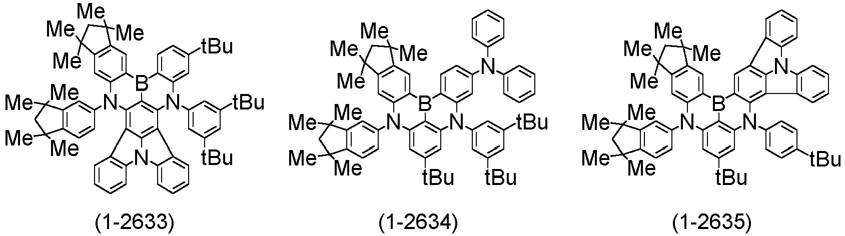

In this paragraph all compounds are inventive compounds.

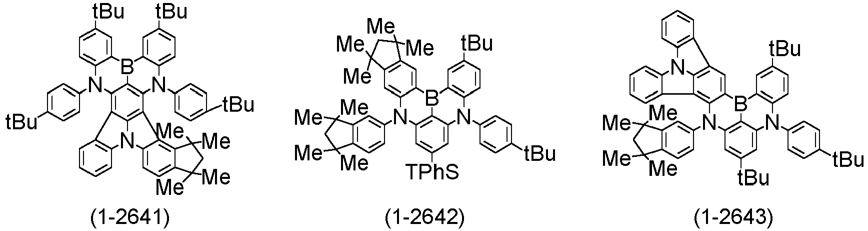

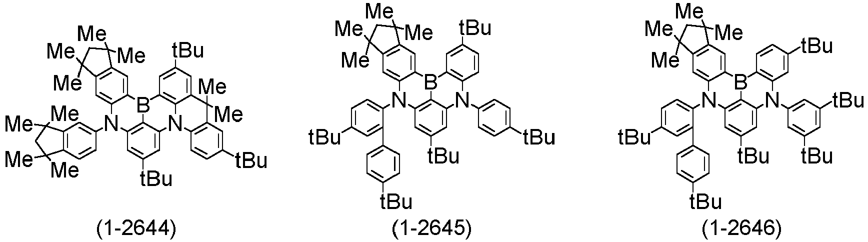

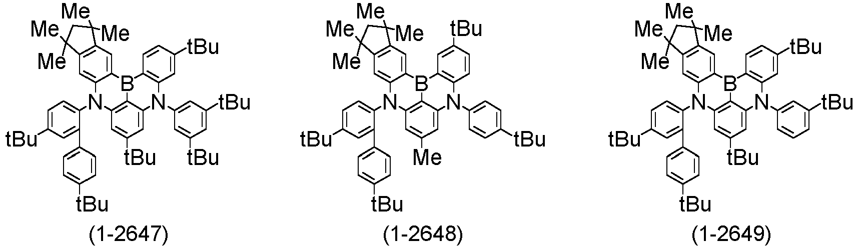

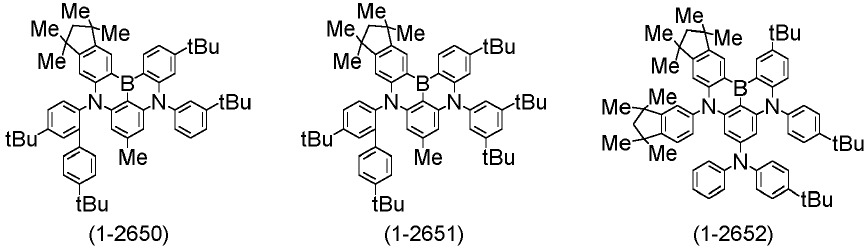

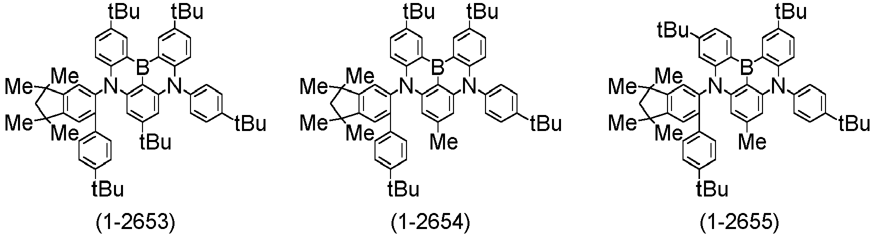

In this paragraph all compounds are inventive compounds.

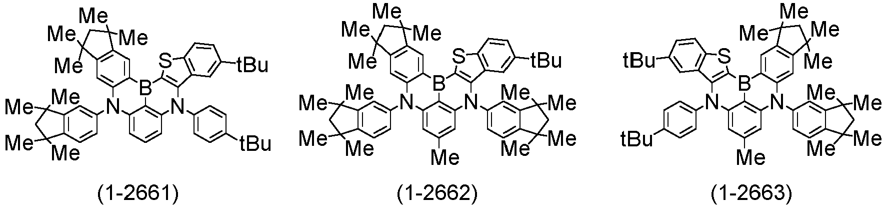

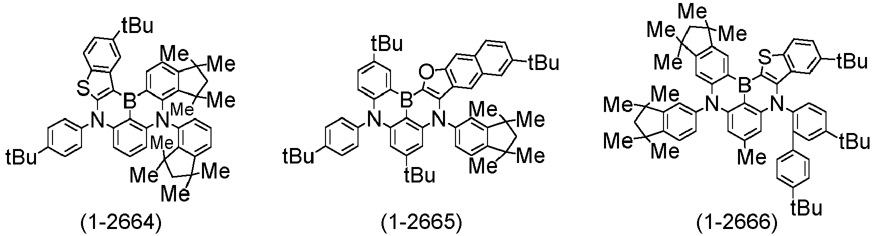

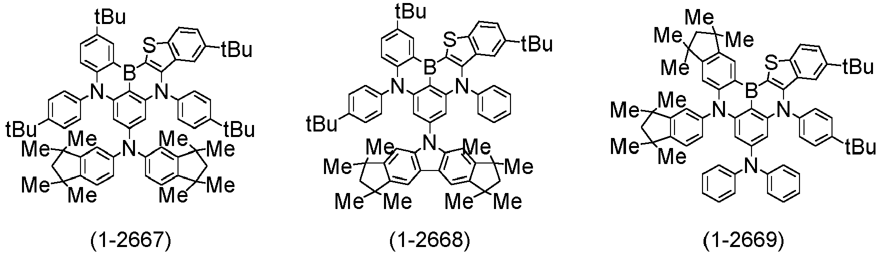

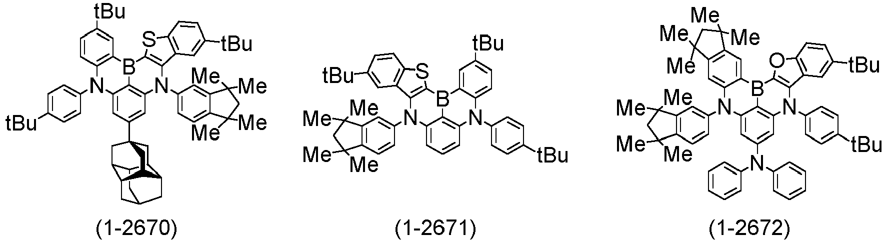

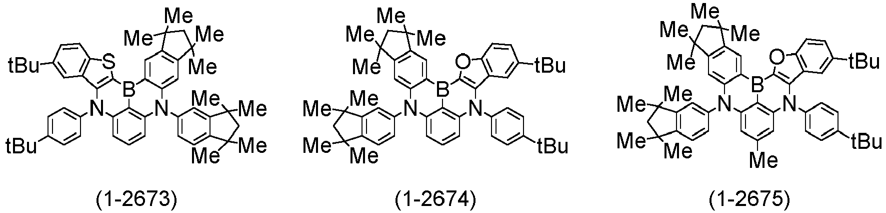

In this paragraph all compounds are inventive compounds.

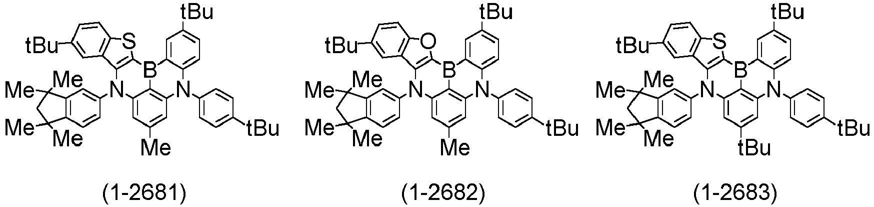

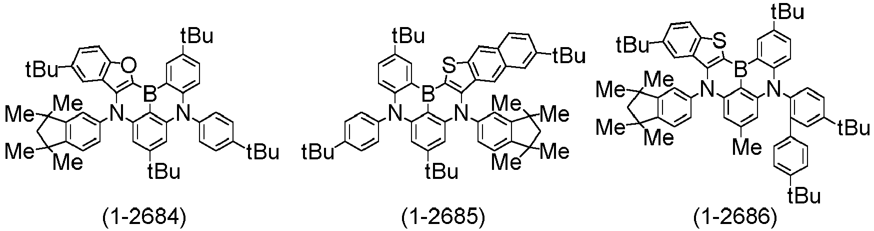

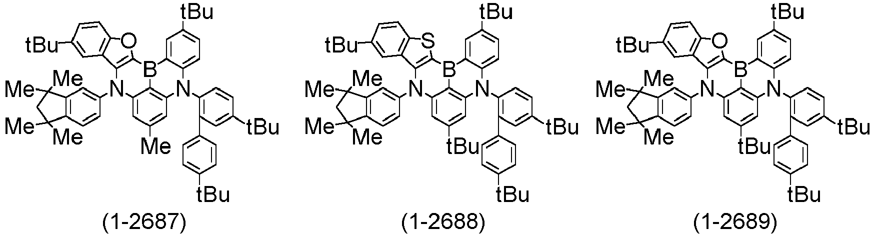

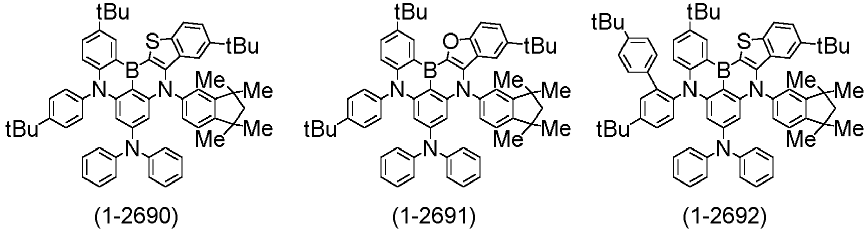

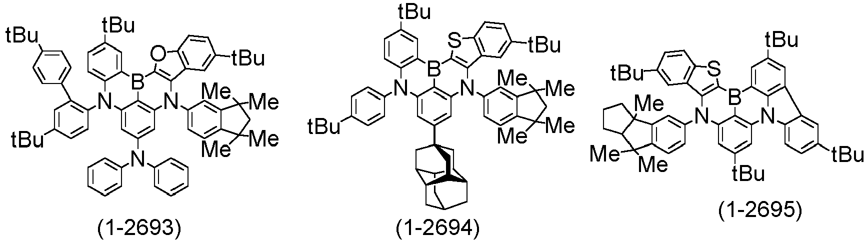

In this paragraph all compounds are inventive compounds.

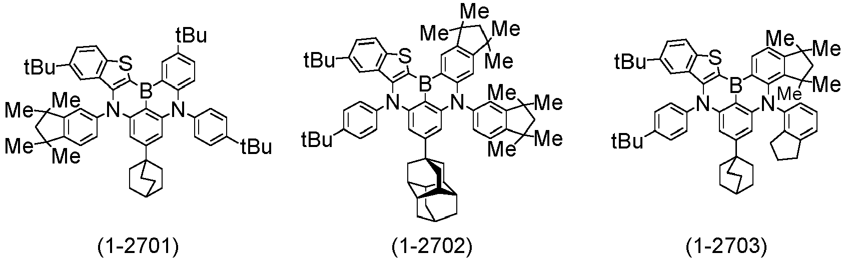

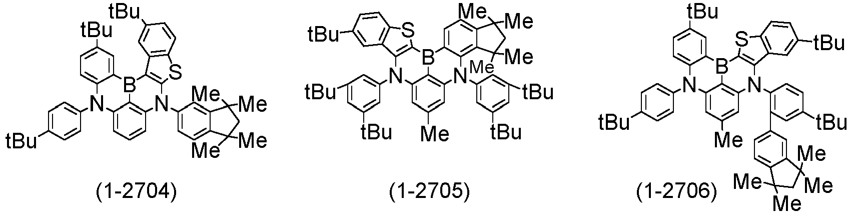

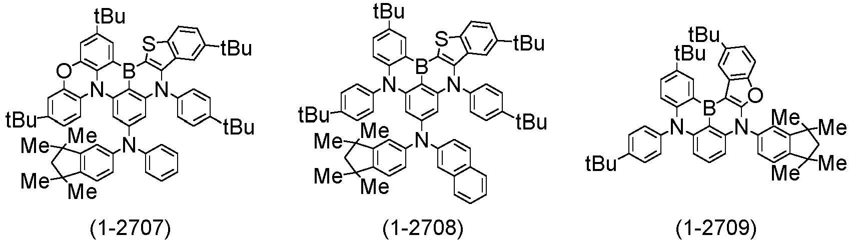

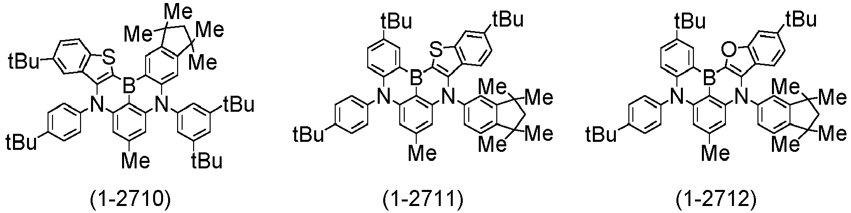

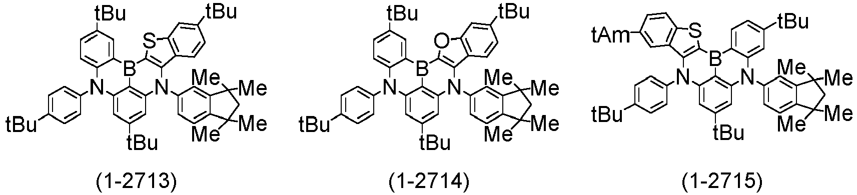

In this paragraph all compounds are inventive compounds.

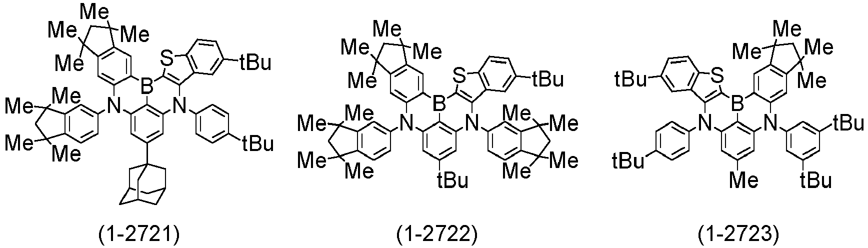

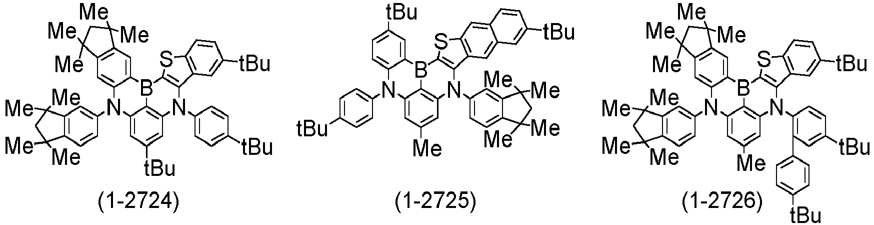

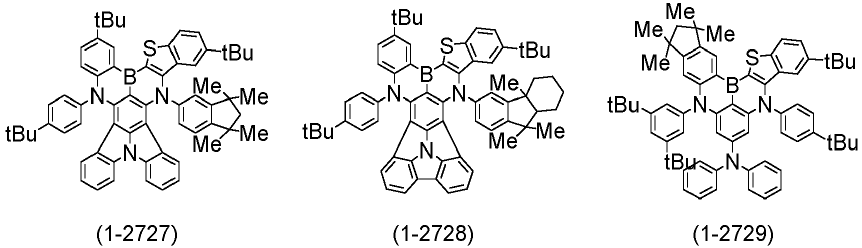

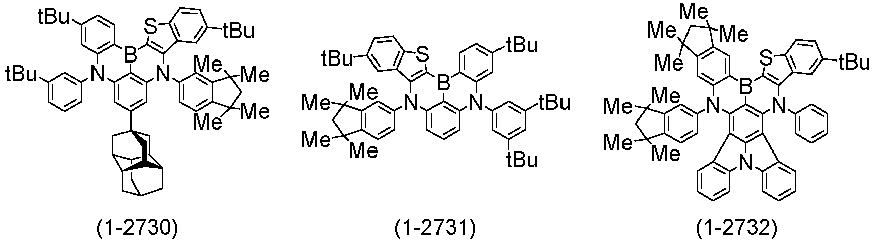

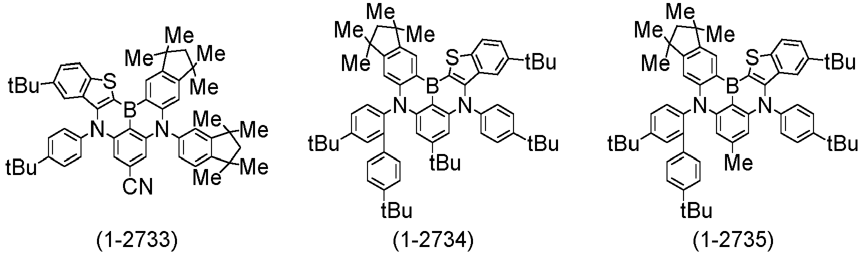

In this paragraph all compounds are inventive compounds.

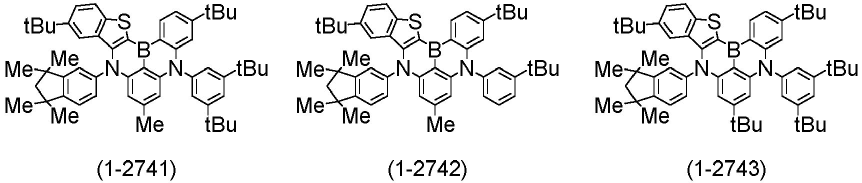

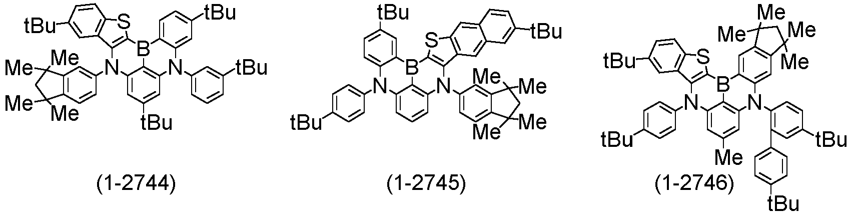

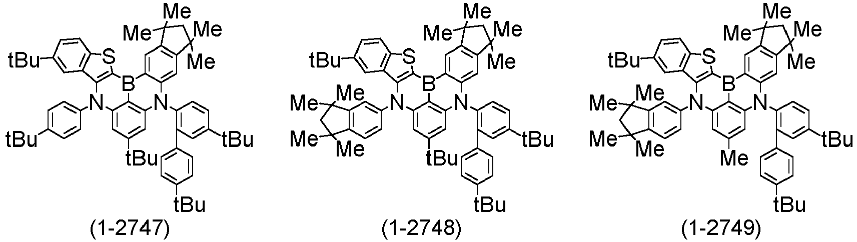

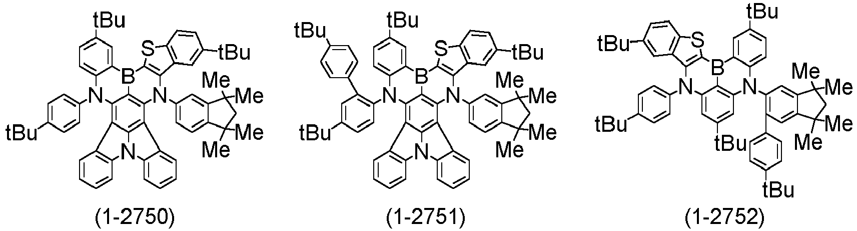

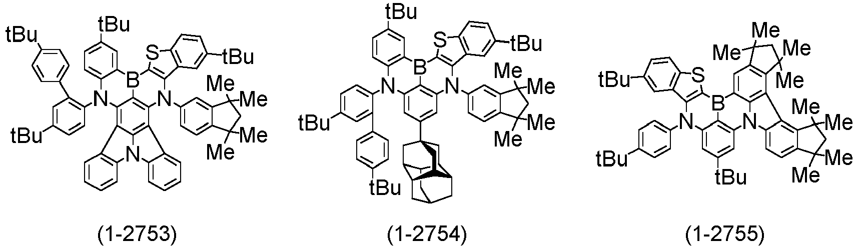

In this paragraph all compounds are inventive compounds.

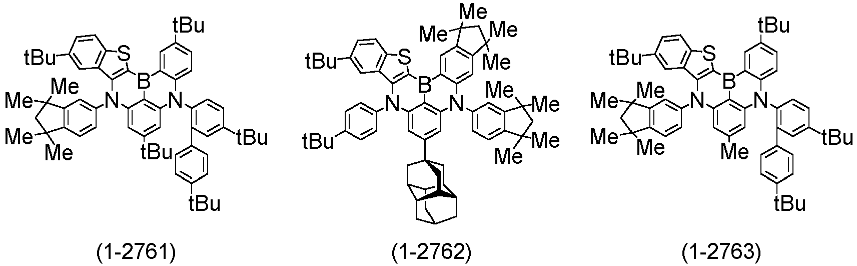

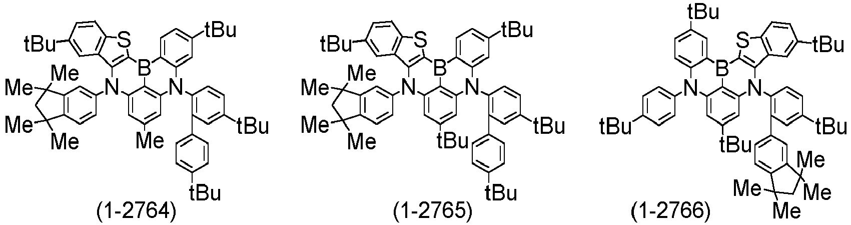

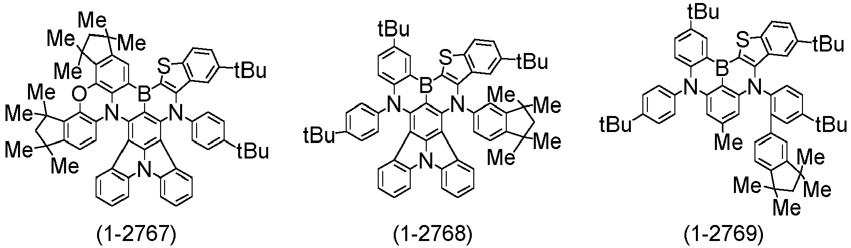

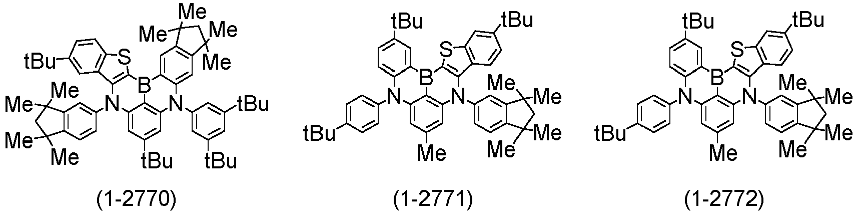

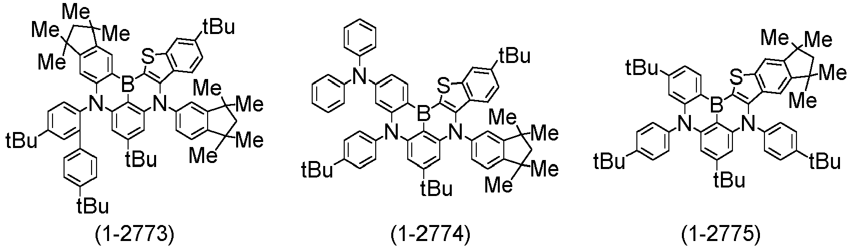

In this paragraph all compounds are inventive compounds.

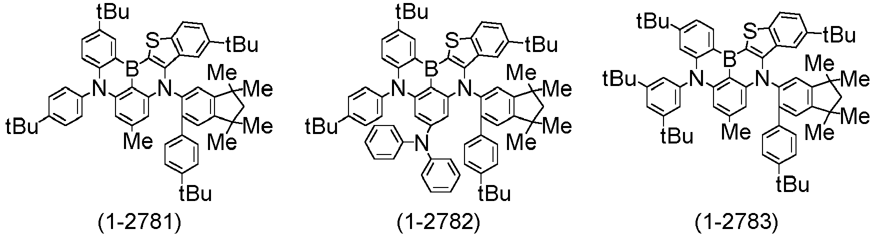

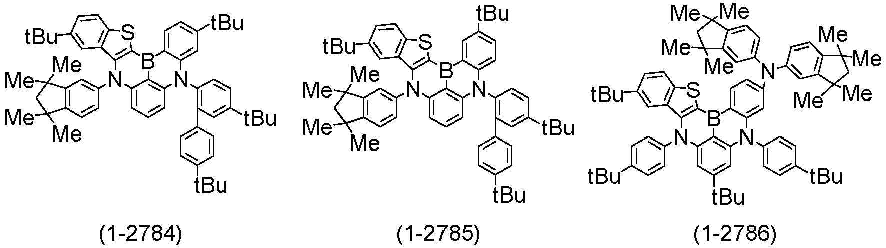

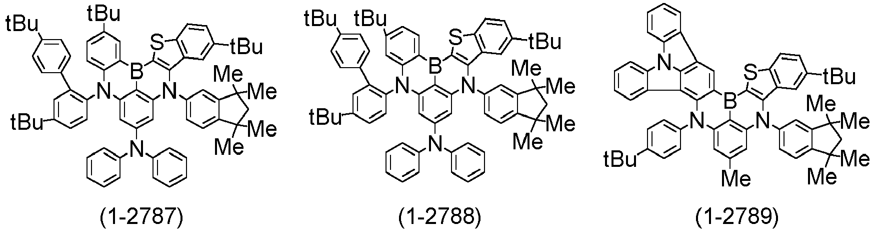

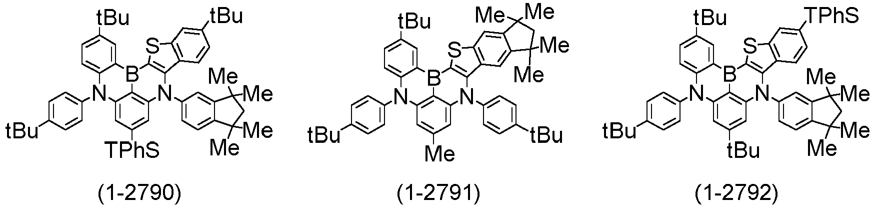

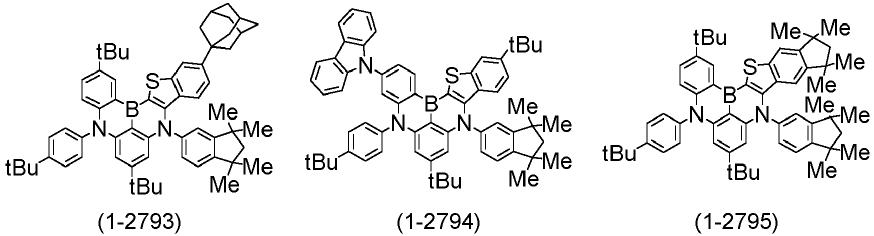

In this paragraph all compounds are inventive compounds.

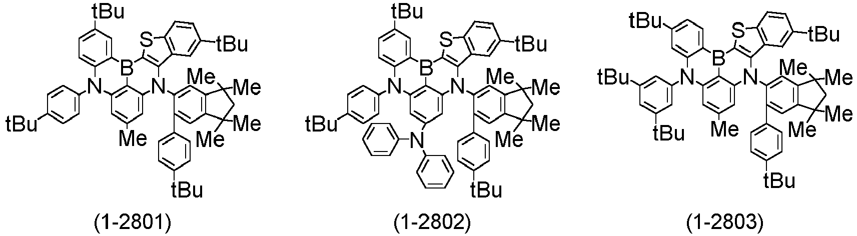

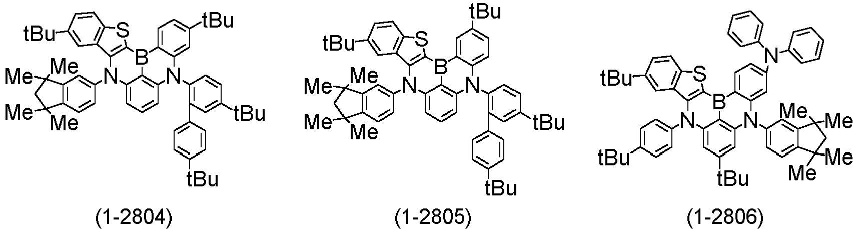

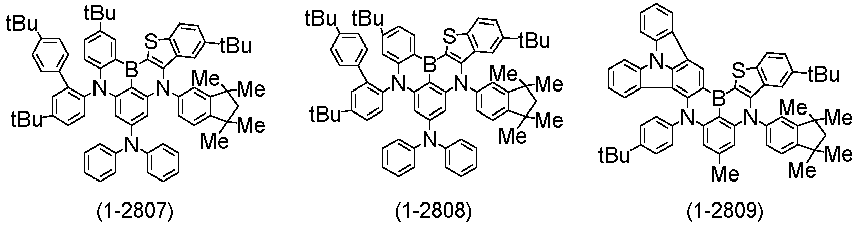

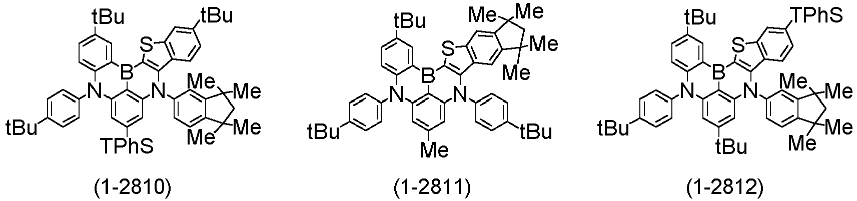

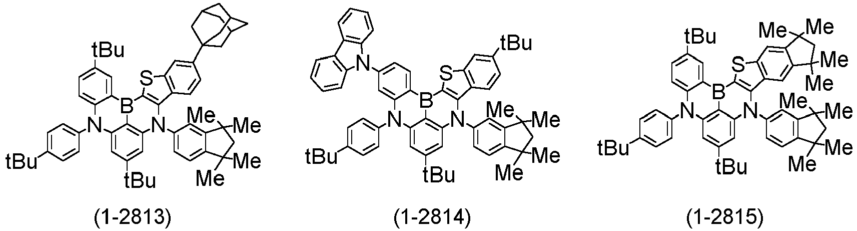

In this paragraph all compounds are inventive compounds.

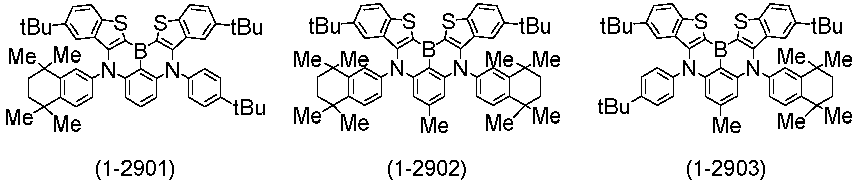

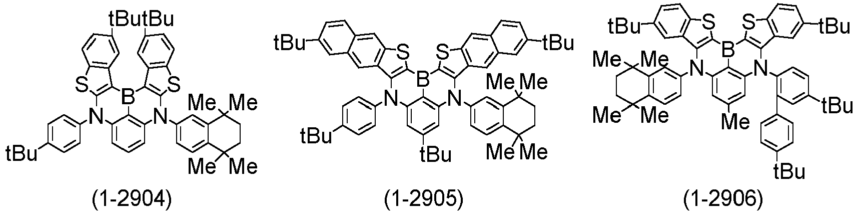

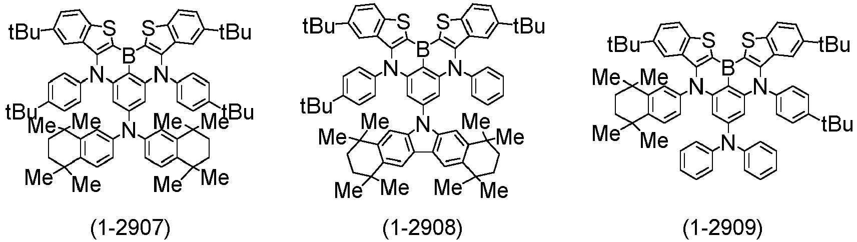

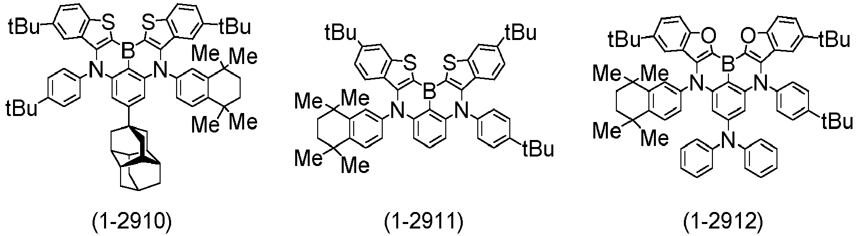

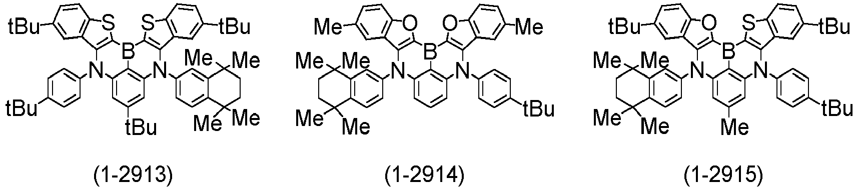

In this paragraph all compounds are inventive compounds.

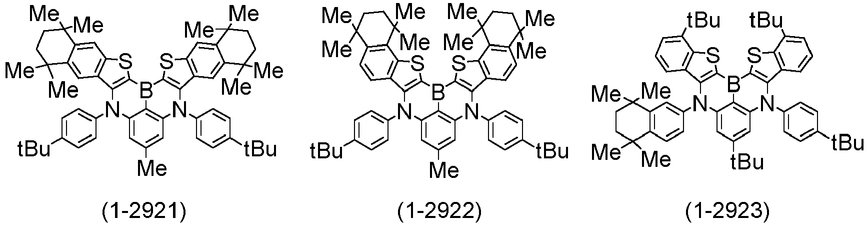

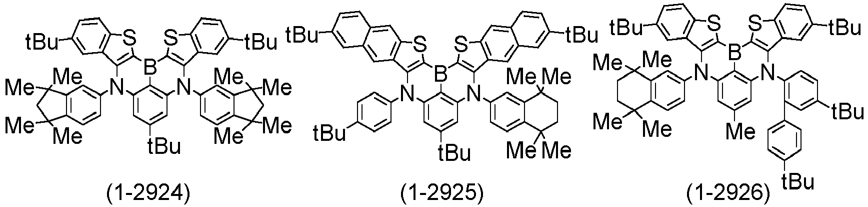

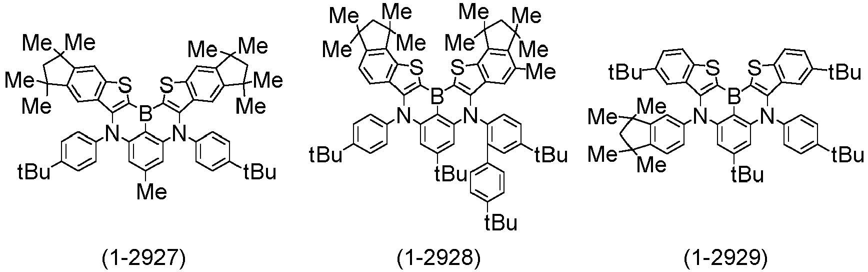

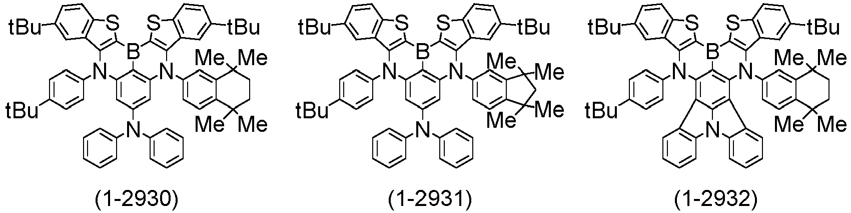

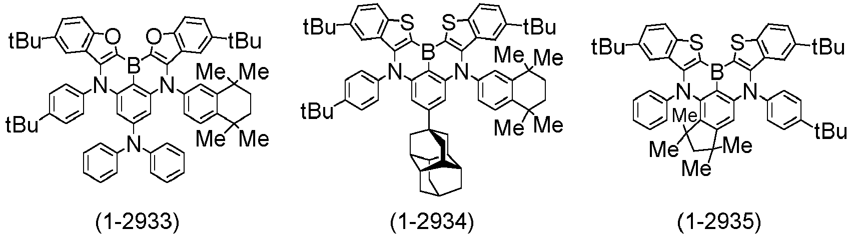

In this paragraph all compounds are inventive compounds.

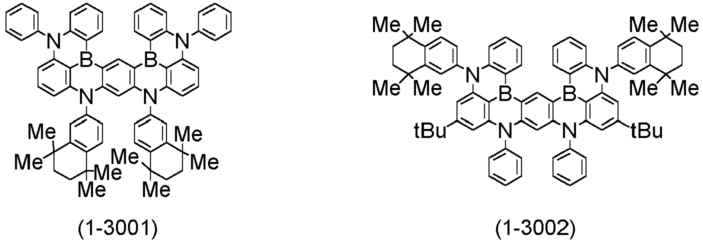

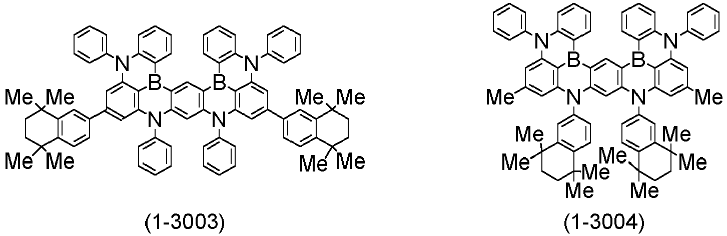

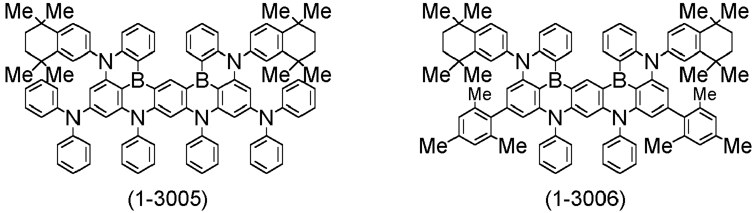

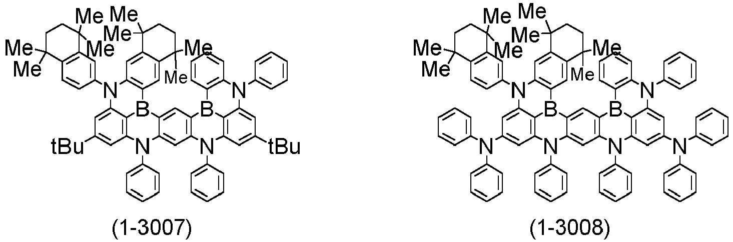

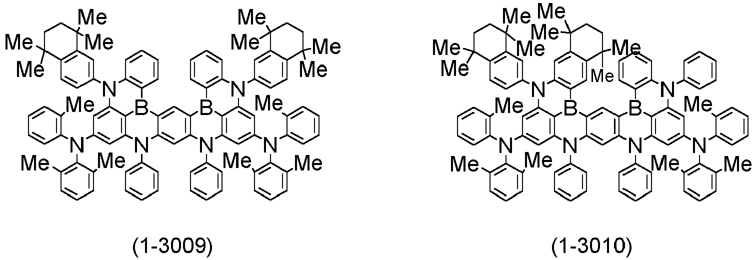

In this paragraph all compounds are inventive compounds.

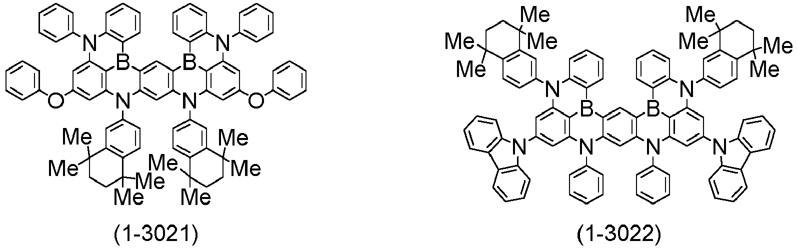

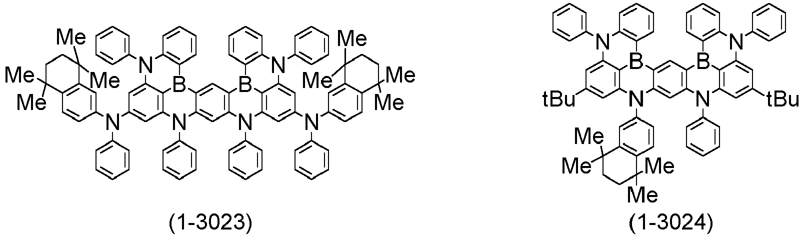

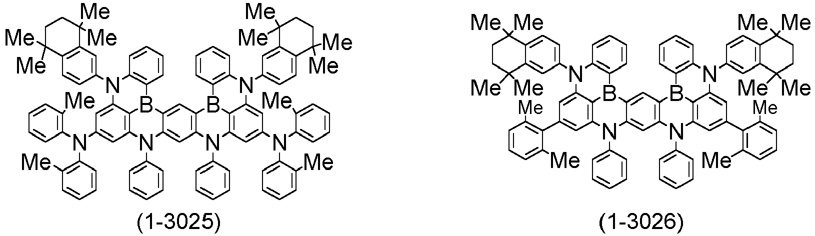

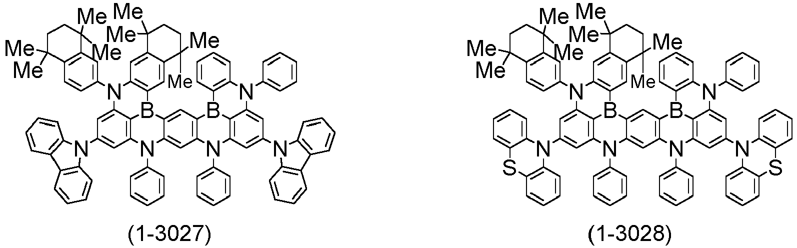

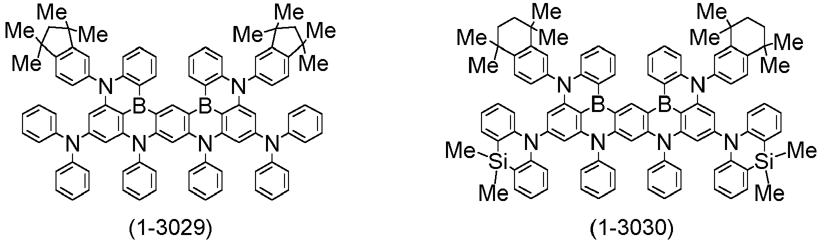

In this paragraph all compounds are inventive compounds.

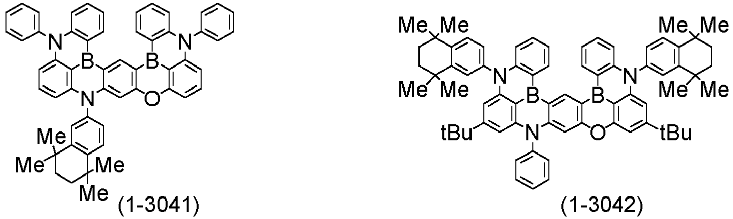

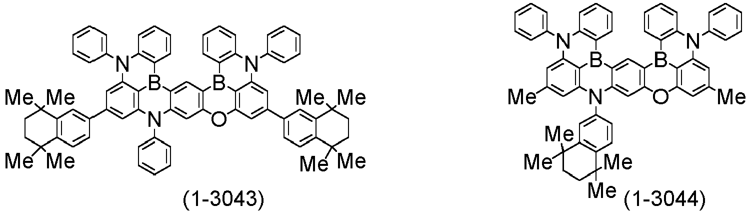

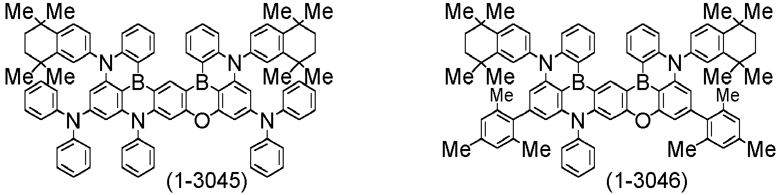

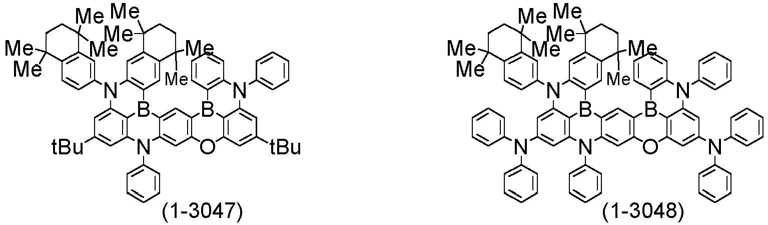

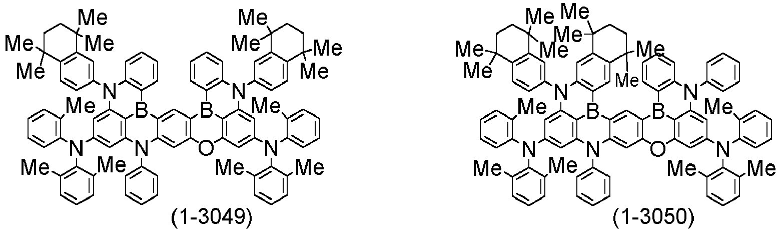

In this paragraph all compounds are inventive compounds.

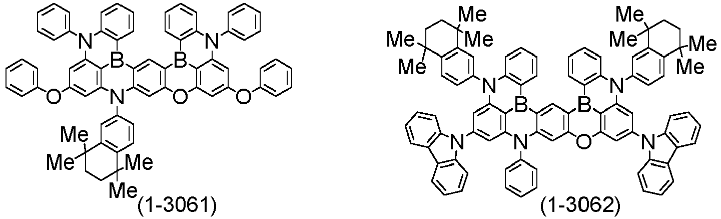

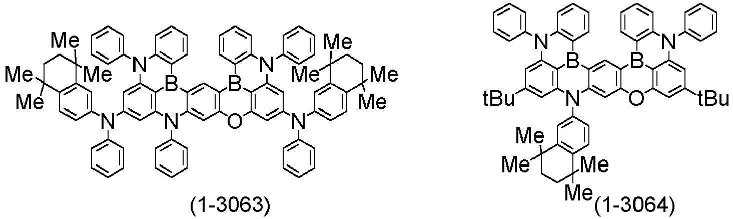

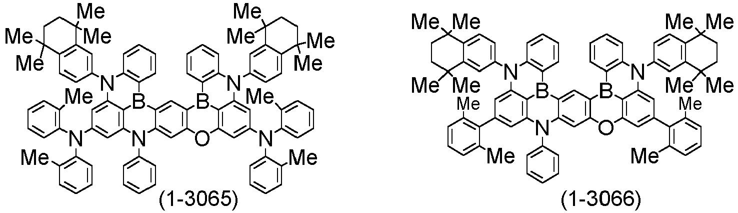

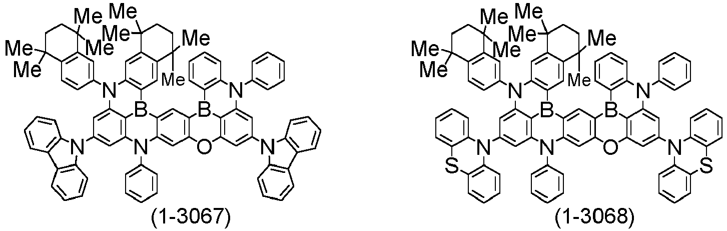

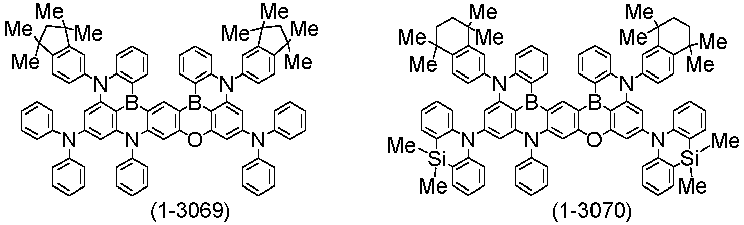

In this paragraph all compounds are inventive compounds.

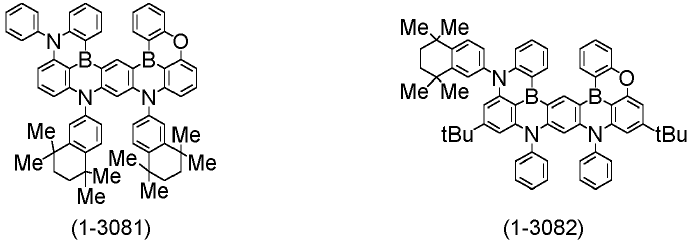

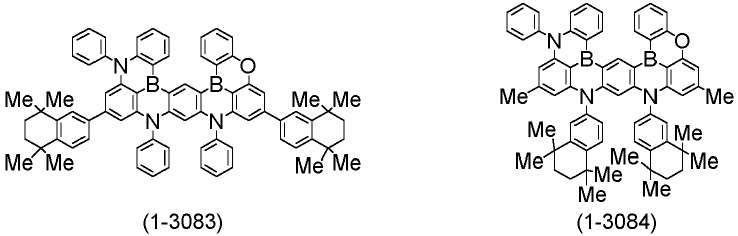

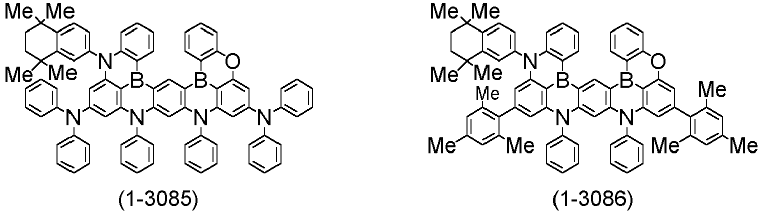

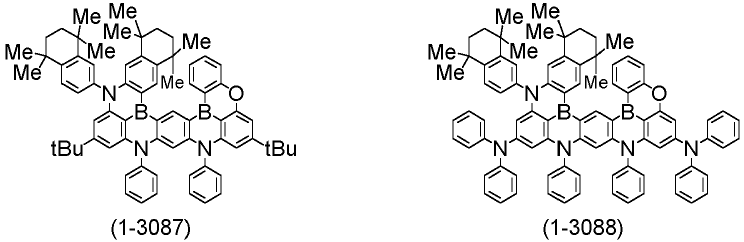

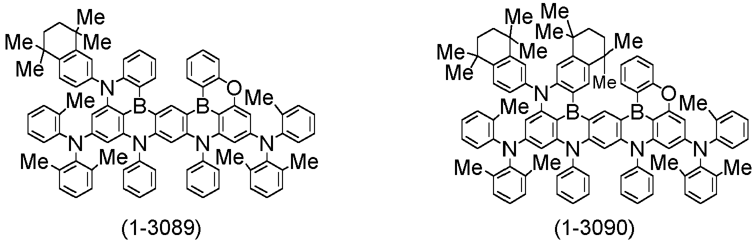

In this paragraph all compounds are inventive compounds.

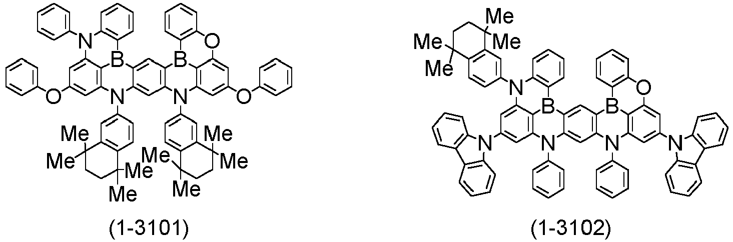

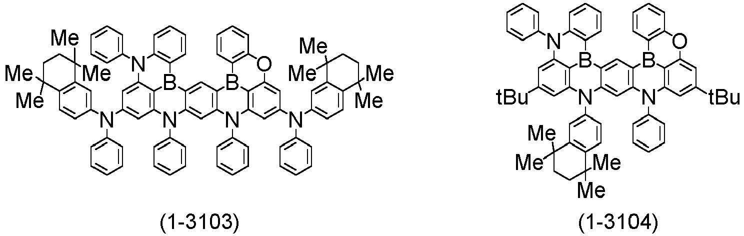

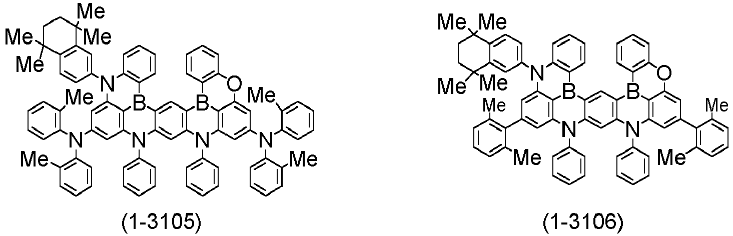

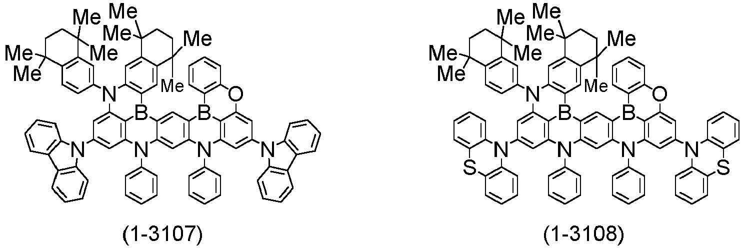

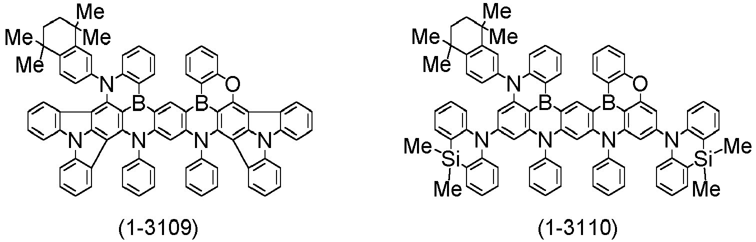

In this paragraph all compounds are inventive compounds.

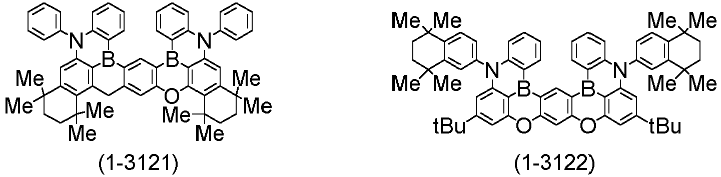

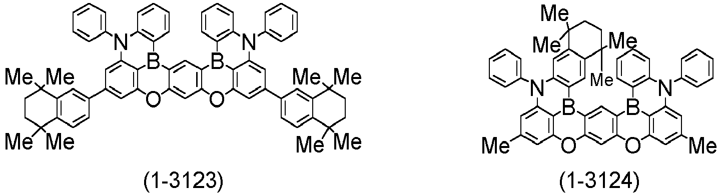

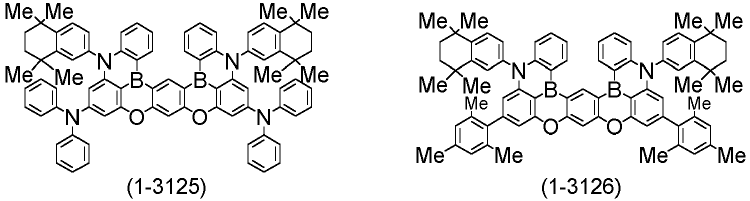

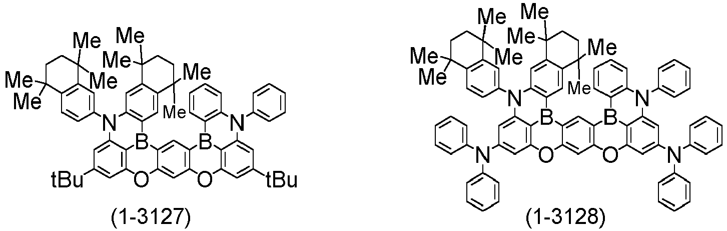

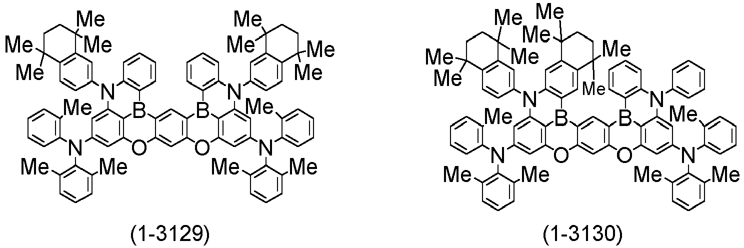

In this paragraph all compounds are inventive compounds.

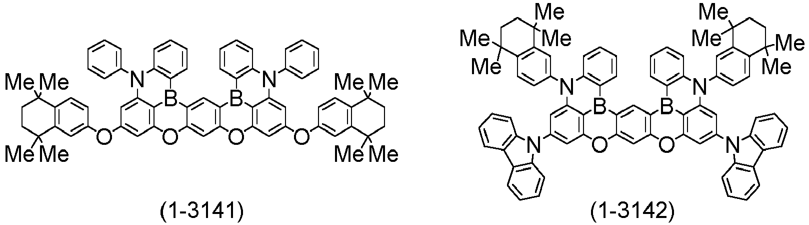

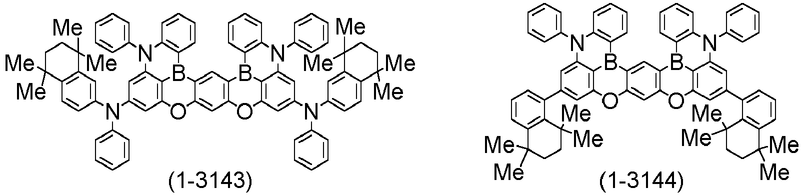

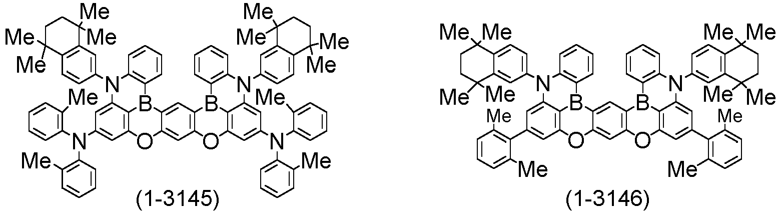

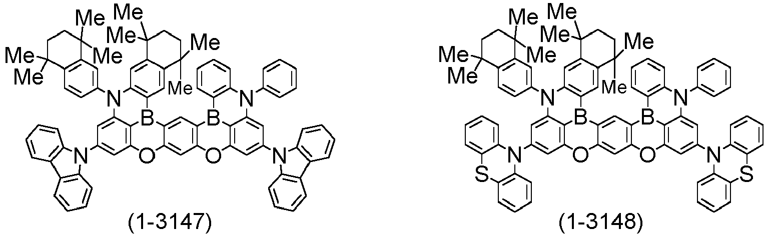

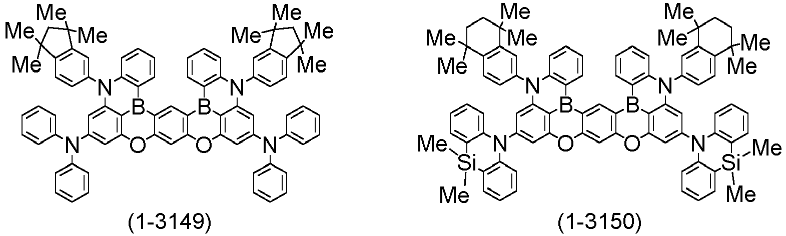

In this paragraph all compounds are inventive compounds.

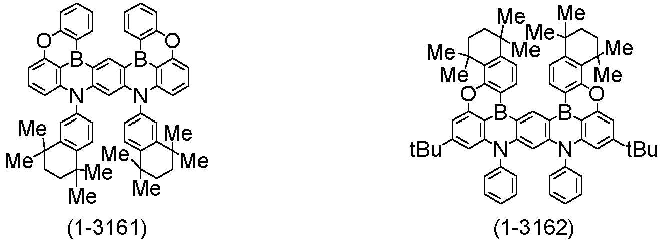

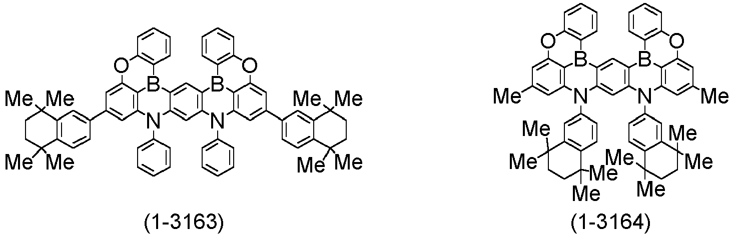

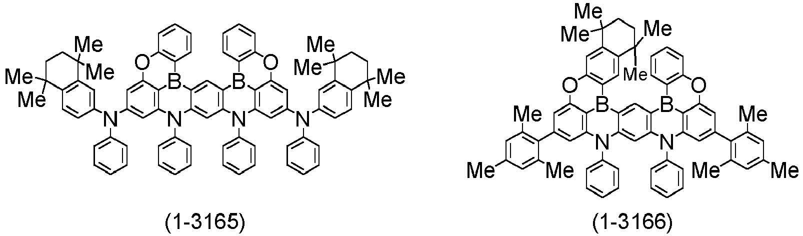

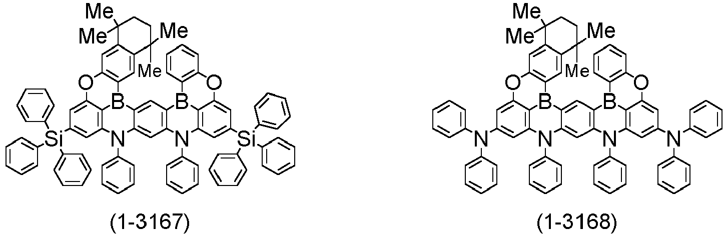

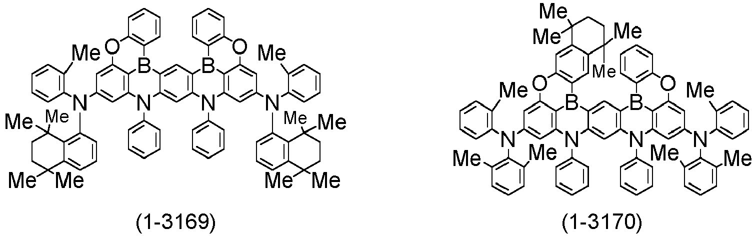

In this paragraph all compounds are inventive compounds.

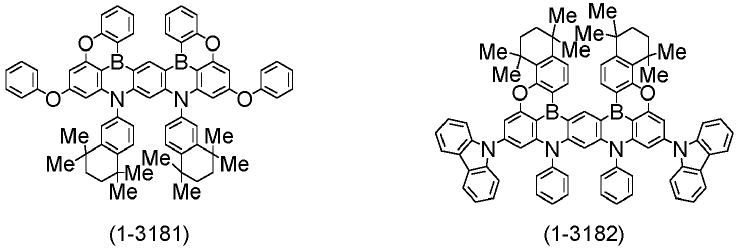

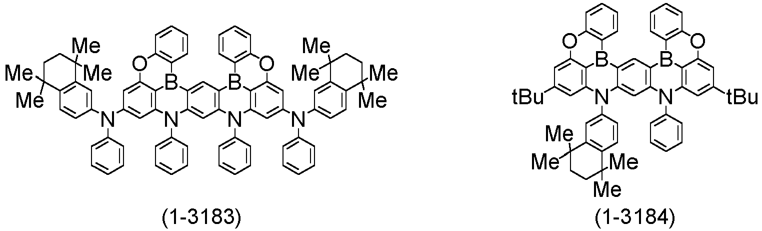

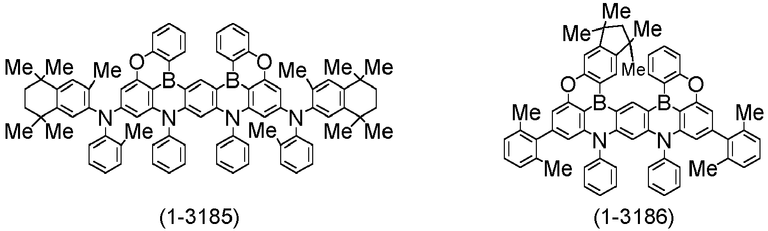

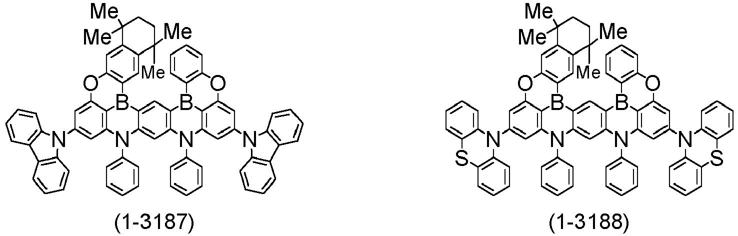

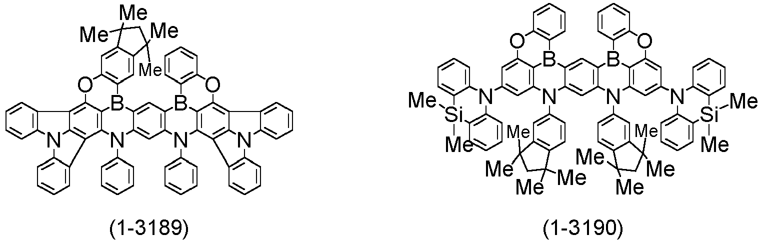

In this paragraph all compounds are inventive compounds.

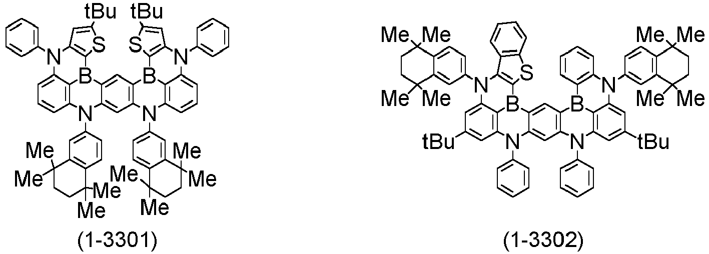

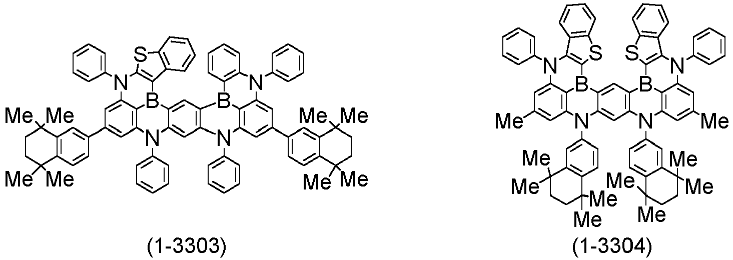

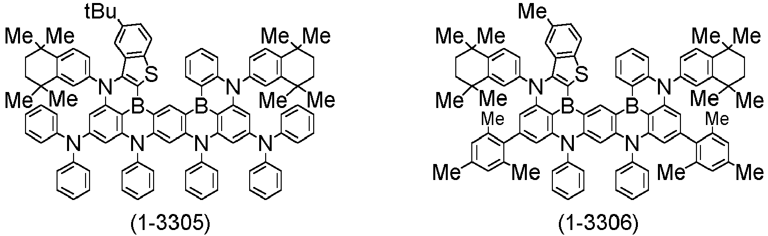

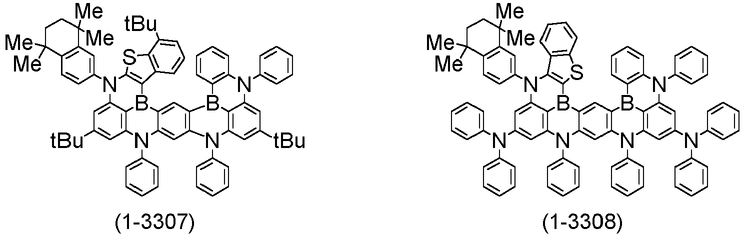

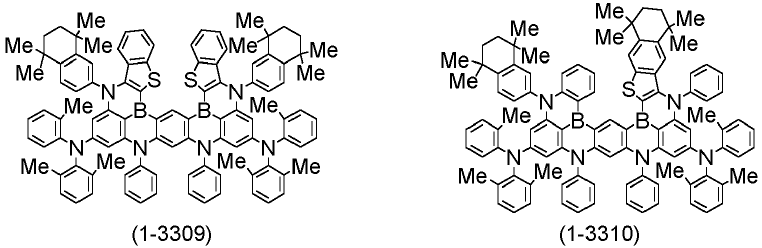

In this paragraph all compounds are inventive compounds.

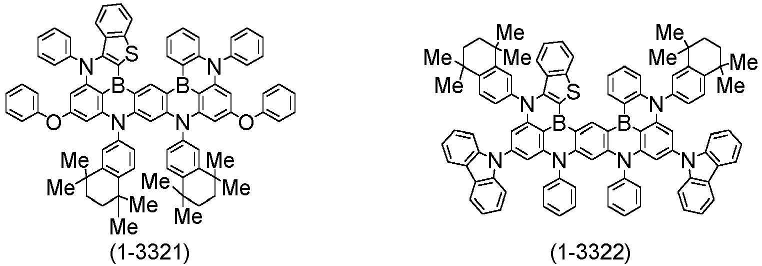

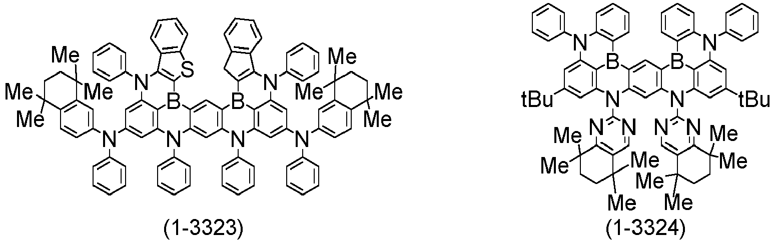

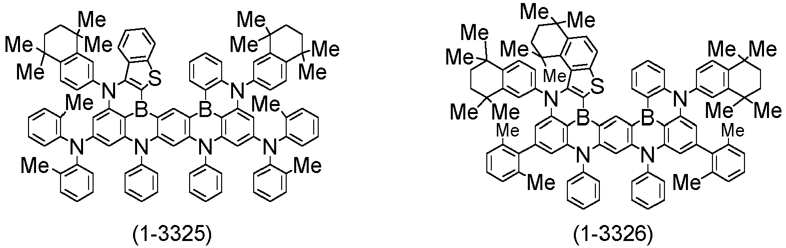

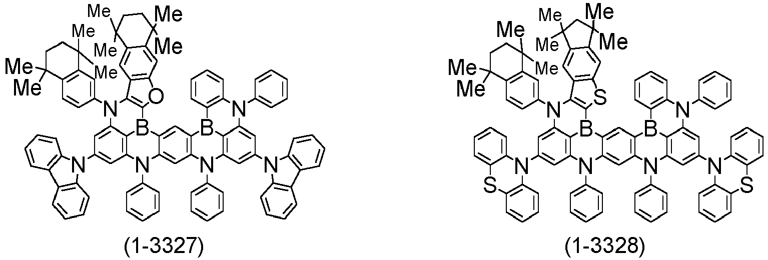

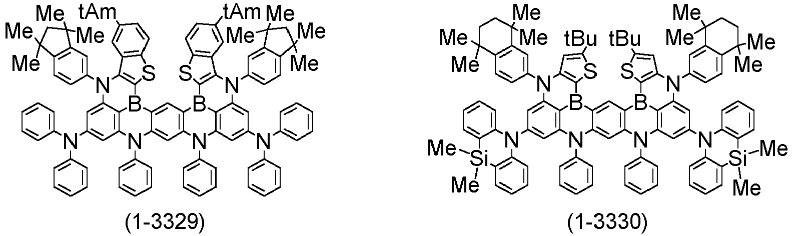

In this paragraph all compounds are inventive compounds.

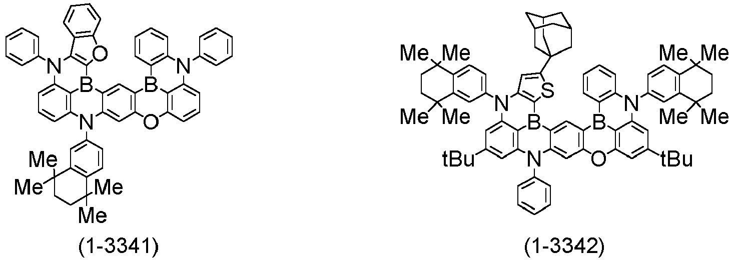

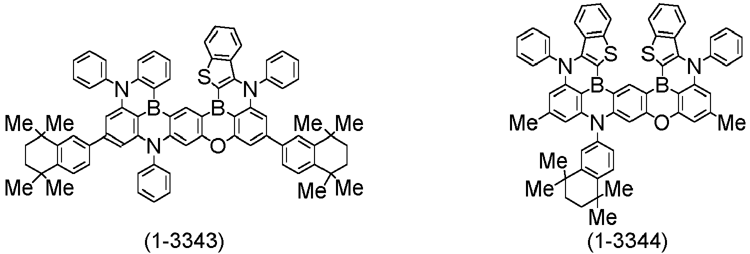

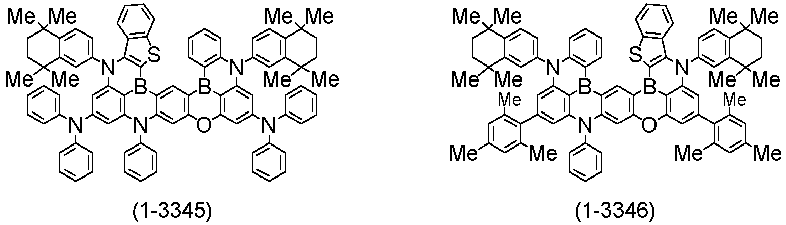

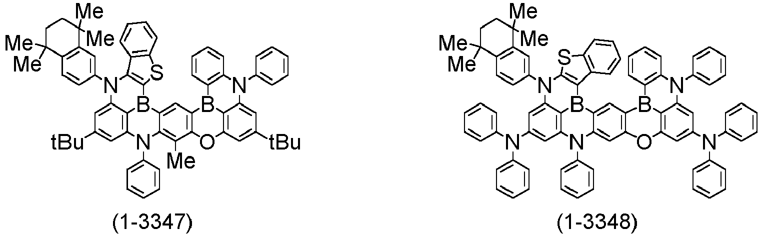

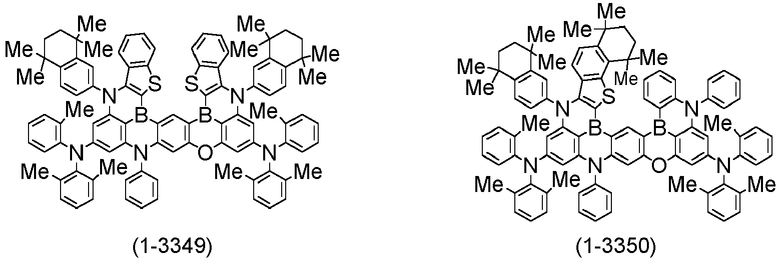

In this paragraph all compounds except compound (1-3365) are inventive compounds.

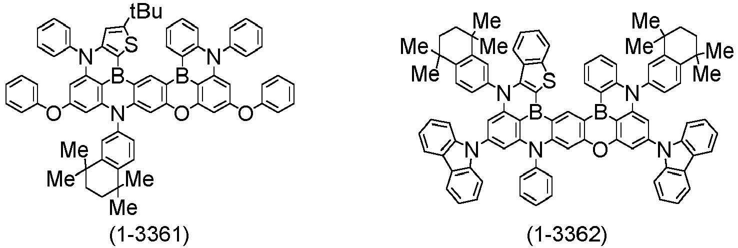

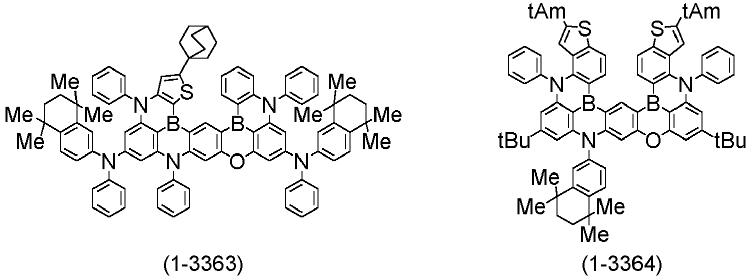

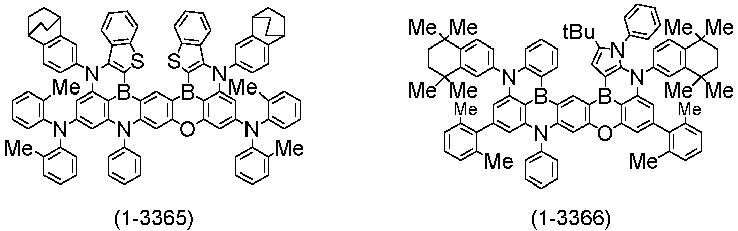

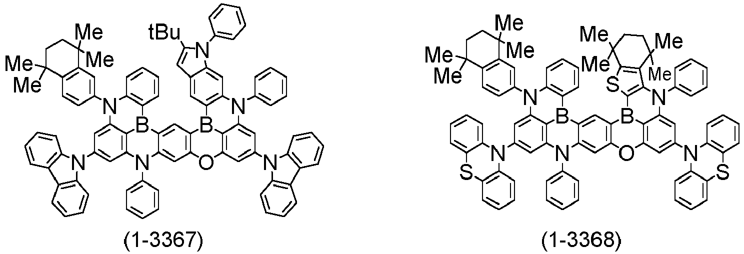

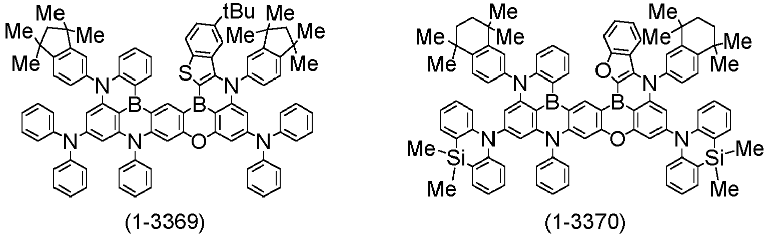

In this paragraph all compounds are inventive compounds.

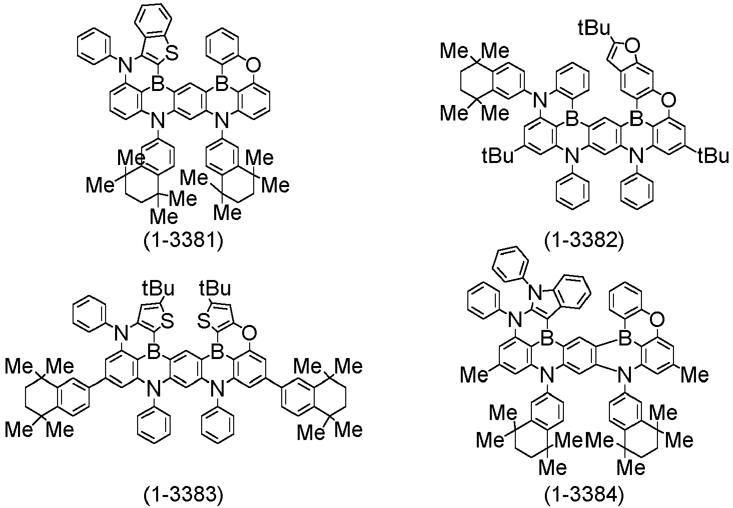

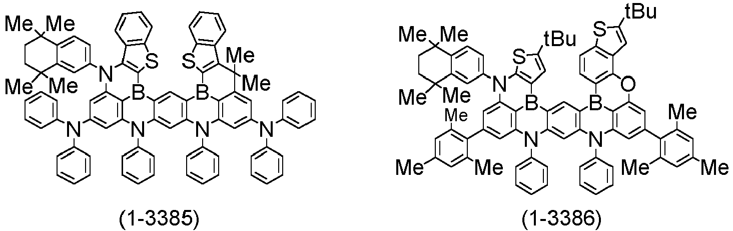

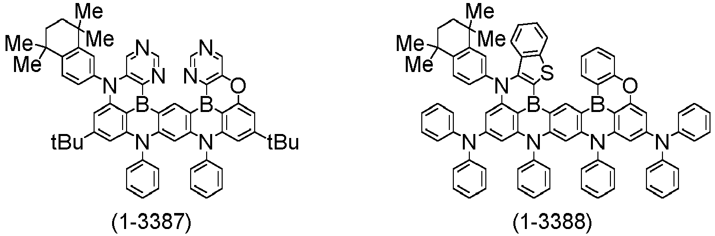

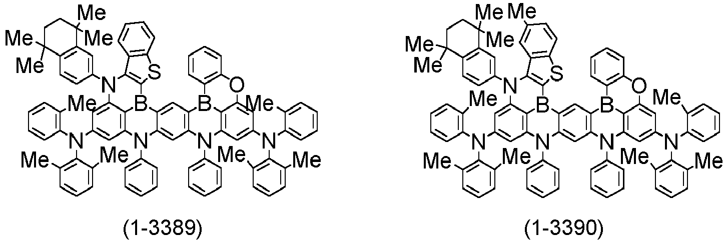

In this paragraph all compounds are inventive compounds.

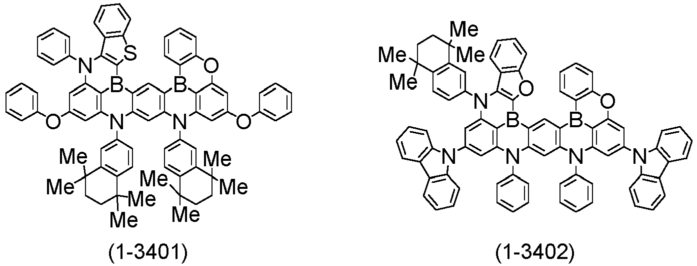

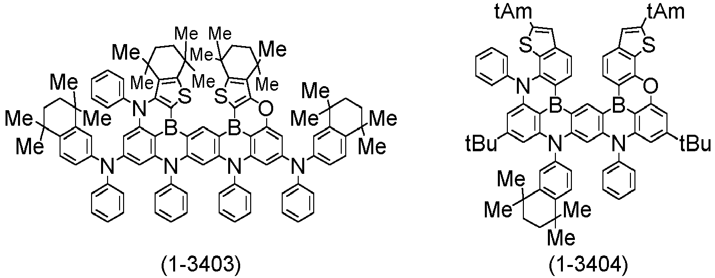

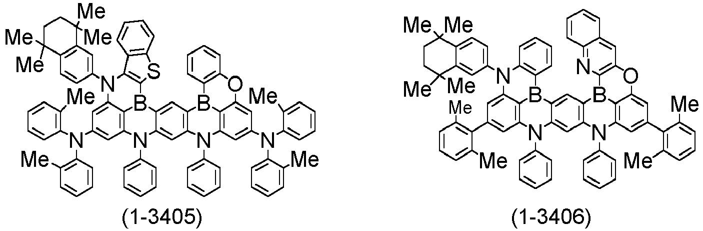

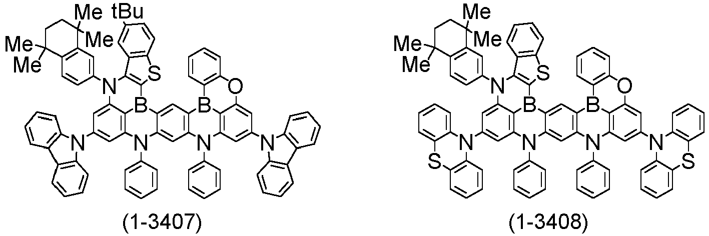

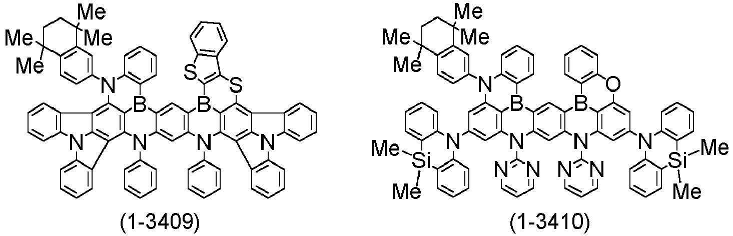

In this paragraph all compounds except compounds (1-3424), (1-3430) are inventive compounds.

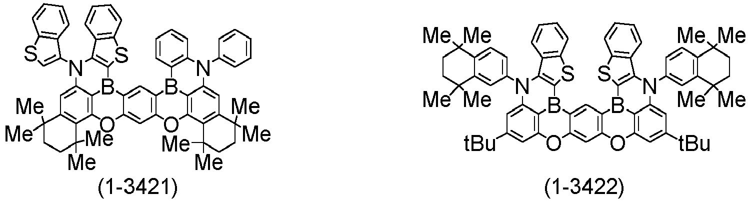

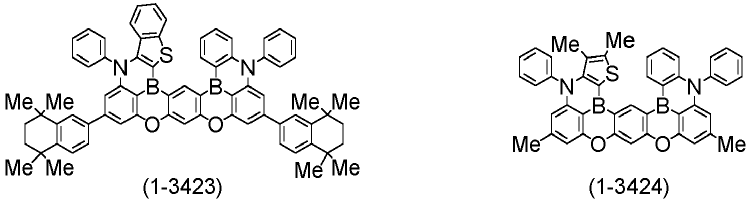

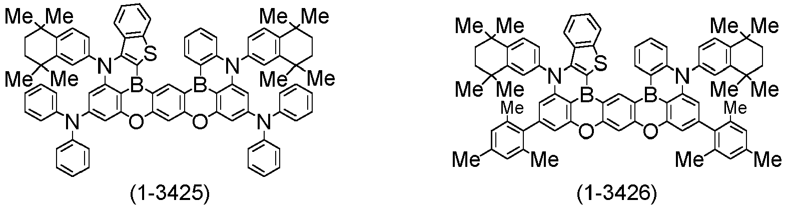

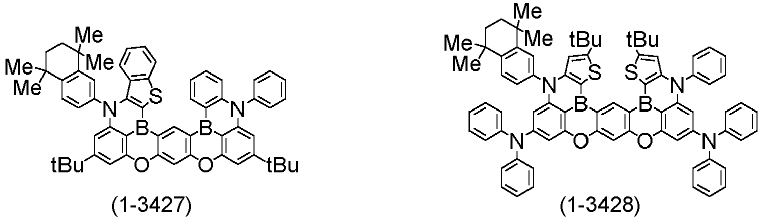

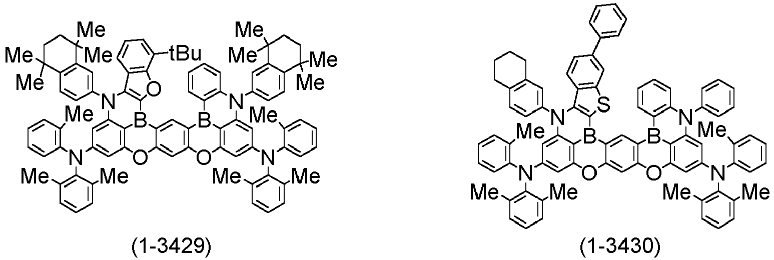

In this paragraph all compounds are inventive compounds.

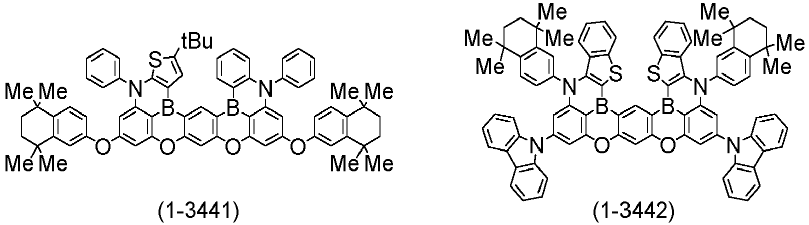

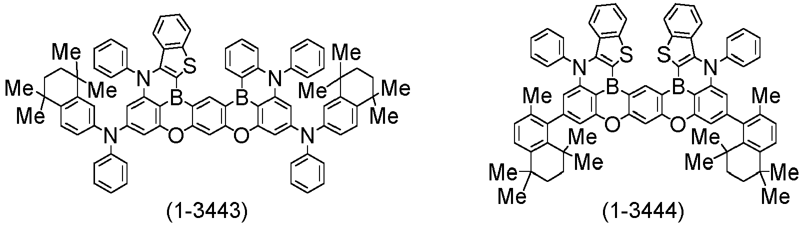

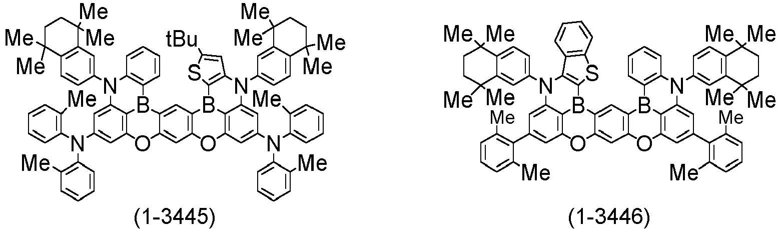

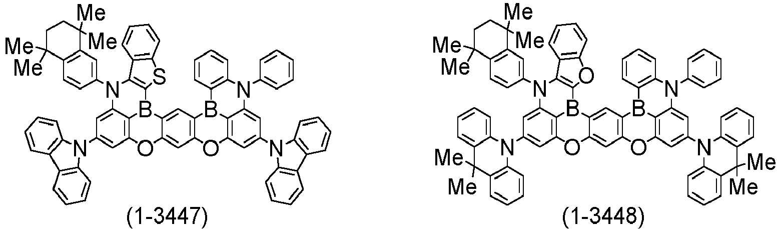

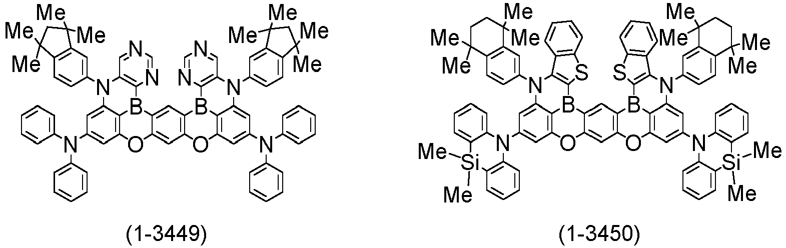

In this paragraph all compounds are inventive compounds.

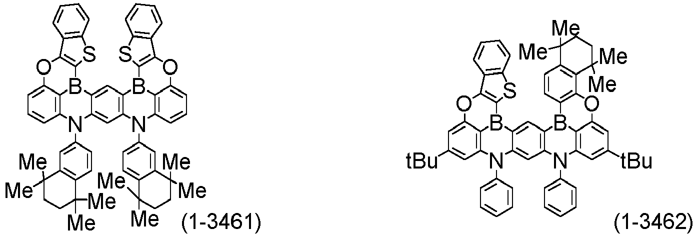

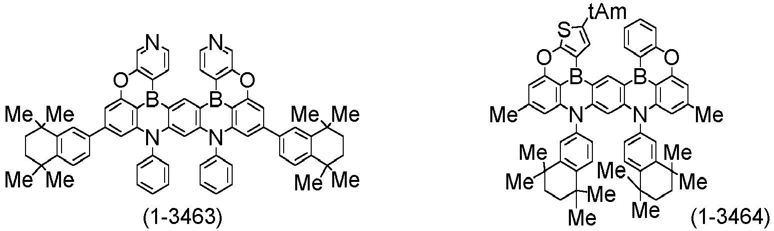

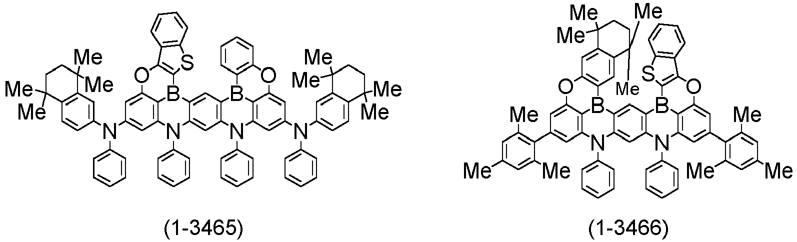

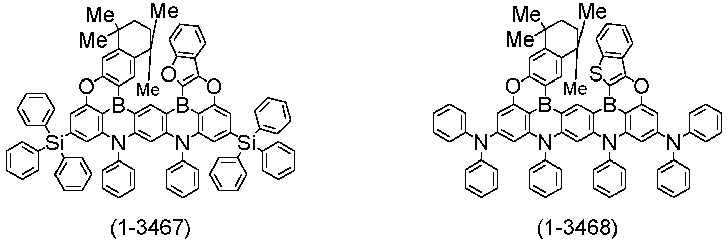

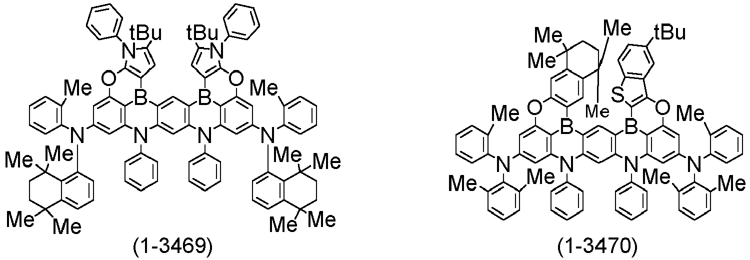

In this paragraph all compounds except compound (1-3489) are inventive compounds.

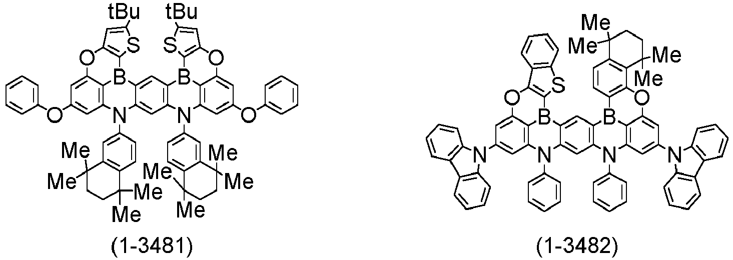

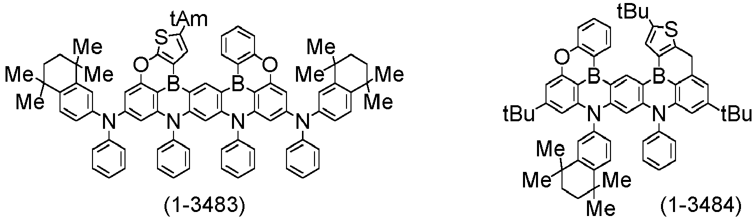

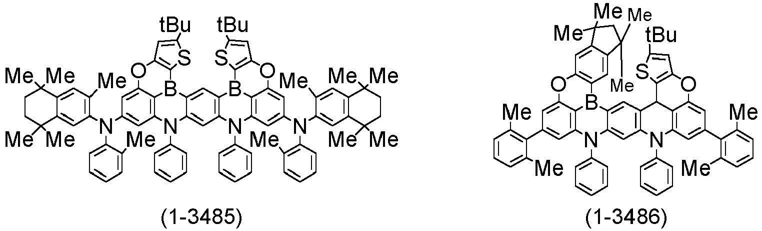

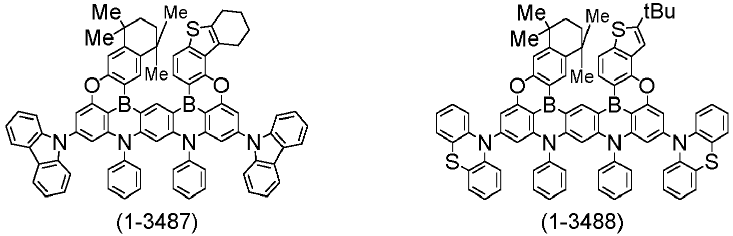

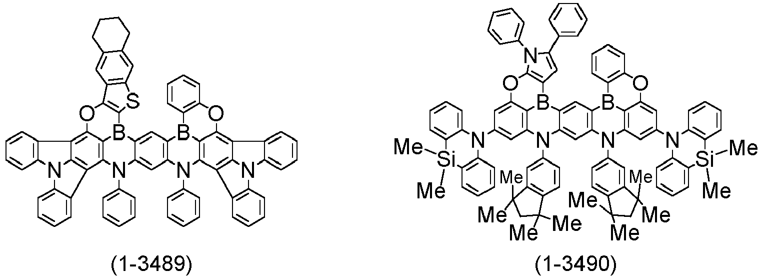

In this paragraph all compounds are inventive compounds.

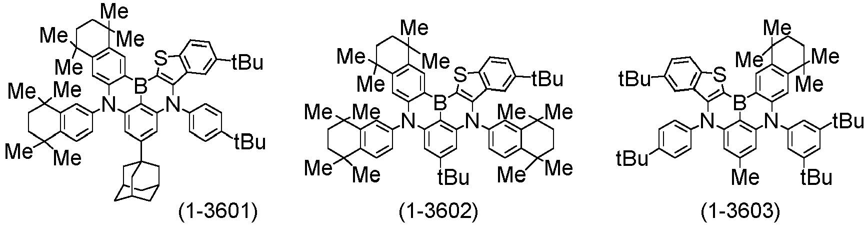

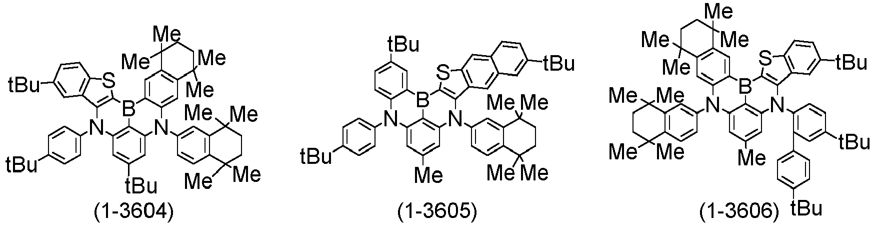

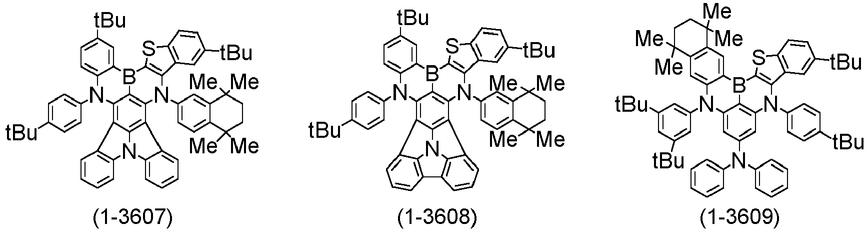

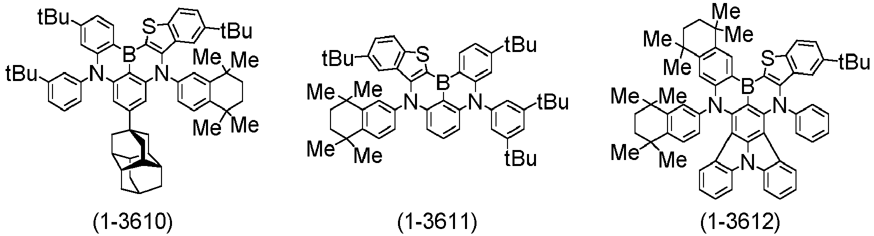

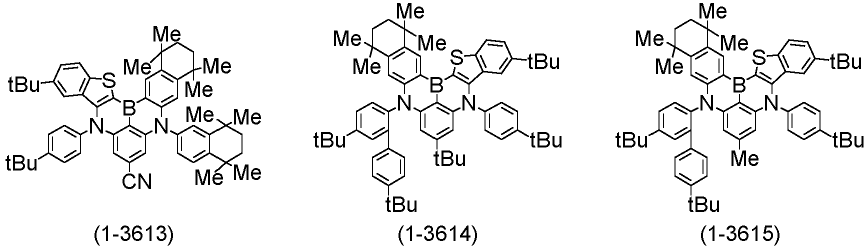

In this paragraph all compounds are inventive compounds.

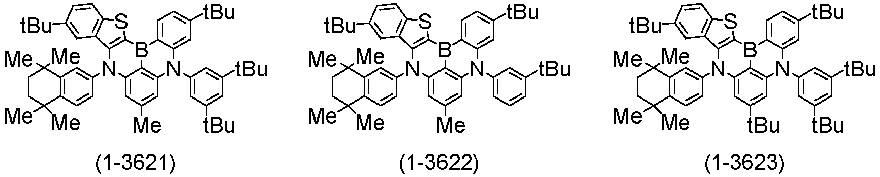

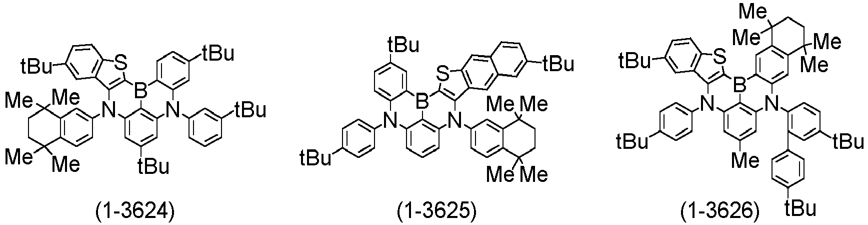

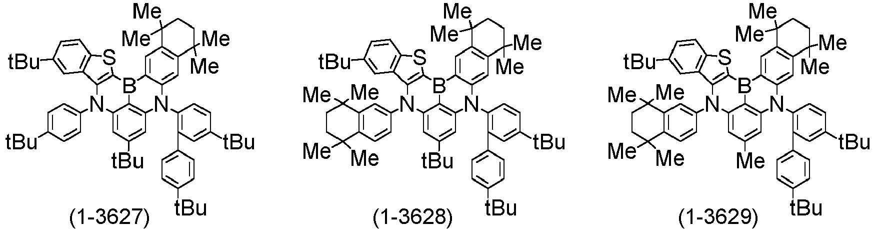

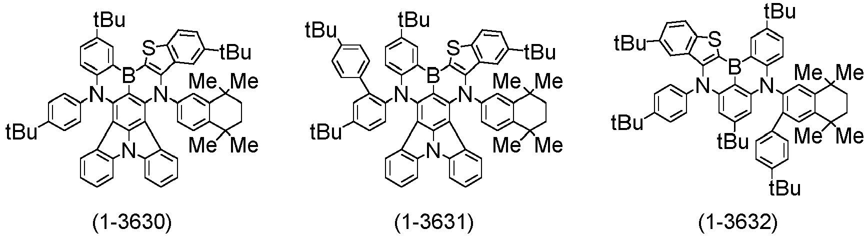

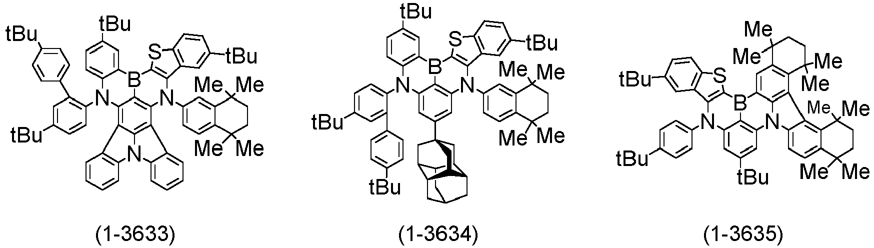

In this paragraph all compounds are inventive compounds.

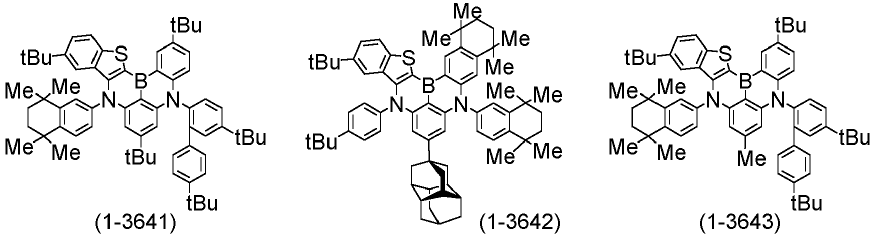

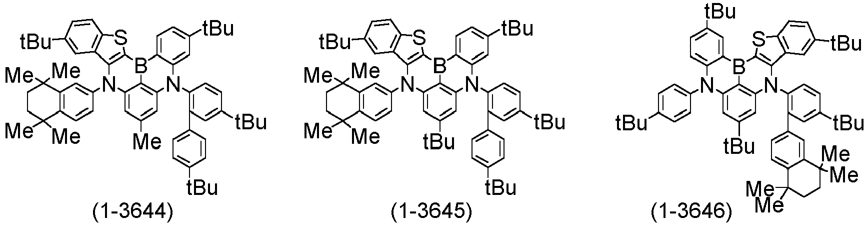

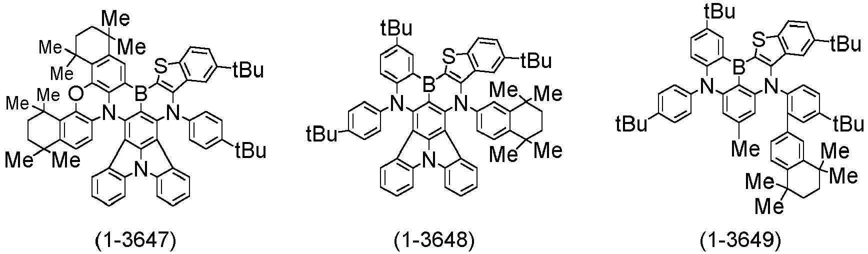

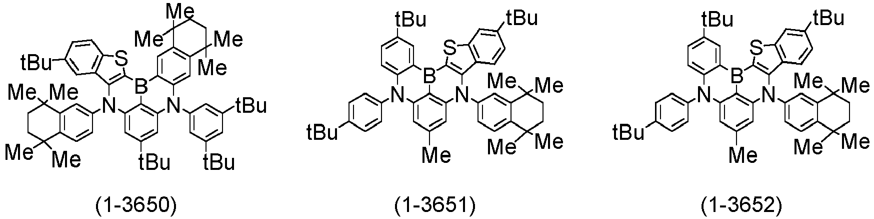

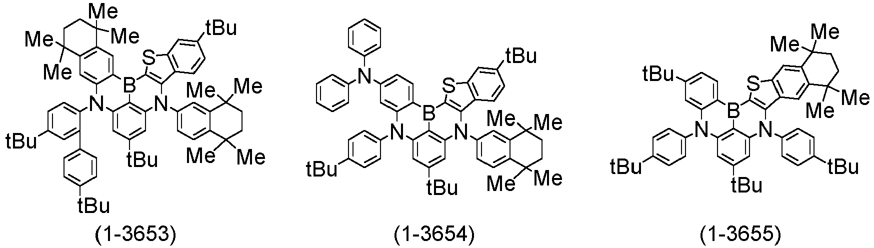

In this paragraph all compounds are inventive compounds.

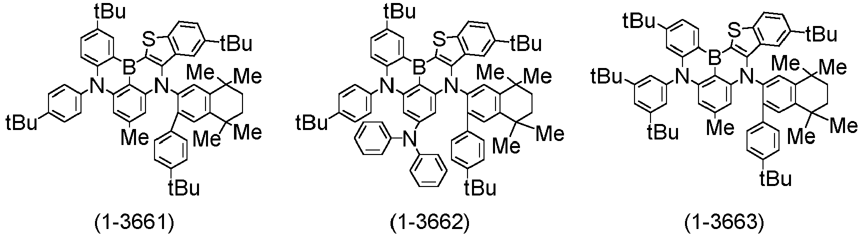

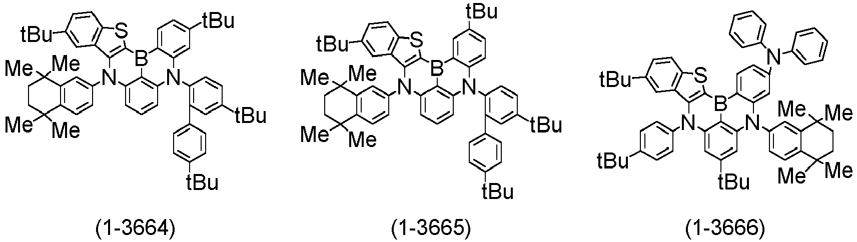

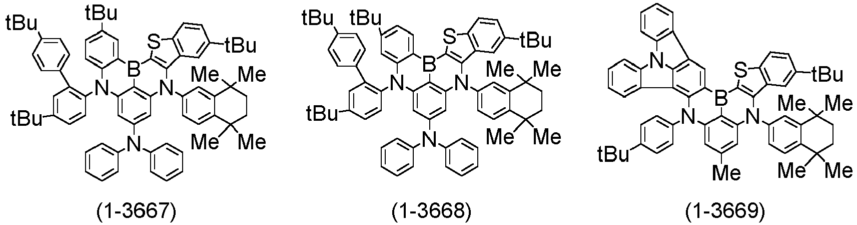

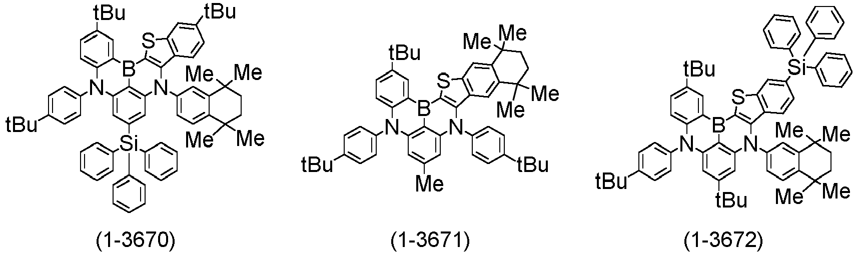

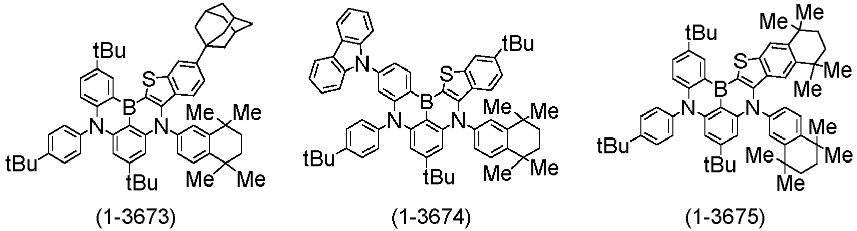

In this paragraph all compounds are inventive compounds.

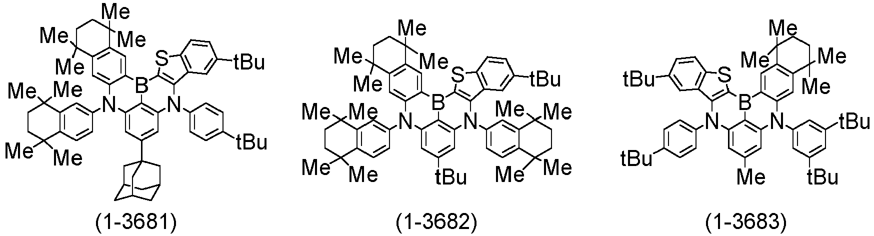

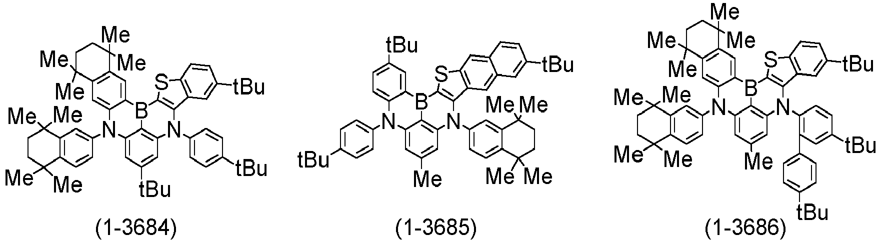

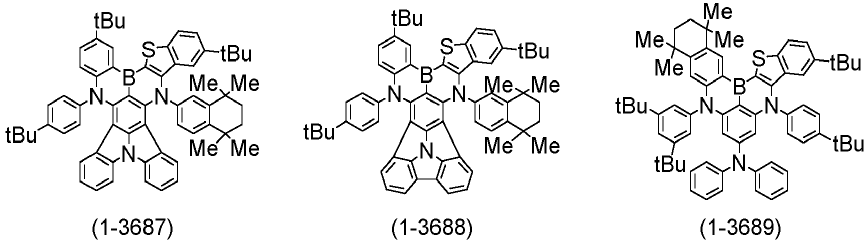

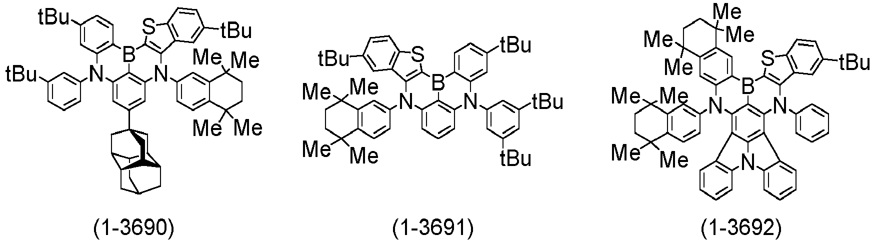

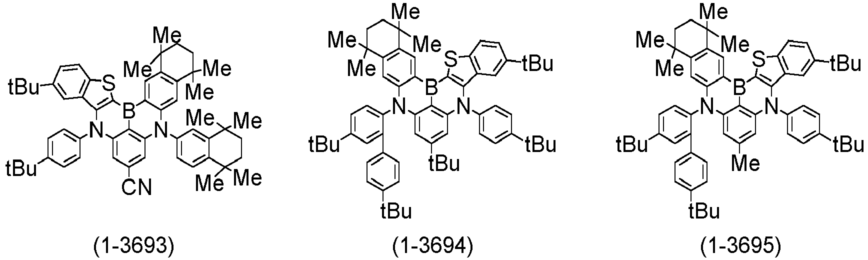

In this paragraph all compounds are inventive compounds.

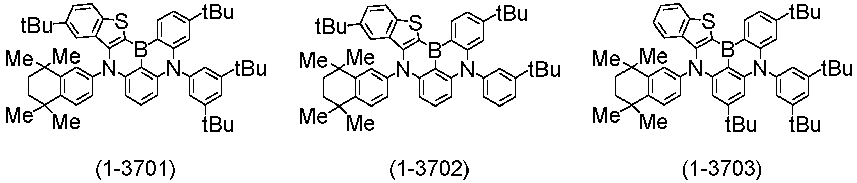

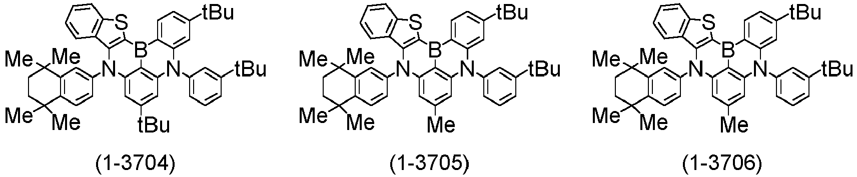

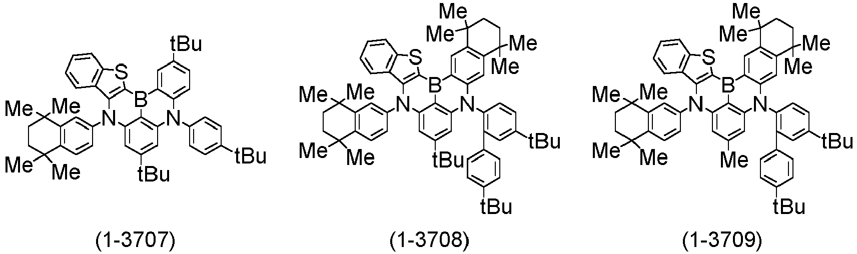

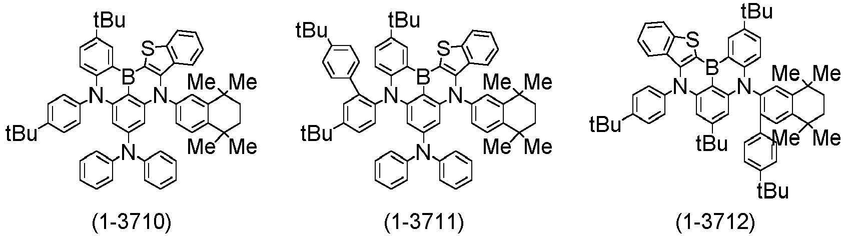

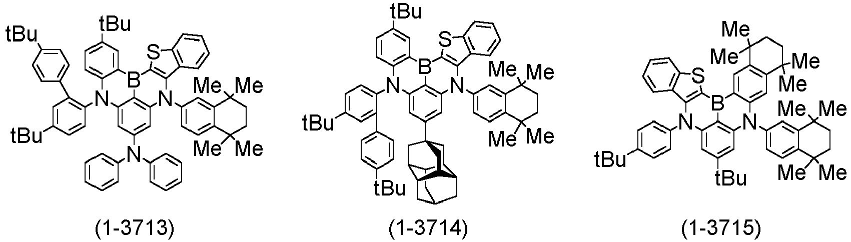

In this paragraph all compounds are inventive compounds.

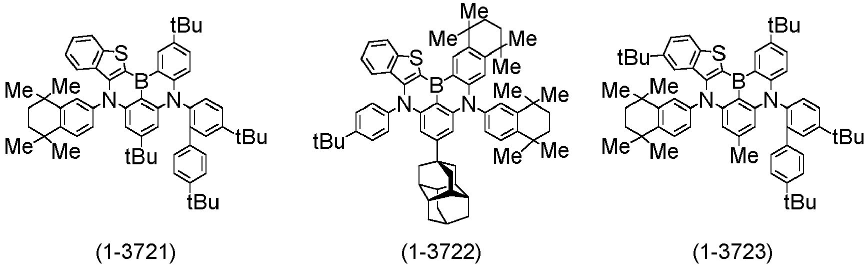

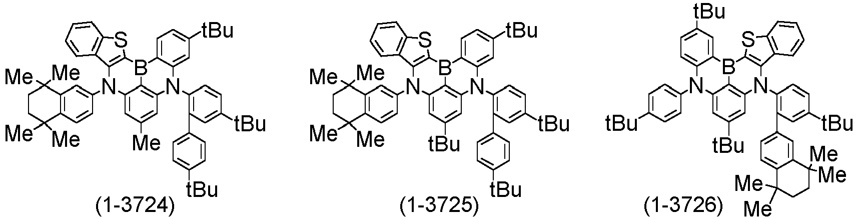

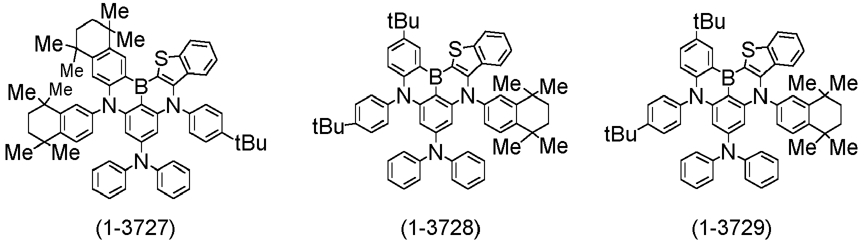

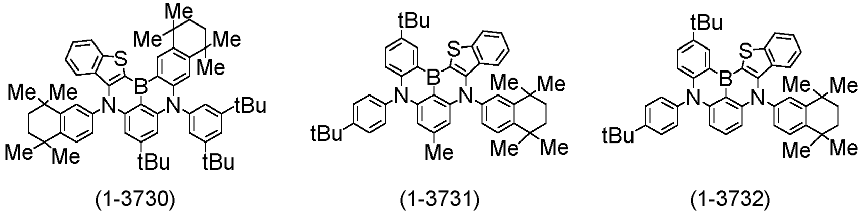

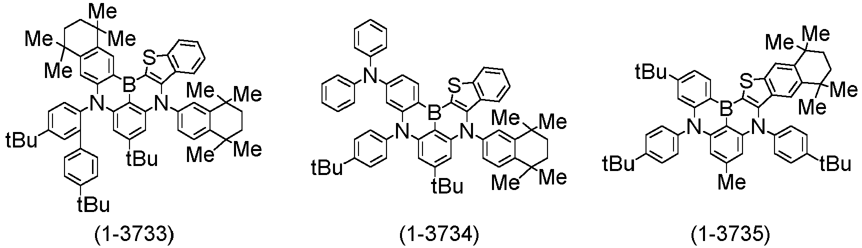

In this paragraph all compounds are inventive compounds.

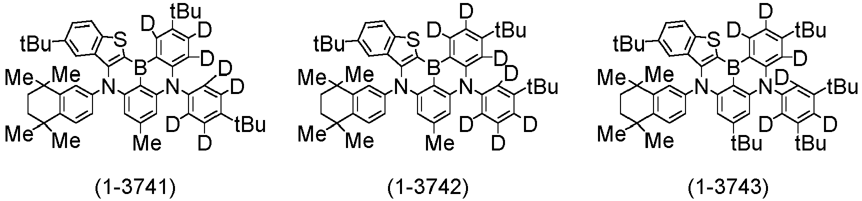

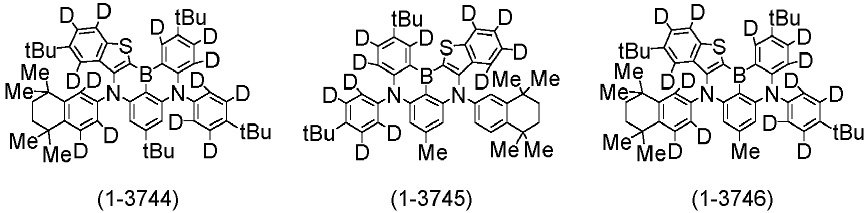

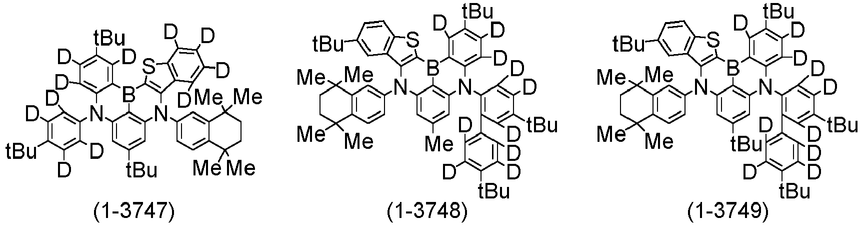

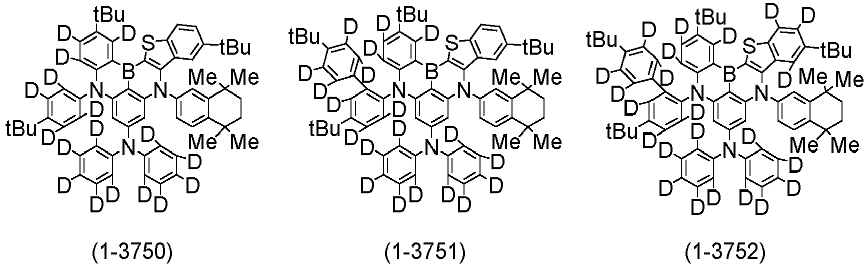

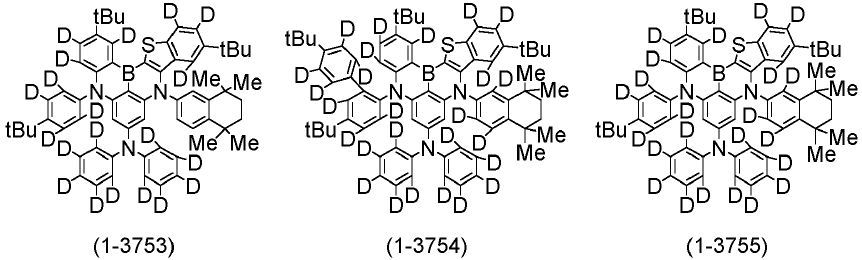

In this paragraph all compounds are inventive compounds.

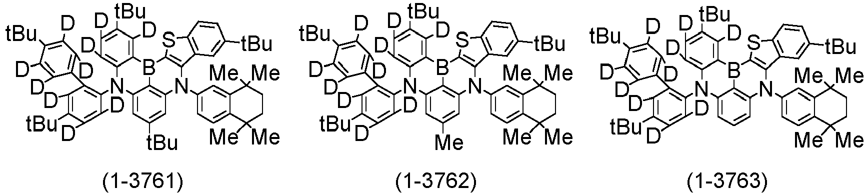

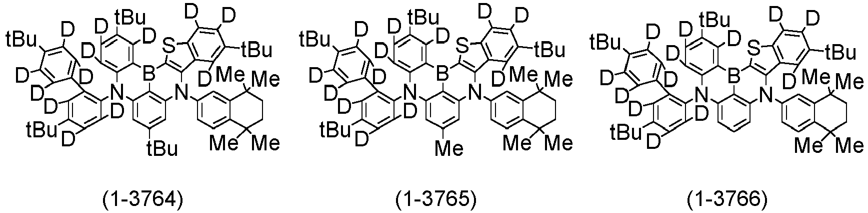

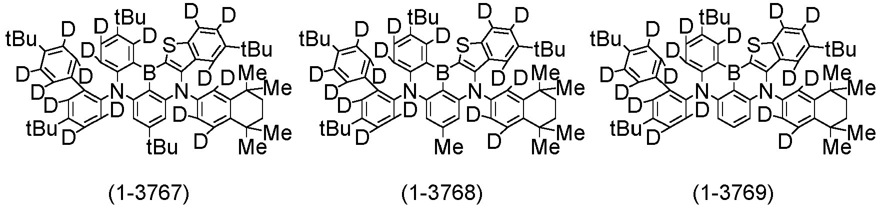

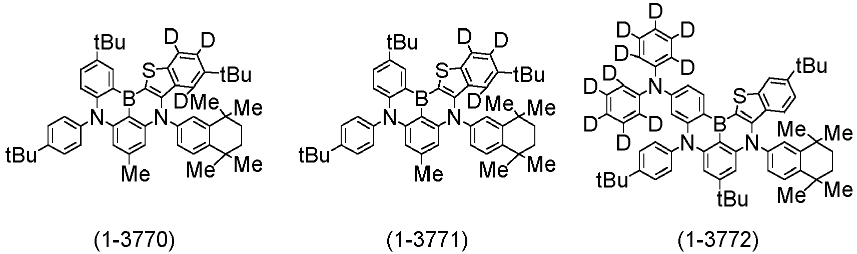

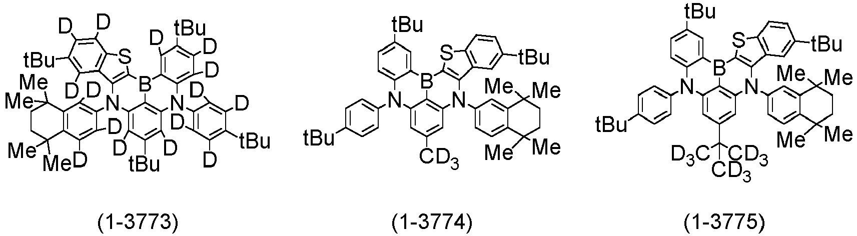

In this paragraph all compounds are inventive compounds.

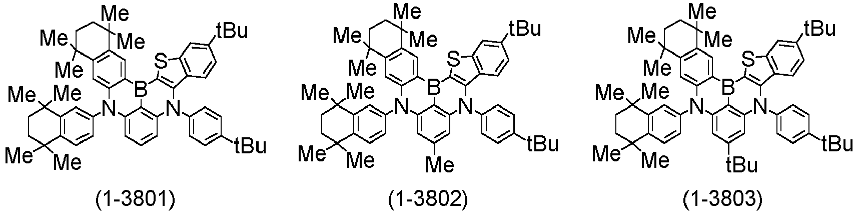

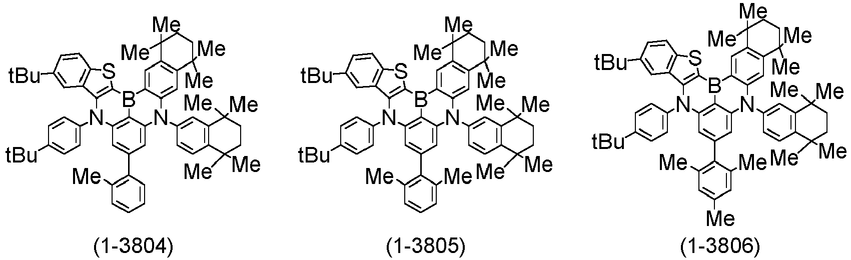

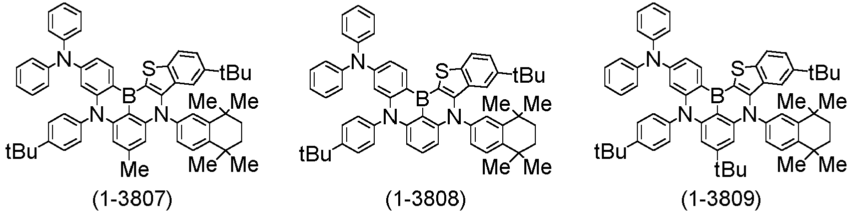

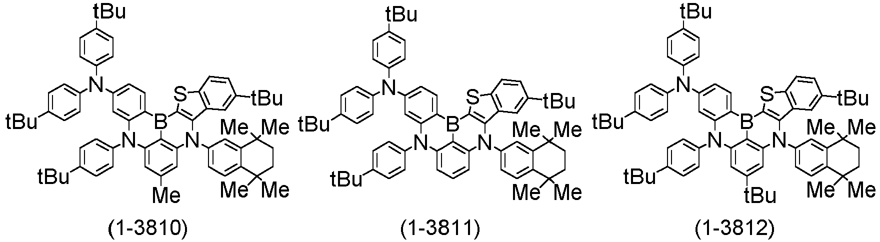

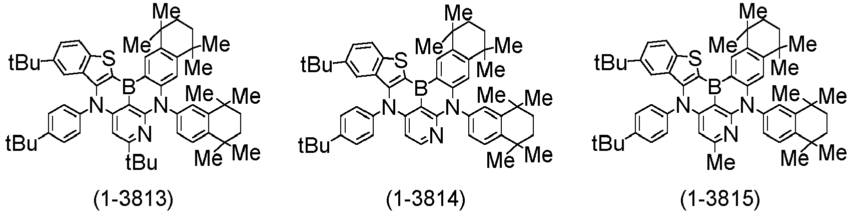

In this paragraph all compounds are inventive compounds.

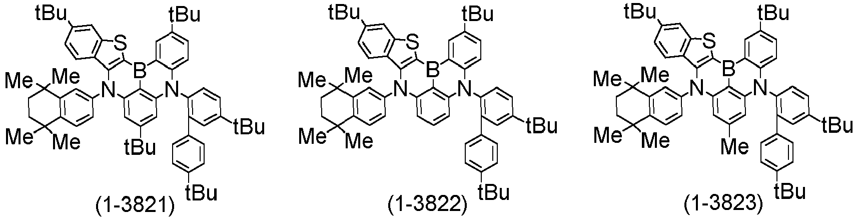

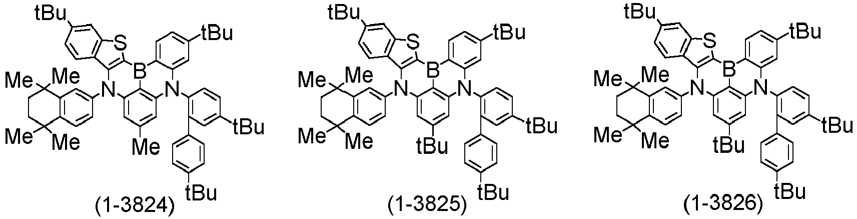

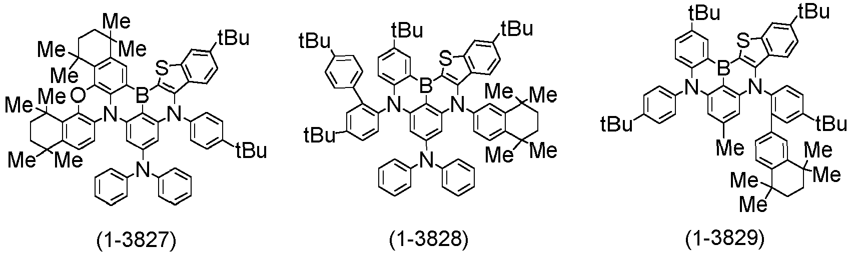

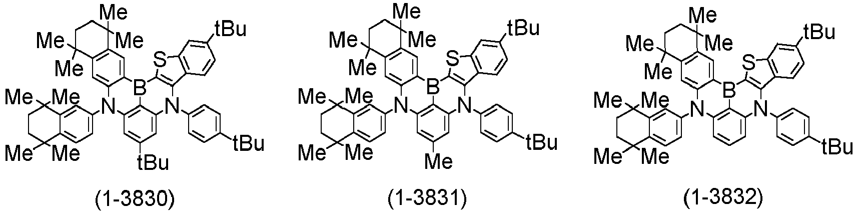

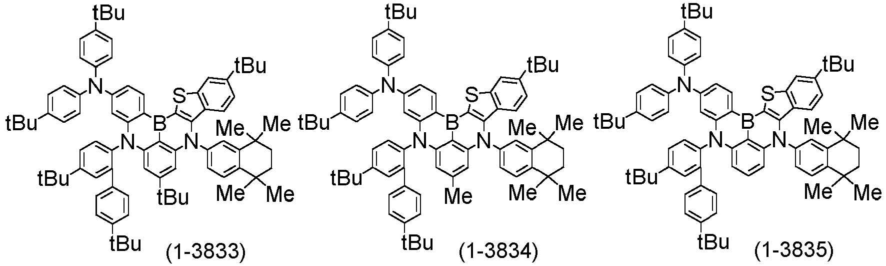

In this paragraph all compounds are inventive compounds.

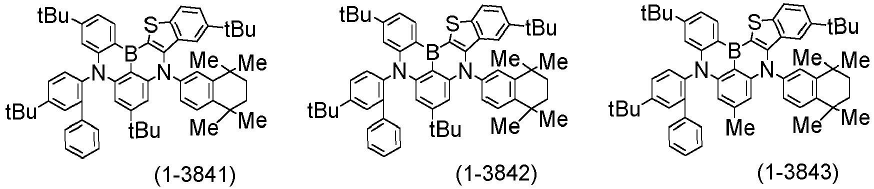

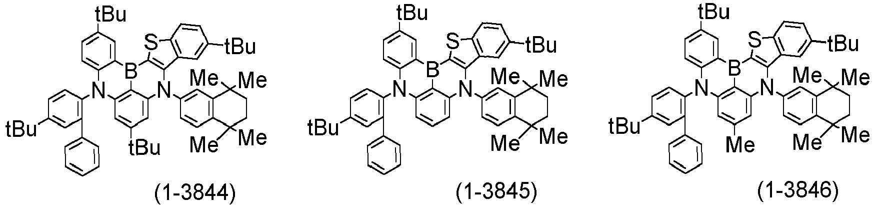

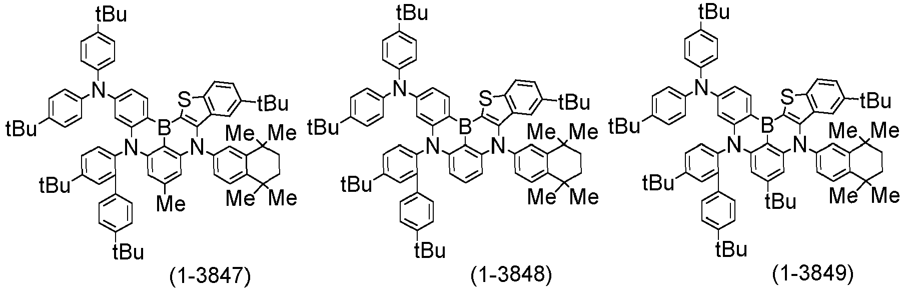

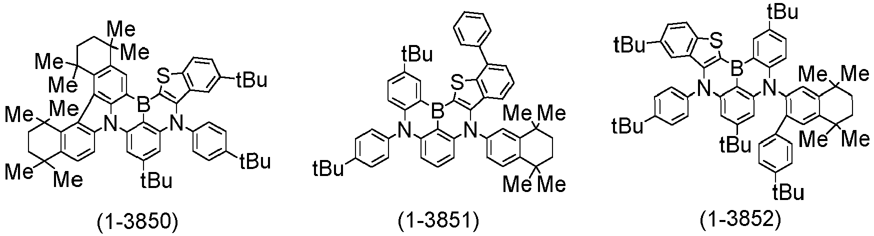

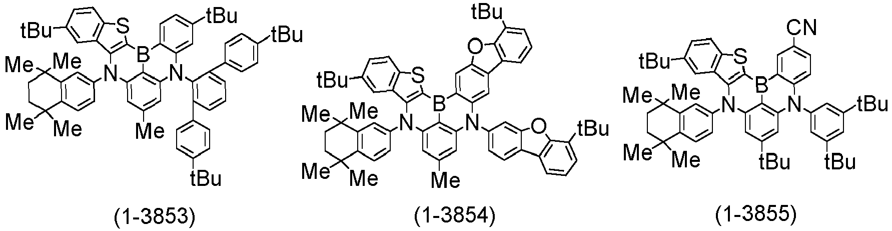

In this paragraph all compounds are inventive compounds.

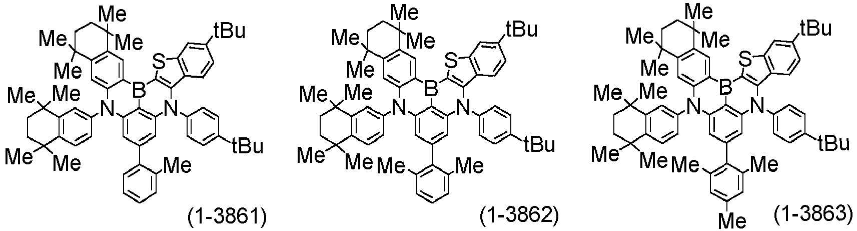

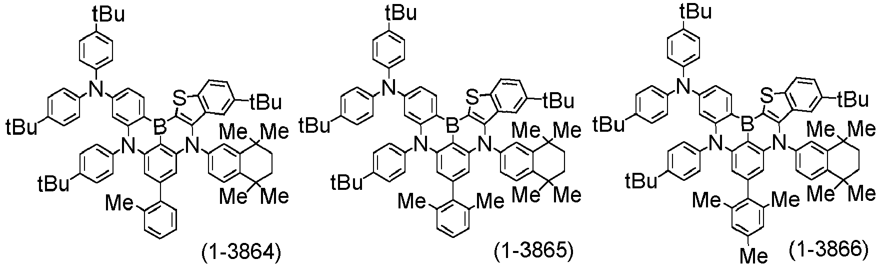

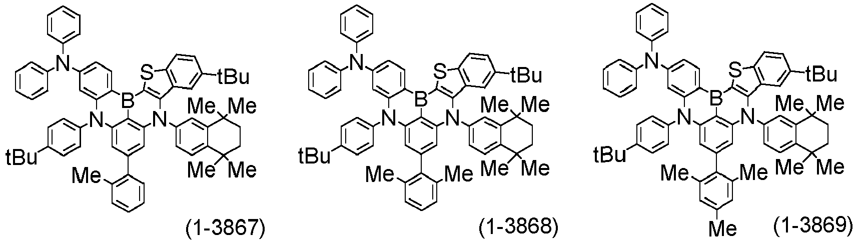

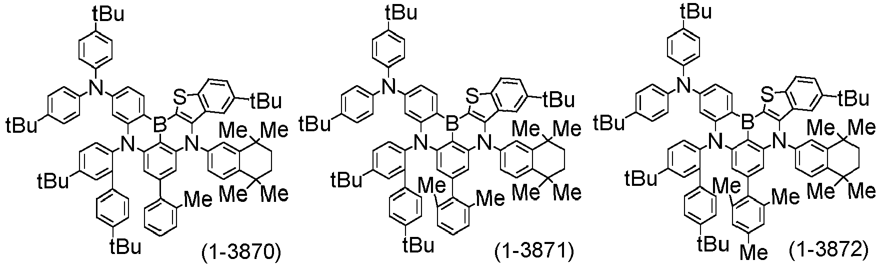

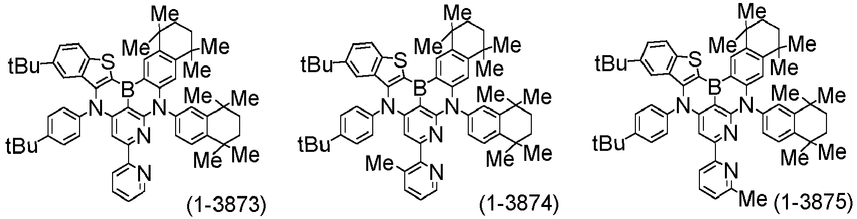

In this paragraph all compounds are inventive compounds.

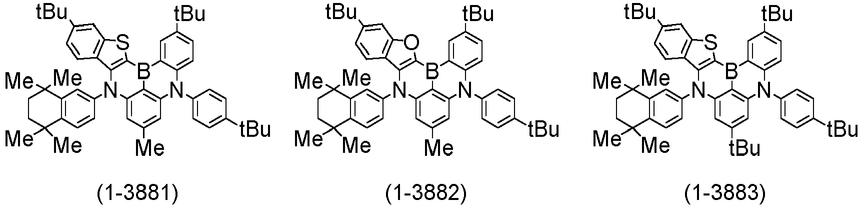

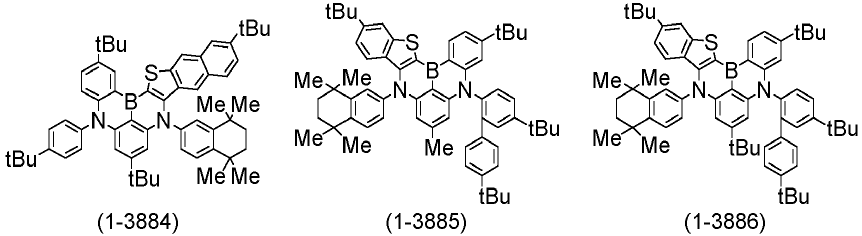

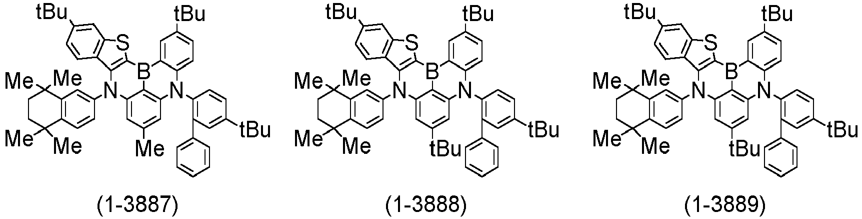

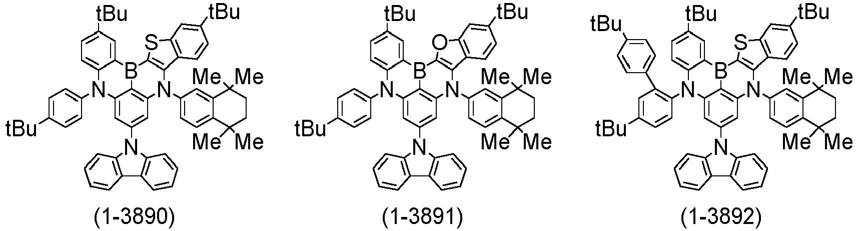

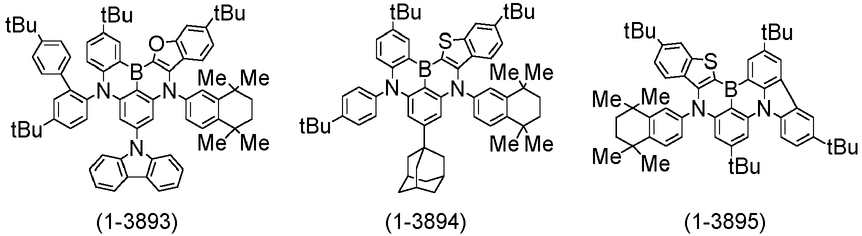

In this paragraph all compounds are inventive compounds.

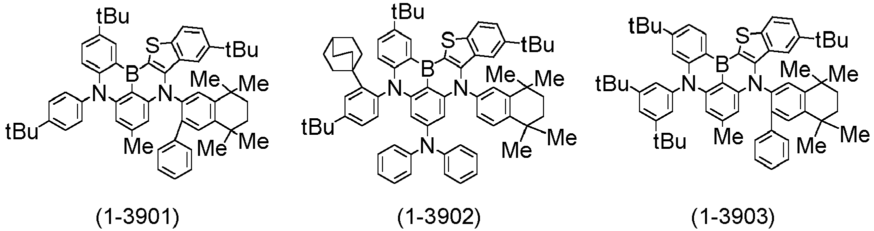

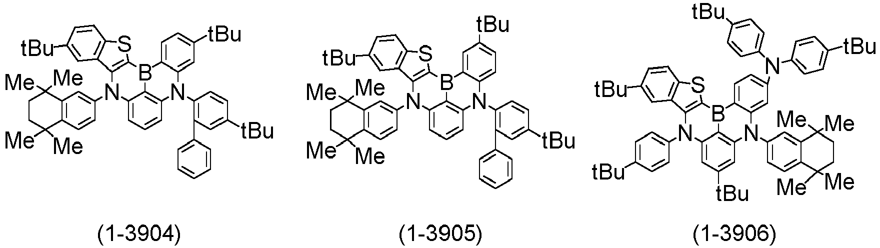

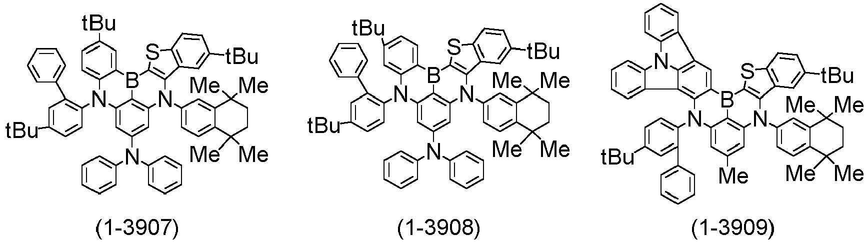

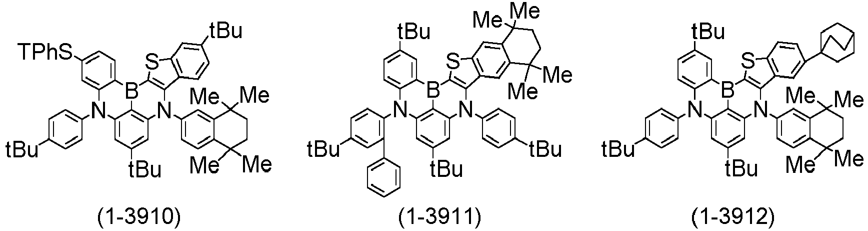

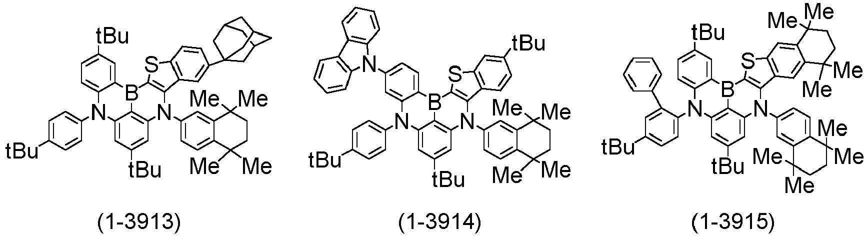

In this paragraph all compounds are inventive compounds.

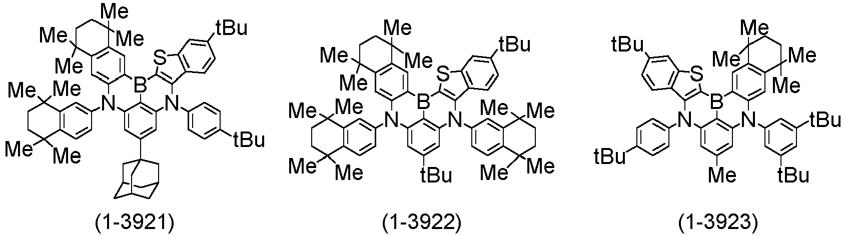

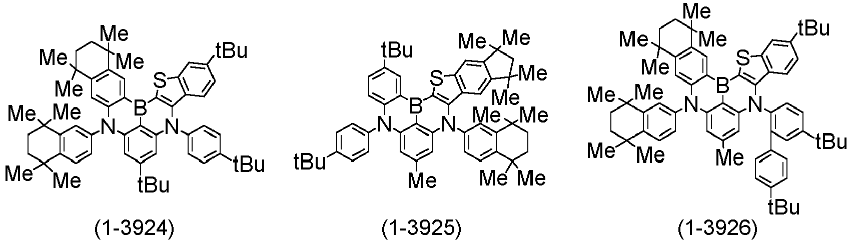

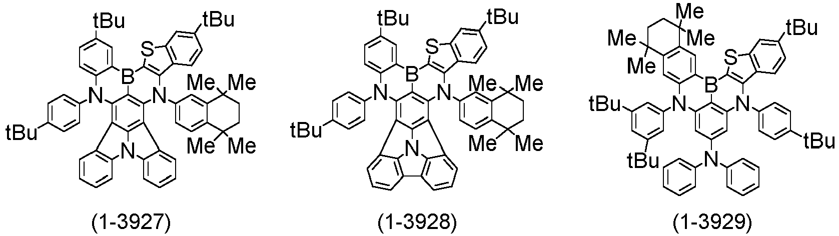

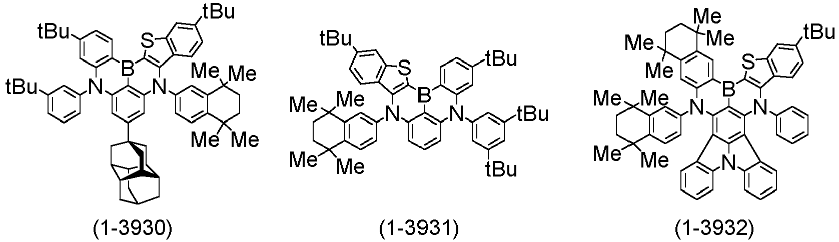

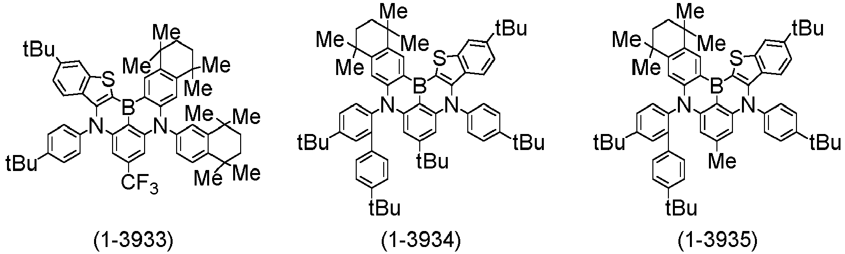

In this paragraph all compounds are inventive compounds.

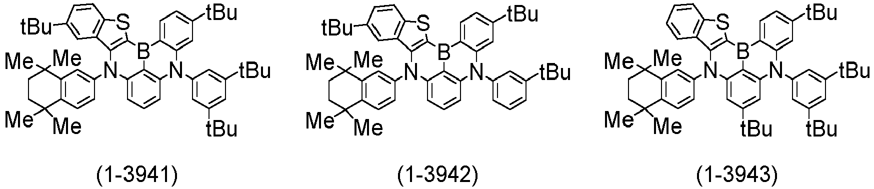

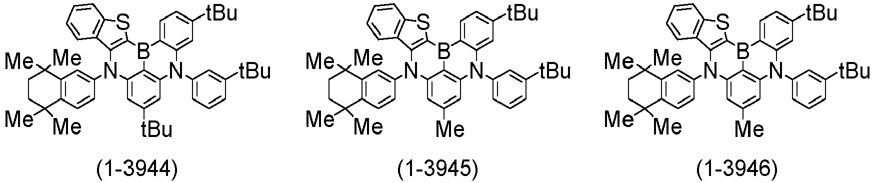

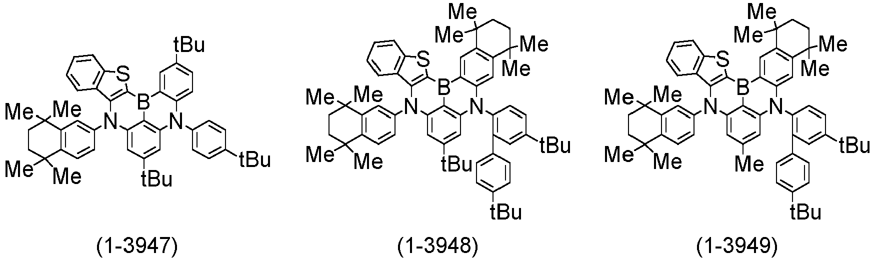

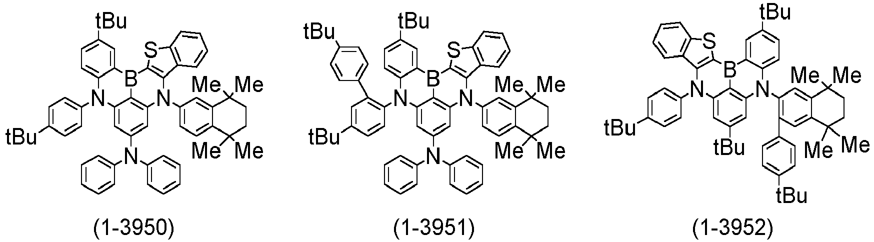

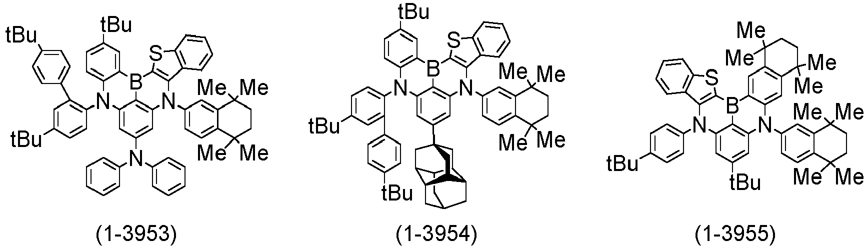

In this paragraph all compounds are inventive compounds.

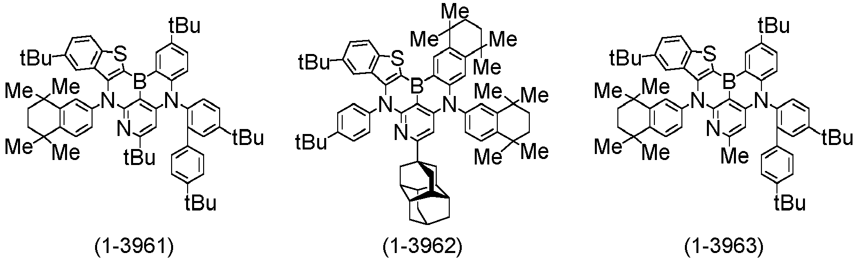

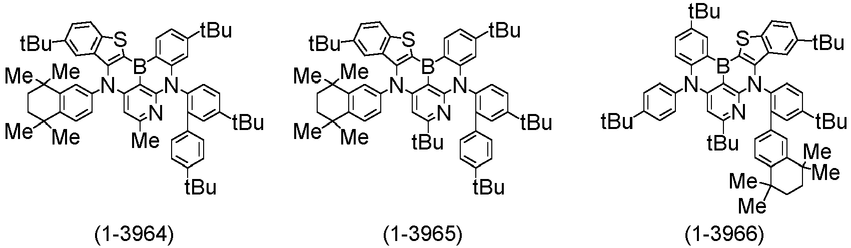

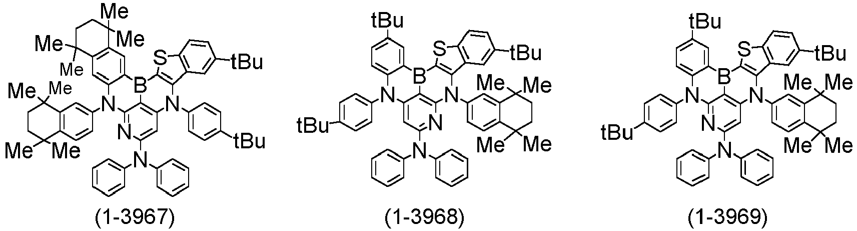

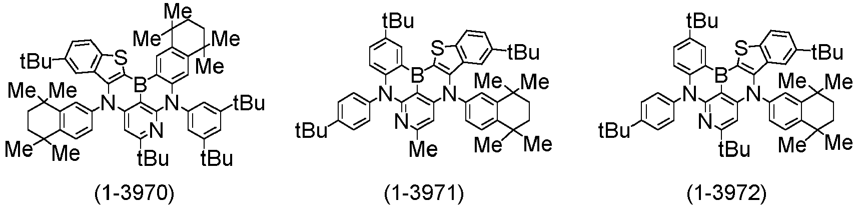

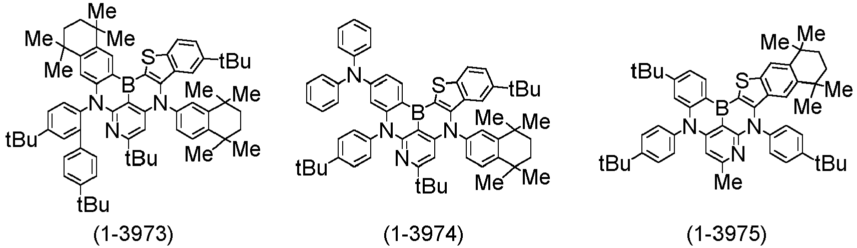

In this paragraph all compounds are inventive compounds.

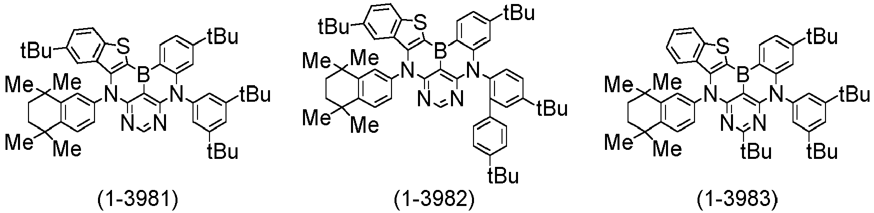

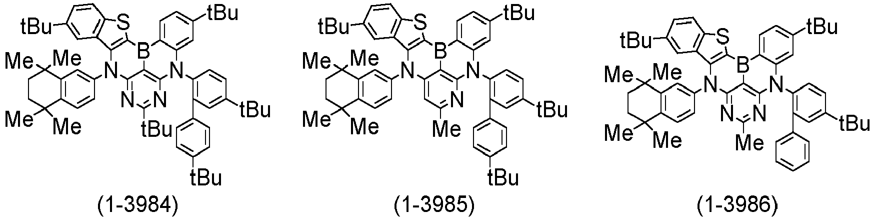

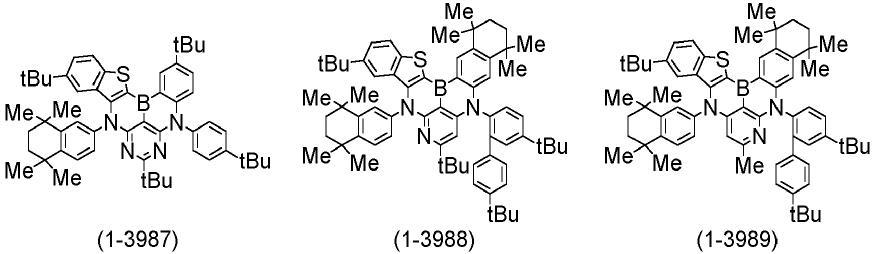

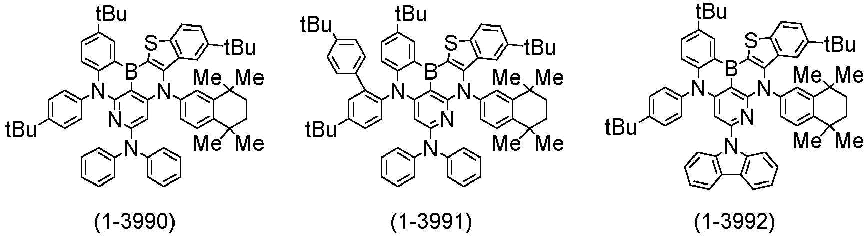

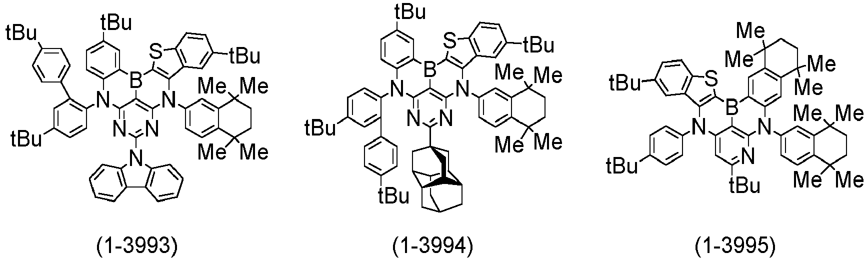

In this paragraph all compounds are inventive compounds.

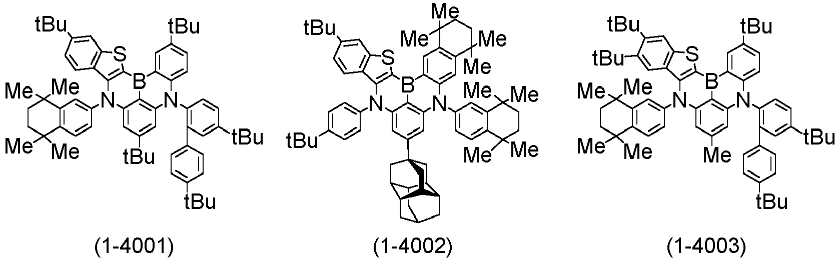

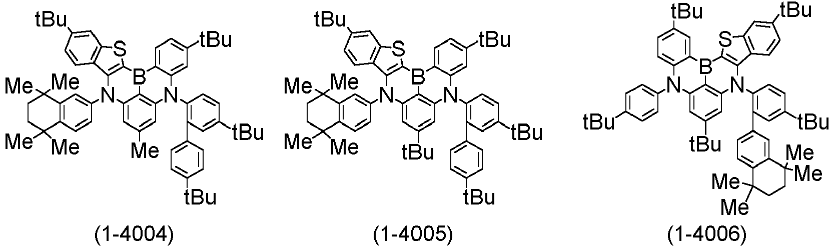

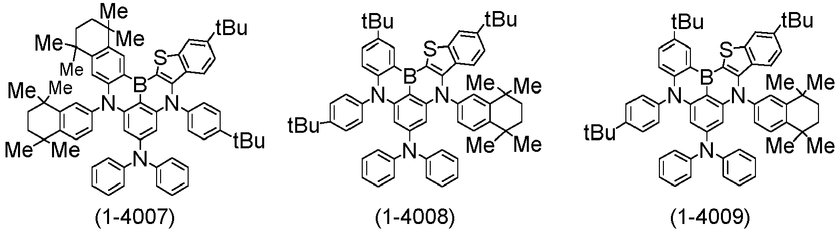

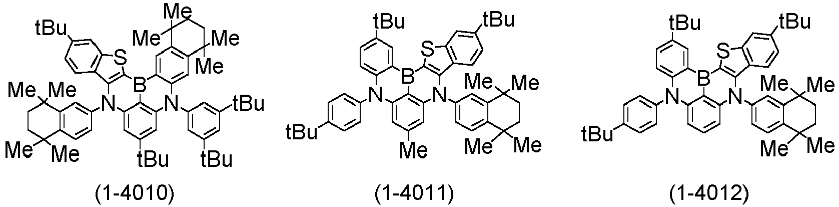

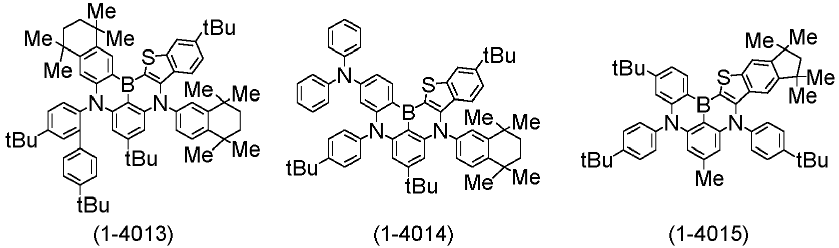

In this paragraph all compounds are inventive compounds.

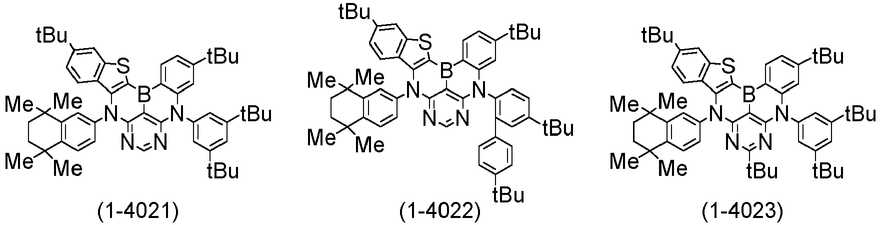

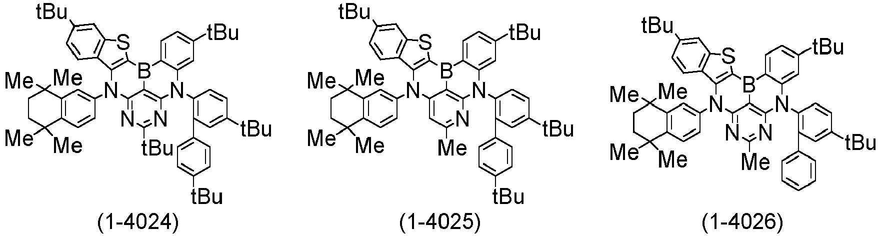

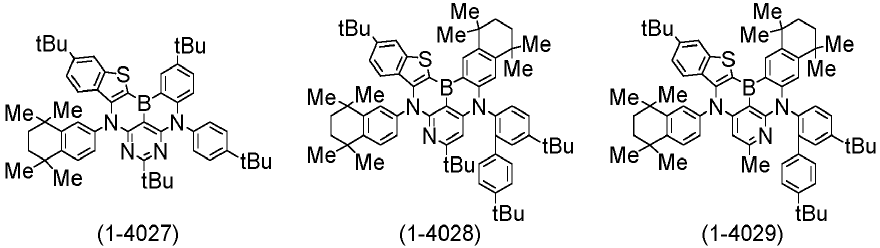

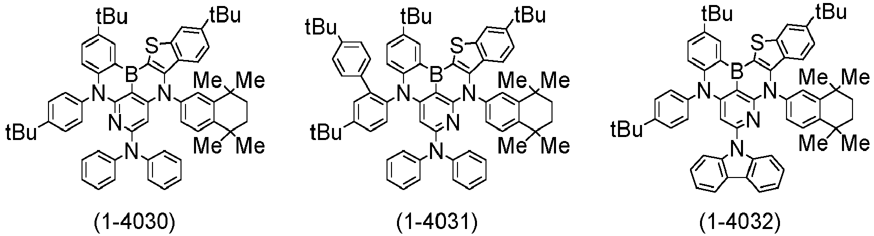

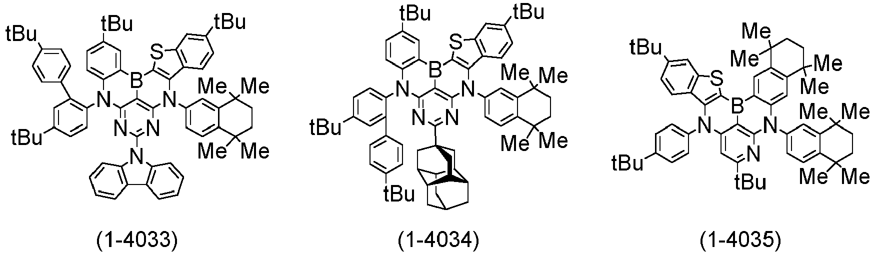

In this paragraph all compounds are inventive compounds.

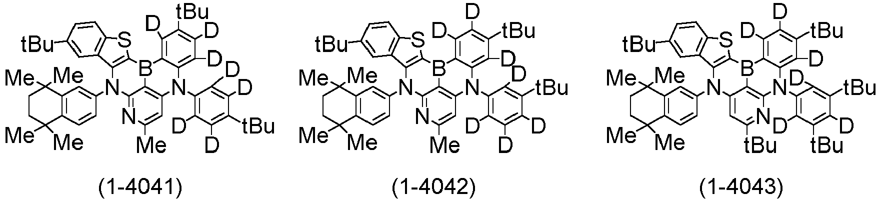

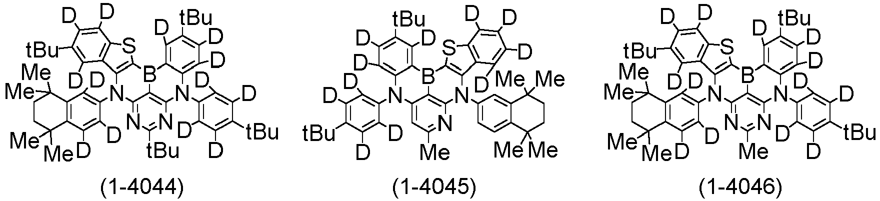

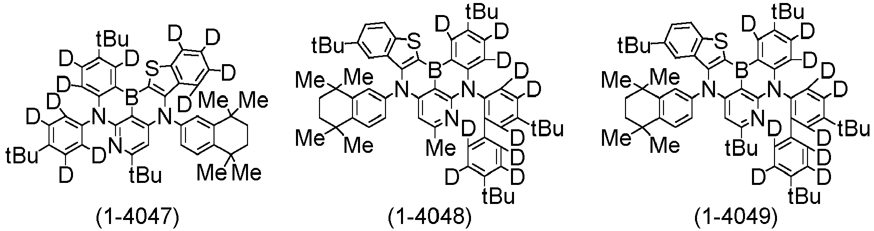

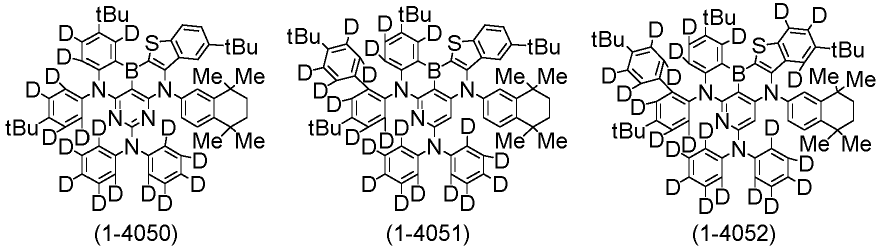

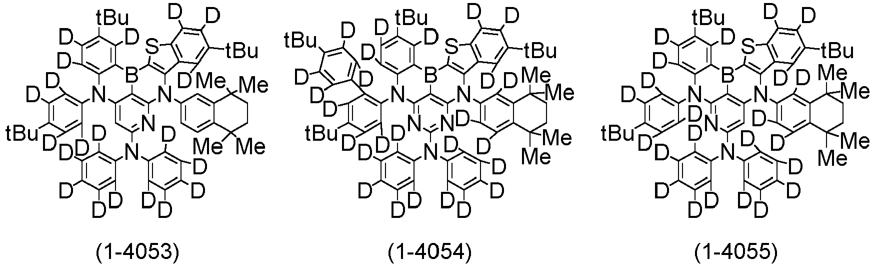

In this paragraph all compounds are inventive compounds.

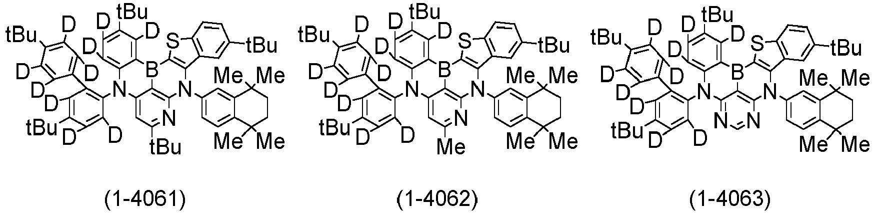

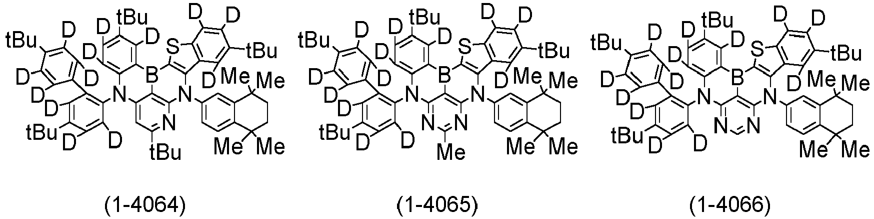

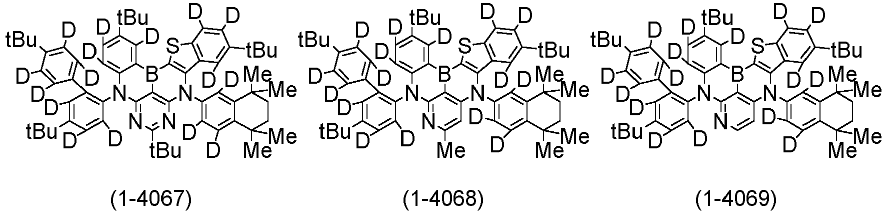

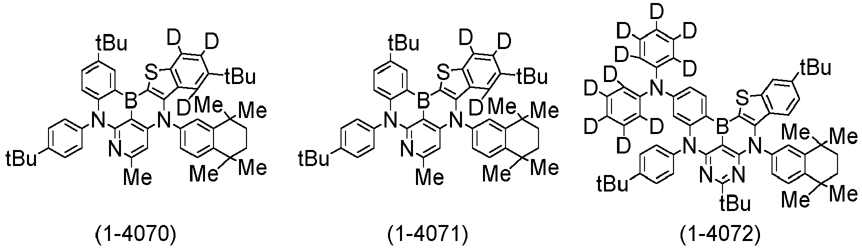

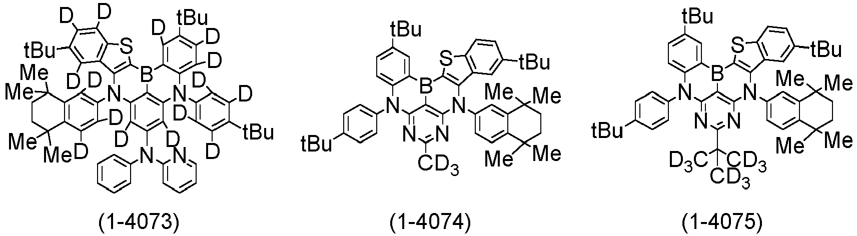

In this paragraph all compounds are inventive compounds.

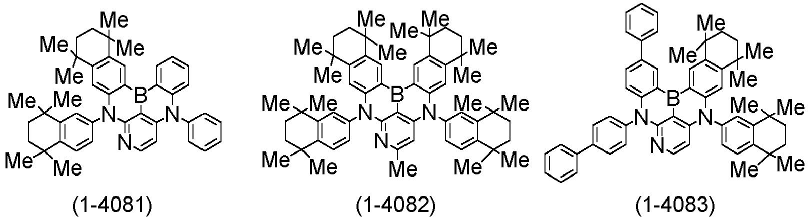

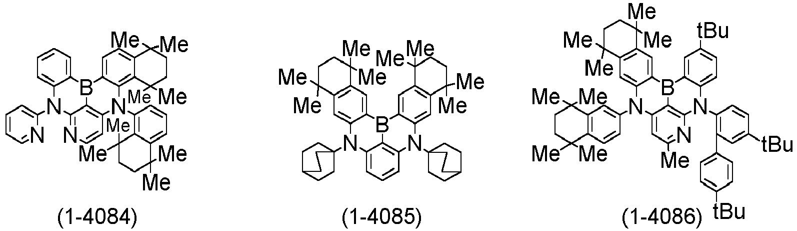

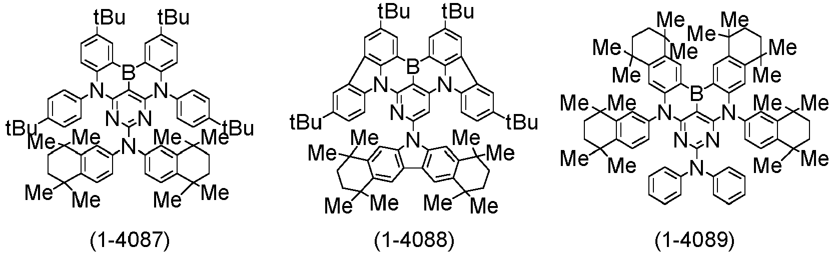

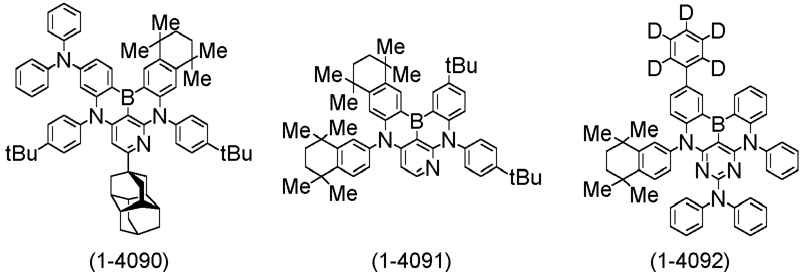

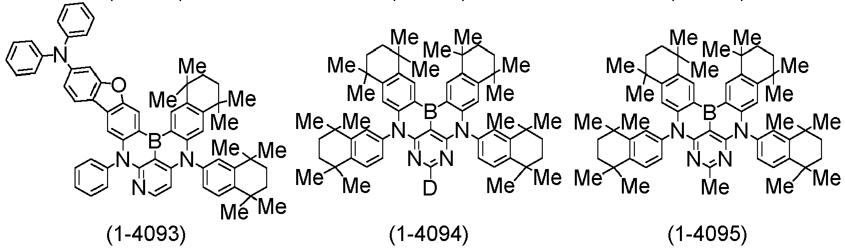

In this paragraph all compounds except compound (1-4113) are inventive compounds.

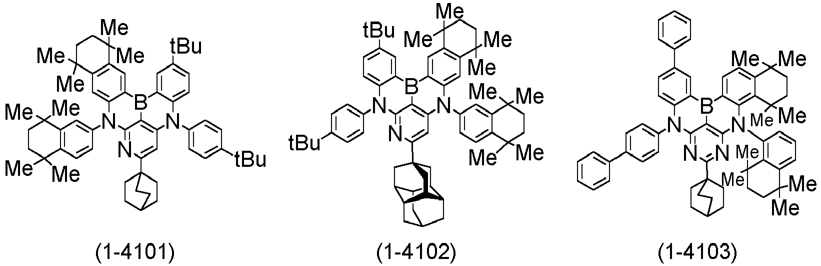

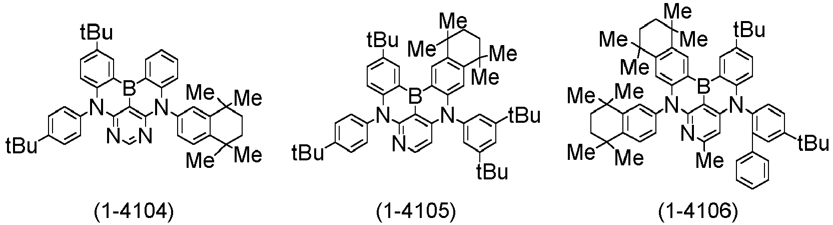

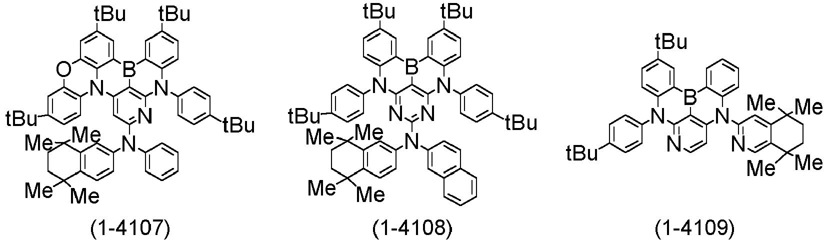

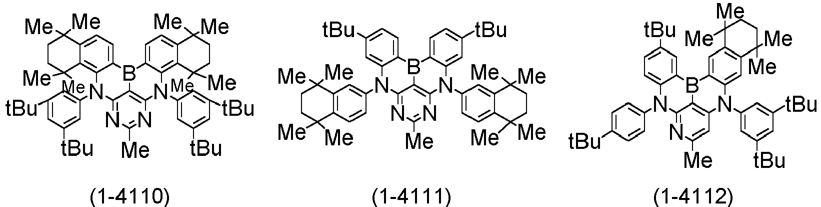

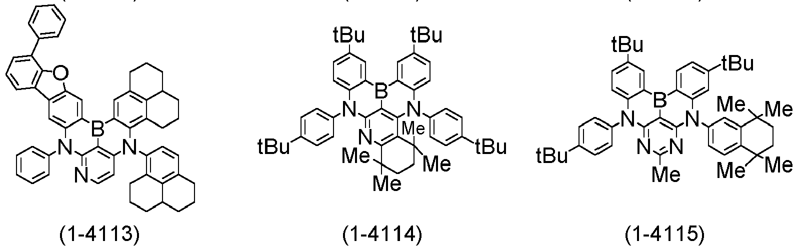

In this paragraph all compounds are inventive compounds.

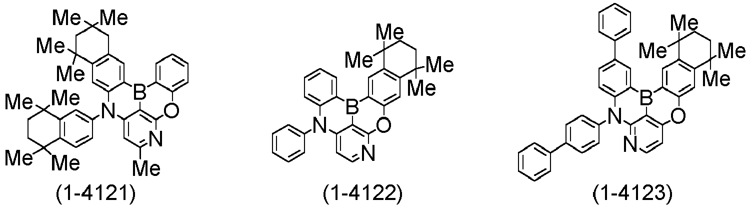

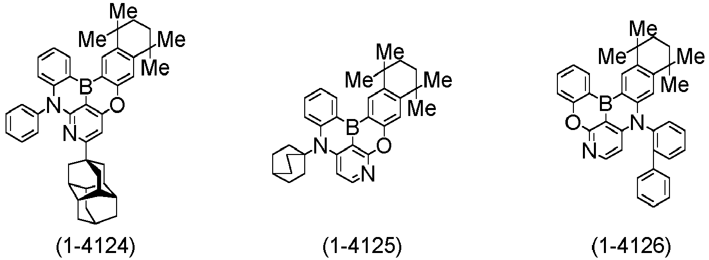

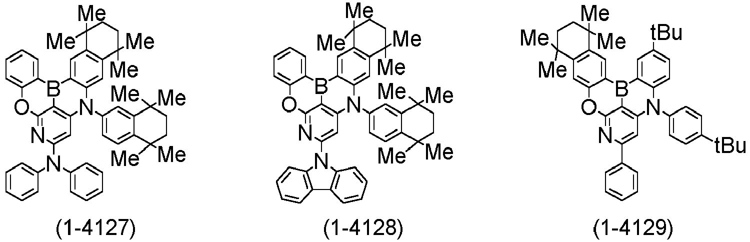

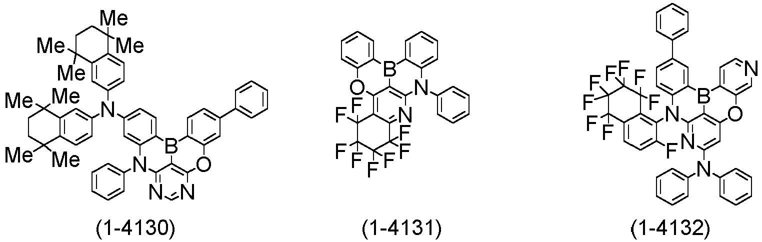

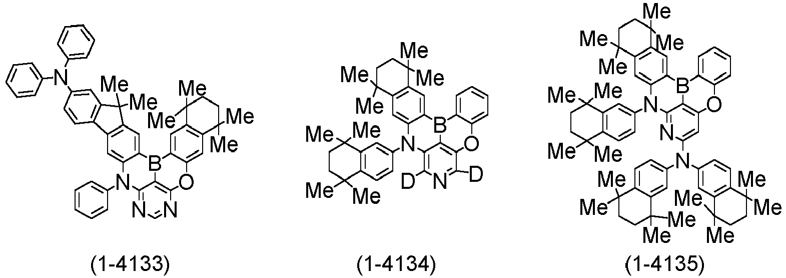

In this paragraph all compounds except compound (1-4154) are inventive compounds.

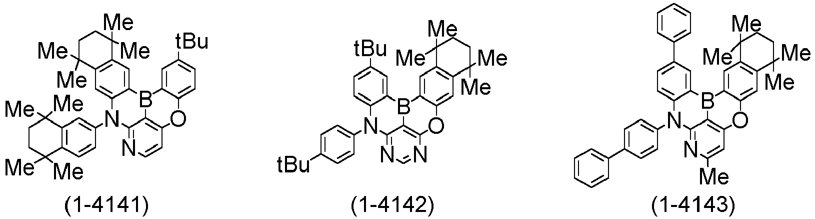

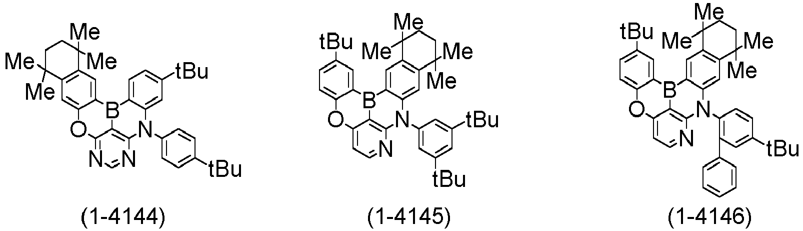

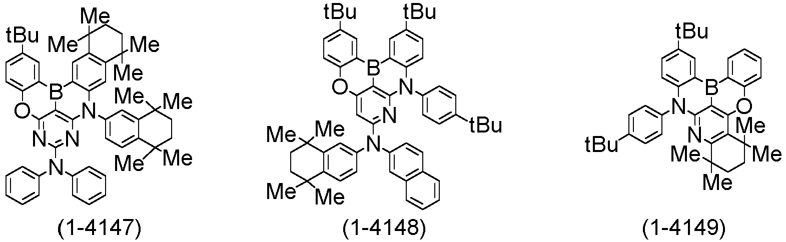

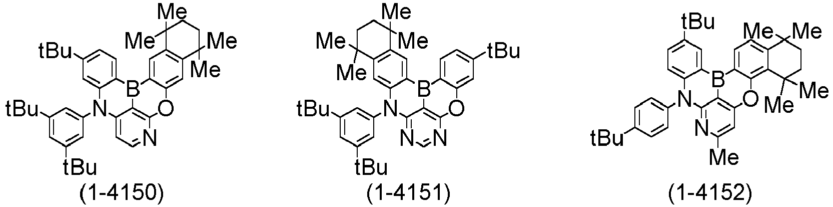

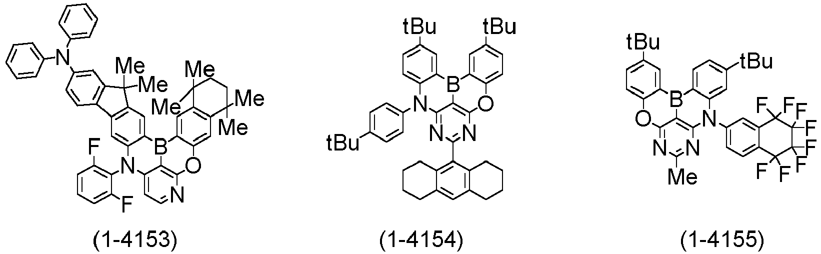

In this paragraph all compounds except compound (1-4166) are inventive compounds.

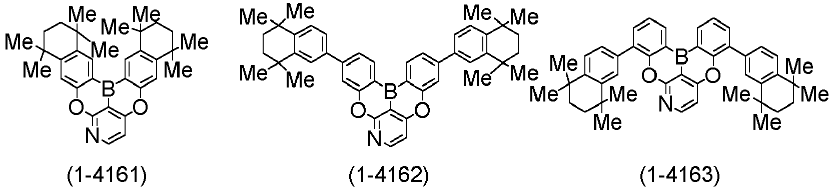

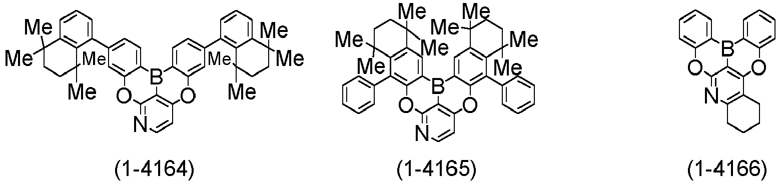

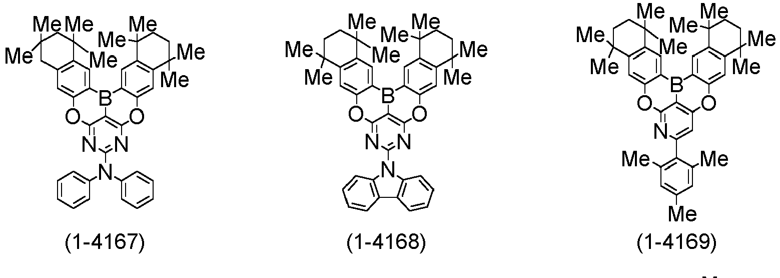

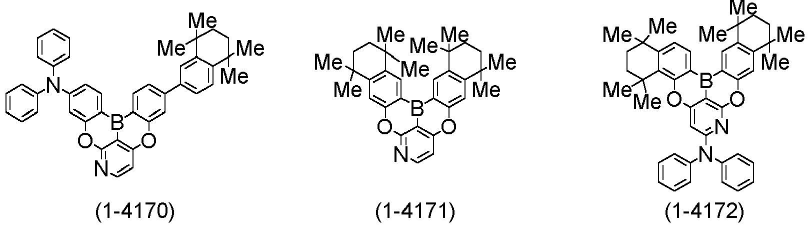

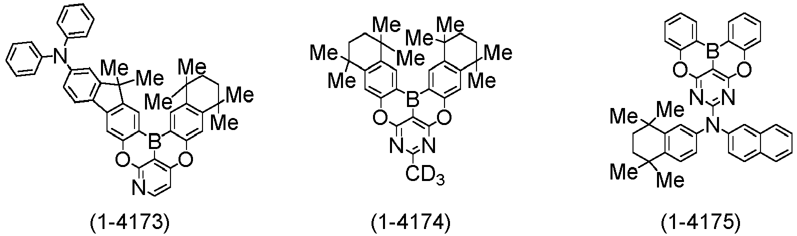

In this paragraph all compounds are inventive compounds.

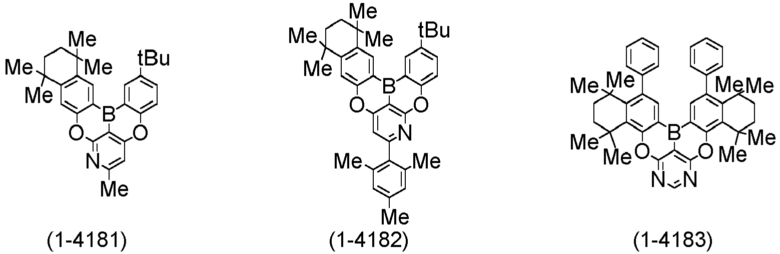

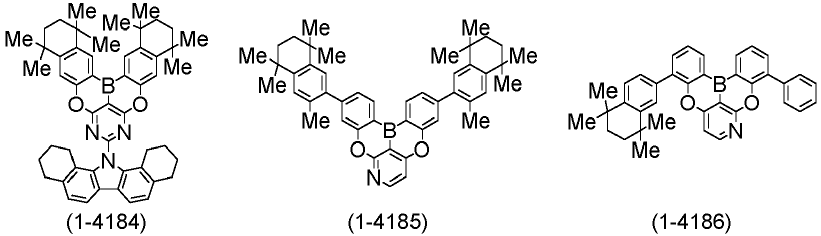

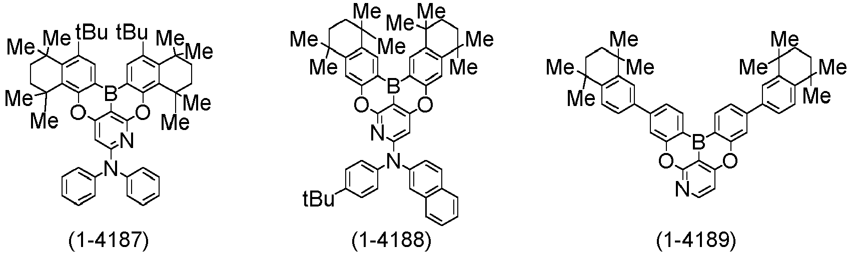

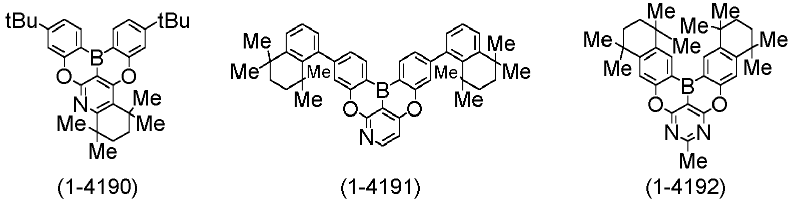

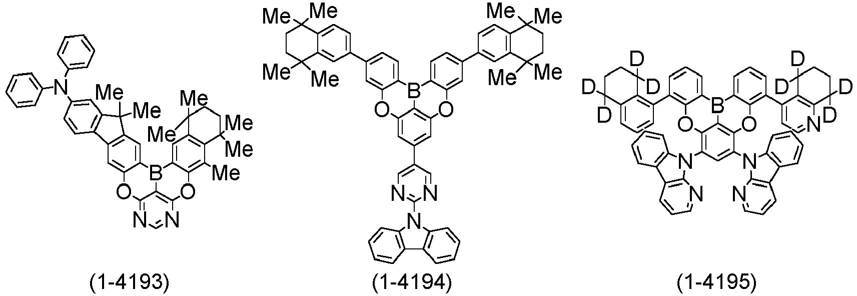

In this paragraph all compounds are inventive compounds.

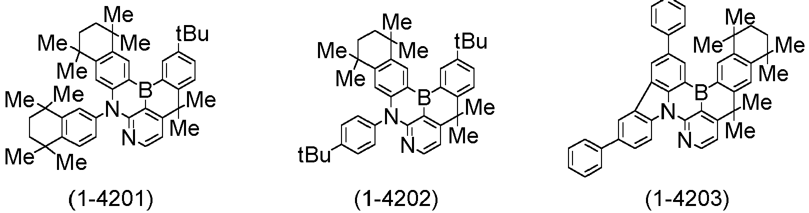

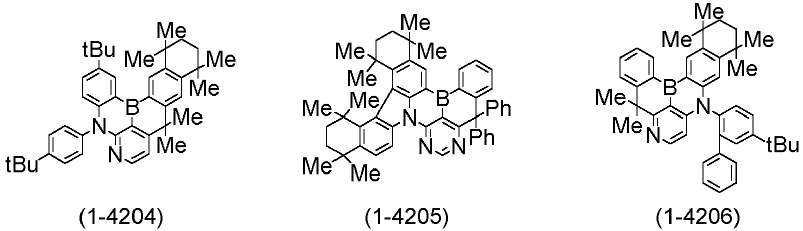

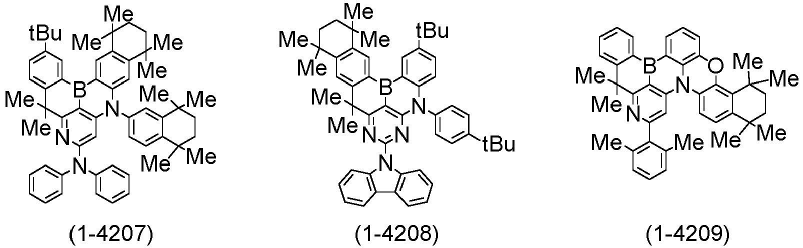

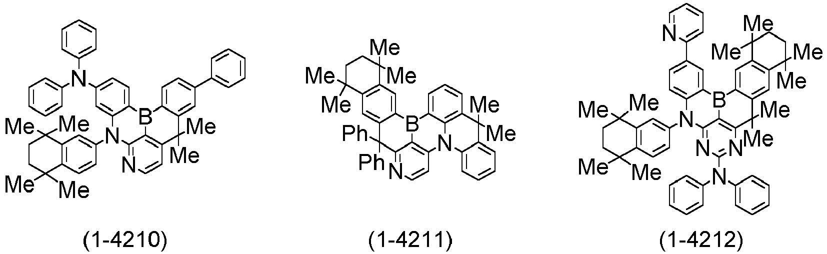

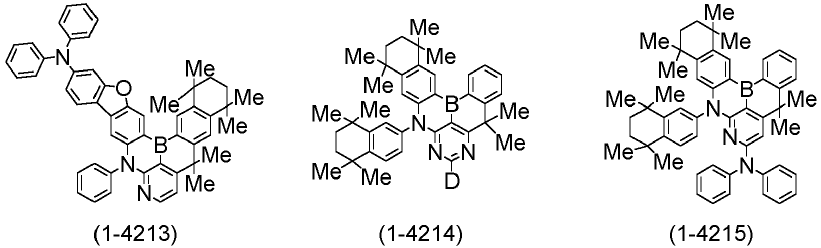

In this paragraph all compounds except compound (1-4234) are inventive compounds.

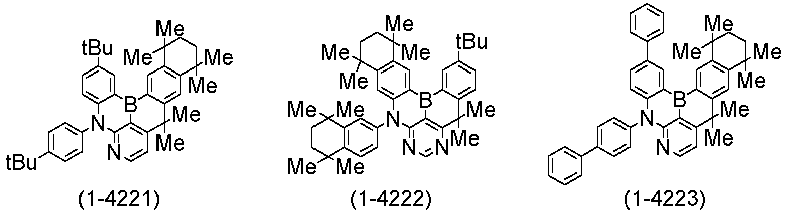

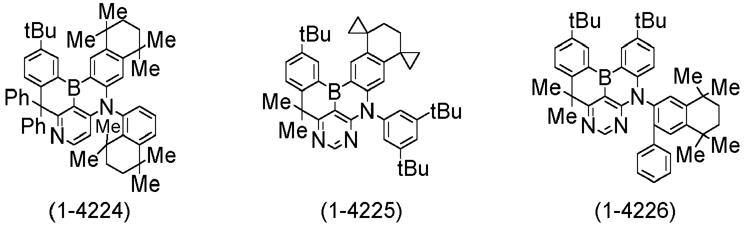

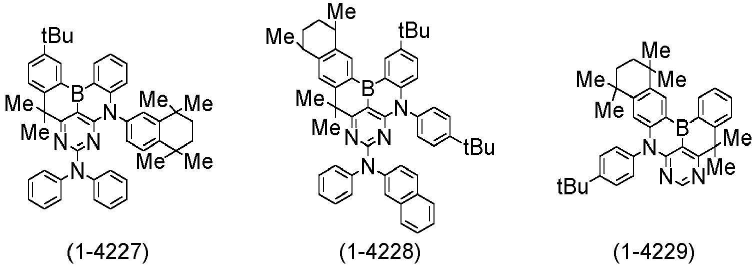

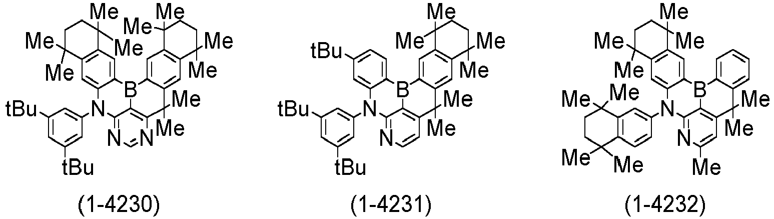

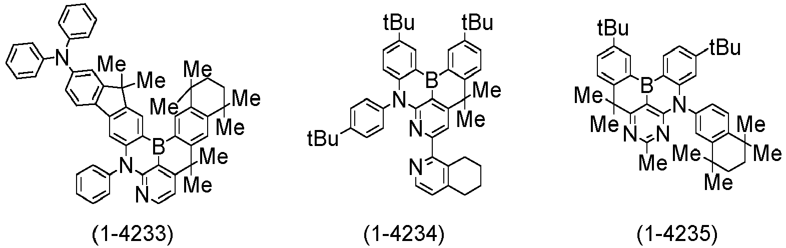

In this paragraph all compounds except compounds (1-4245), (1-4248) are inventive compounds.

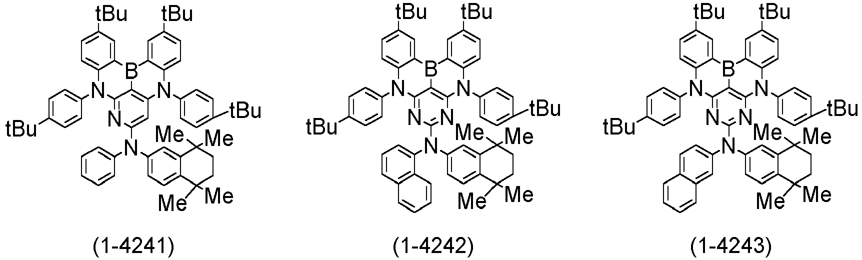

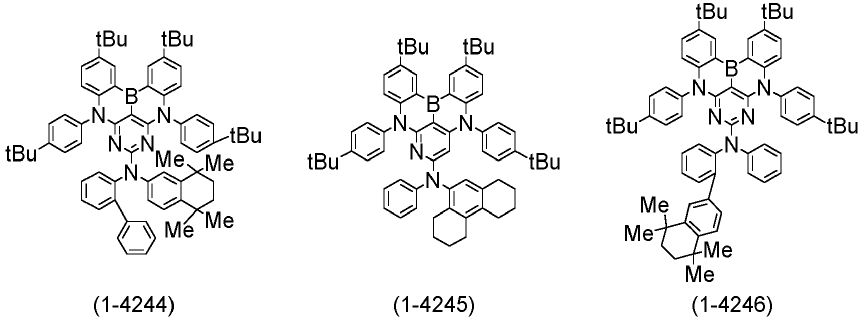

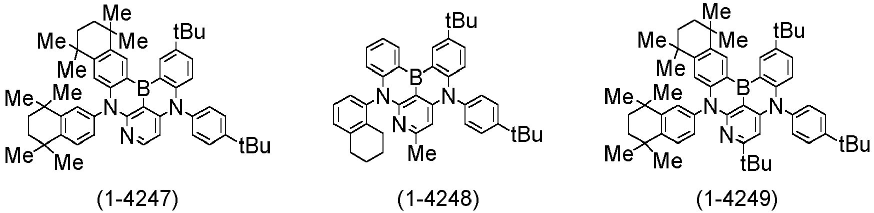

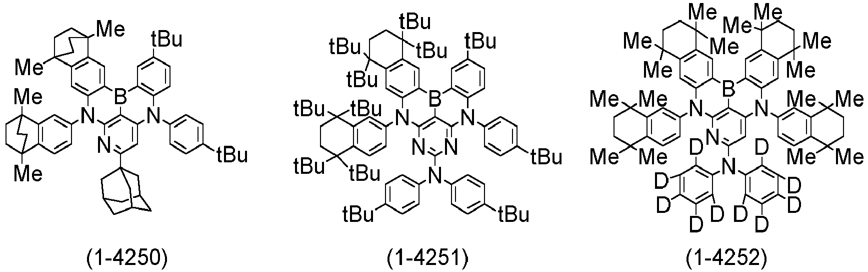

In this paragraph all compounds except compounds (1-4262) to (1-4264) are inventive compounds.

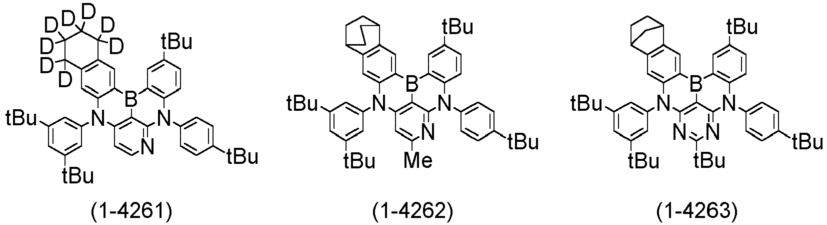

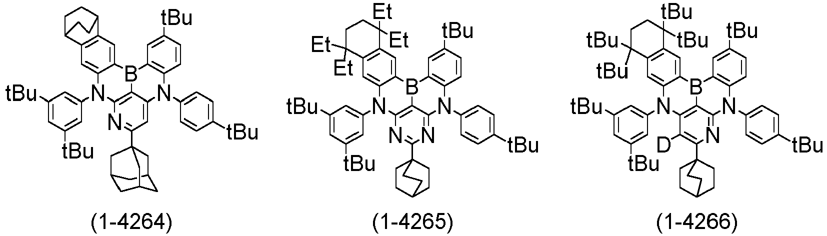

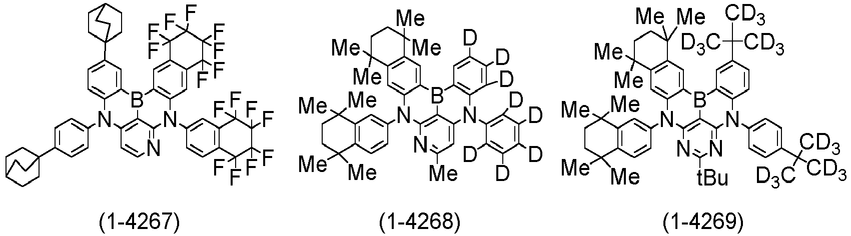

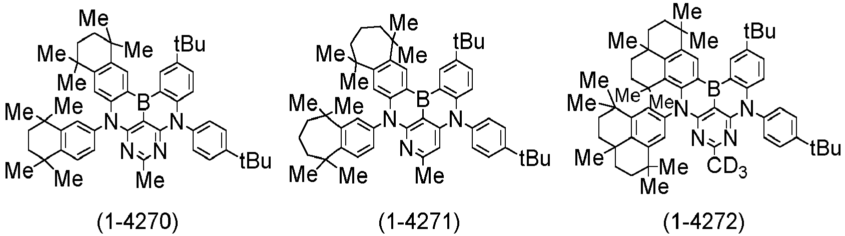

In this paragraph all compounds are inventive compounds.

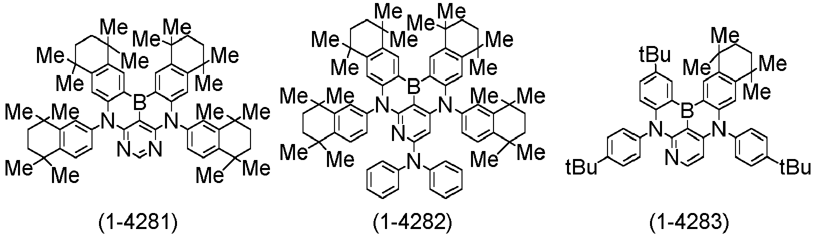

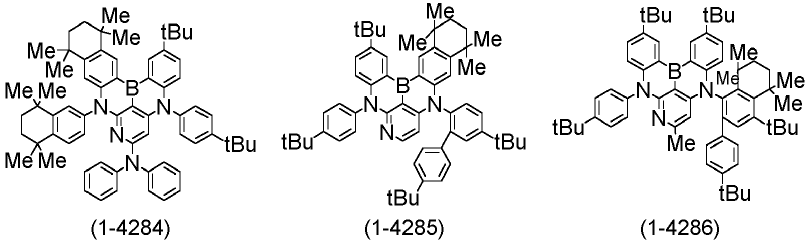

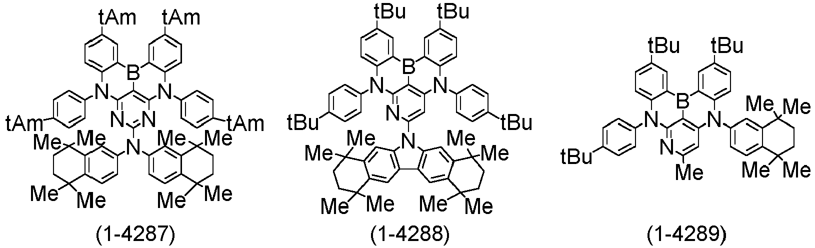

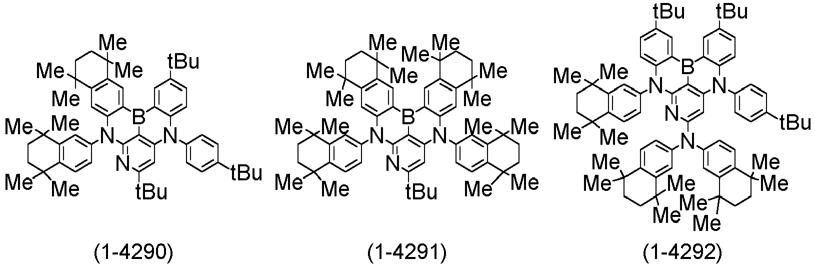

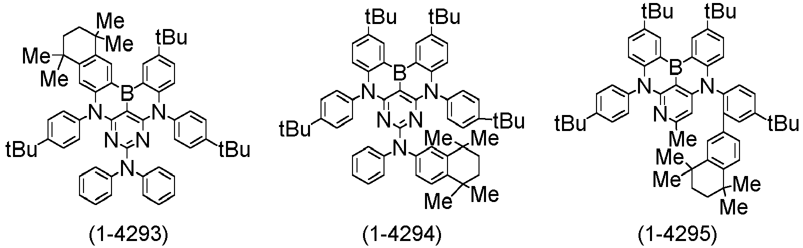

In this paragraph all compounds except compound (1-4313) are inventive compounds.

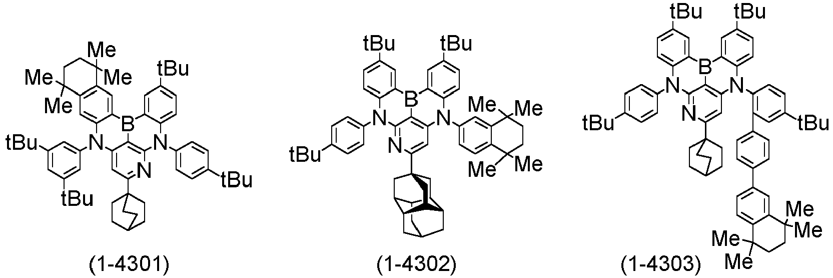

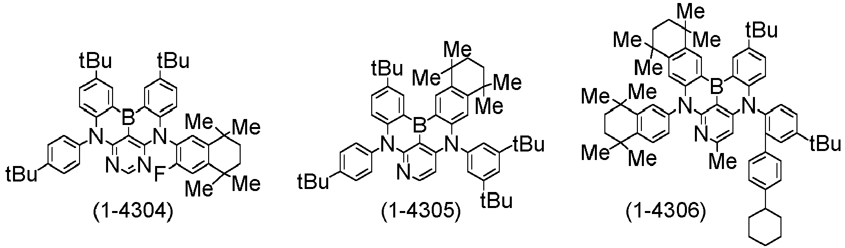

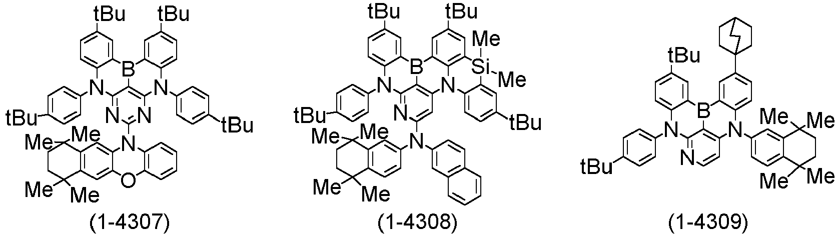

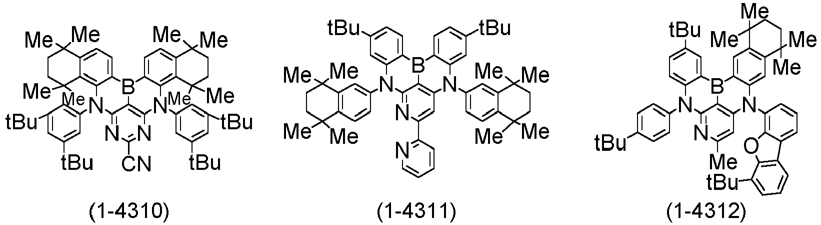

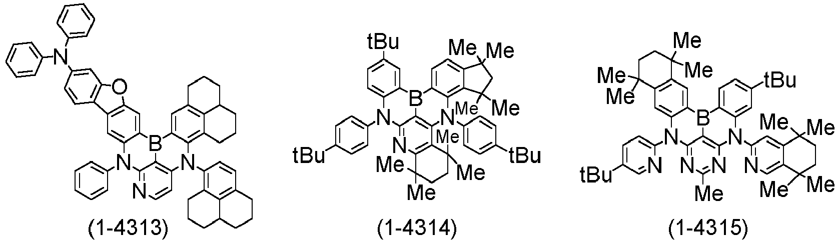

In this paragraph all compounds are inventive compounds.

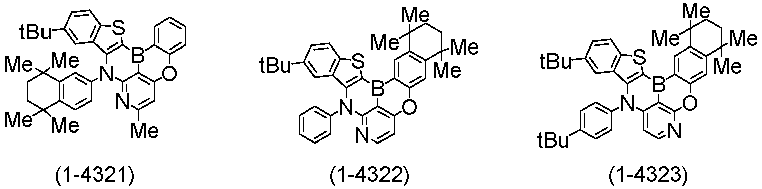

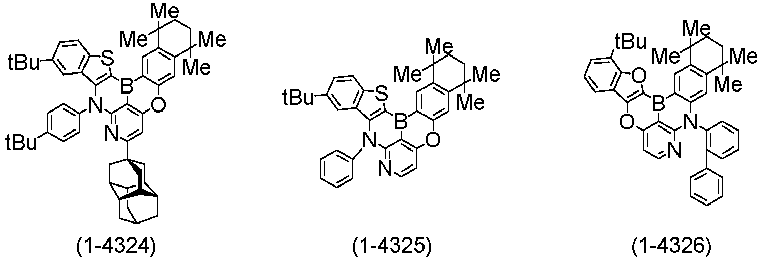

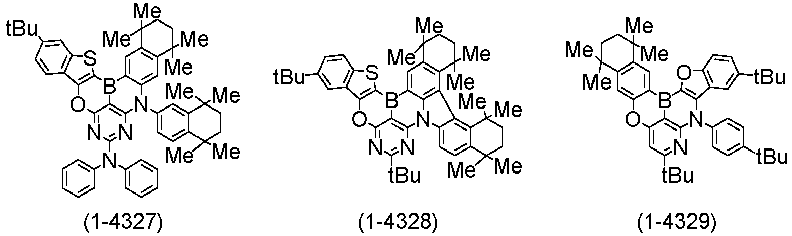

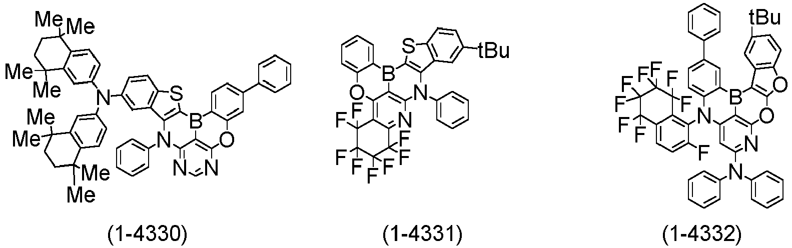

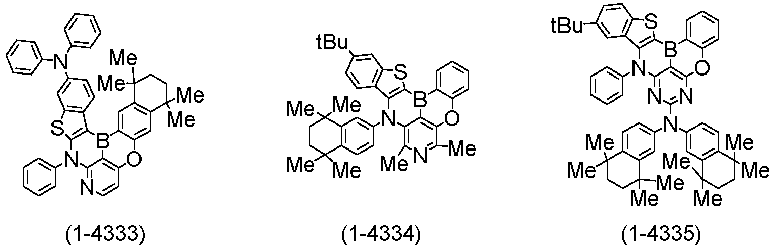

In this paragraph all compounds except compound (1-4354) are inventive compounds.

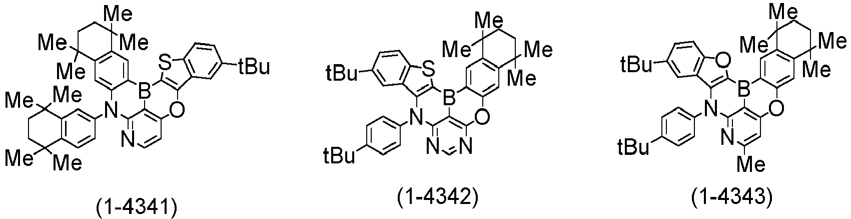

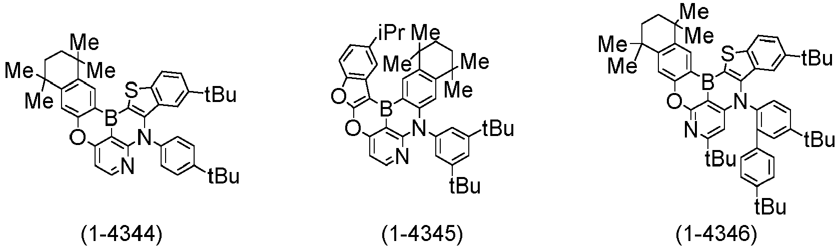

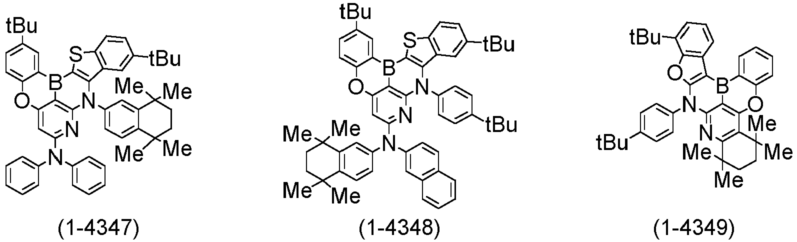

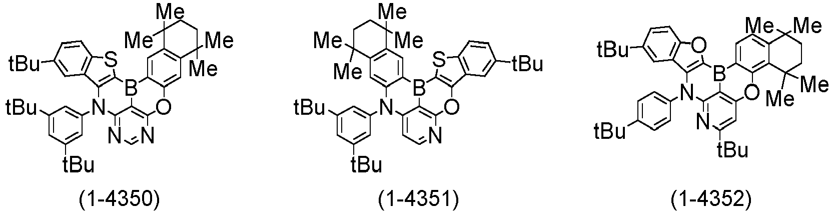

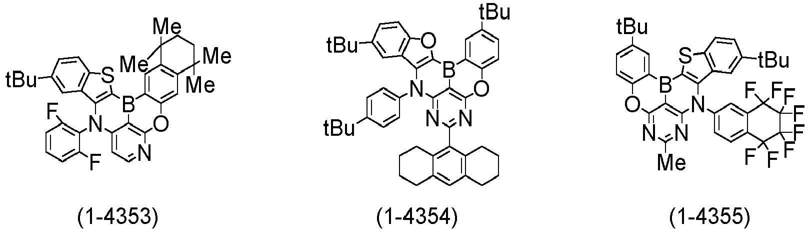

In this paragraph all compounds except compound (1-4366) are inventive compounds.

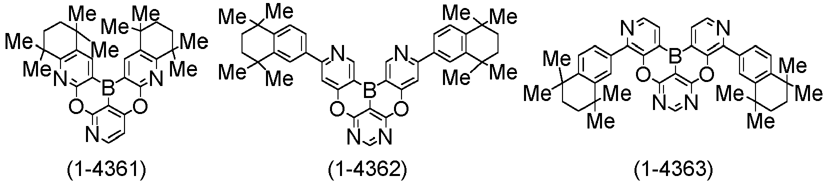

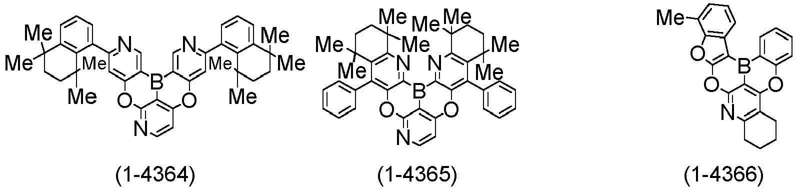

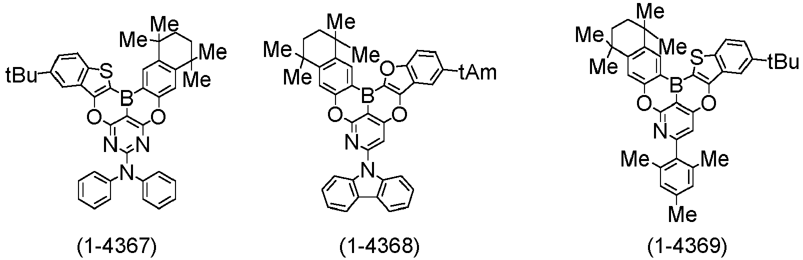

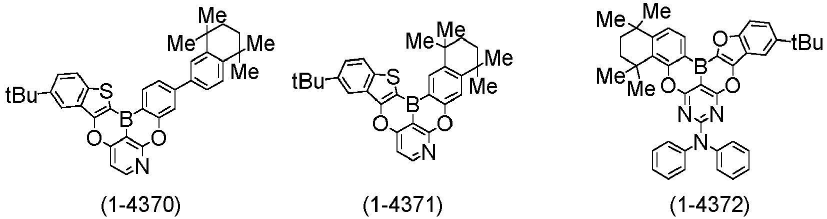

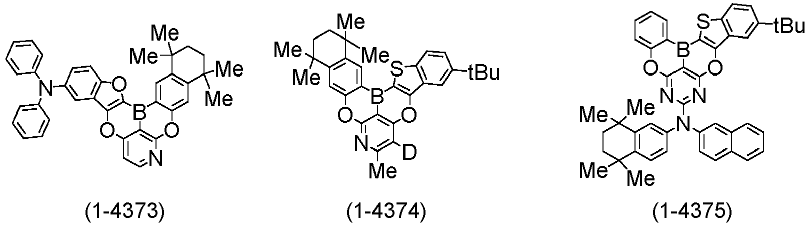

In this paragraph all compounds are inventive compounds.

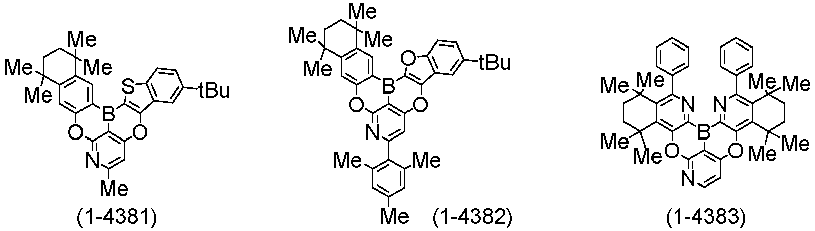

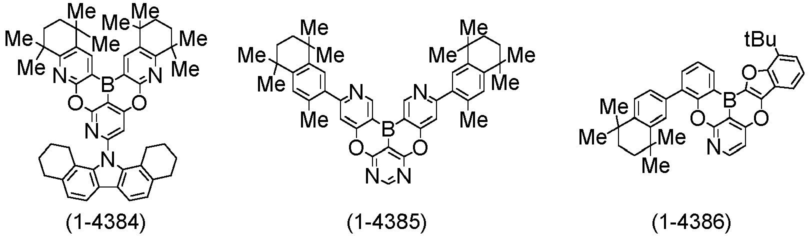

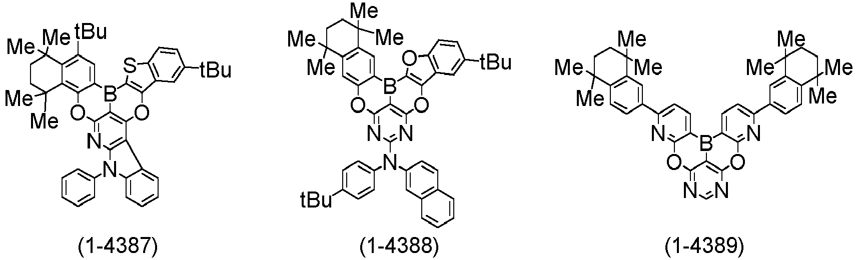

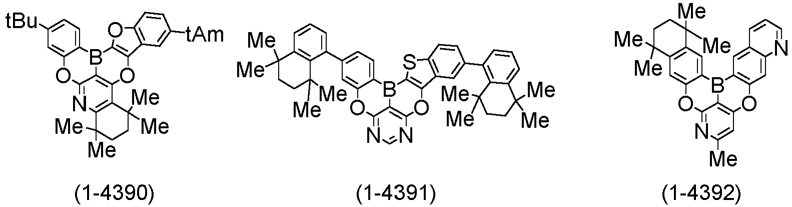

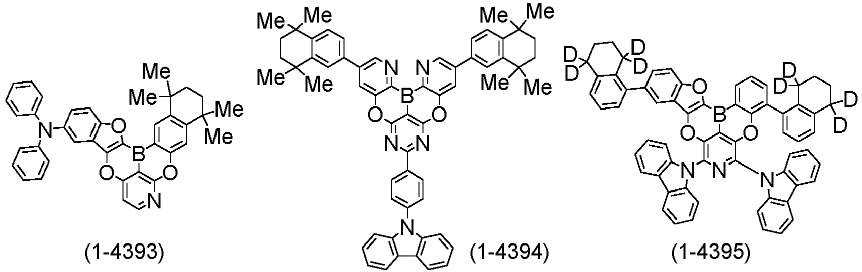

In this paragraph all compounds are inventive compounds.

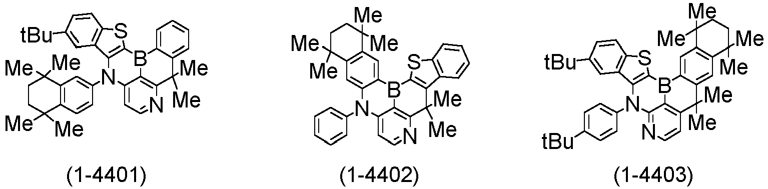

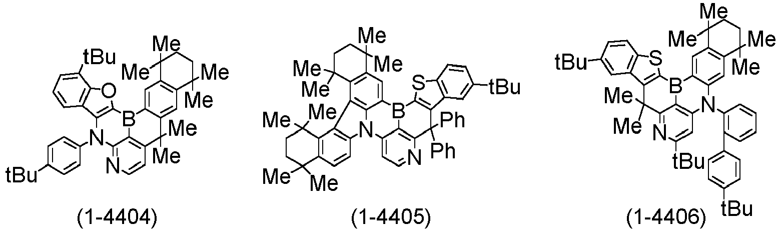

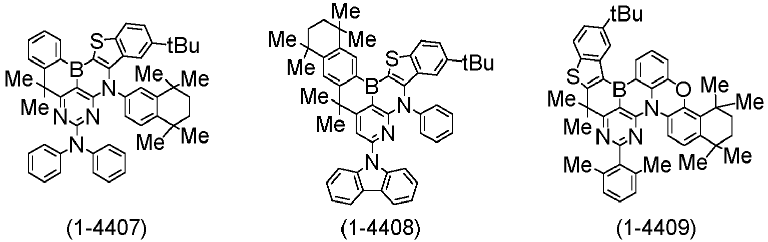

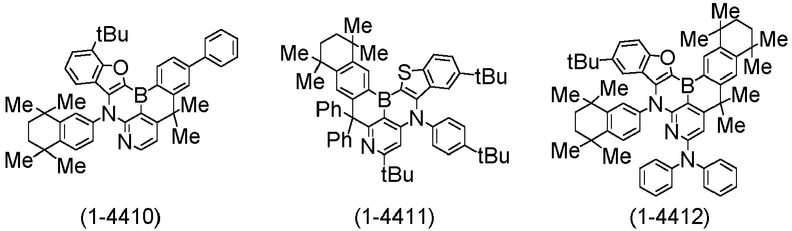

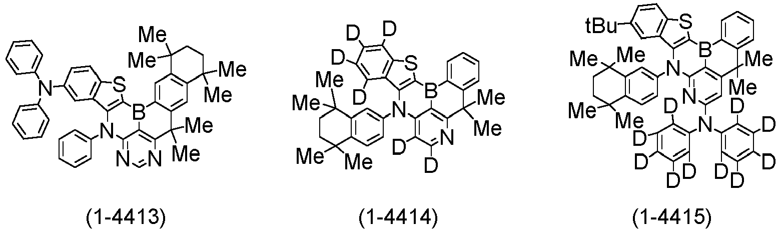

In this paragraph all compounds except compounds (1-4428), (1-4434) are inventive compounds.

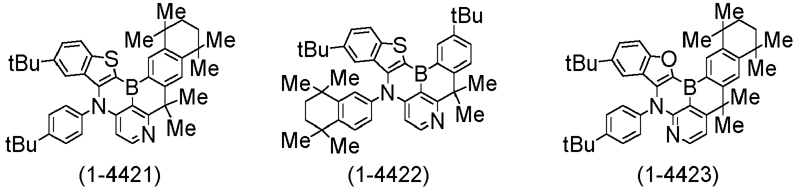

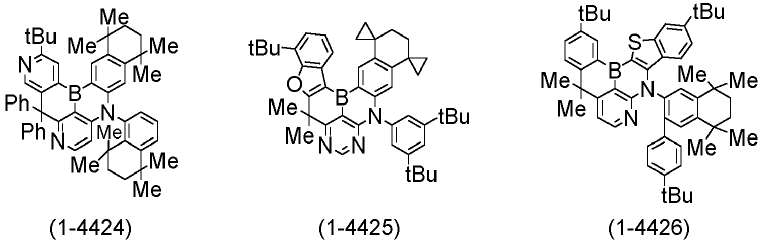

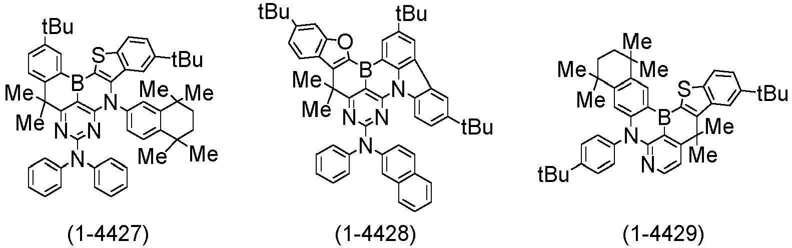

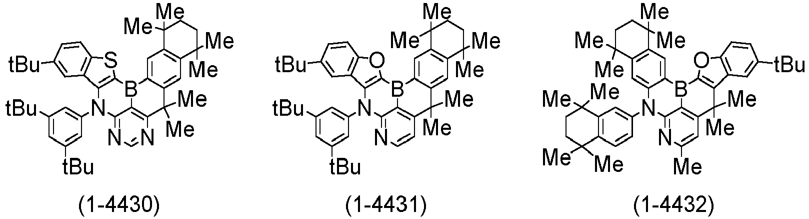

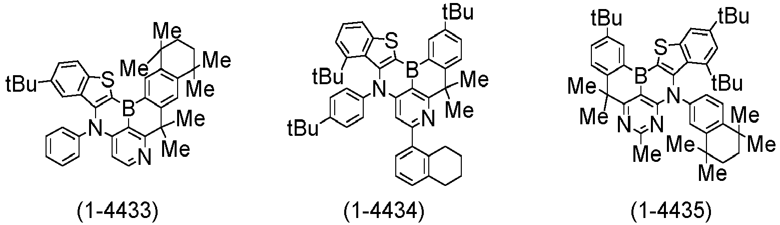

In this paragraph all compounds are inventive compounds.

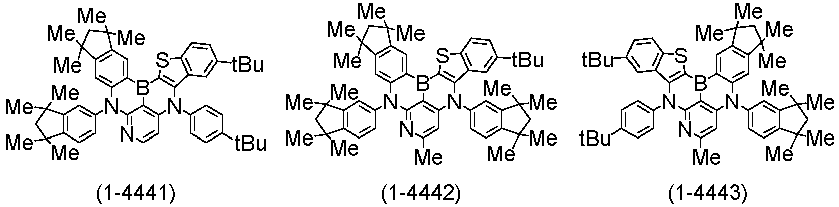

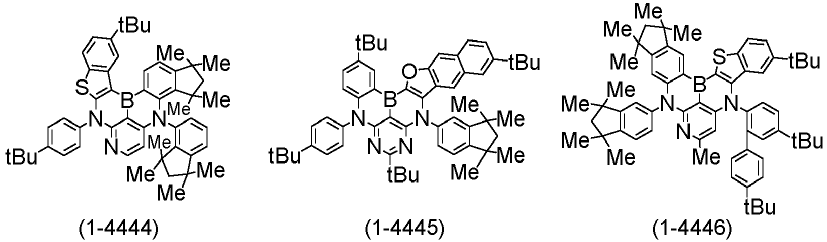

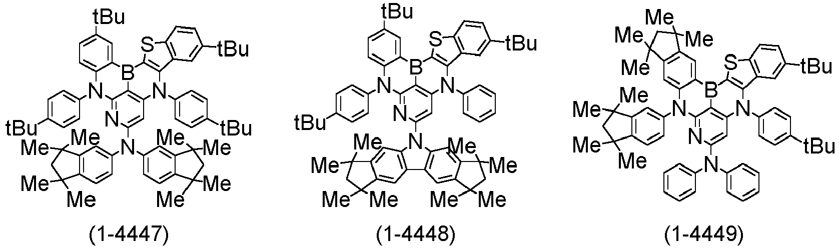

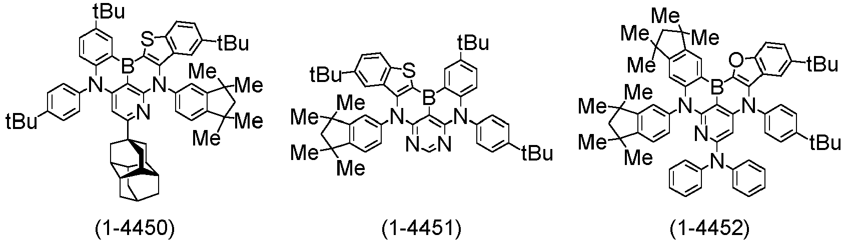

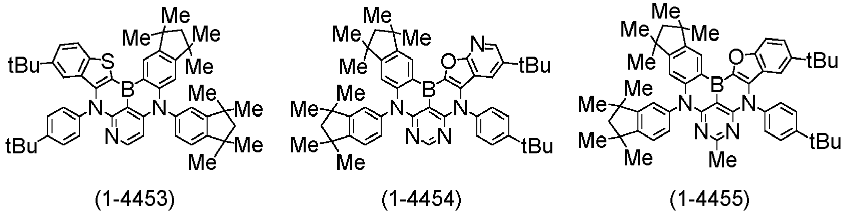

In this paragraph all compounds are inventive compounds.

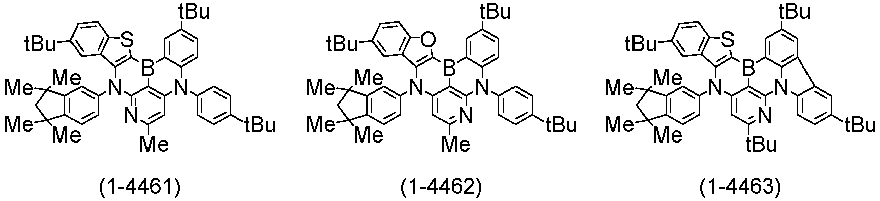

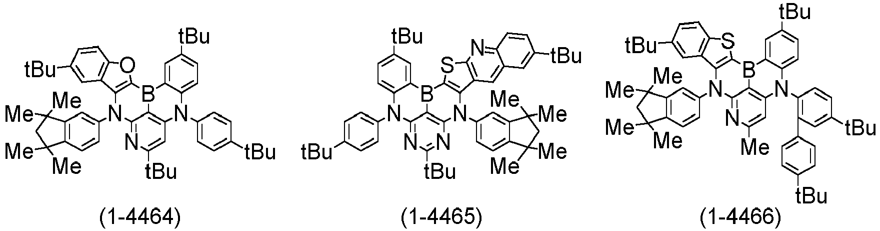

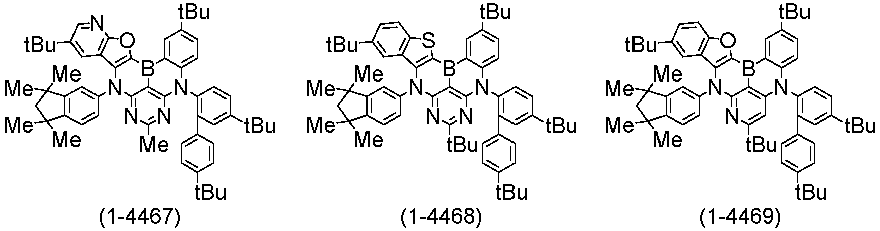

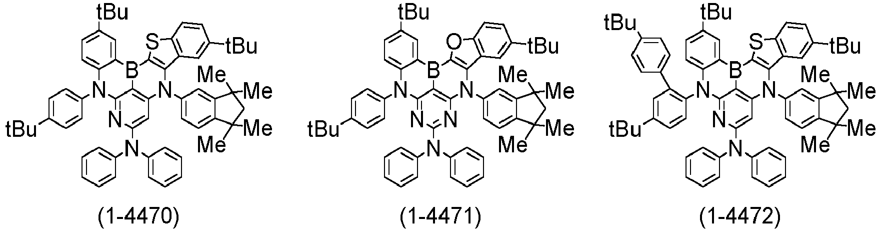

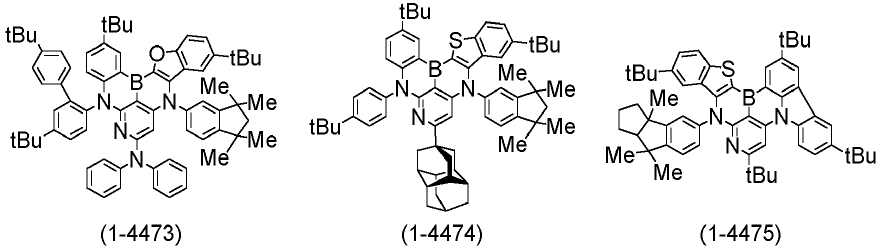

In this paragraph all compounds are inventive compounds.

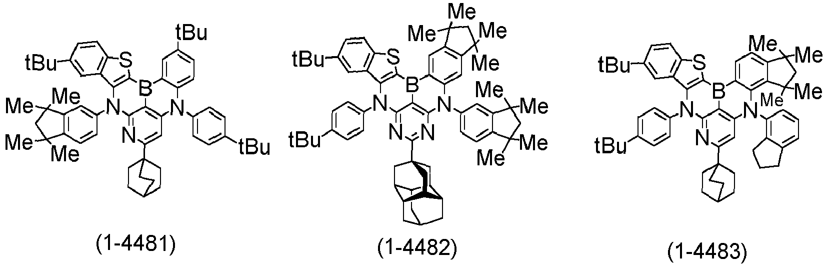

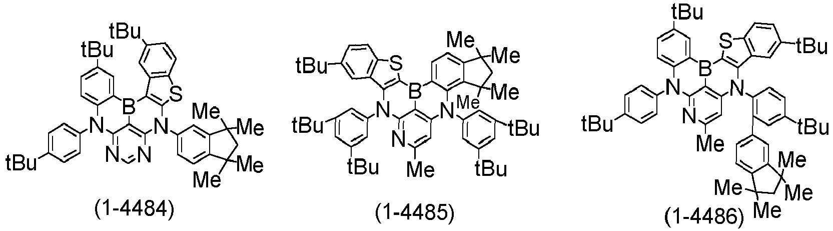

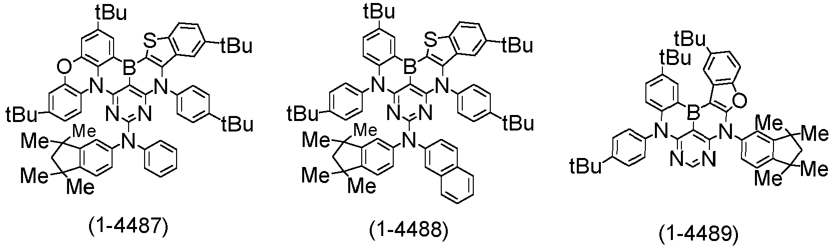

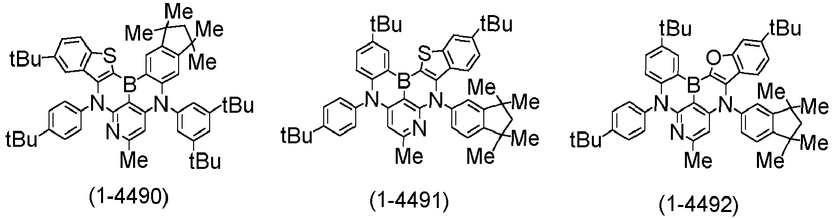

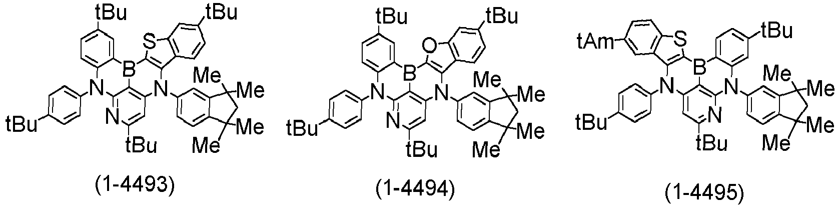

In this paragraph all compounds are inventive compounds.

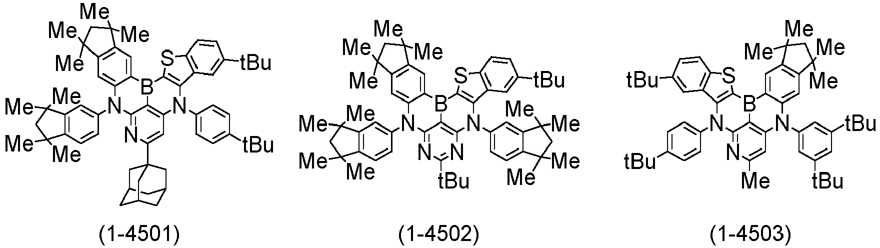

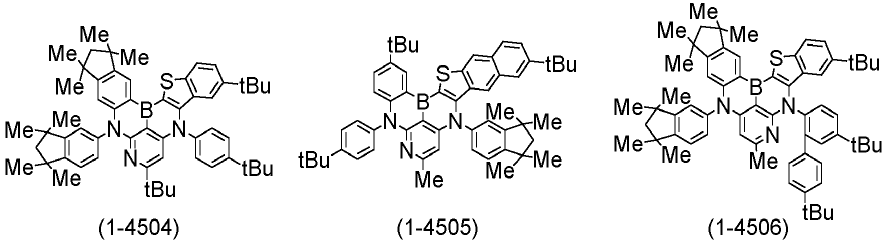

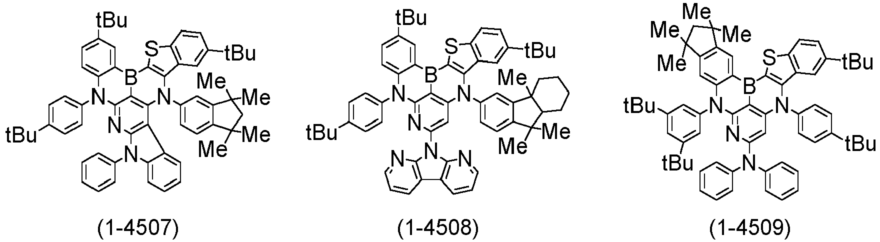

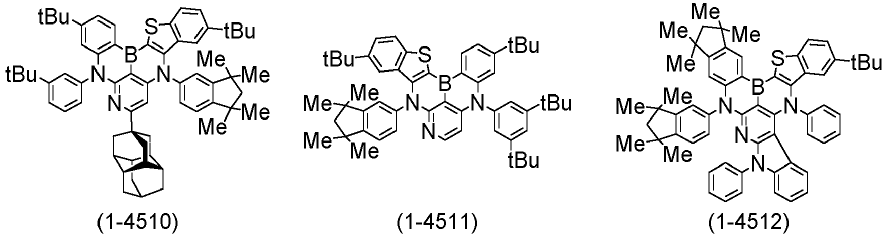

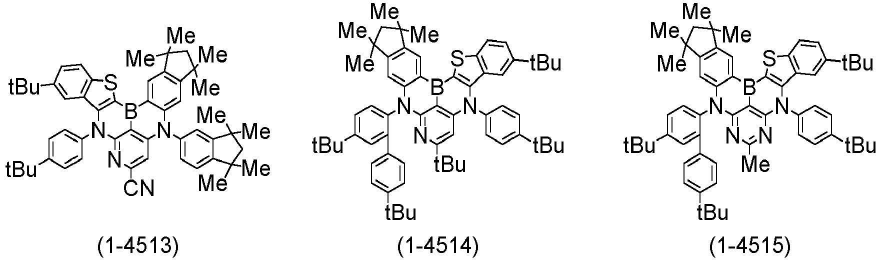

In this paragraph all compounds are inventive compounds.

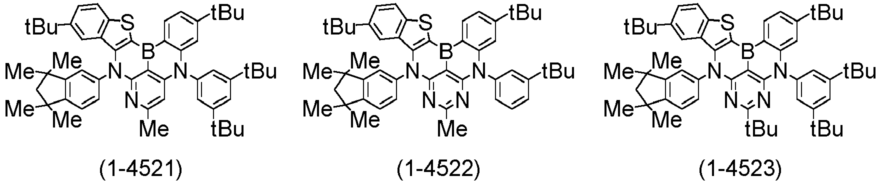

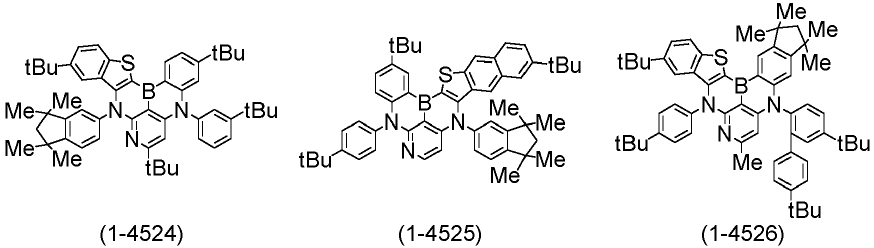

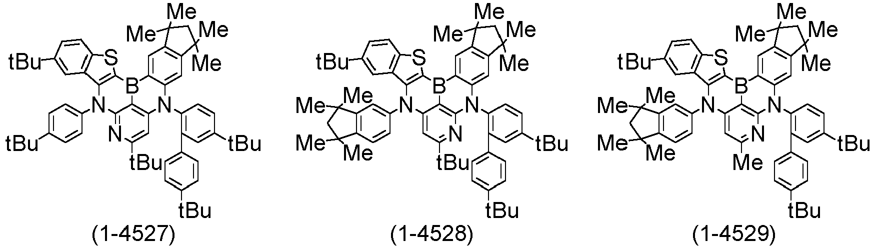

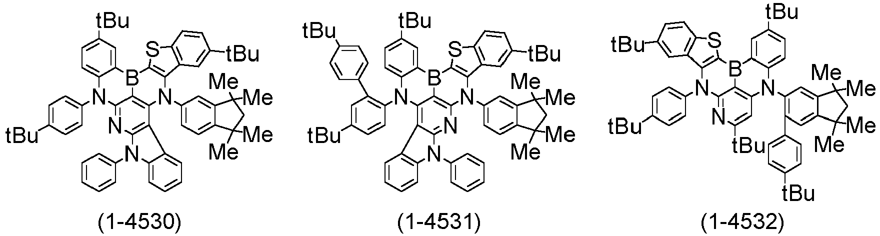

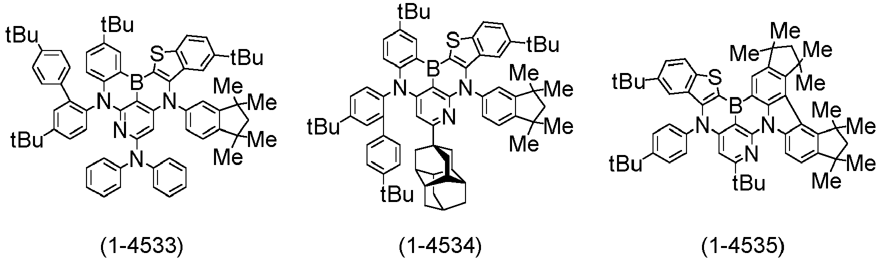

In this paragraph all compounds except compound (1-4545) are inventive compounds.

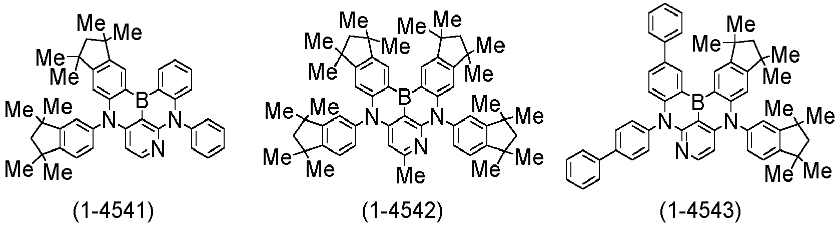

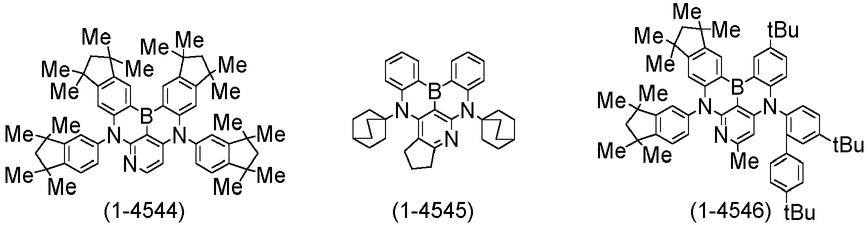

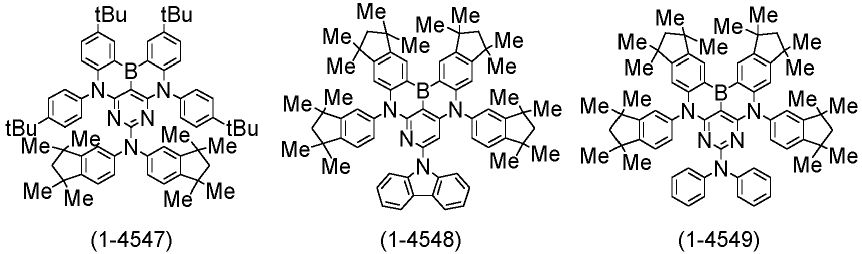

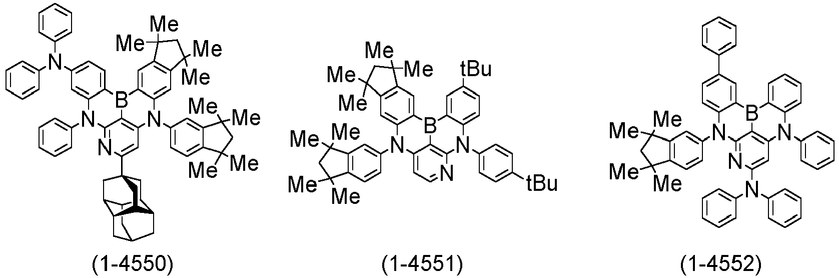

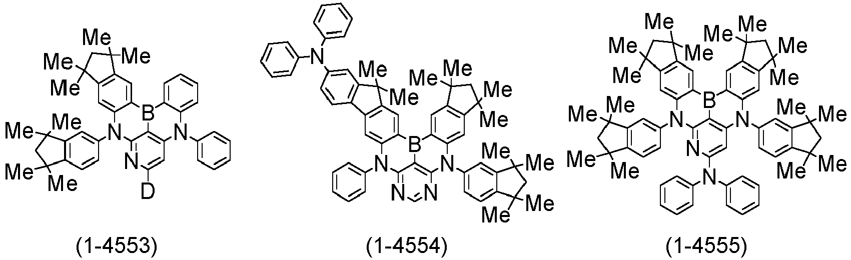

In this paragraph all compounds except compound (1-4574) are inventive compounds.

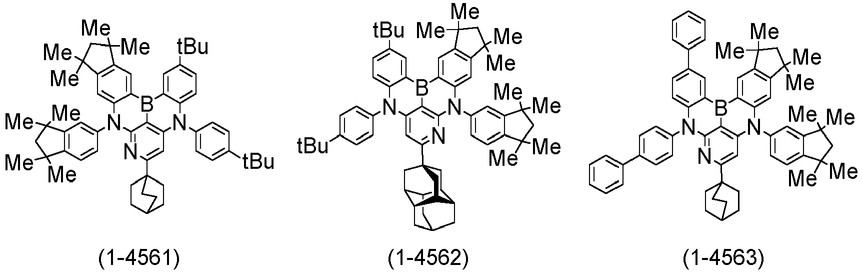

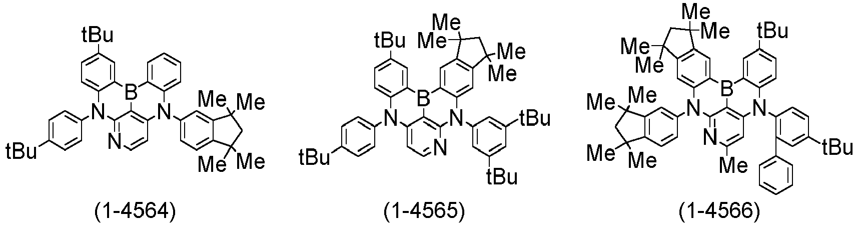

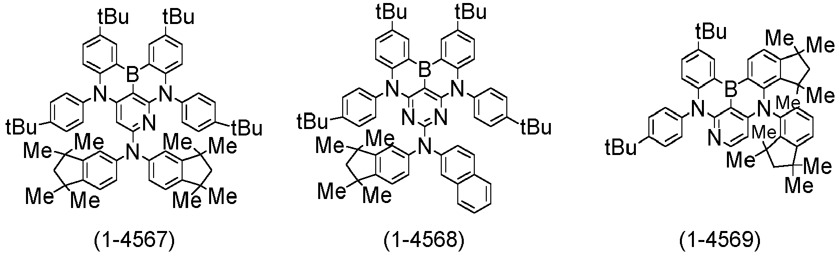

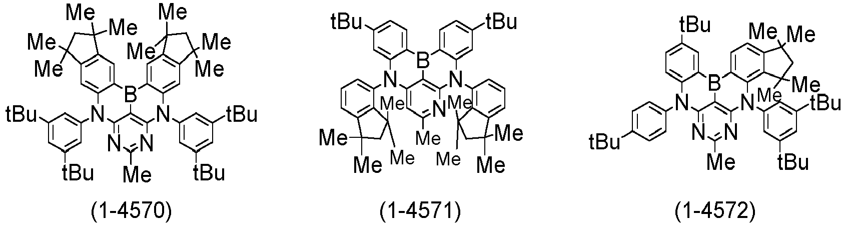

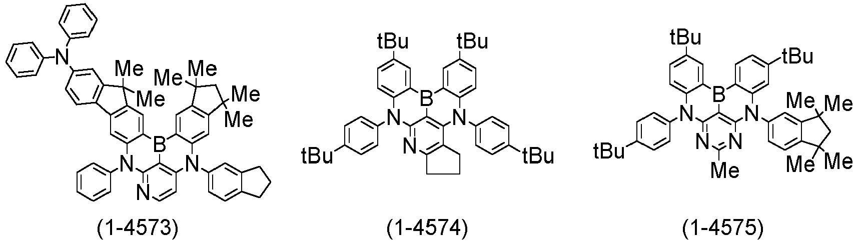

In this paragraph all compounds are inventive compounds.

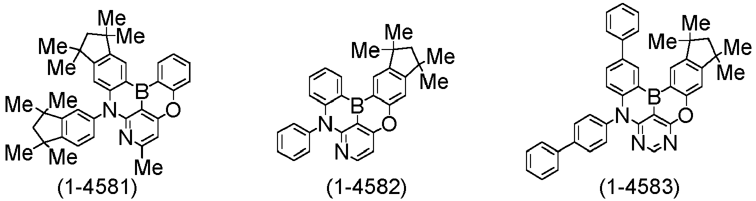

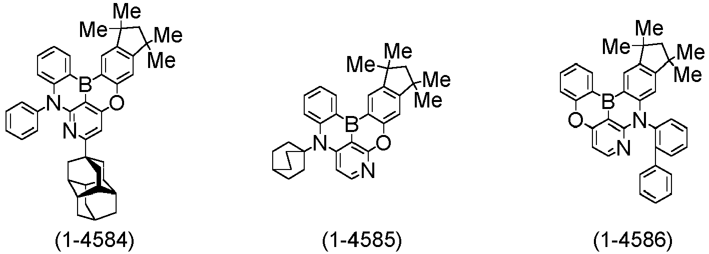

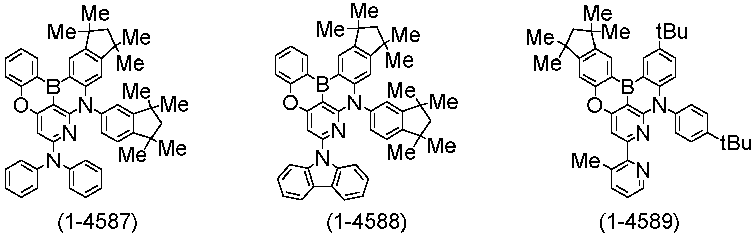

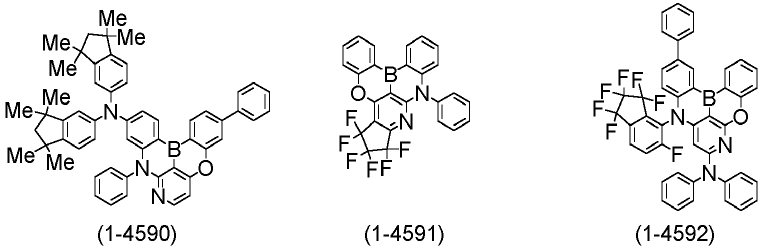

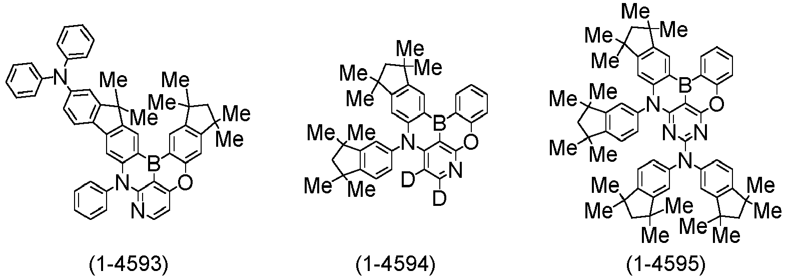

In this paragraph all compounds except compounds (1-4608), (1-4614) are inventive compounds.

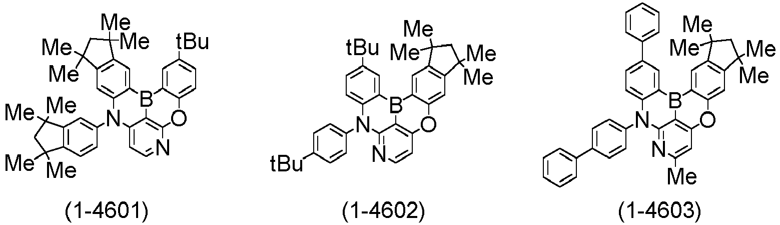

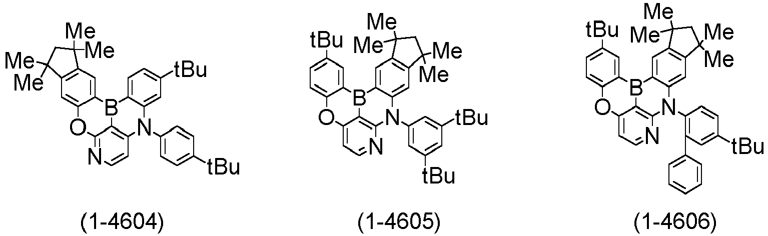

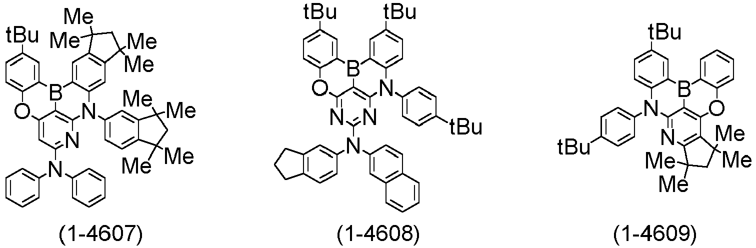

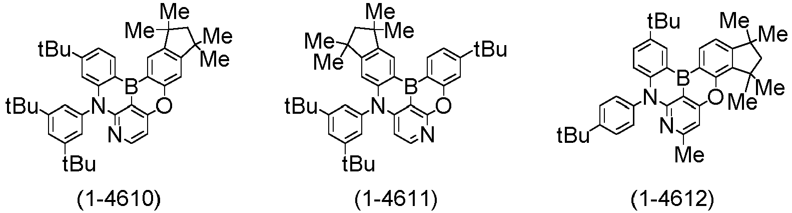

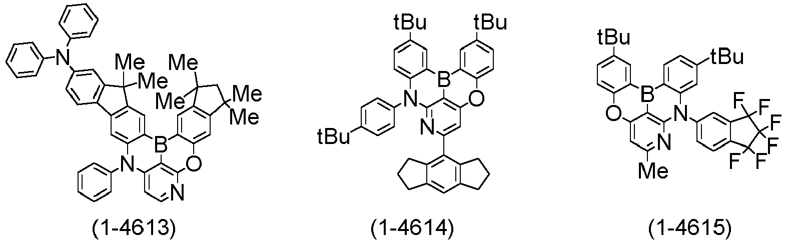

In this paragraph all compounds except compound (1-4626) are inventive compounds.

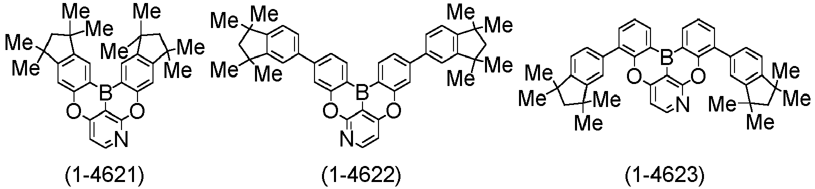

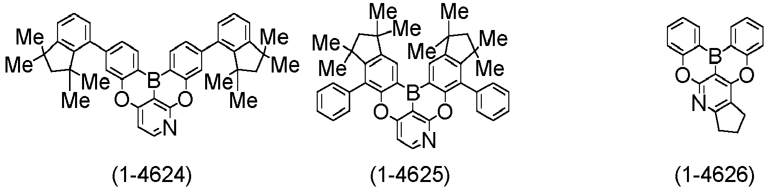

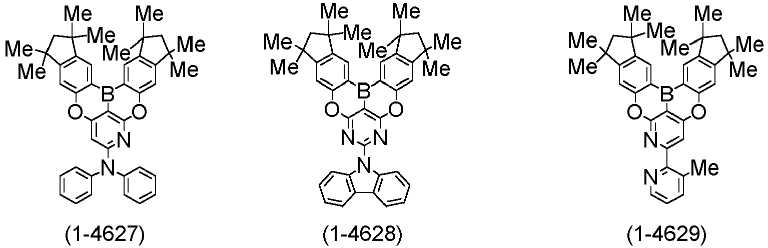

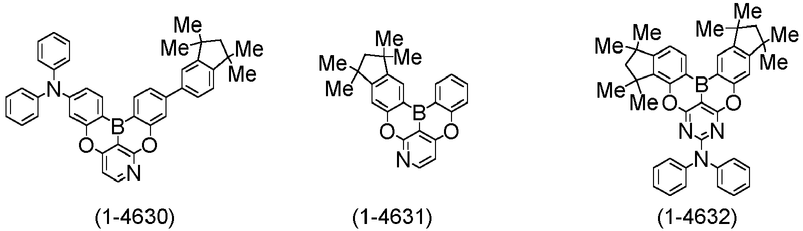

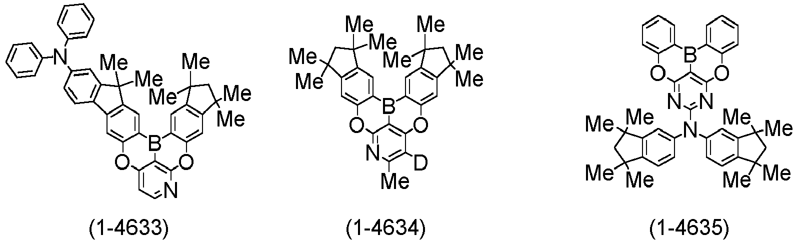

In this paragraph all compounds except compounds (1-4650), (1-4651) are inventive compounds.

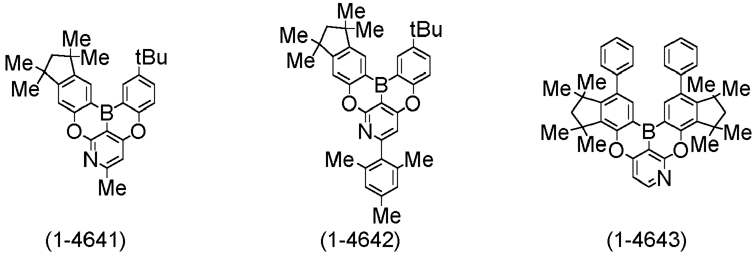

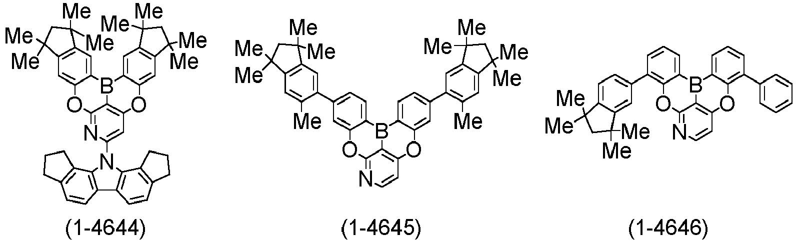

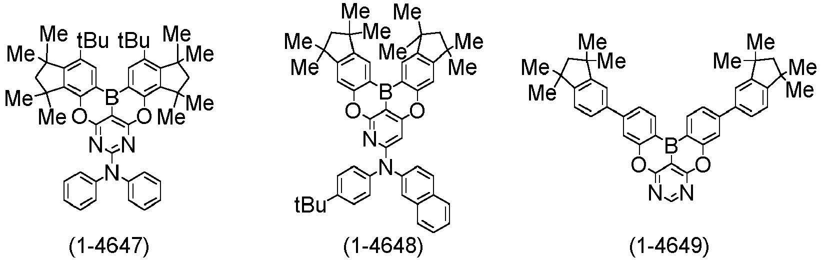

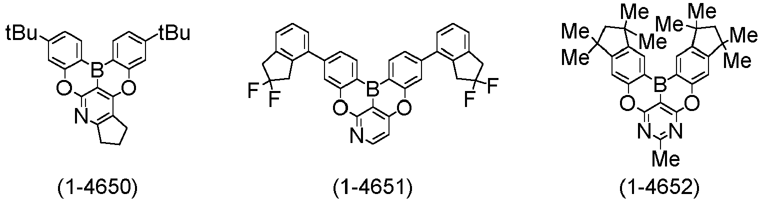

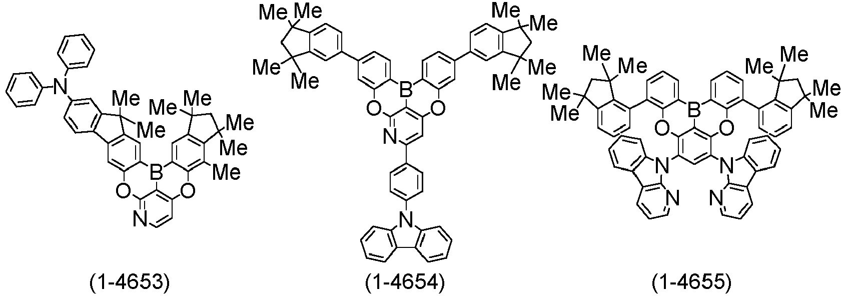

In this paragraph all compounds are inventive compounds.

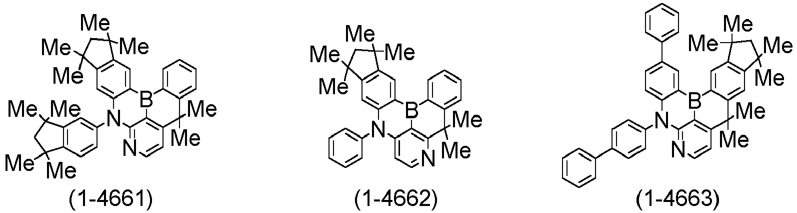

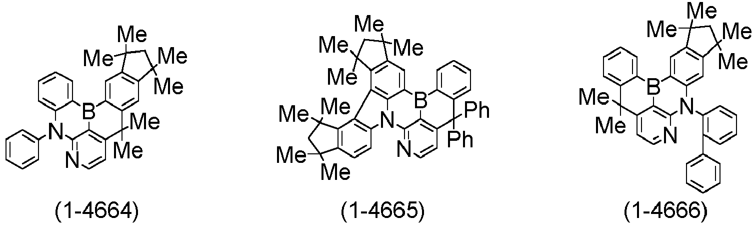

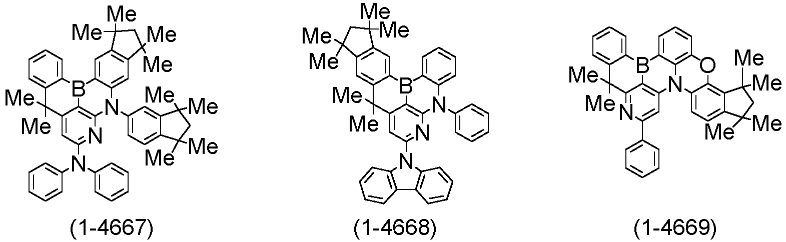

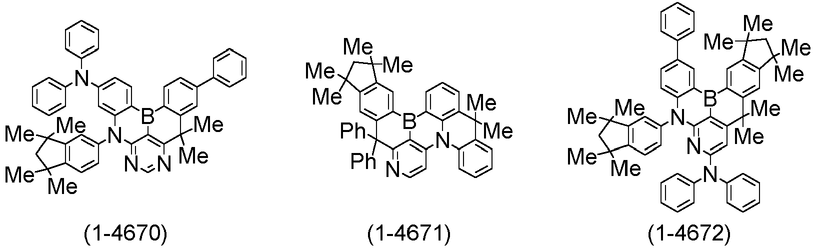

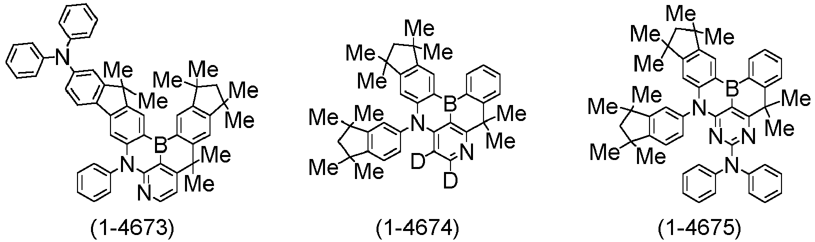

In this paragraph all compounds are inventive compounds.

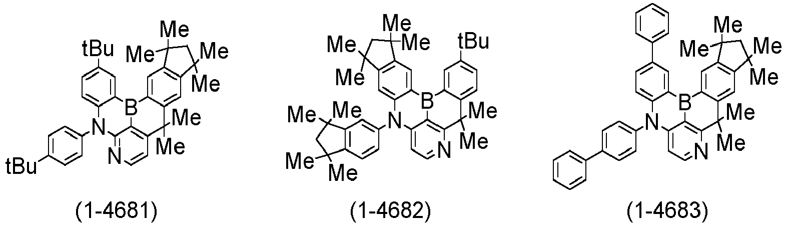

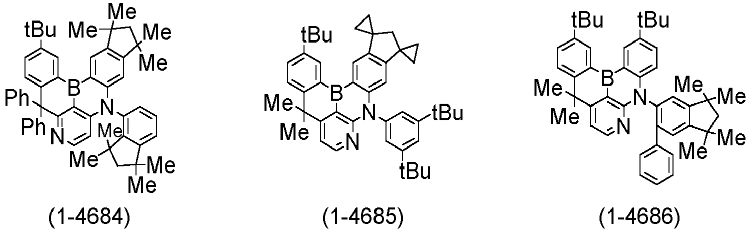

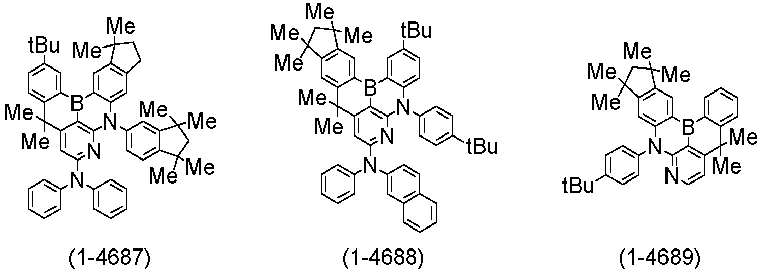

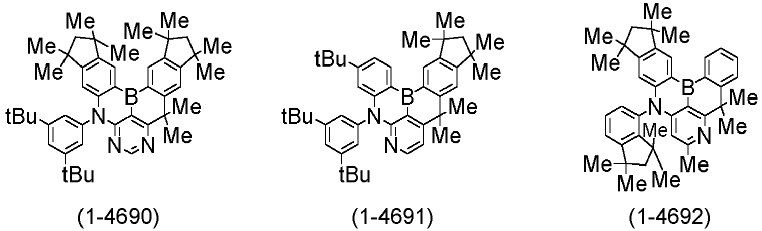

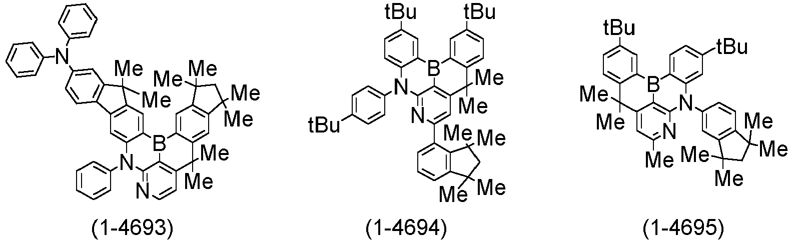

In this paragraph all compounds are inventive compounds.

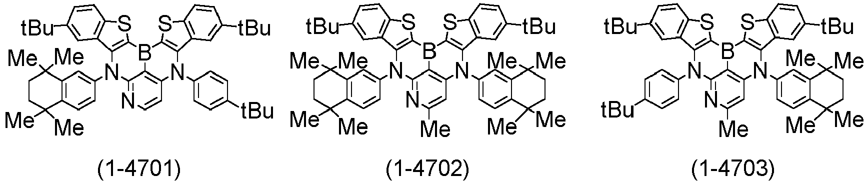

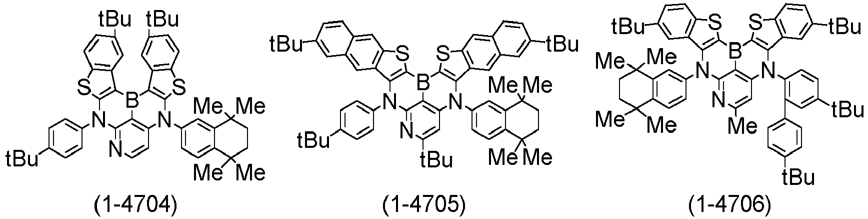

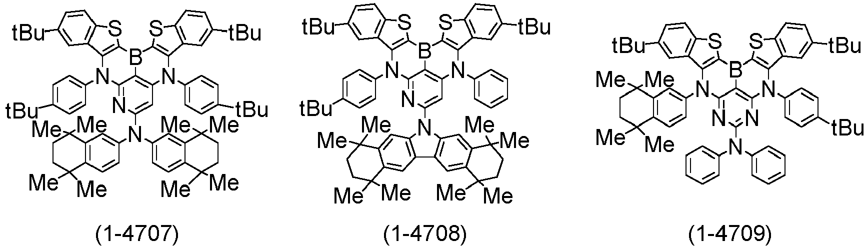

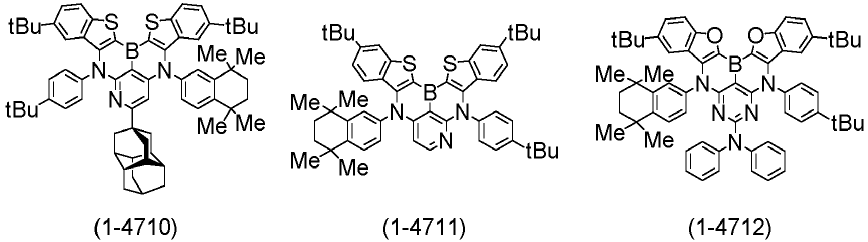

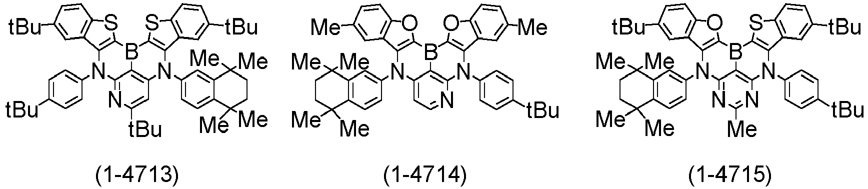

In this paragraph all compounds are inventive compounds.

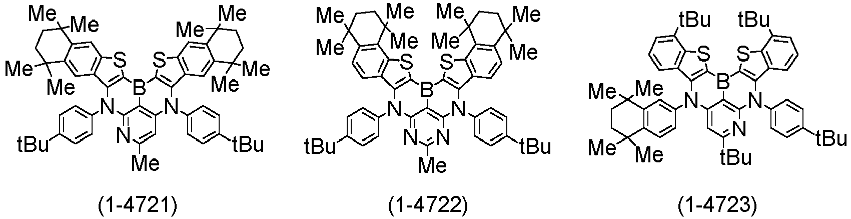

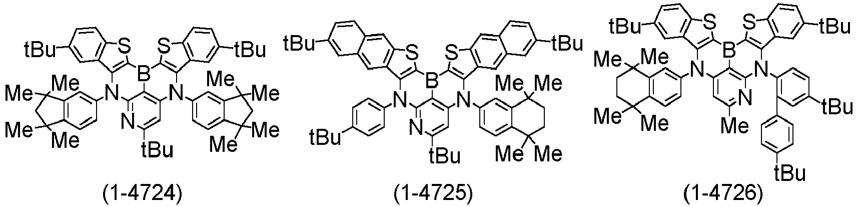

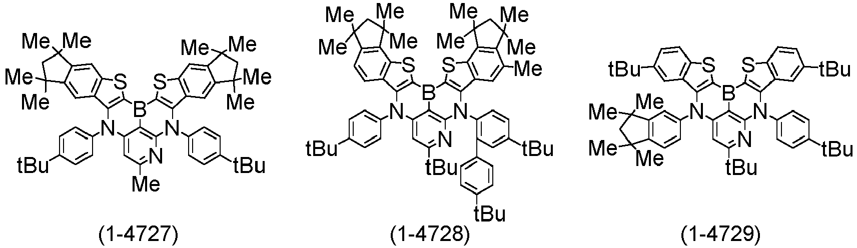

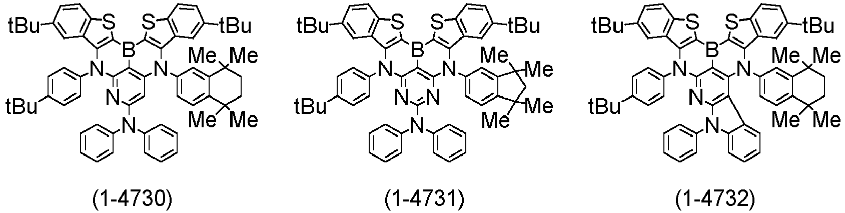

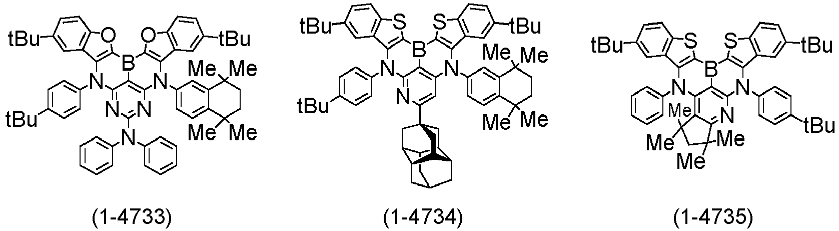

In this paragraph all compounds are inventive compounds.

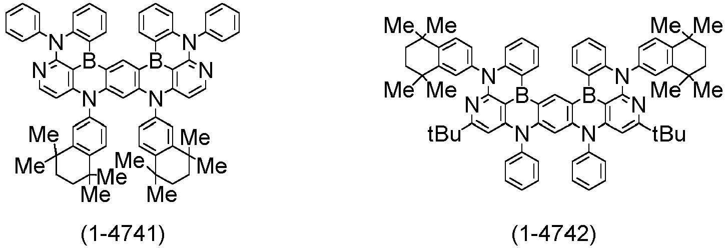

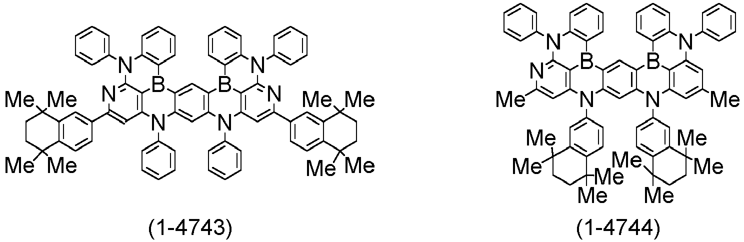

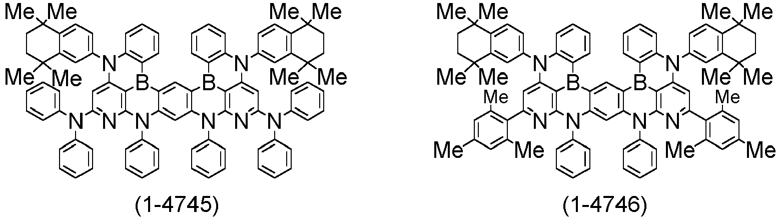

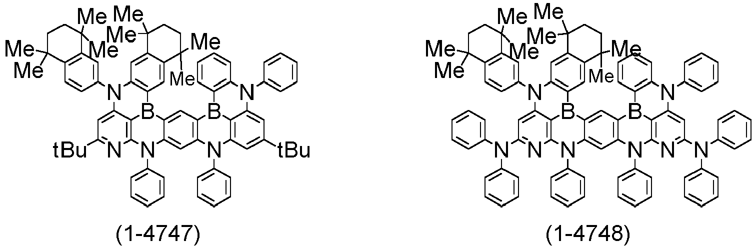

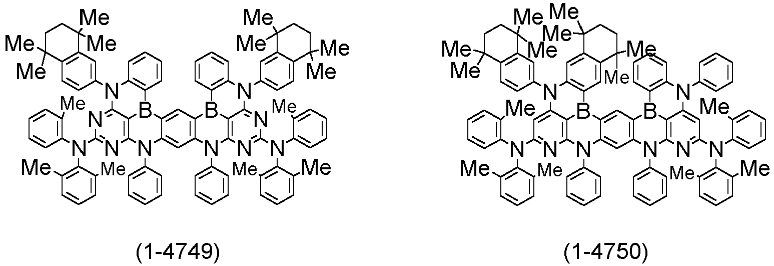

In this paragraph all compounds are inventive compounds.

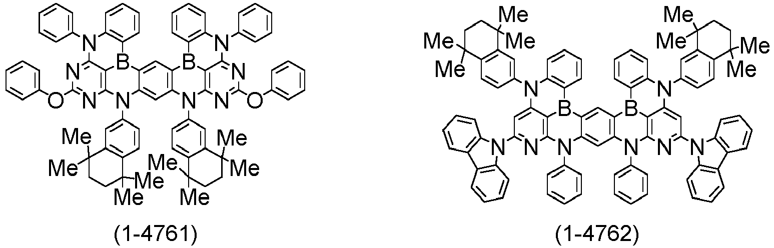

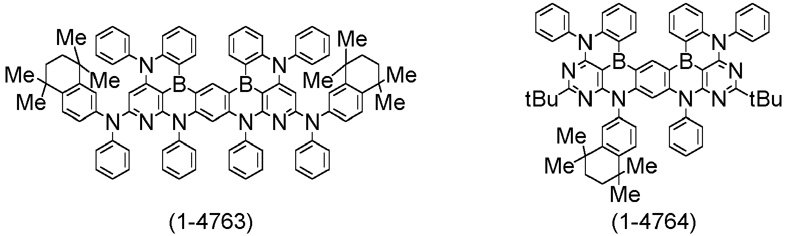

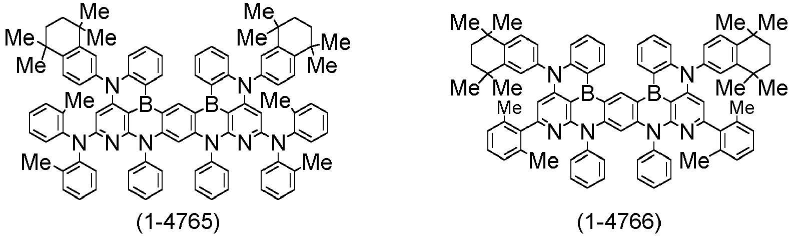

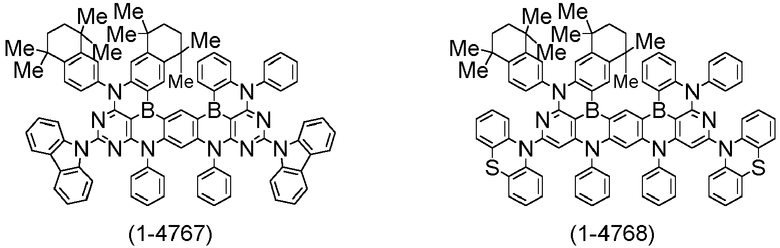

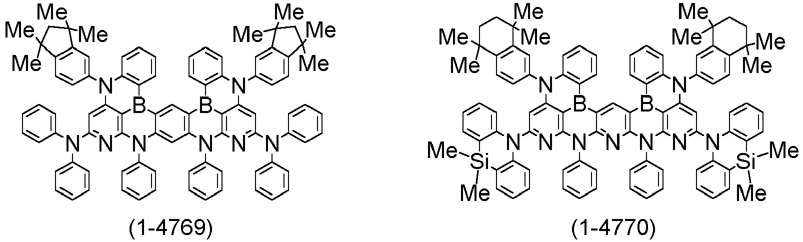

In this paragraph all compounds are inventive compounds.

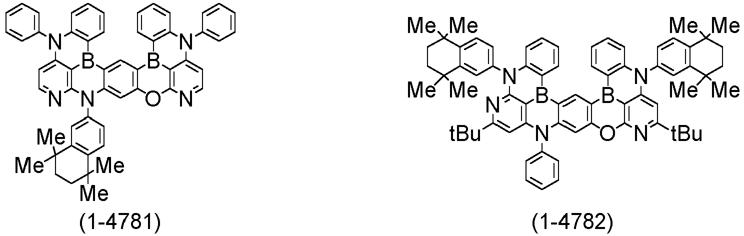

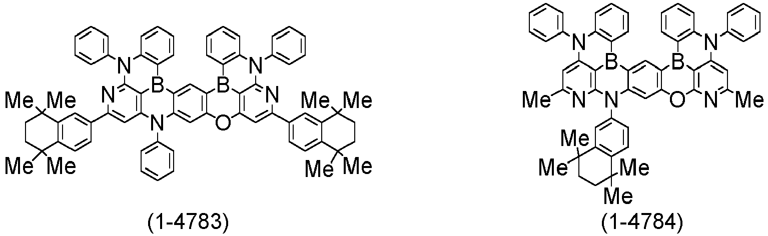

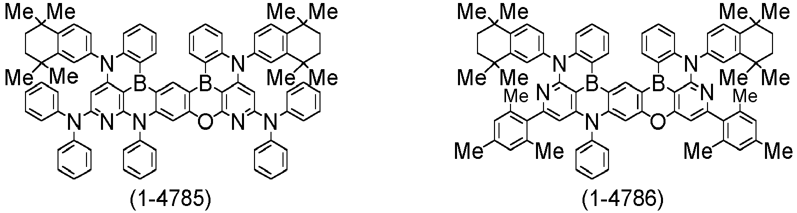

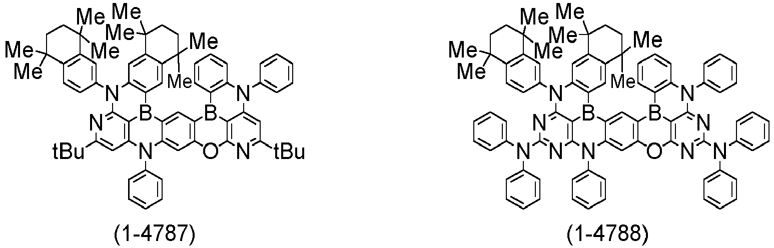

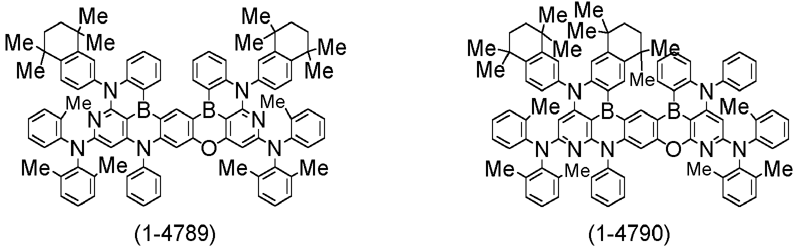

In this paragraph all compounds are inventive compounds.

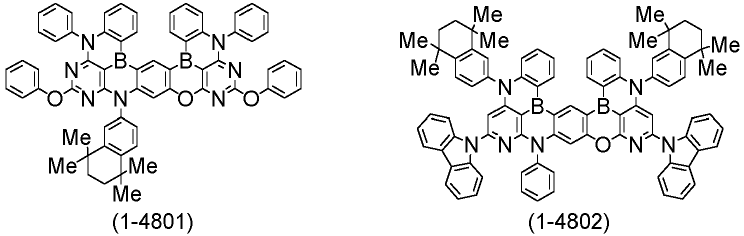

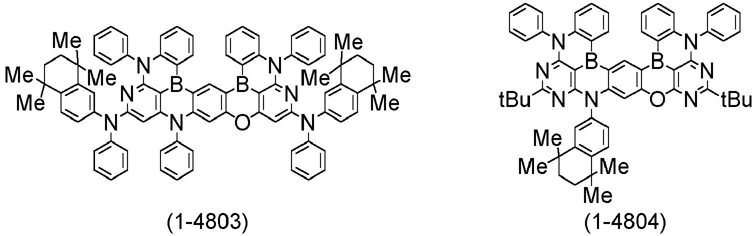

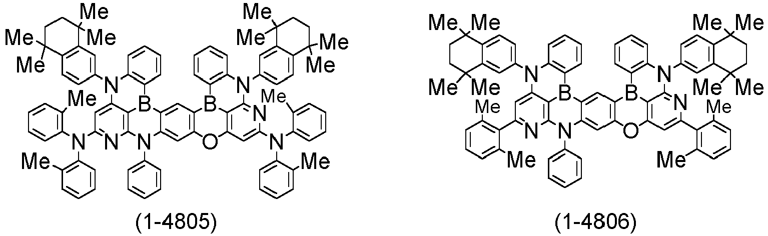

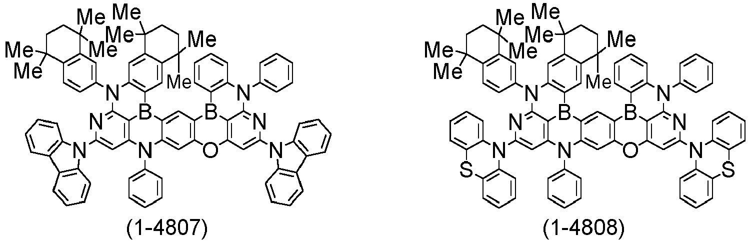

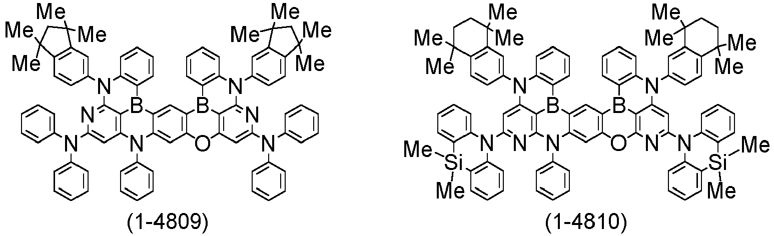

In this paragraph all compounds are inventive compounds.

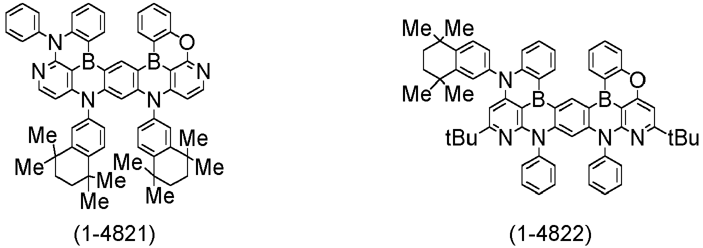

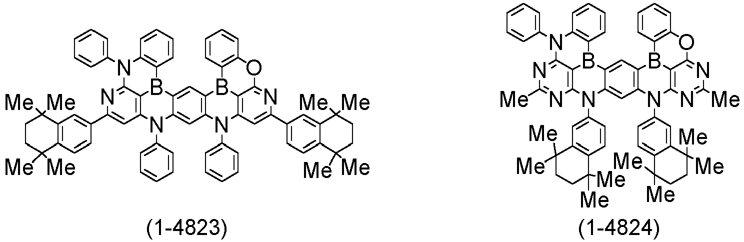

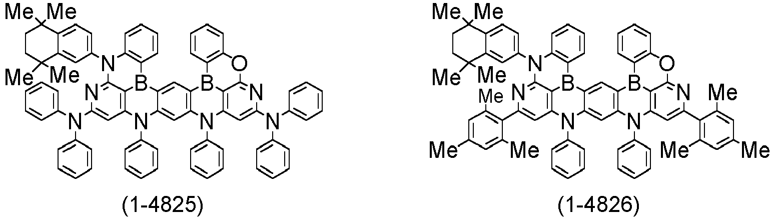

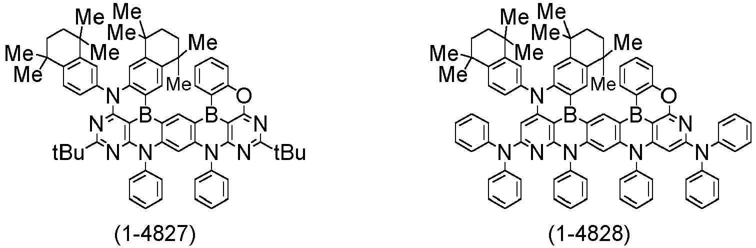

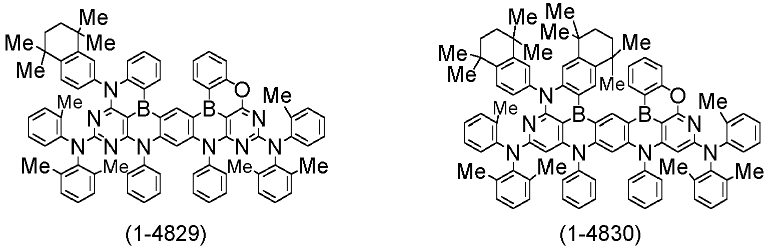

In this paragraph all compounds are inventive compounds.

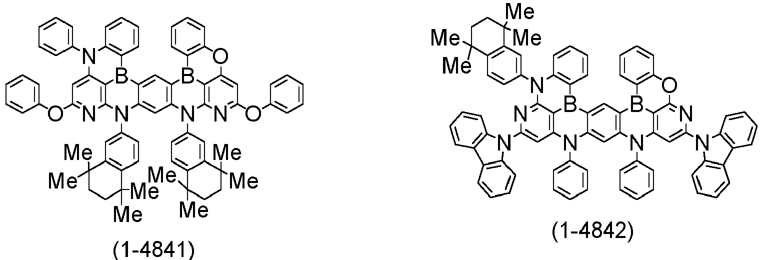

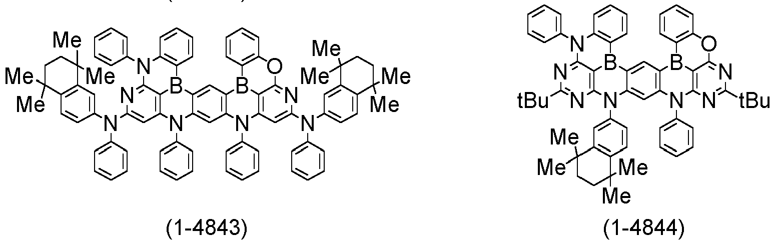

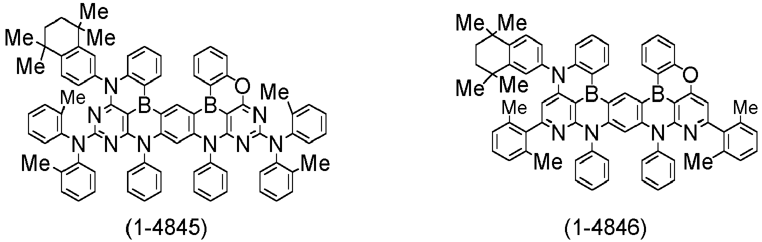

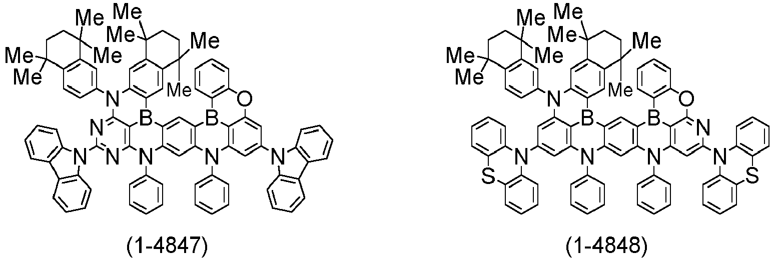

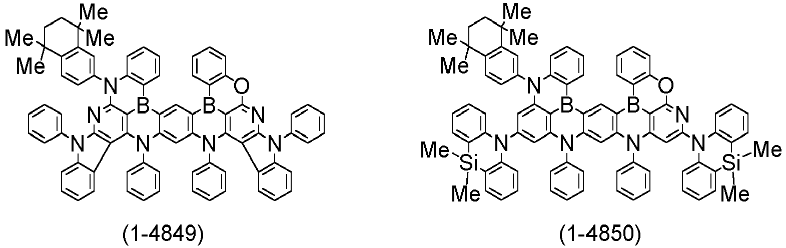

In this paragraph all compounds are inventive compounds.

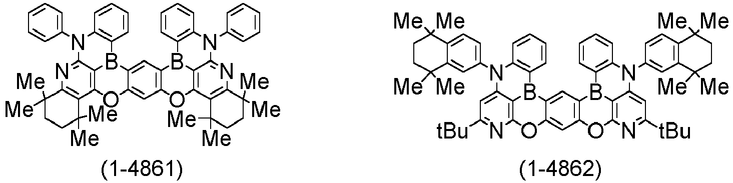

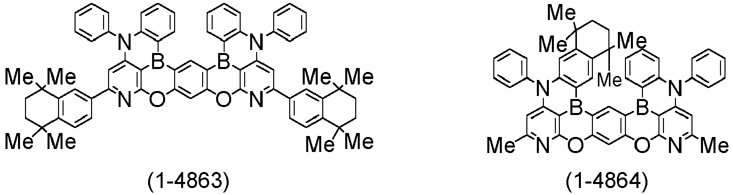

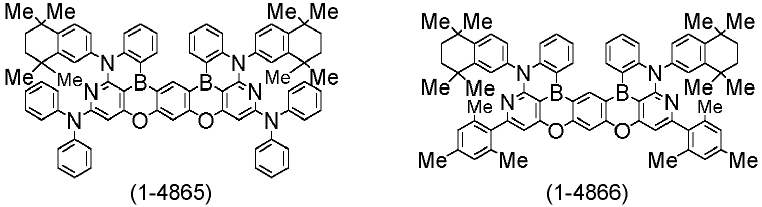

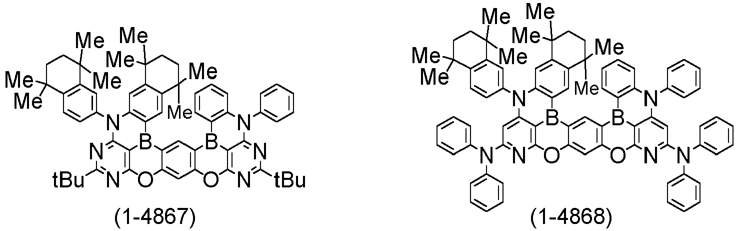

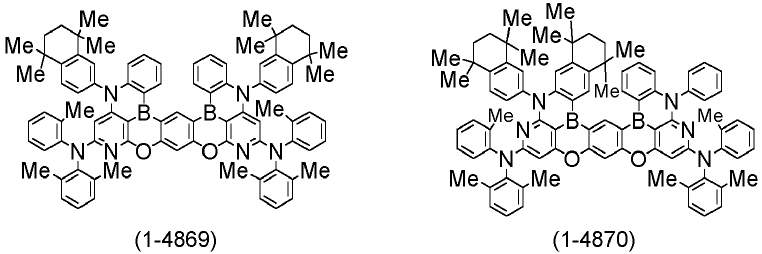

In this paragraph all compounds are inventive compounds.

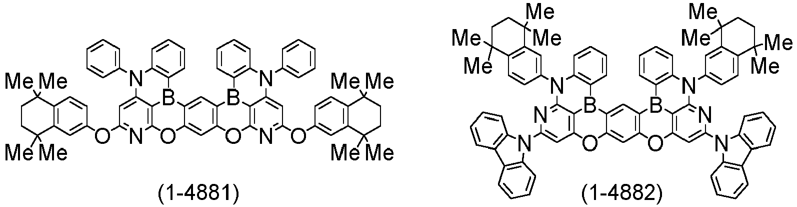

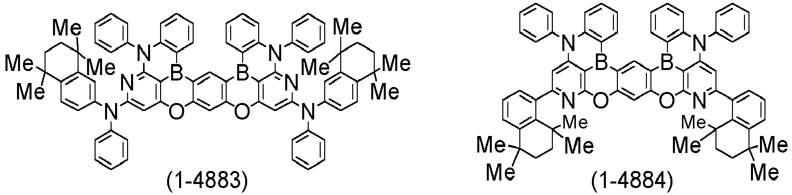

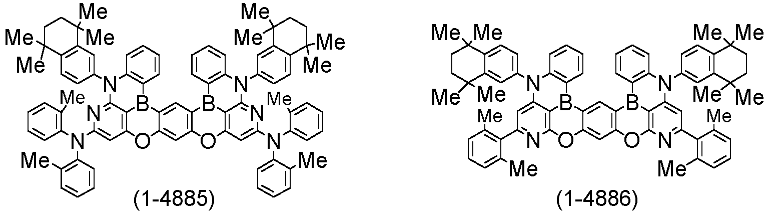

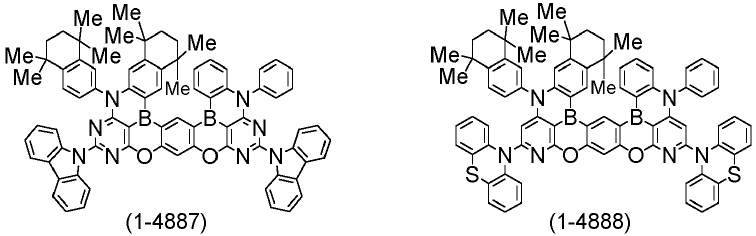

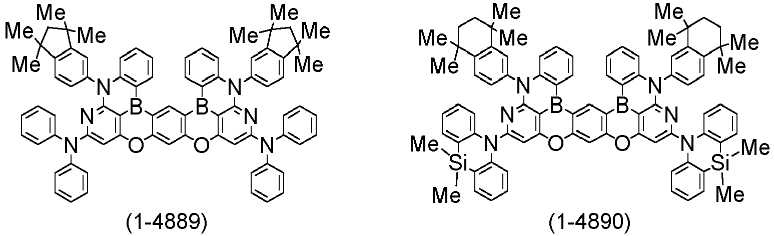

In this paragraph all compounds are inventive compounds.

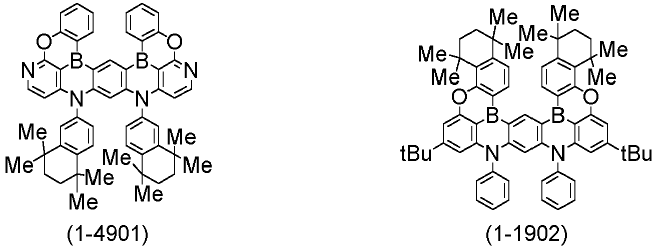

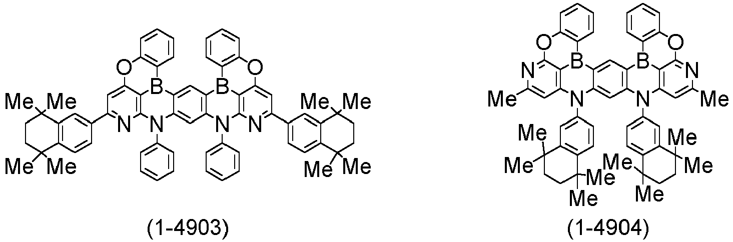

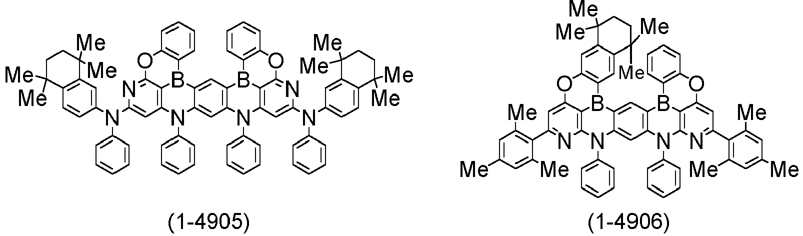

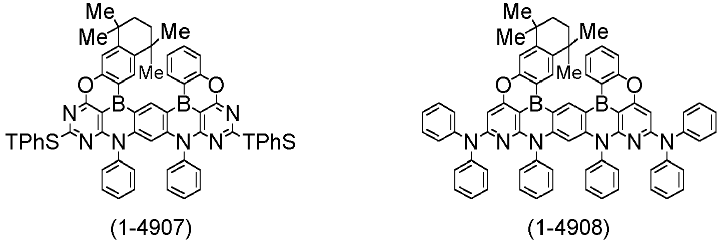

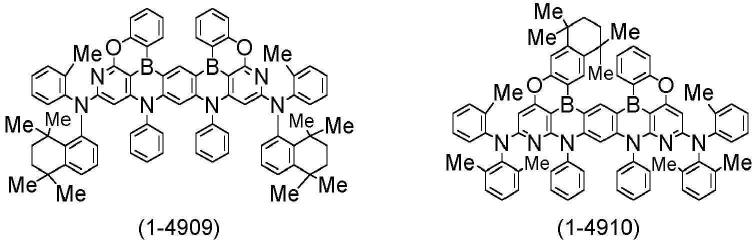

In this paragraph all compounds are inventive compounds.

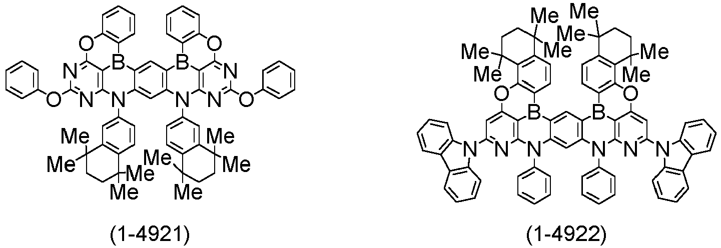

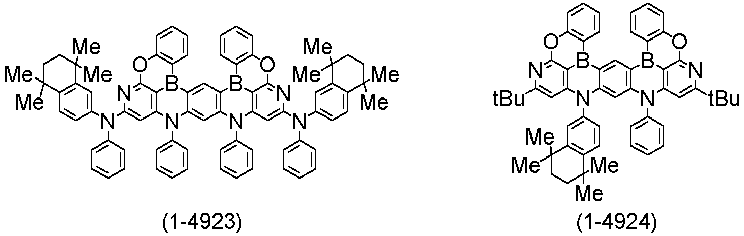

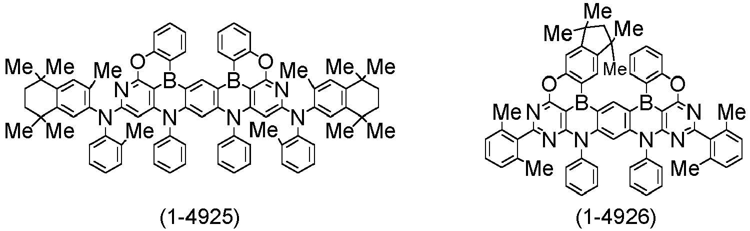

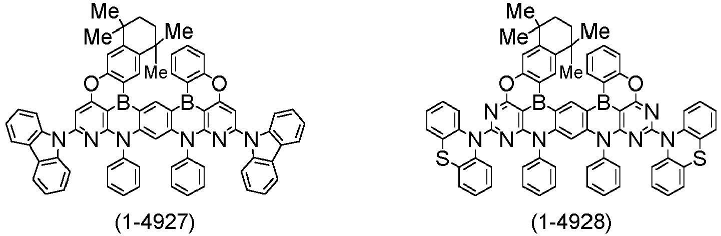

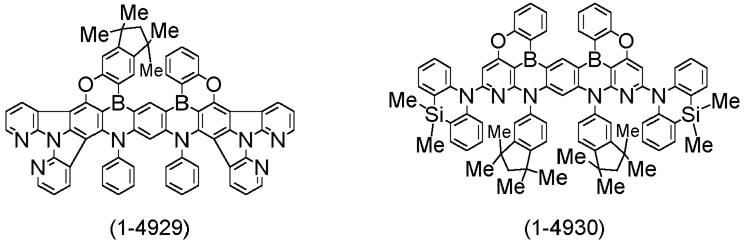

In this paragraph all compounds are inventive compounds.

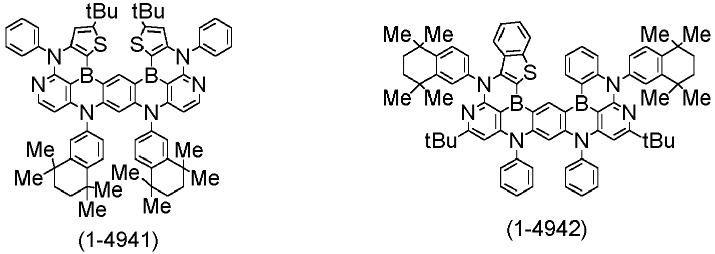

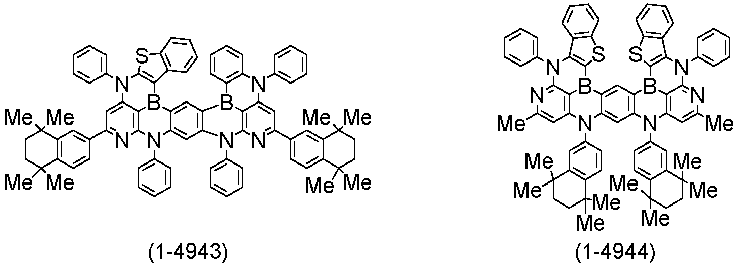

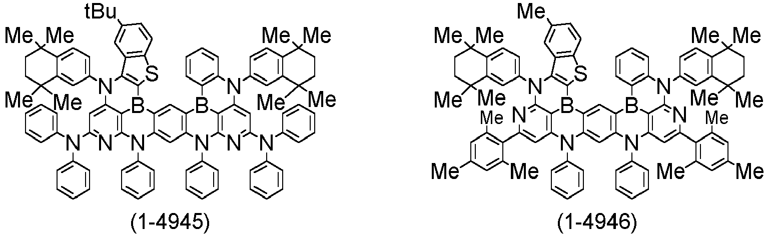

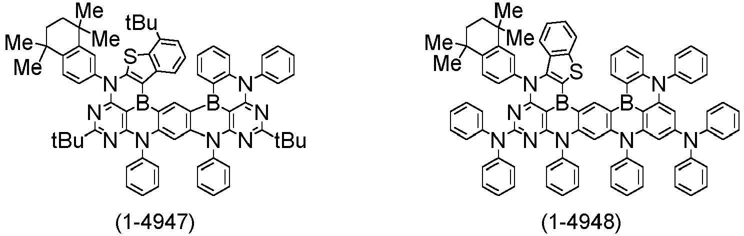

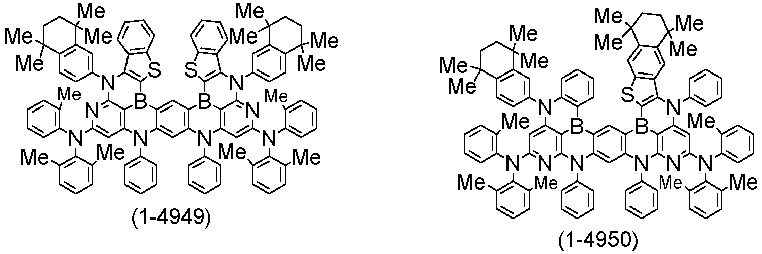

In this paragraph all compounds are inventive compounds.

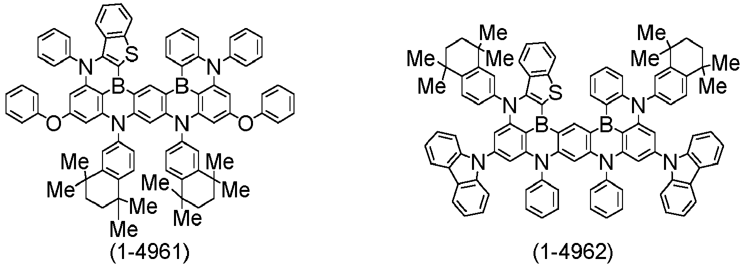

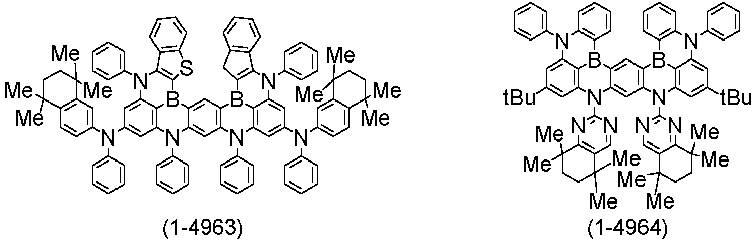

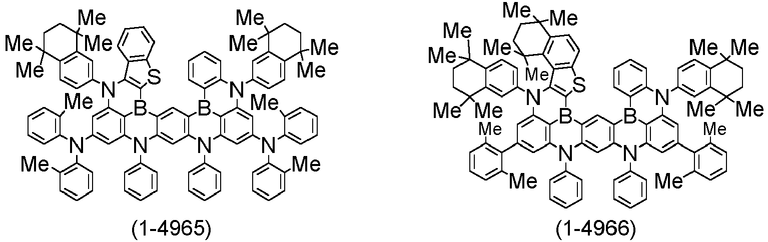

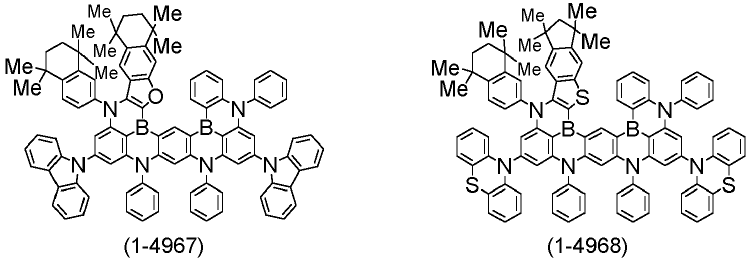

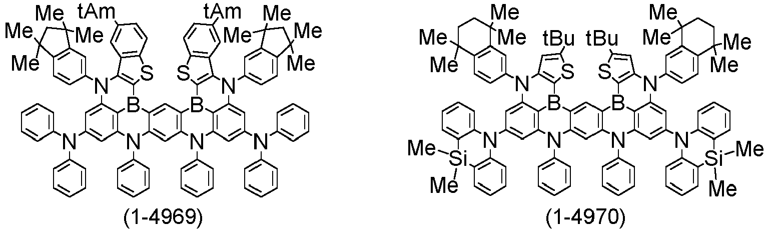

In this paragraph all compounds are inventive compounds.

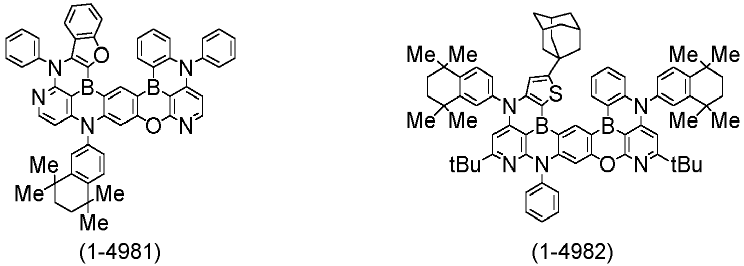

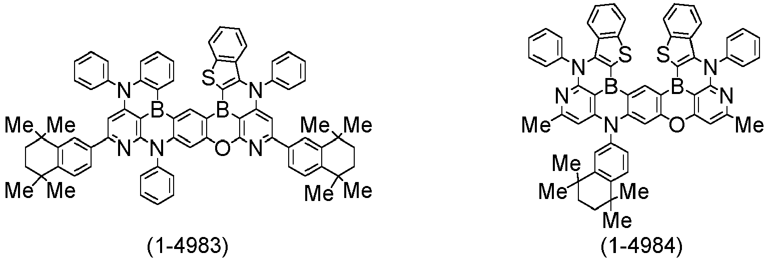

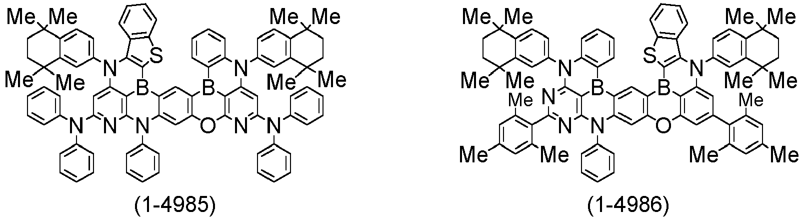

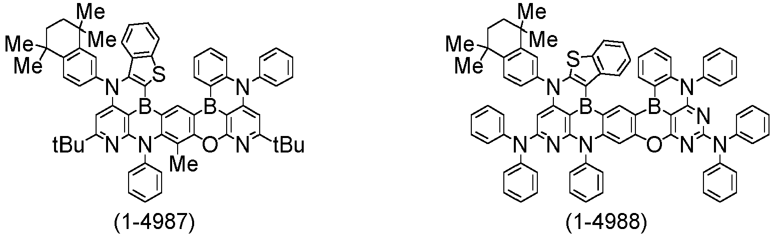

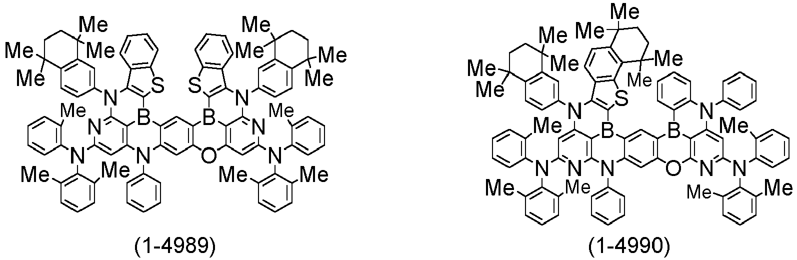

In this paragraph all compounds except compound (1-5005) are inventive compounds.

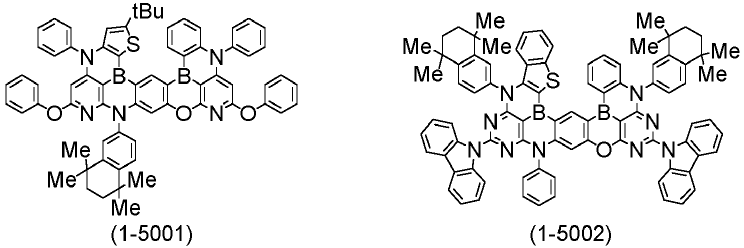

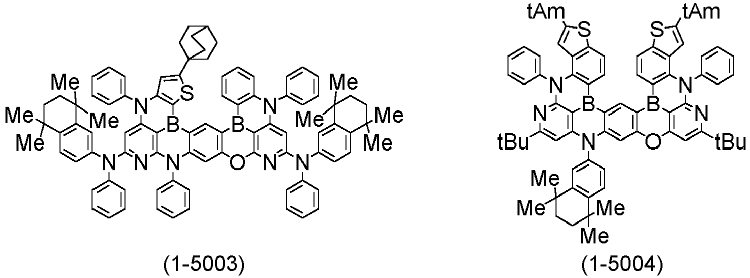

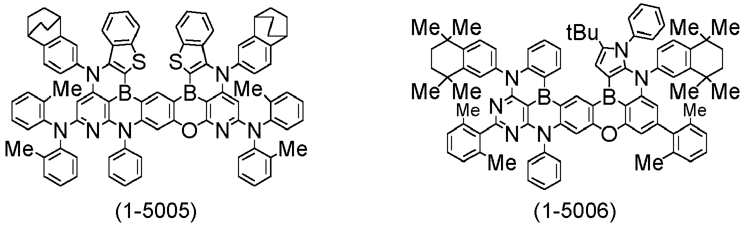

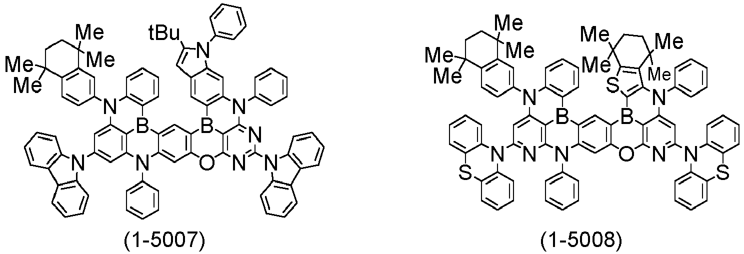

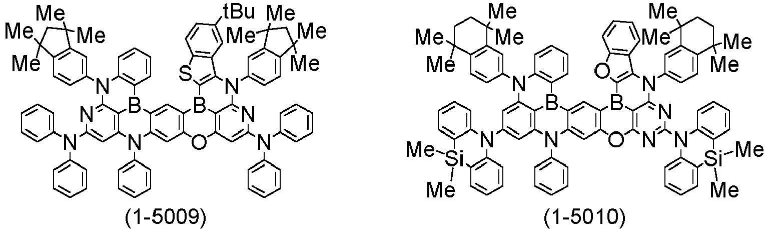

In this paragraph all compounds are inventive compounds.

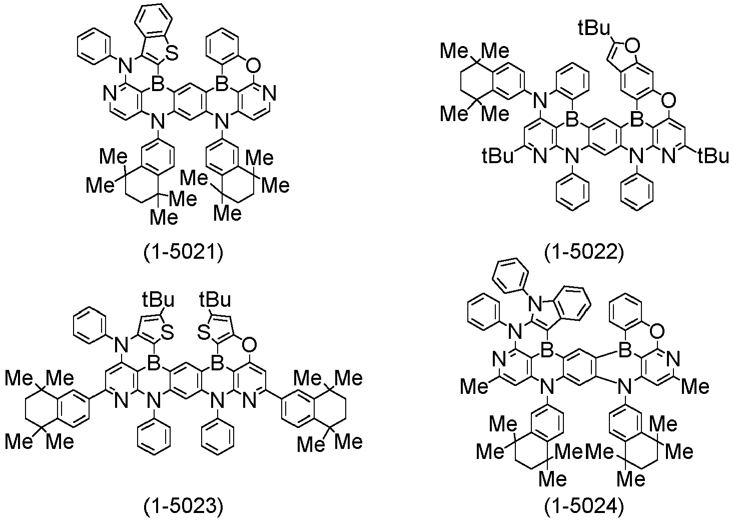

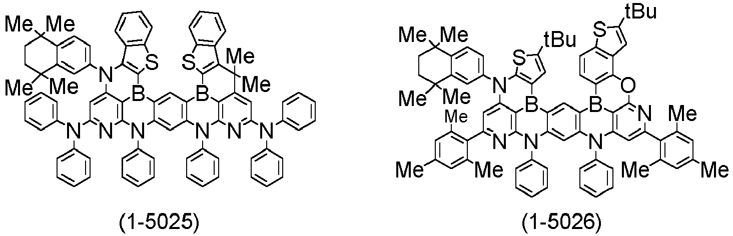

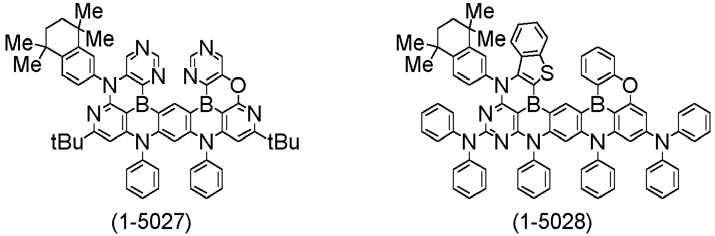

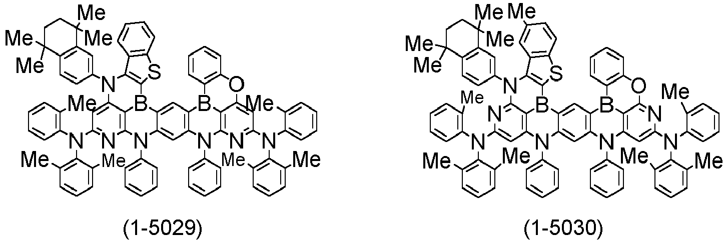

In this paragraph all compounds are inventive compounds.

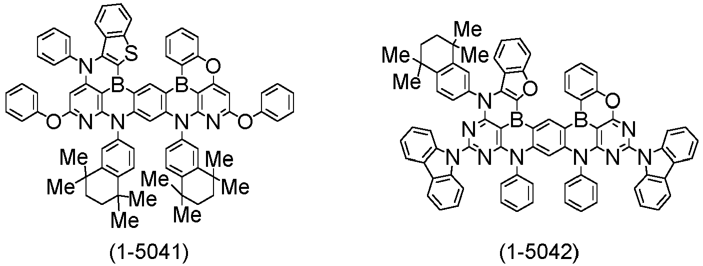

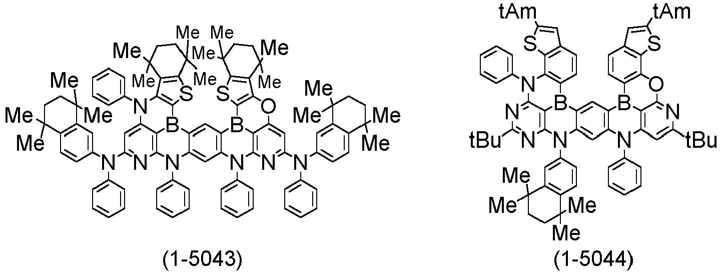

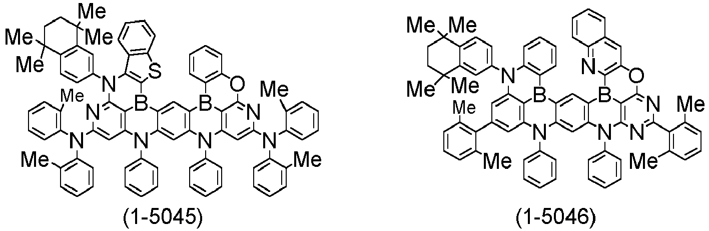

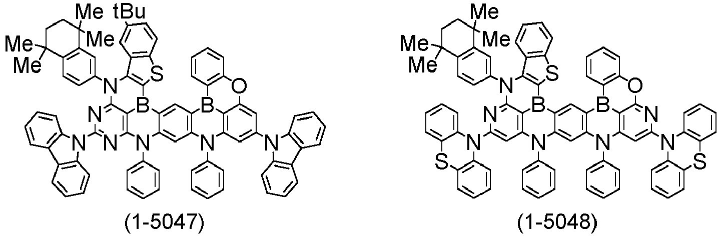

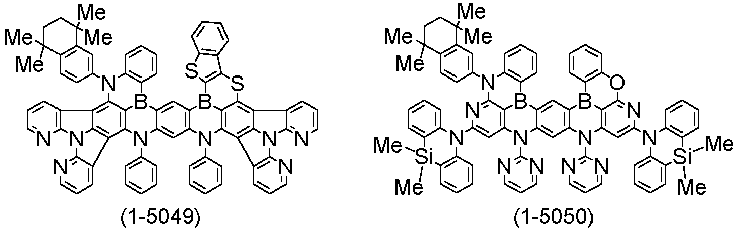

In this paragraph all compounds except compounds (1-5064), (1-5070) are inventive compounds.

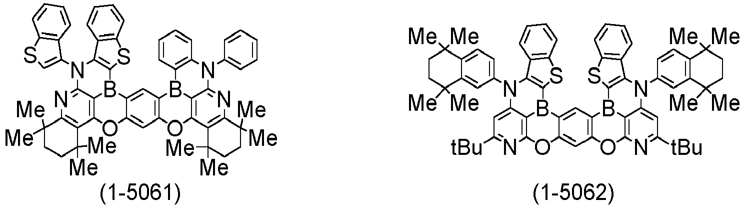

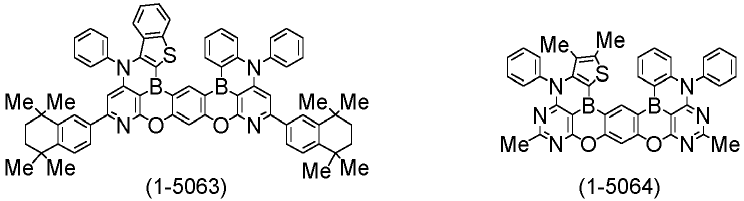

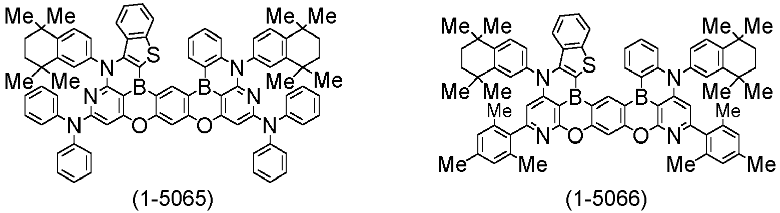

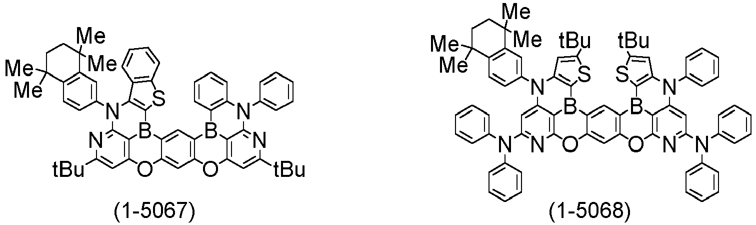

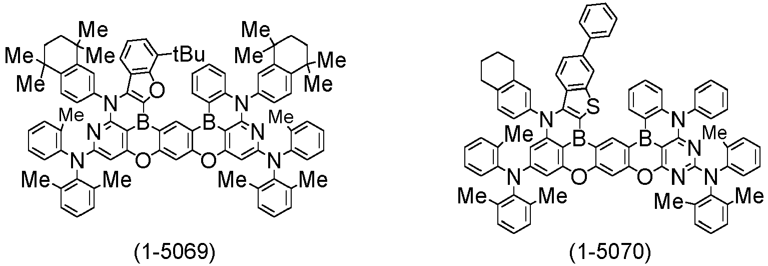

In this paragraph all compounds are inventive compounds.

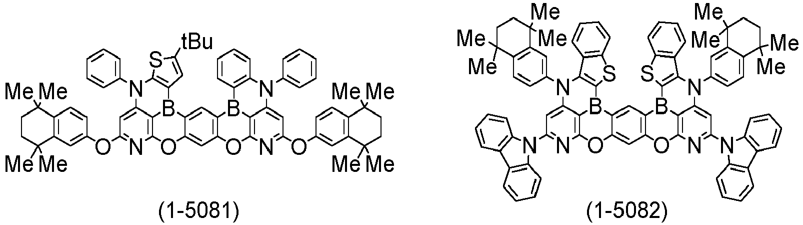

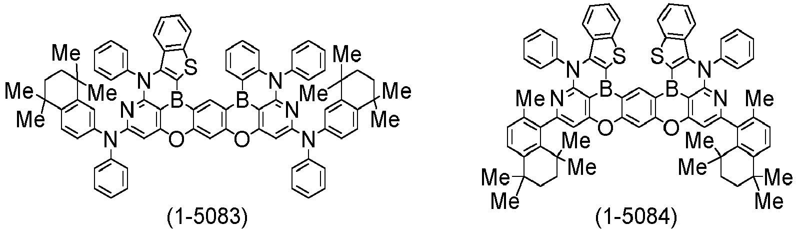

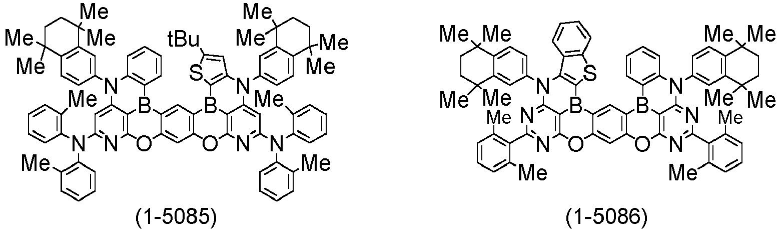

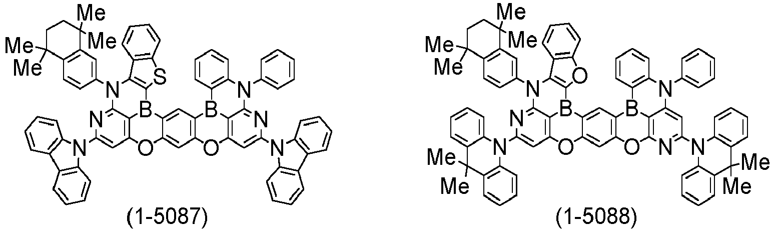

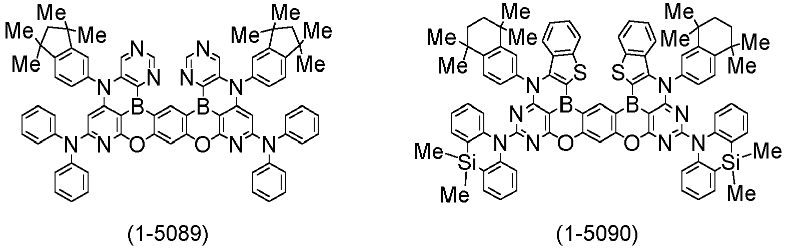

In this paragraph all compounds are inventive compounds.

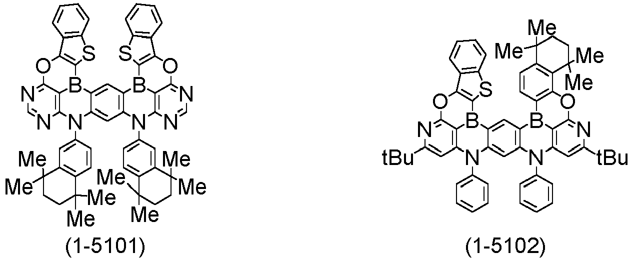

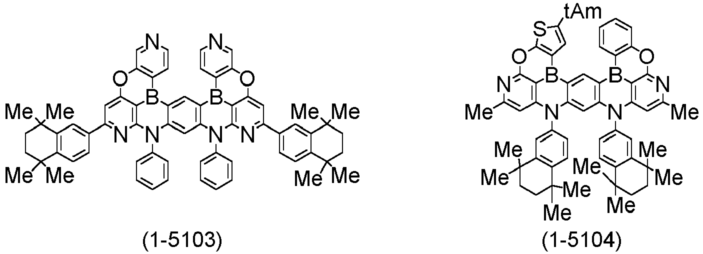

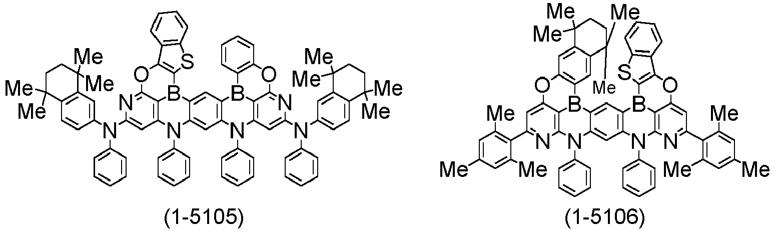

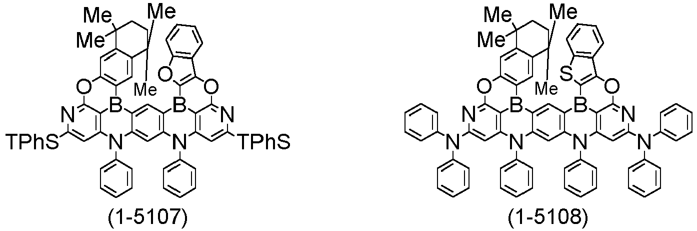

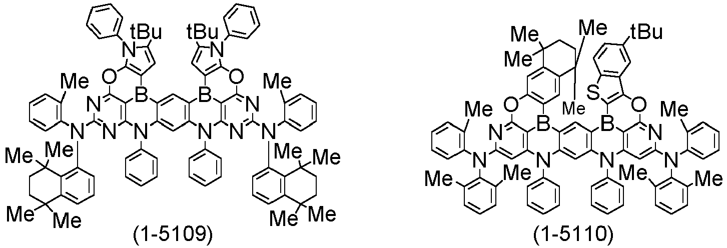

In this paragraph all compounds are inventive compounds.

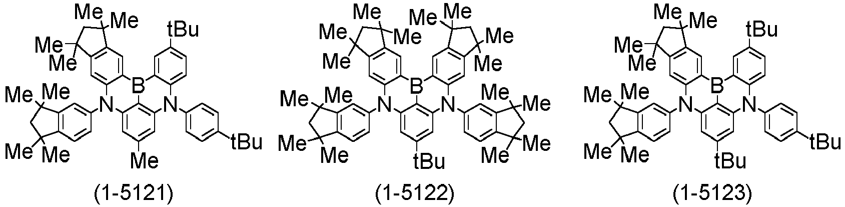

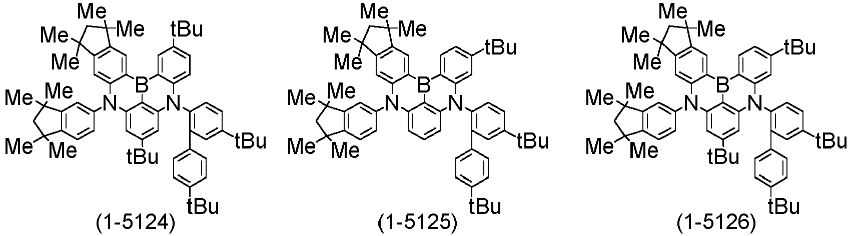

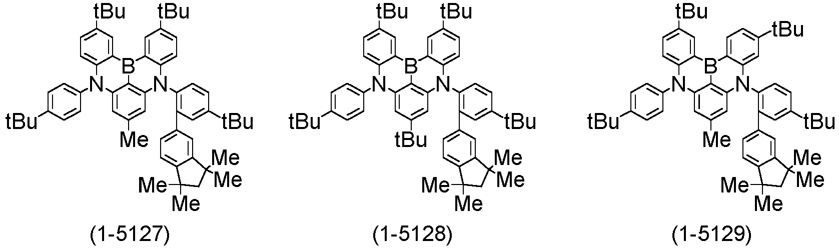

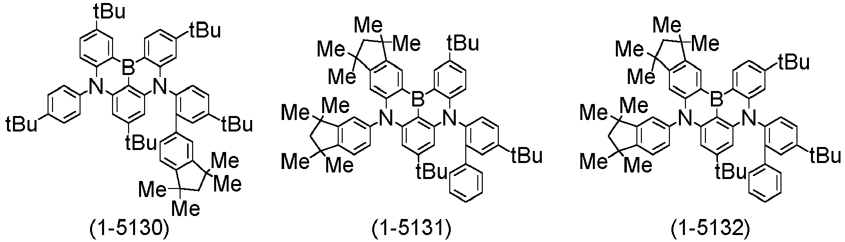

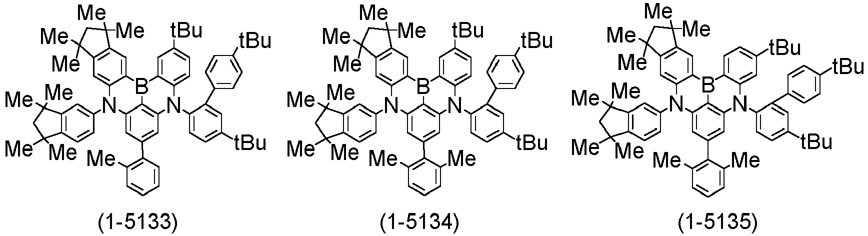

In this paragraph all compounds are inventive compounds.

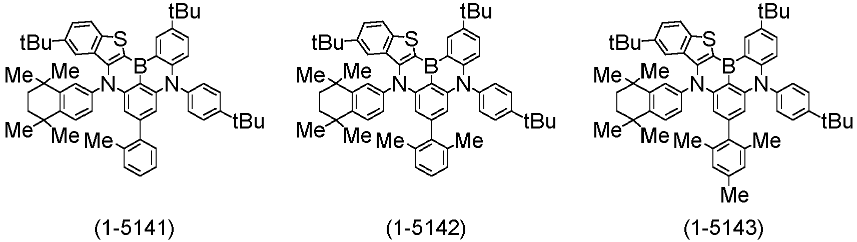

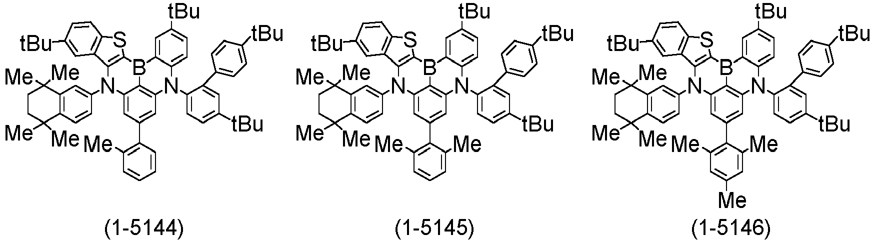

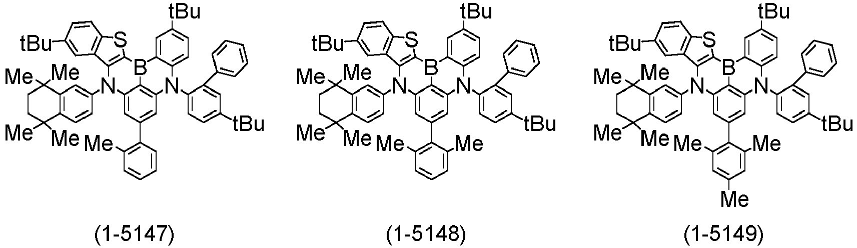

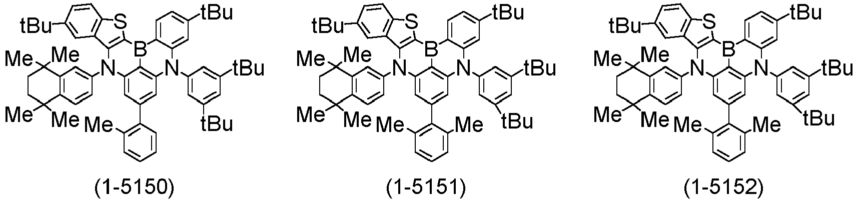

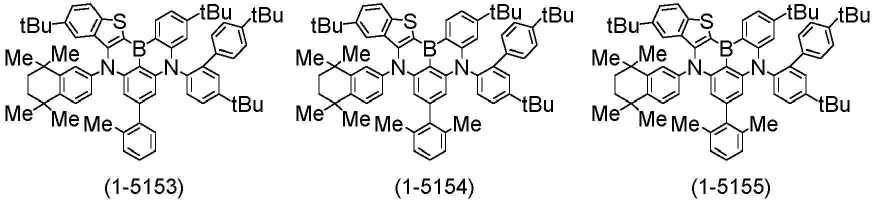

In this paragraph all compounds are inventive compounds.

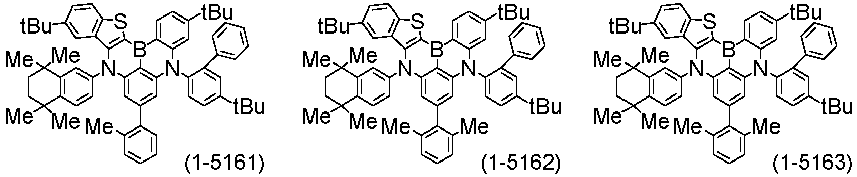

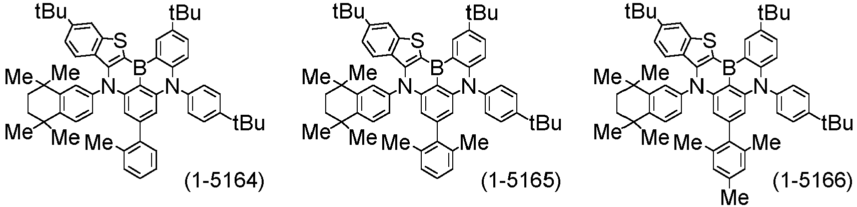

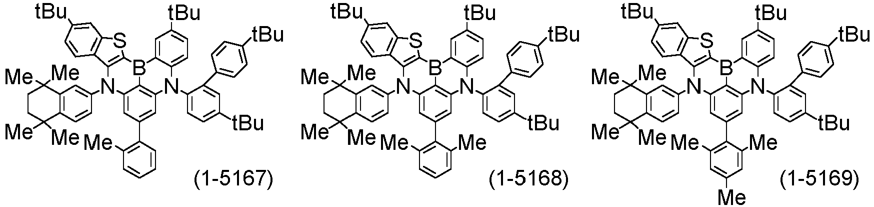

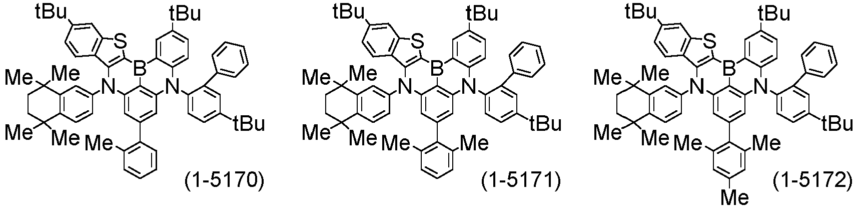

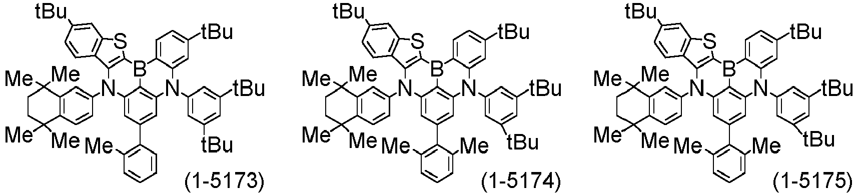

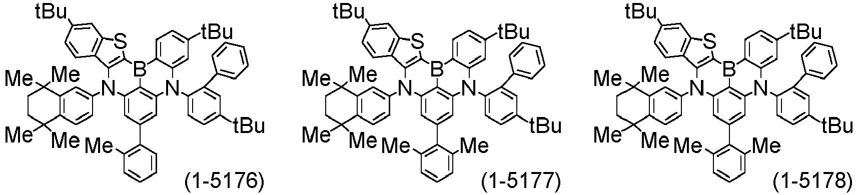

In this paragraph all compounds are inventive compounds.

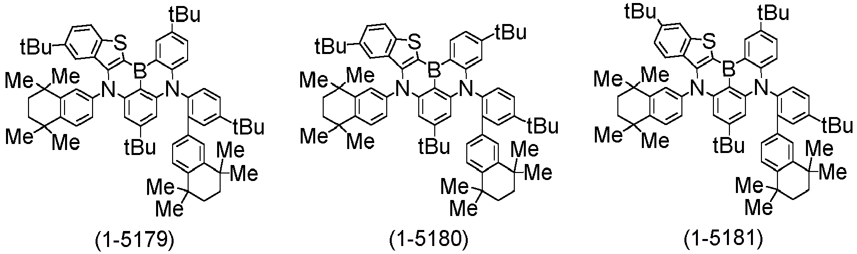

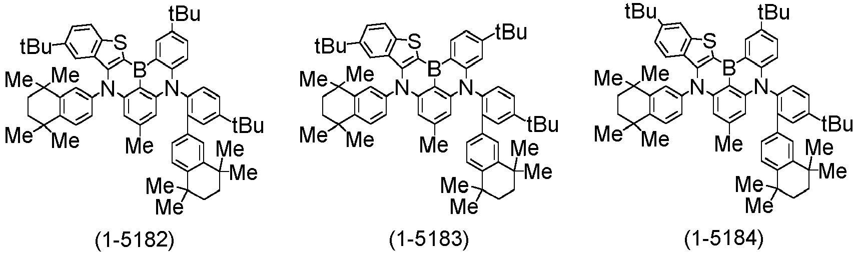

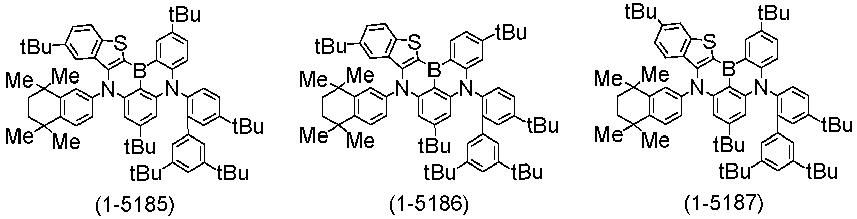

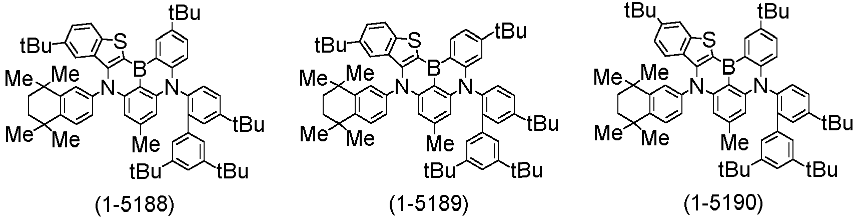

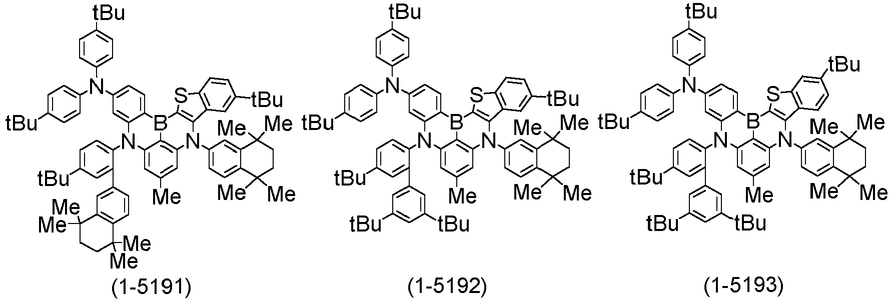

### Note that

(A) at least one of R's of ring A, ring B, ring C, and >N-R in the above formula (1) is preferably a heteroaryl ring or a heteroaryl group. At least one of R's of ring a, ring b, ring c, a ring formed by bonding adjacent groups of R¹ to R¹¹ together with ring a, ring b, or ring c, and >N-R in the above formula (2) is preferably a heteroaryl ring or a heteroaryl group.

In addition,
(B) at least one "-C(-R)=" (here, R represents any one of R¹ to R¹¹ in formula (2)) in ring a, ring b, and ring c in the above formula (2) is preferably replaced with "-N=", or at least one "-C(-R)=C(-R)-" (here, R represents any one of R¹ to R¹¹ in formula (2)) is preferably replaced with "-N(-R)-", "-O-", or "-S-".

Furthermore,
(C) at least one "-C(-R)=C(-R)-" (here, R represents any one of R¹ to R¹¹ in formula (2)) in ring a, ring b, and ring c in the above formula (2) is preferably replaced with "-S-".

In the above (A) to (C), more preferably, Y¹ represents B, and X¹ and X² each represent >N-R.

The polycyclic aromatic compound represented by general formula (1) according to the present invention and a multimer thereof can be used as a material for an organic device such as a material for an organic electroluminescent element, a material for an organic field effect transistor, or a material for an organic thin film solar cell also in a form of a polymer compound obtained by polymerizing, as a monomer, a reactive compound in which the polycyclic aromatic compound or the multimer is substituted by a reactive substituent (the monomer for obtaining the polymer compound has a polymerizable substituent), a crosslinked polymer obtained by further crosslinking the polymer compound (the polymer compound for obtaining the crosslinked polymer has a crosslinkable substituent), a pendant type polymer compound obtained by a reaction between a main chain type polymer and the reactive compound (the reactive compound for obtaining the pendant type polymer compound has a reactive substituent), or a pendant type crosslinked polymer obtained by further crosslinking the pendant type polymer compound (the pendant type polymer compound for obtaining the pendant type crosslinked polymer has a crosslinkable substituent).

The above reactive substituent (which includes the polymerizable substituent, the crosslinkable substituent, and the reactive substituent for obtaining the pendant type polymer, and is also simply referred to as "reactive substituent" hereinafter) is not particularly limited as long as it is a substituent capable of polymerizing the polycyclic aromatic compound or a multimer thereof, a substituent capable of further crosslinking a polymer compound obtained in such a manner, or a substituent capable of causing a pendant reaction with a main chain type polymer, but is preferably any one of substituents having the following structures. The symbol "*" in each of the structural formulas represents a bonding position.

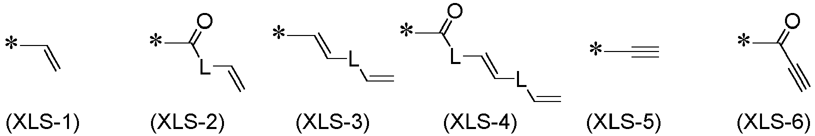

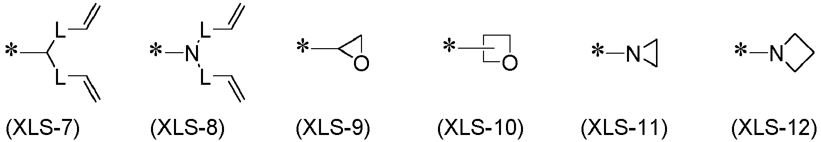

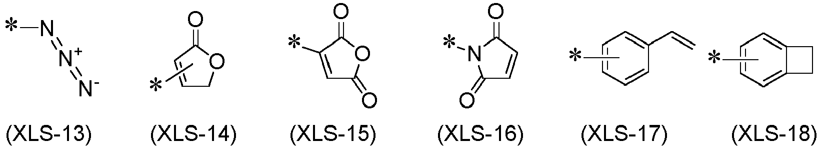

L's each independently represent a single bond, -O-, -S-, >C=O, -O-C(=O)-, an alkylene having 1 to 12 carbon atoms, an oxyalkylene having 1 to 12 carbon atoms, or a polyoxyalkylene having 1 to 12 carbon atoms. Among the above substituents, a group represented by formula (XLS-1), (XLS-2), (XLS-3), (XLS-9), (XLS-10), or (XLS-17) is preferable, and a group represented by formula (XLS-1), (XLS-3), or (XLS-17) is more preferable.

Details of applications of the polymer compound, the crosslinked polymer, and the pendant type polymer compound (hereinafter, also simply referred to as "polymer compound and crosslinked polymer") will be described later.

### 2. Method for manufacturing cycloalkane-fused polycyclic aromatic compound and multimer thereof

In regard to the polycyclic aromatic compound represented by general formula (1) or (2) and a multimer thereof, basically, an intermediate is manufactured by first bonding the ring A (ring a), ring B (ring b), and ring C (ring c) with bonding groups (groups containing X¹ or X²) (first reaction), and then a final product can be manufactured by bonding the ring A (ring a), ring B (ring b), and ring C (ring c) with bonding groups (groups containing Y¹) (second reaction). In the first reaction, for example, in an etherification reaction, a general reaction such as a nucleophilic substitution reaction or an Ullmann reaction can be utilized, and in an amination reaction, a general reaction such as a Buchwald-Hartwig reaction can be utilized. Furthermore, in the second reaction, a Tandem Hetero-Friedel-Crafts reaction (continuous aromatic electrophilic substitution reaction, the same hereinafter) can be utilized. Furthermore, by using a cycloalkane-fused raw material or adding a cycloalkane-fusion step somewhere in these reaction steps, the compound in which a desired position is cycloalkane-fused according to an aspect of the present invention can be manufactured.

The second reaction is a reaction for introducing Y¹ that bonds the ring A (ring a), ring B (ring b), and ring C (ring c) as illustrated in the following scheme (1) or (2), and as an example, a case where Y¹ represents a boron atom, and X¹ and X² represent oxygen atoms is illustrated below. First, a hydrogen atom between X¹ and X² is ortho-metalated with n-butyllithium, secbutyllithium, t-butyllithium, or the like. Subsequently, boron trichloride, boron tribromide, or the like is added thereto to perform lithium-boron metal exchange, and then a Brønsted base such as N,N-diisopropylethylamine is added thereto to induce a Tandem Bora-Friedel-Crafts reaction. Thus, a desired product can be obtained. In the second reaction, a Lewis acid such as aluminum trichloride may be added in order to accelerate the reaction. Incidentally, the definitions of the reference numerals in the structural formulas in the following schemes (1) and (2) and subsequent schemes (3) to (28) are the same as the definitions described above.

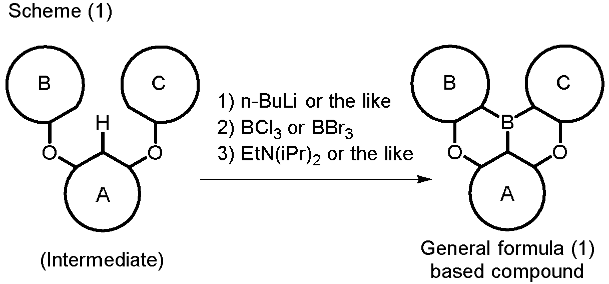

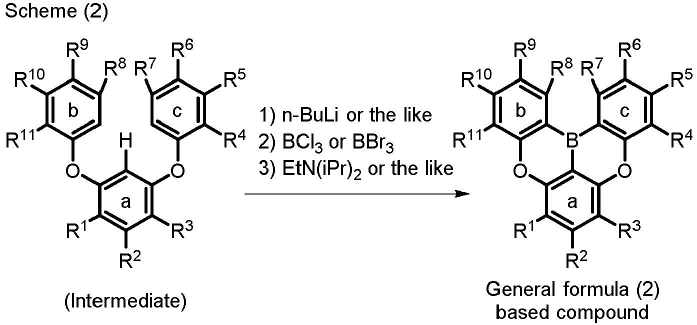

Incidentally, the scheme (1) or (2) mainly illustrates a method for manufacturing a polycyclic aromatic compound represented by general formula (1) or (2). However, a multimer thereof can be manufactured using an intermediate having a plurality of ring A's (ring a's), ring B's (ring b's) and ring C's (ring c's). Specifically, the manufacturing method will be described with the following schemes (3) to (5). In this case, a desired product can be obtained by increasing the amount of a reagent to be used, such as butyllithium, to a double amount or a triple amount.

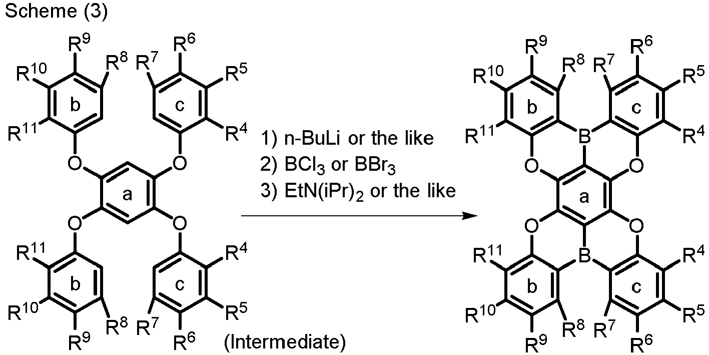

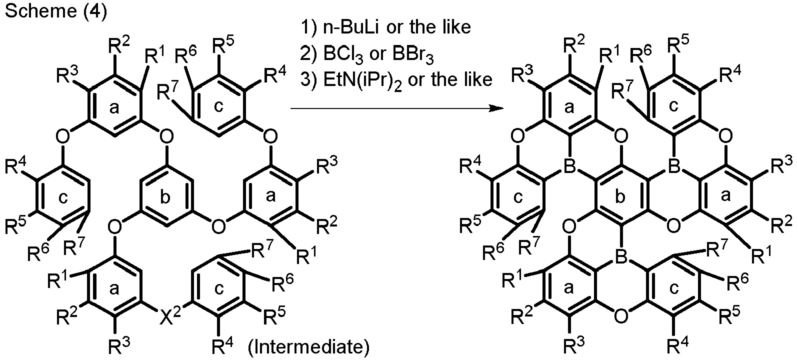

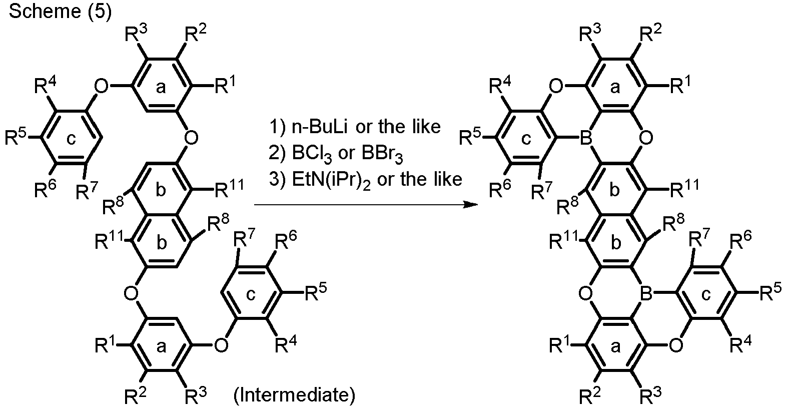

In the above schemes, a lithium atom is introduced into a desired position by ortho-metalation. However, a lithium atom can be introduced into a desired position also by introducing a bromine atom or the like into a position into which it is desired to introduce the lithium atom and performing halogen-metal exchange as in the following schemes (6) and (7).

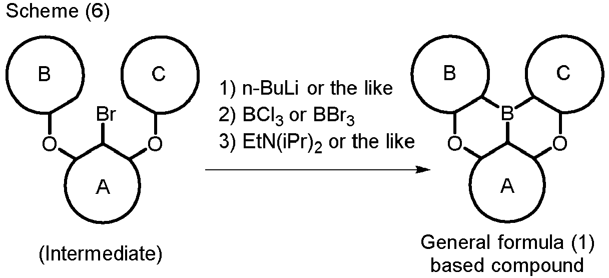

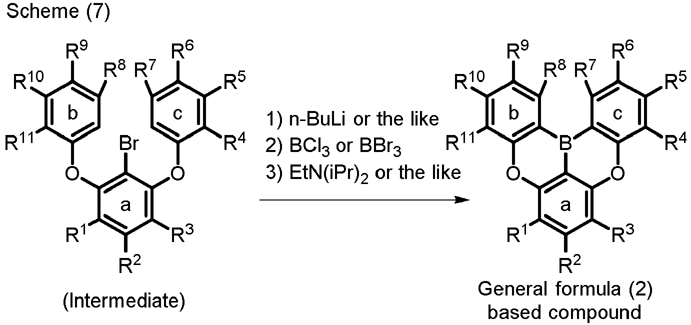

Furthermore, also in regard to the method for manufacturing a multimer described in scheme (3), a lithium atom can be introduced into a desired position also by introducing a halogen atom such as a bromine atom or a chlorine atom into a position into which it is desired to introduce the lithium atom and performing halogen-metal exchange as in the above schemes (6) and (7) (the following schemes (8), (9), and (10)).

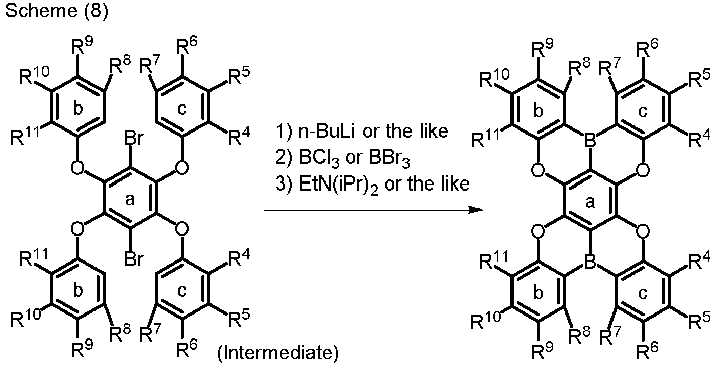

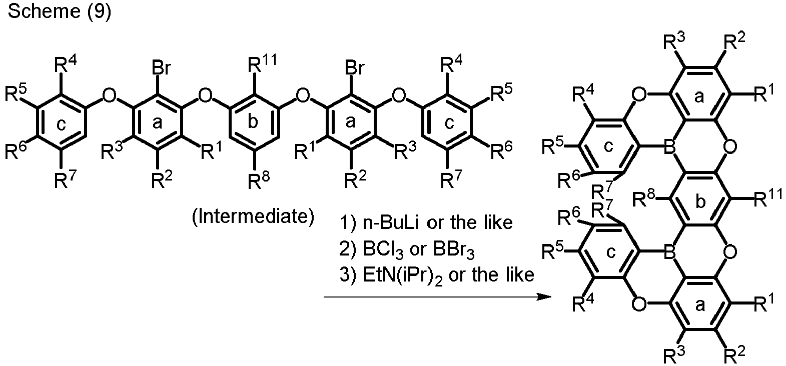

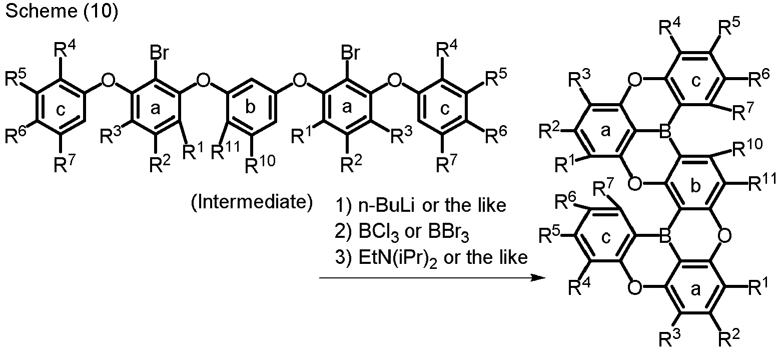

According to this method, a desired product can also be synthesized even in a case where ortho-metalation cannot be achieved due to an influence of a substituent, and therefore the method is useful.

By appropriately selecting the synthesis method described above and appropriately selecting a raw material to be used, a polycyclic aromatic compound in which a desired position is cycloalkane-fused, a substituent is present at a desired position, Y¹ represents a boron atom, and X¹ and X² represent oxygen atoms, and a multimer thereof can be synthesized.

Next, as an example, a case where Y¹ represents a boron atom, and X¹ and X² represent nitrogen atoms is illustrated in the following schemes (11) and (12). As in the case where X¹ and X² represent oxygen atoms, a hydrogen atom between X¹ and X² is first ortho-metalated with n-butyllithium or the like. Subsequently, boron tribromide or the like is added thereto to induce lithium-boron metal exchange, and then a Brønsted base such as N,N-diisopropylethylamine is added thereto to induce a Tandem Bora-Friedel-Crafts reaction. Thus, a desired product can be obtained. Here, a Lewis acid such as aluminum trichloride may also be added in order to accelerate the reaction. Furthermore, by using a cycloalkane-fused raw material or adding a cycloalkane-fusion step somewhere in these reaction steps, a compound in which a desired position is cycloalkane-fused according to the appended claims can be manufactured.

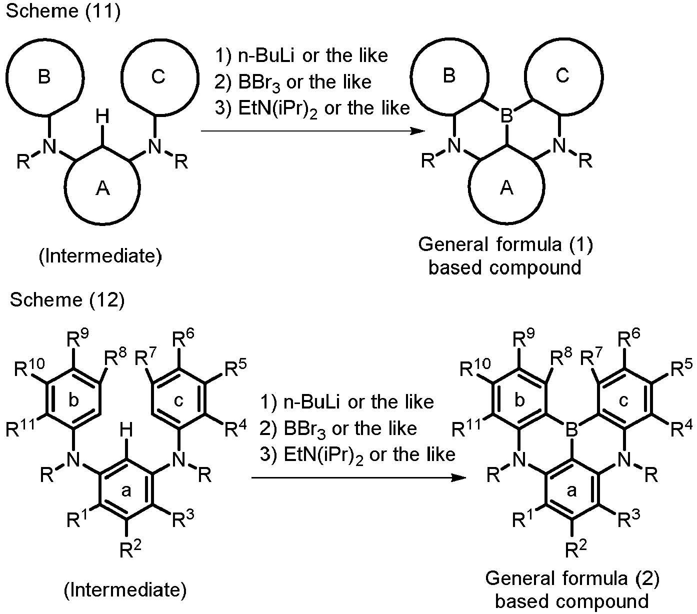

Furthermore, also for a multimer in which Y¹ represents a boron atom, and X¹ and X² represent nitrogen atoms, a lithium atom can be introduced into a desired position also by introducing a halogen atom such as a bromine atom or a chlorine atom into a position into which it is desired to introduce the lithium atom and performing halogen-metal exchange as in the above schemes (6) and (7) (the following schemes (13), (14), and (15)).

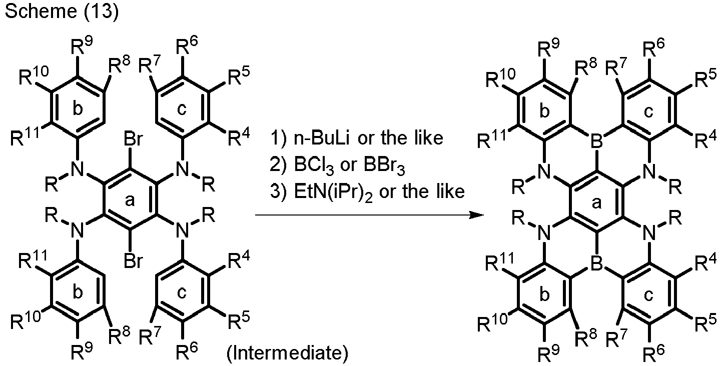

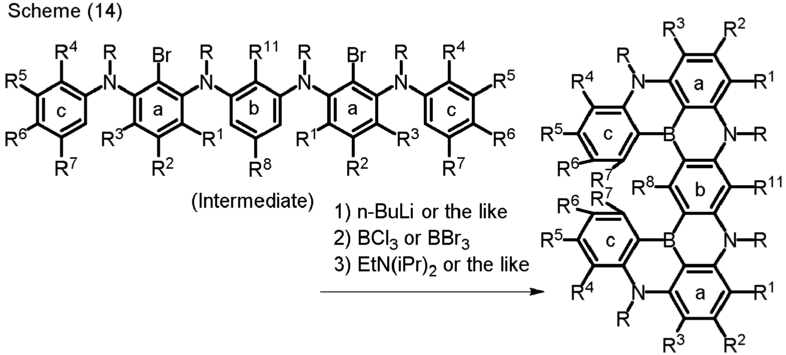

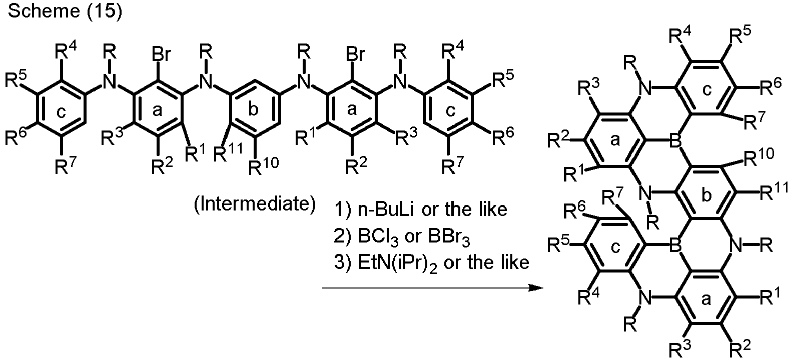

Next, as an example, a case where Y¹ represents phosphorus sulfide, phosphorus oxide, or a phosphorus atom, and X¹ and X² represent oxygen atoms is illustrated in the following schemes (16) to (19). As in the cases described above, a hydrogen atom between X¹ and X² is first ortho-metalated with n-butyllithium or the like. Subsequently, phosphorus trichloride and sulfur are added thereto in this order, and finally a Lewis acid such as aluminum trichloride and a Brønsted base such as N,N-diisopropylethylamine are added thereto to induce a Tandem Phospha-Friedel-Crafts reaction. Thus, a compound in which Y¹ represents phosphorus sulfide can be obtained. Furthermore, by treating the phosphorus sulfide compound thus obtained with m-chloroperbenzoic acid (m-CPBA), a compound in which Y¹ represents phosphorus oxide can be obtained, and by treating the phosphorus sulfide compound with triethylphosphine, a compound in which Y¹ represents a phosphorus atom can be obtained. Furthermore, by using a cycloalkane-fused raw material or adding a cycloalkane-fusion step somewhere in these reaction steps, a compound in which a desired position is cycloalkane-fused according to the appended claims can be manufactured.

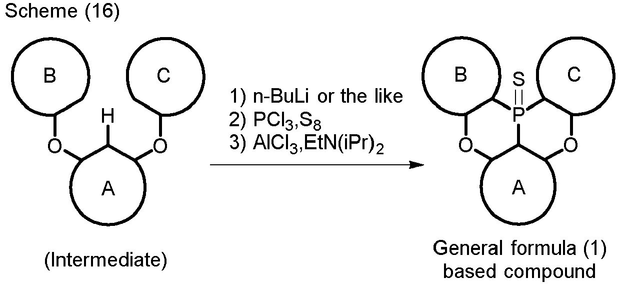

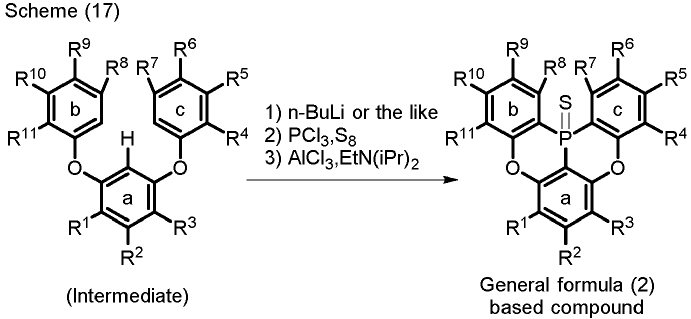

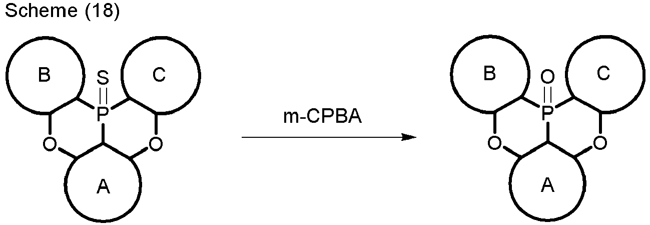

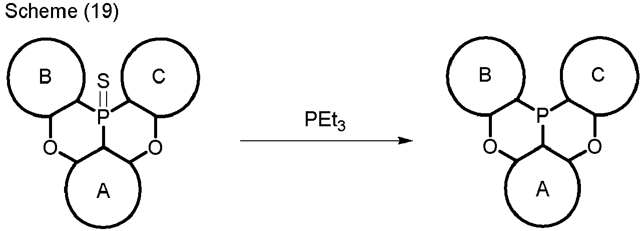

Furthermore, also for a multimer in a case where Y¹ represents phosphorus sulfide, and X¹ and X² represent oxygen atoms, a lithium atom can be introduced into a desired position also by introducing a halogen atom such as a bromine atom or a chlorine atom into a position into which it is desired to introduce the lithium atom and performing halogen-metal exchange as in the above schemes (6) and (7) (the following schemes (20), (21), and (22)). Furthermore, also for the thus formed multimer in which Y¹ represents phosphorus sulfide, and X¹ and X² represent oxygen atoms, as in the above schemes (18) and (19), by treating the multimer with m-chloroperbenzoic acid (m-CPBA), a compound in which Y¹ represents phosphorus oxide can be obtained, and by treating the multimer with triethylphosphine, a compound in which Y¹ represents a phosphorus atom can be obtained.

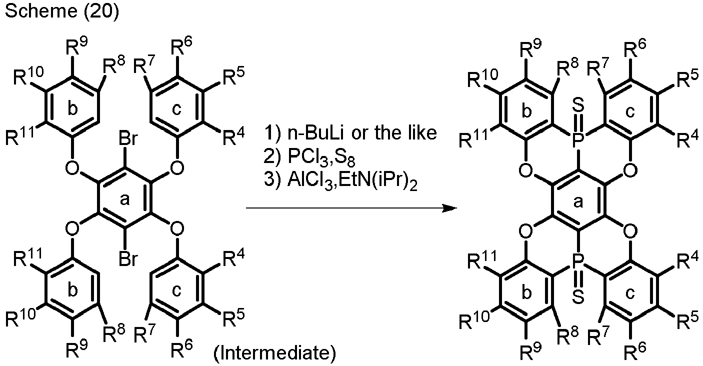

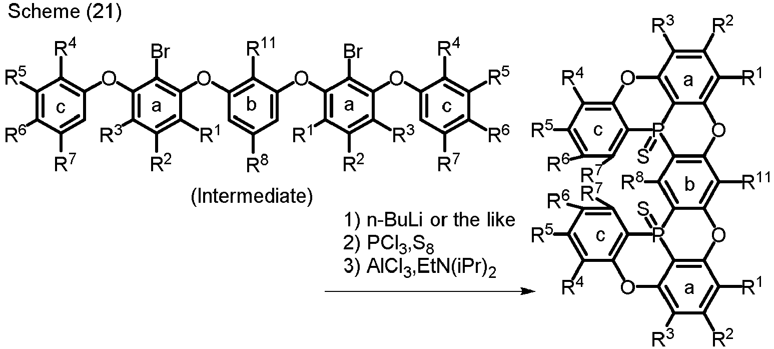

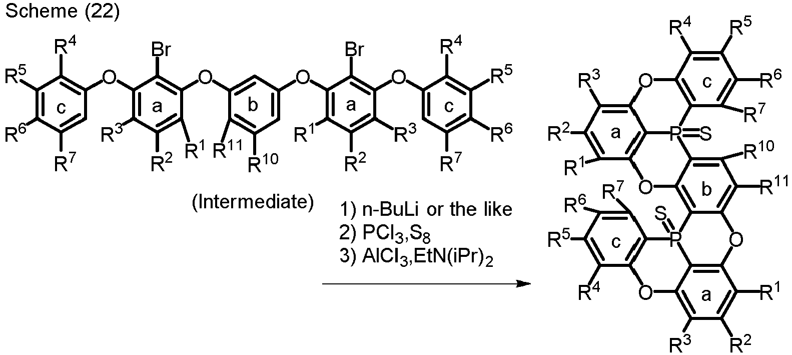

Here, an example in which Y¹ represents B, P, P=O, or P=S, and X¹ and X² each represent O or NR has been described. However, by changing the raw materials appropriately, a compound in which Y¹ represents Al, Ga, As, SiR, or Ge-R, and X¹ and X² each represent S can also be synthesized.

Specific examples of a solvent used in the above reactions include t-butylbenzene and xylene.

Furthermore, in general formula (2), adjacent groups among the substituents R¹ to R¹¹ of the ring a, ring b, and ring c may be bonded to each other to form an aryl ring or a heteroaryl ring together with the ring a, ring b, or ring c, and at least one hydrogen atom in the ring thus formed may be substituted by an aryl or a heteroaryl. Therefore, in a polycyclic aromatic compound represented by general formula (2), a ring structure constituting the compound changes as represented by formulas (2-1) and (2-2) of the following schemes (23) and (24) according to a mutual bonding form of substituents in the ring a, ring b, and ring c. These compounds can be synthesized by applying synthesis methods illustrated in the above schemes (1) to (19) to intermediates illustrated in the following schemes (23) and (24). Furthermore, by using a cycloalkane-fused raw material or adding a cycloalkane-fusion step somewhere in these reaction steps, a compound in which a desired position is cycloalkane-fused according to the appended claims can be manufactured.

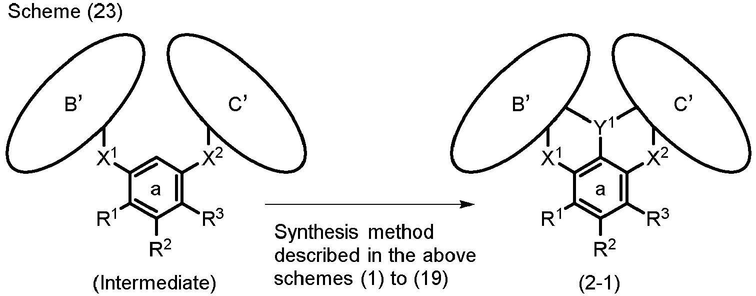

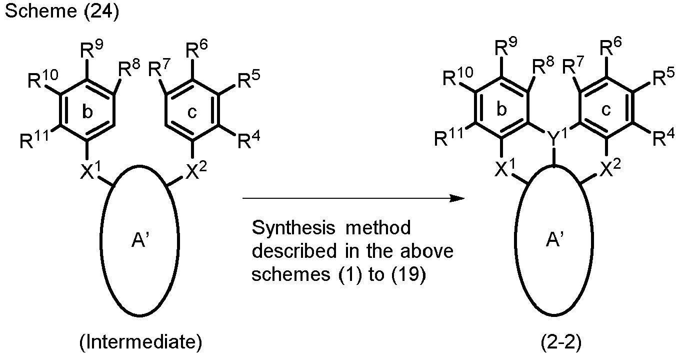

Ring A', ring B' and ring C' in the above formulas (2-1) and (2-2) each represent an aryl ring or a heteroaryl ring formed by bonding adjacent groups among the substituents R¹ to R¹¹ together with the ring a, ring b, and ring c, respectively (may also be a fused ring obtained by fusing another ring structure to the ring a, ring b, or ring c). Incidentally, although not indicated in the formula, there is also a compound in which all of the ring a, ring b, and ring c have been changed to the ring A', ring B' and ring C'.

Furthermore, the provision that "at least one of R in the >N-R and R in the >C(-R)₂ is bonded to at least one of the ring a, ring b, and ring c via -O-, -S-, -C(-R)₂-, or a single bond" in general formulas (2) can be expressed as a compound having a ring structure represented by formula (2-3-1) of the following scheme (25), in which X¹ or X² is incorporated into the fused ring B' or fused ring C', or a compound having a ring structure represented by formula (2-3-2) or (2-3-3), in which X¹ or X² is incorporated into the fused ring A'. These compounds can be synthesized by applying the synthesis methods illustrated in the schemes (1) to (19) to the intermediate represented by the following scheme (25). Furthermore, by using a cycloalkane-fused raw material or adding a cycloalkane-fusion step somewhere in these reaction steps, a compound in which a desired position is cycloalkane-fused according to the appended claims can be manufactured.

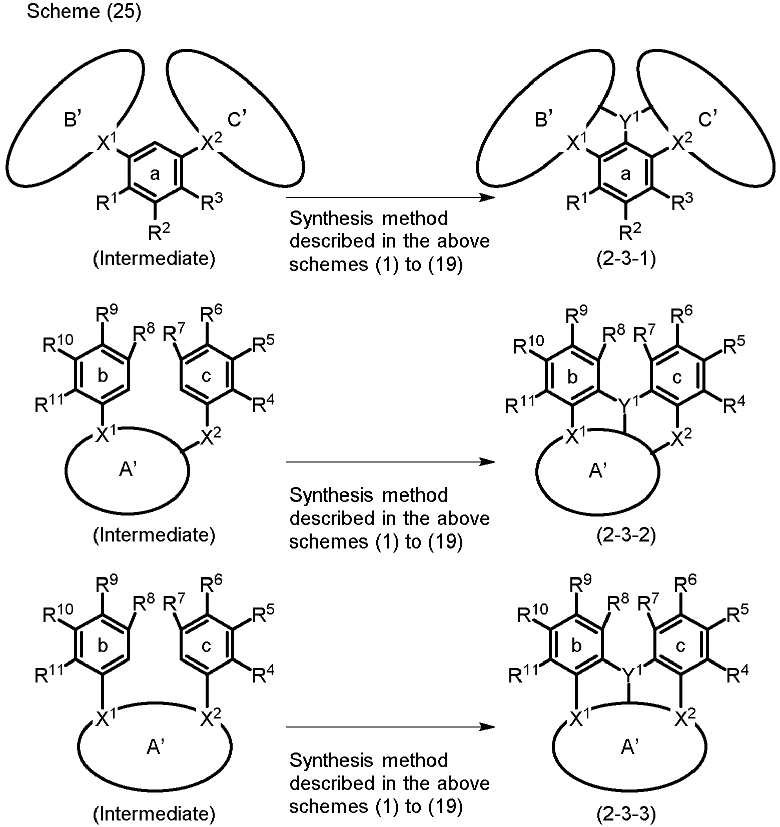

Furthermore, the synthesis methods of the above schemes (1) to (17) and (20) to (25) illustrate an example of performing a Tandem Hetero-Friedel-Crafts reaction by ortho-metalating a hydrogen atom (or a halogen atom) between X¹ and X² with butyllithium or the like before boron trichloride, boron tribromide, or the like is added. However, the reaction can also be advanced by adding boron trichloride, boron tribromide, or the like without performing ortho-metalation using buthyllithium or the like.

Furthermore, in a case where Y¹ represents a phosphorus-based group, as illustrated in the following scheme (26) or (27), a desired product can be obtained by ortho-metalating a hydrogen atom between X¹ and X² (O in the following formula) with n-butyllithium, secbutyllithium, t-butyllithium, or the like, subsequently adding bisdiethylaminochlorophosphine thereto to perform lithium-phosphorus metal exchange, and then adding a Lewis acid such as aluminum trichloride thereto to induce a Tandem Phospha-Friedel-Crafts reaction. This reaction method is also described in WO 2010/104047 A (for example, page 27). Furthermore, by using a cycloalkane-fused raw material or adding a cycloalkane-fusion step somewhere in these reaction steps, a compound in which a desired position is cycloalkane-fused according to the appended claims can be manufactured.

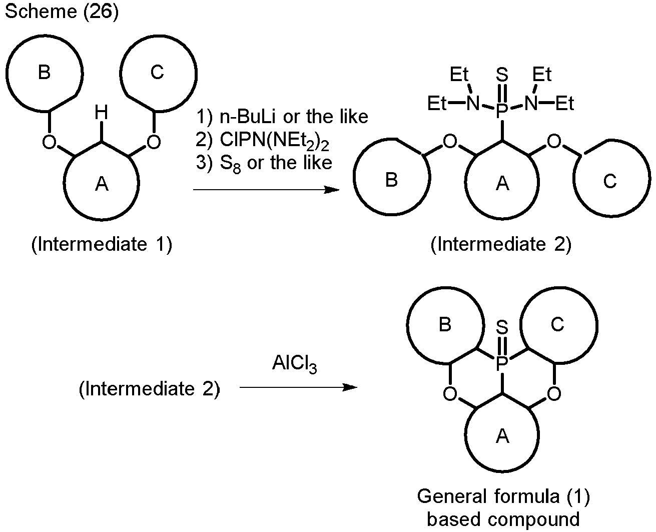

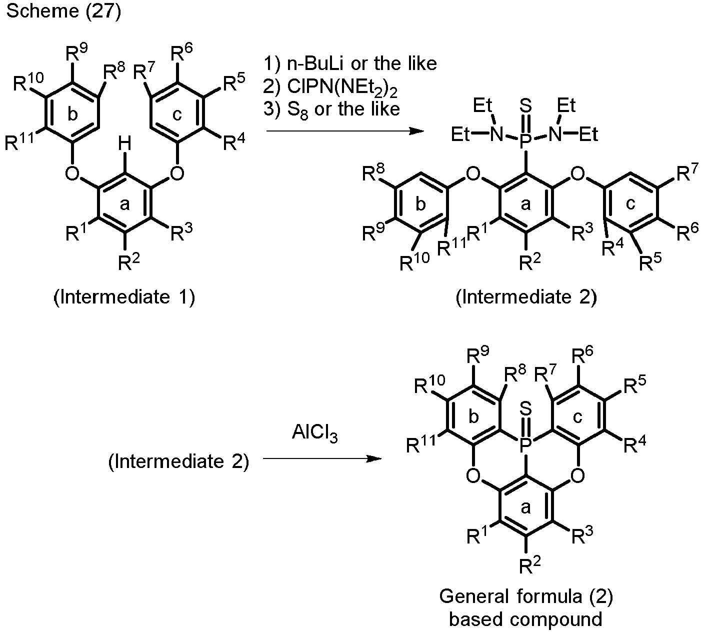

Incidentally, also in the above scheme (26) or (27), a multimer compound can be synthesized using an ortho-metalation reagent such as butyllithium in a molar amount twice or three time the molar amount of an intermediate. Furthermore, a metal atom can be introduced into a desired position by introducing a halogen atom such as a bromine atom or a chlorine atom in advance into a position into which it is desired to introduce a metal atom such as a lithium atom, and performing halogen-metal exchange.

In addition, regarding the polycyclic aromatic compound represented by general formula (2-A), as illustrated in the following scheme (28), a cycloalkane-fused intermediate is synthesized and cyclized to synthesize a polycyclic aromatic compound in which a desired position is fused with a cycloalkane(s). In scheme (28), X represents a halogen atom or a hydrogen atom, and the definitions of the other reference numerals are the same as the definitions of the reference numerals in general formula (2).

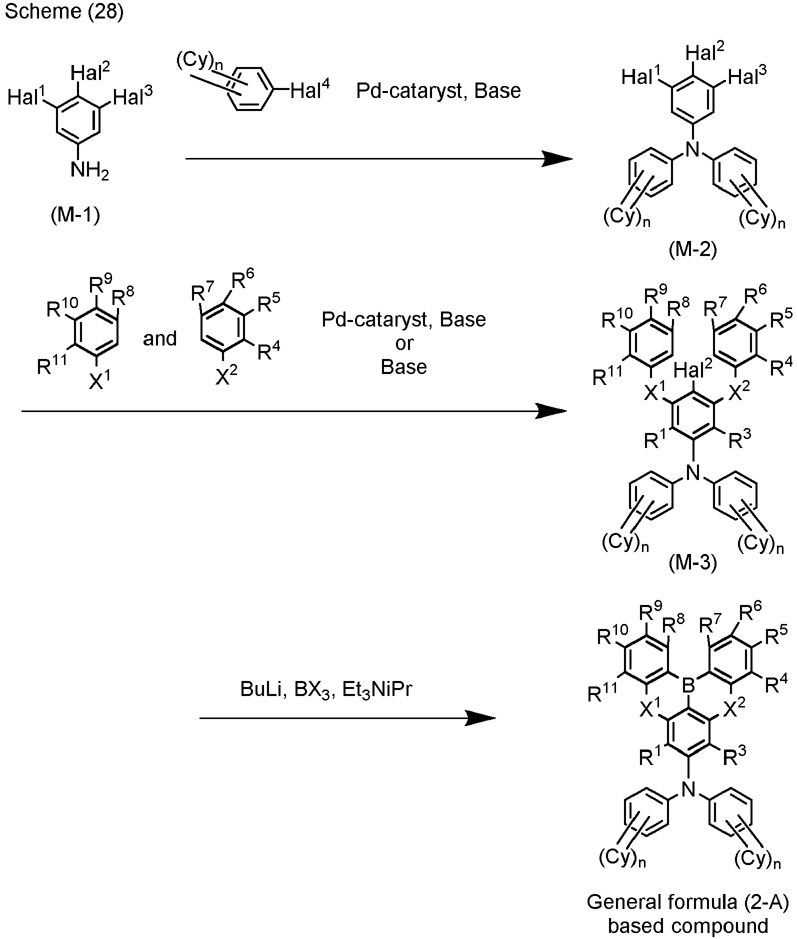

An intermediate before cyclization in scheme (28) can also be synthesized by a method illustrated in scheme (1) or the like. That is, an intermediate having a desired substituent can be synthesized by appropriately combining a Buchwald-Hartwig reaction, a Suzuki coupling reaction, an etherification reaction using a nucleophilic substitution reaction or an Ullmann reaction, and the like. In these reactions, a commercially available product can be used as a raw material serving as a cycloalkane-fused precursor.

A compound of general formula (2-A) having a cycloalkane-fused diphenylamino group can also be synthesized, for example, by the following method. That is, a cycloalkane-fused diphenylamino group is introduced by an amination reaction such as a Buchwald-Hartwig reaction between commercially available cycloalkane-fused bromobenzene and trihalogenated aniline. Thereafter, the resulting product is induced to an intermediate (M-3) by an amination reaction such as a Buchwald-Hartwig reaction when X¹ and X² each represent N-R, or by an etherification using phenol when X¹ and X² each represent O. Thereafter, the resulting product is transmetalated by an action of a metalation reagent such as butyllithium, then caused to react with a boron halide such as boron tribromide, and then caused to react with a Brønsted base such as diethyl isopropylamine to cause a Tandem Bora-Friedel-Crafts reaction. Thus, the compound of formula (2-A) can be synthesized. These reactions can also be applied to other cycloalkane-fused compounds.

Note that examples of an ortho-metalation reagent used in the above schemes (1) to (28) include an alkyllithium such as methyllithium, n-butyllithium, sec-butyllithium, or t-butyllithium; an organic alkali compound such as lithium diisopropylamide, lithium tetramethylpiperidide, lithium hexamethyldisilazide, or potassium hexamethyldisilazide; and a dispersed alkali metal such as organic solvent-dispersed Na or the like.

Incidentally, examples of a metal exchanging reagent for metal-Y¹ used in the above schemes (1) to (28) include a halide of Y¹ such as trifluoride of Y¹, trichloride of Y¹, tribromide of Y¹, or triiodide of Y¹; an aminated halide of Y¹ such as CIPN(NEt₂)₂; an alkoxylated product of Y¹; and an aryloxylated product of Y¹.

Incidentally, examples of the Brønsted base used for the above schemes (1) to (28) include N,N-diisopropylethylamine, triethylamine, 2,2,6,6-tetramethylpiperidine, 1,2,2,6,6-pentamethylpiperidine, N,N-dimethylaniline, N,N-dimethyltoluidine, 2,6-lutidine, sodium tetraphenylborate, potassium tetraphenylborate, triphenylborane, tetraphenylsilane, Ar₄BNa, Ar₄BK, Ar₃B, and Ar₄Si (Ar represents an aryl such as phenyl).

Examples of a Lewis acid used for the above schemes (1) to (28) include AlCl₃, AlBr₃, AlF₃, BF₃·OEt₂, BCl₃, BBr₃, GaCl₃, GaBr₃, InCl₃, InBr₃, In(OTf)₃, SnCl₄, SnBr₄, AgOTf, ScCl₃, Sc(OTf)₃, ZnCl₂, ZnBr₂, Zn(OTf)₂, MgCl₂, MgBr₂, Mg(OTf)₂, LiOTf, NaOTf, KOTf, Me₃SiOTf, Cu(OTf)₂, CuCl₂, YCl₃, Y(OTf)₃, TiCl₄, TiBr₄, ZrCl₄, ZrBr₄, FeCl₃, FeBr₃, CoCl₃, and CoBr₃.

In the above schemes (1) to (28), a Brønsted base or a Lewis acid may be used in order to accelerate the Tandem Hetero Friedel-Crafts reaction. However, in a case where a halide of Y¹ such as trifluoride of Y¹, trichloride of Y¹, tribromide of Y¹, or triiodide of Y¹ is used, an acid such as hydrogen fluoride, hydrogen chloride, hydrogen bromide, or hydrogen iodide is generated along with progress of an aromatic electrophilic substitution reaction. Therefore, it is effective to use a Brønsted base that captures an acid. Meanwhile, in a case where an aminated halide of Y¹ or an alkoxylation product of Y¹ is used, an amine or an alcohol is generated along with progress of the aromatic electrophilic substitution reaction. Therefore, in many cases, it is not necessary to use a Brønsted base. However, leaving ability of an amino group or an alkoxy group is low, and therefore it is effective to use a Lewis acid that promotes leaving of these groups.

Furthermore, the polycyclic aromatic compound or the multimer thereof according to an aspect of the present invention also includes a compound in which at least some of hydrogen atoms are substituted by deuterium atoms, cyanos or substituted by halogen atoms such as fluorine atoms or chlorine atoms. However, these compounds can be synthesized in a similar manner to the above using a raw material in which a desired position is deuterated, cyanized, fluorinated, or chlorinated.

### 3. Organic device

The polycyclic aromatic compound fused with cycloalkanes as specified in the appended claims can be used as a material for an organic device. Examples of the organic device include an organic electroluminescent element, an organic field effect transistor, and an organic thin film solar cell.

### 3-1. Organic electroluminescent element

Hereinafter, an organic EL element according to the present embodiment will be described in detail based on the drawings. FIG. 1 is a schematic cross-sectional view illustrating the organic EL element according to the present embodiment.

### structure of organic electroluminescent element>

An organic EL element 100 illustrated in FIG. 1 includes a substrate 101, a positive electrode 102 provided on the substrate 101, a hole injection layer 103 provided on the positive electrode 102, a hole transport layer 104 provided on the hole injection layer 103, a light emitting layer 105 provided on the hole transport layer 104, an electron transport layer 106 provided on the light emitting layer 105, an electron injection layer 107 provided on the electron transport layer 106, and a negative electrode 108 provided on the electron injection layer 107.

Incidentally, the organic EL element 100 may be constituted, by reversing the manufacturing order, to include, for example, the substrate 101, the negative electrode 108 provided on the substrate 101, the electron injection layer 107 provided on the negative electrode 108, the electron transport layer 106 provided on the electron injection layer 107, the light emitting layer 105 provided on the electron transport layer 106, the hole transport layer 104 provided on the light emitting layer 105, the hole injection layer 103 provided on the hole transport layer 104, and the positive electrode 102 provided on the hole injection layer 103.

Not all of the above layers are essential. The configuration includes the positive electrode 102, the light emitting layer 105, and the negative electrode 108 as a minimum constituent unit, while the hole injection layer 103, the hole transport layer 104, the electron transport layer 106, and the electron injection layer 107 are optionally provided. Furthermore, each of the above layers may be formed of a single layer or a plurality of layers.

A form of layers constituting the organic EL element may be, in addition to the above structure form of "substrate/positive electrode/hole injection layer/hole transport layer/light emitting layer/electron transport layer/electron injection layer/negative electrode", a structure form of "substrate/positive electrode/hole transport layer/light emitting layer/electron transport layer/electron injection layer/negative electrode", "substrate/positive electrode/hole injection layer/light emitting layer/electron transport layer/electron injection layer/negative electrode", "substrate/positive electrode/hole injection layer/hole transport layer/light emitting layer/electron injection layer/negative electrode", "substrate/positive electrode/hole injection layer/hole transport layer/light emitting layer/electron transport layer/negative electrode", "substrate/positive electrode/light emitting layer/electron transport layer/electron injection layer/negative electrode", "substrate/positive electrode/hole transport layer/light emitting layer/electron injection layer/negative electrode", "substrate/positive electrode/hole transport layer/light emitting layer/electron transport layer/negative electrode", "substrate/positive electrode/hole injection layer/light emitting layer/electron injection layer/negative electrode", "substrate/positive electrode/hole injection layer/light emitting layer/electron transport layer/negative electrode", "substrate/positive electrode/light emitting layer/electron transport layer/negative electrode", or "substrate/positive electrode/light emitting layer/electron injection layer/negative electrode".

### <Substrate in organic electroluminescent element>

The substrate 101 serves as a support of the organic EL element 100, and usually, quartz, glass, metals, plastics, and the like are used therefor. The substrate 101 is formed into a plate shape, a film shape, or a sheet shape according to a purpose, and for example, a glass plate, a metal plate, a metal foil, a plastic film, and a plastic sheet are used. Among these examples, a glass plate and a plate made of a transparent synthetic resin such as polyester, polymethacrylate, polycarbonate, or polysulfone are preferable. For a glass substrate, soda lime glass, alkali-free glass, and the like are used. The thickness is only required to be a thickness sufficient for maintaining mechanical strength. Therefore, the thickness is only required to be 0.2 mm or more, for example. The upper limit value of the thickness is, for example, 2 mm or less, and preferably 1 mm or less. Regarding a material of glass, glass having fewer ions eluted from the glass is desirable, and therefore alkali-free glass is preferable. However, soda lime glass which has been subjected to barrier coating with SiO₂ or the like is also commercially available, and therefore this soda lime glass can be used. Furthermore, the substrate 101 may be provided with a gas barrier film such as a dense silicon oxide film on at least one surface in order to increase a gas barrier property. Particularly in a case of using a plate, a film, or a sheet made of a synthetic resin having a low gas barrier property as the substrate 101, a gas barrier film is preferably provided.

### <Positive electrode in organic electroluminescent element>

The positive electrode 102 plays a role of injecting a hole into the light emitting layer 105. Incidentally, in a case where at least one layer of the hole injection layer 103 and the hole transport layer 104 are/is provided between the positive electrode 102 and the light emitting layer 105, a hole is injected into the light emitting layer 105 through these layers.

Examples of a material to form the positive electrode 102 include an inorganic compound and an organic compound. Examples of the inorganic compound include a metal (aluminum, gold, silver, nickel, palladium, chromium, and the like), a metal oxide (indium oxide, tin oxide, indium-tin oxide (ITO), indium-zinc oxide (IZO), and the like), a metal halide (copper iodide and the like), copper sulfide, carbon black, ITO glass, and Nesa glass. Examples of the organic compound include an electrically conductive polymer such as polythiophene such as poly(3-methylthiophene), polypyrrole, or polyaniline. In addition to these compounds, a material can be appropriately selected for use from materials used as a positive electrode of an organic EL element.

A resistance of a transparent electrode is not limited as long as a sufficient current can be supplied to light emission of a luminescent element. However, low resistance is desirable from a viewpoint of consumption power of the luminescent element. For example, an ITO substrate having a resistance of 300 Ω/□ or less functions as an element electrode. However, a substrate having a resistance of about 10 Ω/□ can be also supplied at present, and therefore it is particularly desirable to use a low resistance product having a resistance of, for example, 100 to 5 Ω/□, preferably 50 to 5 Ω/□. The thickness of an ITO can be arbitrarily selected according to a resistance value, but an ITO having a thickness of 50 to 300 nm is often used.

### <Hole injection layer and hole transport layer in organic electroluminescent element>

The hole injection layer 103 plays a role of efficiently injecting a hole that migrates from the positive electrode 102 into the light emitting layer 105 or the hole transport layer 104. The hole transport layer 104 plays a role of efficiently transporting a hole injected from the positive electrode 102 or a hole injected from the positive electrode 102 through the hole injection layer 103 to the light emitting layer 105. The hole injection layer 103 and the hole transport layer 104 are each formed by laminating and mixing one or more kinds of hole injection/transport materials, or by a mixture of a hole injection/transport material and a polymer binder. Furthermore, a layer may be formed by adding an inorganic salt such as iron(III) chloride to the hole injection/transport materials.

A hole injecting/transporting substance needs to efficiently inject/transport a hole from a positive electrode between electrodes to which an electric field is applied, and preferably has high hole injection efficiency and transports an injected hole efficiently. For this purpose, a substance which has low ionization potential, large hole mobility, and excellent stability, and in which impurities that serve as traps are not easily generated at the time of manufacturing and at the time of use, is preferable.

As a material to form the hole injection layer 103 and the hole transport layer 104, any compound can be selected for use among compounds that have been conventionally used as charge transporting materials for holes, p-type semiconductors, and known compounds used in a hole injection layer and a hole transport layer of an organic EL element. Specific examples thereof include a heterocyclic compound including a carbazole derivative (N-phenylcarbazole, polyvinylcarbazole, and the like), a biscarbazole derivative such as bis(N-arylcarbazole) or bis(N-alkylcarbazole), a triarylamine derivative (a polymer having an aromatic tertiary amino in a main chain or a side chain, 1,1-bis(4-di-p-tolylaminophenyl)cyclohexane, N,N'-diphenyl-N,N'-di(3-methylphenyl)-4,4'-diaminobiphenyl, N,N'-diphenyl-N,N'-dinaphthyl-4,4'-diaminobiphenyl, N,N'-diphenyl-N,N'-di(3-methylphenyl)-4,4'-diphenyl-1,1'-diamine, N,N'-dinaphthyl-N,N'-diphenyl-4,4'-dphenyl-1,1'-diamine, N⁴,N⁴'-diphenyl-N⁴,N⁴'-bis(9-phenyl-9H-carbazol-3-yl)-[1,1'-biphenyl]-4,4'-diamine, N⁴,N⁴,N⁴',N⁴'-tetra[1,1'-biphenyl]-4-yl)-[1,1'-biphenyl]-4,4'-diamine, a triphenylamine derivative such as 4,4',4"-tris (3-methylphenyl(phenyl)amino)triphenylamine, a starburst amine derivative, and the like), a stilbene derivative, a phthalocyanine derivative (non-metal, copper phthalocyanine, and the like), a pyrazoline derivative, a hydrazone-based compound, a benzofuran derivative, a thiophene derivative, an oxadiazole derivative, a quinoxaline derivative (for example, 1,4,5,8,9,12-hexaazatriphenylene-2,3,6,7,10,11-hexacarbonitrile, and the like), and a porphyrin derivative, and a polysilane. Among the polymer-based materials, a polycarbonate, a styrene derivative, a polyvinylcarbazole, a polysilane, and the like having the above monomers in side chains are preferable. However, there is no particular limitation as long as a compound can form a thin film required for manufacturing a luminescent element, can inject a hole from a positive electrode, and can further transport a hole.

Furthermore, it is also known that electroconductivity of an organic semiconductor is strongly affected by doping into the organic semiconductor. Such an organic semiconductor matrix substance is formed of a compound having a good electron-donating property, or a compound having a good electron-accepting property. For doping with an electron-donating substance, a strong electron acceptor such as tetracyanoquinonedimethane (TCNQ) or 2,3,5,6-tetrafluorotetracyano-1,4-benzoquinonedimethane (F4TCNQ) is known (see, for example, "M. Pfeiffer, A. Beyer, T. Fritz, K. Leo, Appl. Phys. Lett., 73(22), 3202-3204 (1998)" and "J. Blochwitz, M. Pheiffer, T. Fritz, K. Leo, Appl. Phys. Lett., 73(6), 729-731 (1998)"). These compounds generate a socalled hole by an electron transfer process in an electron-donating type base substance (hole transporting substance). Electroconductivity of the base substance depends on the number and mobility of the holes fairly significantly. Known examples of a matrix substance having a hole transporting characteristic include a benzidine derivative (TPD and the like), a starburst amine derivative (TDATA and the like), and a specific metal phthalocyanine (particularly, zinc phthalocyanine (ZnPc) and the like) (JP 2005-167175 A).

Each of the material for the hole injection layer and the material for the hole transport layer can be used as a material for the hole layer also in a form of a polymer compound obtained by polymerizing, as a monomer, a reactive compound in which each of the material for the hole injection layer and the material for the hole transport layer is substituted by a reactive substituent, a crosslinked polymer thereof, a pendant type polymer compound obtained by a reaction between a main chain type polymer and the reactive compound, or a pendant type crosslinked polymer thereof. As the reactive substituent in this case, the description for the polycyclic aromatic compound represented by formula (1) can be cited.

Details of applications of the polymer compound and the crosslinked polymer will be described later.

### <Light emitting layer in organic electroluminescent element>

The light emitting layer 105 emits light by recombining a hole injected from the positive electrode 102 and an electron injected from the negative electrode 108 between electrodes to which an electric field is applied. A material to form the light emitting layer 105 is only required to be a compound which is excited by recombination between a hole and an electron and emits light (luminescent compound), and is preferably a compound which can form a stable thin film shape, and exhibits strong light emission (fluorescence) efficiency in a solid state. In the present invention, as the material for the light emitting layer, a host material and, for example, a polycyclic aromatic compound represented by the above general formula (1) as a dopant material can be used.

The light emitting layer may be formed of a single layer or a plurality of layers, and each layer is formed of a material for a light emitting layer (a host material and a dopant material). Each of the host material and the dopant material may be formed of a single kind, or a combination of a plurality of kinds. The dopant material may be included in the host material wholly or partially. Regarding a doping method, doping can be performed by a co-deposition method with a host material, or alternatively, a dopant material may be mixed in advance with a host material, and then vapor deposition may be carried out simultaneously.

The amount of use of the host material depends on the kind of the host material, and may be determined according to a characteristic of the host material. The reference of the amount of use of the host material is preferably from 50 to 99.999% by weight, more preferably from 80 to 99.95% by weight, and still more preferably from 90 to 99.9% by weight with respect to the total amount of a material for a light emitting layer.

The amount of use of the dopant material depends on the kind of the dopant material, and may be determined according to a characteristic of the dopant material. The reference of the amount of use of the dopant is preferably from 0.001 to 50% by weight, more preferably from 0.05 to 20% by weight, and still more preferably from 0.1 to 10% by weight with respect to the total amount of a material for a light emitting layer. The amount of use within the above range is preferable, for example, from a viewpoint of being able to prevent a concentration quenching phenomenon.

Examples of the host material include a fused ring derivative of anthracene, pyrene, dibenzochrysene, fluorine, or the like, a bisstyryl derivative such as a bisstyrylanthracene derivative, a distyrylbenzene derivative, or the like, a tetraphenylbutadiene derivative, a cyclopentadiene derivative, or the like, conventionally known as a luminous body. In particular, anthracene compounds, fluorene compounds or dibenzochrysene compounds are preferable.

### <Anthracene-based compound>

The anthracene-based compound as a host is, for example, a compound represented by the following general formula (3).

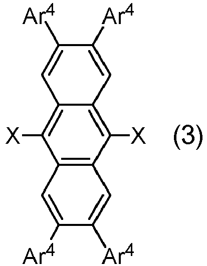

In formula (3),
X's and Ar⁴'s each independently represent a hydrogen atom, an optionally substituted aryl, an optionally substituted heteroaryl, an optionally substituted diarylamino, an optionally substituted diheteroarylamino, an optionally substituted arylheteroarylamino, an optionally substituted alkyl, an optionally substituted cycloalkyl, an optionally substituted alkenyl, an optionally substituted alkoxy, an optionally substituted aryloxy, an optionally substituted arylthio, or an optionally substituted silyl, and not all X's and Ar⁴'s represent hydrogen atoms simultaneously, and
at least one hydrogen atom in the compound represented by formula (3) may be substituted by a halogen atom, cyano, a deuterium atom, or an optionally substituted heteroaryl.

Further, a multimer (preferably a dimer) may be formed by using the structure represented by the formula (3) as a unit structure. In this case, for example, a form in which the unit structures represented by the formula (3) are bonded via X can be mentioned. The X includes a single bond, an arylene (phenylene, biphenylene, naphthylene, and the like) and a heteroarylene (a divalent group derived from pyridine ring, dibenzofuran ring, dibenzothiophene ring, carbazole ring, benzocarbazole ring, phenyl-substituted carbazole ring, or the like) and the like.

The above aryl, heteroaryl, diarylamino, diheteroarylamino, arylheteroarylamino, alkyl, cycloalkyl, alkenyl, alkoxy, aryloxy, arylthio, and silyl are described in detail in the following preferable embodiment. Examples of a substituent on these groups include an aryl, a heteroaryl, a diarylamino, a diheteroarylamino, an arylheteroarylamino, an alkyl, a cycloalkyl, an alkenyl, an alkoxy, an aryloxy, an arylthio, and a silyl, and these are also described in detail in the following preferable embodiment.

A preferable embodiment of the anthracene-based compound will be described below. The definitions of symbols in the following structures are the same as the above definitions.

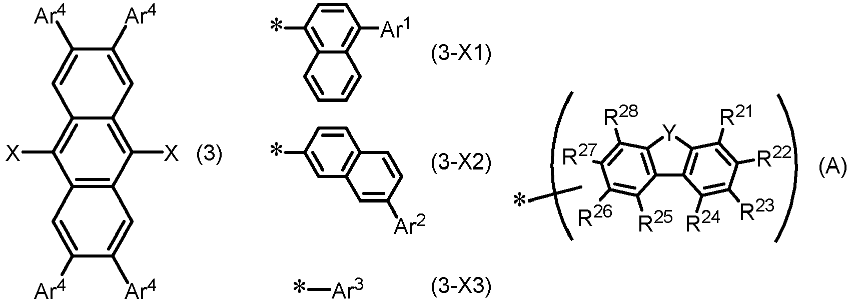

In general formula (3), X's each independently represent a group represented by the above formula (3-X1), (3-X2), or (3-X3), and the group represented by the formula (3-X1), (3-X2), or (3-X3) is bonded to the anthracene ring of formula (3) at the symbol *. Preferably, the two X's do not simultaneously represent a group represented by formula (3-X3). More preferably, the two X's do not simultaneously represent a group represented by formula (3-X2).

Further, a multimer (preferably a dimer) may be formed by using the structure represented by the formula (3) as a unit structure. In this case, for example, a form in which the unit structures represented by the formula (3) are bonded via X can be mentioned. The X includes a single bond, an arylene (phenylene, biphenylene, naphthylene, and the like) and a heteroarylene (a divalent group derived from pyridine ring, dibenzofuran ring, dibenzothiophene ring, carbazole ring, benzocarbazole ring, phenyl-substituted carbazole ring, or the like) and the like.

The naphthylene moiety in formula (3-X1) and formula (3-X2) may be fused with one benzene ring. A structure fused in this way is as follows.

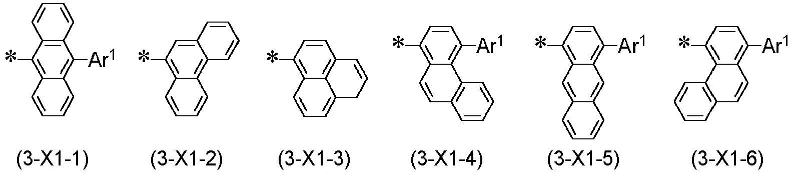

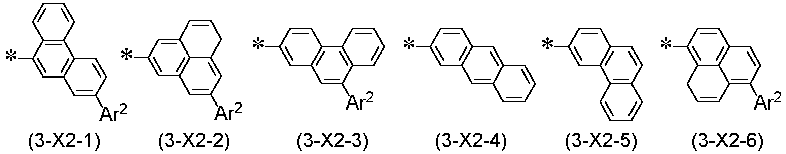

Ar¹ and Ar² each independently represent a hydrogen atom, phenyl, biphenylyl, terphenylyl, quaterphenylyl, naphthyl, phenanthryl, fluorenyl, benzofluorenyl, chrysenyl, triphenylenyl, pyrenylyl, or a group represented by the above formula (A) (including a carbazolyl group, a benzocarbazolyl group, and a phenyl-substituted carbazolyl group). Incidentally, in a case where Ar¹ or Ar² is a group represented by formula (A), the group represented by formula (A) is bonded to a naphthalene ring in formula (3-X1) or (3-X2) at the symbol *.

Ar³ represents phenyl, biphenylyl, terphenylyl, quaterphenylyl, naphthyl, phenanthryl, fluorenyl, benzofluorenyl, chrysenyl, triphenylenyl, pyrenylyl, or a group represented by the above formula (A) (including a carbazolyl group, a benzocarbazolyl group, and a phenyl-substituted carbazolyl group). Incidentally, in a case where Ar³ is a group represented by formula (A), the group represented by formula (A) is bonded to a single bond represented by the straight line in formula (3-X3) at the symbol *. That is, the anthracene ring of formula (3) and the group represented by formula (A) are directly bonded to each other.

Furthermore, Ar³ may have a substituent, and at least one hydrogen atom in Ar³ may be further substituted by an alkyl having 1 to 4 carbon atoms, a cycloalkyl having 5 to 10 carbon atoms, phenyl, biphenylyl, terphenylyl, naphthyl, phenanthryl, fluorenyl, chrysenyl, triphenylenyl, pyrenylyl, or a group represented by the above formula (A) (including a carbazolyl group and a phenyl-substituted carbazolyl group). Incidentally, in a case where the substituent included in Ar³ is a group represented by formula (A), the group represented by formula (A) is bonded to Ar³ in formula (3-X3) at the symbol *.

Ar⁴'s each independently represent a hydrogen atom, phenyl, biphenylyl, terphenylyl, naphthyl, or a silyl substituted by an alkyl having 1 to 4 carbon atoms (methyl, ethyl, t-butyl, and the like) and/or a cycloalkyl having 5 to 10 carbon atoms.

Examples of the alkyl having 1 to 4 carbon atoms, by which a silyl is substituted, include methyl, ethyl, propyl, i-propyl, butyl, sec-butyl, t-butyl, and cyclobutyl, and three hydrogen atoms in the silyl are each independently substituted by these alkyls.

Specific examples of the "silyl substituted by an alkyl having 1 to 4 carbon atoms" include a trimethylsilyl, a triethylsilyl, a tripropylsilyl, a tri-i-propylsilyl, a tributylsilyl, a tri sec-butylsilyl, a tri-t-butylsilyl, an ethyl dimethylsilyl, a propyldimethylsilyl, an i-propyldimethylsilyl, a butyldimethylsilyl, a sec-butyldimethylsilyl, a t-butyldimethylsilyl, a methyldiethylsilyl, a propyldiethylsilyl, an i-propyldiethylsilyl, a butyldiethylsilyl, a sec-butyl diethylsilyl, a t-butyldiethylsilyl, a methyldipropylsilyl, an ethyldipropylsilyl, a butyldipropylsilyl, a sec-butyldipropylsilyl, a t-butyldipropylsilyl, a methyl di-i-propylsilyl, an ethyl di-i-propylsilyl, a butyl di-i-propylsilyl, a sec-butyl di-i-propylsilyl, and a t-butyl di-i-propylsilyl.

Examples of the cycloalkyl having 5 to 10 carbon atoms, by which a silyl is substituted, include cyclopentyl, cyclohexyl, cycloheptyl, cyclooctyl, cyclononyl, cyclodecyl, norbornenyl, bicyclo[1.1.1]pentyl, bicyclo[2.0.1]pentyl, bicyclo[1.2.1]hexyl, bicyclo[3.0.1]hexyl, bicyclo[2.1.2]heptyl, bicyclo[2.2.2]octyl, adamantyl, decahydronaphthalenyl, and decahydroazulenyl. Three hydrogen atoms in the silyl are each independently substituted by these cycloalkyls.

Specific examples of the "silyl substituted by a cycloalkyl having 5 to 10 carbon atoms" include tricyclopentylsilyl and tricyclohexylsilyl.

Examples of the silyl substituted include a dialkylcycloalkylsilyl substituted by two alkyls and one cycloalkyl and an alkyldicycloalkylsilyl substituted by one alkyl and two cycloalkyls. Specific examples of the alkyl and cycloalkyl for substitution include the groups described above.

Furthermore, a hydrogen atom in a chemical structure of the anthracene-based compound represented by general formula (3) may be substituted by a group represented by the above formula (A). In a case where the hydrogen atom is substituted by the group represented by formula (A), the group represented by formula (A) is substituted by at least one hydrogen atom in the compound represented by formula (3) at the symbol *.

The group represented by the formula (A) is one of the substituents that the anthracene-based compound represented by the formula (3) may have.

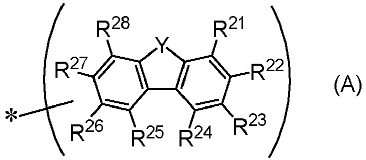

In the above formula (A), Y represents - O-, -S-, or > N-R²⁹, R²¹ to R²⁸ each independently represent a hydrogen atom, an optionally substituted alkyl, an optionally substituted cycloalkyl an optionally substituted aryl, an optionally substituted heteroaryl, an optionally substituted alkoxy, an optionally substituted aryloxy, an optionally substituted arylthio, a trialkylsilyl, a tricycloalkylsilyl, a dialkylcycloalkylsilyl, an alkyldicycloalkylsilyl, an optionally substituted amino, a halogen atom, hydroxy, or cyano, adjacent groups among R²¹ to R²⁸ may be bonded to each other to form a hydrocarbon ring, an aryl ring, or a heteroaryl ring, and R²⁹ represents a hydrogen atom or an optionally substituted aryl.

The "alkyl" as the "optionally substituted alkyl" in R²¹ to R²⁸ may be either linear or branched, and examples thereof include a linear alkyl having 1 to 24 carbon atoms and a branched alkyl having 3 to 24 carbon atoms. An alkyl having 1 to 18 carbon atoms (branched alkyl having 3 to 18 carbon atoms) is preferable, an alkyl having 1 to 12 carbon atoms (branched alkyl having 3 to 12 carbon atoms) is more preferable, an alkyl having 1 to 6 carbon atoms (branched alkyl having 3 to 6 carbon atoms) is still more preferable, and an alkyl having 1 to 4 carbon atoms (branched alkyl having 3 or 4 carbon atoms) is particularly preferable.

Specific examples of the "alkyl" include methyl, ethyl, n-propyl, isopropyl, n-butyl, isobutyl, s-butyl, t-butyl, n-pentyl, isopentyl, neopentyl, t-pentyl (t-amyl), n-hexyl, 1-methylpentyl, 3,3-dimethylbutyl, 2-ethylbutyl, n-heptyl, 1-methylhexyl, n-octyl, t-octyl (1,1,3,3-tetramethylbuthyl), 1-methylheptyl, 2-ethylhexyl, 2-propylpentyl, n-nonyl, 2,2-dimethylheptyl, 2,6-dimethyl-4-heptyl, 3,5,5-trimethylhexyl, n-decyl, n-undecyl, 1-methyldecyl, n-dodecyl, n-tridecyl, 1-hexylheptyl, n-tetradecyl, n-pentadecyl, n-hexadecyl, n-heptadecyl, n-octadecyl, and n-eicosyl.

Examples of the "cycloalkyl" as the "optionally substituted cycloalkyl" in R²¹ to R²⁸ include a cycloalkyl having 3 to 24 carbon atoms, a cycloalkyl having 3 to 20 carbon atoms, a cycloalkyl having 3 to 16 carbon atoms, a cycloalkyl having 3 to 14 carbon atoms, a cycloalkyl having 5 to 10 carbon atoms, a cycloalkyl having 5 to 8 carbon atoms, a cycloalkyl having 5 or 6 carbon atoms, and a cycloalkyl having 5 carbon atoms.

Specific examples of the "cycloalkyl" include cyclopropyl, cyclobutyl, cyclopentyl, cyclohexyl, cycloheptyl, cyclooctyl, cyclononyl, cyclodecyl, alkyl (especially methyl) substituents thereof having 1 to 4 carbon atoms, norbornenyl, bicyclo[1.0.1]butyl, bicyclo[1.1.1]pentyl, bicyclo[2.0.1]pentyl, bicyclo[1.2.1]hexyl, bicyclo[3.0.1]hexyl, bicyclo[2.1.2]heptyl, bicyclo[2.2.2]octyl, adamantyl, diamantyl, decahydronaphthalenyl, and decahydroazulenyl.

Examples of the "aryl" as the "optionally substituted aryl" in R²¹ to R²⁸ include an aryl having 6 to 30 carbon atoms. An aryl having 6 to 16 carbon atoms is preferable, an aryl having 6 to 12 carbon atoms is more preferable, and an aryl having 6 to 10 carbon atoms is particularly preferable.

Specific examples of the "aryl" include phenyl which is a monocyclic system; biphenylyl which is a bicyclic system; naphthyl which is a fused bicyclic system; terphenylyl (m-terphenylyl, o-terphenylyl, or p-terphenylyl) which is a tricyclic system; acenaphthylenyl, fluorenyl, phenalenyl, and phenanthrenyl which are fused tricyclic systems; triphenylenyl, pyrenyl, and naphthacenyl which are fused tetracyclic systems; and perylenyl and pentacenyl which are fused pentacyclic systems.

Examples of the "heteroaryl" as the "optionally substituted heteroaryl" in R²¹ to R²⁸ include a heteroaryl having 2 to 30 carbon atoms. A heteroaryl having 2 to 25 carbon atoms is preferable, a heteroaryl having 2 to 20 carbon atoms is more preferable, a heteroaryl having 2 to 15 carbon atoms is still more preferable, and a heteroaryl having 2 to 10 carbon atoms is particularly preferable. In addition, examples of the heteroaryl include a heterocyclic ring containing 1 to 5 heteroatoms, selected from an oxygen atom, a sulfur atom, and a nitrogen atom in addition to a carbon atom as a ringconstituting atom.

Specific examples of the "heteroaryl" include pyrrolyl, oxazolyl, isoxazolyl, thiazolyl, isothiazolyl, imidazolyl, oxadiazolyl, thiadiazolyl, triazolyl, tetrazolyl, pyrazolyl, pyridyl, pyrimidinyl, pyridazinyl, pyrazinyl, triazinyl, indolyl, isoindolyl, 1H-indazolyl, benzoimidazolyl, benzoxazolyl, benzothiazolyl, 1H-benzotriazolyl, quinolyl, isoquinolyl, cinnolyl, quinazolyl, quinoxalinyl, phthalazinyl, naphthyridinyl, purinyl, pteridinyl, carbazolyl, acridinyl, phenoxathiinyl, phenoxazinyl, phenothiazinyl, phenazinyl, phenazasilinyl, indolizinyl, furanyl, benzofuranyl, isobenzofuranyl, dibenzofuranyl, naphthobenzofuranyl, thiophenyl, benzothiophenyl, isobenzothiophenyl, dibenzothiophenyl, naphthobenzothiophenyl, benzophospholyl, dibenzophospholyl, monovalent group of benzophosphole oxide ring, monovalent group of dibenzophosphole oxide ring, furazanyl, thianthrenyl, indolocarbazolyl, benzoindolocarbazolyl, and benzobenzoindolocarbazolyl.

Examples of the "alkoxy" as the "optionally substituted alkoxy" in R²¹ to R²⁸ include a linear alkoxy having 1 to 24 carbon atoms and a branched alkoxy having 3 to 24 carbon atoms. An alkoxy having 1 to 18 carbon atoms (branched alkoxy having 3 to 18 carbon atoms) is preferable, an alkoxy having 1 to 12 carbon atoms (branched alkoxy having 3 to 12 carbon atoms) is more preferable, an alkoxy having 1 to 6 carbon atoms (branched alkoxy having 3 to 6 carbon atoms) is still more preferable, and an alkoxy having 1 to 4 carbon atoms (branched alkoxy having 3 or 4 carbon atoms) is particularly preferable.

Specific examples of the "alkoxy" include methoxy, ethoxy, propoxy, isopropoxy, butoxy, isobutoxy, s-butoxy, t-butoxy, pentyloxy, hexyloxy, heptyloxy, and octyloxy.

Examples of the "aryloxy" as the "optionally substituted aryloxy" in R²¹ to R²⁸ include a group in which a hydrogen atom of an -OH group is substituted by an aryl. For this aryl, the groups described as the above "aryl" in R²¹ to R²⁸ can be cited.

Examples of the "arylthio" as the "optionally substituted arylthio" in R²¹ to R²⁸ include a group in which a hydrogen atom of an -SH group is substituted by an aryl. For this aryl, the groups described as the above "aryl" in R²¹ to R²⁸ can be cited.

Examples of the "trialkylsilyl" in R²¹ to R²⁸ include a group in which three hydrogen atoms in a silyl group are each independently substituted by an alkyl. For this alkyl, the groups described as the above "alkyl" in R²¹ to R²⁸ can be cited. A preferable alkyl for substitution is an alkyl having 1 to 4 carbon atoms, and specific examples thereof include methyl, ethyl, propyl, i-propyl, butyl, sec-butyl, t-butyl, and cyclobutyl.

Specific examples of the "trialkylsilyl" include a trimethylsilyl, a triethylsilyl, a tripropylsilyl, a tri-i-propylsilyl, a tributylsilyl, a tri sec-butylsilyl, a tri-t-butylsilyl, an ethyl dimethylsilyl, a propyldimethylsilyl, an i-propyldimethylsilyl, a butyldimethylsilyl, a sec-butyldimethylsilyl, a t-butyldimethylsilyl, a methyldiethylsilyl, a propyldiethylsilyl, an i-propyldiethylsilyl, a butyldiethylsilyl, a sec-butyl diethylsilyl, a t-butyldiethylsilyl, a methyldipropylsilyl, an ethyldipropylsilyl, a butyldipropylsilyl, a sec-butyldipropylsilyl, a t-butyldipropylsilyl, a methyl di-i-propylsilyl, an ethyl di-i-propylsilyl, a butyl di-i-propylsilyl, a sec-butyl di-i-propylsilyl, and a t-butyl di-i-propylsilyl.

Examples of the "tricycloalkylsilyl" in R²¹ to R²⁸ include a group in which three hydrogen atoms in a silyl group are each independently substituted by a cycloalkyl. For this cycloalkyl, the groups described as the above "cycloalkyl" in R²¹ to R²⁸ can be cited. A preferable cycloalkyl for substitution is a cycloalkyl having 5 to 10 carbon atoms. Specific examples thereof include cyclopentyl, cyclohexyl, cycloheptyl, cyclooctyl, cyclononyl, cyclodecyl, bicyclo[1.1.1]pentyl, bicyclo[2.0.1]pentyl, bicyclo[1.2.1]hexyl, bicyclo[3.0.1]hexyl, bicyclo[2.1.2]heptyl, bicyclo[2.2.2]octyl, adamantyl, decahydronaphthalenyl, and decahydroazulenyl.

Specific examples of the "tricycloalkylsilyl" include tricyclopentylsilyl and tricyclohexylsilyl.

Specific examples of the dialkylcycloalkylsilyl substituted by two alkyls and one cycloalkyl and the alkyldicycloalkylsilyl substituted by one alkyl and two cycloalkyls include a silyl substituted by a group selected from the specific alkyls and cycloalkyls described above.

Examples of the "substituted amino" as the "optionally substituted amino" in R²¹ to R²⁸ include an amino group in which two hydrogen atoms are substituted by an aryl or a heteroaryl. An amino in which two hydrogen atoms are substituted by aryls is a diaryl-substituted amino, an amino in which two hydrogen atoms are substituted by heteroaryls is a diheteroaryl-substituted amino, and an amino in which two hydrogen atom are substituted by an aryl and a heteroaryl is an arylheteroaryl-substituted amino. For the aryl and heteroaryl, the groups described as the above "aryl" and "heteroaryl" in R²¹ to R²⁸ can be cited.

Specific examples of the "substituted amino" include diphenylamino, dinaphthylamino, phenylnaphthylamino, dipyridylamino, phenylpyridylamino, and naphthylpyridylamino.

Examples of the "halogen atom" in R²¹ to R²⁸ include a fluorine atom, a chlorine atom, a bromine atom, and an iodine atom.

Some of the groups described as R²¹ to R²⁸ may be substituted as described above, and examples of the substituent in this case include an alkyl, a cycloalkyl, an aryl, and a heteroaryl. For the alkyl, cycloalkyl, aryl, or heteroaryl, the groups described as the above "alkyl", "cycloalkyl", "aryl", or "heteroaryl" in R²¹ to R²⁸ can be cited.

R²⁹ in the moiety "> N-R²⁹" as Y is a hydrogen atom or an optionally substituted aryl. For the aryl, the groups described as the above "aryl" in R²¹ to R²⁸ can be cited. As the substituent, the groups described as the substituent for R²¹ to R²⁸ can be cited.

Adjacent groups among R²¹ to R²⁸ may be bonded to each other to form a hydrocarbon ring, an aryl ring, or a heteroaryl ring. Examples of a case of not forming a ring include a group represented by the following formula (A-1). Examples of a case of forming a ring include groups represented by any of the following formulas (A-2) to (A-14). Y and * in the formula have the same definition as above. Note that at least one hydrogen atom in a group represented by any one of formulas (A-1) to (A-14) may be substituted by an alkyl, a cycloalkyl, an aryl, a heteroaryl, an alkoxy, an aryloxy, an arylthio, a trialkylsilyl, a tricycloalkylsilyl, a dialkylcycloalkylsilyl, an alkyldicycloalkylsilyl, a diaryl-substituted amino, a diheteroaryl-substituted amino, an arylheteroaryl-substituted amino, a halogen atom, hydroxy, or cyano. The symbol "*" in each structural formula represents a connection position.

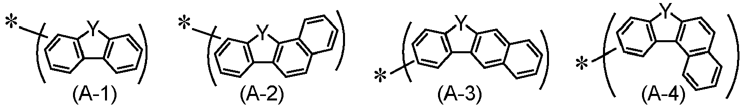

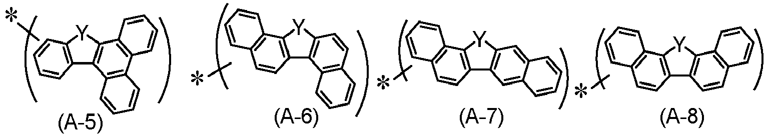

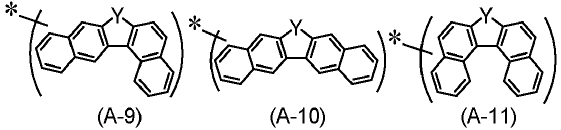

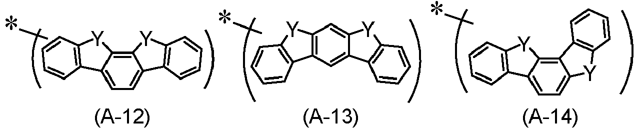

Examples of the ring formed by bonding adjacent groups to each other include a cyclohexane ring in a case of a hydrocarbon ring. Examples of the aryl ring and heteroaryl ring include ring structures described in the above "aryl" and "heteroaryl" in R²¹ to R²⁸, and these rings are formed so as to be fused with one or two benzene rings in the above formula (A-1).

Examples of the group represented by formula (A) include a group represented by any one of the above formulas (A-1) to (A-14). A group represented by any one of the above formulas (A-1) to (A-5) and (A-12) to (A-14) is preferable, a group represented by any one of the above formulas (A-1) to (A-4) is more preferable, a group represented by any one of the above formulas (A-1), (A-3), and (A-4) is still more preferable, and a group represented by the above formula (A-1) is particularly preferable.

As described above, at the symbol * in formula (A), the group represented by formula (A) is bonded to a naphthalene ring in formula (3-X1) or (3-X2), a single bond in formula (3-X3), or Ar³ in formula (3-X3), and at least one hydrogen atom in a compound represented by formula (3) is substituted by the group represented by formula (A). Among these bonding forms, a form in which the group represented by formula (A) is bonded to at least one of a naphthalene ring in formula (3-X1) or (3-X2), a single bond in formula (3-X3), and Ar³ in formula (3-X3) is preferable.

A position at which a naphthalene ring in formula (3-X1) or (3-X2), a single bond in formula (3-X3), or Ar³ in formula (3-X3) is bonded to the group represented by formula (A) in the structure of the group represented by formula (A), and a position at which at least one hydrogen atom in a compound represented by formula (3) is substituted by the group represented by formula (A) in the structure of the group represented by formula (A) may be any position in the structure of formula (A). For example, bonding can be made at any one of the two benzene rings in the structure of formula (A), at any ring formed by bonding adjacent groups among R²¹ to R²⁸ in the structure of formula (A), or at any position in R²⁹ in the moiety "> N-R²⁹" as Y in the structure of formula (A).

Examples of the group represented by formula (A) include the following groups. Y and the * in the formula have the same definitions as above.

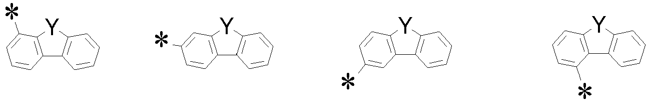

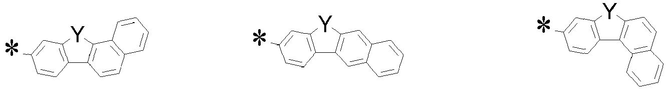

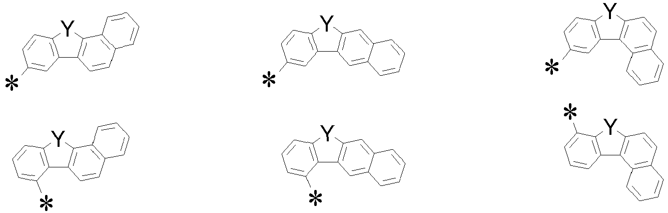

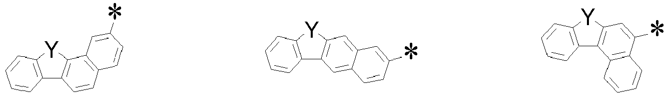

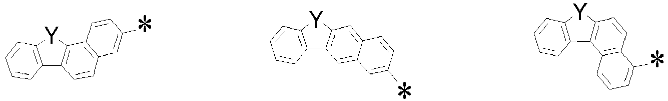

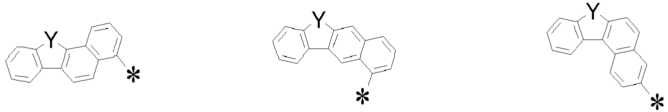

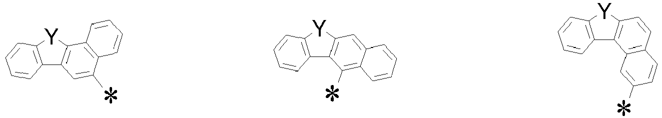

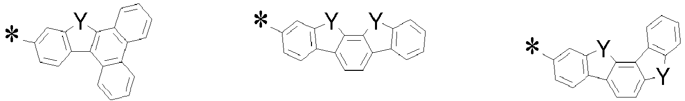

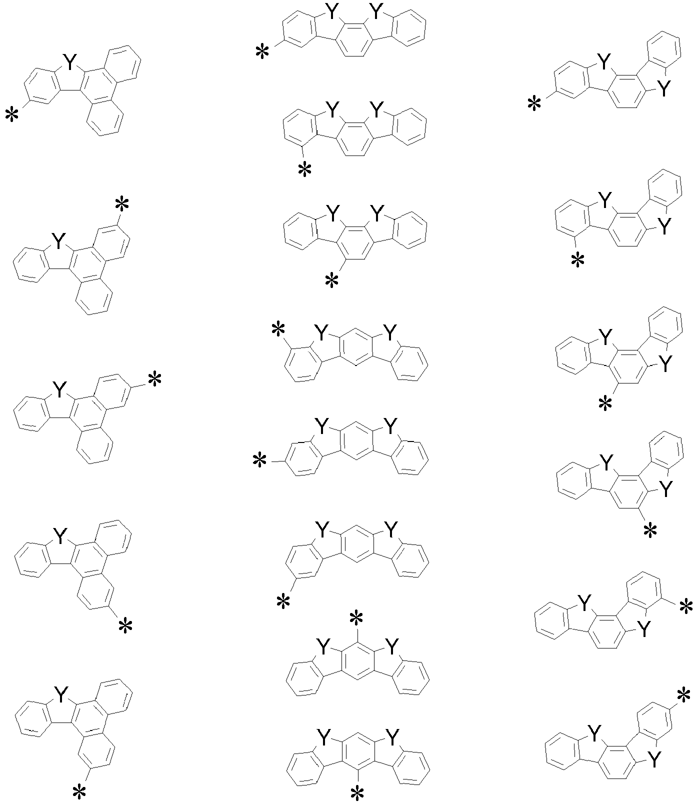

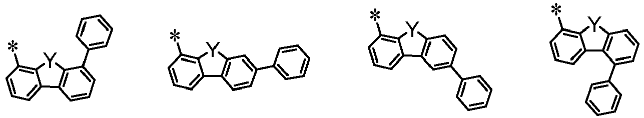

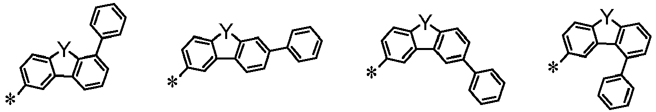

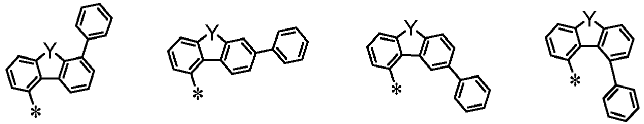

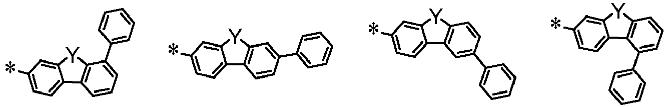

Furthermore, all or a part of the hydrogen atoms in the chemical structure of an anthracene-based compound represented by general formula (3) may be deuterium atoms.

Specific examples of the anthracene-based compound include compounds represented by any of the following formulas (3-1) to (3-142). In the following structural formula, "Me" indicates a methyl group, "D" indicates a deuterium, and "tBu" indicates a t-butyl group.

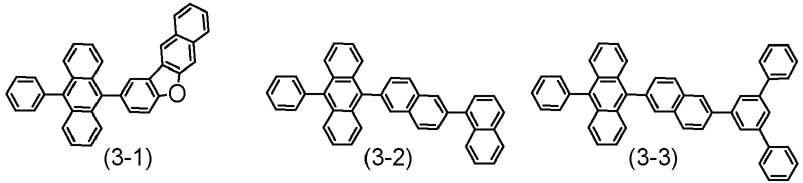

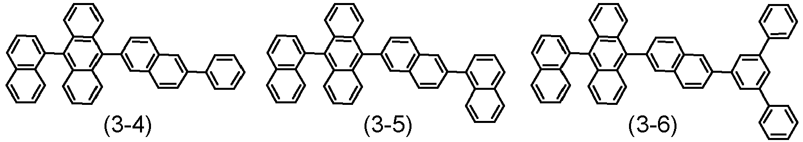

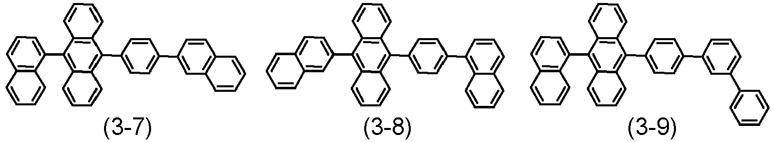

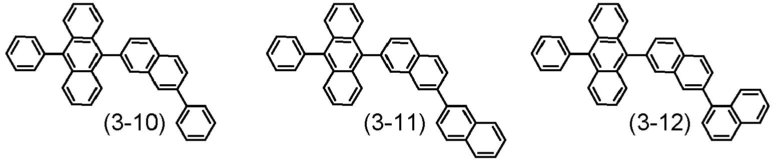

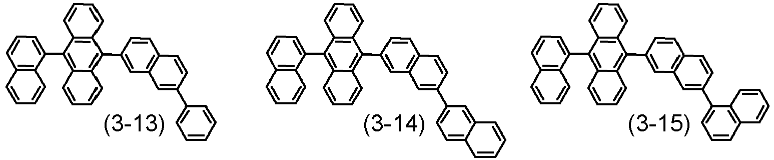

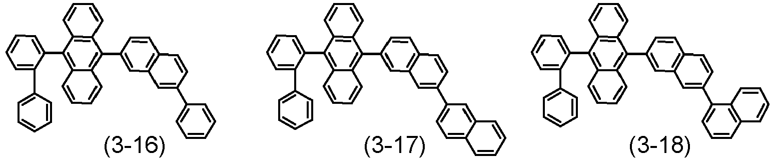

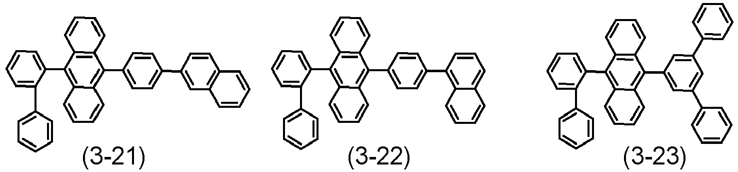

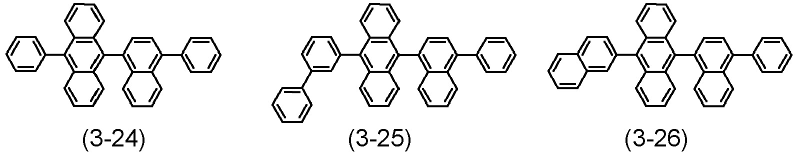

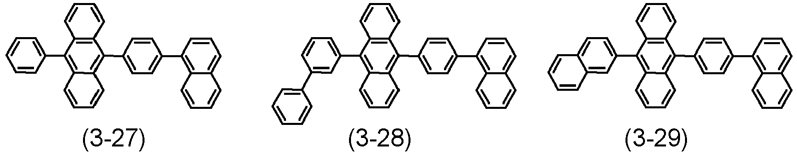

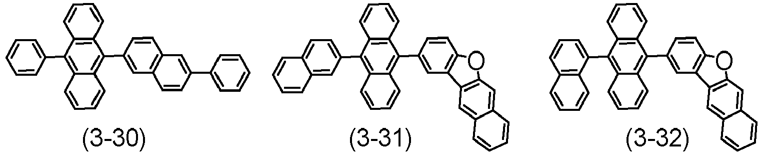

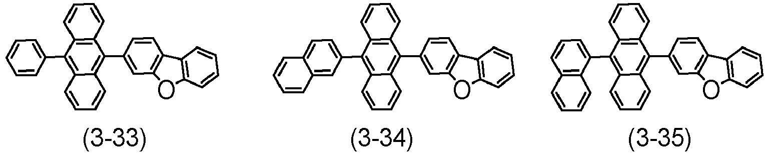

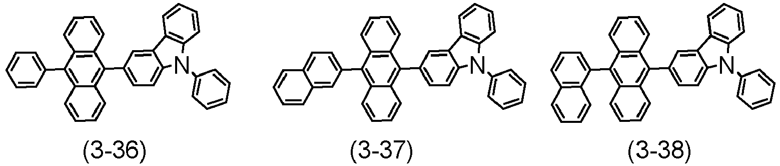

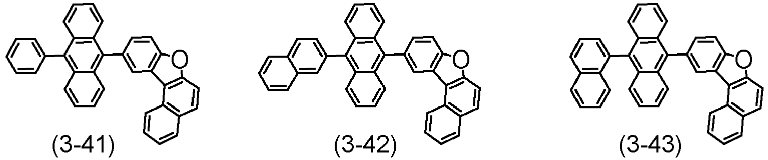

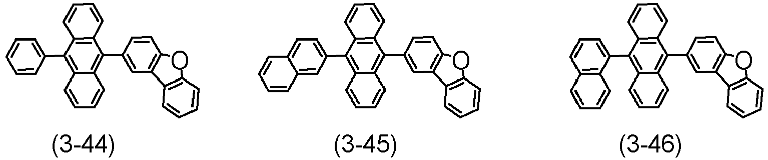

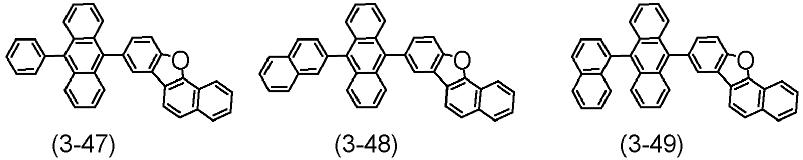

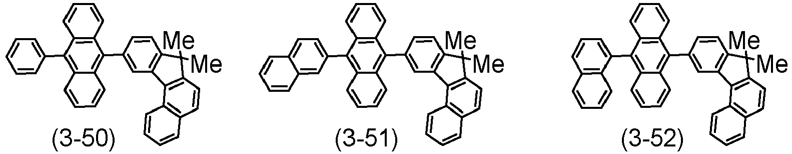

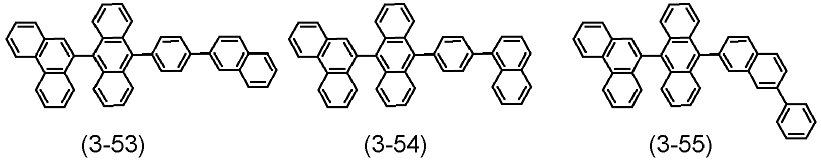

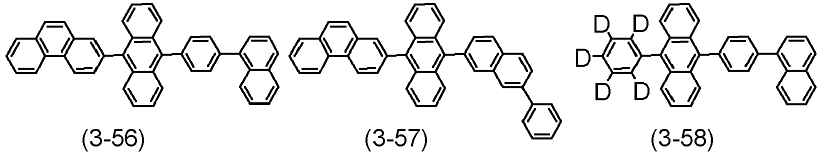

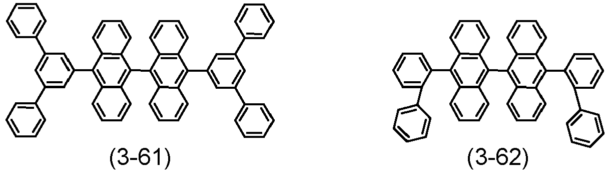

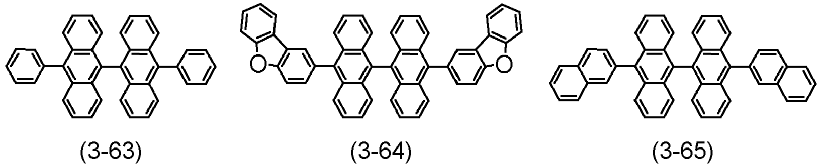

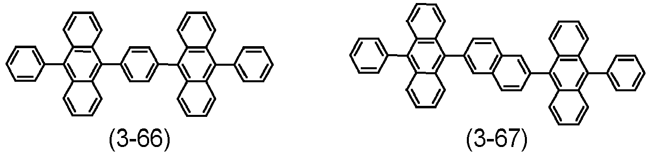

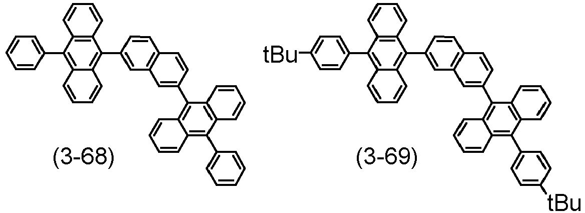

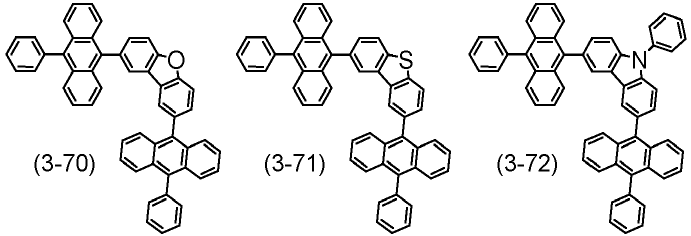

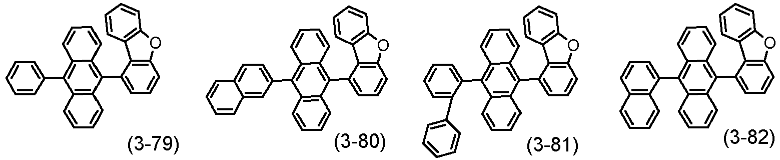

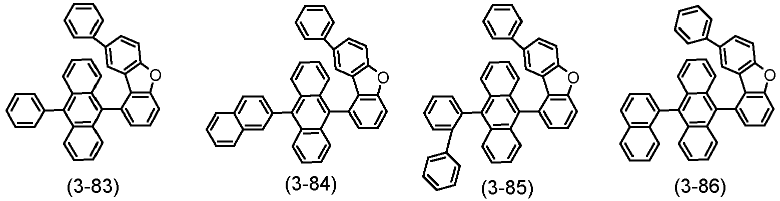

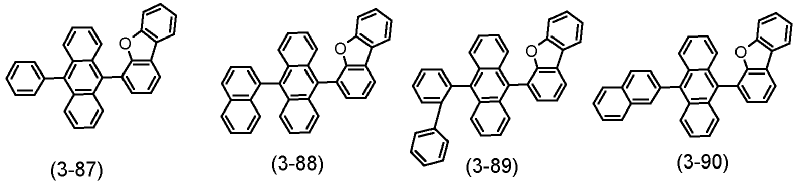

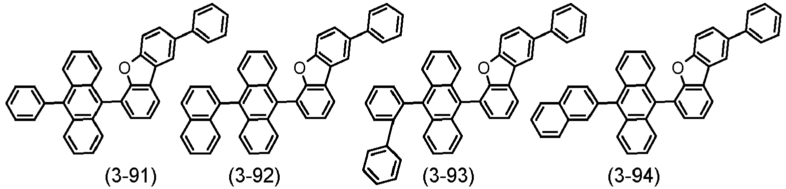

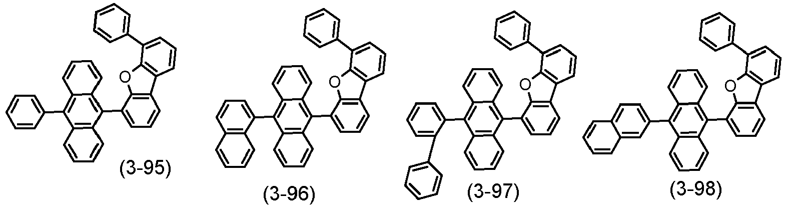

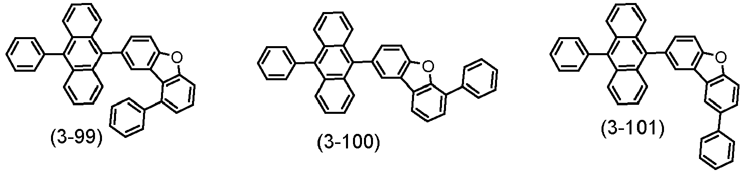

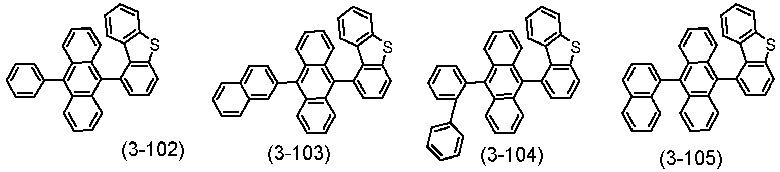

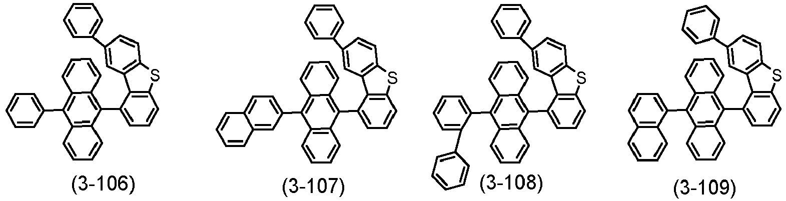

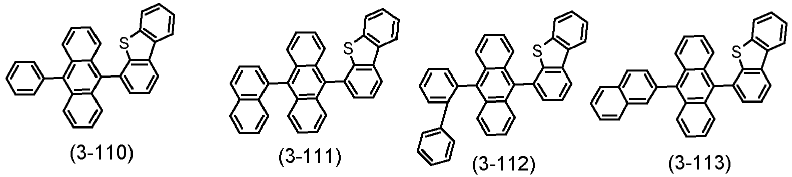

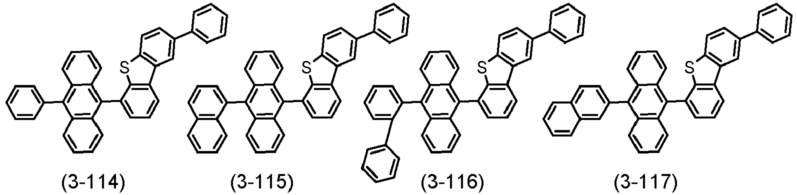

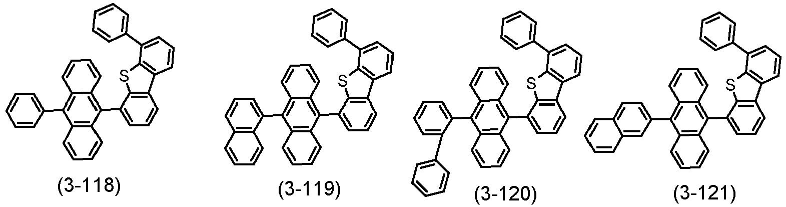

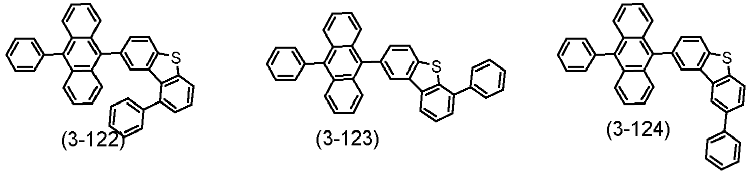

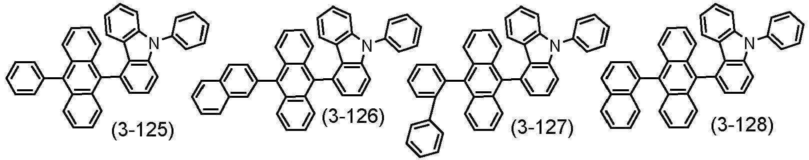

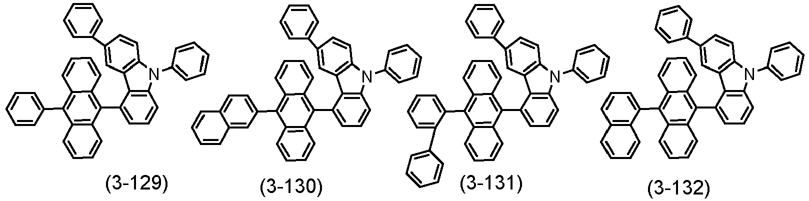

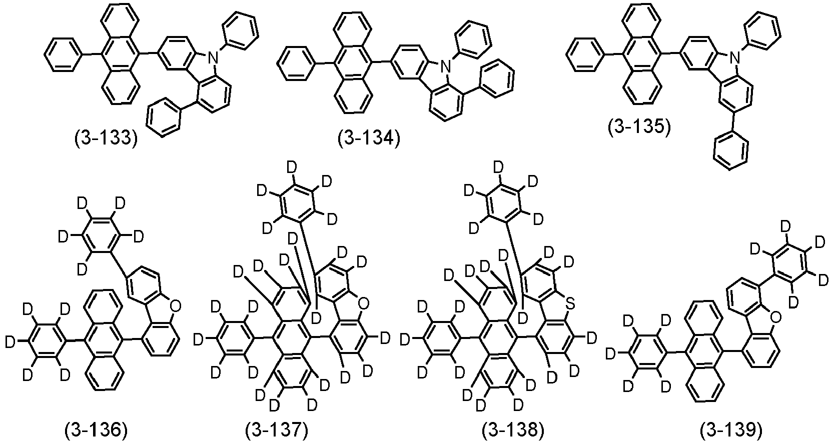

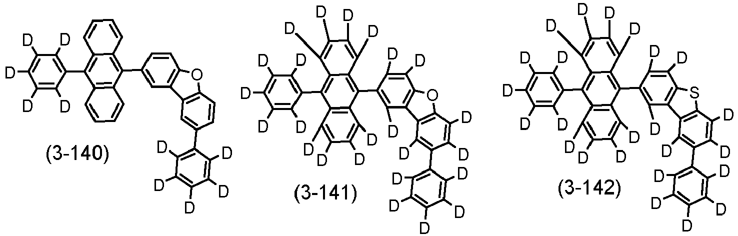

The anthracene compound represented by the formula (3) can be manufactured by applying Suzuki coupling, Negishi coupling, or other known coupling reactions, using, as a starting material, a compound having a reactive group at a desired position on the anthracene skeleton, and compounds having a reactive group in a partial structure such as the structure of X, Ar⁴ and the formula (A). Examples of the reactive group of these reactive compounds include halogen and boronic acid. As a specific production method, for example, the synthesis method in paragraphs [0089] to [0175] of WO2014/141725 can be referred to.

### <Fluorene-based compound>

Basically, a compound represented by general formula (4) functions as a host.

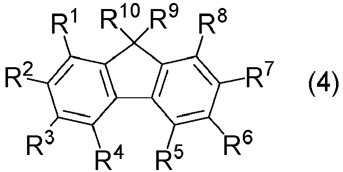

In the above formula (4),
R¹ to R¹⁰ each independently represent a hydrogen atom, an aryl, a heteroaryl (the heteroaryl may be bonded to a fluorene skeleton in the above formula (4) via a linking group), a diarylamino, a diheteroarylamino, an arylheteroarylamino, an alkyl, a cycloalkyl, an alkenyl, an alkoxy, or an aryloxy, in which at least one hydrogen atom may be substituted by an aryl, a heteroaryl, an alkyl, or a cycloalkyl,
R¹ and R², R² and R³, R³ and R⁴, R⁵ and R⁶, R⁶ and R⁷, R⁷ and R⁸, and R⁹ and R¹⁰ may be each independently bonded to each other to form a fused ring or a spiro ring, at least one hydrogen atom in the ring thus formed may be substituted by an aryl, a heteroaryl (the heteroaryl may be bonded to the ring thus formed via a linking group), a diarylamino, a diheteroarylamino, an arylheteroarylamino, an alkyl, a cycloalkyl, an alkenyl, an alkoxy, or an aryloxy, in which at least one hydrogen atom in these may be substituted by an aryl, a heteroaryl, an alkyl, or a cycloalkyl, and
at least one hydrogen atom in the compound represented by formula (4) may be substituted by a halogen atom, cyano, or a deuterium atom.

For the details of each group in the definition of the above formula (4), the above description for the polycyclic aromatic compound of formula (1) can be cited.

Examples of the alkenyl in R¹ to R¹⁰ include an alkenyl having 2 to 30 carbon atoms. An alkenyl having 2 to 20 carbon atoms is preferable, an alkenyl having 2 to 10 carbon atoms is more preferable, an alkenyl having 2 to 6 carbon atom is still more preferable, and an alkenyl having 2 to 4 carbon atoms is particularly preferable. The alkenyl is preferably vinyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, or 5-hexenyl.

Note that specific examples of the heteroaryl include a monovalent group represented by removing any one hydrogen atom from a compound of the following formula (4-Ar1), (4-Ar2), (4-Ar3), (4-Ar4), or (4-Ar5).

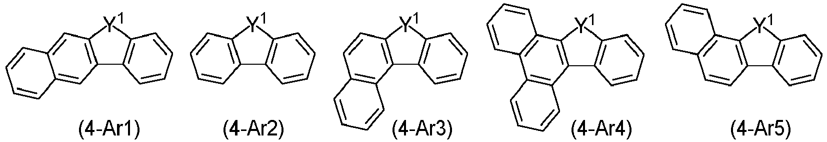

In formulas (4-Ar1) to (4-Ar5), Y¹' s each independently represent O, S, or N-R, while R represents phenyl, biphenylyl, naphthyl, anthracenyl, or a hydrogen atom, and
at least one hydrogen atom in the structures of the above formulas (4-Ar1) to (4-Ar5) may be substituted by phenyl, biphenylyl, naphthyl, anthracenyl, phenanthrenyl, methyl, ethyl, propyl, or butyl.

These heteroaryls may be bonded to the fluorene skeleton in the above formula (4) via a linking group. That is, the fluorene skeleton in formula (4) and the above heteroaryl may be bound not only directly but also via a linking group therebetween. Examples of the linking group include phenylene, biphenylene, naphthylene, anthracenylene, methylene, ethylene, -OCH₂CH₂-, -CH₂CH₂O-, and -OCH₂CH₂O-.

Furthermore, R¹ and R², R² and R³, R³ and R⁴, R⁵ and R⁶, R⁶ and R⁷, or R⁷ and R⁸ in formula (4) may be each independently bonded to each other to form a fused ring, and R⁹ and R¹⁰ may be bonded to each other to form a spiro ring. The fused ring formed by R¹ to R⁸ is a ring fused to the benzene ring in formula (4), and is an aliphatic ring or an aromatic ring. The fused ring is preferably an aromatic ring, and examples of the structure thereof including the benzene ring in formula (4) include a naphthalene ring and a phenanthrene ring. The spiro ring formed by R⁹ and R¹⁰ is a ring spiro-bonded to the 5-membered ring in formula (4), and is an aliphatic ring or an aromatic ring. The spiro ring is preferably an aromatic ring, and examples thereof include a fluorene ring.

The compound represented by general formula (4) is preferably a compound represented by the following formula (4-1), (4-2), or (4-3). The compound represented by formula (4-1) is a compound in which a benzene ring formed by bonding between R¹ and R² in general formula (4) is fused. The compound represented by formula (4-2) is a compound in which a benzene ring formed by bonding between R³ and R⁴ in general formula (4) is fused. The compound represented by formula (4-3) is a compound in which any two of R¹ to R⁸ in general formula (4) are not bonded to each other.

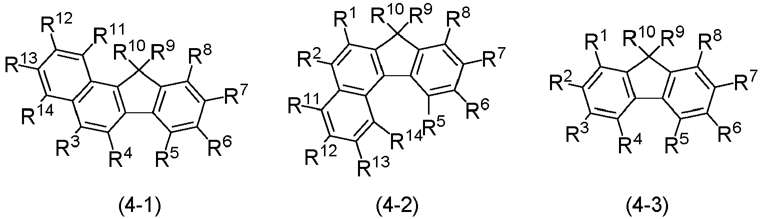

The definitions of R¹ to R¹⁰ in formulas (4-1), (4-2), and (4-3) are the same as those of the corresponding R¹ to R¹⁰ in formula (4). The definitions of R¹¹ to R¹⁴ in formulas (4-1) and (4-2) are the same as those of R¹ to R¹⁰ in formula (4).

The compound represented by general formula (4) is more preferably a compound represented by the following formula (4-1A), (4-2A), or (4-3A), which is a compound in which R⁹ and R¹⁰ are bonded to each other to form a spiro-fluorene ring in formula (4-1), (4-2), or (4-3).

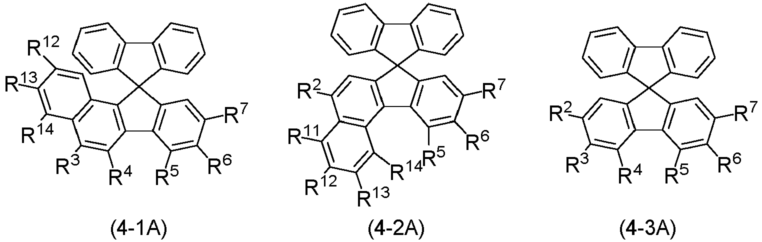

The definitions of R² to R⁷ in formulas (4-1A), (4-2A), and (4-3A) are the same as those of the corresponding R² to R⁷ in formulas (4-1), (4-2), and (4-3). The definitions of R¹¹ to R¹⁴ in formulas (4-1A) and (4-2A) are the same as those of R¹¹ to R¹⁴ in formulas (4-1) and (4-2).

Furthermore, all or a part of hydrogen atoms in the compound represented by formula (4) may be substituted by halogen atoms, cyanos, or deuterium atoms.

Specific examples of the fluorene based compound include compounds represented by any of the following formulas (4-4) to (4-22). In addition, "Me" in the following structural formula indicates a methyl group.

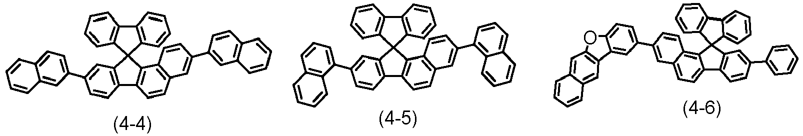

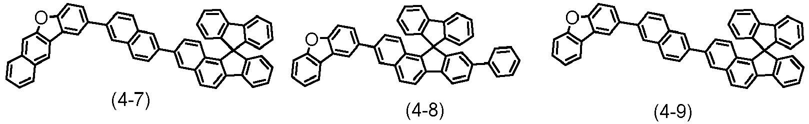

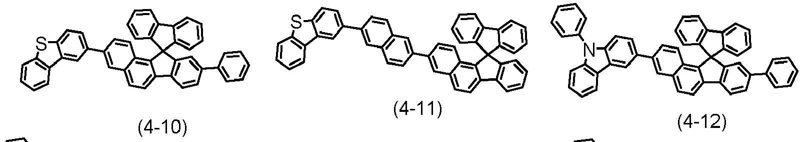

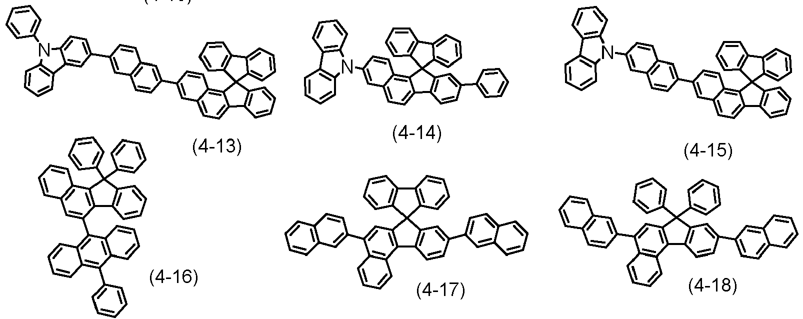

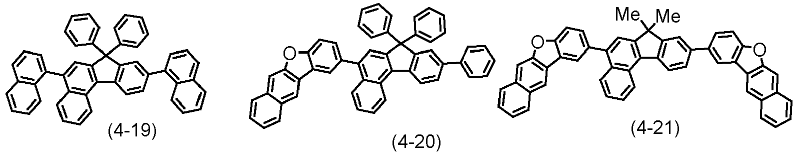

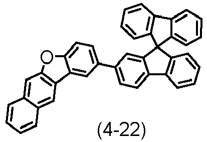

### <Dibenzochrysene-based compound>

A dibenzochrysene-based compound as a host is, for example, a compound represented by the following general formula (5).

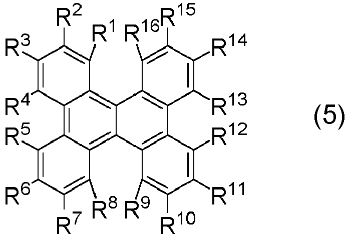

In the above formula (5),
R¹ to R¹⁶ each independently represent a hydrogen atom, an aryl, a heteroaryl (the heteroaryl may be bonded to a dibenzochrysene skeleton in the above formula (5) via a linking group), a diarylamino, a diheteroarylamino, an arylheteroarylamino, an alkyl, a cycloalkyl, an alkenyl, an alkoxy, or an aryloxy, in which at least one hydrogen atom may be substituted by an aryl, a heteroaryl, an alkyl, or a cycloalkyl,
adjacent groups among R¹ to R¹⁶ may be bonded to each other to form a fused ring, at least one hydrogen atom in the ring thus formed may be substituted by an aryl, a heteroaryl (the heteroaryl may be bonded to the ring thus formed via a linking group), a diarylamino, a diheteroarylamino, an arylheteroarylamino, an alkyl, a cycloalkyl, an alkenyl, an alkoxy, or an aryloxy, in which at least one hydrogen atom in these may be substituted by an aryl, a heteroaryl, an alkyl, or a cycloalkyl, and
at least one hydrogen atom in the compound represented by formula (5) may be substituted by a halogen atom, cyano, or a deuterium atom.

For the details of each group in the definition of the above formula (5), the above description for the polycyclic aromatic compound of formula (1) can be cited.

Examples of the alkenyl in the definition of the above formula (5) include an alkenyl having 2 to 30 carbon atoms. An alkenyl having 2 to 20 carbon atoms is preferable, an alkenyl having 2 to 10 carbon atoms is more preferable, an alkenyl having 2 to 6 carbon atom is still more preferable, and an alkenyl having 2 to 4 carbon atoms is particularly preferable. The alkenyl is preferably vinyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, or 5-hexenyl.

Note that specific examples of the heteroaryl include a monovalent group represented by removing any one hydrogen atom from a compound of the following formula (5-Ar1), (5-Ar2), (5-Ar3), (5-Ar4), or (5-Ar5).

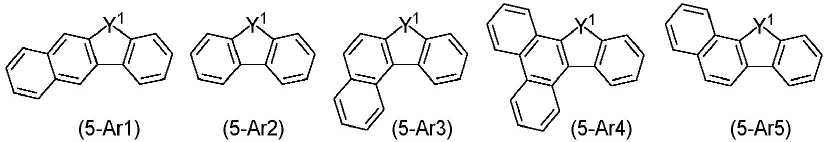

In formulas (5-Ar1) to (5-Ar5), Y¹'s each independently represent O, S, or N-R, while R represents phenyl, biphenylyl, naphthyl, anthracenyl, or a hydrogen atom, and
at least one hydrogen atom in the structures of the above formulas (5-Ar1) to (5-Ar5) may be substituted by phenyl, biphenylyl, naphthyl, anthracenyl, phenanthrenyl, methyl, ethyl, propyl, or butyl.

These heteroaryls may be bonded to the dibenzochrysene skeleton in the above formula (5) via a linking group. That is, the dibenzochrysene skeleton in formula (5) and the above heteroaryl may be bound not only directly but also via a linking group therebetween. Examples of the linking group include phenylene, biphenylene, naphthylene, anthracenylene, methylene, ethylene, -OCH₂CH₂-, -CH₂CH₂O-, and -OCH₂CH₂O-.

The compound represented by general formula (5) is preferably a compound in which R¹, R⁴, R⁵, R⁸, R⁹, R¹², R¹³, and R¹⁶ represent hydrogen atoms. In this case, R², R³, R⁶, R⁷, R¹⁰, R¹¹, R¹⁴, and R¹⁵ in formula (5) preferably each independently represent a hydrogen atom, phenyl, biphenylyl, naphthyl, anthracenyl, phenanthrenyl, a monovalent group having a structure of the above formula (5-Ar1), (5-Ar2), (5-Ar3), (5-Ar4), or (5-Ar5) (the monovalent group having the structure may be bonded to the dibenzochrysene skeleton in the above formula (5) via phenylene, biphenylene, naphthylene, anthracenylene, methylene, ethylene, -OCH₂CH₂-, -CH₂CH₂O-, or -OCH₂CH₂O-), methyl, ethyl, propyl, or butyl.

The compound represented by general formula (5) is more preferably a compound in which R¹, R², R⁴, R⁵, R⁷, R⁸, R⁹, R¹⁰, R¹², R¹³, R¹⁵, and R¹⁶ represent hydrogen atoms. In this case, at least one (preferably one or two, more preferably one) of R³, R⁶, R¹¹, and R¹⁴ in formula (5) represents a monovalent group having a structure of the above formula (5-Ar1), (5-Ar2), (5-Ar3), (5-Ar4), or (5-Ar5) via a single bond, phenylene, biphenylene, naphthylene, anthracenylene, methylene, ethylene, -OCH₂CH₂-, -CH₂CH₂O-, or -OCH₂CH₂O-,
the groups other than the above at least one (that is, groups at positions other than the position substituted by a monovalent group having the above structure) each represent a hydrogen atom, phenyl, biphenylyl, naphthyl, anthracenyl, methyl, ethyl, propyl, or butyl, in which at least one hydrogen atom may be substituted by phenyl, biphenylyl, naphthyl, anthracenyl, methyl, ethyl, propyl, or butyl.

Furthermore, in a case where a monovalent group having the structure represented by any one of the above formulas (5-Ar1) to the formula (5-Ar5) is selected as R², R³, R⁶, R⁷, R¹⁰, R¹¹, R¹⁴, or R¹⁵ in formula (5), at least one hydrogen atom in the structure may be bonded to any one of R¹ to R¹⁶ in formula (5) to form a single bond.

Specific examples of the dibenzochrysene based compound include compounds represented by any of the following formulas (5-1) to (5-39). In addition, "tBu" in the following structural formula indicates a t-buthyl group.

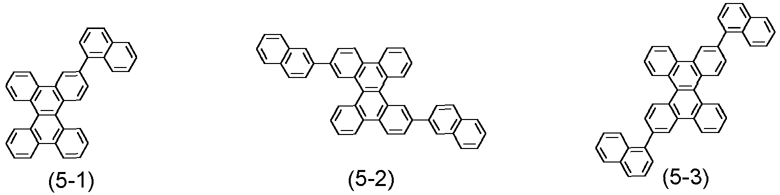

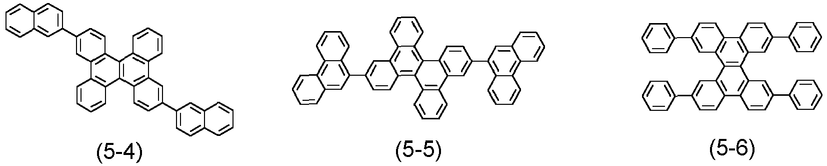

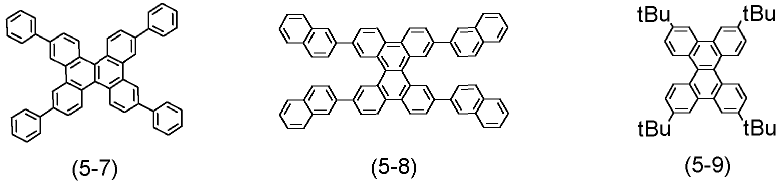

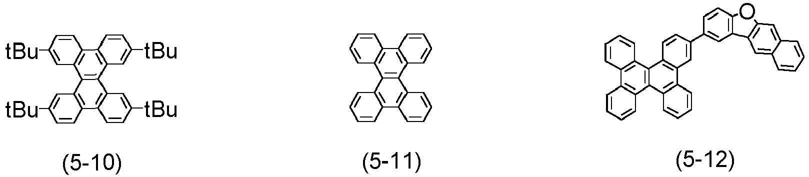

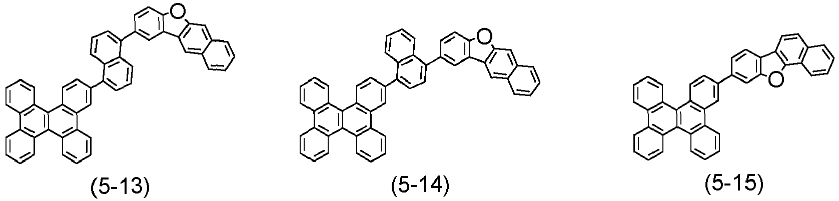

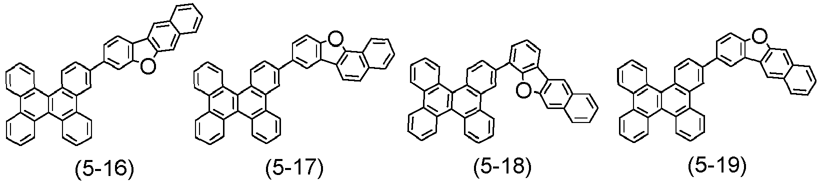

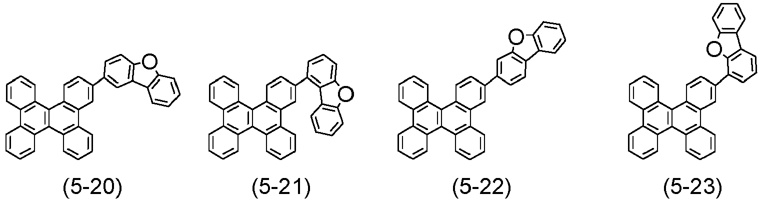

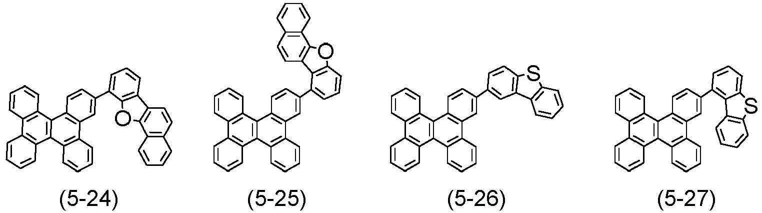

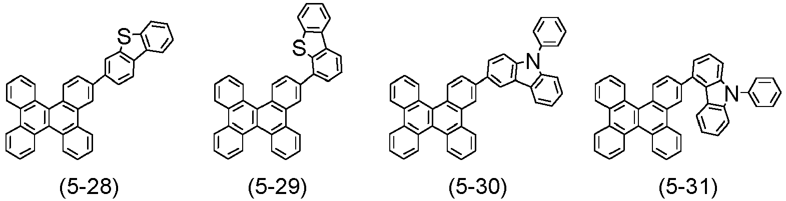

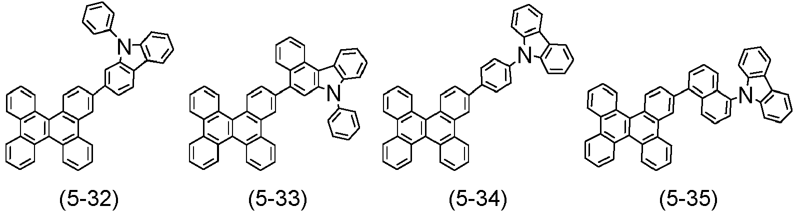

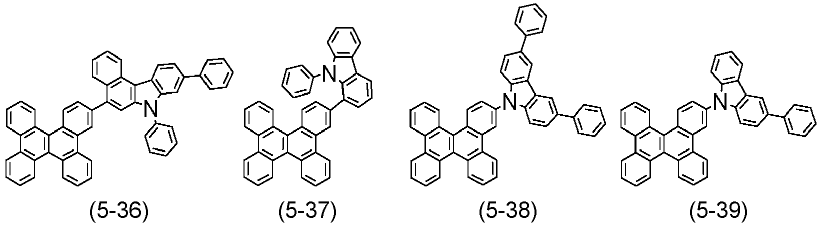

The above material for the light emitting layer (host material and dopant material) can be used as a material for the light emitting layer also in a form of a polymer compound obtained by polymerizing, as a monomer, a reactive compound in which the material for the light emitting layer is substituted by a reactive substituent, a crosslinked polymer thereof, a pendant type polymer compound obtained by a reaction between a main chain type polymer and the reactive compound, or a pendant type crosslinked polymer thereof. As the reactive substituent in this case, the description for the polycyclic aromatic compound represented by formula (1) can be cited.

Details of applications of the polymer compound and the crosslinked polymer will be described later.

### <Example of polymer host material>

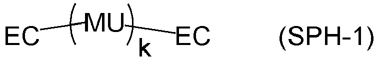

In formula (SPH-1),
MU's each independently represent a divalent group obtained by removing any two hydrogen atoms from an aromatic compound, EC's each independently represent a monovalent group obtained by removing any one hydrogen atom from an aromatic compound, each of two hydrogen atoms in MU is replaced with EC or MU, and k represents an integer of 2 to 50000.

More specifically,
MU's each independently represent an arylene, a heteroarylene, a diarylenearylamino, a diarylenearylboryl, an oxaborine-diyl, or an azaborine-diyl,
EC's each independently represent a hydrogen atom, an aryl, a heteroaryl, a diarylamino, a diheteroarylamino, an arylheteroarylamino, or an aryloxy,
at least one hydrogen atom in MU and EC may be further replaced with an aryl, a heteroaryl, a diarylamino, an alkyl, or a cycloalkyl, and
k represents an integer of 2 to 50000.
k is preferably an integer of 20 to 50000, and more preferably an integer of 100 to 50000.

At least one hydrogen atom in MU and EC in formula (SPH-1) may be replaced with an alkyl having 1 to 24 carbon atoms, a cycloalkyl having 3 to 24 carbon atoms, a halogen atom, or a deuterium atom. Furthermore, any -CH₂- in the alkyl may be replaced with -O- or - Si(CH₃)₂-, any -CH₂- except for -CH₂- directly bonded to EC in formula (SPH-1) in the alkyl may be replaced with an arylene having 6 to 24 carbon atoms, and any hydrogen atom in the alkyl may be replaced with a fluorine atom.

Examples of MU include a divalent group obtained by removing any two hydrogen atoms from any one of the following compounds.

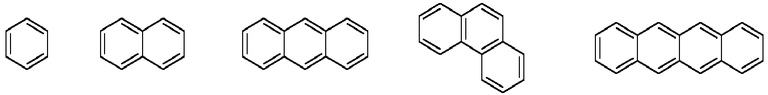

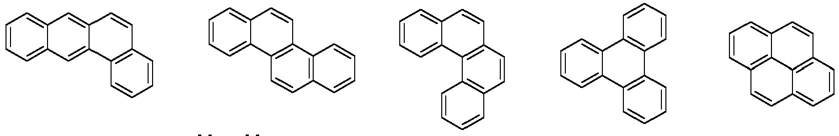

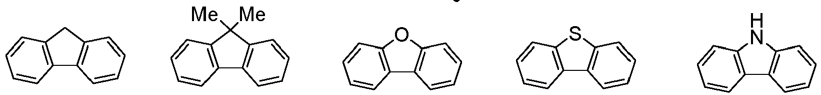

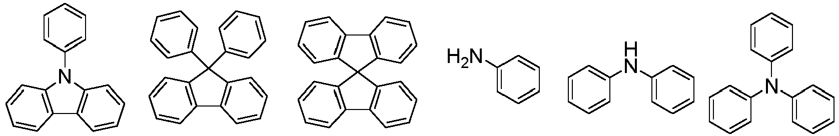

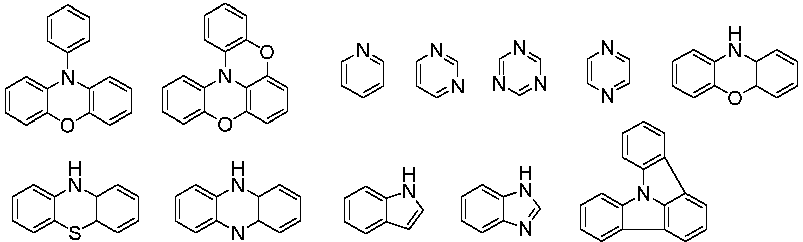

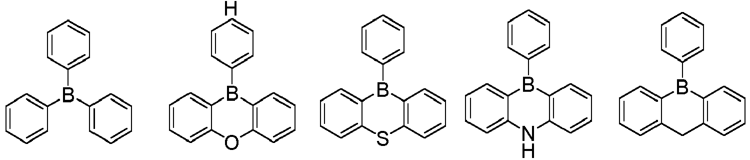

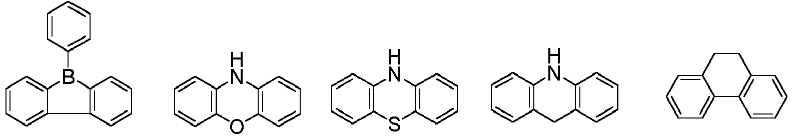

More specific examples thereof include divalent groups represented by the following structures. In these structures, MU is bonded to another MU or EC at *.

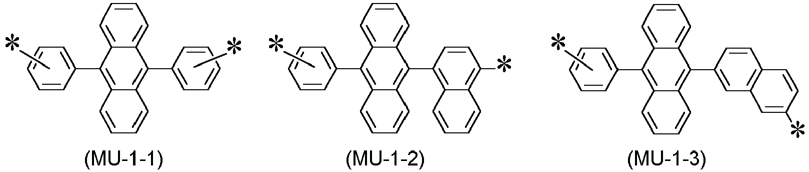

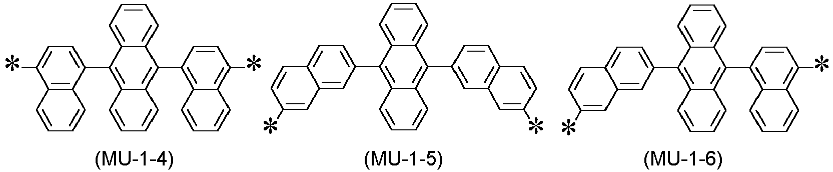

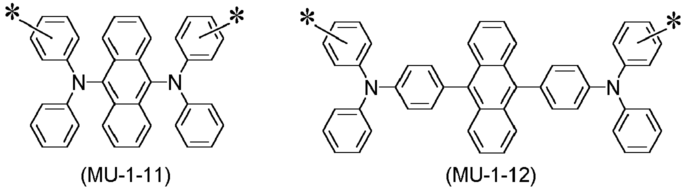

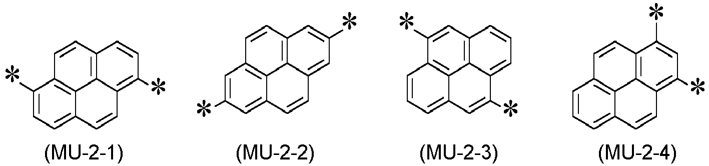

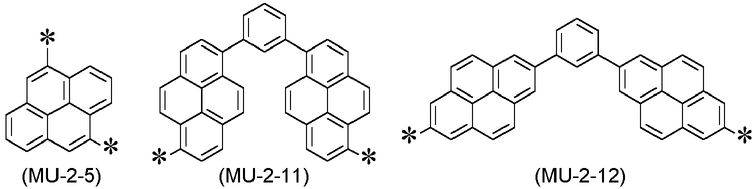

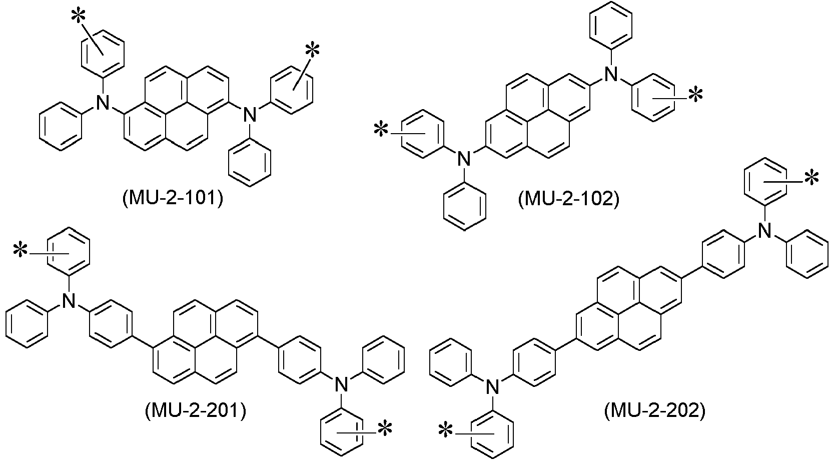

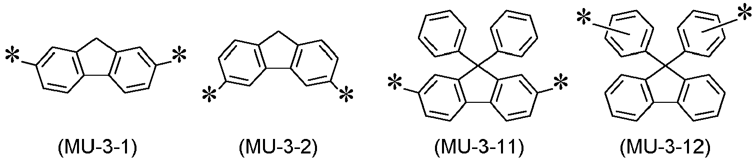

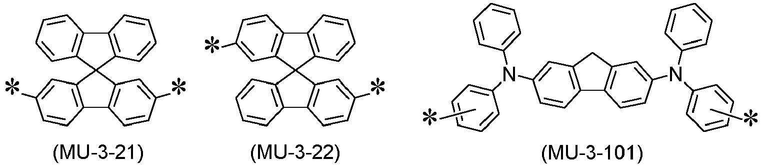

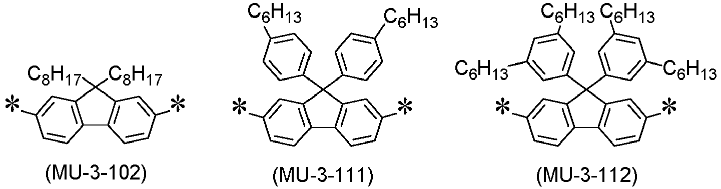

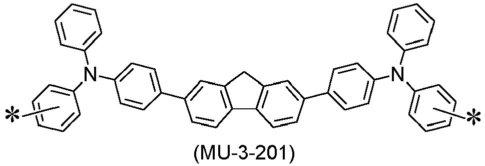

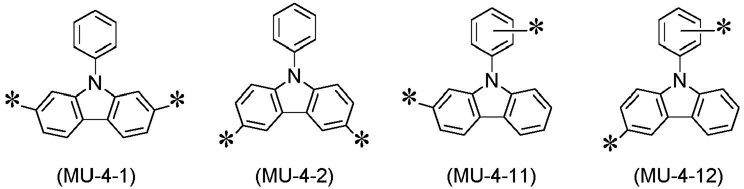

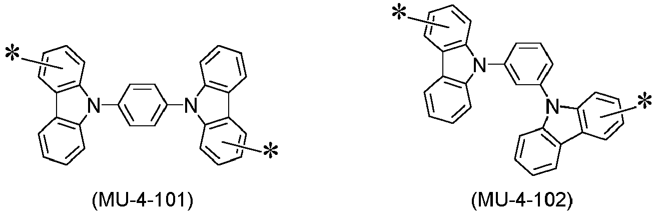

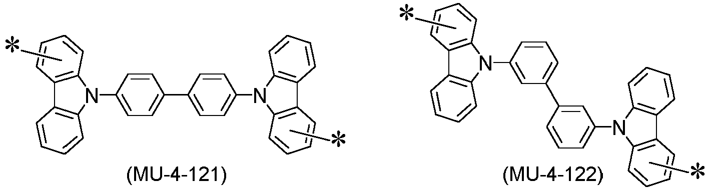

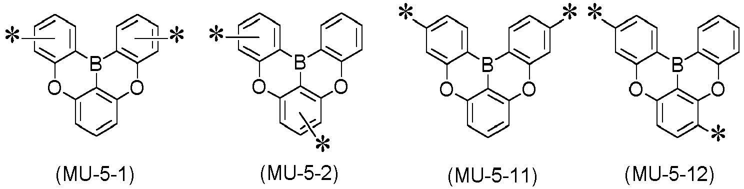

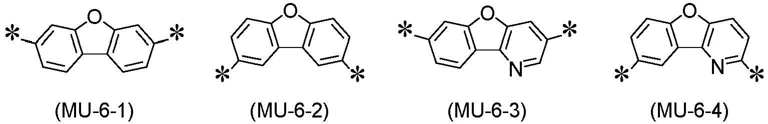

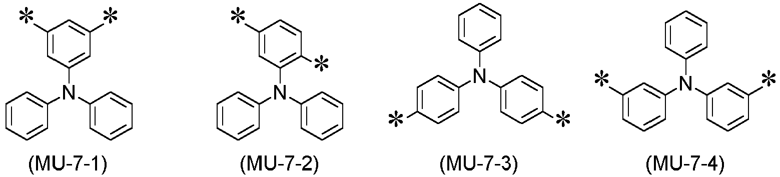

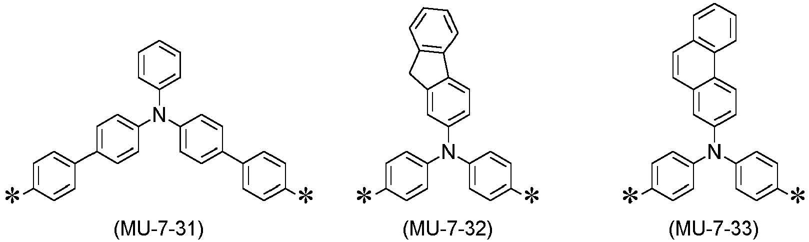

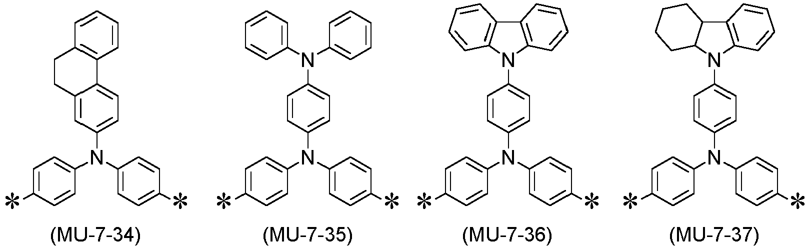

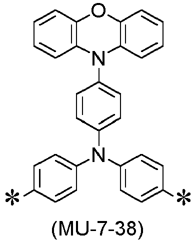

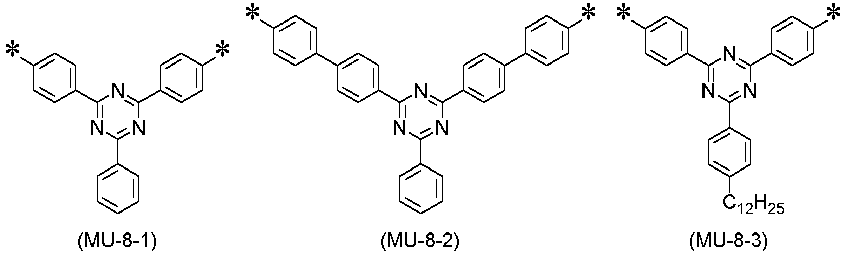

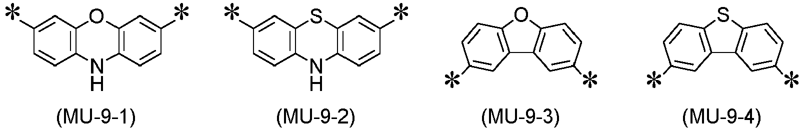

Examples of EC include monovalent groups represented by the following structures. In these structures, EC is bonded to MU at *.

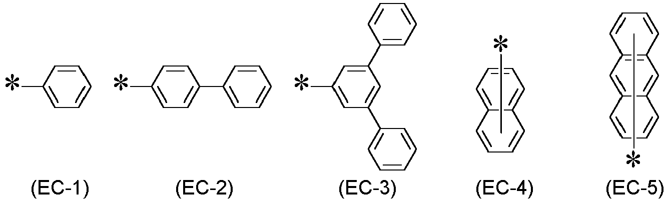

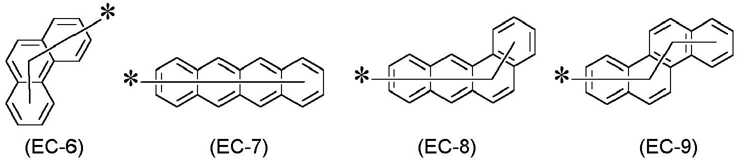

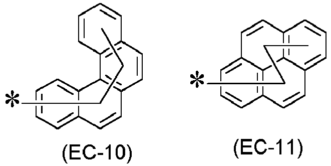

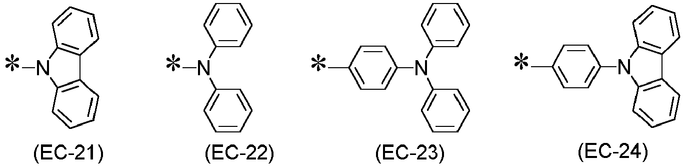

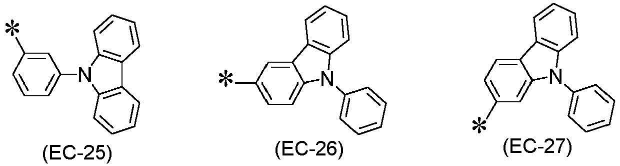

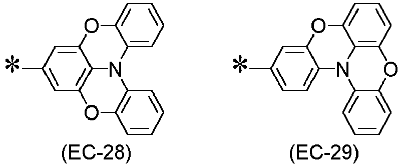

In the compound represented by formula (SPH-1), preferably, 10 to 100% of MU's in the MU total number (k) in a molecule each have an alkyl having 1 to 24 carbon atoms, more preferably, 30 to 100% of MU's in the MU total number (k) in a molecule each have an alkyl having 1 to 18 carbon atoms (branched alkyl having 3 to 18 carbon atoms), and still more preferably, 50 to 100% of MU's in the MU total number (k) in a molecule each have an alkyl having 1 to 12 carbon atoms (branched alkyl having 3 to 12 carbon atoms) from a viewpoint of solubility and coating film formability. Meanwhile, preferably, 10 to 100% of MU's in the MU total number (k) in a molecule each have an alkyl having 7 to 24 carbon atoms, and more preferably, 30 to 100% of MU's in the MU total number (k) in a molecule each have an alkyl having 7 to 24 carbon atoms (branched alkyl having 7 to 24 carbon atoms) from a viewpoint of in-plane orientation and charge transfer.

Details of applications of the polymer compound and the crosslinked polymer will be described later.

### <Electron injection layer and electron transport layer in organic electroluminescent element>

The electron injection layer 107 plays a role of efficiently injecting an electron migrating from the negative electrode 108 into the light emitting layer 105 or the electron transport layer 106. The electron transport layer 106 plays a role of efficiently transporting an electron injected from the negative electrode 108, or an electron injected from the negative electrode 108 through the electron injection layer 107 to the light emitting layer 105. The electron transport layer 106 and the electron injection layer 107 are each formed by laminating and mixing one or more kinds of electron transport/injection materials, or by a mixture of an electron transport/injection material and a polymeric binder.

An electron injection/transport layer is a layer that manages injection of an electron from a negative electrode and transport of an electron, and is preferably a layer that has high electron injection efficiency and can efficiently transport an injected electron. For this purpose, a substance which has high electron affinity, large electron mobility, and excellent stability, and in which impurities that serve as traps are not easily generated at the time of manufacturing and at the time of use, is preferable. However, when a transport balance between a hole and an electron is considered, in a case where the electron injection/transport layer mainly plays a role of efficiently preventing a hole coming from a positive electrode from flowing toward a negative electrode side without being recombined, even if electron transporting ability is not so high, an effect of enhancing luminous efficiency is equal to that of a material having high electron transporting ability. Therefore, the electron injection/transport layer according to the present embodiment may also include a function of a layer that can efficiently prevent migration of a hole.

A material (electron transport material) for forming the electron transport layer 106 or the electron injection layer 107 can be arbitrarily selected for use from compounds conventionally used as electron transfer compounds in a photoconductive material, and known compounds that are used in an electron injection layer and an electron transport layer of an organic EL element.

A material used in an electron transport layer or an electron injection layer preferably includes at least one selected from a compound formed of an aromatic ring or a heteroaromatic ring including one or more kinds of atoms selected from carbon, hydrogen, oxygen, sulfur, silicon, and phosphorus atoms, a pyrrole derivative and a fused ring derivative thereof, and a metal complex having an electron-accepting nitrogen atom. Specific examples of the material include a fused ring-based aromatic ring derivative of naphthalene, anthracene, or the like, a styryl-based aromatic ring derivative represented by 4,4'-bis(diphenylethenyl)biphenyl, a perinone derivative, a coumarin derivative, a naphthalimide derivative, a quinone derivative such as anthraquinone or diphenoquinone, a phosphorus oxide derivative, a carbazole derivative, and an indole derivative. Examples of the metal complex having an electron-accepting nitrogen atom include a hydroxyazole complex such as a hydroxyphenyloxazole complex, an azomethine complex, a tropolone metal complex, a flavonol metal complex, and a benzoquinoline metal complex. These materials are used singly, but may also be used in a mixture with other materials.

Furthermore, specific examples of other electron transfer compounds include a pyridine derivative, a naphthalene derivative, an anthracene derivative, a phenanthroline derivative, a perinone derivative, a coumarin derivative, a naphthalimide derivative, an anthraquinone derivative, a diphenoquinone derivative, a diphenylquinone derivative, a perylene derivative, an oxadiazole derivative (1,3-bis[(4-t-butylphenyl)-1,3,4-oxadiazolyl]phenylene and the like), a thiophene derivative, a triazole derivative (N-naphthyl-2,5-diphenyl-1,3,4-triazole and the like), a thiadiazole derivative, a metal complex of an oxine derivative, a quinolinol-based metal complex, a quinoxaline derivative, a polymer of a quinoxaline derivative, a benzazole compound, a gallium complex, a pyrazole derivative, a perfluorinated phenylene derivative, a triazine derivative, a pyrazine derivative, a benzoquinoline derivative (2,2'-bis(benzo[h]quinolin-2-yl)-9,9'-spirobifluorene and the like), an imidazopyridine derivative, a borane derivative, a benzimidazole derivative (tris(N-phenylbenzimidazol-2-yl)benzene and the like), a benzoxazole derivative, a benzothiazole derivative, a quinoline derivative, an oligopyridine derivative such as terpyridine, a bipyridine derivative, a terpyridine derivative (1,3-bis(4'-(2,2':6'2"-terpyridinyl))benzene and the like), a naphthyridine derivative (bis(1-naphthyl)-4-(1,8-naphthyridin-2-yl)phenylphosphine oxide and the like), an aldazine derivative, a carbazole derivative, an indole derivative, a phosphorus oxide derivative, and a bisstyryl derivative.

Furthermore, a metal complex having an electron-accepting nitrogen atom can also be used, and examples thereof include a quinolinol-based metal complex, a hydroxyazole complex such as a hydroxyphenyloxazole complex, an azomethine complex, a tropolone-metal complex, a flavonol-metal complex, and a benzoquinoline-metal complex.

The materials described above are used singly, but may also be used in a mixture with other materials.

Among the above materials, a borane derivative, a pyridine derivative, a fluoranthene derivative, a BO-based derivative, an anthracene derivative, a benzofluorene derivative, a phosphine oxide derivative, a pyrimidine derivative, a carbazole derivative, a triazine derivative, a benzimidazole derivative, a phenanthroline derivative, and a quinolinol-based metal complex are preferable.

### <Borane derivative>

The borane derivative is, for example, a compound represented by the following general formula (ETM-1), and specifically disclosed in JP 2007-27587 A.

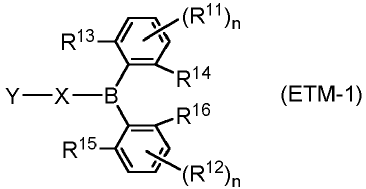

In the above formula (ETM-1), R¹¹ and R¹² each independently represent at least one of a hydrogen atom, an alkyl, a cycloalkyl, an optionally substituted aryl, a substituted silyl, an optionally substituted nitrogen-containing heterocyclic ring, and cyano, R¹³ to R¹⁶ each independently represent an optionally substituted alkyl, an optionally substituted cycloalkyl, or an optionally substituted aryl, X represents an optionally substituted arylene, Y represents an optionally substituted aryl having 16 or fewer carbon atoms, a substituted boryl, or an optionally substituted carbazolyl, and n's each independently represent an integer of 0 to 3. In addition, examples of the substituent in the case of "optionally substituted" or "substituted" include aryl, heteroaryl, alkyl, cycloalkyl and the like.

Among compounds represented by the above general formula (ETM-1), a compound represented by the following general formula (ETM-1-1) and a compound represented by the following general formula (ETM-1-2) are preferable.

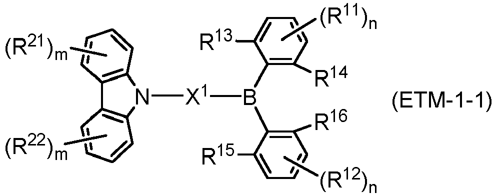

In formula (ETM-1-1), R¹¹ and R¹² each independently represent at least one of a hydrogen atom, an alkyl, a cycloalkyl, an optionally substituted aryl, a substituted silyl, an optionally substituted nitrogen-containing heterocyclic ring, and cyano, R¹³ to R¹⁶ each independently represent an optionally substituted alkyl, an optionally substituted cycloalkyl, or an optionally substituted aryl, R²¹ and R²² each independently represent at least one of a hydrogen atom, an alkyl, a cycloalkyl, an optionally substituted aryl, a substituted silyl, an optionally substituted nitrogen-containing heterocyclic ring, and cyano, X¹ represents an optionally substituted arylene having 20 or fewer carbon atoms, n's each independently represent an integer of 0 to 3, and m's each independently represent an integer of 0 to 4. In addition, examples of the substituent in the case of "optionally substituted" or "substituted" include aryl, heteroaryl, alkyl, cycloalkyl and the like.

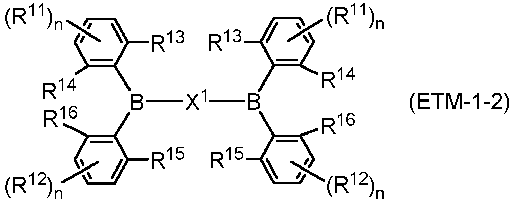

In formula (ETM-1-2), R¹¹ and R¹² each independently represent at least one of a hydrogen atom, an alkyl, a alkyl, an optionally substituted aryl, a substituted silyl, an optionally substituted nitrogen-containing heterocyclic ring, and cyano, R¹³ to R¹⁶ each independently represent an optionally substituted alkyl, an optionally substituted cycloalkyl, or an optionally substituted aryl, X¹ represents an optionally substituted arylene having 20 or fewer carbon atoms, and n's each independently represent an integer of 0 to 3. In addition, examples of the substituent in the case of "optionally substituted" or "substituted" include aryl, heteroaryl, alkyl, cycloalkyl and the like.

Specific examples of X¹ include divalent groups represented by any of the following formulas (X-1) to (X-9). The symbol "*" in each structural formula represents a bonding position. 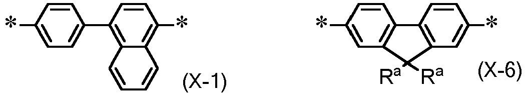 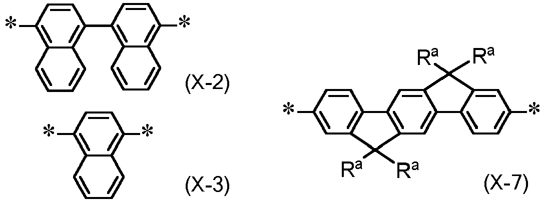 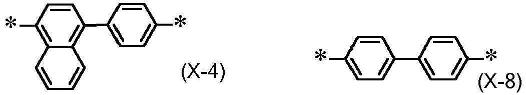 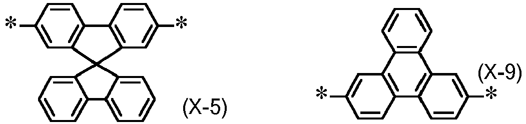 (In each formula, R^{a}'s each independently represent an alkyl group, a cycloalkyl group, or an optionally substituted phenyl group.)

Specific examples of this borane derivative include the following compounds.

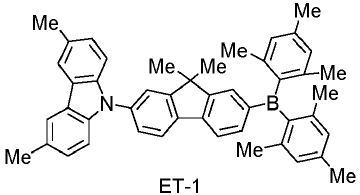

This borane derivative can be manufactured using known raw materials and known synthesis methods.

### <Pyridine derivative>

A pyridine derivative is, for example, a compound represented by the following formula (ETM-2), and preferably a compound represented by formula (ETM-2-1) or (ETM-2-2).

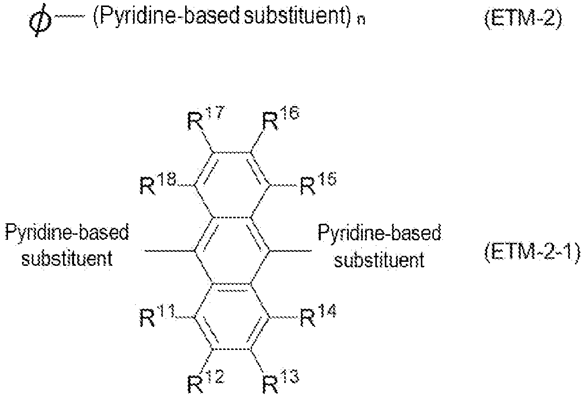

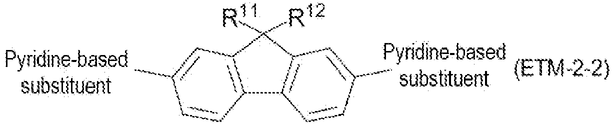

ϕ represents an n-valent aryl ring (preferably, an n-valent benzene ring, naphthalene ring, anthracene ring, fluorene ring, benzofluorene ring, phenalene ring, phenanthrene ring, or triphenylene ring), and n represents an integer of 1 to 4.

In the above formula (ETM-2-1), R¹¹ to R¹⁸ each independently represent a hydrogen atom, an alkyl (preferably, an alkyl having 1 to 24 carbon atoms), a cycloalkyl (preferably, a cycloalkyl having 3 to 12 carbon atoms), or an aryl (preferably, an aryl having 6 to 30 carbon atoms).

In the above formula (ETM-2-2), R¹¹ and R¹² each independently represent a hydrogen atom, an alkyl (preferably, an alkyl having 1 to 24 carbon atoms), a cycloalkyl (preferably, a cycloalkyl having 3 to 12 carbon atoms), or an aryl (preferably, an aryl having 6 to 30 carbon atoms), and R¹¹ and R¹² may be bonded to each other to form a ring.

In each formula, the "pyridine-based substituent" is any one of the following formulas (Py-1) to (Py-15), and the pyridine-based substituents may be each independently substituted by an alkyl having 1 to 4 carbon atoms or a cycloalkyl having 5 to 10 carbon atoms. Furthermore, the pyridine-based substituent may be bonded to ϕ, an anthracene ring, or a fluorene ring in each formula via a phenylene group or a naphthylene group. The symbol "*" in each structural formula represents a bonding position.

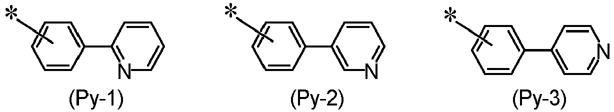

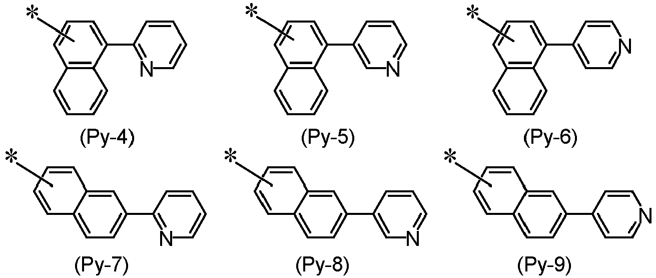

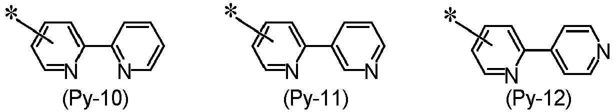

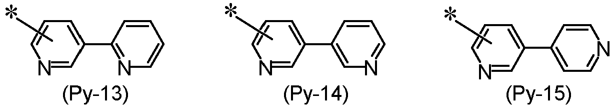

The pyridine-based substituent is any one of the above-formulas (Py-1) to (Py-15). However, among these formulas, the pyridine-based substituent is preferably any one of the following formulas (Py-21) to (Py-44). The symbol "*" in each structural formula represents a bonding position.

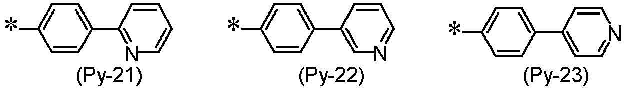

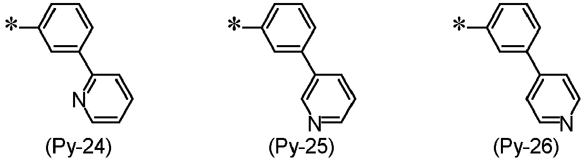

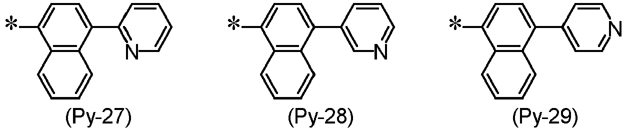

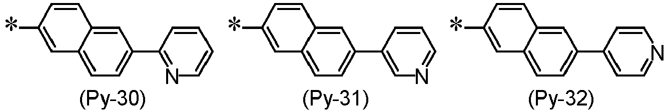

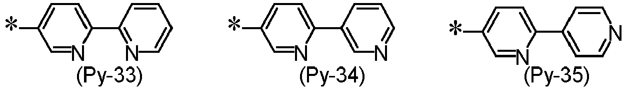

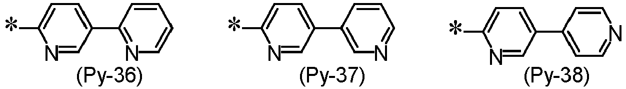

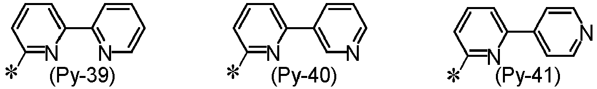

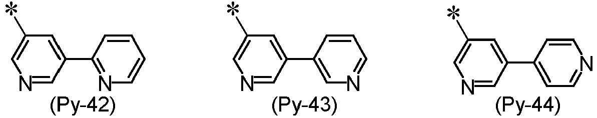

At least one hydrogen atom in each pyridine derivative may be substituted by a deuterium atom. Furthermore, one of the two "pyridine-based substituents" in the above formulas (ETM-2-1) and (ETM-2-2) may be substituted by an aryl.

The "alkyl" in R¹¹ to R¹⁸ may be either linear or branched, and examples thereof include a linear alkyl having 1 to 24 carbon atoms and a branched alkyl having 3 to 24 carbon atoms. A preferable "alkyl" is an alkyl having 1 to 18 carbon atoms (branched alkyl having 3 to 18 carbon atoms). A more preferable "alkyl" is an alkyl having 1 to 12 carbons (branched alkyl having 3 to 12 carbons). A still more preferable "alkyl" is an alkyl having 1 to 6 carbon atoms (branched alkyl having 3 to 6 carbon atoms). A particularly preferable "alkyl" is an alkyl having 1 to 4 carbon atoms (branched alkyl having 3 to 4 carbon atoms).

Specific examples of the "alkyl" include methyl, ethyl, n-propyl, isopropyl, n-butyl, isobutyl, s-butyl, t-butyl, n-pentyl, isopentyl, neopentyl, t-pentyl (t-amyl), n-hexyl, 1-methylpentyl, 3,3-dimethylbutyl, 2-ethylbutyl, n-heptyl, 1-methylhexyl, n-octyl, t-octyl (1,1,3,3-tetramethylbutyl), 1-methylheptyl, 2-ethylhexyl, 2-propylpentyl, n-nonyl, 2,2-dimethylheptyl, 2,6-dimethyl-4-heptyl, 3,5,5-trimethylhexyl, n-decyl, n-undecyl, 1-methyldecyl, n-dodecyl, n-tridecyl, 1-hexylheptyl, n-tetradecyl, n-pentadecyl, n-hexadecyl, n-heptadecyl, n-octadecyl, and n-eicosyl.

Also, for example, 1-ethyl-1-methylpropyl, 1,1-diethylpropyl, 1,1-dimethylbutyl, 1-ethyl-1-methylbutyl, 1,1,4-trimethylpentyl, 1,1,2-trimethylpropyl, 1,1-dimethyloctyl, 1,1-dimethylpentyl, 1,1-dimethylheptyl, 1,1,5-trimethylhexyl, 1-ethyl-1-methylhexyl, 1-ethyl-1,3-dimethylbutyl, 1,1,2,2-tetramethylpropyl, 1-butyl-1-methylpentyl, 1,1-diethylbutyl, 1-ethyl-1-methylpentyl, 1,1,3-trimethylbutyl, 1-propyl-1-methylpentyl, 1,1,2-trimethylpropyl, 1-ethyl-1,2,2-trimethylpropyl, 1-propyl-1-methylbutyl, 1,1-dimethylhexyl and the like can also be mentioned.

As the alkyl having 1 to 4 carbon atoms by which the pyridine-based substituent is substituted, the above description of the alkyl can be cited.

Examples of the "cycloalkyl" in R¹¹ to R¹⁸ include a cycloalkyl having 3 to 12 carbon atoms. A preferable "cycloalkyl" is a cycloalkyl having 3 to 10 carbons. A more preferable "cycloalkyl" is a cycloalkyl having 3 to 8 carbon atoms. A still more preferable "cycloalkyl" is a cycloalkyl having 3 to 6 carbon atoms.

Specific examples of the "cycloalkyl" include cyclopropyl, cyclobutyl, cyclopentyl, cyclohexyl, methylcyclopentyl, cycloheptyl, methylcyclohexyl, cyclooctyl, and dimethylcyclohexyl.

As the "aryl" in R¹¹ to R¹⁸, a preferable aryl is an aryl having 6 to 30 carbon atoms, a more preferable aryl is an aryl having 6 to 18 carbon atoms, a still more preferable aryl is an aryl having 6 to 14 carbon atoms, and a particularly preferable aryl is an aryl having 6 to 12 carbon atoms.

Specific examples of the "aryl having 6 to 30 carbon atoms" include phenyl which is a monocyclic aryl; (1-,2-)naphthyl which is a fused bicyclic aryl; acenaphthylene-(1-,3-,4-,5-)yl, a fluorene-(1-,2-,3-,4-,9-)yl, phenalene-(1-, 2-)yl, and (1-,2-,3-,4-,9-)phenanthryl which are fused tricyclic aryls; triphenylene-(1-, 2-)yl, pyrene-(1- ,2-, 4-)yl, and naphthacene-(1-2-,5-)yl which are fused tetracyclic aryls; and perylene-(1-,2-,3-)yl and pentacene-(1-, 2-, 5-, 6-)yl which are fused pentacyclic aryls.

Preferable examples of the "aryl having 6 to 30 carbon atoms" include a phenyl, a naphthyl, a phenanthryl, a chrysenyl, and a triphenylenyl. More preferable examples thereof include a phenyl, a 1-naphthyl, a 2-naphthyl, and a phenanthryl. Particularly preferable examples thereof include a phenyl, a 1-naphthyl, and a 2-naphthyl.

R¹¹ and R¹² in the above formula (ETM-2-2) may be bonded to each other to form a ring. As a result, cyclobutane, cyclopentane, cyclopentene, cyclopentadiene, cyclohexane, fluorene, indene, or the like may be spiro-bonded to a 5-membered ring of a fluorene skeleton.

Specific examples of this pyridine derivative include the following compounds.

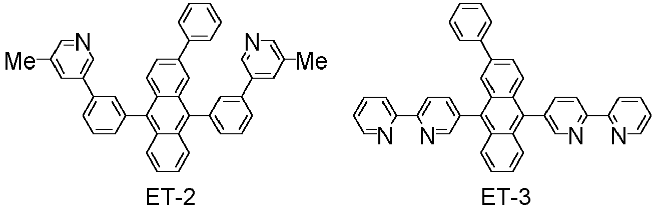

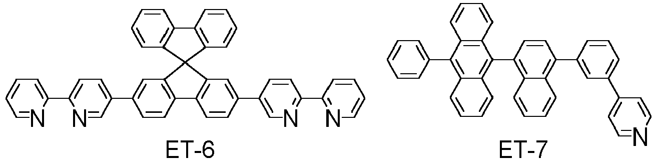

This pyridine derivative can be manufactured using known raw materials and known synthesis methods.

### <Fluoranthene derivative>

The fluoranthene derivative is, for example, a compound represented by the following general formula (ETM-3), and specifically disclosed in WO 2010/134352 A.

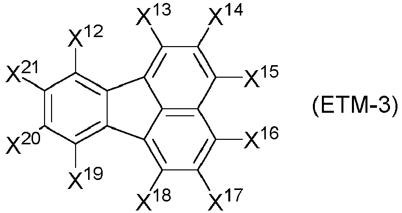

In the above formula (ETM-3), X¹² to X²¹ each represent a hydrogen atom, a halogen atom, a linear, branched or cyclic alkyl, a linear, branched or cyclic alkoxy, a substituted or unsubstituted aryl, or a substituted or unsubstituted heteroaryl. Examples of the substituent when substituted include aryl, heteroaryl, alkyl, cycloalkyl and the like.

Specific examples of this fluoranthene derivative include the following compounds.

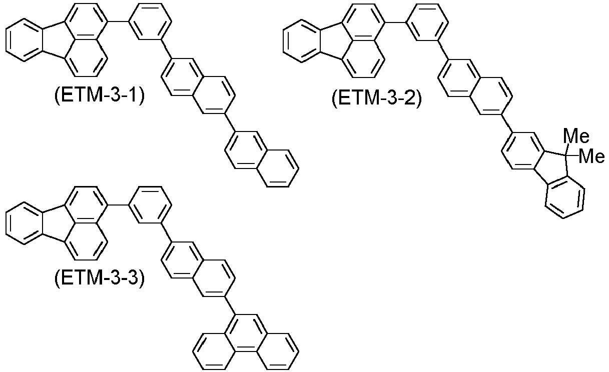

### <BO-based derivative>

The BO-based derivative is, for example, a polycyclic aromatic compound represented by the following formula (ETM-4) or a polycyclic aromatic compound multimer having a plurality of structures represented by the following formula (ETM-4).

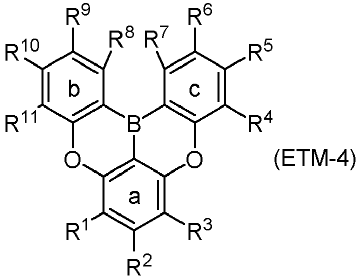

R¹ to R¹¹ each independently represent a hydrogen atom, an aryl, a heteroaryl, a diarylamino, a diheteroarylamino, an arylheteroarylamino, an alkyl, a cycloalkyl, an alkoxy, or an aryloxy, while at least one hydrogen atom in these may be substituted by an aryl, a heteroaryl, an alkyl, or a cycloalkyl.

Furthermore, adjacent groups among R¹ to R1¹ may be bonded to each other to form an aryl ring or a heteroaryl ring together with the ring a, ring b, or ring c, and at least one hydrogen atom in the ring thus formed may be substituted by an aryl, a heteroaryl, a diarylamino, a diheteroarylamino, an arylheteroarylamino, an alkyl, a cycloalkyl, an alkoxy, or an aryloxy, while at least one hydrogen atom in these may be substituted by an aryl, a heteroaryl, an alkyl, or a cycloalkyl.

Furthermore, at least one hydrogen atom in a compound or structure represented by formula (ETM-4) may be substituted by a halogen atom or a deuterium atom.

For description of a substituent in formula (ETM-4), a form of ring formation, the description of the polycyclic aromatic compound represented by the above general formula (1) or (2) can be cited.

Specific examples of this BO-based derivative include the following compounds.

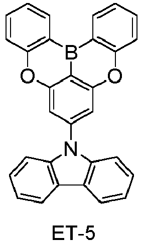

This BO-based derivative can be manufactured using known raw materials and known synthesis methods.

### <Anthracene derivative>

One of the anthracene derivatives is, for example, a compound represented by the following formula (ETM-5-1).

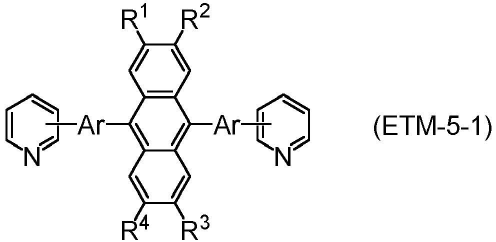

Ar's each independently represent a divalent benzene or naphthalene, R¹ to R⁴ each independently represent a hydrogen atom, an alkyl having 1 to 6 carbon atoms, a cycloalkyl having 3 to 6 carbon atoms, or an aryl having 6 to 20 carbon atoms.

Ar's can be each independently selected from a divalent benzene and naphthalene appropriately. Two Ar's may be different from or the same as each other, but are preferably the same from a viewpoint of easiness of synthesis of an anthracene derivative. Ar is bonded to pyridine to form "a moiety formed of Ar and pyridine". For example, this moiety is bonded to anthracene as a group represented by any one of the following formulas (Py-1) to (Py-12). The symbol "*" in each structural formula represents a bonding position.

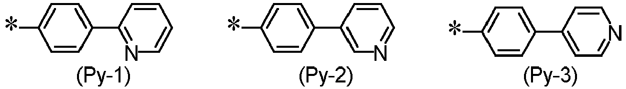

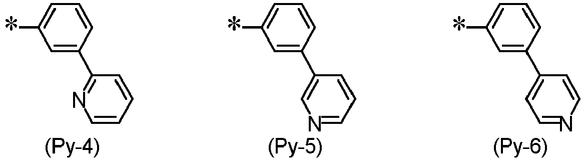

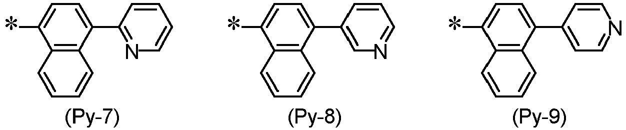

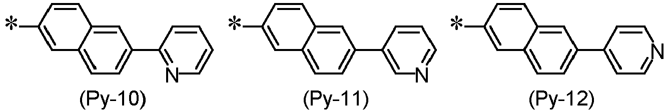

Among these groups, a group represented by any one of the above formulas (Py-1) to (Py-9) is preferable, and a group represented by any one of the above formulas (Py-1) to (Py-6) is more preferable. Two "moieties formed of Ar and pyridine" bonded to anthracene may have the same structure as or different structures from each other, but preferably have the same structure from a viewpoint of easiness of synthesis of an anthracene derivative. However, two "moieties formed of Ar and pyridine" preferably have the same structure or different structures from a viewpoint of element characteristics.

The alkyl having 1 to 6 carbon atoms in R¹ to R⁴ may be either linear or branched. That is, the alkyl having 1 to 6 carbon atoms is a linear alkyl having 1 to 6 carbon atoms or a branched alkyl having 3 to 6 carbon atoms. More preferably, the alkyl having 1 to 6 carbon atoms is an alkyl having 1 to 4 carbon atoms (branched alkyl having 3 to 4 carbon atoms). Specific examples thereof include methyl, ethyl, n-propyl, isopropyl, n-butyl, isobutyl, s-butyl, t-butyl, n-pentyl, isopentyl, neopentyl, t-pentyl (t-amyl), n-hexyl, 1-methylpentyl, 3,3-dimethylbutyl, and 2-ethylbutyl. Methyl, ethyl, n-propyl, isopropyl, n-butyl, isobutyl, s-butyl, and t-butyl are preferable. Methyl, ethyl, and t-butyl are more preferable.

Specific examples of the cycloalkyl having 3 to 6 carbon atoms in R¹ to R⁴ include cyclopropyl, cyclobutyl, cyclopentyl, cyclohexyl, methylcyclopentyl, cycloheptyl, methylcyclohexyl, cyclooctyl, and dimethylcyclohexyl.

For the aryl having 6 to 20 carbon atoms in R¹ to R⁴, an aryl having 6 to 16 carbon atoms is preferable, an aryl having 6 to 12 carbon atoms is more preferable, and an aryl having 6 to 10 carbon atoms is particularly preferable.

Specific examples of the "aryl having 6 to 20 carbon atoms" include phenyl, (o-, m-, p-) tolyl, (2,3-, 2,4-, 2,5-, 2,6-, 3,4-, 3,5-) xylyl, mesityl (2,4,6-trimethylphenyl), and (o-, m-, p-)cumenyl which are monocyclic aryls; (2-, 3-, 4-)biphenylyl which is a bicyclic aryl; (1-, 2-)naphthyl which is a fused bicyclic aryl; terphenylyl (m-terphenyl-2'-yl, m-terphenyl-4'-yl, m-terphenyl-5'-yl, o-terphenyl-3'-yl, o-terphenyl-4'-yl, p-terphenyl-2'-yl, m-terphenyl-2-yl, m-terphenyl-3-yl, m-terphenyl-4-yl, o-terphenyl-2-yl, o-terphenyl-3-yl, o-terphenyl-4-yl, p-terphenyl-2-yl, p-terphenyl-3-yl, p-terphenyl-4-yl) which is a tricyclic aryl; anthracene-(1-, 2-, 9-)yl, acenaphthylene-(1-, 3-, 4-, 5-)yl, fluorene-(1-, 2-, 3-, 4-, 9-)yl, phenalene-(1-, 2-)yl, and (1-, 2-, 3-, 4-, 9-)phenanthryl which are fused tricyclic aryls; triphenylene-(1-, 2-)yl, pyrene-(1-, 2-, 4-)yl, and tetracene-(1-, 2-, 5-)yl which are fused tetracyclic aryls; and perylene-(1-, 2-, 3-)yl which is a fused pentacyclic aryl.

The "aryl having 6 to 20 carbon atoms" is preferably a phenyl, a biphenylyl, a terphenylyl, or a naphthyl, more preferably a phenyl, a biphenylyl, a 1-naphthyl, a 2-naphthyl, or an m-terphenyl-5'-yl, still more preferably a phenyl, a biphenylyl, a 1-naphthyl, or a 2-naphthyl, and most preferably a phenyl.

One of the anthracene derivatives is, for example, a compound represented by the following formula (ETM-5-2).

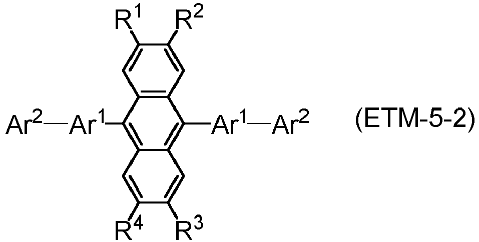

Ar¹'s each independently represent a single bond, a divalent benzene, naphthalene, anthracene, fluorene, or phenalene.

Ar²'s each independently represent an aryl having 6 to 20 carbon atoms. The same description as the "aryl having 6 to 20 carbon atoms" in the above formula (ETM-5-1) can be cited. An aryl having 6 to 16 carbon atoms is preferable, an aryl having 6 to 12 carbon atoms is more preferable, and an aryl having 6 to 10 carbon atoms is particularly preferable. Specific examples thereof include phenyl, biphenylyl, naphthyl, terphenylyl, anthracenyl, acenaphthylenyl, fluorenyl, phenalenyl, phenanthryl, triphenylenyl, pyrenyl, tetracenyl, and perylenyl.

R¹ to R⁴ each independently represent a hydrogen atom, an alkyl having 1 to 6 carbon atoms, a cycloalkyl having 3 to 6 carbon atoms, or an aryl having 6 to 20 carbon atoms. The same description as in the above formula (ETM-5-1) can be cited.

Specific examples of these anthracene derivatives include the following compounds.

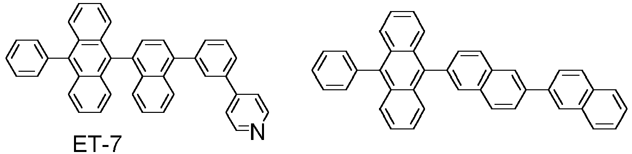

These anthracene derivatives can be manufactured using known raw materials and known synthesis methods.

### <Benzofluorene derivative>

The benzofluorene derivative is, for example, a compound represented by the following formula (ETM-6).

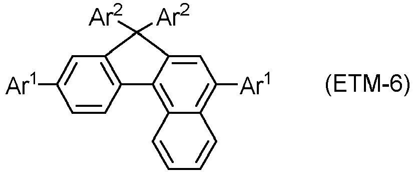

Ar¹'s each independently represent an aryl having 6 to 20 carbon atoms. The same description as the "aryl having 6 to 20 carbon atoms" in the above formula (ETM-5-1) can be cited. An aryl having 6 to 16 carbon atoms is preferable, an aryl having 6 to 12 carbon atoms is more preferable, and an aryl having 6 to 10 carbon atoms is particularly preferable. Specific examples thereof include phenyl, biphenylyl, naphthyl, terphenylyl, anthracenyl, acenaphthylenyl, fluorenyl, phenalenyl, phenanthryl, triphenylenyl, pyrenyl, tetracenyl, and perylenyl.

Ar²'s each independently represent a hydrogen atom, an alkyl (preferably, an alkyl having 1 to 24 carbon atoms), a cycloalkyl (preferably, a cycloalkyl having 3 to 12 carbon atoms), or an aryl (preferably, an aryl having 6 to 30 carbon atoms), and two Ar²'s may be bonded to each other to form a ring.

The "alkyl" in Ar² may be either linear or branched, and examples thereof include a linear alkyl having 1 to 24 carbon atoms and a branched alkyl having 3 to 24 carbon atoms. A preferable "alkyl" is an alkyl having 1 to 18 carbon atoms (branched alkyl having 3 to 18 carbon atoms). A more preferable "alkyl" is an alkyl having 1 to 12 carbons (branched alkyl having 3 to 12 carbons). A still more preferable "alkyl" is an alkyl having 1 to 6 carbon atoms (branched alkyl having 3 to 6 carbon atoms). A particularly preferable "alkyl" is an alkyl having 1 to 4 carbon atoms (branched alkyl having 3 to 4 carbon atoms). Specific examples of the "alkyl" include methyl, ethyl, n-propyl, isopropyl, n-butyl, isobutyl, s-butyl, t-butyl, n-pentyl, isopentyl, neopentyl, t-pentyl (t-amyl), n-hexyl, 1-methylpentyl, 3,3-dimethylbutyl, 2-ethylbutyl, n-heptyl, and 1-methylhexyl.

Examples of the "cycloalkyl" in Ar² include a cycloalkyl having 3 to 12 carbon atoms. A preferable "cycloalkyl" is a cycloalkyl having 3 to 10 carbons. A more preferable "cycloalkyl" is a cycloalkyl having 3 to 8 carbon atoms. A still more preferable "cycloalkyl" is a cycloalkyl having 3 to 6 carbon atoms. Specific examples of the "cycloalkyl" include cyclopropyl, cyclobutyl, cyclopentyl, cyclohexyl, methylcyclopentyl, cycloheptyl, methylcyclohexyl, cyclooctyl, and dimethylcyclohexyl.

As the "aryl" in Ar², a preferable aryl is an aryl having 6 to 30 carbon atoms, a more preferable aryl is an aryl having 6 to 18 carbon atoms, a still more preferable aryl is an aryl having 6 to 14 carbon atoms, and a particularly preferable aryl is an aryl having 6 to 12 carbon atoms.

Specific examples of the "aryl having 6 to 30 carbon atoms" include phenyl, naphthyl, acenaphthylenyl, fluorenyl, phenalenyl, phenanthryl, triphenylenyl, pyrenyl, naphthacenyl, perylenyl, and pentacenyl.

Two Ar²'s may be bonded to each other to form a ring. As a result, cyclobutane, cyclopentane, cyclopentene, cyclopentadiene, cyclohexane, fluorene, indene, or the like may be spiro-bonded to a 5-membered ring of a fluorene skeleton.

Specific examples of this benzofluorene derivative include the following compounds.

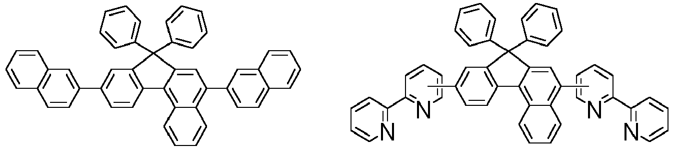

This benzofluorene derivative can be manufactured using known raw materials and known synthesis methods.

### <Phosphine oxide derivative>

The phosphine oxide derivative is, for example, a compound represented by the following formula (ETM-7-1). Details are also described in WO 2013/079217 A. 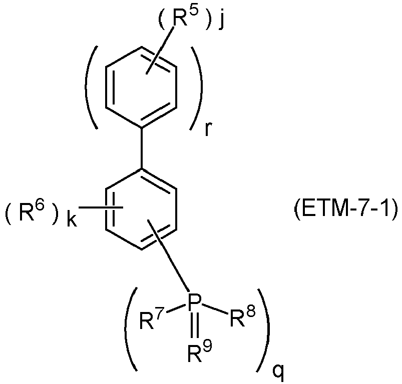
R⁵ represents a substituted or unsubstituted, alkyl having 1 to 20 carbon atoms, cycloalkyl having 3 to 16 carbon atoms, aryl having 6 to 20 carbon atoms, or heteroaryl having 5 to 20 carbon atoms, R⁶ represents CN, a substituted or unsubstituted, alkyl having 1 to 20 carbons, cycloalkyl having 3 to 16 carbon atoms, heteroalkyl having 1 to 20 carbons, aryl having 6 to 20 carbons, heteroaryl having 5 to 20 carbons, alkoxy having 1 to 20 carbons, or aryloxy having 6 to 20 carbon atoms,
R⁷ and R⁸ each independently represent a substituted or unsubstituted, aryl having 6 to 20 carbon atoms or heteroaryl having 5 to 20 carbon atoms,
R⁹ represents an oxygen atom or a sulfur atom,
j represents 0 or 1, k represents 0 or 1, r represents an integer of 0 to 4, and q represents an integer of 1 to 3.

Examples of the substituent when substituted includes aryl, heteroaryl, alkyl, cycloalkyl and the like.

The phosphine oxide derivative may be, for example, a compound represented by the following formula (ETM-7-2).

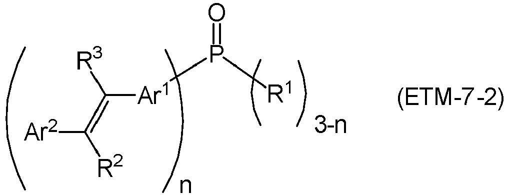

R¹ to R³ may be the same as or different from each other and are selected from a hydrogen atom, an alkyl group, a cycloalkyl group, an aralkyl group, an alkenyl group, a cycloalkenyl group, an alkynyl group, an alkoxy group, an alkylthio group, a cycloalkylthio group, an aryl ether group, an aryl thioether group, an aryl group, a heterocyclic group, a halogen atom, cyano group, an aldehyde group, a carbonyl group, a carboxyl group, an amino group, a nitro group, a silyl group, and a fused ring formed with an adjacent substituent.

Ar¹'s may be the same as or different from each other, and represents an arylene group or a heteroarylene group. Ar²'s may be the same as or different from each other, and represents an aryl group or a heteroaryl group. However, at least one of Ar¹ and Ar² has a substituent or forms a fused ring with an adjacent substituent. n represents an integer of 0 to 3. When n is 0, no unsaturated structure portion is present. When n is 3, R¹ is not present.

Among these substituents, the alkyl group represents a saturated aliphatic hydrocarbon group such as a methyl group, an ethyl group, a propyl group, or a butyl group. This saturated aliphatic hydrocarbon group may be unsubstituted or substituted. The substituent in a case of being substituted is not particularly limited, and examples thereof include an alkyl group, an aryl group, and a heterocyclic group, and this point is also common to the following description. Furthermore, the number of carbon atoms in the alkyl group is not particularly limited, but is usually in a range of 1 to 20 from a viewpoint of availability and cost.

Furthermore, the cycloalkyl group represents a saturated alicyclic hydrocarbon group such as cyclopropyl, cyclohexyl, norbornyl, or adamantyl. This saturated alicyclic hydrocarbon group may be unsubstituted or substituted. The carbon number of the alkyl group moiety is not particularly limited, but is usually in a range of 3 to 20.

Furthermore, the aralkyl group represents an aromatic hydrocarbon group via an aliphatic hydrocarbon, such as a benzyl group or a phenylethyl group. Both the aliphatic hydrocarbon and the aromatic hydrocarbon may be unsubstituted or substituted. The carbon number of the aliphatic moiety is not particularly limited, but is usually in a range of 1 to 20.

Furthermore, the alkenyl group represents an unsaturated aliphatic hydrocarbon group containing a double bond, such as a vinyl group, an allyl group, or a butadienyl group. This unsaturated aliphatic hydrocarbon group may be unsubstituted or substituted. The carbon number of the alkenyl group is not particularly limited, but is usually in a range of 2 to 20.

Furthermore, the cycloalkenyl group represents an unsaturated alicyclic hydrocarbon group containing a double bond, such as a cyclopentenyl group, a cyclopentadienyl group, or a cyclohexene group. This unsaturated alicyclic hydrocarbon group may be unsubstituted or substituted.

Furthermore, the alkynyl group represents an unsaturated aliphatic hydrocarbon group containing a triple bond, such as an acetylenyl group. This unsaturated aliphatic hydrocarbon group may be unsubstituted or substituted. The carbon number of the alkynyl group is not particularly limited, but is usually in a range of 2 to 20.

Furthermore, the alkoxy group represents an aliphatic hydrocarbon group via an ether bond, such as a methoxy group. The aliphatic hydrocarbon group may be unsubstituted or substituted. The carbon number of the alkoxy group is not particularly limited, but is usually in a range of 1 to 20.

Furthermore, the alkylthio group is a group in which an oxygen atom of an ether bond of an alkoxy group is substituted by a sulfur atom.

Furthermore, the cycloalkylthio group is a group in which an oxygen atom of an ether bond of an cycloalkoxy group is substituted by a sulfur atom.

Furthermore, the aryl ether group represents an aromatic hydrocarbon group via an ether bond, such as a phenoxy group. The aromatic hydrocarbon group may be unsubstituted or substituted. The carbon number of the aryl ether group is not particularly limited, but is usually in a range of 6 to 40.

Furthermore, the aryl thioether group is a group in which an oxygen atom of an ether bond of an aryl ether group is substituted by a sulfur atom.

Furthermore, the aryl group represents an aromatic hydrocarbon group such as a phenyl group, a naphthyl group, a biphenylyl group, a phenanthryl group, a terphenyl group, or a pyrenyl group. The aryl group may be unsubstituted or substituted. The carbon number of the aryl group is not particularly limited, but is usually in a range of 6 to 40.

Furthermore, the heterocyclic group represents a cyclic structural group having an atom other than a carbon atom, such as a furanyl group, a thiophenyl group, an oxazolyl group, a pyridyl group, a quinolinyl group, or a carbazolyl group. This cyclic structural group may be unsubstituted or substituted. The carbon number of the heterocyclic group is not particularly limited, but is usually in a range of 2 to 30.

Halogen refers to fluorine, chlorine, bromine, and iodine.

The aldehyde group, the carbonyl group, and the amino group can include a group substituted by an aliphatic hydrocarbon, an alicyclic hydrocarbon, an aromatic hydrocarbon, a heterocyclic ring, or the like.

Furthermore, the aliphatic hydrocarbon, the alicyclic hydrocarbon, the aromatic hydrocarbon, and the heterocyclic ring may be unsubstituted or substituted.

The silyl group represents, for example, a silicon compound group such as a trimethylsilyl group. This silicon compound group may be unsubstituted or substituted. The number of carbon atoms of the silyl group is not particularly limited, but is usually in a range of 3 to 20. Furthermore, the number of silicon atoms is usually 1 to 6.

The fused ring formed with an adjacent substituent is, for example, a conjugated or unconjugated fused ring formed between Ar¹ and R², Ar¹ and R³, Ar² and R², Ar² and R³, R² and R³, or Ar¹ and Ar². Here, when n is 1, two R¹'s may form a conjugated or unconjugated fused ring. These fused rings may contain a nitrogen atom, an oxygen atom, or a sulfur atom in the ring structure, or may be fused with another ring.

Specific examples of this phosphine oxide derivative include the following compounds.

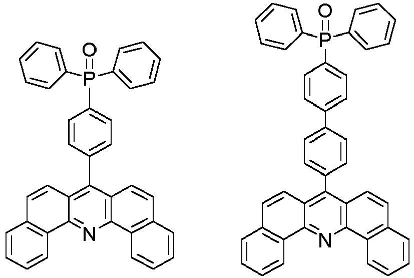

This phosphine oxide derivative can be manufactured using known raw materials and known synthesis methods.

### <Pyrimidine derivative>

The pyrimidine derivative is, for example, a compound represented by the following formula (ETM-8), and preferably a compound represented by the following formula (ETM-8-1). Details are also described in WO 2011/021689 A.

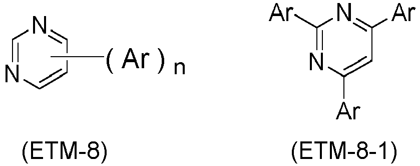

Ar's each independently represent an optionally substituted aryl or an optionally substituted heteroaryl. n represents an integer of 1 to 4, preferably an integer of 1 to 3, and more preferably 2 or 3.

Examples of the "aryl" as the "optionally substituted aryl" include an aryl having 6 to 30 carbon atoms. An aryl having 6 to 24 carbon atoms is preferable, an aryl having 6 to 20 carbon atoms is more preferable, and an aryl having 6 to 12 carbon atoms is still more preferable.

Specific examples of the "aryl" include phenyl which is a monocyclic aryl; (2-, 3-, 4-)biphenylyl which is a bicyclic aryl; (1-, 2-)naphthyl which is a fused bicyclic aryl; terphenylyl (m-terphenyl-2'-yl, m-terphenyl-4'-yl, m-terphenyl-5'-yl, o-terphenyl-3'-yl, o-terphenyl-4'-yl, p-terphenyl-2'-yl, m-terphenyl-2-yl, m-terphenyl-3-yl, m-terphenyl-4-yl, o-terphenyl-2-yl, o-terphenyl-3-yl, o-terphenyl-4-yl, p-terphenyl-2-yl, p-terphenyl-3-yl, p-terphenyl-4-yl) which is a tricyclic aryl; acenaphthylene-(1-, 3-, 4-, 5-)yl, fluorene-(1-, 2-, 3-, 4-, 9-)yl, phenalene-(1-, 2-)yl, and (1-, 2-, 3-, 4-, 9-)phenanthryl which are fused tricyclic aryls; quaterphenylyl-(5'-phenyl-m-terphenyl-2-yl, 5'-phenyl-m-terphenyl-3-yl, 5'-phenyl-m-terphenyl-4-yl, m-quaterphenylyl) which is a tetracyclic aryl; triphenylene-(1-, 2-)yl, pyrene-(1-, 2-, 4-)yl, and naphthacene-(1-, 2-, 5-)yl which are fused tetracyclic aryls; and perylene-(1-, 2-, 3-)yl and pentacene-(1-, 2-, 5-, 6-)yl which are fused pentacyclic aryls.

Examples of the "heteroaryl" as the "optionally substituted heteroaryl" include a heteroaryl having 2 to 30 carbon atoms. A heteroaryl having 2 to 25 carbon atoms is preferable, a heteroaryl having 2 to 20 carbon atoms is more preferable, a heteroaryl having 2 to 15 carbon atoms is still more preferable, and a heteroaryl having 2 to 10 carbon atoms is particularly preferable. Furthermore, examples of the "heteroaryl" include a heterocyclic ring containing 1 to 5 heteroatoms selected from an oxygen atom, a sulfur atom, and a nitrogen atom in addition to a carbon atom as a ring-constituting atom.

Specific examples of the "heteroaryl" include pyrrolyl, oxazolyl, isoxazolyl, thiazolyl, isothiazolyl, imidazolyl, oxadiazolyl, thiadiazolyl, triazolyl, tetrazolyl, pyrazolyl, pyridyl, pyrimidinyl, pyridazinyl, pyrazinyl, triazinyl, indolyl, isoindolyl, 1H-indazolyl, benzoimidazolyl, benzoxazolyl, benzothiazolyl, 1H-benzotriazolyl, quinolyl, isoquinolyl, cinnolyl, quinazolyl, quinoxalinyl, phthalazinyl, naphthyridinyl, purinyl, pteridinyl, carbazolyl, acridinyl, phenoxathiinyl, phenoxazinyl, phenothiazinyl, phenazinyl, phenazasilinyl, indolizinyl, furanyl, benzofuranyl, isobenzofuranyl, dibenzofuranyl, naphthobenzofuranyl, thiophenyl, benzothiophenyl, isobenzothiophenyl, dibenzothiophenyl, naphthobenzothiophenyl, benzophospholyl, dibenzophospholyl, monovalent group of benzophosphole oxide ring, monovalent group of dibenzophosphole oxide ring, furazanyl, thianthrenyl, indolocarbazolyl, benzoindolocarbazolyl, and benzobenzoindolocarbazolyl.

Furthermore, the above aryl and heteroaryl may be substituted, and may be each substituted by, for example, the above aryl or heteroaryl.

Specific examples of this pyrimidine derivative include the following compounds.

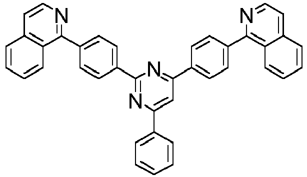

This pyrimidine derivative can be manufactured using known raw materials and known synthesis methods.

### <Carbazole derivative>

The carbazole derivative is, for example, a compound represented by the following formula (ETM-9), or a multimer obtained by bonding a plurality of the compounds with a single bond or the like. Details are described in US 2014/0197/386 A.

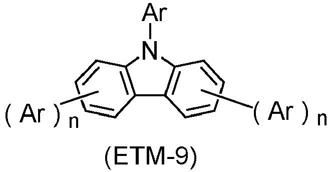

Ar's each independently represent an optionally substituted aryl or an optionally substituted heteroaryl. n each independently represents an integer of 0 to 4, preferably an integer of 0 to 3, and more preferably 0 or 1.

Examples of the "aryl" as the "optionally substituted aryl" include an aryl having 6 to 30 carbon atoms. An aryl having 6 to 24 carbon atoms is preferable, an aryl having 6 to 20 carbon atoms is more preferable, and an aryl having 6 to 12 carbon atoms is still more preferable.

Specific examples of the "aryl" include phenyl which is a monocyclic aryl; (2-, 3-, 4-)biphenylyl which is a bicyclic aryl; (1-, 2-)naphthyl which is a fused bicyclic aryl; terphenylyl (m-terphenyl-2'-yl, m-terphenyl-4'-yl, m-terphenyl-5'-yl, o-terphenyl-3'-yl, o-terphenyl-4'-yl, p-terphenyl-2'-yl, m-terphenyl-2-yl, m-terphenyl-3-yl, m-terphenyl-4-yl, o-terphenyl-2-yl, o-terphenyl-3-yl, o-terphenyl-4-yl, p-terphenyl-2-yl, p-terphenyl-3-yl, p-terphenyl-4-yl) which is a tricyclic aryl; acenaphthylene-(1-, 3-, 4-, 5-)yl, fluorene-(1-, 2-, 3-, 4-, 9-)yl, phenalene-(1-, 2-)yl, and (1-, 2-, 3-, 4-, 9-)phenanthryl which are fused tricyclic aryls; quaterphenylyl-(5'-phenyl-m-terphenyl-2-yl, 5'-phenyl-m-terphenyl-3-yl, 5'-phenyl-m-terphenyl-4-yl, m-quaterphenylyl) which is a tetracyclic aryl; triphenylene-(1-, 2-)yl, pyrene-(1-, 2-, 4-)yl, and naphthacene-(1-, 2-, 5-)yl which are fused tetracyclic aryls; and perylene-(1-, 2-, 3-)yl and pentacene-(1-, 2-, 5-, 6-)yl which are fused pentacyclic aryls.

Examples of the "heteroaryl" as the "optionally substituted heteroaryl" include a heteroaryl having 2 to 30 carbon atoms. A heteroaryl having 2 to 25 carbon atoms is preferable, a heteroaryl having 2 to 20 carbon atoms is more preferable, a heteroaryl having 2 to 15 carbon atoms is still more preferable, and a heteroaryl having 2 to 10 carbon atoms is particularly preferable. Furthermore, examples of the "heteroaryl" include a heterocyclic ring containing 1 to 5 heteroatoms selected from an oxygen atom, a sulfur atom, and a nitrogen atom in addition to a carbon atom as a ring-constituting atom.

Specific examples of the "heteroaryl" include pyrrolyl, oxazolyl, isoxazolyl, thiazolyl, isothiazolyl, imidazolyl, oxadiazolyl, thiadiazolyl, triazolyl, tetrazolyl, pyrazolyl, pyridyl, pyrimidinyl, pyridazinyl, pyrazinyl, triazinyl, indolyl, isoindolyl, 1H-indazolyl, benzoimidazolyl, benzoxazolyl, benzothiazolyl, 1H-benzotriazolyl, quinolyl, isoquinolyl, cinnolyl, quinazolyl, quinoxalinyl, phthalazinyl, naphthyridinyl, purinyl, pteridinyl, carbazolyl, acridinyl, phenoxathiinyl, phenoxazinyl, phenothiazinyl, phenazinyl, phenazasilinyl, indolizinyl, furanyl, benzofuranyl, isobenzofuranyl, dibenzofuranyl, naphthobenzofuranyl, thiophenyl, benzothiophenyl, isobenzothiophenyl, dibenzothiophenyl, naphthobenzothiophenyl, benzophospholyl, dibenzophospholyl, monovalent group of benzophosphole oxide ring, monovalent group of dibenzophosphole oxide ring, furazanyl, thianthrenyl, indolocarbazolyl, benzoindolocarbazolyl, and benzobenzoindolocarbazolyl.

Furthermore, the above aryl and heteroaryl may be substituted, and may be each substituted by, for example, the above aryl or heteroaryl.

The carbazole derivative may be a multimer obtained by bonding a plurality of compounds represented by the above formula (ETM-9) with a single bond or the like. In this case, the compounds may be bonded with an aryl ring (preferably, a polyvalent benzene ring, naphthalene ring, anthracene ring, fluorene ring, benzofluorene ring, phenalene ring, phenanthrene ring or triphenylene ring) in addition to a single bond.

Specific examples of this carbazole derivative include the following compounds.

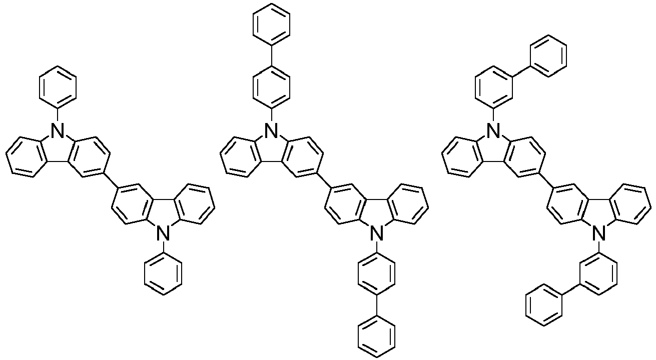

This carbazole derivative can be manufactured using known raw materials and known synthesis methods.

### <Triazine derivative>

The triazine derivative is, for example, a compound represented by the following formula (ETM-10), and preferably a compound represented by the following formula (ETM-10-1). Details are described in US 2011/0156013 A.

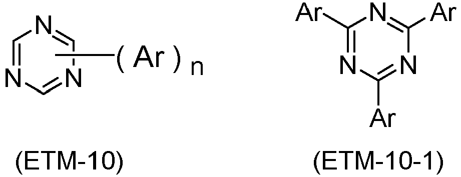

Ar's each independently represent an optionally substituted aryl or an optionally substituted heteroaryl. n each independently represents an integer of 1 to 3, preferably 2 or 3.

Examples of the "aryl" as the "optionally substituted aryl" include an aryl having 6 to 30 carbon atoms. An aryl having 6 to 24 carbon atoms is preferable, an aryl having 6 to 20 carbon atoms is more preferable, and an aryl having 6 to 12 carbon atoms is still more preferable.

Specific examples of the "aryl" include phenyl which is a monocyclic aryl; (2-, 3-, 4-)biphenylyl which is a bicyclic aryl; (1-, 2-)naphthyl which is a fused bicyclic aryl; terphenylyl (m-terphenyl-2'-yl, m-terphenyl-4'-yl, m-terphenyl-5'-yl, o-terphenyl-3'-yl, o-terphenyl-4'-yl, p-terphenyl-2'-yl, m-terphenyl-2-yl, m-terphenyl-3-yl, m-terphenyl-4-yl, o-terphenyl-2-yl, o-terphenyl-3-yl, o-terphenyl-4-yl, p-terphenyl-2-yl, p-terphenyl-3-yl, p-terphenyl-4-yl) which is a tricyclic aryl; acenaphthylene-(1-, 3-, 4-, 5-)yl, fluorene-(1-, 2-, 3-, 4-, 9-)yl, phenalene-(1-, 2-)yl, and (1-, 2-, 3-, 4-, 9-)phenanthryl which are fused tricyclic aryls; quaterphenylyl-(5'-phenyl-m-terphenyl-2-yl, 5'-phenyl-m-terphenyl-3-yl, 5'-phenyl-m-terphenyl-4-yl, m-quaterphenylyl) which is a tetracyclic aryl; triphenylene-(1-, 2-)yl, pyrene-(1-, 2-, 4-)yl, and naphthacene-(1-, 2-, 5-)yl which are fused tetracyclic aryls; and perylene-(1-, 2-, 3-)yl and pentacene-(1-, 2-, 5-, 6-)yl which are fused pentacyclic aryls.

Examples of the "heteroaryl" as the "optionally substituted heteroaryl" include a heteroaryl having 2 to 30 carbon atoms. A heteroaryl having 2 to 25 carbon atoms is preferable, a heteroaryl having 2 to 20 carbon atoms is more preferable, a heteroaryl having 2 to 15 carbon atoms is still more preferable, and a heteroaryl having 2 to 10 carbon atoms is particularly preferable. Furthermore, examples of the "heteroaryl" include a heterocyclic ring containing 1 to 5 heteroatoms selected from an oxygen atom, a sulfur atom, and a nitrogen atom in addition to a carbon atom as a ring-constituting atom.

Specific examples of the "heteroaryl" include pyrrolyl, oxazolyl, isoxazolyl, thiazolyl, isothiazolyl, imidazolyl, oxadiazolyl, thiadiazolyl, triazolyl, tetrazolyl, pyrazolyl, pyridyl, pyrimidinyl, pyridazinyl, pyrazinyl, triazinyl, indolyl, isoindolyl, 1H-indazolyl, benzoimidazolyl, benzoxazolyl, benzothiazolyl, 1H-benzotriazolyl, quinolyl, isoquinolyl, cinnolyl, quinazolyl, quinoxalinyl, phthalazinyl, naphthyridinyl, purinyl, pteridinyl, carbazolyl, acridinyl, phenoxathiinyl, phenoxazinyl, phenothiazinyl, phenazinyl, phenazasilinyl, indolizinyl, furanyl, benzofuranyl, isobenzofuranyl, dibenzofuranyl, naphthobenzofuranyl, thiophenyl, benzothiophenyl, isobenzothiophenyl, dibenzothiophenyl, naphthobenzothiophenyl, benzophospholyl, dibenzophospholyl, monovalent group of benzophosphole oxide ring, monovalent group of dibenzophosphole oxide ring, furazanyl, thianthrenyl, indolocarbazolyl, benzoindolocarbazolyl, and benzobenzoindolocarbazolyl.

Furthermore, the above aryl and heteroaryl may be substituted, and may be each substituted by, for example, the above aryl or heteroaryl.

Specific examples of this triazine derivative include the following compounds.

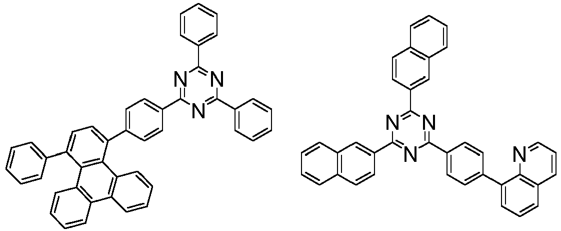

This triazine derivative can be manufactured using known raw materials and known synthesis methods.

### <Benzimidazole derivative>

The benzimidazole derivative is, for example, a compound represented by the following formula (ETM-11).

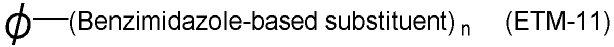

ϕ represents an n-valent aryl ring (preferably, an n-valent benzene ring, naphthalene ring, anthracene ring, fluorene ring, benzofluorene ring, phenalene ring, phenanthrene ring, or triphenylene ring), and n represents an integer of 1 to 4. A "benzimidazole-based substituent" is a substituent in which the pyridyl group in the "pyridine-based substituent" in the formulas (ETM-2), (ETM-2-1), and (ETM-2-2) is substituted by a benzimidazole group, and at least one hydrogen atom in the benzimidazole derivative may be substituted by a deuterium atom. The symbol "*" in each structural formula represents a bonding position.

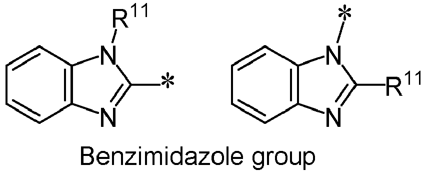

R¹¹ in the above benzimidazole represents a hydrogen atom, an alkyl having 1 to 24 carbon atoms, a cycloalkyl having 3 to 12 carbon atoms, or an aryl having 6 to 30 carbon atoms. The description of R¹¹ in the above formulas (ETM-2-1), and (ETM-2-2) can be cited.

Moreover, ϕ is preferably an anthracene ring or a fluorene ring. For the structure in this case, the description for the above formula (ETM-2-1) or (ETM-2-2) can be cited. For R¹¹ to R¹⁸ in each formula, the description for the above formula (ETM-2-1) or (ETM-2-2) can be cited. Furthermore, in the above formula (ETM-2-1) or (ETM-2-2), a form in which two pyridine-based substituents are bonded has been described. However, when these substituents are substituted by benzimidazole-based substituents, both the pyridine-based substituents may be substituted by benzimidazole-based substituents (that is, n = 2), or one of the pyridine-based substituents may be substituted by a benzimidazole-based substituent and the other pyridine-based substituent may be substituted by any one of R¹¹ to R¹⁸ (that is, n = 1). Moreover, for example, at least one of R¹¹ to R¹⁸ in the above formula (ETM-2-1) may be substituted by a benzimidazole-based substituent and the "pyridine-based substituent" may be substituted by any one of R¹¹ to R¹⁸.

Specific examples of this benzimidazole derivative include 1-phenyl-2-(4-(10-phenylanthracen-9-yl)phenyl)-1H-benzo[d]imidazole, 2-(4-(10-(naphthalen-2-yl)anthracen-9-yl)phenyl)-1-phenyl-1H-benzo[d]imidazole, 2-(3-(10-(naphthalen-2-yl)anthracen-9-yl)phenyl)-1-phenyl-1H-benzo[d]imidazole, 5-(10-(naphthlen-2-yl)anthracen-9-yl)-1,2-diphenyl-1H-benzo[d]imidazole, 1-(4-(10-(naphthalen-2-yl)anthracen-9-yl)phenyl)-2-phenyl-1H-benzo[d]imidazole, 2-(4-(9,10-di(naphthalen-2-yl)anthracen-2-yl)phenyl)-1-phenyl-1H-benzo[d]imidazole, 1-(4-(9,10-di(naphthalen-2-yl)anthracen-2-yl)phenyl)-2-phenyl-1H-benzo[d]imidazole, and 5-(9,10-di(naphthalen-2-yl)anthracen-2-yl)-1,2-diphenyl-1H-benzo[d]imidazole.

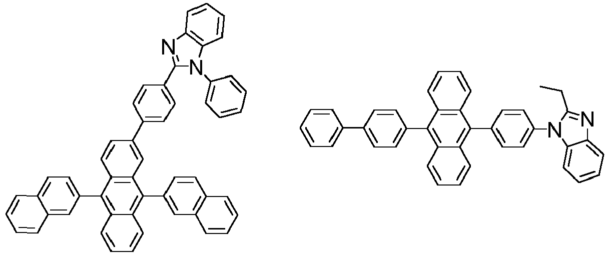

This benzimidazole derivative can be manufactured using known raw materials and known synthesis methods.

### <Phenanthroline derivative>

The phenanthroline derivative is, for example, a compound represented by the following formula (ETM-12) or (ETM-12-1). Details are described in WO 2006/021982 A.

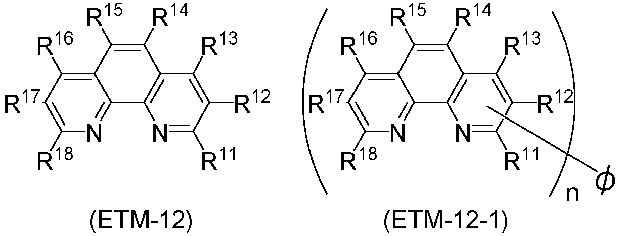

ϕ represents an n-valent aryl ring (preferably, an n-valent benzene ring, naphthalene ring, anthracene ring, fluorene ring, benzofluorene ring, phenalene ring, phenanthrene ring, or triphenylene ring), and n represents an integer of 1 to 4.

In each formula, R¹¹ to R¹⁸ each independently represent a hydrogen atom, an alkyl (preferably, an alkyl having 1 to 24 carbon atoms), a cycloalkyl (preferably, a cycloalkyl having 3 to 12 carbon atoms), or an aryl (preferably, an aryl having 6 to 30 carbon atoms). Furthermore, in the above formula (ETM-12-1), any one of R¹¹ to R¹⁸ is bonded to ϕ which is an aryl ring.

At least one hydrogen atom in each phenanthroline derivative may be substituted by a deuterium atom.

For the alkyl, cycloalkyl, and aryl in R¹¹ to R¹⁸, the description of R¹¹ to R¹⁸ in the above formula (ETM-2) can be cited. Furthermore, in addition to the above examples, examples of the ϕ include those having the following structural formulas. Note that R's in the following structural formulas each independently represent a hydrogen atom, methyl, ethyl, isopropyl, cyclohexyl, phenyl, 1-naphthyl, 2-naphthyl, biphenylyl, or terphenylyl. The symbol "*" in each structural formula represents a bonding position.

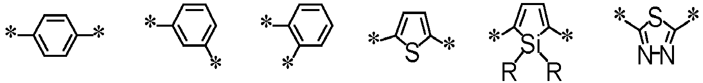

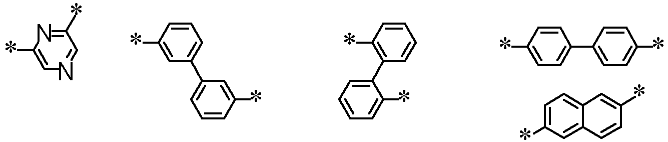

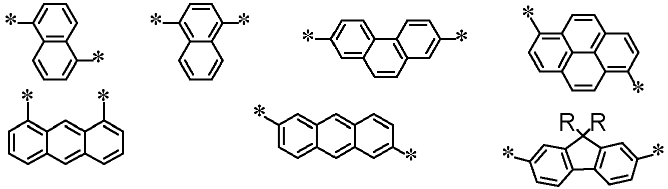

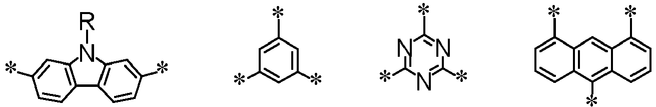

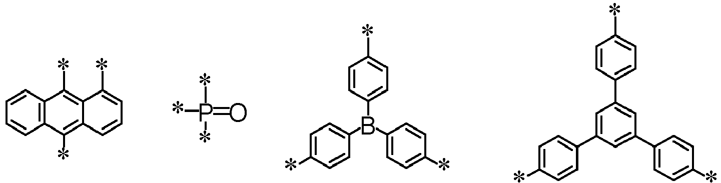

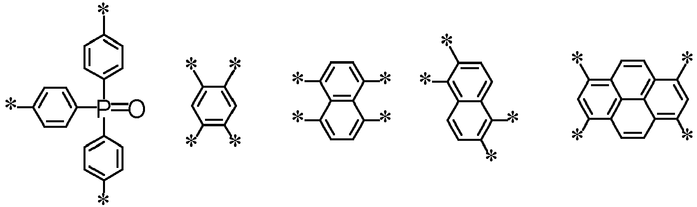

Specific examples of this phenanthroline derivative include 4,7-diphenyl-1,10-phenanthroline, 2,9-dimethyl-4,7-diphenyl-1,10-phenanthroline, 9,10-di(1,10-phenanthrolin-2-yl)anthracene, 2,6-di(1,10-phenanthrolin-5-yl)pyridine, 1,3,5-tri(1,10-phenanthrolin-5-yl)benzene, 9,9'-difluoro-bis(1,10-phenanthrolin-5-yl), bathocuproine, 1,3-bis(2-phenyl-1,10-phenanthrolin-9-yl)benzene, a compound represented by the following structural formula, and the like.

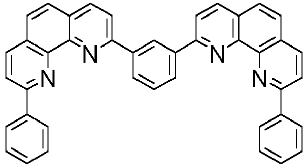

This phenanthroline derivative can be manufactured using known raw materials and known synthesis methods.

### <Quinolinol-based metal complex>

The quinolinol-based metal complex is, for example, a compound represented by the following general formula (ETM-13).

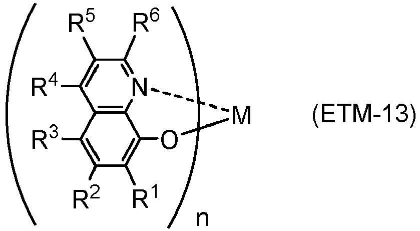

In the formula, R¹ to R⁶ each dependently represent a hydrogen atom, fluorine, alkyl, cycloalkyl, aralkyl, alkenyl, cyano, alkoxy or aryl, M represents Li, Al, Ga, Be, or Zn, and n represents an integer of 1 to 3.

Specific examples of the quinolinol-based metal complex include 8-quinolinol lithium, tris(8-quinolinolato) aluminum, tris(4-methyl-8-quinolinolato) aluminum, tris(5-methyl-8-quinolinolato) aluminum, tris(3,4-dimethyl-8-quinolinolato) aluminum, tris(4,5-dimethyl-8-quinolinolato) aluminum, tris(4,6-dimethyl-8-quinolinolato) aluminum, bis(2-methyl-8-quinolinolato) (phenolato) aluminum, bis(2-methyl-8-quinolinolato) (2-methylphenolato) aluminum, bis(2-methyl-8-quinolinolato) (3-methylphenolato) aluminum, bis(2-methyl-8-quinolinolato) (4-methylphenolato) aluminum, bis(2-methyl-8-quinolinolato) (2-phenylphenolato) aluminum, bis(2-methyl-8-quinolinolato) (3-phenylphenolato) aluminum, bis(2-methyl-8-quinolinolato) (4-phenylphenolato) aluminum, bis(2-methyl-8-quinolinolato) (2,3-dimethylphenolato) aluminum, bis(2-methyl-8-quinolinolato) (2,6-dimethylphenolato) aluminum, bis(2-methyl-8-quinolinolato) (3,4-dimethylphenolato) aluminum, bis(2-methyl-8-quinolinolato) (3,5-dimethylphenolato) aluminum, bis(2-methyl-8-quinolinolato) (3,5-di-t-butylphenolato) aluminum, bis(2-methyl-8-quinolinolato) (2,6-diphenylphenolato) aluminum, bis(2-methyl-8-quinolinolato) (2,4,6-triphenylphenolato) aluminum, bis(2-methyl-8-quinolinolato) (2,4,6-trimethylphenolato) aluminum, bis(2-methyl-8-quinolinolato)(2,4,5,6-tetramethylphenolato) aluminum, bis(2-methyl-8-quinolinolato) (1-naphtholato) aluminum, bis(2-methyl-8-quinolinolato) (2-naphtholato) aluminum, bis(2,4-dimethyl-8-quinolinolato) (2-phenylphenolato) aluminum, bis(2,4-dimethyl-8-quinolinolato) (3-phenylphenolato) aluminum, bis(2,4-dimethyl-8-quinolinolato) (4-phenylphenolato) aluminum, bis(2,4-dimethyl-8-quinolinolato) (3,5-dimethylphenolato) aluminum, bis(2,4-dimethyl-8-quinolinolato) (3,5-di-t-butylphenolato) aluminum, bis(2-methyl-8-quinolinolato) aluminum-µ-oxo-bis(2-methyl-8-quinolinolato) aluminum, bis(2,4-dimethyl-8-quinolinolato) aluminum-µ-oxo-bis(2,4-dimethyl-8-quinolinolato) aluminum, bis(2-methyl-4-ethyl-8-quinolinolato) aluminum-µ-oxo-bis(2-methyl-4-ethyl-8-quinolinolato) aluminum, bis(2-methyl-4-methoxy-8-quinolinolato) aluminum-u-oxo-bis(2-methyl-4-methoxy-8-quinolinolato) aluminum, bis(2-methyl-5-cyano-8-quinolinolato) aluminum-p-oxo-bis(2-methyl-5-cyano-8-quinolinolato) aluminum, bis(2-methyl-5-trifluoromethyl-8-quinolinolato) aluminum-u-oxo-bis(2-methyl-5-trifluoromethyl-8-quinolinolato) aluminum, and bis(10-hydroxybenzo[h]quinoline) beryllium.

This quinolinol-based metal complex can be manufactured using known raw materials and known synthesis methods.

### <Thiazole derivative and benzothiazole derivative>

The thiazole derivative is, for example, a compound represented by the following formula (ETM-14-1).

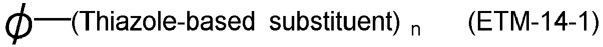

The benzothiazole derivative is, for example, a compound represented by the following formula (ETM-14-2).

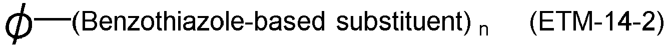

ϕ in each formula represents an n-valent aryl ring (preferably, an n-valent benzene ring, naphthalene ring, anthracene ring, fluorene ring, benzofluorene ring, phenalene ring, phenanthrene ring, or triphenylene ring), and n represents an integer of 1 to 4. A "thiazole-based substituent" or a "benzothiazole-based substituent" is a substituent in which the pyridyl group in the "pyridine-based substituent" in the formulas (ETM-2), (ETM-2-1), and (ETM-2-2) is substituted by the following thiazole group or benzothiazole group, and at least one hydrogen atom in the thiazole derivative and the benzothiazole derivative may be substituted by a deuterium atom. The symbol "*" in each structural formula represents a bonding position.

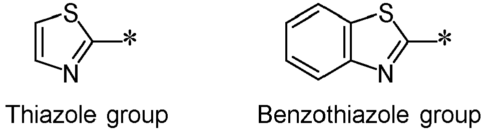

Moreover, ϕ is preferably an anthracene ring or a fluorene ring. For the structure in this case, the description for the above formula (ETM-2-1) or (ETM-2-2) can be cited. For R¹¹ to R¹⁸ in each formula, the description for the above formula (ETM-2-1) or (ETM-2-2) can be cited. Furthermore, in the above formula (ETM-2-1) or (ETM-2-2), a form in which two pyridine-based substituents are bonded has been described. However, when these substituents are substituted by thiazole-based substituents (or benzothiazole-based substituents), both the pyridine-based substituents may be substituted by thiazole-based substituents (or benzothiazole-based substituents) (that is, n = 2), or one of the pyridine-based substituents may be substituted by a thiazole-based substituent (or benzothiazole-based substituent) and the other pyridine-based substituent may be substituted by any one of R¹¹ to R¹⁸ (that is, n = 1). Moreover, for example, at least one of R¹¹ to R¹⁸ in the above formula (ETM-2-1) may be substituted by a thiazole-based substituent (or benzothiazole-based substituent) and the "pyridine-based substituent" may be substituted by any one of R¹¹ to R¹⁸.

These thiazole derivatives or benzothiazole derivatives can be manufactured using known raw materials and known synthesis methods.

An electron transport layer or an electron injection layer may further contain a substance that can reduce a material to form an electron transport layer or an electron injection layer. As this reducing substance, various substances are used as long as having reducibility to a certain extent. For example, at least one selected from the group consisting of an alkali metal, an alkaline earth metal, a rare earth metal, an oxide of an alkali metal, a halide of an alkali metal, an oxide of an alkaline earth metal, a halide of an alkaline earth metal, an oxide of a rare earth metal, a halide of a rare earth metal, an organic complex of an alkali metal, an organic complex of an alkaline earth metal, and an organic complex of a rare earth metal, can be suitably used.

Preferable examples of the reducing substance include an alkali metal such as Na (work function 2.36 eV), K (work function 2.28 eV), Rb (work function 2.16 eV), or Cs (work function 1.95 eV); and an alkaline earth metal such as Ca (work function 2.9 eV), Sr (work function 2.0 to 2.5 eV), or Ba (work function 2.52 eV). A substance having a work function of 2.9 eV or less is particularly preferable. Among these substances, an alkali metal such as K, Rb, or Cs is a more preferable reducing substance, Rb or Cs is a still more preferable reducing substance, and Cs is the most preferable reducing substance. These alkali metals have particularly high reducing ability, and can enhance emission luminance of an organic EL element or can lengthen a lifetime thereof by adding the alkali metals in a relatively small amount to a material to form an electron transport layer or an electron injection layer. Furthermore, as the reducing substance having a work function of 2.9 eV or less, a combination of two or more kinds of these alkali metals is also preferable, and particularly, a combination including Cs, for example, a combination of Cs with Na, a combination of Cs with K, a combination of Cs with Rb, or a combination of Cs with Na and K, is preferable. By inclusion of Cs, reducing ability can be efficiently exhibited, and emission luminance of an organic EL element is enhanced, or a lifetime thereof is lengthened by adding Cs to a material to form an electron transport layer or an electron injection layer.

Each of the above material for the electron injection layer and the above material for the electron transport layer can be used as a material for the electron layer also in a form of a polymer compound obtained by polymerizing, as a monomer, a reactive compound in which each of the material for the electron injection layer and the material for the electron transport layer is substituted by a reactive substituent, a crosslinked polymer thereof, a pendant type polymer compound obtained by a reaction between a main chain type polymer and the reactive compound, or a pendant type crosslinked polymer thereof. As the reactive substituent in this case, the description for the polycyclic aromatic compound represented by formula (1) can be cited.

Details of applications of the polymer compound and the crosslinked polymer will be described later.

### <Negative electrode in organic electroluminescent element>

The negative electrode 108 plays a role of injecting an electron to the light emitting layer 105 through the electron injection layer 107 and the electron transport layer 106.

A material to form the negative electrode 108 is not particularly limited as long as being a substance capable of efficiently injecting an electron to an organic layer. However, a material similar to a material to form the positive electrode 102 can be used. Among these materials, a metal such as tin, indium, calcium, aluminum, silver, copper, nickel, chromium, gold, platinum, iron, zinc, lithium, sodium, potassium, cesium, or magnesium, and an alloy thereof (a magnesium-silver alloy, a magnesium-indium alloy, an aluminum-lithium alloy such as lithium fluoride/aluminum, or the like) are preferable. In order to enhance element characteristics by increasing electron injection efficiency, lithium, sodium, potassium, cesium, calcium, magnesium, or an alloy containing these low work function-metals is effective. However, many of these low work function-metals are generally unstable in air. In order to ameliorate this problem, for example, a method for using an electrode having high stability obtained by doping an organic layer with a trace amount of lithium, cesium, or magnesium is known. Other examples of a dopant that can be used include an inorganic salt such as lithium fluoride, cesium fluoride, lithium oxide, or cesium oxide. However, the dopant is not limited thereto.

Furthermore, in order to protect an electrode, a metal such as platinum, gold, silver, copper, iron, tin, aluminum, or indium, an alloy using these metals, an inorganic substance such as silica, titania, or silicon nitride, polyvinyl alcohol, vinyl chloride, a hydrocarbon-based polymer compound, or the like may be laminated as a preferable example. These method for manufacturing an electrode are not particularly limited as long as being capable of conduction, such as resistance heating, electron beam deposition, sputtering, ion plating, or coating.

### <Binder that may be used in each layer>

The materials used in the above-described hole injection layer, hole transport layer, light emitting layer, electron transport layer, and electron injection layer can form each layer by being used singly. However, it is also possible to use the materials by dispersing the materials in a solvent-soluble resin such as polyvinyl chloride, polycarbonate, polystyrene, poly(N-vinylcarbazole), polymethyl methacrylate, polybutyl methacrylate, polyester, polysulfone, polyphenylene oxide, polybutadiene, a hydrocarbon resin, a ketone resin, a phenoxy resin, polyamide, ethyl cellulose, a vinyl acetate resin, an ABS resin, or a polyurethane resin; or a curable resin such as a phenolic resin, a xylene resin, a petroleum resin, a urea resin, a melamine resin, an unsaturated polyester resin, an alkyd resin, an epoxy resin, or a silicone resin.

### <Method for manufacturing organic electroluminescent element>

Each layer constituting an organic EL element can be formed by forming thin films of the materials to constitute each layer by methods such as a vapor deposition method, resistance heating deposition, electron beam deposition, sputtering, a molecular lamination method, a printing method, a spin coating method, a casting method, and a coating method. The film thickness of each layer thus formed is not particularly limited, and can be appropriately set according to a property of a material, but is usually within a range of 2 nm to 5000 nm. The film thickness can be usually measured using a crystal oscillation type film thickness analyzer or the like. In a case of forming a thin film using a vapor deposition method, deposition conditions depend on the kind of a material, an intended crystal structure and association structure of the film, and the like. It is preferable to appropriately set the vapor deposition conditions generally in ranges of a boat heating temperature of +50 to +400°C, a degree of vacuum of 10⁻⁶ to 10⁻³ Pa, a vapor deposition rate of 0.01 to 50 nm/sec, a substrate temperature of -150 to +300°C, and a film thickness of 2 nm to 5 µm.

In a case where a direct current voltage is applied to the organic EL element thus obtained, it is only required to apply the voltage by assuming a positive electrode as a positive polarity and assuming a negative electrode as a negative polarity. By applying a voltage of about 2 to 40 V, light emission can be observed from a transparent or semitransparent electrode side (the positive electrode or the negative electrode, or both the electrodes). This organic EL element also emits light even in a case where a pulse current or an alternating current is applied. Note that a waveform of an alternating current applied may be any waveform.

Next, as an example of a method for manufacturing an organic EL element, a method for manufacturing an organic EL element formed of positive electrode/hole injection layer/hole transport layer/light emitting layer including a host material and a dopant material/electron transport layer/electron injection layer/negative electrode will be described.

### <Vapor deposition method>

A thin film of a positive electrode material is formed on an appropriate substrate by a vapor deposition method or the like to manufacture a positive electrode, and then thin films of a hole injection layer and a hole transport layer are formed on this positive electrode. A thin film is formed thereon by co-depositing a host material and a dopant material to obtain a light emitting layer. An electron transport layer and an electron injection layer are formed on this light emitting layer, and a thin film formed of a substance for a negative electrode is formed by a vapor deposition method or the like to obtain a negative electrode. An intended organic EL element is thereby obtained. Incidentally, in manufacturing the above organic EL element, it is also possible to manufacture the organic EL element by reversing the manufacturing order, that is, in order of a negative electrode, an electron injection layer, an electron transport layer, a light emitting layer, a hole transport layer, a hole injection layer, and a positive electrode.

### <Wet film formation method>

The wet film formation method is performed by preparing a low molecular weight compound that can form each of the organic layers of the organic EL element as a liquid organic layer-forming composition, and using the low molecular weight compound. When there is no appropriate organic solvent in which this low molecular weight compound is dissolved, the organic layer-forming composition may be prepared from, for example, a polymer compound obtained by polymerizing another monomer having a solubility function as a reactive compound in which the low molecular weight compound is substituted by a reactive substituent and a main chain type polymer.

In the wet film formation method, generally, a coating film is formed through an applying step of applying an organic layer-forming composition onto a substrate and a drying step of removing a solvent from the applied organic layer-forming composition. When the polymer compound has a crosslinkable substituent (also referred to as a crosslinkable polymer compound), the polymer compound is further crosslinked by this drying step to form a crosslinked polymer. According to a difference in the applying step, a method using a spin coater is referred to as a spin coating method, a method using a slit coater is referred to as a slit coating method, a method using a plate is referred to gravure, offset, reverse offset, and flexographic printing methods, a method using an ink jet printer is referred to as an ink jet method, and a method for spraying the composition is referred to as a spraying method. Examples of the drying step include methods of air drying, heating, and drying under reduced pressure. The drying step may be performed only once, or may be performed a plurality of times using different methods and conditions.
Furthermore, different methods may be used in combination like calcination under reduced pressure.

The wet film formation method is a film formation method using a solution, and examples thereof include a part of printing methods (ink jet method), a spin coating method, a casting method, and a coating method. Unlike a vacuum deposition method, the wet film formation method does not need to use an expensive vacuum deposition apparatus, and a film can be formed under atmospheric pressure. In addition, the wet film formation method can increase an area and manufacture a product continuously, leading to reduction in manufacturing cost.

Meanwhile, as compared with the vacuum deposition method, lamination may be difficult by the wet film formation method. In a case where a laminated film is manufactured using the wet film formation method, it is necessary to prevent dissolution of a lower layer due to a composition of an upper layer, and techniques of using a composition with controlled solubility, crosslinking the lower layer, using orthogonal solvents (solvents which are not dissolved in each other), and the like are used. However, even with these techniques, it may be difficult to use the wet film formation method for application to all the films.

Therefore, in general, a method is adopted in which only some of the layers are formed by the wet film formation method and the remaining layers are formed by the vacuum deposition method to manufacture an organic EL element.

For example, a procedure for partially applying the wet film formation method to manufacture an organic EL element will be described below.
(Procedure 1) Film formation of positive electrode by vacuum deposition method (Procedure 2) Film formation by wet film formation method using hole injection layer-forming composition containing hole injection layer-forming material
(Procedure 3) Film formation by wet film formation method using hole transport layer-forming composition containing hole transport layer-forming material
(Procedure 4) Film formation by wet film formation method using light emitting layer-forming composition containing host material and dopant material
(Procedure 5) Film formation of electron transport layer by vacuum deposition method (Procedure 6) Film formation of electron injection layer by vacuum deposition method (Procedure 7) Film formation of negative electrode by vacuum deposition method

Through this procedure, an organic EL element formed of anode/hole injection layer/hole transport layer/light emitting layer including a host material and a dopant material/electron transport layer/electron injection layer/negative electrode is obtained. Of course, a film can be formed by wet film formation method using layer-forming composition containing electron transport layer forming material or electron injection layer forming material, by using a means for preventing dissolution of the light emitting layer of the lower layer, and a means for forming a film from the negative electrode side, which is contrary to the above described procedure.

### <Other film formation method>

For film formation of the organic layer-forming composition, a laser heating drawing method (LITI) can be used. LITI is a method for heating and depositing a compound attached to a base material with a laser, and the organic layer-forming composition can be used for a material to be applied to a base material.

### <Optional step>

A suitable treatment step, washing step, and drying step may be appropriately performed before and after each of the steps of film formation. Examples of the treatment step include an exposure treatment, a plasma surface treatment, an ultrasonic treatment, an ozone treatment, a washing treatment using a suitable solvent, and a heat treatment. Examples of the treatment step further include a series of steps for manufacturing a bank.

A photolithography technique can be used for manufacturing a bank. As a bank material that can be used for photolithography, a positive resist material and a negative resist material can be used. A patternable printing method such as an ink jet method, gravure offset printing, reverse offset printing, or screen printing can also be used. In this case, a permanent resist material can also be used.

Examples of a material used for a bank include a polysaccharide and a derivative thereof, a homopolymer and a copolymer of a hydroxyl-containing ethylenic monomer, a biopolymer compound, a polyacryloyl compound, polyester, polystyrene, polyimide, polyamideimide, polyetherimide, polysulfide, polysulfone, polyphenylene, polyphenyl ether, polyurethane, epoxy (meth)acrylate, melamine (meth)acrylate, polyolefin, cyclic polyolefin, an acrylonitrile-butadiene-styrene copolymer (ABS), a silicone resin, polyvinyl chloride, chlorinated polyethylene, chlorinated polypropylene, polyacetate, polynorbornene, a synthetic rubber, a fluorinated polymer such as polyfluorovinylidene, polytetrafluoroethylene, or polyhexafluoropropylene pyrene, a fluoroolefin-hydrocarbon olefin copolymer, and a fluorocarbon polymer, but are not limited thereto.

### <Organic layer-forming composition for wet film formation method>

The organic layer-forming composition can be obtained by dissolving a low molecular weight compound that can form each of an organic layers of an organic EL element or a polymer compound obtained by polymerizing the low molecular weight compound into an organic solvent. For example, a light emitting layer-forming composition includes at least one polycyclic aromatic compound (and a polymer compound thereof) that is a dopant material as a first component, at least one host material as a second component, and at least one organic solvent as a third component. The first component functions as a dopant component of a light emitting layer obtained from the composition, and the second component functions as a host component of the light emitting layer. The third component functions as a solvent for dissolving the first component and the second component in the composition. At the time of application, the third component provides a smooth and uniform surface shape due to a controlled evaporation rate of the third component itself.

### <Organic solvents

The organic layer-forming composition contains at least one organic solvent. By controlling an evaporation rate of an organic solvent at the time of film formation, it is possible to control and improve film formability, presence or absence of defects in a coating film, surface roughness, and smoothness. At the time of film formation using an ink jet method, by controlling meniscus stability at a pinhole of an ink jet head, ejection performance can be controlled and improved. In addition, by controlling a drying speed of a film and orientation of a derivative molecule, it is possible to improve electrical characteristics, luminescence characteristics, efficiency, and a lifetime of an organic EL element having an organic layer obtained from the organic layer-forming composition.

### (1) Physical properties of organic solvent

The boiling point of at least one organic solvent is from 130°C to 300°C, more preferably from 140°C to 270°C, and still more preferably from 150°C to 250°C. A case where the boiling point is higher than 130°C is preferable from a viewpoint of ink jet ejection performance. A case where the boiling point is lower than 300°C is preferable from a viewpoint of defects in a coating film, surface roughness, a residual solvent, and smoothness. The organic solvent more preferably contains two or more kinds of organic solvents from a viewpoint of good ink jet ejection performance, film formability, smoothness, and the small amount of a residual solvent. Meanwhile, in some cases, in consideration of transportability and the like, the composition may be a solid composition obtained by removing a solvent from the organic layer-forming composition.

Furthermore, a particularly preferable configuration is that the organic solvent contains a good solvent (GS) and a poor solvent (PS) for at least one of solute, and the boiling point (BP_{GS}) of the good solvent (GS) is lower than the boiling point (BP_{PS}) of the poor solvent (PS).

By adding a poor solvent having a high boiling point, a good solvent having a low boiling point is volatilized earlier at the time of film formation, and the concentration of contents in the composition and the concentration of the poor solvent are increased to promote prompt film formation. As a result, a coating film having few defects, less surface roughness, and high smoothness can be obtained.

A difference in solubility (S_{GS}-S_{PS}) is preferably 1% or more, more preferably 3% or more, and still more preferably 5% or more. A difference in boiling point (BP_{PS}-BP_{GS}) is preferably 10°C or more, more preferably 30°C or more, and still more preferably 50°C or more.

After the film formation, an organic solvent is removed from a coating film through a drying step such as evacuation, reduction in pressure, or heating. In a case of heating, heating is preferably performed at a glass transition temperature (Tg) of at least one of solute + 30°C or lower from a viewpoint of improving coating film formability. Heating is preferably performed at a glass transition point (Tg) of at least one of solute -30°C or higher from a viewpoint of reducing a residual solvent. Even when the heating temperature is lower than the boiling point of an organic solvent, the organic solvent is sufficiently removed because the film is thin. Drying may be performed a plurality of times at different temperatures, or a plurality of drying methods may be used in combination.

### (2) Specific examples of organic solvent

Examples of an organic solvent used in the organic layer-forming composition include an alkylbenzene-based solvent, a phenyl ether-based solvent, an alkyl ether-based solvent, a cyclic ketone-based solvent, an aliphatic ketone-based solvent, a monocyclic ketone-based solvent, a solvent having a diester skeleton, and a fluorine-containing solvent. Specific examples thereof include pentanol, hexanol, heptanol, octanol, nonanol, decanol, undecanol, dodecanol, tetradecanol, hexan-2-ol, heptan-2-ol, octan-2-ol, decan-2-ol, dodecan-2-ol, cyclohexanol, α-terpineol, β-terpineol, γ-terpineol, δ-terpineol, terpineol (mixture), ethylene glycol monomethyl ether acetate, propylene glycol monomethyl ether acetate, diethylene glycol dimethyl ether, dipropylene glycol dimethyl ether, diethylene glycol ethyl methyl ether, diethylene glycol isopropyl methyl ether, dipropylene glycol monomethyl ether, diethylene glycol diethyl ether, diethylene glycol monomethyl ether, diethylene glycol butyl methyl ether, tripropylene glycol dimethyl ether, triethylene glycol dimethyl ether, diethylene glycol monobutyl ether, ethylene glycol monophenyl ether, triethylene glycol monomethyl ether, diethylene glycol dibutyl ether, triethylene glycol butyl methyl ether, polyethylene glycol dimethyl ether, tetraethylene glycol dimethyl ether, p-xylene, m-xylene, o-xylene, 2,6-lutidine, 2-fluoro-m-xylene, 3-fluoro-o-xylene, 2-chlorobenzo trifluoride, cumene, toluene, 2-chloro-6-fluorotoluene, 2-fluoroanisole, anisole, 2,3-dimethylpyrazine, bromobenzene, 4-fluoroanisole, 3-fluoroanisole, 3-trifluoromethylanisole, mesitylene, 1,2,4-trimethylbenzene, t-butylbenzene, 2-methylanisole, phenetole, benzodioxole, 4-methylanisole, s-butylbenzene, 3-methylanisole, 4-fluoro-3-methylanisole, cymene, 1,2,3-trimethylbenzene, 1,2-dichlorobenzene, 2-fluorobenzonitrile, 4-fluorobellaterol, 2,6-dimethylanisole, n-butylbenzene, 3-fluorobenzonitrile, decalin (decahydronaphthalene), neopentylbenzene, 2,5-dimethylanisole, 2,4-dimethylanisole, benzonitrile, 3,5-dimethylanisole, diphenyl ether, 1-fluoro-3,5-dimethoxybenzene, methyl benzoate, isopentylbenzene, 3,4-dimethylanisole, o-tolunitrile, n-amylbenzene, veratrole, 1,2,3,4-tetrahydronaphthalene, ethyl benzoate, n-hexylbenzene, propyl benzoate, cyclohexylbenzene, 1-methylnaphthalene, butyl benzoate, 2-methylbiphenyl, 3-phenoxytoluene, 2,2'-vitrile, dodecylbenzene, dipentylbenzene, tetramethylbenzene, trimethoxy benzene, trimethoxytoluene, 2,3-dihydrobenzofuran, 1-methyl-4-(propoxymethyl)benzene, 1-methyl-4-(butyloxymethyl)benzene, 1-methyl-4-(pentyloxymethyl)benzene, 1-methyl-4-(hexyloxymethyl)benzene, 1-methyl-4-(heptyloxymethyl)benzene benzyl butyl ether, benzyl pentyl ether, benzyl hexyl ether, benzyl heptyl ether, and benzyl octyl ether, but are not limited thereto. Furthermore, these solvents may be used singly or in a mixture thereof.

### <Optional components>

The organic layer-forming composition may contain an optional component as long as properties thereof are not impaired. Examples of an optional component include a binder and a surfactant.

### (1) Binder

The organic layer-forming composition may contain a binder. The binder forms a film at the time of film formation, and bonds the obtained film to a substrate. The binder also plays a role of dissolving, dispersing, and binding other components in the organic layer-forming composition.

Examples of a binder used in the organic layer-forming composition include an acrylic resin, polyethylene terephthalate, an ethylene-vinyl acetate copolymer, an ethylene-vinyl alcohol copolymer, an acrylonitrile-ethylene-styrene copolymer (AES) resin, an ionomer, chlorinated polyether, a diallyl phthalate resin, an unsaturated polyester resin, polyethylene, polypropylene, polyvinyl chloride, polyvinylidene chloride, polystyrene, polyvinyl acetate, Teflon, an acrylonitrile-butadiene-styrene copolymer (ABS) resin, an acrylonitrile-styrene copolymer (AS) resin, a phenol resin, an epoxy resin, a melamine resin, a urea resin, an alkyd resin, polyurethane, and a copolymer of the above resins and polymers, but are not limited thereto.

The binder used in the organic layer-forming composition may be used singly or in a mixture of a plurality of kinds thereof.

### (2) Surfactant

The organic layer-forming composition may contain, for example, a surfactant for controlling film surface uniformity of the organic layer-forming composition, solvent affinity of a film surface, and liquid repellency. The surfactant is classified into an ionic surfactant and a nonionic surfactant based on the structure of a hydrophilic group, and is further classified into an alkyl-based surfactant, a silicon-based surfactant, and a fluorine-based surfactant based on the structure of a hydrophobic group. The surfactant is classified into a monomolecule-based surfactant having a relatively small molecular weight and a simple structure, and a polymer-based surfactant having a large molecular weight and a side chain or a branched chain based on the structure of a molecule. The surfactant is classified into a single surfactant and a mixed surfactant obtained by mixing two or more kinds of surfactants with a base material based on the composition. As a surfactant that can be used in the organic layer-forming composition, all kinds of surfactants can be used.

Examples of the surfactant include Polyflow No. 45, Polyflow KL-245, Polyflow No. 75, Polyflow No. 90, Polyflow No. 95 (trade names, manufactured by Kyoeisha Chemical Co., Ltd.), Disperbyk 161, Disperbyk 162, Disperbyk 163, Disperbyk 164, Disperbyk 166, Disperbyk 170, Disperbyk 180, Disperbyk 181, Disperbyk 182, BYK 300, BYK 306, BYK 310, BYK 320, BYK 330, BYK 342, BYK 344, BYK 346 (trade names, manufactured by BYK Japan KK), KP-341, KP-358, KP-368, KF-96-50CS, KF-50-100CS (trade names, manufactured by Shin-Etsu Chemical Co., Ltd.), Surflon SC-101, Surflon KH-40 (trade names, manufactured by Seimi Chemical Co., Ltd.), Futargent 222F, Futargent 251, FTX-218 (trade names, manufactured by Neos Co., Ltd.), EFTOP EF-351, EFTOP EF-352, EFTOP EF-601, EFTOP EF-801, EFTOP EF-802 (trade names, manufactured by Mitsubishi Materials Corporation), Megafac F-470, Megafac F-471, Megafac F-475, Megafac R-08, Megafac F-477, Megafac F-479, Megafac F-553, Megafac F-554, (trade names, manufactured by DIC Corporation), fluoroalkylbenzene sulfonate, fluoroalkyl carboxylate, fluoroalkyl polyoxyethylene ether, fluoroalkyl ammonium iodide, fluoroalkyl betaine, fluoroalkyl sulfonate, diglycerin tetrakis(fluoroalkyl polyoxyethylene ether), fluoroalkyltrimethylammonium salt, fluoroalkyl aminosulfonate, polyoxyethylene nonylphenyl ether, polyoxyethylene octylphenyl ether, polyoxyethylene alkyl ether, polyoxyethylene laurate, polyoxyethylene oleate, polyoxyethylene stearate, polyoxyethylene lauryl amine, sorbitan laurate, sorbitan palmitate, sorbitan stearate, sorbitan oleate, sorbitan fatty acid ester, polyoxyethylene sorbitan laurate, polyoxyethylene sorbitan palmitate, polyoxyethylene sorbitan stearate, polyoxyethylene sorbitan oleate, polyoxyethylene naphthyl ether, alkylbenzene sulfonate, and alkyl diphenyl ether disulfonate.

The surfactant may be used singly or in combination of two or more kinds thereof.

### <Composition and physical properties of organic layer-forming composition>

The contents of the components in the organic layer-forming composition are determined from a viewpoint of good solubility of the components in the organic layer-forming composition, storage stability, film formability, high quality of a coating film obtained from the organic layer-forming composition, good ejection performance in a case of using an ink jet method, and good electrical characteristics, luminescent characteristics, efficiency, and a lifetime of an organic EL element having an organic layer manufactured using the composition. For example, in the case of a light emitting-forming composition, preferably, the content of the first component is from 0.0001% by weight to 2.0% by weight with respect to the total weight of the light emitting layer-forming composition, the content of the second component is from 0.0999% by weight to 8.0% by weight with respect to the total weight of the light emitting layer-forming composition, and the content of the third component is from 90.0% by weight to 99.9% by weight with respect to the total weight of the light emitting layer-forming composition

More preferably, the content of the first component is from 0.005% by weight to 1.0% by weight with respect to the total weight of the light emitting layer-forming composition, the content of the second component is from 0.095% by weight to 4.0% by weight with respect to the total weight of the light emitting layer-forming composition, and the content of the third component is from 95.0% by weight to 99.9% by with respect to the total weight of the light emitting layer-forming composition. Still more preferably, the content of the first component is from 0.05% by weight to 0.5% by weight with respect to the total weight of the light emitting layer-forming composition, the content of the second component is from 0.25% by weight to 2.5% by weight with respect to the total weight of the light emitting layer-forming composition, and the content of the third component is from 97.0% by weight to 99.7% by with respect to the total weight of the light emitting layer-forming composition.

The organic layer-forming composition can be manufactured by appropriately selecting and performing stirring, mixing, heating, cooling, dissolving, dispersing, and the like of the above components by a known method. After preparation, filtration, removal of gas (also referred to as degassing), an ion exchange treatment, an inert gas replacement/encapsulation treatment, and the like may be appropriately selected and performed.

The organic layer-forming composition having a high viscosity brings about good film formability and good ejection performance in a case of using an ink jet method. Meanwhile, the lower viscosity makes it easier to make a thin film. Therefore, the viscosity of the organic layer-forming composition is preferably from 0.3 to 3 mPa·s, and more preferably from 1 to 3 mPa·s at 25°C. In the present invention, the viscosity is a value measured using a cone plate type rotational viscometer (cone plate type).

The organic layer-forming composition having a low surface tension brings about a coating film having good film formability and no defects. Meanwhile, the organic layer-forming composition having a high surface tension brings about good ink jet ejection performance. Therefore, the surface tension of the organic layer-forming composition is preferably from 20 to 40 mN/m, and more preferably from 20 to 30 mN/m at 25°C. In the present invention, the surface tension is a value measured using a hanging drop method.

### <Crosslinkable polymer compound: compound represented by general formula (XLP-1)>

Next, a case where the above polymer compound has a crosslinkable substituent will be described. Examples of the crosslinkable polymer compound include a compound represented by the following general formula (XLP-1).

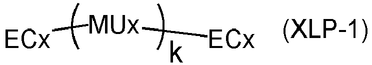

In formula (XLP-1),
MUx, ECx, and k have the same definitions as MU, EC, and k in the above formula (SPH-1), respectively. However, a compound represented by formula (XLP-1) has at least one crosslinkable substituent (XLS), and preferably, the content of a monovalent or divalent aromatic compound having a crosslinkable substituent is 0.1 to 80% by weight in a molecule.

The content of the monovalent or divalent aromatic compound having a crosslinkable substituent is preferably 0.5 to 50% by weight, and more preferably 1 to 20% by weight.

The crosslinkable substituent (XLS) is not particularly limited as long as it is a group capable of further crosslinking the above polymer compound, but is preferably a substituent having any one of the following structures. The symbol "*" in each of the structural formulas represents a bonding position.

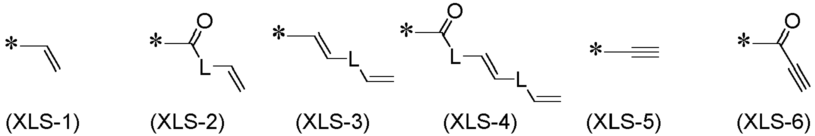

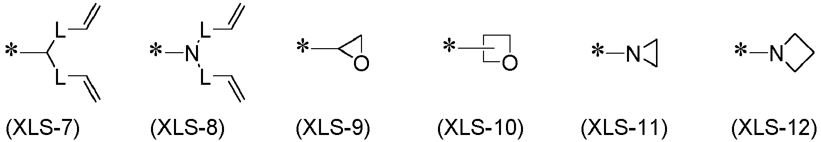

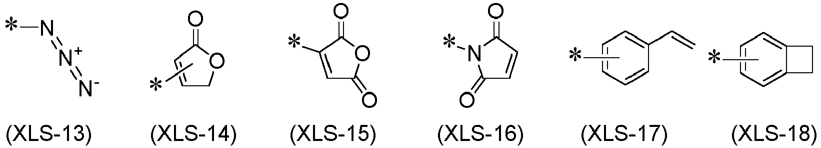

L's each independently represent a single bond, -O-, -S-, >C=O, -O-C(=O)-, an alkylene having 1 to 12 carbon atoms, an oxyalkylene having 1 to 12 carbon atoms, or a polyoxyalkylene having 1 to 12 carbon atoms. Among the above substituents, a group represented by formula (XLS-1), (XLS-2), (XLS-3), (XLS-9), (XLS-10), or (XLS-17) is preferable, and a group represented by formula (XLS-1), (XLS-3), or (XLS-17) is more preferable.

Examples of the divalent aromatic compound having a crosslinkable substituent include compounds having the following partial structures. The symbol "*" in each of the following structural formulas represents a bonding position.

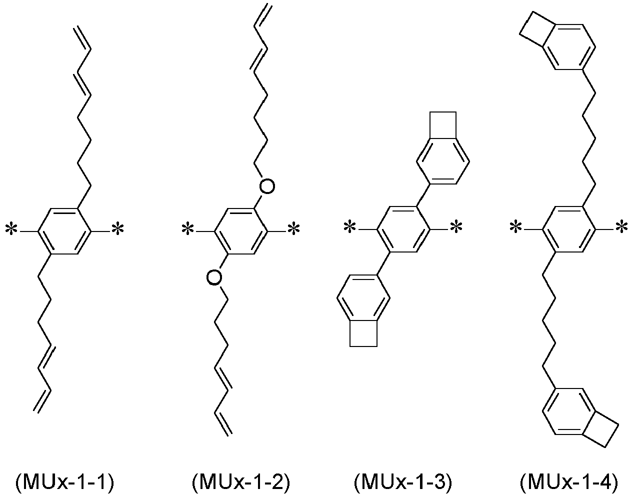

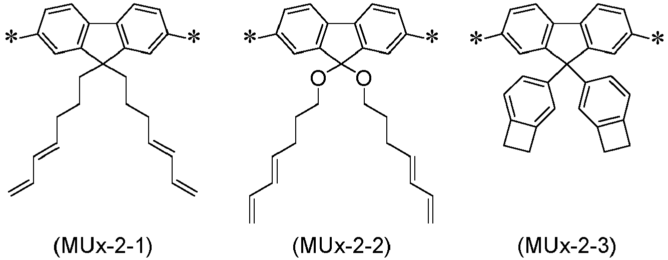

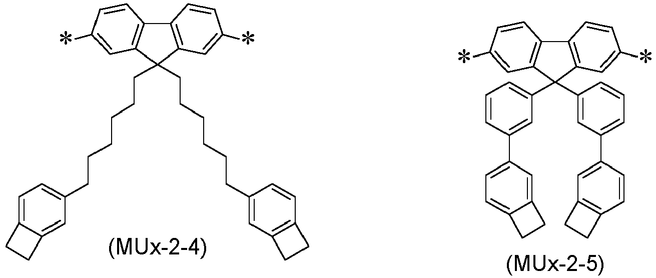

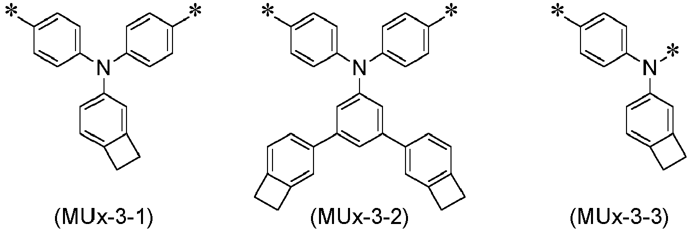

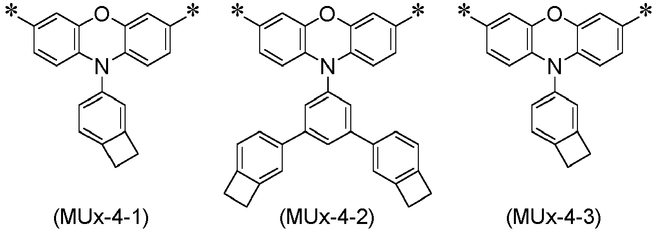

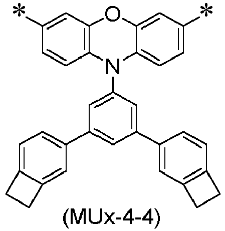

### <Method for manufacturing polymer compound and crosslinkable polymer compound>

A method for manufacturing the polymer compound and the crosslinkable polymer compound will be described by exemplifying the above compound represented by formula (SPH-1) and the above compound represented by formula (XLP-1). These compounds can be synthesized by appropriately combining known methods.

Examples of a solvent used for a reaction include an aromatic solvent, a saturated/unsaturated hydrocarbon solvent, an alcohol solvent, and an ether-based solvent. Examples thereof include dimethoxyethane, 2-(2-methoxyethoxy)ethane, and 2-(2-ethoxyethoxy)ethane.

The reaction may be performed in a two-phase system. When the reaction is performed in a two-phase system, a phase transfer catalyst such as a quaternary ammonium salt may be added as necessary.

When the compound represented by formula (SPH-1) and the compound represented by formula (XLP-1) are manufactured, the compounds may be manufactured through a single stage or a multistage. The compounds may be manufactured by a batch polymerization method in which all raw materials are put into a reaction vessel and then a reaction is started, may be manufactured by a dropping polymerization method in which raw materials are dropped into a reaction vessel, or may be manufactured by a precipitation polymerization method in which a product is precipitated as a reaction proceeds. The compounds can be synthesized by appropriately combining these methods. For example, when the compound represented by formula (SPH-1) is synthesized through a single stage, the desired product is obtained by causing a reaction in a state where a monomer unit (MU) and an end cap unit (EC) are contained in a reaction vessel. When the compound represented by formula (SPH-1) is synthesized through a multistage, the desired product is obtained by polymerizing a monomer unit (MU) until a desired molecular weight is reached and then adding an end cap unit (EC) thereto to cause a reaction. If different types of monomer units (MU) are added through a multistage to cause a reaction, a polymer having a concentration gradient for the structures of the monomer units can be manufactured. By preparing a precursor polymer and then causing a post-reaction, the desired polymer can be obtained.

If a polymerizable group of a monomer unit (MU) is selected, a primary structure of a polymer can be controlled. For example, as indicated by synthesis schemes 1 to 3, a polymer having a random primary structure (synthesis scheme 1) or a polymer having a regular primary structure (synthesis schemes 2 and 3) can be synthesized, and these polymers can be appropriately combined to be used according to a desired product. Furthermore, if a monomer unit having three or more polymerizable groups is used, a hyperbranched polymer or a dendrimer can be synthesized.

a, b = MU or MUx

### Polymerizable group = x, y (each x and y are bonded)

1) Polymer synthesized using two types of monomers (x-a-y) and (x-b-y)

   n x-a-y + n x-b-y → --a-b-a-a-b-b--- A polymer in which a and b are randomly bonded is synthesized.
2) Polymer synthesized using two types of monomers (x-a-x) and (y-b-y)

   n x-a-x + n y-b-y → --a-b-a-b-a-b--- A polymer in which a and b are alternately bonded is synthesized.
3) Polymer synthesized using two types of monomers (x-a-y) and (y-b-y)

   n x-a-y + n y-b-y → --a-a-b-a-b-a--- A polymer in which b are not adjacent is synthesized.

A monomer unit that can be used in the present invention can be synthesized in accordance with the methods disclosed in JP 2010-189630 A, WO 2012/086671 A, WO 2013/191088 A, WO 2002/045184 A, WO 2011/049241 A, WO 2013/146806 A, WO 2005/049546 A, WO 2015/145871 A, JP 2010-215886 A, JP 2008-106241 A, JP 2010-215886 A, WO 2016/031639 A, JP 2011-174062 A, WO 2016/031639 A, WO 2016/031639 A, and WO 2002/045184 A.

A polymer can be synthesized specifically in accordance with the methods disclosed in JP 2012-036388 A, WO 2015/008851 A, JP 2012-36381 A, JP 2012-144722 A, WO 2015/194448 A, WO 2013/146806 A, WO 2015/145871 A, WO 2016/031639 A, WO 2016/125560 A, WO 2016/031639 A, WO 2016/031639 A, WO 2016/125560 A, WO 2015/145871 A, WO 2011/049241 A, and JP 2012-144722 A.

### <Application examples of organic electroluminescent element>

Furthermore, the present invention can also be applied to a display apparatus including an organic EL element, a lighting apparatus including an organic EL element, or the like.

The display apparatus or lighting apparatus including an organic EL element can be manufactured by a known method such as connecting the organic EL element according to the present embodiment to a known driving apparatus, and can be driven by appropriately using a known driving method such as direct driving, pulse driving, or alternating driving.

Examples of the display apparatus include panel displays such as color flat panel displays; and flexible displays such as flexible organic electroluminescent (EL) displays (see, for example, JP 10-335066 A, JP 2003-321546 A, JP 2004-281086 A, and the like). Furthermore, examples of a display method of the display include a matrix method and a segment method. Note that the matrix display and the segment display may co-exist in the same panel.

In the matrix, pixels for display are arranged two-dimensionally as in a lattice form or a mosaic form, and characters or images are displayed by an assembly of pixels. The shape or size of a pixel depends on intended use. For example, for display of images and characters of a personal computer, a monitor, or a television, square pixels each having a size of 300 µm or less on each side are usually used. Furthermore, in a case of a large-sized display such as a display panel, pixels having a size in the order of millimeters on each side are used. In a case of monochromic display, it is only required to arrange pixels of the same color. However, in a case of color display, display is performed by arranging pixels of red, green and blue. In this case, typically, delta type display and stripe type display are available. For this matrix driving method, either a line sequential driving method or an active matrix method may be employed. The line sequential driving method has an advantage of having a simpler structure. However, in consideration of operation characteristics, the active matrix method may be superior. Therefore, it is necessary to use the line sequential driving method or the active matrix method properly according to intended use.

In the segment method (type), a pattern is formed so as to display predetermined information, and a determined region emits light. Examples of the segment method include display of time or temperature in a digital clock or a digital thermometer, display of a state of operation in an audio instrument or an electromagnetic cooker, and panel display in an automobile.

Examples of the lighting apparatus include a lighting apparatuses for indoor lighting or the like, and a backlight of a liquid crystal display apparatus (see, for example, JP 2003-257621 A, JP 2003-277741 A, and JP 2004-119211 A). The backlight is mainly used for enhancing visibility of a display apparatus that is not self-luminous, and is used in a liquid crystal display apparatus, a timepiece, an audio apparatus, an automotive panel, a display plate, a sign, and the like. Particularly, in a backlight for use in a liquid crystal display apparatus, among the liquid crystal display apparatuses, for use in a personal computer in which thickness reduction has been a problem to be solved, in consideration of difficulty in thickness reduction because a conventional type backlight is formed from a fluorescent lamp or a light guide plate, a backlight using the luminescent element according to the present embodiment is characterized by its thinness and lightweightness.

### 3-2. Other organic devices

The polycyclic aromatic compound according to an aspect of the present invention can be used for manufacturing an organic field effect transistor, an organic thin film solar cell, or the like, in addition to the organic electroluminescent element described above.

The organic field effect transistor is a transistor that controls a current by means of an electric field generated by voltage input, and is provided with a source electrode, a drain electrode, and a gate electrode. When a voltage is applied to the gate electrode, an electric field is generated, and the organic field effect transistor can control a current by arbitrarily damming a flow of electrons (or holes) that flow between the source electrode and the drain electrode. The field effect transistor can be easily miniaturized compared with a simple transistor (bipolar transistor), and is often used as an element constituting an integrated circuit or the like.

The structure of the organic field effect transistor is usually as follows. That is, a source electrode and a drain electrode are provided in contact with an organic semiconductor active layer formed using the polycyclic aromatic compound according to an aspect of the present invention, and it is only required that a gate electrode is further provided so as to interpose an insulating layer (dielectric layer) in contact with the organic semiconductor active layer. Examples of the element structure include the following structures.
(1) Substrate/gate electrode/insulator layer/source electrode and drain electrode/organic semiconductor active layer
(2) Substrate/gate electrode/insulator layer/organic semiconductor active layer/source electrode and drain electrode
(3) Substrate/organic semiconductor active layer/source electrode and drain electrode/insulator layer/gate electrode
(4) Substrate/source electrode and drain electrode/organic semiconductor active layer/insulator layer/gate electrode

An organic field effect transistor thus constituted can be applied as a pixel driving switching element of an active matrix driving type liquid crystal display or an organic electroluminescent display, or the like.

An organic thin film solar cell has a structure in which a positive electrode such as ITO, a hole transport layer, a photoelectric conversion layer, an electron transport layer, and a negative electrode are laminated on a transparent substrate of glass or the like. The photoelectric conversion layer has a p-type semiconductor layer on the positive electrode side, and has an n-type semiconductor layer on the negative electrode side. The polycyclic aromatic compound according to an aspect of the present invention can be used as a material for a hole transport layer, a p-type semiconductor layer, an n-type semiconductor layer, or an electron transport layer, depending on physical properties thereof. The polycyclic aromatic compound according to an aspect of the present invention can function as a hole transport material or an electron transport material in an organic thin film solar cell. The organic thin film solar cell may appropriately include a hole blocking layer, an electron blocking layer, an electron injection layer, a hole injection layer, a smoothing layer, and the like, in addition to the members described above. For the organic thin film solar cell, known materials used for an organic thin film solar cell can be appropriately selected and used in combination.

### [Examples]

Hereinafter, the present invention will be described more specifically with Examples, but the present invention is specified by the appended claims and not limited to the examples. First, Synthesis Examples of the polycyclic aromatic compound will be described below.

### Synthesis Example (1): Synthesis of compound (1-411)

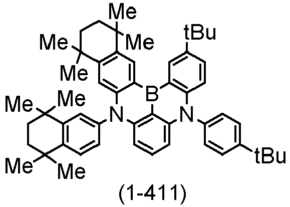

In a nitrogen atmosphere, 2,3-dichloroaniline (6.0 g), 6-bromo-1,1,4,4-tetramethyl-2,3-dihydronaphthalene (25 g), dichlorobis[(di-t-butyl(4-dimethylamonophenyl)phosphino)palladium (Pd-132, 0.44 g), sodium-t-butoxide (NaOtBu, 14.8 g), and xylene (120 ml) were put into a flask and heated at 120°C for two hours. After a reaction, to the reaction solution, water and ethyl acetate were added, and the mixture was stirred. Thereafter, an organic layer was separated from the mixture and washed with water. Thereafter, the organic layer was concentrated to obtain a crude product. The crude product was purified by a silica gel short column (eluent: toluene), and then a solvent was concentrated to obtain a crude product. To the crude product, methanol was added, and the mixture was cooled with ice. A precipitated crystal was filtered and washed with methanol to obtain compound (I-A) (17.0 g).

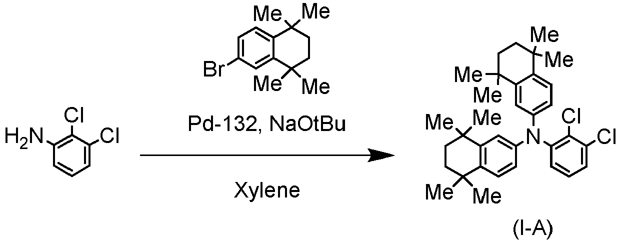

In a nitrogen atmosphere, compound (I-A) (8.0 g), bis(4-t-butylphenyl)amine (25 g), Pd-132 (0.10 g), NaOtBu (2.1 g), and xylene (40 ml) were put into a flask and heated at 120°C for one hour. After a reaction, to the reaction solution, water and ethyl acetate were added, and the mixture was stirred. Thereafter, an organic layer was separated from the mixture and washed with water. Thereafter, the organic layer was concentrated to obtain a crude product. The crude product was purified by a silica gel short column (eluent: toluene), and then a solvent was concentrated to obtain a crude product. To the crude product, methanol was added, and the mixture was cooled with ice. A precipitated crystal was filtered and washed with methanol to obtain compound (I-B) (9.7 g).

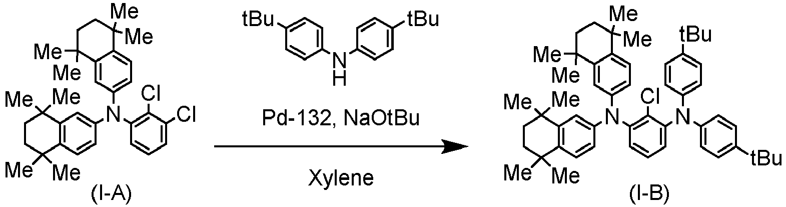

Into a flask containing compound (I-B) (8.6 g) and t-butylbenzene (80 ml), in a nitrogen atmosphere, a 1.53 M t-butyllithium pentane solution (15.2 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (5.8 g) was added thereto. The mixture was heated to room temperature and stirred for 0.5 hours. Thereafter, the resulting solution was cooled to 0°C again, N,N-diisopropylethylamine (2.6 g) was added thereto, and the mixture was stirred at room temperature until heat generation was stopped. Thereafter, the resulting solution was heated to 100°C and heated and stirred for one hour. The reaction solution was cooled to room temperature. To the reaction solution, a sodium acetate aqueous solution cooled in an ice bath was added, and subsequently ethyl acetate was added. The mixture was stirred for one hour. A yellow suspension was filtered, and a precipitate thereof was washed with methanol. A yellow crystal was heated and dissolved in toluene, and then purified by a silica gel short column (eluent: toluene). The obtained crude product was added to toluene, and the mixture was concentrated. Thereafter, solmix (A-11) was added thereto, and a precipitated crystal was filtered and further washed with methanol to obtain compound (1-411) (3.0 g).

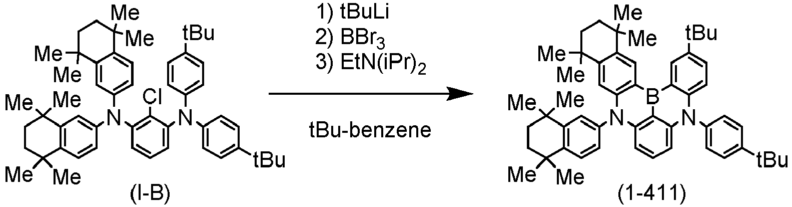

The structure of the obtained compound was confirmed by NMR measurement.
¹H-NMR (CDCl₃): δ = 1.09 (s, 6H), 1.27 (s, 6H), 1.42 (s, 6H), 1.46 (s, 9H), 1.48 (s, 9H), 1.49 (s, 6H), 1.71-1.80 (m, 8H), 6.11 (d, 1H), 6.23 (d, 1H), 6.59 (s, 1H), 6.73 (d, 1H), 7.09 (dd, 1H), 7.25-7.30 (m, 5H), 7.51 (dd, 1H), 7.59 (d, 1H), 7.67 (d, 2H), 8.92 (s, 1H), 8.99 (d, 1H).

### Synthesis Example (2): Synthesis of compound (1-590)

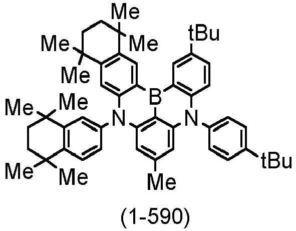

In a nitrogen atmosphere, 2,3-dichloro-5-methylaniline (6.6 g), 6-bromo-1,1,4,4-tetramethyl-2,3-dihydronaphthalene (25 g), Pd-132 (0.54 g), NaOtBu (9.0 g), and xylene (90 ml) were put into a flask and heated at 120°C for two hours. After a reaction, to the reaction solution, water and ethyl acetate were added, and the mixture was stirred. Thereafter, an organic layer was separated from the mixture and washed with water. Thereafter, the organic layer was concentrated to obtain a crude product. The crude product was purified by a silica gel short column (eluent: toluene), and then a solvent was concentrated to obtain a crude product. To the crude product, methanol was added, and the mixture was cooled with ice. A precipitated crystal was filtered and washed with methanol to obtain compound (I-C) (13.0 g).

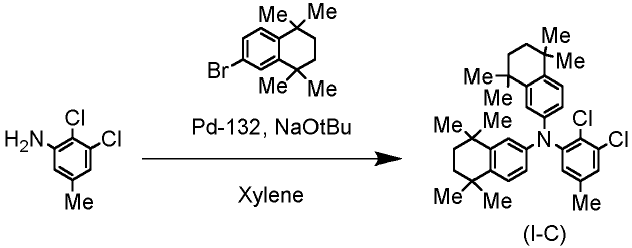

In a nitrogen atmosphere, compound (I-C) (8.0 g), bis(4-t-butylphenyl) amine (4 g), Pd-132 (0.10 g), NaOtBu (2.1 g), and xylene (40 ml) were put into a flask and heated at 120°C for one hour. After a reaction, to the reaction solution, water and ethyl acetate were added, and the mixture was stirred. Thereafter, an organic layer was separated from the mixture and washed with water. Thereafter, the organic layer was concentrated to obtain a crude product. The crude product was purified by a silica gel short column (eluent: toluene), and then a solvent was concentrated to obtain a crude product. To the crude product, ethyl acetate was added, and subsequently methanol was added. A precipitated crystal was filtered and washed with methanol to obtain compound (I-D) (11.0 g).

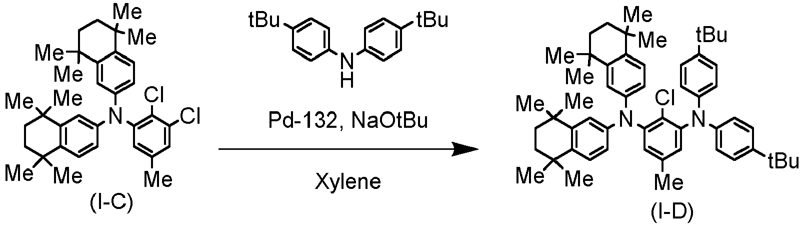

Into a flask containing compound (I-D) (11.0 g) and t-butylbenzene (110 ml), in a nitrogen atmosphere, a 1.53 M t-butyllithium pentane solution (18.9 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (7.3 g) was added thereto. The mixture was heated to room temperature and stirred for 0.5 hours. Thereafter, the resulting solution was cooled to 0°C again, N,N-diisopropylethylamine (2.6 g) was added thereto, and the mixture was stirred at room temperature until heat generation was stopped. Thereafter, the resulting solution was heated to 100°C and heated and stirred for one hour. The reaction solution was cooled to room temperature. To the reaction solution, a sodium acetate aqueous solution cooled in an ice bath was added, and subsequently ethyl acetate was added. The mixture was stirred for one hour. An organic layer was separated from the mixture and then washed with water (twice), and a solvent was distilled off under reduced pressure. To the obtained residue, methanol was added, and the mixture was cooled with ice. A precipitate thereof was filtered and washed with methanol. A yellow crystal was heated and dissolved in toluene, and then purified by a silica gel short column (eluent: toluene). The obtained crude product was added to toluene, and the mixture was concentrated. Thereafter, ethyl acetate was added, and subsequently solmix (A-11) was added thereto. Thereafter, ethyl acetate was distilled off, and a precipitated crystal was filtered and further washed with methanol to obtain compound (1-590) (4.0 g).

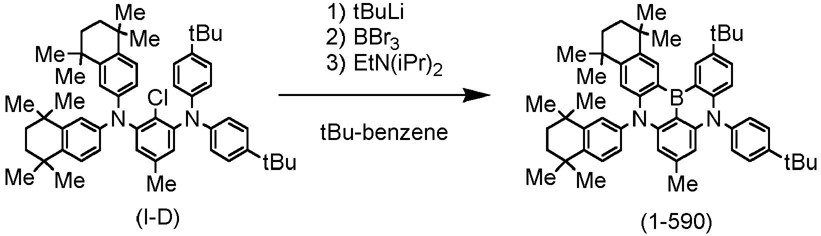

The structure of the obtained compound was confirmed by NMR measurement.
¹H-NMR (CDCl₃): δ = 1.08 (s, 6H), 1.27 (s, 6H), 1.42 (s, 6H), 1.46 (s, 9H), 1.47 (s, 9H), 1.48 (s, 6H), 1.69-1.81 (m, 8H), 2.18 (s, 3H), 5.97 (s, 1H), 6.06 (s, 1H), 6.52 (s, 1H), 6.67 (d, 1H), 7.08(dd, 1H), 7.25-7.29 (m, 3H), 7.48 (dd, 1H), 7.59 (d, 1H), 7.67 (d, 2H), 8.89 (s, 1H), 8.97 (d, 1H).

### Synthesis Example (3): Synthesis of compound (1-1301)

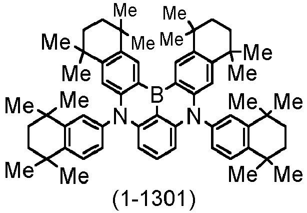

In a nitrogen atmosphere, compound (I-A) (8.0 g), compound (I-E) (5.6 g), tris(dibenzylideneacetone)dipalladium(0) [Pd₂(dba)₃, 0.16 g], dicyclohexyl(2',6'-dimethoxy-[1,1'-biphenyl]-2-yl]phosphine (SPhos, 0.24 g), NaOtBu (2.1 g), and xylene (40 ml) were put into a flask and heated at 120°C for one hour. After a reaction, to the reaction solution, water and toluene were added, and the mixture was stirred. Thereafter, an organic layer was separated from the mixture and washed with water. Thereafter, the organic layer was concentrated to obtain a crude product. The crude product was purified by a silica gel short column (eluent: toluene), and then a solvent was concentrated to obtain a crude product. To the crude product, heptane was added, and the mixture was cooled with ice. A precipitated crystal was filtered and washed with heptane to obtain compound (I-F) (10.5 g).

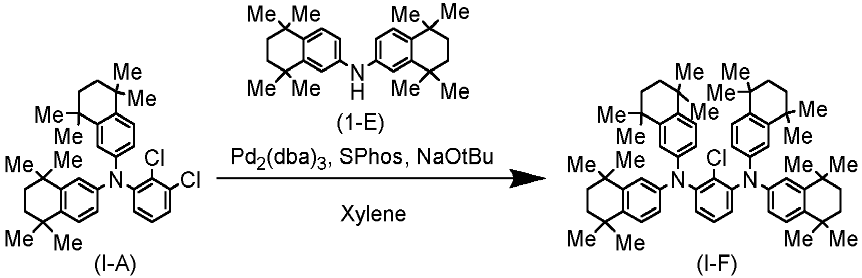

Into a flask containing compound (I-F) (10.5 g) and t-butylbenzene (200 ml), in a nitrogen atmosphere, a 1.53 M t-butyllithium pentane solution (14.4 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (5.9 g) was added thereto. The mixture was heated to room temperature and stirred for 0.5 hours. Thereafter, the resulting solution was cooled to 0°C again, N,N-diisopropylethylamine (3.1 g) was added thereto, and the mixture was stirred at room temperature until heat generation was stopped. Thereafter, the resulting solution was heated to 100°C and heated and stirred for one hour. The reaction solution was cooled to room temperature. To the reaction solution, a sodium acetate aqueous solution cooled in an ice bath was added, and subsequently ethyl acetate was added. The mixture was stirred for one hour. An organic layer was separated from the mixture and then washed with water twice. Thereafter, the organic layer was concentrated to obtain a crude product. The crude product was purified by a silica gel (NH silica) short column (eluent: toluene/ heptane = 1/9). The obtained crude product was concentrated, and then heptane was added thereto to precipitate a crystal. The crystal was filtered, and further washed with heptane to obtain compound (1-1301) (1.9 g).

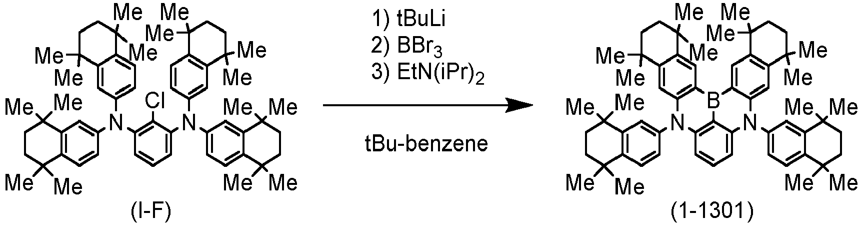

The structure of the obtained compound was confirmed by NMR measurement.
¹H-NMR (CDCl₃): δ = 1.09 (s, 12H), 1.27 (s, 12H), 1.42 (s, 12H), 1.49 (s, 12H), 1.70-1.80 (m, 16H), 6.20 (d, 2H), 6.56 (s, 2H),7.09 (dd, 2H), 7.25-7.30 (m, 3H), 7.58 (d, 2H), 8.91 (s, 2H) .

### Synthesis Example (4): Synthesis of compound (1-1302)

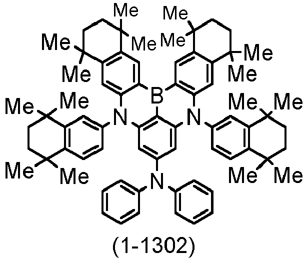

In a nitrogen atmosphere, compound (I-G) (8.0 g), compound (I-E) (19.7 g), Pd₂(dba)₃ (0.52 g), SPhos (0.94 g), NaOtBu (5.5 g), and xylene (80 ml) were put into a flask, heated and stirred at 120°C for three hours, and then stirred at 130°C for one hour. After a reaction, to the reaction solution, water and toluene were added, and the mixture was stirred. Thereafter, an organic layer was separated from the mixture and washed with water. Thereafter, the organic layer was concentrated to obtain a crude product. The crude product was purified by a silica gel column (eluent: toluene/heptane = 10/90 (volume ratio) → 15/85 (volume ratio)), and then a solvent was concentrated to obtain compound (I-H) (15.3 g).

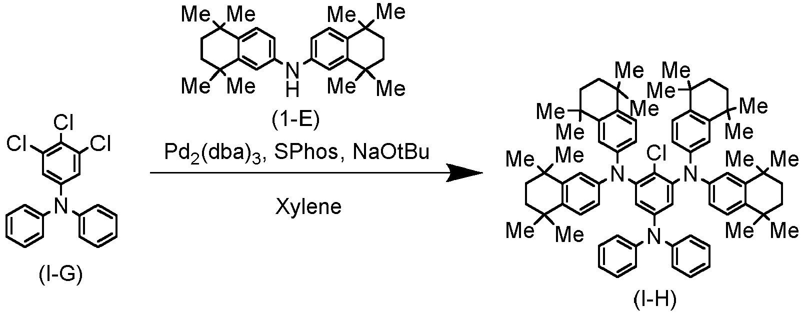

Into a flask containing compound (I-H) (15.3 g) and t-butylbenzene (100 ml), in a nitrogen atmosphere, a 1.53 M t-butyllithium pentane solution (13.2 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (5.3 g) was added thereto. The mixture was heated to room temperature and stirred for 0.5 hours. Thereafter, the resulting solution was cooled to 0°C again, N,N-diisopropylethylamine (2.7 g) was added thereto, and the mixture was stirred at room temperature until heat generation was stopped. Thereafter, the resulting solution was heated to 100°C and heated and stirred for one hour. The reaction solution was cooled to room temperature. To the reaction solution, a sodium acetate aqueous solution cooled in an ice bath was added, and subsequently ethyl acetate was added. The mixture was stirred for one hour. An organic layer was separated from the mixture and then washed with water (twice). To the organic layer, heptane was added to form a precipitate, and the precipitate was filtered. The precipitate was washed with methanol and water. An obtained yellow crystal was heated and dissolved in toluene, and then purified by a silica gel short column (eluent: toluene). A solvent was distilled off to obtain a crude product. To the crude product, heptane was added. An obtained crystal was filtered and then further washed with heptane to obtain compound (1-1302) (7.23 g).

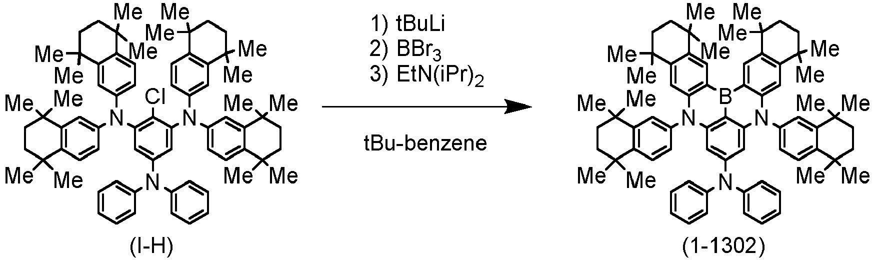

The structure of the obtained compound was confirmed by NMR measurement.
¹H-NMR (CDCl₃): δ = 1.07 (s, 12H), 1.11 (s, 6H), 1.19 (s, 6H), 1.24 (s, 6H), 1.35 (s, 6H), 1.48 (s, 12H), 1.68-1.75 (m, 16H), 5.63 (s, 2H), 6.55 (s, 2H),6.89 (t, 2H), 6.91-6.92 (m, 2H), 6.93-6.95 (m, 4H), 7.04 (t, 4H), 7.13 (dd, 2H), 7.37 (d, 2H), 8.85 (s, 2H).

### Synthesis Example (5): Synthesis of compound (1-1823)

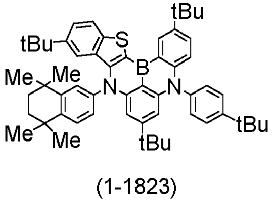

In a nitrogen atmosphere, compound (I-I) (35.0 g), 3-bromo-5-t-butyl-benzo[b]thiophene (compound (I-J, 17.4 g), Pd(dba)₂ (1.86 g), NaOtBu (10.4 g), tri-t-butylphosphonium tetrafluoroborate([(tBu)₃PH]BF₄): 1.87 g, and xylene (300 ml) were put into a flask and heated at 120°C for one hour. After a reaction, to the reaction solution, water and toluene were added, and the mixture was stirred. Thereafter, an organic layer was separated from the mixture and washed with water. Thereafter, the organic layer was concentrated to obtain a crude product. The crude product was purified by a silica gel column (eluent: toluene/heptane = 1/4 (volume ratio)), and then a solvent was concentrated. A glass-like solid was dried by a vacuum pump to obtain compound (I-K) (35 g).

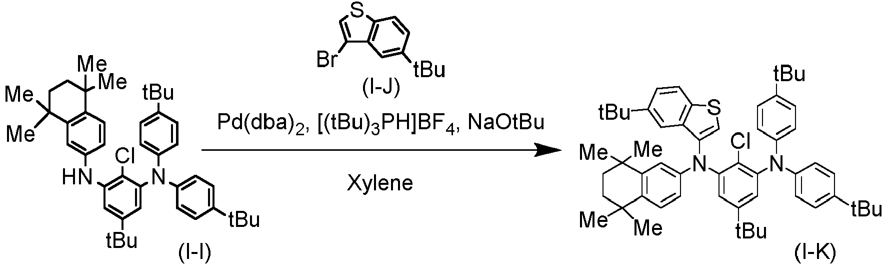

Into a flask containing compound (I-K) (16.7 g) and t-butylbenzene (400 ml), in a nitrogen atmosphere, a 1.60 M t-butyllithium pentane solution (25.0 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (10.0 g) was added thereto. The mixture was heated to room temperature and stirred for 0.5 hours. Thereafter, the resulting solution was cooled to 0°C again, N,N-diisopropylethylamine (5.2 g) was added thereto, and the mixture was stirred at room temperature until heat generation was stopped. Thereafter, the resulting solution was heated to 90°C and heated and stirred for one hour. The reaction solution was cooled to room temperature. To the reaction solution, a sodium acetate aqueous solution cooled in an ice bath was added, and subsequently toluene was added. The mixture was stirred for one hour. The toluene solution was concentrated, and then heptane was added thereto. The mixture was purified by a silica gel column (eluent: toluene/heptane = 1/4 (volume ratio)). The obtained crude product was recrystallized by toluene repeatedly to obtain compound (1-1823) (2.8 g).

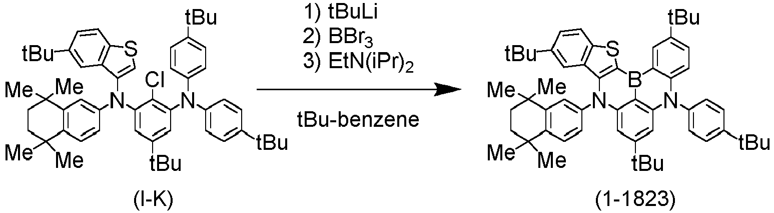

The structure of the obtained compound was confirmed by NMR measurement.
¹H-NMR (CDCl₃): δ = 0.99 (s, 9H), 1.11 (s, 9H), 1.30 (s, 6H), 1.43 (s, 3H), 1.46 (m, 12H), 1.51 (s, 9H), 1.78-1.86 (m, 4H), 6.15 (s, 1H), 6.65 (s, 1H),6.74 (s, 1H), 7.23-7.29 (d, 4H), 7.41 (d, 1H), 7.46 (d, 1H), 7.53 (s, 1H), 7.66-7.68 (m, 3H), 7.90 (d, 1H), 8.78 (s, 1H).

### Synthesis Example (6): Synthesis of compound (1-1821)

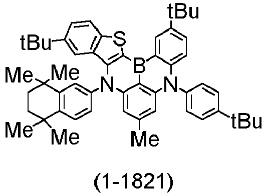

In a nitrogen atmosphere, compound (I-L) (71.0 g), compound (I-J) (37.8 g), Pd(dba)₂ (2.14 g), NaOtBu (16.9 g), [(tBu)₃PH]BF₄) (1.36 g), and xylene (710 ml) were put into a flask and heated and refluxed at 130°C for one hour. The reaction solution was cooled to room temperature, and then water and toluene were added thereto. An organic layer was separated from the mixture and washed with water. This solution was concentrated under reduced pressure. The residue was purified by a silica gel column (eluent: toluene), and further washed with heptane to obtain compound (I-M) (71.3 g).

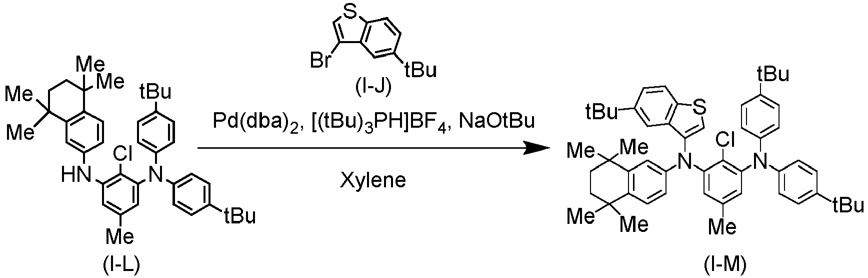

Into a flask containing compound (I-M) (32. 0 g) and t-butylbenzene (400 ml), in a nitrogen atmosphere, a 1.60 M t-butyllithium pentane solution (50.0 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (20.0 g) was added thereto. The mixture was heated to room temperature and stirred for 0.5 hours. Thereafter, the resulting solution was cooled to 0°C again, N,N-diisopropylethylamine (10.4 g) was added thereto, and the mixture was stirred at room temperature until heat generation was stopped. Thereafter, the resulting solution was heated to 70°C and heated and stirred for one hour. The reaction solution was cooled to room temperature. To the reaction solution, a sodium acetate aqueous solution cooled in an ice bath was added, and subsequently ethyl acetate was added. The mixture was stirred for one hour. An organic layer was separated from the mixture and washed with water. This solution was concentrated under reduced pressure. The residue was washed with ethyl acetate and then purified by a silica gel column (eluent: toluene/heptane = 1/1 (volume ratio)). The obtained crude product was washed with heptane to obtain compound (1-1821) (7.1 g).

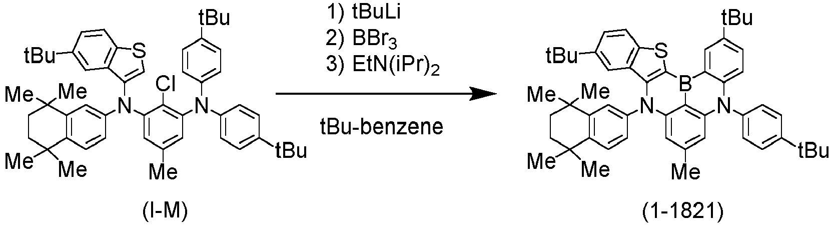

### Synthesis Example (7): Synthesis of compound (1-3623)

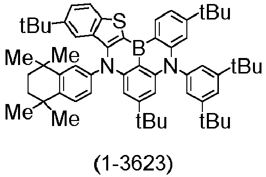

In a nitrogen atmosphere, compound (I-N) (60.0 g), compound (I-J) (28.6 g), Pd(dba)₂ (2.93 g), NaOtBu (12.3 g), [(tBu)₃PH]BF₄) (2.96 g), and xylene (660 ml) were put into a flask and heated at 120°C for two hours. The reaction solution was cooled to room temperature, and then water and toluene were added thereto. An organic layer was separated from the mixture and washed with water. This solution was concentrated under reduced pressure. The residue was purified by a silica gel column (eluent: toluene/heptane = 1/3 (volume ratio)), and further washed with methanol to obtain compound (I-O) (68.0 g).

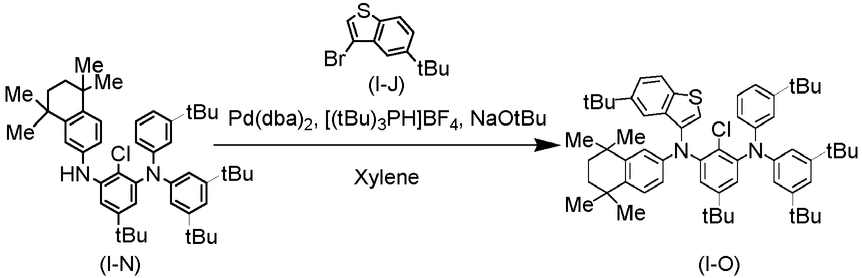

Into a flask containing compound (I-O) (35.8 g) and t-butylbenzene (800 ml), in a nitrogen atmosphere, a 1.61 M t-butyllithium pentane solution (50.0 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (20.0 g) was added thereto. The mixture was heated to room temperature and stirred for 0.5 hours. Thereafter, the resulting solution was heated to 50°C and heated and stirred for 0.5 hours. The reaction solution was cooled to room temperature. To the reaction solution, a sodium acetate aqueous solution cooled in an ice bath was added, and subsequently ethyl acetate was added. The mixture was stirred for one hour. An organic layer was separated from the mixture and washed with water. This solution was concentrated under reduced pressure. The residue was washed with ethyl acetate and then purified by a silica gel column (eluent: toluene). The obtained product was further purified by recrystallization from toluene to obtain compound (1-3623) (9.3 g).

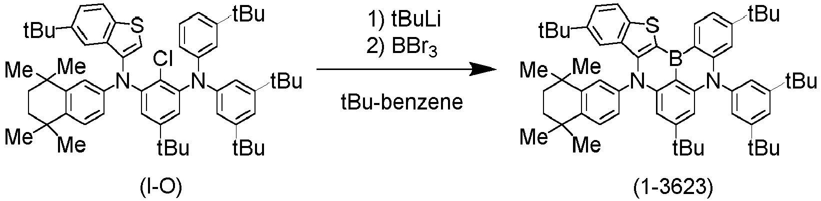

### Synthesis Example (8): Synthesis of compound (1-3621)

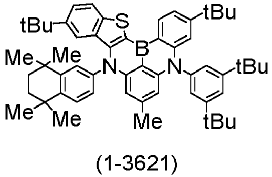

In a nitrogen atmosphere, compound (I-P) (65.0 g), compound (I-J) (31.7 g), Pd(dba)₂ (1.79 g), NaOtBu (14.1 g), [(tBu)₃PH]BF₄ (1.14 g), and xylene (520 ml) were put into a flask and heated at 130°C for two hours. The reaction solution was cooled to room temperature, and then water and toluene were added thereto. An organic layer was separated from the mixture and washed with water. This solution was concentrated under reduced pressure. The residue was purified by a silica gel column (eluent: toluene/heptane = 1/4 (volume ratio)). A glass-like solid was dried by a vacuum pump to obtain compound (I-Q) (52 g) .

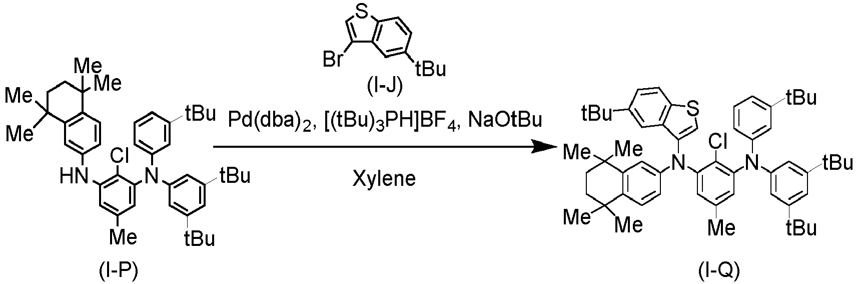

Into a flask containing compound (I-Q) (35.0 g) and t-butylbenzene (280 ml), in a nitrogen atmosphere, a 1.60 M t-butyllithium pentane solution (50.0 ml) was added at 0°C. The mixture was heated to 60°C and stirred for 0.5 hours. Subsequently, the mixture was cooled to -50°C, and boron tribromide (20.6 g) was added thereto. The mixture was heated to room temperature and stirred for 0.5 hours. Thereafter, the resulting solution was cooled to 0°C again, N,N-diisopropylethylamine (10.6 g) was added thereto, and the mixture was stirred at room temperature until heat generation was stopped. Thereafter, the resulting solution was heated to 90°C and heated and stirred for one hour. The reaction solution was cooled to room temperature. To the reaction solution, a sodium acetate aqueous solution cooled in an ice bath was added, and subsequently ethyl acetate was added. The mixture was stirred for one hour. An organic layer was separated from the mixture and washed with water. This solution was concentrated under reduced pressure. The residue was washed with ethyl acetate and then purified by a silica gel column (eluent: toluene/heptane = 1/1 (volume ratio)). The obtained product was further purified by recrystallization from toluene to obtain compound (1-3621) (7.2 g).

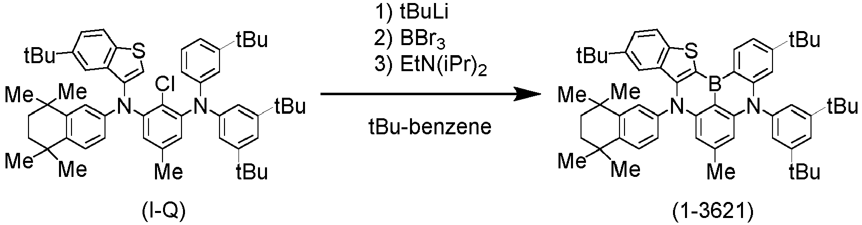

The structure of the obtained compound was confirmed by NMR measurement.
¹H-NMR (CDCl₃): δ = 1.11 (s, 9H), 1.19 (s, 9H), 1.26 (s, 3H), 1.28 (s, 3H), 1.37 (s, 18H), 1.44-1.47 (m, 6H), 1.77-1.86 (m, 4H), 2.21 (s, 3H), 6.08 (s, 1H), 6.10 (s, 1H), 6.53 (d, 1H), 6.61 (d, 1H), 7.20 (d, 2H), 7.26 (dd, 1H), 7.31 (dd, 1H), 7.40 (dd, 1H), 7.48 (d , 1H), 7.61 (t, 1H),7.66 (d, 1H), 7.88 (d, 1H), 8.69 (d, 1H) .

### Synthesis Example (9): Synthesis of compound (1-1828)

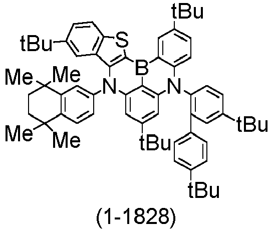

In a nitrogen atmosphere, compound (I-R) (63.8 g), compound (I-J) (24.2 g), Pd(dba)₂ (0.939 g), NaOtBu (11.8 g), tri-t-butylphosphine ((tBu)₃P: 3.27 ml), and toluene (630 ml) were put into a flask and heated at 110°C for four hours. The reaction solution was cooled to room temperature, and then water and toluene were added thereto. An organic layer was separated from the mixture and washed with water. This solution was concentrated under reduced pressure. The residue was purified by a silica gel column (eluent: toluene/heptane = 1/6 (volume ratio)). A glass-like solid was dried by a vacuum pump to obtain compound (I-S) (44 g).

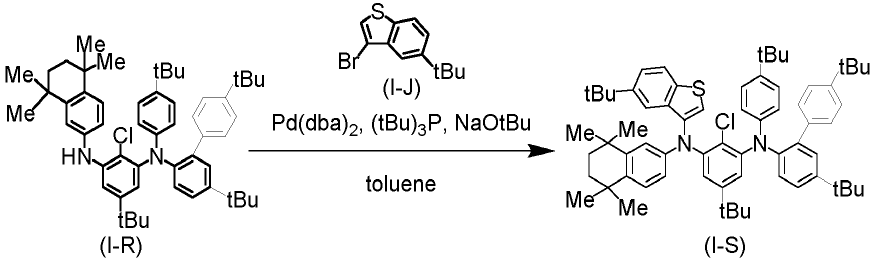

Into a flask containing compound (I-S) (44.3 g) and t-butylbenzene (400 ml), in a nitrogen atmosphere, a 1.61 M t-butyllithium pentane solution (56.7 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (22.9 g) was added thereto. The mixture was heated to room temperature and stirred for 0.5 hours. Thereafter, the resulting solution was cooled to 0°C again, N,N-diisopropylethylamine (16.0 g) was added thereto, and the mixture was stirred at room temperature until heat generation was stopped. Thereafter, the resulting solution was heated to 120°C and heated and stirred for one hour. The reaction solution was cooled to room temperature. To the reaction solution, a sodium acetate aqueous solution cooled in an ice bath and toluene were added. The mixture was stirred. Thereafter, an organic layer was separated from the mixture and washed with water. Thereafter, the organic layer was concentrated to obtain a crude product. The crude product was purified by a silica gel column (eluent: toluene/heptane = 1/4 (volume ratio)) and further purified by recrystallization from a mixed solvent of toluene and heptane (toluene/heptane = 1/1 (volume ratio)) to obtain compound (1-1828) (5.0 g).

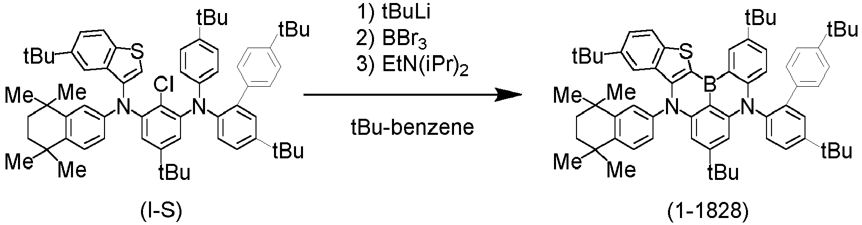

The structure of the obtained compound was confirmed by NMR measurement.
¹H-NMR (CDCl₃): δ = 0.96-0.99 (m, 9H), 1.10 (s, 9H), 1.12 (s, 9H), 1.25-1.32 (m, 6H), 1.39-1.51 (m, 24H), 1.76-1.86 (m, 4H), 6.05-6.28 (m, 2H), 6.66-6.80 (m, 2H), 7.01-7.08 (m, 2H), 7.12-7.24 (m, 4H), 7.37-7.47 (m, 2H), 7.48-7.69 (m, 3H), 7.70 (s, 1H), 7.88 (d, 1H), 8.70 (s, 1H) .

### Synthesis Example (10): Synthesis of compound (1-3604)

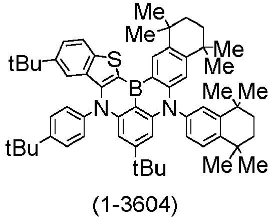

In a nitrogen atmosphere, compound (I-T) (49.7 g), compound (I-J) (22.8 g), Pd(dba)₂ (2.44 g), NaOtBu (13.6 g), [(tBu)₃PH]BF₄ (2.46 g), and xylene (600 ml) were put into a flask and heated at 120°C for three hours. The reaction solution was cooled to room temperature, and then water and toluene were added thereto. An organic layer was separated from the mixture and washed with water. This solution was concentrated under reduced pressure. The residue was purified by a silica gel column (eluent: heptane) and further purified by recrystallization from heptane to obtain compound (I-U) (45 g).

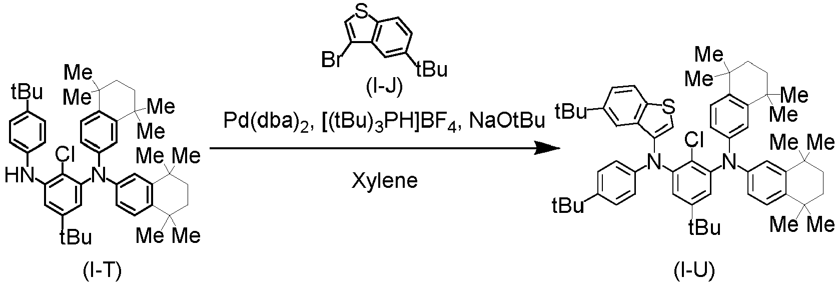

Into a flask containing compound (I-U) (40.4 g) and t-butylbenzene (270 ml), in a nitrogen atmosphere, a 1.60 M t-butyllithium pentane solution (50.0 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (20.0 g) was added thereto. The mixture was heated to 50°C and stirred for 0.5 hours. Thereafter, the resulting solution was cooled to 0°C again, N,N-diisopropylethylamine (10.4 g) was added thereto, and the mixture was stirred at room temperature until heat generation was stopped. Thereafter, the resulting solution was heated to 80°C and heated and stirred for 0.5 hours. The reaction solution was cooled to room temperature. To the reaction solution, a sodium acetate aqueous solution cooled in an ice bath was added, and subsequently ethyl acetate was added. The mixture was stirred for one hour. An organic layer was separated from the mixture and washed with water. This solution was concentrated under reduced pressure. The residue was washed with ethyl acetate, then purified by a silica gel column (eluent: toluene), and further purified by recrystallization from toluene to obtain compound (1-3604) (3.8 g).

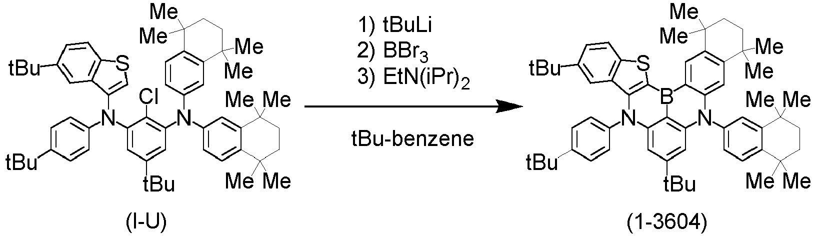

### Synthesis Example (11): Synthesis of compound (1-1851)

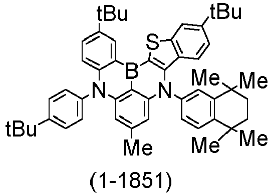

In a nitrogen atmosphere, compound (I-L) (29.0 g), compound (I-V) (18.9 g), Pd(dba)₂ (0.55 g), NaOtBu (6.9 g), [(tBu)₃PH]BF₄ (0.55 g), and xylene (100 ml) were put into a flask and heated and refluxed at 120°C for one hour. The reaction solution was cooled to room temperature, and then water and ethyl acetate were added thereto. An organic layer was separated from the mixture and washed with water. This solution was concentrated under reduced pressure. The residue was purified by a silica gel column (eluent: toluene/heptane = 1/1 (volume ratio)) and concentrated. Thereafter, ethyl acetate was added thereto to obtain a crystal. The crystal was filtered and then washed with methanol to obtain compound (I-W) (25.0 g).

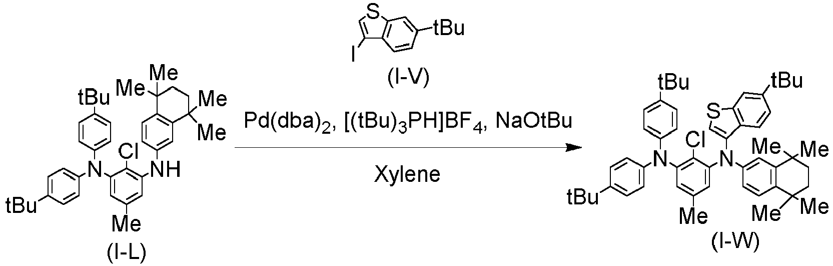

Into a flask containing compound (I-W) (25.0 g) and t-butylbenzene (160 ml), in a nitrogen atmosphere, a 1.60 M t-butyllithium pentane solution (39.3 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (15.7 g) was added thereto. The mixture was heated to room temperature and stirred for 0.5 hours. Thereafter, the resulting solution was cooled to 0°C again, N,N-diisopropylethylamine (8.1 g) was added thereto, and the mixture was stirred at room temperature until heat generation was stopped. Thereafter, the resulting solution was heated to 80°C and heated and stirred for one hour. The reaction solution was cooled to room temperature. To the reaction solution, a sodium acetate aqueous solution cooled in an ice bath was added, and subsequently ethyl acetate was added. The mixture was stirred for one hour. An organic layer was separated from the mixture and washed with water. This solution was concentrated under reduced pressure. The residue was washed with ethyl acetate and then purified by a silica gel column (eluent: toluene/heptane = 2/8 (volume ratio)). The obtained crude product was washed with heptane to obtain compound (1-1851) (7.5 g).

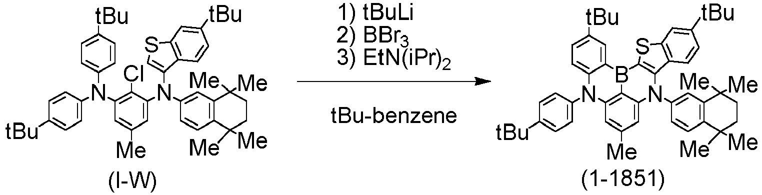

The structure of the obtained compound was confirmed by NMR measurement.
¹H-NMR (CDCl₃): δ = 1.23 (s, 3H), 1.29 (s, 3H), 1.36 (s, 9H), 1.46 (s, 9H), 1.49 (s, 6H), 1.51 (s, 9H), 1.82-1.89 (m, 4H), 2.22 (s, 3H), 5.80 (d, 1H), 5.99 (s, 1H), 6.24 (s, 1H), 6.56 (d, 1H), 6.92 (d, 1H), 7.20 (dd, 1H), 7.22-7.28 (m, 2H), 7.44-7.47 (m, 2H), 7.63 (d, 1H), 7.68 (d, 2H), 7.96 (s, 1H), 8.78 (d, 1H).

### Synthesis Example (12): Synthesis of compound (1-5121)

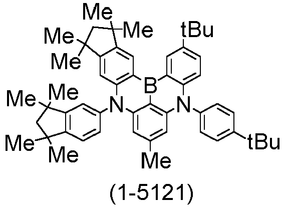

In a nitrogen atmosphere, compound (I-X) (10.0 g), bis(4-t-butylphenyl)amine (5.15 g), Pd-132 (0.13 g), NaOtBu (2.6 g), and xylene (40 ml) were put into a flask and heated at 120°C for one hour. After a reaction, to the reaction solution, water and ethyl acetate were added, and the mixture was stirred. Thereafter, an organic layer was separated from the mixture and washed with water. Thereafter, the organic layer was concentrated to obtain a crude product. To the crude product, solmix was added. A precipitated solid was separated by filtration and washed with methanol. Thereafter, the solid was purified by a silica gel short column (eluent: toluene/heptane = 1/1 (volume ratio)), and then a solvent was concentrated to obtain a crude product. Thereafter, to the crude product, ethyl acetate was added to precipitate a crystal. The crystal was filtered and washed with methanol to obtain compound (I-Y) (8.5 g).

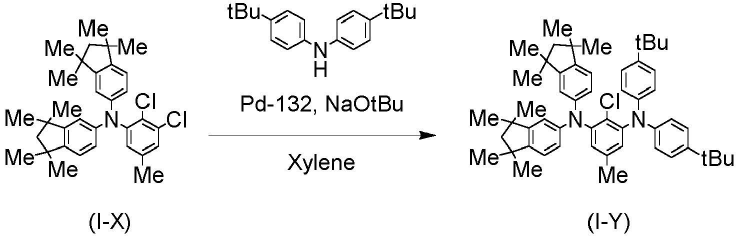

Into a flask containing compound (I-Y) (8.5 g) and t-butylbenzene (85 ml), in a nitrogen atmosphere, a 1.60 M t-butyllithium pentane solution (13.9 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (5.6 g) was added thereto. The mixture was heated to room temperature and stirred for 0.5 hours. Thereafter, the resulting solution was cooled to 0°C again, N,N-diisopropylethylamine (2.9 g) was added thereto, and the mixture was stirred at room temperature until heat generation was stopped. Thereafter, the resulting solution was heated to 80°C and heated and stirred for one hour. The reaction solution was cooled to room temperature. To the reaction solution, a sodium acetate aqueous solution cooled in an ice bath was added, and subsequently ethyl acetate was added. The mixture was stirred for one hour. An organic layer was washed with water twice and then concentrated to obtain a crude product. After concentration, solmix was added to precipitate a solid. The solid was filtered. Thereafter, the crude product was purified by a silica gel column (eluent: toluene/heptane = 1/1 (volume ratio)). A step of adding solmix to an obtained crude product and filtering an obtained crystal was repeated to obtain compound (1-5121) (3.0 g).

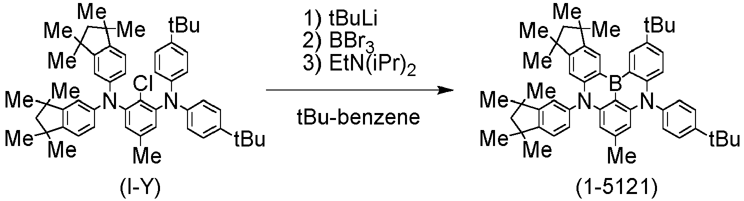

The structure of the obtained compound was confirmed by NMR measurement.
¹H-NMR (CDCl₃): δ = 1.19 (s, 6H), 1.32 (s, 6H), 1.46 (s, 30H), 1.97 (s, 2H), 2.06 (s, 2H), 2.18 (s, 3H), 5.95 (s, 1H), 6.05 (s, 1H), 6.36 (s, 1H), 6.66 (d, 1H), 7.10 (d, 1H), 7.18 (dd, 1H), 7.27 (d, 2H), 7.39 (d, 1H), 7.48 (d, 1H), 7.67 (d, 2H), 8.67 (s, 1H), 8.96 (d, 1H).

### Synthesis Example (13): Synthesis of compound (1-2663)

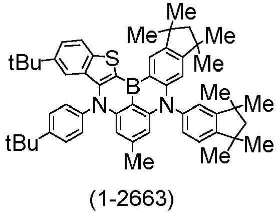

In a nitrogen atmosphere, compound (I-Z) (20.0 g), compound (I-J) (10.2 g), Pd(dba)₂ (0.54 g), NaOtBu (4.6 g), [(tBu)₃PH]BF₄ (0.55 g), and xylene (60 ml) were put into a flask and heated and refluxed at 120°C for one hour. The reaction solution was cooled to room temperature, and then water and ethyl acetate were added thereto. An organic layer was separated from the mixture and washed with water. This solution was concentrated under reduced pressure. The residue was purified by a silica gel column (eluent: toluene/heptane = 1/1 (volume ratio)) and concentrated. Thereafter, solmix was added thereto to obtain a crystal. The crystal was filtered and then washed with methanol to obtain compound (I-1) (24.0 g).

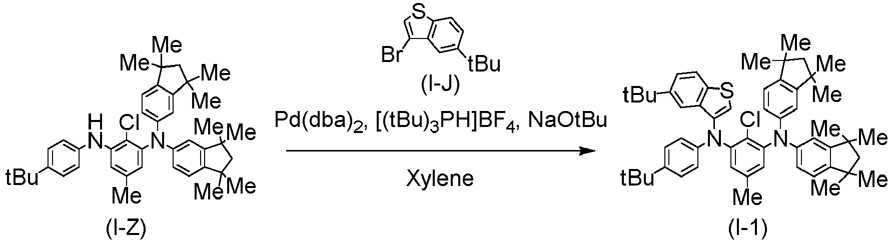

Into a flask containing compound (I-1) (24.0 g) and t-butylbenzene (200 ml), in a nitrogen atmosphere, a 1.60 M t-butyllithium pentane solution (36.5 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (14.6 g) was added thereto. The mixture was heated to room temperature and stirred for 0.5 hours. Thereafter, the resulting solution was cooled to 0°C again, N,N-diisopropylethylamine (7.6 g) was added thereto, and the mixture was stirred at room temperature until heat generation was stopped. Thereafter, the resulting solution was heated to 80°C and heated and stirred for one hour. The reaction solution was cooled to room temperature. To the reaction solution, a sodium acetate aqueous solution cooled in an ice bath was added, and subsequently ethyl acetate was added. The mixture was stirred for one hour. An organic layer was separated from the mixture and washed with water. This solution was concentrated under reduced pressure. To the residue, heptane was added, and a precipitated solid was washed with heptane and then purified by a silica gel column (eluent: toluene/heptane = 1/1 (volume ratio)). A step of adding heptane to an obtained crude product to precipitate a solid and washing the precipitated solid with heptane was repeated to obtain compound (1-2663) (5.7 g).

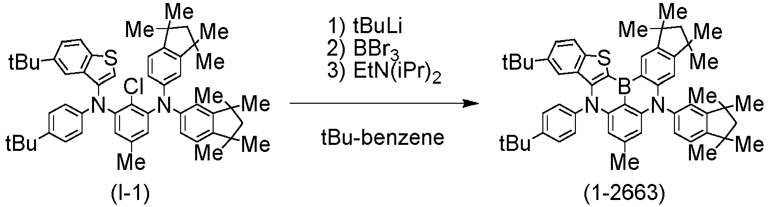

The structure of the obtained compound was confirmed by NMR measurement.
¹H-NMR (CDCl₃): δ = 1.09 (s, 9H), 1.18 (d, 6H), 1.32 (d, 6H), 1.46 (d, 6H), 1.50 (s, 9H), 1.52 (s, 6H), 1.96 (s, 2H), 2.06 (s, 2H), 2.21 (s, 3H), 6.07 (s, 2H), 6.25 (s, 1H), 6.51 (s, 1H), 7.08 (d, 1H), 7.17 (dd, 1H), 7/38-7.41 (m, 2H), 7.47 (d, 2H), 7.65 (d, 2H), 7.88 (d, 1H), 8.51 (s, 1H).

### Synthesis Example (14): Synthesis of compound (1-1826)

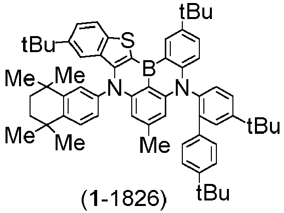

Into a flask containing compound (I-2) (55.5 g) and t-butylbenzene (450 ml), in a nitrogen atmosphere, a 1.60 M t-butyllithium pentane solution (75.0 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (30.0 g) was added thereto. The mixture was heated to room temperature and stirred for 0.5 hours, and then heated to 80°C and heated and stirred for one hour. The reaction solution was cooled to room temperature. To the reaction solution, a sodium acetate aqueous solution cooled in an ice bath and toluene were added. The mixture was stirred. Thereafter, an organic layer was separated from the mixture and washed with water. Thereafter, the organic layer was concentrated, and the residue was purified by a silica gel column (eluent: toluene/heptane = 1/1 (volume ratio)). The obtained crude product was sequentially washed with acetone and heptane to obtain compound (1-1826) (6.60 g).

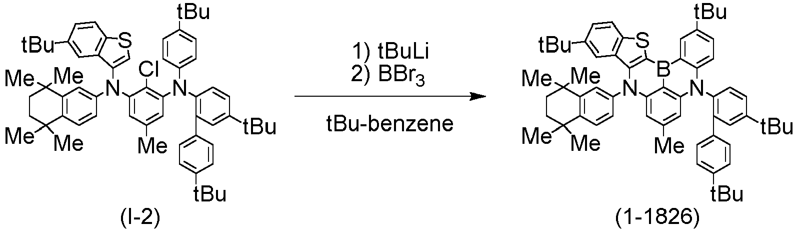

The structure of the obtained compound was confirmed by NMR measurement.
¹H-NMR (CDCl₃): δ = 1.09-1.16 (m, 18H), 1.25-1.34 (m, 6H), 1.42-1.50 (m, 24H), 1.76-1.86 (m, 4H), 2.19 (s, 3H), 6.03-6.12 (m, 2H), 6.57-6.66 (m, 2H), 7.01-7.08 (m, 2H), 7.12-7.25 (m, 4H), 7.37-7.44 (m, 2H), 7.48-7.54 (m, 1H), 7.58 (dd, 1H), 7.60-7.67 (m, 1H), 7.67-7.71 (m, 1H), 7.88 (d, 1H), 8.68-8.72 (m, 1H).

### Synthesis Example (15): Synthesis of compound (1-3645)

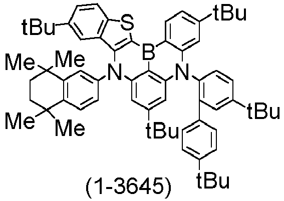

Into a flask containing compound (I-3) (58.5 g) and t-butylbenzene (300 ml), in a nitrogen atmosphere, a 1.60 M t-butyllithium pentane solution (75.0 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (30.0 g) was added thereto. The mixture was heated to room temperature and stirred for 0.5 hours. Thereafter, the resulting solution was cooled to 0°C again, N,N-diisopropylethylamine (15.6 g) was added thereto, and the mixture was stirred at room temperature until heat generation was stopped. Thereafter, the resulting solution was heated to 80°C and heated and stirred for one hour. The reaction solution was cooled to room temperature. To the reaction solution, a sodium acetate aqueous solution cooled in an ice bath and toluene were added. The mixture was stirred. Thereafter, an organic layer was separated from the mixture and washed with water. Thereafter, the organic layer was concentrated, and the residue was purified by a silica gel column (eluent: toluene/heptane = 1/1 (volume ratio)). The obtained crude product was washed with heptane to obtain compound (1-3645) (10.2 g).

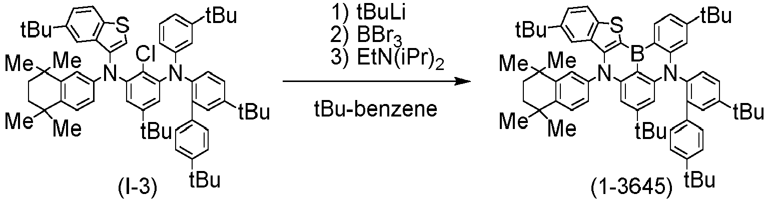

The structure of the obtained compound was confirmed by NMR measurement.
¹H-NMR (CDCl₃) : δ = 0.98-1.02 (m, 9H), 1.09-1.14 (m, 18H), 1.20 (s, 9H), 1.26-1.34 (m, 6H), 1.39-1.50 (m, 15H), 1.75-1.88 (m, 4H), 6.13-6.35 (m, 2H), 6.66-6.73 (m, 2H), 7.00-7.07 (m, 2H), 7.12-7.26 (m, 5H), 7/39 (dd, 1H), 7.48-7.79 (m, 3H), 7.71 (s, 1H), 7.86 (d, 1H), 8.58 (d, 1H).

### Synthesis Example (16): Synthesis of compound (1-5142)

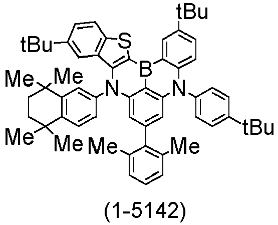

Into a flask containing compound (I-4) (51.0 g) and t-butylbenzene (610 ml), in a nitrogen atmosphere, a 1.61 M t-butyllithium pentane solution (75.0 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (30.0 g) was added thereto. The mixture was heated to room temperature and stirred for 0.5 hours. Thereafter, the resulting solution was cooled to 0°C again, N,N-diisopropylethylamine (14.9 g) was added thereto, and the mixture was stirred at room temperature until heat generation was stopped. Thereafter, the resulting solution was heated to 80°C and heated and stirred for one hour. The reaction solution was cooled to room temperature. To the reaction solution, a sodium acetate aqueous solution cooled in an ice bath and toluene were added. The mixture was stirred. Thereafter, an organic layer was separated from the mixture and washed with water. Thereafter, the organic layer was concentrated, and the residue was purified by a silica gel column (eluent: toluene/heptane = 7/1 (volume ratio)). The obtained crude product was washed with heptane and further purified by recrystallization from toluene to obtain compound (1-5142) (6.51 g).

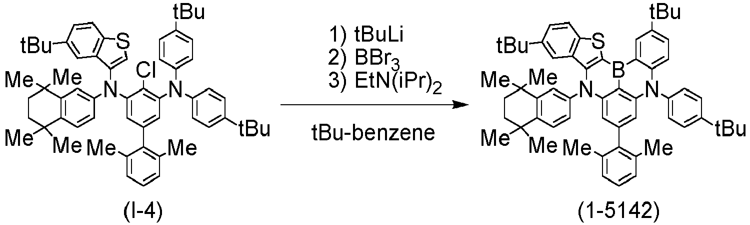

The structure of the obtained compound was confirmed by NMR measurement.
¹H-NMR (CDCl₃): δ = 1.11 (s, 9H), 1.18-1.24 (m, 6H), 1.37-1.45 (m, 15H), 1.51 (s, 9H), 1.71-1.80 (m, 4H), 1.94 (s, 6H), 6.07 (s, 1H), 6.11 (s, 1H), 6.48-6.55 (m, 2H), 6.97-7.09 (m, 3H), 7.23 (d, 2H), 7/30 (dd, 1H), 7/39-7.43 (m, 2H), 7.46 (dd, 1H), 7.57-7.64 (m, 3H), 7.91 (d, 1H), 8.82 (d, 1H).

### Synthesis Example (17): Synthesis of compound (1-5151)

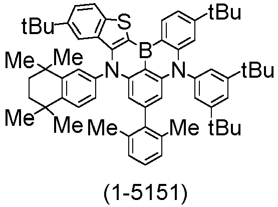

Into a flask containing compound (I-5) (56.5 g) and t-butylbenzene (670 ml), in a nitrogen atmosphere, a 1.61 M t-butyllithium pentane solution (75.0 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (30.0 g) was added thereto. The mixture was heated to room temperature and stirred for 0.5 hours. Thereafter, the resulting solution was cooled to 0°C again, N,N-diisopropylethylamine (15.5 g) was added thereto, and the mixture was stirred at room temperature until heat generation was stopped. Thereafter, the resulting solution was heated to 80°C and heated and stirred for one hour. The reaction solution was cooled to room temperature. To the reaction solution, a sodium acetate aqueous solution cooled in an ice bath and toluene were added. The mixture was stirred. Thereafter, an organic layer was separated from the mixture and washed with water. Thereafter, the organic layer was concentrated, and the residue was purified by a silica gel column (eluent: toluene/heptane = 7/1 (volume ratio)). The obtained crude product was sequentially washed with acetone and heptane to obtain compound (1-5151) (6.34 g).

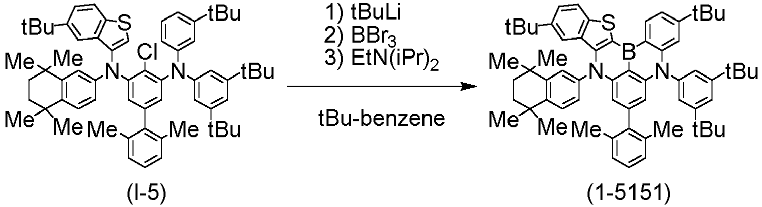

The structure of the obtained compound was confirmed by NMR measurement.
¹H-NMR (CDCl₃): δ = 1.11 (s, 9H), 1.17-1.24 (m, 15H), 1.32 (s, 18H), 1.37-1.45 (m, 6H), 1.71-1.80 (m, 4H), 1.97 (s, 6H), 6.11 (s, 1H), 6.14 (s, 1H), 6.52 (dd, 2H), 6.97-7.09 (m, 3H), 7.18 (d, 2H), 7.28-7/34 (m, 2H), 7/38-7.43 (m, 2H), 7.52 (t, 1H), 7.60 (d, 1H), 7.89 (d, 1H), 8.72 (d, 1H).

### Synthesis Example (18): Synthesis of compound (1-1803)

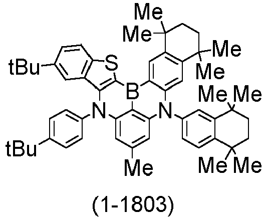

Into a flask containing compound (I-6) (86.6 g) and t-butylbenzene (520 ml), in a nitrogen atmosphere, a 1.61 M t-butyllithium pentane solution (125 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (51.1 g) was added thereto. The mixture was heated to room temperature and stirred for 2.5 hours. To the reaction solution, a sodium acetate aqueous solution cooled in an ice bath and toluene were added. The mixture was stirred. Thereafter, an organic layer was separated from the mixture and washed with water. Thereafter, the organic layer was concentrated, and the residue was purified by a silica gel column (eluent: toluene/heptane = 1/1 (volume ratio)). The obtained crude product was sequentially washed with acetonitrile and heptane and further purified by recrystallization from toluene to obtain compound (1-1803) (10.2 g).

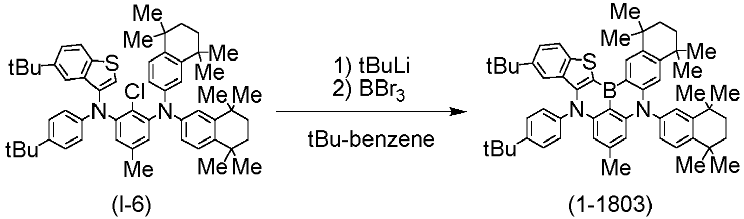

The structure of the obtained compound was confirmed by NMR measurement.
¹H-NMR (CDCl₃): δ = 1.04-1.10 (m, 15H), 1.25 (s, 6H), 1.38-1.45 (m, 6H), 1.49 (s, 9H), 1.53 (s, 6H), 1.66-1.83 (m, 8H), 2.20 (s, 3H), 6.06 (dd, 2H), 6.41 (s, 1H), 6.50 (d, 1H), 7.07 (dd, 1H), 7.26 (d, 1H), 7/38 (dd, 1H), 7.46 (d, 2H), 7.59 (d, 1H), 7.74 (d, 2H), 7.88 (d, 1H), 8.70 (d, 1H).

### Synthesis Example (19): Synthesis of compound (1-3644)

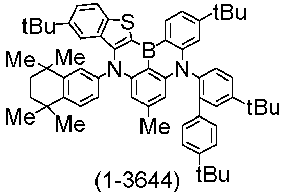

Into a flask containing compound (I-7) (110 g) and t-butylbenzene (1000 ml), in a nitrogen atmosphere, a 1.61 M t-butyllithium pentane solution (150 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (60.0 g) was added thereto. The mixture was heated to room temperature and stirred for 0.5 hours. Thereafter, the resulting solution was cooled to 0°C again, N,N-diisopropylethylamine (30.6 g) was added thereto, and the mixture was stirred at room temperature until heat generation was stopped. Thereafter, the resulting solution was heated to 80°C and heated and stirred for one hour. The reaction solution was cooled to room temperature. To the reaction solution, a sodium acetate aqueous solution cooled in an ice bath and toluene were added. The mixture was stirred. Thereafter, an organic layer was separated from the mixture and washed with water. Thereafter, the organic layer was concentrated, and the residue was purified by a silica gel column (eluent: toluene/heptane = 1/5 (volume ratio)). The obtained crude product was sequentially washed with acetonitrile, acetone, and heptane to obtain compound (1-3644) (11.1 g).

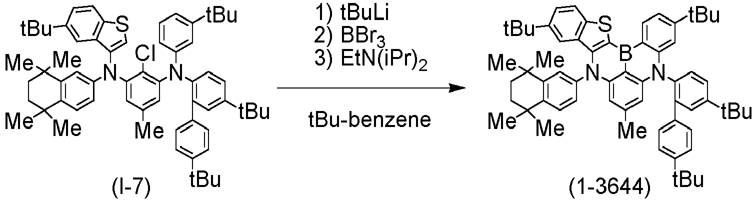

The structure of the obtained compound was confirmed by NMR measurement.
¹H-NMR (CDCl₃): δ = 1.10 (s, 9H), 1.11-1.13 (m, 9H), 1.16-1.18 (m, 9H), 1.25-1.36 (m, 6H), 1.43-1.47 (m, 6H), 1.48 (s, 9H), 1.77-1.87 (m, 4H), 2.22 (s, 3H), 6.07-6.11 (m, 1H), 6.20 (s, 1H), 6.54-6.62 (m, 2H), 6.99-7.08 (m, 2H), 7.15-7.30 (m, 5H), 7.37 (dd, 1H), 7.46-7.56 (m, 1H), 7.58-7.68 (m, 2H), 7.69-7.73 (m, 1H), 7.85 (d, 1H), 8.59-8.55 (m, 1H).

### Synthesis Example (20): Synthesis of compound (1-3812)

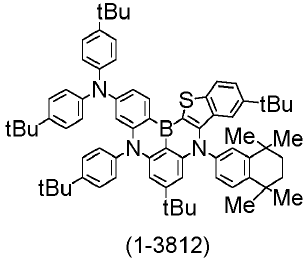

Into a flask containing compound (I-8) (21.2 g) and t-butylbenzene (170 ml), in a nitrogen atmosphere, a 1.61 M t-butyllithium pentane solution (25.0 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (10.0 g) was added thereto. The mixture was heated to room temperature and stirred for 0.5 hours. Thereafter, the mixture was heated to 80°C and heated and stirred for one hour. The reaction solution was cooled to room temperature. To the reaction solution, a sodium acetate aqueous solution cooled in an ice bath and toluene were added. The mixture was stirred. Thereafter, an organic layer was separated from the mixture and washed with water. Thereafter, the organic layer was concentrated to obtain a crude product. The crude product was purified by a silica gel column (eluent: toluene) and further washed with acetone to obtain compound (1-3812) (5.0 g).

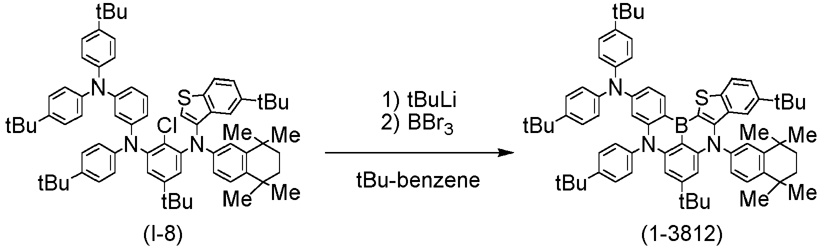

The structure of the obtained compound was confirmed by NMR measurement.
¹H-NMR (CDCl₃): δ = 0.97 (s, 9H), 1.10 (s, 9H), 1.27-1.34 (m, 33H), 1.40-1.47 (m, 6H), 1.75-1.90 (m, 4H), 6.00-6.28 (m, 2H), 6.36 (s, 1H), 6.65-6.80 (m, 1H), 6.80-6.96 (m, 1H), 7.00 (d, 4H), 7.14 (d, 2H), 7.18-7.26 (m, 5H), 7.38 (d, 1H), 7.43-7.58 (m, 3H), 7.65 (d, 1H), 7.85 (d, 1H), 8.53 (s, 1H).

### Synthesis Example (21): Synthesis of compound (1-3847)

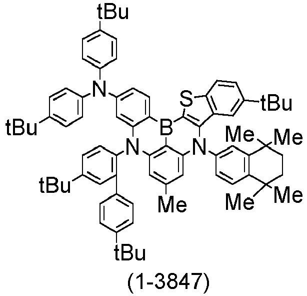

Into a flask containing compound (I-9) (23.0 g) and t-butylbenzene (140 ml), in a nitrogen atmosphere, a 1.61 M t-butyllithium pentane solution (25.0 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (10.0 g) was added thereto. The mixture was heated to room temperature and stirred for 0.5 hours. Thereafter, the mixture was heated to 80°C and heated and stirred for one hour. The reaction solution was cooled to room temperature. To the reaction solution, a sodium acetate aqueous solution cooled in an ice bath and toluene were added. The mixture was stirred. Thereafter, an organic layer was separated from the mixture and washed with water. Thereafter, the organic layer was concentrated to obtain a crude product. The crude product was purified by a silica gel column (eluent: toluene/heptane = 1/3 (volume ratio)) and further washed with acetone to obtain compound (1-3847) (2.5 g).

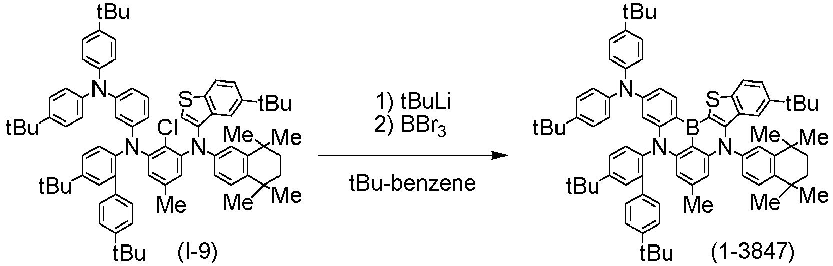

The structure of the obtained compound was confirmed by NMR measurement.
¹H-NMR (CDCl₃): δ = 1.10 (s, 9H), 1.17-1.20 (m, 9H), 1.25-1.38 (m, 33H), 1.42-1.48 (m, 6H), 1.76-1.88 (m, 4H), 2.19 (s, 3H), 6.02-6.16 (m, 3H), 6.57-6.62 (m, 2H), 6.86-6.96 (m, 7H), 7.01-7.14 (m, 3H), 7.20 (d, 4H), 7.36 (dd, 1H), 7.39-7.50 (m, 3H), 7.60-7.67 (m, 1H), 7.83 (d, 1H), 8.47 (d, 1H).

### Synthesis Example (22): Synthesis of compound (1-3842)

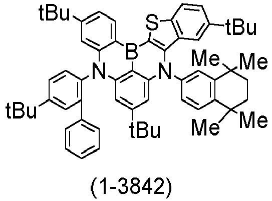

Into a flask containing compound (I-10) (36.6 g) and t-butylbenzene (290 ml), in a nitrogen atmosphere, a 1.61 M t-butyllithium pentane solution (50.0 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (20.0 g) was added thereto. The mixture was heated to room temperature and stirred for 0.5 hours. Thereafter, the resulting solution was cooled to 0°C again, N,N-diisopropylethylamine (30.6 g) was added thereto, and the mixture was stirred at room temperature until heat generation was stopped. Thereafter, the resulting solution was heated to 60°C and heated and stirred for one hour. The reaction solution was cooled to room temperature. To the reaction solution, a sodium acetate aqueous solution cooled in an ice bath and toluene were added. The mixture was stirred. Thereafter, an organic layer was separated from the mixture and washed with water. Thereafter, the organic layer was concentrated, and the residue was washed with heptane. The obtained crude product was purified by a silica gel column (eluent: toluene) and further washed with heptane to obtain compound (1-3842) (9.80 g).

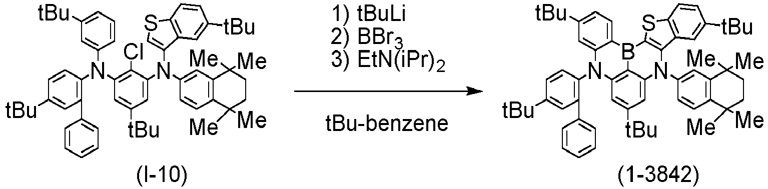

The structure of the obtained compound was confirmed by NMR measurement.
¹H-NMR (CDCl₃): δ = 0.99-1.02 (m, 9H), 1.10 (s, 9H), 1.21 (s, 9H), 1.25-1.34 (m, 6H), 1.38-1.47 (m, 6H), 1.49 (s, 9H), 1.75-1.88 (m, 4H), 6.02-6.45 (m, 2H), 6.62-6.78 (m, 2H), 6.96-7.50 (m, 3H), 7.14-7.29 (m, 5H), 7.38 (dd, 1H), 7.47-7.73 (m, 4H), 7.85 (d, 1H), 8.58 (d, 1H) .

### Synthesis Example (23): Synthesis of compound (1-5179)

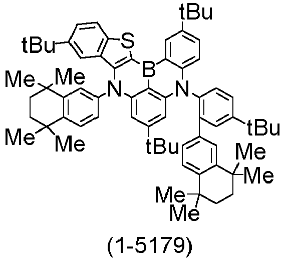

Into a flask containing compound (I-11) (63.0 g) and t-butylbenzene (440 ml), in a nitrogen atmosphere, a 1.61 M t-butyllithium pentane solution (75.0 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (30.0 g) was added thereto. The mixture was heated to room temperature and stirred for 1.5 hours. To the reaction solution, a sodium acetate aqueous solution cooled in an ice bath and toluene were added. The mixture was stirred. Thereafter, an organic layer was separated from the mixture and washed with water. Thereafter, the organic layer was concentrated to obtain a crude product. The crude product was purified by a silica gel column (eluent: toluene/heptane = 1/2 (volume ratio)) and further purified by recrystallization from heptane to obtain compound (1-5179) (16.0 g).

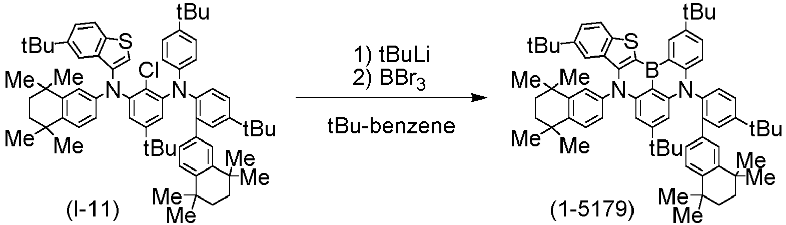

The structure of the obtained compound was confirmed by NMR measurement.
¹H-NMR (CDCl₃): δ = 0.53-0.60 (m, 6H), 0.99 (s, 9H), 1.04-1.09 (m, 6H), 1.10 (s, 9H), 1.29 (s, 6H), 1.40-1.58 (m, 28H), 1.86-1.76 (m, 4H), 6.05-6.38 (m, 2H), 6.72 (d, 2H), 6.96-7.04 (m, 2H), 7.06 (d, 1H), 7.14-7.31 (m, 2H), 7.36-7.55 (m, 3H), 7.56-7.74 (m, 3H), 7.88 (d, 1H), 8.66 (s, 1H).

### Synthesis Example (24): Synthesis of compound (1-5185)

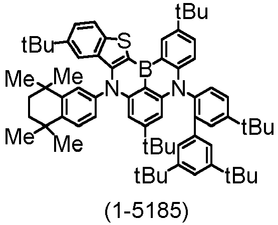

Into a flask containing compound (I-12) (78.0 g) and t-butylbenzene (780 ml), in a nitrogen atmosphere, a 1.61 M t-butyllithium pentane solution (100 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (40.0 g) was added thereto. The mixture was heated to room temperature and stirred for 0.5 hours. Thereafter, the resulting solution was cooled to 0°C again, N,N-diisopropylethylamine (19.7 g) was added thereto, and the mixture was stirred at room temperature until heat generation was stopped. Thereafter, the resulting solution was heated to 80°C and heated and stirred for one hour. The reaction solution was cooled to room temperature. To the reaction solution, a sodium acetate aqueous solution cooled in an ice bath and toluene were added. The mixture was stirred. Thereafter, an organic layer was separated from the mixture and washed with water. Thereafter, the organic layer was concentrated, and the residue was sequentially washed with ethyl acetate and heptane. The obtained crude product was purified by a silica gel column (eluent: toluene) and further purified by recrystallization from toluene to obtain compound (1-5185) (13.8 g).

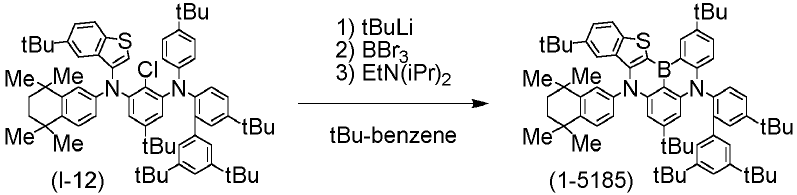

The structure of the obtained compound was confirmed by NMR measurement.
¹H-NMR (CDCl₃): δ = 0.86-0.90 (m, 18H), 1.02 (s, 9H), 1.10 (s, 9H), 1.27-1.30 (m, 6H), 1.40-1.50 (m, 24H), 1.76-1.86 (m, 4H), 6.02-6.38 (m, 2H), 6.55-6.80 (m, 2H), 6.84-90 (m, 2H), 6.93-6.96 (m, 2H), 7.08-7.24 (m, 1H), 7.30-7.54 (m, 4H), 7.58-7.73 (m, 2H), 7.86 (d, 1H), 8.61 (s, 1H).

### Synthesis Example (25): Synthesis of compound (1-1853)

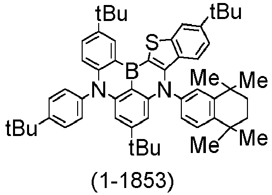

In a nitrogen atmosphere, compound (I-13) (20.0 g), compound (I-V) (12.0 g), Pd(dba)₂ (0.53 g), NaOtBu (4.4 g), [(tBu)₃PH]BF₄ (0.54 g), and xylene (60 ml) were put into a flask and heated and refluxed at 120°C for 0.5 hours. The reaction solution was cooled to room temperature, and then water and ethyl acetate were added thereto. An organic layer was separated from the mixture and washed with water. This solution was concentrated under reduced pressure. The residue was purified by a silica gel column (eluent: toluene/heptane = 1/1 (volume ratio)) and concentrated. Thereafter, heptane was added thereto to obtain a crystal. The crystal was filtered and then washed with heptane to obtain compound (I-14) (30.0 g).

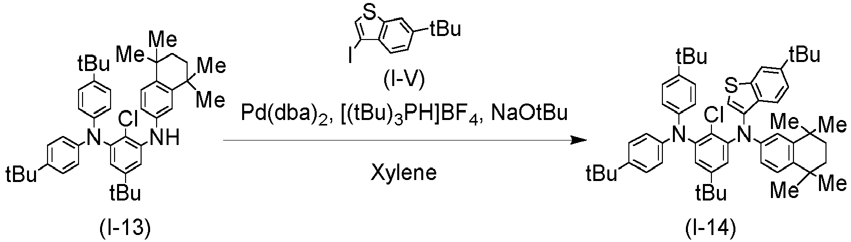

Into a flask containing compound (I-14) (30.0 g) and t-butylbenzene (180 ml), in a nitrogen atmosphere, a 1.60 M t-butyllithium pentane solution (44.8 ml) was added at 0°C. After dropping, the mixture was heated to 60°C and stirred for 0.5 hours. Thereafter, a component having a lower boiling point than t-butylbenzene was distilled off under reduced pressure. The resulting solution was cooled to -50°C, and boron tribromide (17.9 g) was added thereto. The mixture was heated to room temperature and stirred for 0.5 hours. Thereafter, the resulting solution was cooled to 0°C again, N,N-diisopropylethylamine (9.3 g) was added thereto, and the mixture was stirred at room temperature until heat generation was stopped. Thereafter, the resulting solution was heated to 80°C and heated and stirred for one hour. The reaction solution was cooled to room temperature. To the reaction solution, a sodium acetate aqueous solution cooled in an ice bath was added, and subsequently ethyl acetate was added. The mixture was stirred for one hour. An organic layer was separated from the mixture and washed with water. This solution was concentrated under reduced pressure. The residue was washed with heptane, and then purified by a silica gel column (eluent: toluene/heptane = 1/1 (volume ratio)). The obtained crude product was washed with heptane to obtain compound (1-1853) (4.7 g).

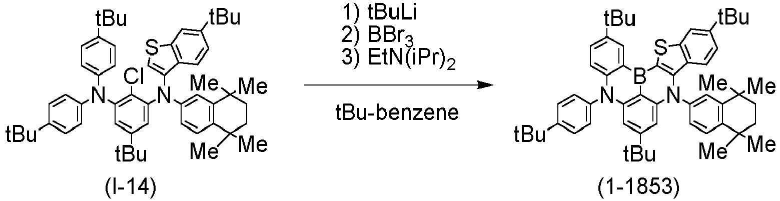

The structure of the obtained compound was confirmed by NMR measurement.
¹H-NMR (CDCl₃): δ = 1.01 (s, 9H), 1.27 (s, 3H), 1.29 (s, 3H), 1.36 (s, 9H), 1.45 (s, 3H), 1.46 (s, 9H), 1.49 (s, 3H), 1.51 (s, 9H), 1.81-1.89 (m, 4H), 6.05 (d, 1H), 6.14 (br, 1H), 6.36 (br, 1H), 6.64 (br, 1H), 6.96 (dd, 1H), 7.19 (dd, 1H), 7.28 (d, 2H), 7.46 (dd, 1H), 7.49 (d, 1H), 7.63 (d, 1H), 7.68 (d, 2H), 7.97 (d, 1H), 8.76 (s, 1H).

By appropriately changing compounds as a raw material, another polycyclic aromatic compound of the present invention can be synthesized by a method in accordance with the above Synthesis Examples.

Compound (BH-2) was synthesized in accordance with the method disclosed in Synthesis Example 1 of JP 2016-88927 A. In addition, compounds represented by the following formulas (BH-3) to (BH-7) were synthesized by a similar method.

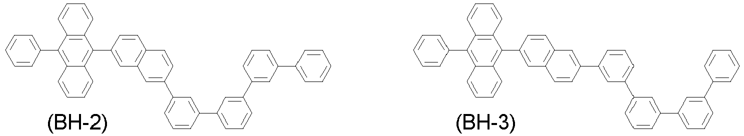

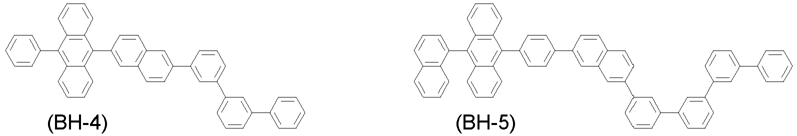

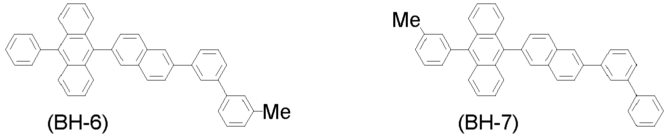

Next, in order to describe the present invention in more detail, Examples of an organic EL element using the compound of the present invention will be described, but the present invention is not limited thereto.

### <Evaluation of vapor deposition type organic EL element>

Organic EL elements in Examples 1-1 to 1-13 were manufactured. Voltage (V), emission wavelength (nm), and external quantum efficiency (%), which are characteristics at the time of emission at 1000 cd/m², were measured. Next, time required for holding 90% or more of initial brightness was measured when constant current drive was performed at a current density of 10 mA/cm².

Quantum efficiency of a light emitting element includes internal quantum efficiency and external quantum efficiency. The internal quantum efficiency indicates a ratio at which external energy injected into a light emitting layer of the light emitting element as electrons (or holes) is purely converted into photons. Meanwhile, the external quantum efficiency is calculated based on the amount of the photons emitted to the outside of the light emitting element. Some of the photons generated in the light emitting layer are absorbed or continuously reflected inside the light emitting element and are not emitted to the outside of the light emitting element. Therefore, the external quantum efficiency is lower than the internal quantum efficiency.

A method for measuring the external quantum efficiency is as follows. Using a voltage/current generator R6144 manufactured by Advantest Corporation, a voltage at which the brightness of an element was 1000 cd/m² was applied to cause the element to emit light. Using a spectral radiance meter SR-3AR manufactured by TOPCON Corporation, spectral radiance in a visible light region was measured from a direction perpendicular to a light emitting surface. The number of photons at each wavelength is a numerical value obtained by dividing a measured value of spectral radiance of each wavelength component by wavelength energy and multiplying the obtained value by n assuming that the light emitting surface is a complete diffusing surface. Subsequently, the number of photons was integrated in all the observed wavelength regions, and the obtained value was defined as the total number of photons emitted from the element. A numerical value obtained by dividing an applied current value by an elementary charge is defined as the number of carriers injected into the element. A numerical value obtained by dividing the total number of photons emitted from the element by the number of carriers injected into the element is the external quantum efficiency.

### <Examples 1-1 to 1-13>

The following Table 1A indicates material composition of each layer in an organic EL element in each of Examples 1-1 to 1-13.
HIL: Hole injection layer
HTL: Hole transport layer
LEL: Light emitting layer
ETL: Electron transport layer
NE: Negative electrode

**[Table 1A]**

| | HIL 1 40nm | HIL 2 5nm | HTL 1 45nm | HTL 2 10nm | LEL 25nm | | ETL 1 5nm | ETL 2 25nm | NE 1 nm/ 100nm |
|---|---|---|---|---|---|---|---|---|---|
| | | | | | Host | Dopant | | | |
| Ex. 1-1 | HI | HAT-CN | HT-1 | HT-2 | BH-1 | Compound (1-411) | ET-1 | ET-2 +Liq | LiF/Al |
| Ex. 1-2 | HI | HAT-CN | HT-1 | HT-2 | BH-1 | Compound (1-590) | ET-1 | ET-2 +Liq | LiF/Al |
| Ex. 1-3 | HI | HAT-CN | HT-1 | HT-2 | BH-1 | Compound (1-1301) | ET-1 | ET-2 +Liq | LiF/Al |
| Ex. 1-4 | HI | HAT-CN | HT-1 | HT-2 | BH-1 | Compound (1-1823) | ET-1 | ET-2 +Liq | LiF/Al |
| Ex. 1-5 | HI | HAT-CN | HT-1 | HT-2 | Compound (3-44) | Compound (1-1821) | ET-1 | ET-2 +Liq | LiF/Al |
| Ex. 1-6 | HI | HAT-CN | HT-1 | HT-2 | Compound (3-44) | Compound (1-1826) | ET-1 | ET-2 +Liq | LiF/Al |
| Ex. 1-7 | HI | HAT-CN | HT-1 | HT-2 | Compound (3-44) | Compound (1-3645) | ET-1 | ET-2 +Liq | LiF/Al |
| Ex. 1-8 | HI | HAT-CN | HT-1 | HT-2 | Compound (3-44) | Compound (1-1851) | ET-1 | ET-2 +Liq | LiF/Al |
| Ex. 1-9 | HI | HAT-CN | HT-1 | HT-2 | BH-1 | Compound (1-1853) | ET-1 | ET-2 +Liq | LiF/Al |
| Ex. 1-10 | HI | HAT-CN | HT-1 | HT-2 | BH-1 | Compound (1-3644) | ET-1 | ET-2 +Liq | LiF/Al |
| Ex. 1-11 | HI | HAT-CN | HT-1 | HT-2 | BH-1 | Compound (1-3812) | ET-1 | ET-2 +Liq | LiF/Al |
| Ex. 1-12 | HI | HAT-CN | HT-1 | HT-2 | Compound (3-44) | Compound (1-3604) | ET-1 | ET-2 +Liq | LiF/Al |
| Ex. 1-13 | HI | HAT-CN | HT-1 | HT-2 | BH-1 | Compound (1-3842) | ET-1 | ET-2 +Liq | LiF/Al |

In Table 1A, "HI" represents N⁴,N⁴'-diphenyl-N⁴,N⁴'-bis (9-phenyl-9H-carbazol-3-yl) - [1,1'-biphenyl]-4,4'-diamine, "HAT-CN" represents 1,4,5,8,9,12-hexaazatriphenylenehexacarbonitrile, "HT-1" represents N-([1,1'-biphenyl]-4-yl-9,9-dimethyl-N-[4-(9-phenyl-9H-carbazol-3-yl)phenyl]-9H-fluoren-2-amine[1,1'-biphenyl]-4-amine, "HT-2" represents N,N-bis(4-(dibenzo[b,d]furan-4-yl)phenyl)-[1,1':4',1"-terphenyl]-4-amine, "BH-1" represents 2-(10-phenylanthracen-9-yl)naphtho[2,3-b]benzofuran, "ET-1" represents 4,6,8,10-tetraphenyl[1,4]benzoxazaborinino[2,3,4-kl]phenoxaborinine, and "ET-2" represents 3,3'-((2-phenylanthracene-9,10-diyl)bis(4,1-phnylene))bis(4-methylpyridine). Chemical structures thereof are indicated below together with that of "Liq".

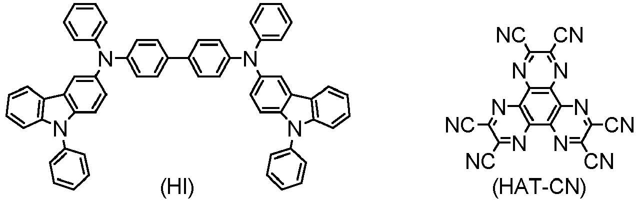

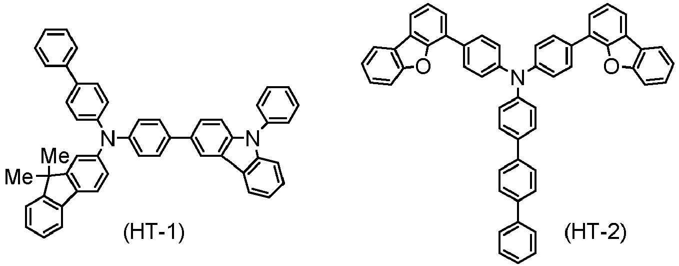

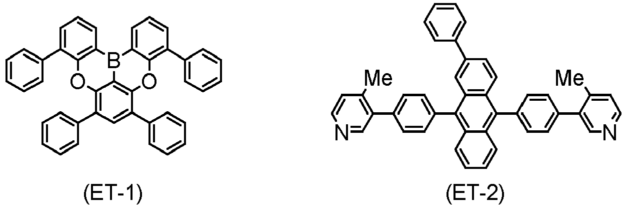

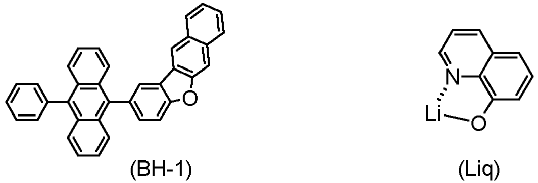

### <Example 1-1>

A glass substrate having a size of 26 mm × 28 mm × 0.7mm (manufactured by OptScienece Co., Ltd.) in which ITO that had been formed into a film having a thickness of 180 nm by sputtering was polished so as to have a thickness of 150 nm was used as a transparent support substrate. This transparent support substrate was fixed to a substrate holder of a commercially available vapor deposition apparatus (manufactured by Showa Shinku Co., Ltd.). A molybdenum vapor deposition boat containing each of HI, HAT-CN, HT-1, HT-2, BH-1, compound (1-411), ET-1, and ET-2, and an aluminum nitride vapor deposition boat containing each of Liq, LiF, and aluminum were mounted thereon.

On the ITO film of the transparent support substrate, the following layers were sequentially formed. The pressure of a vacuum chamber was reduced to 5 × 10⁻⁴ Pa. First, HI was heated and vapor-deposited so as to have a film thickness of 40 nm, next, HAT-CN was heated and vapor-deposited so as to have a film thickness of 5 nm, next, HT-1 was heated and vapor-deposited so as to have a film thickness of 45 nm, and next, HT-2 was heated and vapor-deposited so as to have a film thickness of 10 nm to form a hole layer including four layers. Next, BH-1 and compound (1-411) were heated simultaneously and vapor-deposited so as to have a film thickness of 25 nm to form a light emitting layer. A vapor deposition rate was adjusted such that a weight ratio between BH-1 and compound (1-411) was approximately 98 : 2. Furthermore, ET-1 was heated and vapor-deposited so as to have a film thickness of 5 nm, and next, ET-2 and Liq were heated simultaneously and vapor-deposited so as to have a film thickness of 25 nm to form an electron layer including two layers. A vapor deposition rate was adjusted such that a weight ratio between ET-2 and Liq was approximately 50 : 50. A vapor deposition rate of each layer was 0.01 to 1 nm/sec. Thereafter, LiF was heated and vapor-deposited so as to have a film thickness of 1 nm at a vapor deposition rate of 0.01 to 0.1 nm/sec, and next, aluminum was heated and vapor-deposited so as to have a film thickness of 100 nm to form a negative electrode, thus obtaining an organic EL element.

By using the ITO electrode as a positive electrode, and using the LiF/ aluminum electrode as a negative electrode, a direct current voltage was applied. Characteristics (emission wavelength, drive voltage, and external quantum efficiency) at the time of emission at 1000 cd/m² were measured. In addition, time required for holding 90% or more of initial brightness was measured.

### <Examples 1-2 to 1-4>

An organic EL element was manufactured in a similar manner to Example 1-1, and EL characteristics were evaluated.

### <Examples 1-5 to 1-13>

An organic EL element was manufactured in a similar manner to Example 1-1, and EL characteristics were evaluated.

The following Table 1B indicates EL characteristics confirmed in Examples 1-1 to 1-13.

**[Table 1B]**

| | Wavelength (nm) | Drive Voltage (V) | External Quantum Efficiency (%) |
|---|---|---|---|
| Example 1-1 | 466 (blue emission) | 3.77 | 8.57 |
| Example 1-2 | 463 (blue emission) | 3.72 | 7.93 |
| Example 1-3 | 465 (blue emission) | 3.71 | 8.09 |
| Example 1-4 | 463 (blue emission) | 3.57 | 8.05 |
| Example 1-5 | 459 (blue emission) | 3.93 | 7/33 |
| Example 1-6 | 459 (blue emission) | 3.94 | 7.61 |
| Example 1-7 | 457 (blue emission) | 3.88 | 7/36 |
| Example 1-8 | 456 (blue emission) | 3.99 | 7.44 |
| Example 1-9 | 460 (blue emission) | 3.89 | 7.23 |
| Example 1-10 | 456 (blue emission) | 3.71 | 8.07 |
| Example 1-11 | 458 (blue emission) | 3.60 | 8.59 |
| Example 1-12 | 464 (blue emission) | 3.55 | 8.26 |
| Example 1-13 | 456 (blue emission) | 3.92 | 7.60 |

### <Evaluation of application type organic EL element>

Next, an organic EL element obtained by applying and forming an organic layer will be described.

### <Synthesis of polymer host compound: SPH-101>

SPH-101 was synthesized in accordance with the method disclosed in WO 2015/008851 A. A copolymer in which M2 or M3 was bonded adjacent to M1 was obtained. It is estimated that a ratio among the units is 50 : 26 : 24 (molar ratio) from a charge ratio.

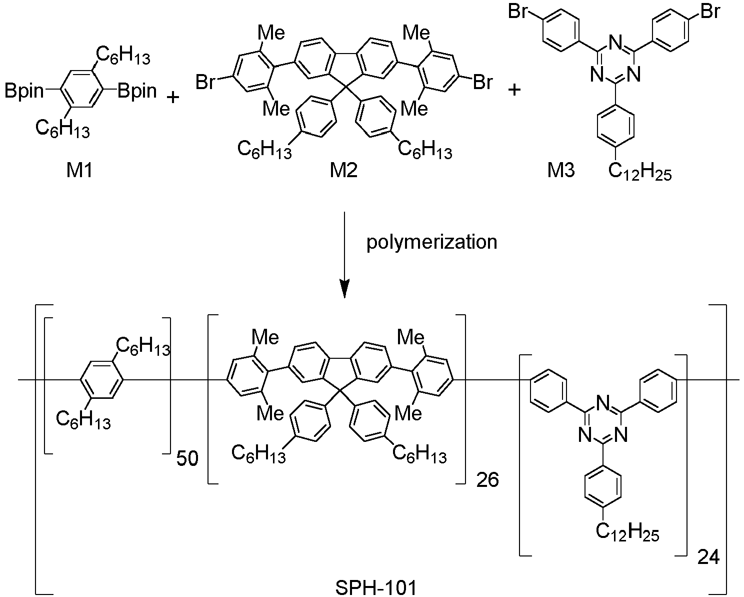

### <Synthesis of polymer hole transport compound: XLP-101>

XLP-101 was synthesized in accordance with the method disclosed in WO 2018/61028 A. A copolymer in which M5 or M6 was bonded adjacent to M4 was obtained. It is estimated that a ratio among the units is 40 : 10 : 50 (molar ratio) from a charge ratio.

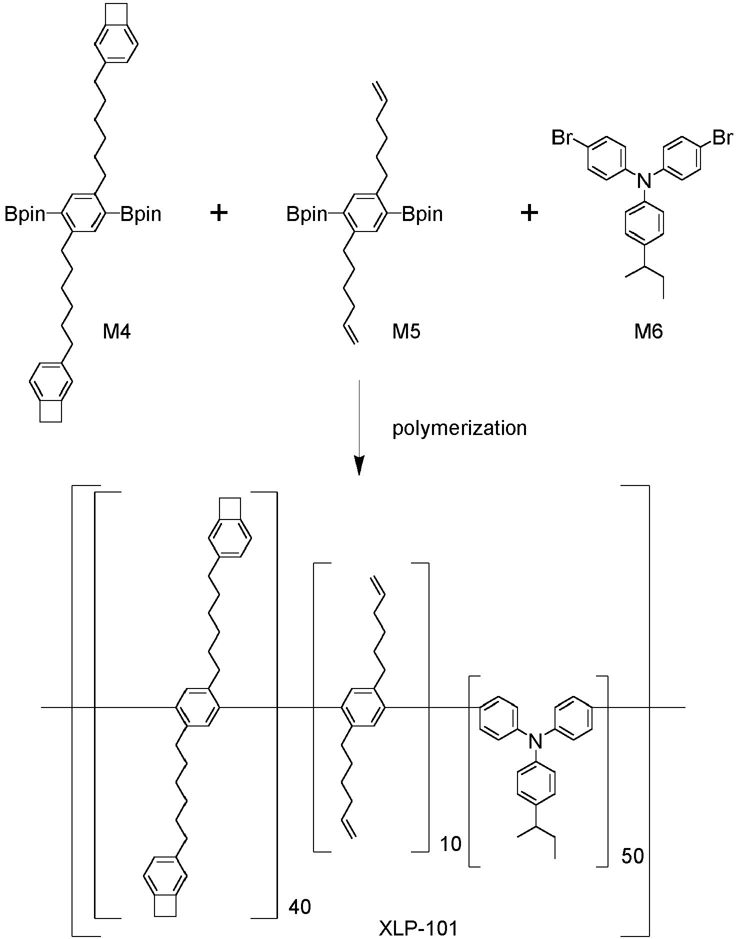

### <Examples 2 to 10>

An application solution of a material forming each layer is prepared, and an application type organic EL element is manufactured.

### <Manufacture of organic EL elements in Examples 2 to 4>

Table 2 indicates material composition of each layer in an organic EL element. [Table 2]

| | HIL 40nm | HTL 30nm | LEL 20nm | | ETL 30nm | NE 1nm /100nm |
|---|---|---|---|---|---|---|
| | | | Host | Dopant | | |
| Ex. 2 | PEDOT : PSS | OTPD | SPH -101 | (A) | ET1 | LiF /Al |
| Ex. 3 | PEDOT : PSS | XLP -101 | SPH -101 | (A) | ET1 | LiF /Al |
| Ex. 4 | PEDOT : PSS | PCz | SPH -101 | (A) | ET1 | LiF /Al |

The structure of "ET1" in Table 2 is indicated below.

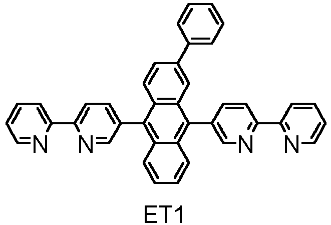

### <Preparation of light emitting layer-forming composition (1)>

By stirring the following components until a uniform solution was formed, light emitting layer-forming composition (1) is prepared. A glass substrate is spin-coated with the prepared light emitting layer-forming composition. The light emitting layer-forming composition is heated and dried under reduced pressure to obtain a coating film having no film defect and excellent smoothness.

| | |
|---|---|
| Compound (A) | 0.04% by weight |
| SPH-101 | 1.96% by weight |
| Xylene | 69.00% by weight |
| Decalin | 29.00% by weight |

Note that compound (A) is a polycyclic aromatic compound represented by general formula (1), a multimer thereof, a polymer compound obtained by polymerizing, as a monomer, the polycyclic aromatic compound or a multimer thereof (that is, the monomer has a reactive substituent), or a crosslinked polymer obtained by further crosslinking the polymer compound. The polymer compound for obtaining the crosslinked polymer has a reactive substituent.

### <PEDOT: PSS solution>

A commercially available PEDOT: PSS solution (Clevios (TM) P VP AI4083, PEDOT: PSS aqueous dispersion, manufactured by Heraeus Holdings) is used.

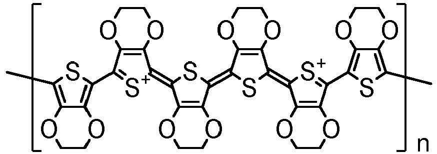

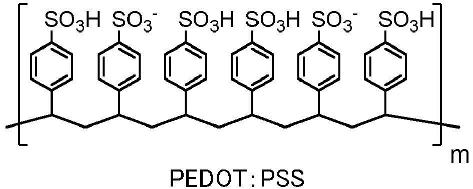

### <Preparation of OTPD solution>

OTPD (LT-N159, manufactured by Luminescence Technology Corp) and IK-2 (photocationic polymerization initiator, manufactured by San-Apro Ltd.) are dissolved in toluene to prepare an OTPD solution having an OTPD concentration of 0.7% by weight and an IK-2 concentration of 0.007% by weight.

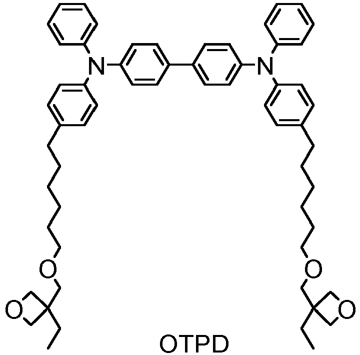

### <Preparation of XLP-101 solution>

XLP-101 is dissolved in xylene at a concentration of 0.6% by weight to prepare a 0.7% by weight XLP-101 solution.

### <Preparation of PCz solution>

PCz (polyvinylcarbazole) is dissolved in dichlorobenzene to prepare a 0.7% by weight PCz solution.

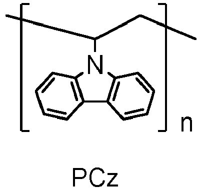

### <Example 2>

A glass substrate on which ITO has been vapor-deposited at a thickness of 150 nm is spin-coated with a PEDOT: PSS solution, and fired on a hot plate at 200°C for one hour to form a PEDOT: PSS film having a film thickness of 40 nm (hole injection layer). Subsequently, the PEDOT: PSS film is spin-coated with an OTPD solution, dried on a hot plate at 80°C for 10 minutes, then exposed to light at an exposure intensity of 100 mJ/cm² using an exposure machine, and fired on a hot plate at 100°C for one hour to form an OTPD film that is insoluble in a solution and has a film thickness of 30 nm (hole transport layer). Subsequently, the OTPD film is spin-coated with light emitting layer-forming composition (1) and fired on a hot plate at 120°C for one hour to form a light emitting layer having a film thickness of 20 nm.

The manufactured multilayer film is fixed to a substrate holder of a commercially available vapor deposition apparatus (manufactured by Showa Shinku Co., Ltd.). A molybdenum vapor deposition boat containing ET1, a molybdenum vapor deposition boat containing LiF, and a tungsten vapor deposition boat containing aluminum are mounted thereon. The pressure of a vacuum chamber is reduced to 5 × 10⁻⁴ Pa. Thereafter, ET1 is heated and vapor-deposited so as to have a film thickness of 30 nm, thus forming an electron transport layer. A vapor deposition rate at the time of forming the electron transport layer is 1 nm/sec. Thereafter, LiF is heated and vapor-deposited at a vapor deposition rate of 0.01 to 0.1 nm so as to have a film thickness of 1 nm. Subsequently, aluminum is heated and vapor-deposited so as to have a film thickness of 100 nm to form a negative electrode, thus obtaining an organic EL element.

### <Example 3>

An organic EL element is obtained in a similar manner to Example 2. Note that for the hole transport layer, spin coating is performed with an XLP-101 solution, and firing is performed on a hot plate at 200°C for one hour to form a film having a film thickness of 30 nm.

### <Example 4>

An organic EL element is obtained in a similar manner to Example 2. Note that for the hole transport layer, spin coating is performed with an PCz solution, and firing is performed on a hot plate at 120°C for one hour to form a film having a film thickness of 30 nm.

### <Manufacture of organic EL elements in Examples 5 to 7>

Table 3 indicates material composition of each layer in an organic EL element.

**[Table 3]**

| | HIL 50nm | HTL 20 nm | LEL 20nm | | ETL 30nm | NE 1nm /100nm |
|---|---|---|---|---|---|---|
| | | | Host | Dopant | | |
| Ex. 5 | ND -3202 | XLP -101 | mCBP | (A) | TSPO1 | LiF /Al |
| Ex. 6 | ND -3202 | XLP -101 | SPH -101 | (A) | TSPO1 | LiF /Al |
| Ex. 7 | ND -3202 | XLP -101 | DOBNA | (A) | TSPO1 | LiF /Al |

### <Preparation of light emitting layer-forming compositions (2) to (4)>

The following components are stirred until a uniform solution is formed, and a light emitting layer-forming composition (2) is thereby prepared.

| | |
|---|---|
| Compound (A) | 0.02% by weight |
| mCBP | 1.98% by weight |
| Toluene | 98.00% by weight |

The following components are stirred until a uniform solution is formed, and a light emitting layer-forming composition (3) is thereby prepared.

| | |
|---|---|
| Compound (A) | 0.02% by weight |
| SPH-101 | 1.98% by weight |
| Xylene | 98.00% by weight |

The following components are stirred until a uniform solution is formed, and a light emitting layer-forming composition (4) is thereby prepared.

| | |
|---|---|
| Compound (A) | 0.02% by weight |
| DOBNA | 1.98% by weight |
| Toluene | 98.00% by weight |

In Table 3, "mCBP" represents 3,3'-bis(N-carbazolyl)-1,1'-biphenyl, "DOBNA" represents 3,11-di-o-tolyl-5,9-dioxa-13b-boranaphtho[3,2,1-de]anthracene, and "TSPO1" represents diphenyl[4-(triphenylsilyl)phenyl]phosphine oxide. Chemical structures thereof are indicated below.

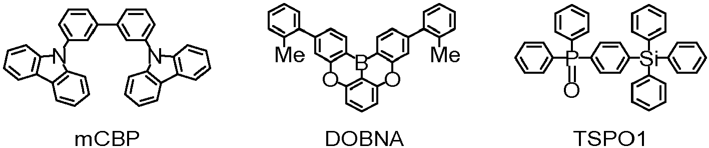

### <Example 5>

A glass substrate on which ITO has been formed at a thickness of 45 nm is spin-coated with an ND-3202 (manufactured by Nissan Chemical Corporation) solution, then heated at 50°C for three minutes in an air atmosphere, and further heated at 230°C for 15 minutes to form an ND-3202 film having a film thickness of 50 nm (hole injection layer).
Subsequently, the ND-3202 film is spin-coated with an XLP-101 solution and heated on a hot plate at 200°C for 30 minutes in a nitrogen gas atmosphere to form an XLP-101 film having a film thickness of 20 nm (hole transport layer). Subsequently, the XLP-101 film is spin-coated with light emitting layer-forming composition (2) and heated at 130°C for 10 minutes in a nitrogen gas atmosphere to form a light emitting layer of 20 nm.

The manufactured multilayer film is fixed to a substrate holder of a commercially available vapor deposition apparatus (manufactured by Showa Shinku Co., Ltd.). A molybdenum vapor deposition boat containing TSPO1, a molybdenum vapor deposition boat containing LiF, and a tungsten vapor deposition boat containing aluminum are mounted thereon. The pressure of a vacuum chamber is reduced to 5 × 10⁻⁴ Pa. Thereafter, TSPO1 is heated and vapor-deposited so as to have a film thickness of 30 nm, thus forming an electron transport layer. A vapor deposition rate at the time of forming the electron transport layer is 1 nm/sec. Thereafter, LiF is heated and vapor-deposited at a vapor deposition rate of 0.01 to 0.1 nm so as to have a film thickness of 1 nm. Subsequently, aluminum is heated and vapor-deposited so as to have a film thickness of 100 nm to form a negative electrode, thus obtaining an organic EL element.

### <Examples 6 and 7>

An organic EL element is obtained in a similar manner to Example 5 using light emitting layer-forming composition (3) or (4).

### <Manufacture of organic EL elements in Examples 8 to 10>

Table 4 indicates material composition of each layer in an organic EL element.

**[Table 4]**

| | HIL 50 nm | HTL 20 nm | LEL 20 nm | | | ETL 30nm | NE 1 nm/ 100 nm |
|---|---|---|---|---|---|---|---|
| | | | Host | Assisting Dopant | Dopant | | |
| Ex. 8 | ND -3202 | XLP -101 | mCBP | 2PXZ -TAZ | (A) | TSPO1 | LiF/ Al |
| Ex. 9 | ND -3202 | XLP -101 | SPH -101 | 2PXZ -TAZ | (A) | TSPO1 | LiF/ Al |
| Ex. 10 | ND -3202 | XLP -101 | DOBNA | 2PXZ -TAZ | (A) | TSPO1 | LiF/ Al |

### <Preparation of light emitting layer-forming compositions (5) to (7)>

The following components are stirred until a uniform solution is formed, and a light emitting layer-forming composition (5) is thereby prepared.

| | |
|---|---|
| Compound (A) | 0.02% by weight |
| 2PXZ-TAZ | 0.18% by weight |
| mCBP | 1.80% by weight |
| Toluene | 98.00% by weight |

The following components are stirred until a uniform solution is formed, and a light emitting layer-forming composition (6) is thereby prepared.

| | |
|---|---|
| Compound (A) | 0.02% by weight |
| 2PXZ-TAZ | 0.18% by weight |
| SPH-101 | 1.80% by weight |
| Xylene | 98.00% by weight |

The following components are stirred until a uniform solution is formed, and a light emitting layer-forming composition (7) is thereby prepared.

| | |
|---|---|
| Compound (A) | 0.02% by weight |
| 2PXZ-TAZ | 0.18% by weight |
| DOBNA | 1.80% by weight |
| Toluene | 98.00% by weight |

In Table 4, "2PXZ-TAZ" represents 10,10'-((4-phenyl-4H-1,2,4-triazole-3,5-diyl)bis(4,1-phenyl))bis(10H-phenoxazine). Chemical structures thereof are indicated below.

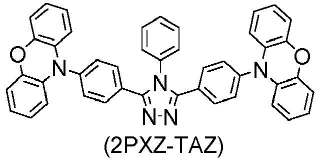

### <Example 8>

A glass substrate on which ITO has been formed at a thickness of 45 nm is spin-coated with an ND-3202 (manufactured by Nissan Chemical Corporation) solution, then heated at 50°C for three minutes in an air atmosphere, and further heated at 230°C for 15 minutes to form an ND-3202 film having a film thickness of 50 nm (hole injection layer). Subsequently, the ND-3202 film is spin-coated with an XLP-101 solution and heated on a hot plate at 200°C for 30 minutes in a nitrogen gas atmosphere to form an XLP-101 film having a film thickness of 20 nm (hole transport layer). Subsequently, the XLP-101 film is spin-coated with light emitting layer-forming composition (5) and heated at 130°C for 10 minutes in a nitrogen gas atmosphere to form a light emitting layer of 20 nm.

The manufactured multilayer film is fixed to a substrate holder of a commercially available vapor deposition apparatus (manufactured by Showa Shinku Co., Ltd.). A molybdenum vapor deposition boat containing TSPO1, a molybdenum vapor deposition boat containing LiF, and a tungsten vapor deposition boat containing aluminum are mounted thereon. The pressure of a vacuum chamber is reduced to 5 × 10⁻⁴ Pa. Thereafter, TSPO1 is heated and vapor-deposited so as to have a film thickness of 30 nm, thus forming an electron transport layer. A vapor deposition rate at the time of forming the electron transport layer is 1 nm/sec. Thereafter, LiF is heated and vapor-deposited at a vapor deposition rate of 0.01 to 0.1 nm so as to have a film thickness of 1 nm. Subsequently, aluminum is heated and vapor-deposited so as to have a film thickness of 100 nm to form a negative electrode, thus obtaining an organic EL element.

### <Examples 9 and 10>

An organic EL element is obtained in a similar manner to Example 8 using light emitting layer-forming composition (6) or (7).

### <Examples 11 to 29>

### <Preparation of light emitting layer-forming composition>

Light emitting layer-forming compositions in Examples 11 to 29 were prepared. Each of the light emitting layer-forming compositions was prepared by mixing and stirring compounds listed in Tables 5A and 5B.
1st: First component
2nd: Second component
3rd: Third component

**[Table 5A]**

| | Concentration | | | | 1st | 2nd | 3rd |
|---|---|---|---|---|---|---|---|
| | Solid | 1st | 2nd | 3rd | | | |
| Ex. 11 | 1 | 0.01 | 0.99 | 99. 0 | Com. 1-411 | Com. 3-44 | cyclohexylbenzene /3-phennoxytoluene 7/3 |
| Ex. 12 | 1 | 0.01 | 0.99 | 99.0 | Com. 1-590 | Com. 3-44 | cyclohexylbenzene /3-phennoxytoluene 7/3 |
| Ex. 13 | 2 | 0.02 | 1.98 | 98.0 | Com. 1-590 | Com. 3-44 | cyclohexylbenzene /3-phennoxytoluene 7/3 |
| Ex. 14 | 1 | 0.01 | 0.99 | 99.0 | Com. 1-590 | Com. 3-44 | toluene /decahydronaphthalene 9/1 |
| Ex. 15 | 1 | 0.01 | 0.99 | 99.0 | Com. 1-590 | Com. 3-44 | 3-phennoxytoluene /cyclohexylbenzene 9/1 |
| Ex. 16 | 1 | 0.01 | 0.99 | 99.0 | Com. 1-590 | Com. 3-44 | anisole /decahydronaphthalene 7/3 |
| Ex. 17 | 1 | 0.01 | 0.99 | 99.0 | Com. 1-590 | Com. 3-44 | xylene /1-methylnaphthalene 7/3 |
| Ex. 18 | 1 | 0.01 | 0.99 | 99.0 | Com. 1-590 | Com. 3-44 | 3-phennoxytoluene /cyclohexylbenzene 7/3 |
| Ex. 19 | 1 | 0.01 | 0.99 | 99.0 | Com. 1-590 | Com. 3-44 | n-octylbenzene /diphenyl ether 7/3 |
| Ex. 20 | 1 | 0.01 | 0.99 | 99.0 | Com. 1-590 | Com. 3-44 | cyclohexylbenzene /diphenyl ether 7/3 |
| Ex. 21 | 1 | 0.01 | 0.99 | 99.0 | Com. 1-590 | Com. 3-44 | cyclohexylbenzene /3-phennoxytoluene /4-fluoroanisole 6/3/1 |
| Ex. 22 | 1 | 0.01 | 0.99 | 99.0 | Com. 1-590 | Com. 3-44 | Ethyl benzoate /3-phennoxytoluene 5/5 |

**[Table 5B]**

| | Concentration | | | | 1st | 2nd | 3rd |
|---|---|---|---|---|---|---|---|
| | solidi | 1st | 2nd | 3rd | | | |
| Ex. 23 | 1 | 0.01 | 0.99 | 99.0 | Com. 1-411 | Com. BH-2 | cyclohexylbenzene /3-phennoxytoluene 7/3 |
| Ex. 24 | 1 | 0.01 | 0.99 | 99.0 | Com. 1-590 | Com. BH-2 | cyclohexylbenzene /3-phennoxytoluene 7/3 |
| Ex. 25 | 1 | 0.01 | 0.99 | 99 | Com. 1-590 | Com. BH-3 | cyclohexylbenzene /3-phennoxytoluene 7/3 |
| Ex. 26 | 1 | 0.01 | 0.99 | 99 | Com. 1-590 | Com. BH-4 | cyclohexylbenzene/3-phennoxytoluene 7/3 |
| Ex. 27 | 1 | 0.01 | 0.99 | 99 | Com. 1-590 | Com. BH-5 | cyclohexylbenzene /3-phennoxytoluene 7/3 |
| Ex. 28 | 1 | 0.01 | 0.99 | 99 | Com. 1-590 | Com. BH-6 | cyclohexylbenzene /3-phennoxytoluene 7/3 |
| Ex. 29 | 1 | 0.01 | 0.99 | 99 | Com. 1-590 | Com. BH-7 | cyclohexylbenzene /3-phennoxytoluene 7/3 |

### <Evaluation>

For the obtained light emitting layer-forming compositions in Examples 11 to 29, solubility and an application property were evaluated.

### (Evaluation of solubility)

An example in which a whole compound was dissolved to obtain a uniform solution was evaluated to be "soluble", and an example in which an insoluble matter remained was evaluated to be "insoluble".

### (Evaluation of application property)

For an example evaluated to be "soluble" in evaluation of solubility of a prepared ink composition, a film forming property of a film obtained after spin coating film formation or inkjet printing was evaluated. An example in which a pin hole was formed, a compound was precipitated, or an non-uniform portion was observed in a film after film formation was evaluated to be "poor", and an example in which a pin hole was not formed, a compound was not precipitated, and a non-uniform portion was not observed in a film after film formation was evaluated to be "favorable".

### <Application method in spin coating>

UV-O3 treatment was performed by irradiating a clean glass substrate having a thickness of 0.5 mm and a size of 28 × 26 mm with irradiation energy 1000 mJ/cm² (low-pressure mercury lamp (254 nm)). Subsequently, 0.3 to 0.6 mL of an ink composition was dropped onto the glass to perform spin coating (slope, five seconds → 500 to 5000 rpm, 10 seconds → slope, five seconds). Furthermore, the ink composition was dried on a hot plate at 120°C for 10 minutes.

### <Application method in inkjet>

For an application property, an ink composition was discharged into 100 ppi pixels using an inkjet, and dried at 100°C to form a coating film, a film forming property of which was evaluated.

### <Method for measuring viscosity>

Viscosity was measured using a cone plate type rotary viscometer.

### <Method for measuring surface tension>

Surface tension was measured by a suspension method.

**[Table 6A]**

| | Soluble | Application Property | | Inkjet Discharge Stability | | Viscosity | Surface Tension |
|---|---|---|---|---|---|---|---|
| | | Spin Coating | Inkjet | Initial | After lapse of 24 hours | mPa·s | mN/m |
| Ex. 11 | Soluble | Favorable | Favorable | Favorable | Favorable | - | - |
| Ex. 12 | Soluble | Favorable | Favorable | Favorable | Favorable | - | - |
| Ex. 13 | Soluble | Favorable | Favorable | Favorable | Favorable | - | - |
| Ex. 14 | Soluble | Favorable | Favorable | Favorable | Favorable | - | - |
| Ex. 15 | Soluble | Favorable | Favorable | Favorable | Favorable | - | - |
| Ex. 16 | Soluble | Favorable | Favorable | Favorable | Favorable | - | - |
| Ex. 17 | Soluble | Favorable | Favorable | Favorable | Favorable | - | - |
| Ex. 18 | Soluble | Favorable | Favorable | Favorable | Favorable | 3.9 | 35.4 |
| Ex. 19 | Soluble | Favorable | Favorable | Favorable | Favorable | | |
| Ex. 20 | Soluble | Favorable | Favorable | Favorable | Favorable | - | - |
| Ex. 21 | Soluble | Favorable | Favorable | Favorable | Favorable | - | - |
| Ex. 22 | Soluble | Favorable | Favorable | Favorable | Favorable | - | - |

**[Table 6B]**

| | Soluble | Application Property | | Inkjet Discharge Stability | | Viscosity | Surface Tension |
|---|---|---|---|---|---|---|---|
| | | Spin Coating | Inkjet | Initial | After lapse of 24 hours | mPa·s | mN/m |
| Ex. 23 | Soluble | Favorable | Favorable | Favorable | Favorable | - | - |
| Ex. 24 | Soluble | Favorable | Favorable | Favorable | Favorable | - | - |
| Ex. 25 | Soluble | Favorable | Favorable | Favorable | Favorable | - | - |
| Ex. 26 | Soluble | Favorable | Favorable | Favorable | Favorable | - | - |
| Ex. 27 | Soluble | Favorable | Favorable | Favorable | Favorable | - | - |
| Ex. 28 | Soluble | Favorable | Favorable | Favorable | Favorable | - | - |
| Ex. 29 | Soluble | Favorable | Favorable | Favorable | Favorable | - | - |

Each of Examples of the present application exhibited favorable solubility and application property.

### <Examples 30 and 31>

### <Evaluation of application type organic EL element>

Next, an organic EL element obtained by applying and forming an organic layer will be described. Table 7 indicates material composition of each layer in a manufactured organic EL element.
2nd: Second component
1st: First component
EW: Emission wavelength
Ef.: Efficiency
Com.: Compound
Comp.: Composition

**[Table 7]**

| | HIL 40nm | HTL 30nm | LEL 20nm | | | ETL 30nm | NE 1 nm/ 100nm | EW [nm] | Ef . [%] |
|---|---|---|---|---|---|---|---|---|---|
| | | | 2nd | ist | comp. | | | | |
| Ex. 30 | PEDOT : PSS | XLP -101 | Com. 3-44 | Com. 1-411 | Ex. 11 | ET1 | LiF/ Al | 464 | 6.3 |
| Ex. 31 | PEDOT : PSS | XLP -101 | Com. BH-2 | Com. 1-590 | Ex. 24 | ET1 | LiF/ Al | 464 | 6.3 |

### <Example 30>

A glass substrate on which ITO had been vapor-deposited at a thickness of 150 nm was spin-coated with a PEDOT: PSS solution, and fired on a hot plate at 200°C for one hour to form a PEDOT: PSS film having a film thickness of 40 nm (hole injection layer).
Subsequently, the PEDOT: PSS film was spin-coated with an XLP-101 solution, and fired on a hot plate at 200°C for one hour to form a film having a film thickness of 30 nm (hole transport layer). Subsequently, the film was spin-coated with the light emitting layer-forming composition prepared in Example 11 and fired on a hot plate at 120°C for one hour to form a light emitting layer having a film thickness of 20 nm.

The manufactured multilayer film was fixed to a substrate holder of a commercially available vapor deposition apparatus (manufactured by Showa Shinku Co., Ltd.). A molybdenum vapor deposition boat containing ET1, a molybdenum vapor deposition boat containing LiF, and a tungsten vapor deposition boat containing aluminum were mounted thereon. The pressure of a vacuum chamber was reduced to 5 × 10⁻⁴ Pa. Thereafter, ET1 was heated and vapor-deposited so as to have a film thickness of 30 nm, thus forming an electron transport layer. A vapor deposition rate at the time of forming the electron transport layer was 1 nm/sec. Thereafter, LiF was heated and vapor-deposited at a vapor deposition rate of 0.01 to 0.1 nm so as to have a film thickness of 1 nm. Subsequently, aluminum was heated and vapor-deposited so as to have a film thickness of 100 nm to form a negative electrode, thus obtaining an organic EL element.

### <Example 31>

An organic EL element was manufactured in a similar manner to Example 30 except that the light emitting layer-forming composition prepared in Example 24 was used instead of the light emitting layer-forming composition prepared in Example 11.

As described above, in general, an organic EL element formed by a wet film formation method is considered to obtain characteristics equal to those of an organic EL element having a similar element configuration formed by a vacuum vapor deposition method. These elements can obtain characteristics equal to those of a vapor deposition type organic EL element having the same material composition of a light emitting layer if an application step and a drying step are optimized.

Hitherto, some of the compounds according to the present invention have been evaluated as materials for an organic EL element, and have been indicated to be excellent materials. However, other compounds that have not been evaluated each have the same basic skeleton and each have a similar structure as a whole. A person skilled in the art can understand that the other compounds are similarly excellent materials of an organic EL element. [Industrial applicability]

By providing a novel cycloalkane-fused polycyclic aromatic compound, for example, the present invention can increase choices of a material for an organic device such as a material for an organic EL element. In addition, by using the novel cycloalkane-fused polycyclic aromatic compound as a material for an organic EL element, for example, the present invention can provide an organic EL element having excellent emission sufficiency or element life, a display device including the organic EL element, a lighting device including the organic EL element, and the like.

### [Reference Signs List]

- 100: Organic electroluminescent element
- 101: Substrate
- 102: Positive electrode
- 103: Hole injection layer
- 104: Hole transport layer
- 105: Light emitting layer
- 106: Electron transport layer
- 107: Electron injection layer
- 108: Negative electrode

## Claims

1. A polycyclic aromatic compound represented by the following general formula (1) or a multimer of a polycyclic aromatic compound having a plurality of structures each represented by the following general formula (1): 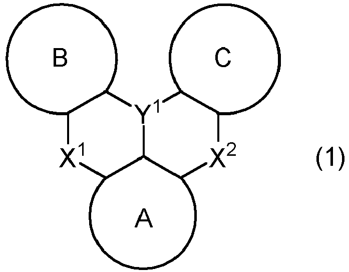
wherein in the above formula (1),
ring A, ring B, and ring C each independently represent an aryl ring or a heteroaryl ring, while at least one hydrogen atom in these rings may be substituted,
Y¹ represents B, P, P=O, P=S, Al, Ga, As, Si-R, or Ge-R, while R in the Si-R and Ge-R represents an aryl, an alkyl or a cycloalkyl,
X¹ and X² each independently represent >O, >N-R, >C(-R)₂, >S, or >Se, while R of the >N-R represents an optionally substituted aryl, an optionally substituted heteroaryl, an optionally substituted alkyl, or an optionally substituted cycloalkyl, R of the >C(-R)₂ represents a hydrogen atom, an optionally substituted aryl, an optionally substituted alkyl, or an optionally substituted cycloalkyl, and at least one of R in the >N-R and R in the >C(-R)₂ may be bonded to at least one of the ring A, ring B, and ring C via a linking group or a single bond,
at least one hydrogen atom in the compound or structure represented by formula (1) may be substituted by a deuterium atom, cyano, or a halogen atom, and
at least one of the ring A, ring B, ring C, aryl and heteroaryl in the compound or structure represented by the formula (1) is fused with at least one cycloalkane, at least one hydrogen atom in the cycloalkane is substituted at an α-position carbon atom of the cycloalkane, at least one -CH₂- in the cycloalkane may be substituted with -O-.

2. The polycyclic aromatic compound or the multimer thereof according to claim 1, wherein
in the above formula (1),
the ring A, ring B, and ring C each independently represent an aryl ring or a heteroaryl ring, while at least one hydrogen atom in these rings may be substituted by a substituted or unsubstituted aryl, a substituted or unsubstituted heteroaryl, a substituted or unsubstituted diarylamino, a substituted or unsubstituted diheteroarylamino, a substituted or unsubstituted arylheteroarylamino, a substituted or unsubstituted diarylboryl (the two aryls may be bonded via a single bond or a linking group), a substituted or unsubstituted alkyl, a substituted or unsubstituted cycloalkyl, a substituted or unsubstituted alkoxy, a substituted or unsubstituted aryloxy, or a substituted silyl, and each of these rings has a 5-membered or 6-membered ring sharing a bond with a fused bicyclic structure at a center of the above formula constituted by Y¹, X¹, and X²,
Y¹ represents B, P, P=O, P=S, Al, Ga, As, Si-R, or Ge-R, while R in the Si-R and Ge-R represents an aryl, an alkyl or a cycloalkyl,
X¹ and X² each independently represent >O, >N-R, >C(-R)₂, >S, or >Se, while R in the >N-R represents an aryl optionally substituted by an alkyl or a cycloalkyl, a heteroaryl optionally substituted by an alkyl or a cycloalkyl, an alkyl, or a cycloalkyl, R in the >C(-R)₂, represents a hydrogen atom, an aryl optionally substituted by an alkyl or a cycloalkyl, an alkyl, or a cycloalkyl, at least one of R in the >N-R and R in the >C(-R)₂ may be bonded to at least one of the ring A, ring B, and ring C via -0-, -S-, -C(-R)₂-, or a single bond, and R in the -C(-R)₂-represents a hydrogen atom or an alkyl or a cycloalkyl,
at least one hydrogen atom in the compound or structure represented by formula (1) may be substituted by a deuterium atom, cyano, or a halogen atom,
in a case of a multimer, the multimer is a dimer or a trimer having two or three structures each represented by general formula (1), and
at least one of the ring A, ring B, ring C, aryl and heteroaryl in the compound or structure represented by the formula (1) is fused with at least one cycloalkane, at least one hydrogen atom in the cycloalkane is substituted at an α-position carbon atom of the cycloalkane, at least one -CH₂- in the cycloalkane may be substituted with -O-.

3. The polycyclic aromatic compound according to claim 1, represented by the following general formula (2): 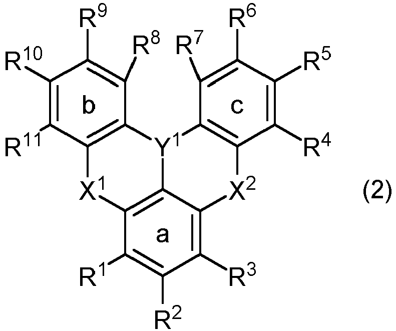
wherein in the above formula (2),
any "-C(-R)=" (where R is R¹ to R¹¹ in the formula (2)) in ring a, ring b, and ring c may be replaced with "-N=", any "-C(-R)=C(-R)-" (where R is R¹ to R¹¹ in the formula (2)) may be replaced with "-N(-R)-", "-O-", or "-S-", R of the "-N(-R)-" represents an aryl, an alkyl, or a cycloalkyl,
R¹ to R¹¹ each independently represent a hydrogen atom, an aryl, a heteroaryl, a diarylamino, a diheteroarylamino, an arylheteroarylamino, a diarylboryl (the two aryls may be bonded via a single bond or a linking group), an alkyl, a cycloalkyl, an alkoxy, an aryloxy, a triarylsilyl, a trialkylsilyl, a tricycloalkylsilyl, a dialkylcycloalkylsilyl, or an alkyldicycloalkylsilyl, while at least one hydrogen atom in these may be substituted by an aryl, a heteroaryl, an alkyl, or a cycloalkyl, adjacent groups among R¹ to R¹¹ may be bonded to each other to form an aryl ring or a heteroaryl ring together with ring a, ring b, or ring c, at least one hydrogen atom in the ring thus formed may be substituted by an aryl, a heteroaryl, a diarylamino, a diheteroarylamino, an arylheteroarylamino, a diarylboryl (the two aryls may be bonded via a single bond or a linking group), an alkyl, a cycloalkyl, an alkoxy, an aryloxy, a triarylsilyl, a trialkylsilyl, a tricycloalkylsilyl, a dialkylcycloalkylsilyl, or an alkyldicycloalkylsilyl, while at least one hydrogen atom in these may be substituted by an aryl, a heteroaryl, an alkyl, or a cycloalkyl,
Y¹ represents B, P, P=O, P=S, Al, Ga, As, Si-R, or Ge-R, while R in the Si-R and Ge-R represents an aryl having 6 to 12 carbon atoms, an alkyl having 1 to 6 carbon atoms or a cycloalkyl having 3 to 14 carbon atoms,
X¹ and X² each independently represent >O, >N-R, >C(-R)₂, >S, or >Se, while R in the >N-R represents an aryl having 6 to 12 carbon atoms, a heteroaryl having 2 to 15 carbon atoms, an alkyl having 1 to 6 carbon atoms, or a cycloalkyl having 3 to 14 carbon atoms, R in the >C(-R)₂ represents a hydrogen atom, an aryl having 6 to 12 carbon atoms, an alkyl having 1 to 6 carbon atoms, or a cycloalkyl having 3 to 14 carbon atoms, at least one of R in the >N-R and R in the >C(-R)₂ may be bonded to at least one of the ring a, ring b, and ring c via -0-, -S-, -C(-R)₂-, or a single bond, and R in the -C(-R)₂- represents an alkyl having 1 to 6 carbon atoms or a cycloalkyl having 3 to 14 carbon atoms,
at least one hydrogen atom in the compound represented by formula (2) may be substituted by a deuterium atom, cyano or a halogen atom, and
at least one of the ring a, ring b, ring c, formed ring, aryl and heteroaryl in the compound represented by the formula (2) is fused with at least one cycloalkane having 3 to 24 carbon atoms, at least one hydrogen atom in the cycloalkane is substituted at an α-position carbon atom of the cycloalkane by an aryl having 6 to 30 carbon atoms, a heteroaryl having 2 to 30 carbon atoms, an alkyl having 1 to 24 carbon atoms, or a cycloalkyl having 3 to 24 carbon atoms, at least one -CH₂- in the cycloalkane may be substituted with -O-.

4. The polycyclic aromatic compound according to claim 3, wherein
in the above formula (2),
any "-C(-R)=" (where R is R¹ to R¹¹ in the formula (2)) in ring a, ring b, and ring c may be replaced with "-N=", any "-C(-R)=C(-R)-" (where R is R¹ to R¹¹ in the formula (2)) may be replaced with "-N(-R)-", "-O-", or "-S-", R of the "-N(-R)-" represents an aryl having 6 to 12 carbon atoms, an alkyl having 1 to 6 carbon atoms, or a cycloalkyl having 3 to 14 carbon atoms,
R¹ to R¹¹ each independently represent a hydrogen atom, an aryl having 6 to 30 carbon atoms, a heteroaryl having 2 to 30 carbon atoms, a diarylamino (the aryl is an aryl having 6 to 12 carbon atoms), a diarylboryl (the aryl is an aryl having 6 to 12 carbon atoms, the two aryls may be bonded via a single bond or a linking group), an alkyl having 1 to 24 carbon atoms, a cycloalkyl having 3 to 24 carbon atoms, a triarylsilyl (the aryl is an aryl having 6 to 12 carbon atoms), or a trialkylsilyl (the alkyl is an alkyl having 1 to 6 carbon atoms), adjacent groups among R¹ to R¹¹ may be bonded to each other to form an aryl ring having 9 to 16 carbon atoms or a heteroaryl ring having 6 to 15 carbon atoms together with ring a, ring b, or ring c, at least one hydrogen atom in the ring thus formed may be substituted by an aryl having 6 to 10 carbon atoms, an alkyl having 1 to 12 carbon atoms, a cycloalkyl having 3 to 16 carbon atoms, a triarylsilyl (the aryl is an aryl having 6 to 12 carbon atoms), or a trialkylsilyl (the alkyl is an alkyl having 1 to 5 carbon atoms),
Y¹ represents B, P, P=O, P=S, or Si-R, while R in the Si-R represents an aryl having 6 to 10 carbon atoms, an alkyl having 1 to 5 carbon atoms or a cycloalkyl having 5 to 10 carbon atoms,
X¹ and X² each independently represent >O, >N-R, >C(-R)₂, or >S, while R in the >N-R represents an aryl having 6 to 10 carbon atoms, an alkyl having 1 to 5 carbon atoms, or a cycloalkyl having 5 to 10 carbon atoms, R in the >C(-R)₂ represents a hydrogen atom, an aryl having 6 to 10 carbon atoms, an alkyl having 1 to 5 carbon atoms, or a cycloalkyl having 5 to 10 carbon atoms,
at least one hydrogen atom in the compound represented by formula (2) may be substituted by a deuterium atom, cyano or a halogen atom, and
at least one of the ring a, ring b, ring c, formed ring, aryl and heteroaryl in the compound represented by the formula (2) is fused with at least one cycloalkane having 3 to 20 carbon atoms, at least one hydrogen atom in the cycloalkane is substituted at an α-position carbon atom of the cycloalkane by an aryl having 6 to 16 carbon atoms, a heteroaryl having 2 to 22 carbon atoms, an alkyl having 1 to 12 carbon atoms, or a cycloalkyl having 3 to 16 carbon atoms
or wherein
in the above formula (2),
any "-C(-R)=" (where R is R¹ to R¹¹ in the formula (2)) in ring a, ring b, and ring c may be replaced with "-N=", any "-C(-R)=C(-R)-" (where R is R¹ to R¹¹ in the formula (2)) may be replaced with "-N(-R)-", "-O-", or "-S-", R of the "-N(-R)-" represents an aryl having 6 to 10 carbon atoms, an alkyl having 1 to 5 carbon atoms, or a cycloalkyl having 5 to 10 carbon atoms,
R¹ to R¹¹ each independently represent a hydrogen atom, an aryl having 6 to 16 carbon atoms, a heteroaryl having 2 to 20 carbon atoms, a diarylamino (the aryl is an aryl having 6 to 10 carbon atoms), an alkyl having 1 to 12 carbon atoms, or a cycloalkyl having 3 to 16 carbon atoms,
Y¹ represents B, P, P=O, or P=S,
X¹ and X² each independently represent >O, >N-R, or >C(-R)₂, while R in the >N-R represents an aryl having 6 to 10 carbon atoms, an alkyl having 1 to 5 carbon atoms, or a cycloalkyl having 5 to 10 carbon atoms, R in the >C(-R)₂ represents a hydrogen atom, an aryl having 6 to 10 carbon atoms, an alkyl having 1 to 5 carbon atoms, or a cycloalkyl having 5 to 10 carbon atoms,
at least one hydrogen atom in the compound represented by formula (2) may be substituted by a deuterium atom, cyano or a halogen atom, and
at least one of the ring a, ring b, ring c, and aryl having 6 to 10 carbon atoms as R of the >N-R in the compound represented by the formula (2) is fused with at least one cycloalkane having 3 to 16 carbon atoms, at least one hydrogen atom in the cycloalkane is substituted at an α-position carbon atom of the cycloalkane by an alkyl having 1 to 6 carbon atoms or a cycloalkyl having 3 to 14 carbon atoms
or wherein
in the above formula (2),
R¹ to R¹¹ each independently represent a hydrogen atom, an aryl having 6 to 16 carbon atoms, a diarylamino (the aryl is an aryl having 6 to 10 carbon atoms), an alkyl having 1 to 12 carbon atoms, or a cycloalkyl having 3 to 16 carbon atoms,
Y¹ represents B,
X¹ and X² both represent >N-R, or X¹ represents >N-R and X² represents >O, while R in the >N-R represents an aryl having 6 to 10 carbon atoms, an alkyl having 1 to 5 carbon atoms, or a cycloalkyl having 5 to 10 carbon atoms,
at least one hydrogen atom in the compound represented by formula (2) may be substituted by a deuterium atom or a halogen atom, and
at least one of the ring a, ring b, ring c, and aryl having 6 to 10 carbon atoms as R of the >N-R in the compound represented by the formula (2) is fused with at least one cycloalkane having 3 to 14 carbon atoms, at least one hydrogen atom in the cycloalkane is substituted at an α-position carbon atom of the cycloalkane by an alkyl having 1 to 5.

5. The polycyclic aromatic compound or the multimer thereof according to any one of claims 1 to 4, which is substituted by a diarylamino group fused with one or more cycloalkanes, a carbazolyl group fused with one or more cycloalkanes, or a benzocarbazolyl group fused with one or more cycloalkanes.

6. The polycyclic aromatic compound according to claim 3 or 4, wherein R² is a diarylamino group fused with one or more cycloalkanes or a carbazolyl group fused with one or more cycloalkanes.

7. The polycyclic aromatic compound according to claim 5 or 6, wherein the cycloalkane is a cycloalkane having 3 to 20 carbon atoms.

8. The polycyclic aromatic compound or the multimer thereof according to any one of claims 1 to 6, wherein the halogen is fluorine.

9. A polycyclic aromatic compound according to claim 1, which is represented by any one of the following structural formulas: 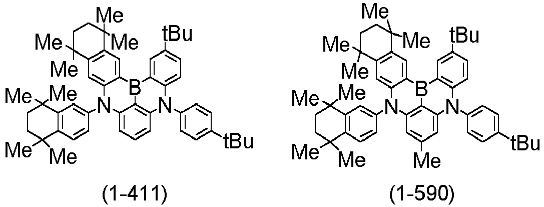 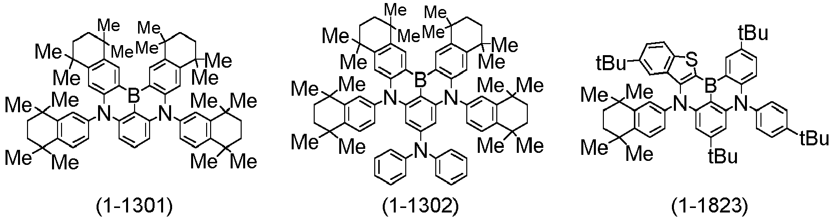 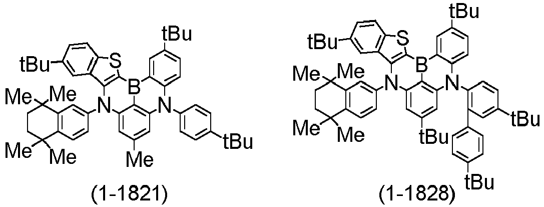 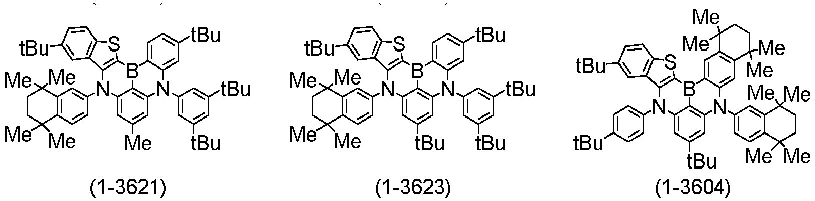 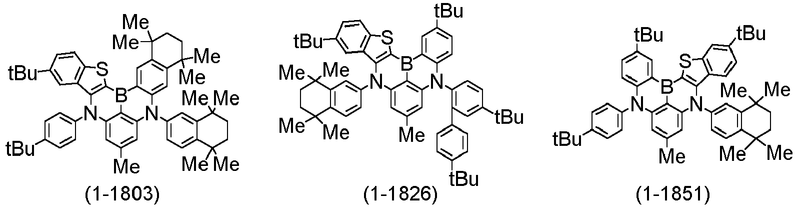 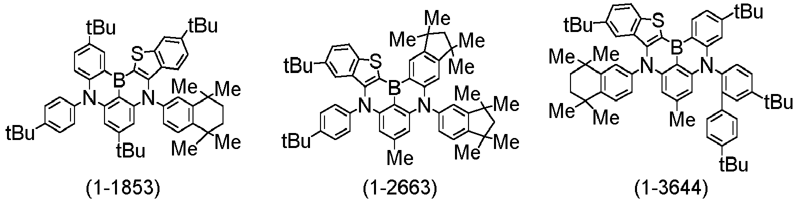 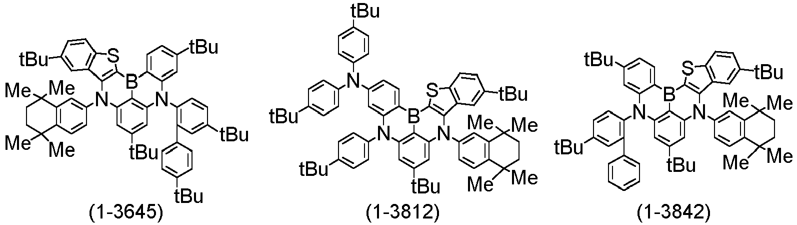 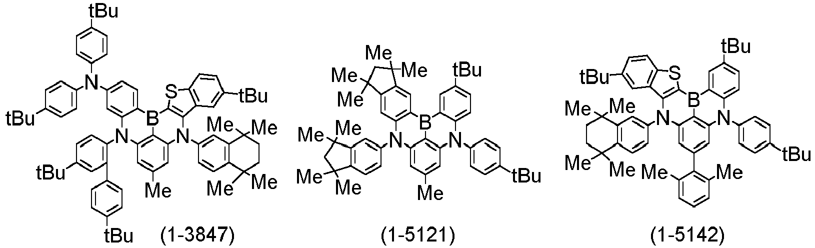 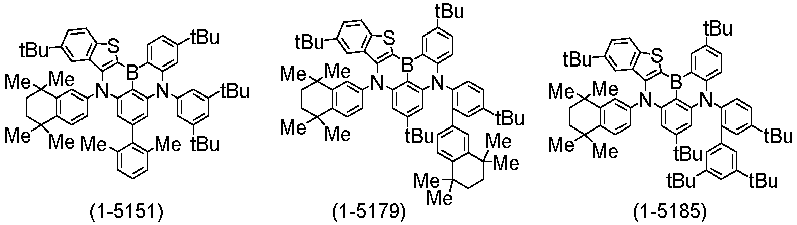 wherein in each of the above structural formulas, "Me" indicates a methyl group and "tBu" indicates a t-butyl group.

10. A material for an organic device, the material comprising the polycyclic aromatic compound or the multimer thereof according to any one of claims 1 to 9.

11. The material for an organic device according to claim 10, which is a material for an organic electroluminescent element, a material for an organic field effect transistor, or a material for an organic thin film solar cell,
preferably wherein the material for an organic electroluminescent element is a material for a light emitting layer.

12. An organic electroluminescent element comprising: a pair of electrodes composed of a positive electrode and a negative electrode; and an organic layer disposed between the pair of electrodes and comprising the polycyclic aromatic compound or the multimer thereof according to any one of claims 1 to 9.

13. The organic electroluminescent element according to claim 12, wherein the organic layer is a light emitting layer,
preferably wherein the light emitting layer comprises a host and the polycyclic aromatic compound or the multimer thereof, the reactive compound, the polymer compound, the crosslinked polymer, the pendant type polymer compound, or the pendant type crosslinked polymer as a dopant,
preferably wherein the host is an anthracene-based compound, a fluorene-based compound, or a dibenzochrysene-based compound.

14. The organic electroluminescent element according to claim 12 or 13, further comprising at least one of an electron transport layer and an electron injection layer disposed between the negative electrode and the light emitting layer, wherein at least one of the electron transport layer and the electron injection layer contains at least one selected from the group consisting of a borane derivative, a pyridine derivative, a fluoranthene derivative, a BO-based derivative, an anthracene derivative, a benzofluorene derivative, a phosphine oxide derivative, a pyrimidine derivative, a carbazole derivative, a triazine derivative, a benzimidazole derivative, a phenanthroline derivative, and a quinolinol-based metal complex,
preferably wherein at least one layer of the electron transport layer and electron injection layer further include at least one selected from the group consisting of an alkali metal, an alkaline earth metal, a rare earth metal, an oxide of an alkali metal, a halide of an alkali metal, an oxide of an alkaline earth metal, a halide of an alkaline earth metal, an oxide of a rare earth metal, a halide of a rare earth metal, an organic complex of an alkali metal, an organic complex of an alkaline earth metal, and an organic complex of a rare earth metal,
further preferably wherein at least one layer of a hole injection layer, a hole transport layer, a light emitting layer, an electron transport layer, and an electron injection layer comprises a polymer compound obtained by polymerizing a low molecular weight compound that can form each of the layers as a monomer or a crosslinked polymer obtained by further crosslinking the polymer compound, or a pendant type polymer compound obtained by a reaction between the low molecular weight compound that can form each of the layers and a main chain type polymer or a pendant type crosslinked polymer obtained by further crosslinking the pendant type polymer compound.

15. A display apparatus or a lighting apparatus comprising the organic electroluminescent element according to any one of claims 12 to 14.

## Patentansprüche

1. Polycyclische aromatische Verbindung, dargestellt durch die folgende allgemeine Formel (1), oder ein Multimer einer polycyclischen aromatischen Verbindung mit einer Vielzahl von Strukturen, die jeweils durch die folgende allgemeine Formel (1) dargestellt sind: 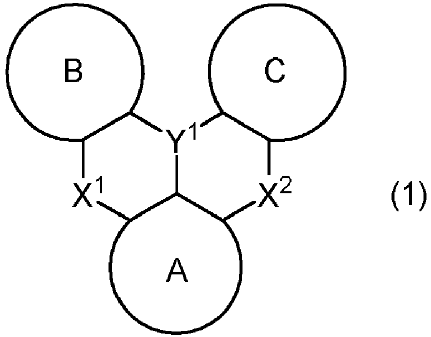
wobei in der obigen Formel (1)
Ring A, Ring B und Ring C jeweils unabhängig einen Arylring oder einen Heteroarylring darstellen, wobei mindestens ein Wasserstoffatom dieser Ringe substituiert sein kann,
Y¹ B, P, P=O, P=S, Al, Ga, As, Si-R oder Ge-R, wobei R in dem Si-R und Ge-R ein Aryl, ein Alkyl oder ein Cycloalkyl darstellt,
X¹ und X² jeweils unabhängig >O, >N-R, >C(-R)₂, >S oder >Se darstellen, wobei R von dem >N-R ein optional substituiertes Aryl, ein optional substituiertes Heteroaryl, ein optional substituiertes Alkyl oder ein optional substituiertes Cycloalkyl darstellt, R von dem >C(-R)₂ ein Wasserstoffatom, ein optional substituiertes Aryl, ein optional substituiertes Alkyl oder ein optional substituiertes Cycloalkyl darstellt und mindestens eines von dem R in dem >N-R und R in dem >C(-R)₂ an mindestens einen von dem Ring A, Ring B und Ring C über eine Verknüpfungsgruppe oder eine Einfachbindung gebunden sein kann,
mindestens ein Wasserstoffatom in der durch Formel (1) dargestellten Verbindung oder Struktur durch ein Deuteriumatom, Cyano oder ein Halogenatom substituiert sein kann und
mindestens einer/eines von dem Ring A, Ring B, Ring C, Aryl und Heteroaryl in der durch die Formel (1) dargestellten Verbindung oder Struktur mit mindestens einem Cycloalkan kondensiert ist, mindestens ein Wasserstoffatom in dem Cycloalkan an einem Kohlenstoffatom in α-Position des Cycloalkans substituiert ist, mindestens ein -CH₂- in dem Cycloalkan mit -O- substituiert sein kann.

2. Polycyclische aromatische Verbindung oder das Multimer davon gemäß Anspruch 1, wobei in der obigen Formel (1)
der Ring A, Ring B und Ring C jeweils unabhängig einen Arylring oder einen Heteroarylring darstellen, wobei mindestens ein Wasserstoffatom in diesen Ringen durch ein substituiertes oder unsubstituiertes Aryl, ein substituiertes oder unsubstituiertes Heteroaryl, ein substituiertes oder unsubstituiertes Diarylamino, ein substituiertes oder unsubstituiertes Diheteroarylamino, ein substituiertes oder unsubstituiertes Arylheteroarylamino, ein substituiertes oder unsubstituiertes Diarylboryl (die zwei Aryle können über eine Einfachbindung oder eine Verknüpfungsgruppe verbunden sein), ein substituiertes oder unsubstituiertes Alkyl, ein substituiertes oder unsubstituiertes Cycloalkyl, ein substituiertes oder unsubstituiertes Alkoxy, ein substituiertes oder unsubstituiertes Aryloxy oder ein substituiertes oder unsubstituiertes Silyl substituiert sein kann und jeder dieser Ringe einen 5-gliedrigen oder 6-gliedrigen Ring aufweist, der eine Bindung mit einer kondensierten bicyclischen Struktur in einem Zentrum der obigen Formel, gebildet durch Y¹, X¹ und X², teilt,
Y¹ B, P, P=O, P=S, Al, Ga, As, Si-R oder Ge-R darstellt, wobei R in dem Si-R und Ge-R ein Aryl, ein Alkyl oder ein Cycloalkyl darstellt,
X¹ und X² jeweils unabhängig >O, >N-R, >C(-R)₂, >S oder >Se darstellen, wobei R von dem >N-R ein Aryl, optional substituiert durch ein Alkyl oder ein Cycloalkyl, ein Heteroaryl, optional substituiert durch ein Alkyl oder ein Cycloalkyl, ein Alkyl oder ein Cycloalkyl darstellt, R in dem >C(-R)₂ ein Wasserstoffatom, ein Aryl, optional substituiert durch ein Alkyl oder ein Cycloalkyl, ein Alkyl oder ein Cycloalkyl darstellt, mindestens eines von R in dem >N-R und R in dem >C(-R)₂ an mindestens einen von Ring A, Ring B und Ring C über -O-, -S-, -C(-R)₂- oder eine Einfachbindung gebunden sein kann und R in dem -C(-R)₂- ein Wasserstoffatom oder ein Alkyl oder ein Cycloalkyl darstellt,
mindestens ein Wasserstoffatom in der durch Formel (1) dargestellten Verbindung oder Struktur kann durch ein Deuteriumatom, Cyano oder ein Halogenatom substituiert sein,
im Falle eines Multimers das Multimer ein Dimer oder ein Trimer mit zwei oder drei Strukturen ist, die jeweils durch die allgemeine Formel (1) dargestellt sind, und
mindestens einer/eines von dem Ring A, Ring B, Ring C, Aryl und Heteroaryl in der durch die Formel (1) dargestellten Verbindung oder Struktur mit mindestens einem Cycloalkan kondensiert ist, mindestens ein Wasserstoffatom in dem Cycloalkan an einem Kohlenstoffatom in α-Position des Cycloalkans substituiert ist, mindestens ein -CH₂- in dem Cycloalkan kann mit -O- substituiert sein.

3. Polycyclische aromatische Verbindung gemäß Anspruch 1, dargestellt durch die folgende allgemeine Formel (2): 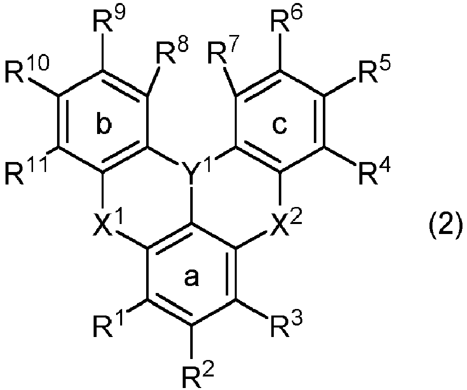 wobei in der Formel (2)
irgendein "-C(-R)=" (worin R R¹ bis R¹¹ in der Formel (2) ist) in Ring a, Ring b und Ring c durch "-N=" ersetzt sein kann, irgendein "-C(-R)=C(-R)-" (worin R R¹ bis R¹¹ in der Formel (2) ist) durch "-N(-R)-", "-O-" oder "-S-" ersetzt sein kann, R von dem "-N(-R)-" ein Aryl, ein Alkyl oder ein Cycloalkyl darstellt,
R¹ bis R¹¹ jeweils unabhängig ein Wasserstoffatom, ein Aryl, ein Heteroaryl, ein Diarylamino, ein Diheteroarylamino, ein Arylheteroarylamino, ein Diarylboryl (die zwei Aryle können über eine Einfachbindung oder eine Verknüpfungsgruppe verbunden sein), ein Alkyl, ein Cycloalkyl, ein Alkoxy, ein Aryloxy, ein Triarylsilyl, ein Trialkylsilyl, ein Tricycloalkylsilyl, ein Dialkylcycloalkylsilyl oder ein Alkyldicycloalkylsilyl darstellen, wobei mindestens ein Wasserstoffatom in diesen durch ein Aryl, ein Heteroaryl, ein Alkyl oder ein Cycloalkyl substituiert sein kann, benachbarte Gruppen unter R¹ bis R¹¹ aneinander gebunden sein können, um einen Arylring oder einen Heteroarylring zusammen mit Ring a, Ring b oder Ring c zu bilden, mindestens ein Wasserstoffatom in dem so gebildeten Ring durch ein Aryl, ein Heteroaryl, ein Diarylamino, ein Diheteroarylamino, ein Arylheteroarylamino, ein Diarylboryl (die zwei Aryle können über eine Einfachbindung oder eine Verknüpfungsgruppe verbunden sein), ein Alkyl, ein Cycloalkyl, ein Alkoxy, ein Aryloxy, ein Triarylsilyl, ein Trialkylsilyl, ein Tricycloalkylsilyl, ein Dialkylcycloalkylsilyl oder ein Alkyldicycloalkylsilyl substituiert sein kann, wobei mindestens ein Wasserstoff in diesen durch ein Aryl, ein Heteroaryl, ein Alkyl oder ein Cycloalkyl substituiert sein kann,
Y¹ B, P, P=O, P=S, Al, Ga, As, Si-R oder Ge-R darstellt, wobei R in dem Si-R und Ge-R ein Aryl mit 6 bis 12 Kohlenstoffatomen, ein Alkyl mit 1 bis 6 Kohlenstoffatomen oder ein Cycloalkyl mit 3 bis 14 Kohlenstoffatomen darstellt,
X¹ und X² jeweils unabhängig >O, >N-R, >C(-R)₂, >S oder >Se darstellen, wobei R in dem >N-R ein Aryl mit 6 bis 12 Kohlenstoffatomen, ein Heteroaryl mit 2 bis 15 Kohlenstoffatomen, ein Alkyl mit 1 bis 6 Kohlenstoffatomen oder ein Cycloalkyl mit 3 bis 14 Kohlenstoffatomen darstellt, R in dem >C(-R)₂ ein Wasserstoffatom, ein Aryl mit 6 bis 12 Kohlenstoffatomen, ein Alkyl mit 1 bis 6 Kohlenstoffatomen oder ein Cycloalkyl mit 3 bis 14 Kohlenstoffatomen darstellt, mindestens eines von R in dem >N-R und R in dem >C(-R)₂ an mindestens einen von dem Ring a, Ring b und Ring c über -O-, -S-, -C(-R)₂-oder eine Einfachbindung gebunden sein kann und R in dem -C(-R)₂- ein Alkyl mit 1 bis 6 Kohlenstoffatomen oder ein Cycloalkyl mit 3 bis 14 Kohlenstoffatomen darstellt,
mindestens ein Wasserstoffatom in der durch Formel (2) dargestellten Verbindung durch ein Deuteriumatom, Cyano oder ein Halogenatom substituiert sein kann und
mindestens einer/eines von dem Ring a, Ring b, Ring c, gebildetem Ring, Aryl und Heteroaryl in der durch die Formel (2) dargestellten Verbindung mit mindestens einem Cycloalkan mit 3 bis 24 Kohlenstoffatomen kondensiert ist, mindestens ein Wasserstoffatom in dem Cycloalkan an einem Kohlenstoffatom in α-Position des Cycloalkans durch ein Aryl mit 6 bis 30 Kohlenstoffatomen, ein Heteroalkyl mit 2 bis 30 Kohlenstoffatomen, ein Alkyl mit 1 bis 24 Kohlenstoffatomen oder ein Cycloalkyl mit 3 bis 24 Kohlenstoffatomen substituiert ist, mindestens ein -CH₂- in dem Cycloalkan mit -O- substituiert sein kann.

4. Polycyclische aromatische Verbindung gemäß Anspruch 3, wobei
in der obigen Formel (2)
irgendein "-C(-R)=" (worin R R¹ bis R¹¹ in der Formel (2) ist) in Ring a, Ring b und Ring c durch "-N=" ersetzt sein kann, irgendein "-C(-R)=C(-R)-" (worin R R¹ bis R¹¹ in der Formel (2) ist) durch "-N(-R)-", "-O-" oder "-S-" ersetzt sein kann, R von dem "-N(-R)-" ein Aryl mit 6 bis 12 Kohlenstoffatomen, ein Alkyl mit 1 bis 6 Kohlenstoffatomen oder ein Cycloalkyl mit 3 bis 14 Kohlenstoffatomen darstellt,
R¹ bis R¹¹ jeweils unabhängig ein Wasserstoffatom, ein Aryl mit 6 bis 30 Kohlenstoffatomen, ein Heteroaryl 2 bis 30 Kohlenstoffatomen, ein Diarylamino (das Aryl ist ein Aryl mit 6 bis 12 Kohlenstoffatomen), ein Diarylboryl (das Aryl ist ein Aryl mit 6 bis 12 Kohlenstoffatomen), ein Diarylboryl (das Aryl ist ein Aryl mit 6 bis 12 Kohlenstoffatomen, die zwei Aryle können über eine Einfachbindung oder eine Verknüpfungsgruppe verbunden sein), ein Alkyl mit 1 bis 24 Kohlenstoffatomen, ein Cycloalkyl mit 3 bis 24 Kohlenstoffatomen, ein Triarylsilyl (das Aryl ist ein Aryl mit 6 bis 12 Kohlenstoffatomen) oder ein Trialkylsilyl (das Alkyl ist ein Alkyl mit 1 bis 6 Kohlenstoffatomen) darstellen, benachbarte Gruppen unter R¹ bis R¹¹ können aneinander gebunden sein können, um zusammen mit Ring a, Ring b oder Ring c einen Arylring mit 9 bis 16 Kohlenstoffatomen oder einem Heteroarylring mit 6 bis 15 Kohlenstoffatomen zu bilden, mindestens ein Wasserstoffatom in dem so gebildeten Ring durch ein Aryl mit 6 bis 10 Kohlenstoffatomen, ein Alkyl mit 1 bis 12 Kohlenstoffatomen, ein Cycloalkyl mit 3 bis 16 Kohlenstoffatomen, ein Triarylsilyl (das Aryl ist ein Aryl mit 6 bis 12 Kohlenstoffatomen) oder ein Trialkylsilyl (das Alkyl ist ein Alkyl mit 1 bis 5 Kohlenstoffatomen) substituiert sein kann,
Y¹ B, P, P=O, P=S oder Si-R darstellt, wobei R in dem Si-R ein Aryl mit 6 bis 10 Kohlenstoffatomen, ein Alkyl mit 1 bis 5 Kohlenstoffatomen oder ein Cycloalkyl mit 5 bis 10 Kohlenstoffatomen darstellt,
X¹ und X² jeweils unabhängig >O, >N-R, >C(-R)₂ oder >S darstellen, wobei R in dem >N-R ein Aryl mit 6 bis 10 Kohlenstoffatomen, ein Alkyl mit 1 bis 5 Kohlenstoffatomen oder ein Cycloalkyl mit 5 bis 10 Kohlenstoffatomen darstellt, R in dem >C(-R)₂ ein Wasserstoffatom, ein Aryl mit 6 bis 10 Kohlenstoffatomen, ein Alkyl mit 1 bis 5 Kohlenstoffatomen oder ein Cycloalkyl mit 5 bis 10 Kohlenstoffatomen darstellt,
mindestens ein Wasserstoffatom in der durch Formel (2) dargestellten Verbindung durch ein Deuteriumatom, Cyano oder ein Halogenatom substituiert sein kann und
mindestens einer/eines von dem Ring a, Ring b, Ring c, gebildetem Ring, Aryl und Heteroaryl in der durch die Formel (2) dargestellten Verbindung mit mindestens einem Cycloalkan mit 3 bis 20 Kohlenstoffatomen kondensiert ist, mindestens ein Wasserstoffatom in dem Cycloalkan an einem Kohlenstoffatom in α-Position des Cycloalkans durch ein Aryl mit 6 bis 16 Kohlenstoffatomen, ein Heteroaryl mit 2 bis 22 Kohlenstoffatomen, ein Alkyl mit 1 bis 12 Kohlenstoffatomen oder ein Cycloalkyl mit 3 bis 16 Kohlenstoffatomen substituiert ist,
oder wobei
in der obigen Formel (2)
irgendein "-C(-R)=" (worin R R¹ bis R¹¹ in der Formel (2) ist) in Ring a, Ring b und Ring c durch "-N=" ersetzt sein kann, irgendein "-C(-R)=C(-R)-" (worin R R¹ bis R¹¹ in der Formel (2) ist) durch "-N(-R)-", "-O-" oder "-S-" ersetzt sein kann, R von dem "-N(-R)-" ein Aryl mit 6 bis 10 Kohlenstoffatomen, ein Alkyl mit 1 bis 5 Kohlenstoffatomen oder ein Cycloalkyl mit 5 bis 10 Kohlenstoffatomen darstellt,
R¹ bis R¹¹ jeweils unabhängig ein Wasserstoffatom, ein Aryl mit 6 bis 16 Kohlenstoffatomen, ein Heteroaryl 2 bis 20 Kohlenstoffatomen, ein Diarylamino (das Aryl ist ein Aryl mit 6 bis 10 Kohlenstoffatomen), ein Alkyl mit 1 bis 12 Kohlenstoffatomen oder ein Cycloalkyl mit 3 bis 16 Kohlenstoffatomen darstellen,
Y¹ B, P, P=O oder P=S darstellt,
X¹ und X² jeweils unabhängig >O, >N-R oder >C(-R)₂ darstellen, wobei R in dem >N-R ein Aryl mit 6 bis 10 Kohlenstoffatomen, ein Alkyl mit 1 bis 5 Kohlenstoffatomen oder ein Cycloalkyl mit 5 bis 10 Kohlenstoffatomen darstellt, R in dem >C(-R)₂ ein Wasserstoffatom, ein Aryl mit 6 bis 10 Kohlenstoffatomen, ein Alkyl mit 1 bis 5 Kohlenstoffatomen oder ein Cycloalkyl mit 5 bis 10 Kohlenstoffatomen darstellt,
mindestens ein Wasserstoffatom in der durch Formel (2) dargestellten Verbindung durch ein Deuteriumatom, Cyano oder ein Halogenatom substituiert sein kann und
mindestens einer/eines von dem Ring a, Ring b, Ring c und Aryl mit 6 bis 10 Kohlenstoffatomen als R von dem >N-R in der durch die Formel (2) dargestellten Verbindung mit mindestens einem Cycloalkan mit 3 bis 16 Kohlenstoffatomen kondensiert ist, mindestens ein Wasserstoffatom in dem Cycloalkan an einem Kohlenstoffatom in α-Position des Cycloalkans durch ein Aryl mit 1 bis 6 Kohlenstoffatomen oder ein Cycloalkyl mit 3 bis 14 Kohlenstoffatomen substituiert ist,
oder wobei
in der obigen Formel 2
R¹ bis R¹¹ jeweils unabhängig ein Wasserstoffatom, ein Aryl mit 6 bis 16 Kohlenstoffatomen, ein Diarylamino (das Aryl ist ein Aryl mit 6 bis 10 Kohlenstoffatomen), ein Alkyl mit 1 bis 12 Kohlenstoffatomen oder ein Cycloalkyl mit 3 bis 16 Kohlenstoffatomen darstellen,
Y¹ B darstellt,
X¹ und X² beide >N-R darstellen oder X¹ >N-R darstellt und X² >O darstellt, wobei R in dem >N-R ein Aryl mit 6 bis 10 Kohlenstoffatomen, ein Alkyl mit 1 bis 5 Kohlenstoffatomen oder ein Cycloalkyl mit 5 bis 10 Kohlenstoffatomen darstellt,
mindestens ein Wasserstoffatom in der durch Formel (2) dargestellten Verbindung durch ein Deuteriumatom oder ein Halogenatom substituiert sein kann und
mindestens einer/eines von dem Ring a, Ring b, Ring c und Aryl mit 6 bis 10 Kohlenstoffatomen als R von dem >N-R in der durch die Formel (2) dargestellten Verbindung mit mindestens einem Cycloalkan mit 3 bis 14 Kohlenstoffatomen kondensiert ist, mindestens ein Wasserstoffatom in dem Cycloalkan an einem Kohlenstoffatom in α-Position des Cycloalkans durch ein Aryl mit 1 bis 5 substituiert ist,

5. Polycyclische aromatische Verbindung oder Multimer davon gemäß einem der Ansprüche 1 bis 4, die/das durch eine Diarylaminogruppe, die mit einem oder mehreren Cycloalkanen kondensiert ist, eine Carbazolylgruppe, die mit einem oder mehreren Cycloalkanen kondensiert ist, oder eine Benzocarbazolylgruppe, die mit einem oder mehreren Cycloalkanen kondensiert ist, substituiert ist.

6. Polycyclische aromatische Verbindung gemäß Anspruch 3 oder 4, wobei R² eine Diarylaminogruppe, die mit einem oder mehreren Cycloalkanen kondensiert ist, oder eine Carbazolylgruppe ist, die mit einem oder mehreren Cycloalkanen kondensiert ist.

7. Polycyclische aromatische Verbindung gemäß Anspruch 5 oder 6, wobei das Cycloalkan ein Cycloalkan mit 3 bis 20 Kohlenstoffatomen ist.

8. Polycyclische aromatische Verbindung oder Multimer davon gemäß einem der Ansprüche 1 bis 6, wobei das Halogen Fluor ist.

9. Polycyclische aromatische Verbindung gemäß Anspruch 1, die durch eine der folgenden Strukturformeln dargestellt ist: 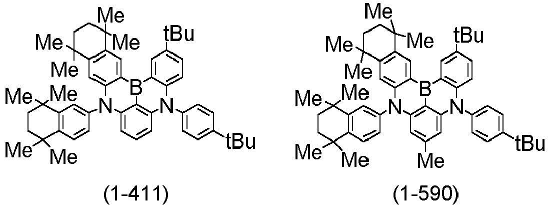 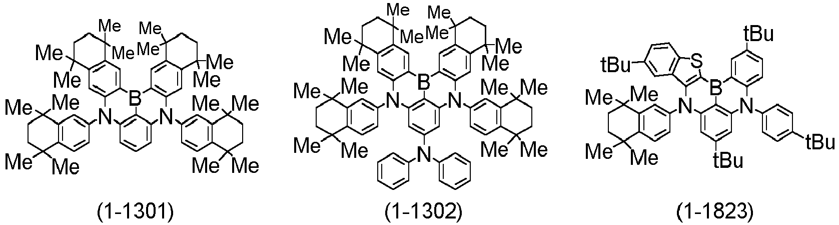 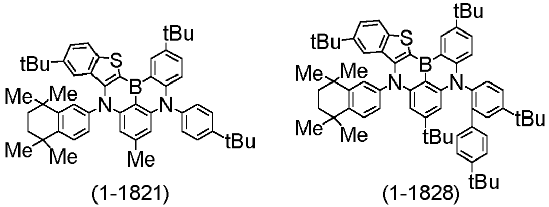 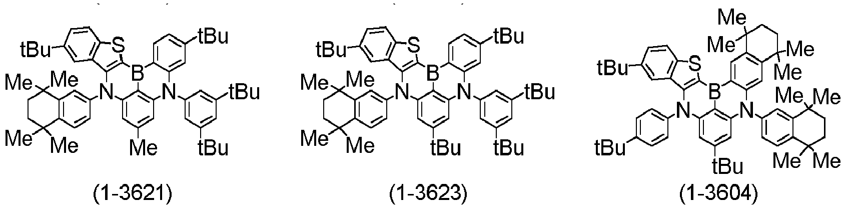 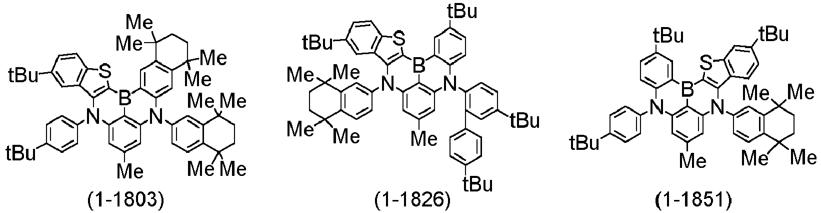 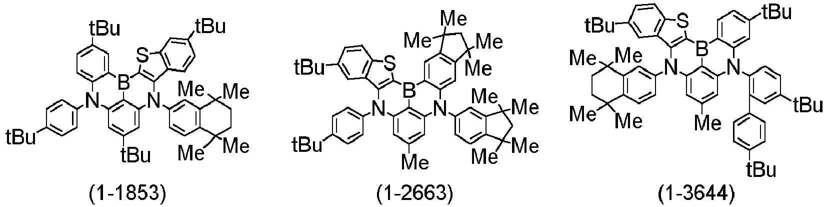 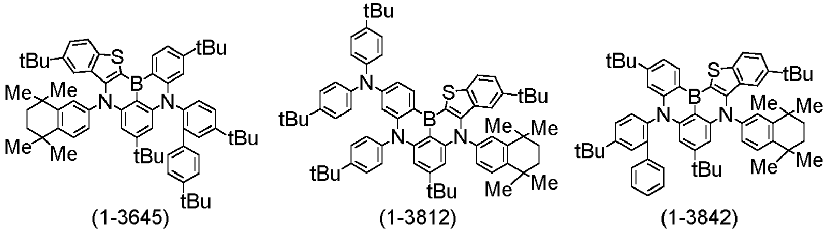 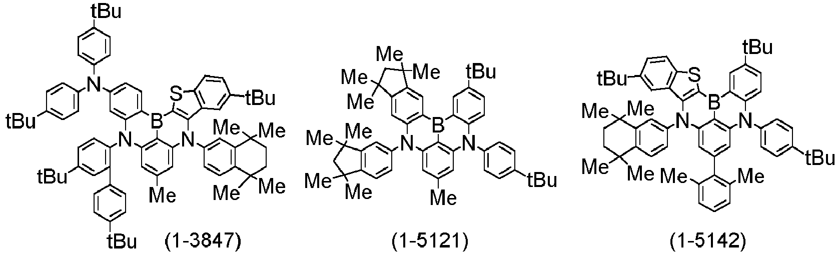 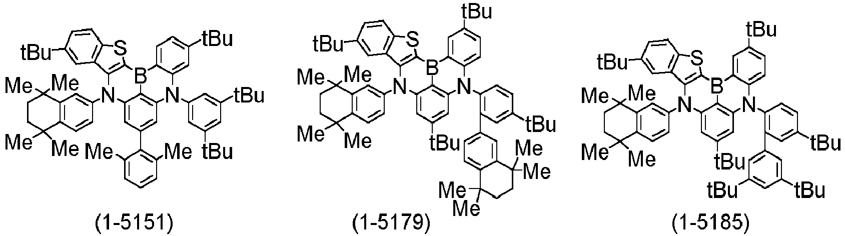 wobei in jeder der obigen Strukturformeln "Me" eine Methylgruppe angibt und "tBu" eine t-Butylgruppe angibt.

10. Material für eine organische Vorrichtung, wobei das Material die polycyclische aromatische Verbindung oder das Multimer davon gemäß einem der Ansprüche 1 bis 9 umfasst.

11. Material für eine organische Vorrichtung gemäß Anspruch 10, das ein Material für ein organisches Elektrolumineszenzelement, ein Material für einen organischen Feldeffekttransistor oder ein Material für eine organische Dünnschichtsolarzelle ist,
wobei das Material für ein organisches Elektrolumineszenzelement vorzugsweise ein Material für eine lichtemittierende Schicht ist.

12. Organisches Elektrolumineszenzelement, umfassend: ein Elektrodenpaar, das aus einer positiven Elektrode und einer negativen Elektrode zusammengesetzt ist, und eine organische Schicht, die zwischen dem Elektrodenpaar angeordnet ist und die polycyclische aromatische Verbindung oder das Multimer davon gemäß einem der Ansprüche 1 bis 9 umfasst.

13. Organisches Elektrolumineszenzelement gemäß Anspruch 12, wobei die organische Schicht eine lichtemittierende Schicht ist,
wobei die lichtemittierende Schicht vorzugsweise einen Wirt und die polycyclische aromatische Verbindung oder das Multimer davon, die reaktive Verbindung, die Polymerverbindung, das vernetzte Polymer, die Polymerverbindung vom Seitenkettentyp oder das vernetzte Polymer vom Seitenkettentyp als ein Dotiermittel umfasst,
wobei der Wirt vorzugsweise eine Verbindung auf Anthracenbasis, eine Verbindung auf Fluorenbasis oder eine Verbindung auf Dibenzochrysenbasis ist.

14. Organisches Elektrolumineszenzelement gemäß Anspruch 12 oder 13, ferner umfassend mindestens eine von einer Elektronentransportschicht und einer Elektroneninjektionsschicht, angeordnet zwischen der negativen Elektrode und der lichtemittierenden Schicht, wobei mindestens eine von der Elektronentransportschicht und der Elektroneninjektionsschicht mindestens eines/einen, ausgewählt aus der Gruppe, bestehend aus einem Boranderivat, einem Pyridinderivat, einem Fluoranthenderivat, einem Derivat auf BO-Basis, einem Anthracenderivat, einem Benzofluorenderivat, einem Phosphinoxidderivat, einem Pyrimidinderivat, einem Carbazolderivat, einem Triazinderivat, einem Benzimidazolderivat, einem Phenanthrolinderivat und einem Metallkomplex auf Chinolin-Basis enthält,
wobei vorzugsweise mindestens eine von der Elektronentransportschicht und der Elektroneninjektionsschicht ferner mindestens eines, ausgewählt aus der Gruppe, bestehend aus einem Alkalimetall, einem Erdalkalimetall, einem Seltenerdmetall, einem Oxid eines Alkalimetalls, einem Halogenid eines Alkalimetalls, einem Oxid eines Erdalkalimetalls, einem Halogenid eines Erdalkalimetalls, einem Oxid eines Seltenerdmetalls, einem Halogenid eines Seltenerdmetalls, einem organischen Komplex eines Alkalimetalls, einem organischen Komplex eines Erdalkalimetalls und einem organischen Komplex eines Seltenerdmetalls, einschließt,
wobei weiter bevorzugt mindestens eine Schicht von einer Lochinjektionsschicht, einer Lochtransportschicht, einer lichtemittierenden Schicht, einer Elektronentransportschicht und einer Elektroneninjektionsschicht eine Polymerverbindung, die durch Polymerisieren einer Verbindung mit niedrigem Molekulargewicht, die jede der Schichten bilden kann, als ein Monomer erhalten wird, oder ein vernetztes Polymer, das durch weiteres Vernetzen der Polymerverbindung erhalten wird, oder eine Polymerverbindung vom Seitenkettentyp, die durch Reaktion der Verbindung mit niedrigem Molekulargewicht, die jede der Schichten bilden kann, und einem Polymer vom Hauptkettentyp erhalten wird, oder eine vernetztes Polymer vom Seitenkettentyp, das durch weiteres Vernetzen der Polymerverbindung vom Seitenkettentyp erhalten wird, umfasst.

15. Anzeigevorrichtung oder Beleuchtungsvorrichtung, umfassend das organische Elektrolumineszenzelement gemäß einem der Ansprüche 12 bis 14.

## Revendications

1. Composé aromatique polycyclique représenté par la formule générale (1) suivante ou multimère d'un composé aromatique polycyclique présentant une pluralité de structures, chacune représentée par la formule générale (1) suivante : 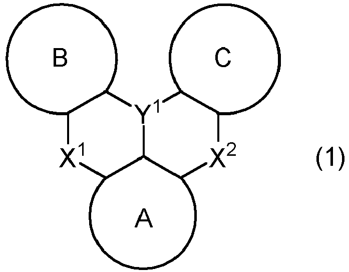
dans lequel, dans la formule (1) ci-dessus,
le cycle A, le cycle B et le cycle C représentent chacun indépendamment un cycle aryle ou un cycle hétéroaryle, tandis qu'au moins un atome d'hydrogène dans ces cycles peut être substitué,
Y¹ représente B, P, P=O, P=S, AI, Ga, As, Si-R, ou Ge-R, tandis que R dans Si-R et Ge-R représente un aryle, un alkyle ou un cycloalkyle,
X¹ et X² représentent chacun indépendamment >O, >N-R, >C(-R)₂, >S ou >Se, tandis que R dans >N-R représente un aryle facultativement substitué, un hétéroaryle facultativement substitué, un alkyle facultativement substitué, ou un cycloalkyle facultativement substitué, R dans >C(-R)₂ représente un atome d'hydrogène, un aryle facultativement substitué, un alkyle facultativement substitué ou un cycloalkyle facultativement substitué, et au moins un parmi R dans >N-R et R dans >C(-R) ₂ peut être lié à au moins l'un parmi le cycle A, le cycle B et le cycle C via 'un groupe de liaison ou une liaison simple,
au moins un atome d'hydrogène dans le composé ou la structure représenté par la formule (1) peut être substitué par un atome de deutérium, un cyano ou un atome d'halogène, et
au moins l'un parmi le cycle A, le cycle B, le cycle C, l'aryle et l'hétéroaryle dans le composé ou la structure représenté par la formule (1) est condensé à au moins un cycloalcane, au moins un atome d'hydrogène dans le cycloalcane est substitué à un atome de carbone en position α du cycloalcane, au moins un -CH₂- dans le cycloalcane peut être substitué par -O-.

2. Composé aromatique polycyclique ou multimère de celui-ci selon la revendication 1, dans lequel
dans la formule (1) ci-dessus,
le cycle A, le cycle B et le cycle C représentent chacun indépendamment un cycle aryle ou un cycle hétéroaryle, tandis qu'au moins un atome d'hydrogène dans ces cycles peut être substitué par un aryle substitué ou non substitué, un hétéroaryle substitué ou non substitué, un diarylamino substitué ou non substitué, un dihétéroarylamino substitué ou non substitué, un arylhétéroarylamino substitué ou non substitué, un diarylboryle substitué ou non (les deux aryles peuvent être liés par une liaison simple ou un groupe de liaison), un alkyle substitué ou non substitué, un cycloalkyle substitué ou non substitué, un alcoxy substitué ou non substitué, un aryloxy substitué ou non substitué, ou un silyle substitué, et chacun de ces cycles présente un cycle à 5 chaînons ou à 6 chaînons partageant une liaison avec une structure bicyclique condensée au centre de la formule ci-dessus constituée par Y', X¹, et X²,
Y¹ représente B, P, P=O, P=S, AI, Ga, As, Si-R, ou Ge-R, tandis que R dans Si-R et Ge-R représente un aryle, un alkyle ou un cycloalkyle,
X¹ et X² représentent chacun indépendamment >O, >N-R, >C(-R)₂, >S, ou >Se, tandis que R dans >N-R représente un aryle facultativement substitué avec un alkyle ou un cycloalkyle, un hétéroaryle facultativement substitué avec un alkyle ou un cycloalkyle, un alkyle ou un cycloalkyle, R dans >C(-R)₂ représente un atome d'hydrogène, un aryle facultativement substitué avec un alkyle ou un cycloalkyle, un alkyle ou un cycloalkyle, au moins un parmi R dans >N-R et R dans >C(-R)₂ peut être lié à au moins l'un parmi le cycle A, le cycle B et le cycle C via -O-, -S-, -C(-R)₂-, ou une liaison simple, et R dans -C(-R)₂- représente un atome d'hydrogène ou un alkyle ou un cycloalkyle,
au moins un atome d'hydrogène dans le composé ou la structure représenté par la formule (1) peut être substitué par un atome de deutérium, un cyano ou un atome d'halogène,
dans un cas d'un multimère, le multimère est un dimère ou un trimère présentant deux ou trois structures représentées chacune par la formule générale (1), et
au moins l'un parmi le cycle A, le cycle B, le cycle C, l'aryle et l'hétéroaryle dans le composé ou la structure représenté par la formule (1) est condensé à au moins un cycloalcane, au moins un atome d'hydrogène dans le cycloalcane est substitué à un atome de carbone en position α du cycloalcane, au moins un -CH₂- dans le cycloalcane peut être substitué par -O-.

3. Composé aromatique polycyclique selon la revendication 1, représenté par la formule générale (2) suivante : 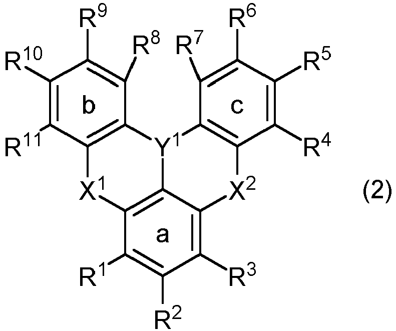
dans lequel dans la formule (2) ci-dessus,
n'importe quel « -C(-R)= » (où R est R¹ à R¹¹ dans la formule (2)) dans le cycle a, le cycle b et le cycle c peut être remplacé par « -N= », n'importe quel « -C(-R)=C(-R)- » (où R est R¹ à R¹¹ dans la formule (2)) peut être remplacé par « -N(-R)- », « - O- », ou « -S- », R dans « -N(-R)- » représente un aryle, un alkyle, ou un cycloalkyle,
R¹ à R¹¹ représentent chacun indépendamment un atome d'hydrogène, un aryle, un hétéroaryle, un diarylamino, un dihétéroarylamino, un arylhétéroarylamino, un diarylboryle (les deux aryles peuvent être liés par l'intermédiaire d'une liaison simple ou d'un groupe de liaison), un alkyle, un cycloalkyle, un alcoxy, un aryloxy, un triarylsilyle, un trialkylsilyle, un tricycloalkylsilyle, un dialkylcycloalkylsilyle ou un alkyldicycloalkylsilyle, tandis qu'au moins un atome d'hydrogène de ceux-ci peut être substitué par un aryle, un hétéroaryle, un alkyle ou un cycloalkyle, des groupes adjacents parmi R¹ à R¹¹ peuvent être liés les uns aux autres pour former un cycle aryle ou un cycle hétéroaryle avec le cycle a, le cycle b ou le cycle c, au moins un atome d'hydrogène dans le cycle ainsi formé peut être substitué par un aryle, un hétéroaryle, un diarylamino, un dihétéroarylamino, un arylhétéroarylamino, un diarylboryle (les deux aryles peuvent être liés par l'intermédiaire d'une liaison simple ou d'un groupe de liaison), un alkyle, un cycloalkyle, un alcoxy, un aryloxy, un triarylsilyle, un trialkylsilyle, un tricycloalkylsilyle, un dialkylcycloalkylsilyle ou un alkyldicycloalkylsilyle, tandis qu'au moins un atome d'hydrogène de ceux-ci peut être substitué par un aryle, un hétéroaryle, un alkyle ou un cycloalkyle,
Y¹ représente B, P, P=O, P=S, Al, Ga, As, Si-R, ou Ge-R, tandis que R dans Si-R et Ge-R représente un aryle présentant 6 à 12 atomes de carbone, un alkyle présentant 1 à 6 atomes de carbone ou un cycloalkyle présentant 3 à 14 atomes de carbone,
X¹ et X² représentent chacun indépendamment >O, >N-R, >C(-R)₂, >S ou >Se, tandis que R dans >N-R représente un aryle présentant 6 à 12 atomes de carbone, un hétéroaryle présentant 2 à 15 atomes de carbone, un alkyle présentant 1 à 6 atomes de carbone, ou un cycloalkyle présentant 3 à 14 atomes de carbone, R dans >C(-R)₂ représente un atome d'hydrogène, un aryle présentant 6 à 12 atomes de carbone, un alkyle présentant 1 à 6 atomes de carbone, ou un cycloalkyle présentant 3 à 14 atomes de carbone, au moins l'un parmi R dans >N-R et R dans >C(-R)₂ peut être lié à au moins l'un parmi le cycle a, le cycle b et le cycle c via - O-, -S-, -C(-R)₂-, ou une liaison simple, et R dans -C(-R)₂- représente un alkyle présentant 1 à 6 atomes de carbone ou un cycloalkyle présentant 3 à 14 atomes de carbone.
au moins un atome d'hydrogène dans le composé représenté par la formule (2) peut être substitué par un atome de deutérium, un cyano ou un atome d'halogène, et
au moins l'un parmi le cycle a, le cycle b, le cycle c, un cycle formé, un aryle et un hétéroaryle dans le composé représenté par la formule (2) est condensé à au moins un cycloalcane présentant 3 à 24 atomes de carbone, au moins un atome d'hydrogène dans le cycloalcane est substitué à un atome de carbone en position α du cycloalcane par un aryle présentant 6 à 30 atomes de carbone, un hétéroaryle présentant 2 à 30 atomes de carbone, un alkyle présentant 1 à 24 atomes de carbone, ou un cycloalkyle présentant 3 à 24 atomes de carbone, au moins un -CH₂- dans le cycloalcane peut être substitué par -O-.

4. Composé aromatique polycyclique selon la revendication 3, dans lequel
dans la formule (2) ci-dessus,
n'importe quel « -C(-R)= » (où R est R¹ à R¹¹ dans la formule (2)) dans le cycle a, le cycle b et le cycle c peut être remplacé par « -N= », n'importe quel « -C(-R)=C(-R)- » (où R est R¹ à R¹¹ dans la formule (2)) peut être remplacé par « -N(-R)- », « - O- », ou « -S- », R dans « -N(-R)- » représente un aryle présentant 6 à 12 atomes de carbone, un alkyle présentant 1 à 6 atomes de carbone, ou un cycloalkyle présentant 3 à 14 atomes de carbone,
R¹ à R¹¹ représentent chacun indépendamment un atome d'hydrogène, un aryle présentant 6 à 30 atomes de carbone, un hétéroaryle présentant 2 à 30 atomes de carbone, un diarylamino (l'aryle est un aryle présentant 6 à 12 atomes de carbone), un diarylboryle (l'aryle est un aryle présentant 6 à 12 atomes de carbone, les deux aryles peuvent être liés par l'intermédiaire d'une liaison simple ou d'un groupe de liaison), un alkyle présentant 1 à 24 atomes de carbone, un cycloalkyle présentant 3 à 24 atomes de carbone, un triarylsilyle (l'aryle est un aryle présentant 6 à 12 atomes de carbone), ou un trialkylsilyle (l'alkyle est un alkyle présentant 1 à 6 atomes de carbone), des groupes adjacents parmi R¹ à R¹¹ peuvent être liés les uns aux autres pour former un cycle aryle présentant 9 à 16 atomes de carbone ou un cycle hétéroaryle présentant 6 à 15 atomes de carbone conjointement avec le cycle a, le cycle b ou le cycle c, au moins un atome d'hydrogène dans le cycle ainsi formé peut être substitué par un aryle présentant 6 à 10 atomes de carbone, un alkyle présentant 1 à 12 atomes de carbone, un cycloalkyle présentant 3 à 16 atomes de carbone, un triarylsilyle (l'aryle est un aryle présentant 6 à 12 atomes de carbone), ou un trialkylsilyle (l'alkyle est un alkyle présentant 1 à 5 atomes de carbone),
Y¹ représente B, P, P=O, P=S, ou Si-R, tandis que R dans Si-R représente un aryle présentant 6 à 10 atomes de carbone, un alkyle présentant 1 à 5 atomes de carbone ou un cycloalkyle présentant 5 à 10 atomes de carbone,
X¹ et X² représentent chacun indépendamment >O, >N-R, >C(-R)₂, ou >S, tandis que R dans >N-R représente un aryle présentant 6 à 10 atomes de carbone, un alkyle présentant 1 à 5 atomes de carbone, ou un cycloalkyle présentant 5 à 10 atomes de carbone, R dans >C(-R)₂ représente un atome d'hydrogène, un aryle présentant 6 à 10 atomes de carbone, un alkyle présentant 1 à 5 atomes de carbone ou un cycloalkyle présentant 5 à 10 atomes de carbone les atomes,
au moins un atome d'hydrogène dans le composé représenté par la formule (2) peut être substitué par un atome de deutérium, un cyano ou un atome d'halogène, et
au moins l'un parmi le cycle a, le cycle b, le cycle c, un cycle formé, un aryle et un hétéroaryle dans le composé représenté par la formule (2) est condensé à au moins un cycloalcane présentant 3 à 20 atomes de carbone, au moins un atome d'hydrogène dans le cycloalcane est substitué à un atome de carbone en position α du cycloalcane par un aryle présentant 6 à 16 atomes de carbone,
un hétéroaryle présentant 2 à 22 atomes de carbone, un alkyle présentant 1 à 12 atomes de carbone, ou un cycloalkyle présentant 3 à 16 atomes de carbone
ou dans lequel
dans la formule (2) ci-dessus,
n'importe quel « -C(-R)= » (où R est R¹ à R¹¹ dans la formule (2)) dans le cycle a, le cycle b et le cycle c peut être remplacé par « -N= », n'importe quel « -C(-R)=C(-R)- » (où R est R¹ à R¹¹ dans la formule (2)) peut être remplacé par « -N(-R)- », « - O- », ou « -S- », R dans « -N(-R)- » représente un aryle présentant 6 à 10 atomes de carbone, un alkyle présentant 1 à 5 atomes de carbone, ou un cycloalkyle présentant 5 à 10 atomes de carbone,
R¹ à R¹¹ représentent chacun indépendamment un atome d'hydrogène, un aryle présentant 6 à 16 atomes de carbone, un hétéroaryle présentant 2 à 20 atomes de carbone, un diarylamino (l'aryle est un aryle présentant 6 à 10 atomes de carbone), un alkyle présentant 1 à 12 atomes de carbone, ou un cycloalkyle présentant 3 à 16 atomes de carbone,
Y¹ représente B, P, P=O, ou P=S,
X¹ et X² représentent chacun indépendamment >O, >N-R, ou >C(-R)₂, tandis que R dans >N-R représente un aryle présentant 6 à 10 atomes de carbone, un alkyle présentant 1 à 5 atomes de carbone, ou un cycloalkyle présentant 5 à 10 atomes de carbone, R dans >C(-R)₂ représente un atome d'hydrogène, un aryle présentant 6 à 10 atomes de carbone, un alkyle présentant 1 à 5 atomes de carbone, ou un cycloalkyle présentant 5 à 10 atomes de carbone,
au moins un atome d'hydrogène dans le composé représenté par la formule (2) peut être substitué par un atome de deutérium, un cyano ou un atome d'halogène, et
au moins l'un parmi le cycle a, le cycle b, le cycle c, et l'aryle présentant 6 à 10 atomes de carbone en tant que R dans >N-R dans le composé représenté par la formule (2) est condensé à au moins un cycloalcane présentant 3 à 16 atomes de carbone atomes, au moins un atome d'hydrogène dans le cycloalcane est substitué à un atome de carbone en position α du cycloalcane par un alkyle présentant 1 à 6 atomes de carbone ou un cycloalkyle présentant 3 à 14 atomes de carbone
ou dans lequel
dans la formule (2) ci-dessus,
R¹ à R¹¹ représentent chacun indépendamment un atome d'hydrogène, un aryle présentant 6 à 16 atomes de carbone, un diarylamino (l'aryle est un aryle présentant 6 à 10 atomes de carbone), un alkyle présentant 1 à 12 atomes de carbone, ou un cycloalkyle présentant 3 à 16 atomes de carbone,
Y¹ représente B,
X¹ et X² représentent tous les deux >N-R, ou X¹ représente >N-R et X² représente >O, tandis que R dans >N-R représente un aryle présentant 6 à 10 atomes de carbone, un alkyle présentant 1 à 5 atomes de carbone ou un cycloalkyle présentant 5 à 10 atomes de carbone,
au moins un atome d'hydrogène dans le composé représenté par la formule (2) peut être substitué par un atome de deutérium ou un atome d'halogène, et
au moins l'un parmi le cycle a, le cycle b, le cycle c, et l'aryle présentant 6 à 10 atomes de carbone en tant que R dans >N-R dans le composé représenté par la formule (2) est condensé à au moins un cycloalcane présentant 3 à 14 atomes de carbone atomes, au moins un atome d'hydrogène dans le cycloalcane est substitué à un atome de carbone en position α du cycloalcane par un alkyle présentant 1 à 5.

5. Composé aromatique polycyclique ou multimère de celui-ci selon l'une quelconque des revendications 1 à 4, qui est substitué avec un groupe diarylamino condensé à un ou plusieurs cycloalcanes, un groupe carbazolyle condensé à un ou plusieurs cycloalcanes, ou un groupe benzocarbazolyle condensé à un ou plusieurs cycloalcanes.

6. Composé aromatique polycyclique selon la revendication 3 ou la revendication 4, dans lequel R² est un groupe diarylamino condensé à un ou plusieurs cycloalcanes, ou un groupe carbazolyle condensé à un ou plusieurs cycloalcanes.

7. Composé aromatique polycyclique selon la revendication 5 ou la revendication 6, dans lequel le cycloalcane est un cycloalcane présentant 3 à 20 atomes de carbone.

8. Composé aromatique polycyclique ou multimère de celui-ci selon l'une quelconque des revendications 1 à 6, dans lequel l'halogène est un fluor.

9. Composé aromatique polycyclique selon la revendication 1, qui est représenté par l'une quelconque des formules développées suivantes : 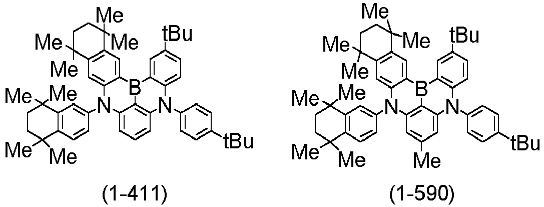 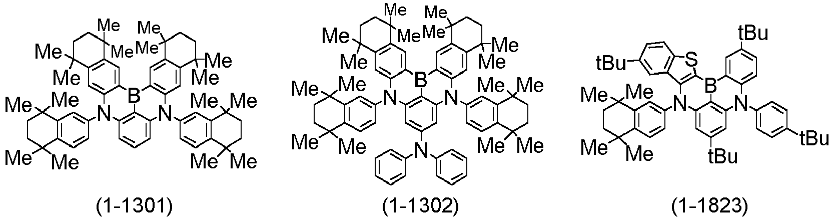 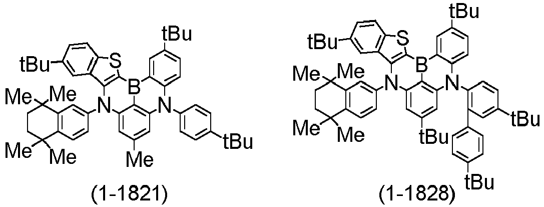 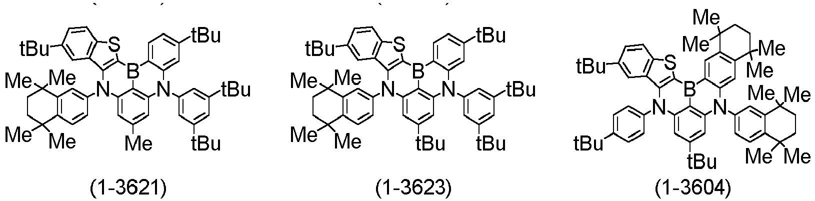 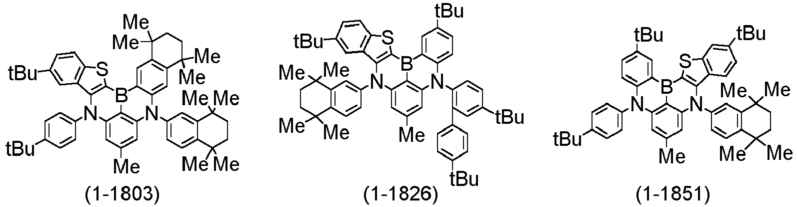 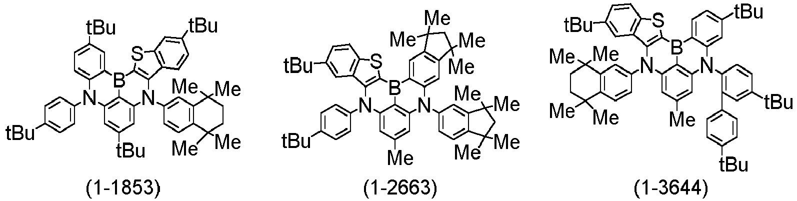 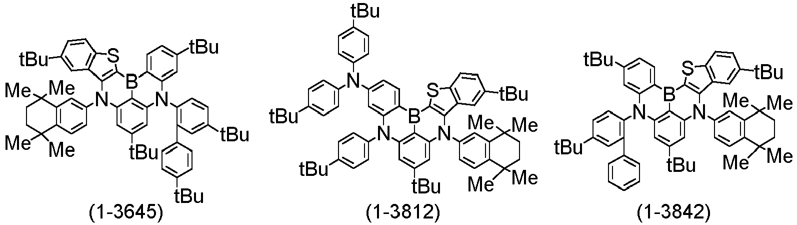 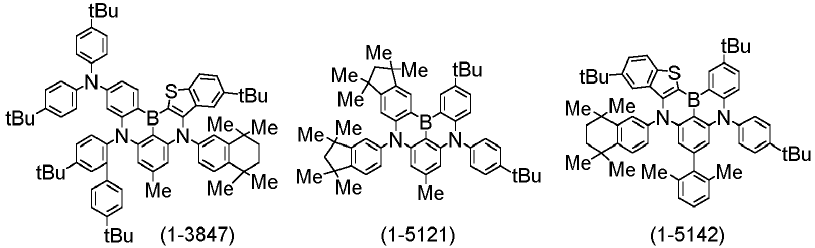 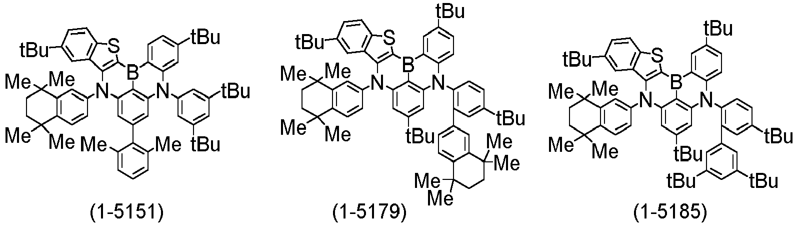 dans lequel dans chacune des formules développées ci-dessus, « Me » indique un groupe méthyle et « tBu » indique un groupe t-butyle.

10. Matériau pour dispositif organique, le matériau comprenant le composé aromatique polycyclique ou le multimère de celui-ci selon l'une quelconque des revendications 1 à 9.

11. Matériau pour dispositif organique selon la revendication 10, qui est un matériau pour élément électroluminescent organique, un matériau pour transistor organique à effet de champ, ou un matériau pour cellule solaire à couches minces organiques,
de préférence dans lequel le matériau pour élément électroluminescent organique est un matériau pour couche électroluminescente.

12. Élément électroluminescent organique comprenant : une paire d'électrodes composée d'une électrode positive et d'une électrode négative ; et une couche organique disposée entre la paire d'électrodes et comprenant le composé aromatique polycyclique ou le multimère de celui-ci selon l'une quelconque des revendications 1 à 9.

13. Élément électroluminescent organique selon la revendication 12, dans lequel la couche organique est une couche électroluminescente,
de préférence dans lequel la couche électroluminescente comprend un hôte et le composé aromatique polycyclique ou le multimère de celui-ci, le composé réactif, le composé polymère, le polymère réticulé, le composé polymère de type pendant, ou le polymère réticulé de type pendant en tant que dopant,
de préférence dans lequel l'hôte est un composé à base d'anthracène, un composé à base de fluorène ou un composé à base de dibenzochrysène.

14. Élément électroluminescent organique selon la revendication 12 ou la revendication 13, comprenant en outre au moins une parmi une couche de transport d'électrons et une couche d'injection d'électrons disposée entre l'électrode négative et la couche électroluminescente, dans lequel au moins l'une parmi la couche de transport d'électrons et la couche d'injection d'électrons contient au moins un sélectionné dans le groupe consistant en un dérivé de borane, un dérivé de pyridine, un dérivé de fluoranthène, un dérivé à base de BO, un dérivé d'anthracène, un dérivé de benzofluorène, un dérivé d'oxyde de phosphine, un dérivé de pyrimidine, un dérivé de carbazole, un dérivé de triazine, un dérivé de benzimidazole, un dérivé de phénanthroline et un complexe métallique à base de quinolinol,
de préférence dans lequel au moins une couche parmi la couche de transport d'électrons et la couche d'injection d'électrons inclut en outre au moins un sélectionné dans le groupe consistant en un métal alcalin, un métal alcalino-terreux, un métal des terres rares,
un oxyde d'un métal alcalin, un halogénure d'un métal alcalin, un oxyde d'un métal alcalino-terreux, un halogénure d'un métal alcalino-terreux, un oxyde d'un métal des terres rares, un halogénure d'un métal des terres rares, un complexe organique d'un métal alcalin, un complexe organique d'un métal alcalino-terreux et un complexe organique d'un métal des terres rares,
de manière davantage préférée dans lequel au moins une couche parmi une couche d'injection de trous, une couche de transport de trous, une couche électroluminescente, une couche de transport d'électrons et une couche d'injection d'électrons comprend un composé polymère obtenu par polymérisation d'un composé de poids moléculaire bas qui peut former chacune des couches en tant que monomère ou un polymère réticulé obtenu par la réticulation supplémentaire du composé polymère, ou un composé polymère de type pendant obtenu par une réaction entre le composé de poids moléculaire bas qui peut former chacune des couches et un polymère de type à chaîne principale ou un polymère réticulé de type pendant obtenu par la réticulation supplémentaire du composé polymère de type pendant.

15. Appareil d'affichage ou appareil d'éclairage comprenant l'élément électroluminescent organique selon l'une quelconque des revendications 12 à 14.
